(19) Europäisches Patentamt
European Patent Office
Office européen des brevets

(11) EP 4 768 045 A2

(12) # EUROPEAN PATENT APPLICATION

(43) Date of publication:
**01.07.2026 Bulletin 2026/27**

(21) Application number: **26177707.2**

(22) Date of filing: **27.05.2022**

(51) International Patent Classification (IPC):
***A61K 47/54*** *(2017.01)*

(52) Cooperative Patent Classification (CPC):
(C-Sets available)
**C12N 15/1137; A61K 31/713; A61K 47/542; A61K 47/549;** C12N 2310/14; C12N 2310/315; C12N 2310/323; C12N 2310/3341; C12N 2310/351; C12N 2310/3515; C12Y 402/01 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.05.2021 US 202163194073 P**
**14.12.2021 US 202163289319 P**

(62) Document number(s) of the earlier application(s) in accordance with Art. 76 EPC:
**22812254.5 / 4 347 836**

(71) Applicant: **Alnylam Pharmaceuticals, Inc.
Cambridge, MA 02142 (US)**

(72) Inventors:
- **Zuber,, Jeffrey
Cambridge 02142 (US)**
- **McIninch,, James D.
Cambridge 02142 (US)**
- **Schlegel,, Mark K.
Cambridge 02142 (US)**
- **Castoreno,, Adam
Cambridge 02142 (US)**

(74) Representative: **Potter Clarkson
Chapel Quarter
Mount Street
Nottingham NG1 6HQ (GB)**

Remarks:
This application was filed on 08-05-2026 as a divisional application to the application mentioned under INID code 62.

(54) **COMPOSITIONS AND METHODS FOR SILENCING CARBONIC ANHYDRASE 2 EXPRESSION**

(57) The disclosure relates to double-stranded ribonucleic acid (dsRNA) compositions targeting carbonic anhydrase 2 (CA2), and methods of using such dsRNA compositions to alter (e.g., inhibit) expression of carbonic anhydrase 2.



Processed by Luminess, 75001 PARIS (FR)

**(Cont. next page)**

(52) Cooperative Patent Classification (CPC): (Cont.)

C-Sets
C12N 2310/321, C12N 2310/3521;
C12N 2310/321, C12N 2310/3525;
C12N 2310/321, C12N 2310/3527;
C12N 2310/322, C12N 2310/3533

## Description

### CROSS-REFERENCE TO RELATED APPLICATIONS

This application claims the benefit of priority to U.S. Provisional Application No. 63/194,073, filed on May 27, 2021, and claims the benefit of priority to U.S. Provisional Application No. 63/289,319, filed on December 14, 2021. The entire contents of the foregoing applications are hereby incorporated herein by reference.

### FIELD OF THE DISCLOSURE

The disclosure relates to the specific inhibition of the expression of carbonic anhydrase 2.

### BACKGROUND OF THE INVENTION

Glaucoma is a leading cause of vision loss. Risk factors for glaucoma include increased intraocular pressure, age, race and vascular disease. The increased intraocular pressure may cause damage to the optic nerve and loss of never fibers. Lowering intraocular pressure can reduce development and progression of vision loss.

Carbonic anhydrase 2 (CA2) is a member of the carbonic anhydrase (CA) family of metalloenzymes. CA2 catalyzes the reversible conversion of carbon dioxide to bicarbonate. Carbonic anhydrases are expressed in the eye and CA2 appears to be the main CA form present in human ciliary epithelium which is responsible for producing aqueous humor. Carbonic anhydrase inhibitors have been shown to reduce aqueous humor production and thereby reduce intraocular pressure in the eye. Accordingly, there is a need for agents that can selectively and efficiently inhibit expression of the CA2 gene such that subjects having a CA2-associated disorder, such as glaucoma, can be effectively treated.

### BRIEF SUMMARY OF THE INVENTION

The present disclosure describes methods and iRNA compositions for modulating the expression of carbonic anhydrase 2 (CA2). In certain embodiments, expression of CA2 is reduced or inhibited using a CA2-specific iRNA. Such inhibition can be useful in treating disorders related to CA2 expression, such as ocular disorders (e.g., glaucoma or conditions associated with glaucoma).

Accordingly, described herein are compositions and methods that effect the RNA-induced silencing complex (RISC)-mediated cleavage of RNA transcripts of CA2, such as in a cell or in a subject (e.g., in a mammal, such as a human subject). Also described are compositions and methods for treating a disorder related to expression of CA2, such as glaucoma or conditions associated with glaucoma.

The iRNAs (e.g., dsRNAs) included in the compositions featured herein include an RNA strand (the antisense strand) having a region, *e.g.,* a region that is 30 nucleotides or less, generally 19-24 nucleotides in length, that is substantially complementary to at least part of an mRNA transcript of CA2 *(e.g.,* a human CA2) (also referred to herein as a "CA2-specific iRNA"). In some embodiments, the CA2 mRNA transcript is a human CA2 mRNA transcript, *e.g.,* SEQ ID NO: 1 herein.

In some embodiments, the iRNA (e.g., dsRNA) described herein comprises an antisense strand having a region that is substantially complementary to a region of a human CA2 mRNA. In some embodiments, the human CA2 mRNA has the sequence NM_000067.3 (SEQ ID NO: 1). The sequence of NM_000067.3is also herein incorporated by reference in its entirety. The reverse complement of SEQ ID NO: 1 is provided as SEQ ID NO: 2 herein.

In some aspects, the present disclosure provides a double stranded ribonucleic acid (dsRNA) agent for inhibiting expression of carbonic anhydrase 2 (CA2), wherein the dsRNA agent comprises a sense strand and an antisense strand forming a double stranded region, wherein the sense strand comprises a nucleotide sequence comprising at least 15 contiguous nucleotides, with 0, 1, 2, or 3 mismatches, of a portion of a coding strand of human CA2 and the antisense strand comprises a nucleotide sequence comprising at least 15 contiguous nucleotides, with 0, 1, 2, or 3 mismatches, of the corresponding portion of a non-coding strand of human CA2 such that the sense strand is complementary to the at least 15 contiguous nucleotides in the antisense strand.

In some aspects, the present disclosure provides a double stranded ribonucleic acid (dsRNA) agent for inhibiting expression of CA2, wherein the dsRNA agent comprises a sense strand and an antisense strand forming a double stranded region, wherein the antisense strand comprises a nucleotide sequence comprising at least 15 contiguous nucleotides, with 0, 1, 2, or 3 mismatches, of a portion of nucleotide sequence of SEQ ID NO: 2 such that the sense strand is complementary to the at least 15 contiguous nucleotides in the antisense strand.

In some aspects, the present disclosure provides a human cell or tissue comprising a reduced level of CA2 mRNA or a level of CA2 protein as compared to an otherwise similar untreated cell or tissue, wherein optionally the cell or tissue is not genetically engineered (e.g., wherein the cell or tissue comprises one or more naturally arising mutations, e.g., CA2),

wherein optionally the level is reduced by at least 10%, 15%, 20%, 25%, 30%, 35%, 40%, 45%, 50%, 55%, 60%, 65%, 70%, 75%, 80%, 85%, 90%, or 95%. In some embodiments, the human cell or tissue is a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an astrocyte, a pericyte, a Miiller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel.

The present disclosure also provides, in some aspects, a cell containing the dsRNA agent described herein.

In another aspect, provided herein is a human ocular cell, *e.g.,* (a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an astrocyte, a pericyte, a Miiller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel) comprising a reduced level of CA2 mRNA or a level of CA2 protein as compared to an otherwise similar untreated cell. In some embodiments, the level is reduced by at least 10%, 15%, 20%, 25%, 30%, 35%, 40%, 45%, 50%, 55%, 60%, 65%, 70%, 75%, 80%, 85%, 90%, or 95%.

In some aspects, the present disclosure also provides a pharmaceutical composition for inhibiting expression of a gene encoding CA2, comprising a dsRNA agent described herein.

The present disclosure also provides, in some aspects, a method of inhibiting expression of CA2 in a cell, the method comprising:

(a) contacting the cell with the dsRNA agent described herein, or a pharmaceutical composition described herein; and
(b) maintaining the cell produced in step (a) for a time sufficient to obtain degradation of the mRNA transcript of CA2, thereby inhibiting expression of the CA2 in the cell.

The present disclosure also provides, in some aspects, a method of inhibiting expression of CA2 in a cell, the method comprising:

(a) contacting the cell with the dsRNA agent described herein, or a pharmaceutical composition described herein; and
(b) maintaining the cell produced in step (a) for a time sufficient to reduce levels of CA2 mRNA, CA2 protein, or both of CA2 mRNA and protein, thereby inhibiting expression of the CA2 in the cell.

The present disclosure also provides, in some aspects, a method of inhibiting expression of CA2 in an ocular cell or tissue, the method comprising:

(a) contacting the cell or tissue with a dsRNA agent that binds CA2; and
(b) maintaining the cell or tissue produced in step (a) for a time sufficient to reduce levels of CA2 mRNA, CA2 protein, or both of CA2 mRNA and protein, thereby inhibiting expression of CA2 in the cell or tissue.

The present disclosure also provides, in some aspects, a method of treating a subject diagnosed with a CA2-associated disorder comprising administering to the subject a therapeutically effective amount of the dsRNA agent described herein or a pharmaceutical composition described herein, thereby treating the disorder.

In any of the aspects herein, *e.g.,* the compositions and methods above, any of the embodiments herein (e.g., below) may apply.

In some embodiments, the coding strand of human CA2 has the sequence of SEQ ID NO: 1. In some embodiments, the non-coding strand of human CA2 has the sequence of SEQ ID NO: 2.

In some embodiments, the sense strand comprises a nucleotide sequence comprising at least 15 contiguous nucleotides, with 0, or 1, 2, or 3 mismatches, of the corresponding portion of the nucleotide sequence of SEQ ID NO: 1.

In some embodiments, the dsRNA agent comprises a sense strand and an antisense strand, wherein the antisense strand comprises a nucleotide sequence comprising at least 17 contiguous nucleotides, with 0, 1, 2, or 3 mismatches, of a portion of nucleotide sequence of SEQ ID NO: 2 such that the sense strand is complementary to the at least 17 contiguous nucleotides in the antisense strand. In some embodiments, the sense strand comprises a nucleotide sequence comprising at least 17 contiguous nucleotides, with 0, or 1, 2, or 3 mismatches, of the corresponding portion of the nucleotide sequence of SEQ ID NO: 1.

In some embodiments, the dsRNA agent comprises a sense strand and an antisense strand, wherein the antisense strand comprises a nucleotide sequence comprising at least 19 contiguous nucleotides, with 0, 1, 2, or 3 mismatches, of a portion of nucleotide sequence of SEQ ID NO: 2 such that the sense strand is complementary to the at least 19 contiguous nucleotides in the antisense strand. In some embodiments, the sense strand comprises a nucleotide sequence comprising at least 19 contiguous nucleotides, with 0, 1, 2, or 3 mismatches, of the corresponding portion of the nucleotide sequence of SEQ ID NO: 1.

In some embodiments, the dsRNA agent comprises a sense strand and an antisense strand, wherein the antisense strand comprises a nucleotide sequence comprising at least 21 contiguous nucleotides, with 0, 1, 2, or 3 mismatches, of a portion of nucleotide sequence of SEQ ID NO: 2 such that the sense strand is complementary to the at least 21 contiguous nucleotides in the antisense strand. In some embodiments, the sense strand comprises a nucleotide sequence comprising at least 21 contiguous nucleotides, with 0, or 1, 2, or 3 mismatches, of the corresponding portion of the nucleotide sequence of SEQ ID NO: 1.

In some embodiments, the portion of the sense strand is a portion within a sense strand in any one of Tables 3-10.

In some embodiments, the portion of the antisense strand is a portion within an antisense strand in any one of Tables 3-10.

In some embodiments, the antisense strand comprises a nucleotide sequence comprising at least 15 contiguous nucleotides, with 0, 1, 2, or 3 mismatches, from one of the antisense sequences listed in any one of Tables 3-10. In some embodiments, the sense strand comprises a nucleotide sequence comprising at least 15 contiguous nucleotides, with 0, 1, 2, or 3 mismatches, from a sense sequence listed in any one of Tables 3-10 that corresponds to the antisense sequence.

In some embodiments, the antisense strand comprises a nucleotide sequence comprising at least 17 contiguous nucleotides, with 0, 1, 2, or 3 mismatches, from one of the antisense sequences listed in any one of Tables 3-10. In some embodiments, the sense strand comprises a nucleotide sequence comprising at least 17 contiguous nucleotides, with 0, 1, 2, or 3 mismatches, from a sense sequence listed in any one of Tables 3-10 that corresponds to the antisense sequence.

In some embodiments, the antisense strand comprises a nucleotide sequence comprising at least 19 contiguous nucleotides, with 0, 1, 2, or 3 mismatches, from one of the antisense sequences listed in any one of Tables 3-10. In some embodiments, the sense strand comprises a nucleotide sequence comprising at least 19 contiguous nucleotides, with 0, 1, 2, or 3 mismatches, from a sense sequence listed in any one of Tables 3-10 that corresponds to the antisense sequence.

In some embodiments, the antisense strand comprises a nucleotide sequence comprising at least 21 contiguous nucleotides, with 0, 1, 2, or 3 mismatches, from one of the antisense sequences listed in any one of Tables 3-10. In some embodiments, the sense strand comprises a nucleotide sequence comprising at least 21 contiguous nucleotides, with 0, 1, 2, or 3 mismatches, from a sense sequence listed in any one of Tables 3-10 that corresponds to the antisense sequence.

In some embodiments, the sense strand of the dsRNA agent is at least 23 nucleotides in length, *e.g.,* 23-30 nucleotides in length.

In some embodiments, at least one of the sense strand and the antisense strand is conjugated to one or more lipophilic moieties. In some embodiments, the lipophilic moiety is conjugated to one or more positions in the double stranded region of the dsRNA agent. In some embodiments, the lipophilic moiety is conjugated via a linker or carrier. In some embodiments, lipophilicity of the lipophilic moiety, measured by logKow, exceeds 0. In some embodiments, In some embodiments, the hydrophobicity of the double-stranded RNAi agent, measured by the unbound fraction in a plasma protein binding assay of the double-stranded RNAi agent, exceeds 0.2. In some embodiments, the plasma protein binding assay is an electrophoretic mobility shift assay using human serum albumin protein.

In some embodiments, the dsRNA agent comprises at least one modified nucleotide. In some embodiments, no more than five of the sense strand nucleotides and not more than five of the nucleotides of the antisense strand are unmodified nucleotides. In some embodiments, all of the nucleotides of the sense strand and all of the nucleotides of the antisense strand comprise a modification.

In some embodiments, at least one of the modified nucleotides is selected from the group consisting of a deoxy-nucleotide, a 3'-terminal deoxythimidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-C-alkyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, a nucleotide comprising a 5'-phosphate mimic, a glycol modified nucleotide, and a 2-O-(N-methylacetamide) modified nucleotide; and combinations thereof. In some embodiments, no more than five of the sense strand nucleotides and not more than five of the nucleotides of the antisense strand include modifications other than 2'-O-methyl modified nucleotide, a 2'-fluoro modified nucleotide, a 2'-deoxy-modified nucleotide, unlocked nucleic acids (UNA) or glycerol nucleic acid (GNA).

In some embodiments, the dsRNA comprises a non-nucleotide spacer (wherein optionally the non-nucleotide spacer comprises a C3-C6 alkyl) between two of the contiguous nucleotides of the sense strand or between two of the contiguous nucleotides of the antisense strand.

In some embodiments, each strand is no more than 30 nucleotides in length. In some embodiments, at least one strand comprises a 3' overhang of at least 1 nucleotide. In some embodiments, at least one strand comprises a 3' overhang of at least 2 nucleotides. In some embodiments, at least one strand comprises a 3' overhang of 2 nucleotides.

In some embodiments, the double stranded region is 15-30 nucleotide pairs in length. In some embodiments, the double stranded region is 17-23 nucleotide pairs in length. In some embodiments, the double stranded region is 17-25

nucleotide pairs in length. In some embodiments, the double stranded region is 23-27 nucleotide pairs in length. In some embodiments, the double stranded region is 19-21 nucleotide pairs in length. In some embodiments, the double stranded region is 21-23 nucleotide pairs in length. In some embodiments, each strand has 19-30 nucleotides. In some embodiments, each strand has 19-23 nucleotides. In some embodiments, each strand has 21-23 nucleotides.

In some embodiments, the agent comprises at least one phosphorothioate or methylphosphonate internucleotide linkage. In some embodiments, the phosphorothioate or methylphosphonate internucleotide linkage is at the 3'-terminus of one strand. In some embodiments, the strand is the antisense strand. In some embodiments, the strand is the sense strand.

In some embodiments, the phosphorothioate or methylphosphonate internucleotide linkage is at the 5'-terminus of one strand. In some embodiments, the strand is the antisense strand. In some embodiments, the strand is the sense strand.

In some embodiments, each of the 5'- and 3'-terminus of one strand comprises a phosphorothioate or methylphosphonate internucleotide linkage. In some embodiments, the strand is the antisense strand.

In some embodiments, the base pair at the 1 position of the 5'-end of the antisense strand of the duplex is an AU base pair.

In some embodiments, the sense strand has a total of 21 nucleotides and the antisense strand has a total of 23 nucleotides. In some embodiments, one or more lipophilic moieties are conjugated to one or more internal positions on at least one strand. In some embodiments, the one or more lipophilic moieties are conjugated to one or more internal positions on at least one strand via a linker or carrier.

In some embodiments conjugating a lipophilic moiety to one or more internal positions on at least one strand of the double-stranded iRNA agent provides surprisingly good results for in vivo intravitreal delivery of the double-stranded iRNAs, resulting in efficient entry into ocular tissues. Examples and synthesis of lipophilic moieties are listed in PCT application number PCT/US2019/031170 which is hereby incorporated by reference in its entirety.

In some embodiments, the internal positions include all positions except the terminal two positions from each end of the at least one strand. In some embodiments, the internal positions include all positions except the terminal three positions from each end of the at least one strand. In some embodiments, the internal positions exclude a cleavage site region of the sense strand. In some embodiments, the internal positions include all positions except positions 9-12, counting from the 5'-end of the sense strand. In some embodiments, the internal positions include all positions except positions 11-13, counting from the 3'-end of the sense strand. In some embodiments, the internal positions exclude a cleavage site region of the antisense strand. In some embodiments, the internal positions include all positions except positions 12-14, counting from the 5'-end of the antisense strand. In some embodiments, the internal positions include all positions except positions 11-13 on the sense strand, counting from the 3'-end, and positions 12-14 on the antisense strand, counting from the 5'-end.

In some embodiments, the one or more lipophilic moieties are conjugated to one or more of the internal positions selected from the group consisting of positions 4-8 and 13-18 on the sense strand, and positions 6-10 and 15-18 on the antisense strand, counting from the 5'end of each strand. In some embodiments, the one or more lipophilic moieties are conjugated to one or more of the internal positions selected from the group consisting of positions 5, 6, 7, 15, and 17 on the sense strand, and positions 15 and 17 on the antisense strand, counting from the 5'-end of each strand.

In some embodiments, the positions in the double stranded region exclude a cleavage site region of the sense strand.

In some embodiments, the sense strand is 21 nucleotides in length, the antisense strand is 23 nucleotides in length, and the lipophilic moiety is conjugated to position 21, position 20, position 15, position 1, position 7, position 6, or position 2 of the sense strand or position 16 of the antisense strand. In some embodiments, the lipophilic moiety is conjugated to position 21, position 20, position 15, position 1, or position 7 of the sense strand. In some embodiments, the lipophilic moiety is conjugated to position 21, position 20, or position 15 of the sense strand. In some embodiments, the lipophilic moiety is conjugated to position 20 or position 15 of the sense strand. In some embodiments, the lipophilic moiety is conjugated to position 16 of the antisense strand. In some embodiments, the lipophilic moiety is conjugated to position 6, counting from the 5'-end of the sense strand. In one embodiment, the saturated or unsaturated C16 hydrocarbon chain is conjugated to position 6, counting from the 5'-end of the strand

In some embodiments, the lipophilic moiety is an aliphatic, alicyclic, or polyalicyclic compound. In some embodiments, the lipophilic moiety is selected from the group consisting of lipid, cholesterol, retinoic acid, cholic acid, adamantane acetic acid, 1-pyrene butyric acid, dihydrotestosterone, 1,3-bis-O(hexadecyl)glycerol, geranyloxyhexyanol, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine. Suitable lipophilic moieties also include those containing a saturated or unsaturated C4-C30 hydrocarbon chain (e.g., C4-C30 alkyl or alkenyl), and an optional functional group selected from the group consisting of hydroxyl, amine, carboxylic acid, sulfonate, phosphate, thiol, azide, and alkyne. The functional groups are useful to attach the lipophilic moiety to the iRNA agent. In some embodiments, the lipophilic moiety contains a saturated or unsaturated C6-C18 hydrocarbon chain (e.g., a linear C6-C18 alkyl or alkenyl).

In one embodiment, the lipophilic moiety contains a saturated or unsaturated C16 hydrocarbon chain (e.g., a linear C16 alkyl or alkenyl).

In some embodiments, the lipophilic moiety is a C6-C30 acid (e.g., hexanoic acid, heptanoic acid, octanoic acid, nonanoic acid, decanoic acid, undecanoic acid, dodcanoic acid, tridecanoic acid, tetradecanoic acid, pentadecanoic acid, hexadecanoic acid, heptadecanoic acid, octadecanoic acid, oleic acid, linoleic acid, arachidonic acid, cis-4,7,10,13,16,19-docosahexanoic acid, vitamin A, vitamin E, cholesterol etc.) or a C6-C30 alcohol (e.g., hexanol, heptanol, octanol, nonanol, decanol, undecanol, dodcanol, tridecanol, tetradecanol, pentadecanol, hexadecanol, heptadecanol, octadecanol, oleyl alcohol, linoleyl alcohol, arachidonic alcohol, cis-4,7,10,13,16,19-docosahexanol, retinol, vitamin E, cholesterol etc.).

In one embodiment, the ligand is conjugated at the 2'-position of a nucleotide or modified nucleotide within the sense or antisense strand. For example, a C16 ligand may be conjugated as shown in the following structure:

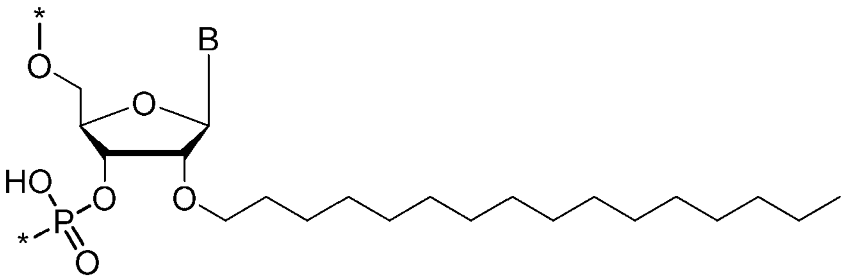

where * denotes a bond to an adjacent nucleotide, and B is a nucleobase or a nucleobase analog, optionally where B is adenine, guanine, cytosine, thymine or uracil.

In some embodiments, the lipophilic moiety is conjugated via a carrier that replaces one or more nucleotide(s) in the internal position(s) or the double stranded region. In some embodiments, the carrier is a cyclic group selected from the group consisting of pyrrolidinyl, pyrazolinyl, pyrazolidinyl, imidazolinyl, imidazolidinyl, piperidinyl, piperazinyl, [1,3] dioxolanyl, oxazolidinyl, isoxazolidinyl, morpholinyl, thiazolidinyl, isothiazolidinyl, quinoxalinyl, pyridazinonyl, tetrahydrofuranyl, and decalinyl; or is an acyclic moiety based on a serinol backbone or a diethanolamine backbone.

In some embodiments, the lipophilic moiety is conjugated to the double-stranded iRNA agent via a linker containing an ether, thioether, urea, carbonate, amine, amide, maleimide-thioether, disulfide, phosphodiester, sulfonamide linkage, a product of a click reaction, or carbamate.

In some embodiments, the lipophilic moiety is conjugated to a nucleobase, sugar moiety, or internucleosidic linkage.

In some embodiments, the lipophilic moiety or targeting ligand is conjugated via a bio-cleavable linker selected from the group consisting of DNA, RNA, disulfide, amide, functionalized monosaccharides or oligosaccharides of galactosamine, glucosamine, glucose, galactose, mannose, and combinations thereof.

In some embodiments, the 3' end of the sense strand is protected via an end cap which is a cyclic group having an amine, said cyclic group being selected from the group consisting of pyrrolidinyl, pyrazolinyl, pyrazolidinyl, imidazolinyl, imidazolidinyl, piperidinyl, piperazinyl, [1,3]dioxolanyl, oxazolidinyl, isoxazolidinyl, morpholinyl, thiazolidinyl, isothiazolidinyl, quinoxalinyl, pyridazinonyl, tetrahydrofuranyl, and decalinyl.

In some embodiments, the dsRNA agent further comprises a targeting ligand, *e.g.,* a ligand that targets an ocular tissue. In some embodiments, the ocular tissue is ciliary epithelium, an optic nerve, a trabecular meshwork, a juxtacanalicular tissue, a ganglion (e.g., including a retinal ganglion), episcleral veins or a Schlemm's canal (e.g., including an endothelial cell).

In some embodiments, the ligand is conjugated to the sense strand. In some embodiments, the ligand is conjugated to the 3' end or the 5' end of the sense strand. In some embodiments, the ligand is conjugated to the 3' end of the sense strand.

In some embodiments, the ligand comprises N-acetylgalactosamine (GalNAc). In some embodiments, the targeting ligand comprises one or more GalNAc conjugates or one or more GalNAc derivatives. In some embodiments, the ligand is one or more GalNAc conjugates or one or more GalNAc derivatives are attached through a monovalent linker, or a bivalent, trivalent, or tetravalent branched linker. In some embodiments, the ligand is

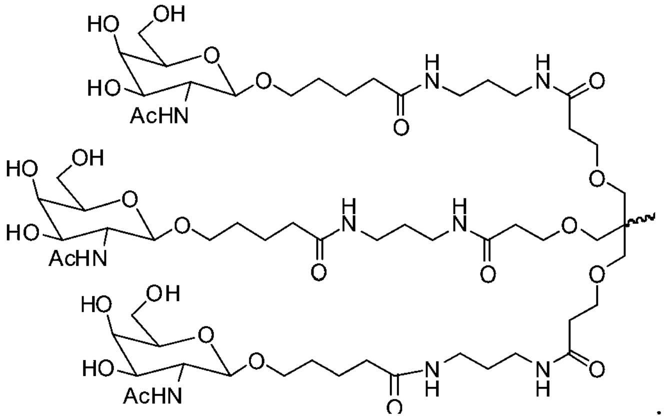

.

In some embodiments, the dsRNA agent is conjugated to the ligand as shown in the following schematic

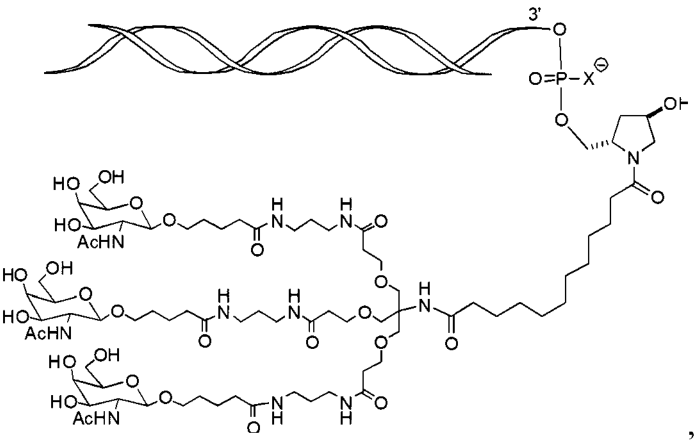

,

wherein X is O or S. In some embodiments, the X is O.

In some embodiments, the dsRNA agent further comprises a terminal, chiral modification occurring at the first internucleotide linkage at the 3' end of the antisense strand, having the linkage phosphorus atom in Sp configuration, a terminal, chiral modification occurring at the first internucleotide linkage at the 5' end of the antisense strand, having the linkage phosphorus atom in Rp configuration, and a terminal, chiral modification occurring at the first internucleotide linkage at the 5' end of the sense strand, having the linkage phosphorus atom in either Rp configuration or Sp configuration.

In some embodiments, the dsRNA agent further comprises a terminal, chiral modification occurring at the first and second internucleotide linkages at the 3' end of the antisense strand, having the linkage phosphorus atom in Sp configuration, a terminal, chiral modification occurring at the first internucleotide linkage at the 5' end of the antisense strand, having the linkage phosphorus atom in Rp configuration, and a terminal, chiral modification occurring at the first internucleotide linkage at the 5' end of the sense strand, having the linkage phosphorus atom in either Rp or Sp configuration.

In some embodiments, the dsRNA agent further comprises a terminal, chiral modification occurring at the first, second and third internucleotide linkages at the 3' end of the antisense strand, having the linkage phosphorus atom in Sp configuration, a terminal, chiral modification occurring at the first internucleotide linkage at the 5' end of the antisense strand, having the linkage phosphorus atom in Rp configuration, and a terminal, chiral modification occurring at the first internucleotide linkage at the 5' end of the sense strand, having the linkage phosphorus atom in either Rp or Sp configuration.

In some embodiments, the dsRNA agent further comprises a terminal, chiral modification occurring at the first, and second internucleotide linkages at the 3' end of the antisense strand, having the linkage phosphorus atom in Sp configuration, a terminal, chiral modification occurring at the third internucleotide linkages at the 3' end of the antisense strand, having the linkage phosphorus atom in Rp configuration, a terminal, chiral modification occurring at the first internucleotide linkage at the 5' end of the antisense strand, having the linkage phosphorus atom in Rp configuration, and a terminal, chiral modification occurring at the first internucleotide linkage at the 5' end of the sense strand, having the linkage phosphorus atom in either Rp or Sp configuration.

In some embodiments, the dsRNA agent further comprises a terminal, chiral modification occurring at the first,

and second internucleotide linkages at the 3' end of the antisense strand, having the linkage phosphorus atom in Sp configuration, a terminal, chiral modification occurring at the first, and second internucleotide linkages at the 5' end of the antisense strand, having the linkage phosphorus atom in Rp configuration, and a terminal, chiral modification occurring at the first internucleotide linkage at the 5' end of the sense strand, having the linkage phosphorus atom in either Rp or Sp configuration.

In some embodiments, the dsRNA agent further comprises a phosphate or phosphate mimic at the 5'-end of the antisense strand. In some embodiments, the phosphate mimic is a 5'-vinyl phosphonate (VP).

In various embodiments of the aforementioned dsRNA agents, the dsRNA agent targets a hotspot region of an mRNA encoding CA2.

In another aspect, the present invention provides a dsRNA agent that targets a hotspot region of a carbonic anhydrase 2 (CA2) mRNA.

In some embodiments, a cell described herein, *e.g.,* a human cell, was produced by a process comprising contacting a human cell with the dsRNA agent described herein.

In some embodiments, a pharmaceutical composition described herein comprises the dsRNA agent and a lipid formulation.

In some embodiments (e.g., embodiments of the methods described herein), the cell is within a subject. In some embodiments, the subject is a human. In some embodiments, the level of CA2 mRNA is inhibited by at least 50%. In some embodiments, the level of CA2 protein is inhibited by at least 50%. In some embodiments, the expression of CA2 is inhibited by at least 50%. In some embodiments, inhibiting expression of CA2 decreases the CA2 protein level in a biological sample (e.g., an optic nerve sample) from the subject by at least 30%, 40%, 50%, 60%, 70%, 80%, 90%, or 95%. In some embodiments, inhibiting expression of CA2 gene decreases the CA2 mRNA level in a biological sample (e.g., an optic nerve sample) from the subject by at least 30%, 40%, 50%, 60%, 70%, 80%, 90%, or 95%.

In some embodiments, the subject has been diagnosed with a CA2-associated disorder. In some embodiments, the subject meets at least one diagnostic criterion for a CA2-associated disorder. In some embodiments, the CA2 associated disorder is glaucoma or conditions associated with glaucoma.

In some embodiments, the ocular cell or tissue is a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an astrocyte, a pericyte, a Müller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel.

In some embodiments, the CA2-associated disorder is glaucoma and/or conditions associated with glaucoma.

In some embodiments, treating comprises amelioration of at least one sign or symptom of the disorder. In some embodiments, the at least one sign or symptom includes a measure of one or more of intraocular pressure, vision loss, optic nerve damage, ocular inflammation, visual acuity, or presence, level, or activity of CA2 *(e.g.,* CA2 gene, CA2 mRNA, or CA2 protein).

In some embodiments, a level of the CA2 that is higher than a reference level is indicative that the subject has glaucoma or a glaucoma associated condition. In some embodiments, treating comprises prevention of progression of the disorder. In some embodiments, the treating comprises one or more of (a) inhibiting or reducing intraocular pressure; (b) inhibiting or reducing the expression or activity of CA2; (c) decreasing the amount of aqueous humor; (d) inhibiting or reducing optic nerve damage; or (e) inhibiting or reducing retinal ganglion cell death.

In some embodiments, the treating results in at least a 30% mean reduction from baseline of CA2 mRNA in the cell or tissue. In some embodiments, the treating results in at least a 60% mean reduction from baseline of CA2 mRNA in the cell or tissue. In some embodiments, the treating results in at least a 90% mean reduction from baseline of CA2 mRNA in the cell or tissue.

In some embodiments, after treatment the subject experiences at least an 8-week duration of knockdown following a single dose of dsRNA as assessed by CA2 protein in, for example, the ciliary epithelium. In some embodiments, treating results in at least a 12-week duration of knockdown following a single dose of dsRNA as assessed by CA2 protein in, for example, the ciliary epithelium. In some embodiments, treating results in at least a 16-week duration of knockdown following a single dose of dsRNA as assessed by CA2 protein in, for example, the ciliary epithelium.

In some embodiments, the subject is human.

In some embodiments, the dsRNA agent is administered at a dose of about 0.01 mg/kg to about 50 mg/kg.

In some embodiments, the dsRNA agent is administered to the subject intraocularly. In some embodiments, the intraocular administration comprises intravitreal administration, e.g., intravitreal injection; transscleral administration, e.g., transscleral injection; subconjunctival administration, e.g., subconjunctival injection; retrobulbar administration, e.g., retrobulbar injection; intracameral administration, e.g., intracameral injection, or subretinal administration, e.g., subretinal injection.

In some embodiments, the dsRNA agent is administered to the subject intravenously. In some embodiments, the dsRNA agent is administered to the subject topically.

In some embodiments, a method described herein further comprises measuring a level of CA2 *(e.g.,* CA2 gene, CA2 mRNA, or CA2 protein) in the subject. In some embodiments, measuring the level of CA2 in the subject comprises measuring the level of CA2 protein in a biological sample from the subject *(e.g.,* a ciliary epithelium sample). In some embodiments, a method described herein further comprises performing a blood test, an imaging test, a tonometry test or a ciliary epithelium biopsy.

In some embodiments, a method described herein further comprises measuring a level of CA2 *(e.g.,* CA2 gene, CA2 mRNA, or CA2 protein) in the subject prior to treatment with the dsRNA agent or the pharmaceutical composition. In some embodiments, upon determination that a subject has a level of CA2 that is greater than a reference level, the dsRNA agent or the pharmaceutical composition is administered to the subject. In some embodiments, measuring a level of CA2 in the subject is performed after treatment with the dsRNA agent or the pharmaceutical composition.

In some embodiments, a method described herein further comprises treating the subject with a therapy suitable for treatment or prevention of a CA2-associated disorder, e.g., glaucoma, wherein the therapy comprises medication to reduce intraocular pressure, laser treatment, surgery or trabeculectomy. In some embodiments, a method described herein further comprises administering to the subject an additional agent suitable for treatment or prevention of a CA2-associated disorder. In some embodiments, the additional agent comprises a prostaglandin analog, a beta blocker, an alpha-adrenergic agonist, a carbonic anhydrase inhibitor, or an anti-CA2 agent.

In some embodiments, the anti-CA2 agent comprises an anti-CA2 antibody or antigen-binding fragment thereof (e.g., an anti-CA2 antibody molecule).

All publications, patent applications, patents, and other references mentioned herein are incorporated by reference in their entirety.

The details of various embodiments of the disclosure are set forth in the description below. Other features, objects, and advantages of the disclosure will be apparent from the description and the drawings, and from the claims.

## DETAILED DESCRIPTION

iRNA directs the sequence-specific degradation of mRNA through a process known as RNA interference (RNAi). Described herein are iRNAs and methods of using them for modulating (e.g., inhibiting) the expression of CA2. Also provided are compositions and methods for treatment of disorders related to CA2 expression, such as glaucoma or conditions associated with glaucoma.

Human CA2, also known as carbonic anhydrase 2, is a metalloenzyme encoded by the CA2 gene. CA2 catalyzes the interconversion between carbon dioxide and bicarbonate. CA2 is expressed by a variety of tissues including tissues of the eye, such as, ciliary epithelium, corneal epithelium, Müller cells, the lens, non-pigmented iris epithelium, retinal pigment epithelium, and pigmented and non-pigmented epithelium of the ciliary processes.

Without wishing to be bound by theory, CA2 may exacerbate the pathogenesis of glaucoma, e.g., by increasing intraocular pressure.CA2 appears to be the main CA form expressed in human ciliary epithelium which is responsible for producing aqueous humor. Carbonic anhydrase inhibitors have been shown to reduce aqueous humor production by up to 40% and thereby reduce intraocular pressure in the eye.

The following description discloses how to make and use compositions containing iRNAs to modulate (e.g., inhibit) the expression of CA2, as well as compositions and methods for treating disorders related to expression of CA2.

In some aspects, pharmaceutical compositions containing CA2 iRNA and a pharmaceutically acceptable carrier, methods of using the compositions to inhibit expression of CA2, and methods of using the pharmaceutical compositions to treat disorders related to expression of CA2 (e.g., glaucoma or conditions associated with glaucoma) are featured herein.

### I. Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

The term "about" when referring to a number or a numerical range means that the number or numerical range referred to is an approximation within experimental variability (or within statistical experimental error), and thus the number or numerical range may vary from, for example, between 1% and 15% of the stated number or numerical range.

The term "at least" prior to a number or series of numbers is understood to include the number adjacent to the term "at least", and all subsequent numbers or integers that could logically be included, as clear from context. For example, the number of nucleotides in a nucleic acid molecule must be an integer. For example, "at least 17 nucleotides of a 20-nucleotide nucleic acid molecule" means that 17, 18, 19, or 20 nucleotides have the indicated property. When at least is present before a series of numbers or a range, it is understood that "at least" can modify each of the numbers in the series or range.

As used herein, "no more than" or "or less" is understood as the value adjacent to the phrase and logical lower

values or integers, as logical from context, to zero. For example, a duplex with mismatches to a target site of "no more than 2 nucleotides" has a 2, 1, or 0 mismatches. When "no more than" is present before a series of numbers or a range, it is understood that "no more than" can modify each of the numbers in the series or range.

As used herein, "up to" as in "up to 10" is understood as up to and including 10, *i.e.,* 0, 1, 2, 3, 4, 5, 6, 7, 8, 9, or 10.

Ranges provided herein are understood to include all individual integer values and all subranges within the ranges.

The terms "activate," "enhance," "up-regulate the expression of," "increase the expression of," and the like, in so far as they refer to a CA2 gene, herein refer to the at least partial activation of the expression of a CA2 gene, as manifested by an increase in the amount of CA2 mRNA, which may be isolated from or detected in a first cell or group of cells in which a CA2 gene is transcribed and which has or have been treated such that the expression of a CA2 gene is increased, as compared to a second cell or group of cells substantially identical to the first cell or group of cells but which has or have not been so treated (control cells).

In some embodiments, expression of a CA2 gene is activated by at least about 10%, 15%, 20%, 25%, 30%, 35%, 40%, 45%, or 50% by administration of an iRNA as described herein. In some embodiments, a CA2 gene is activated by at least about 60%, 70%, or 80% by administration of an iRNA featured in the disclosure. In some embodiments, expression of a CA2 gene is activated by at least about 85%, 90%, or 95% or more by administration of an iRNA as described herein. In some embodiments, the CA2 gene expression is increased by at least 1-fold, at least 2-fold, at least 5-fold, at least 10-fold, at least 50-fold, at least 100-fold, at least 500-fold, at least 1000-fold or more in cells treated with an iRNA as described herein compared to the expression in an untreated cell. Activation of expression by small dsRNAs is described, for example, in Li et al., 2006 Proc. Natl. Acad. Sci. U.S.A. 103:17337-42, and in US2007/0111963 and US2005/226848, each of which is incorporated herein by reference.

The terms "silence," "inhibit expression of," "down-regulate expression of," "suppress expression of," and the like, in so far as they refer to CA2, herein refer to the at least partial suppression of the expression of CA2, as assessed, e.g., based on CA2 mRNA expression, CA2 protein expression, or another parameter functionally linked to CA2 expression. For example, inhibition of CA2 expression may be manifested by a reduction of the amount of CA2 mRNA which may be isolated from or detected in a first cell or group of cells in which CA2 is transcribed and which has or have been treated such that the expression of CA2 is inhibited, as compared to a control. The control may be a second cell or group of cells substantially identical to the first cell or group of cells, except that the second cell or group of cells have not been so treated (control cells). The degree of inhibition is usually expressed as a percentage of a control level, *e.g.,*

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to CA2 expression, e.g., the amount of protein encoded by a CA2 gene. The reduction of a parameter functionally linked to CA2 expression may similarly be expressed as a percentage of a control level. In principle, CA2 silencing may be determined in any cell expressing CA2, either constitutively or by genomic engineering, and by any appropriate assay.

For example, in certain instances, expression of CA2 is suppressed by at least about 10%, 15%, 20%, 25%, 30%, 35%, 40%, 45%, or 50% by administration of an iRNA disclosed herein. In some embodiments, CA2 is suppressed by at least about 60%, 65%, 70%, 75%, or 80% by administration of an iRNA disclosed herein. In some embodiments, CA2 is suppressed by at least about 85%, 90%, 95%, 98%, 99%, or more by administration of an iRNA as described herein.

The term "antisense strand" or "guide strand" refers to the strand of an iRNA, *e.g.,* a dsRNA, which includes a region that is substantially complementary to a target sequence.

As used herein, the term "region of complementarity" refers to the region on the antisense strand that is substantially complementary to a sequence, for example a target sequence, as defined herein. Where the region of complementarity is not fully complementary to the target sequence, the mismatches may be in the internal or terminal regions of the molecule. In some embodiments, the region of complementarity comprises 0, 1, or 2 mismatches.

The term "sense strand" or "passenger strand" as used herein, refers to the strand of an iRNA that includes a region that is substantially complementary to a region of the antisense strand as that term is defined herein.

The terms "blunt" or "blunt ended" as used herein in reference to a dsRNA mean that there are no unpaired nucleotides or nucleotide analogs at a given terminal end of a dsRNA, *i.e.,* no nucleotide overhang. One or both ends of a dsRNA can be blunt. Where both ends of a dsRNA are blunt, the dsRNA is said to be blunt ended. To be clear, a "blunt ended" dsRNA is a dsRNA that is blunt at both ends, *i.e.,* no nucleotide overhang at either end of the molecule. Most often such a molecule will be double-stranded over its entire length.

As used herein, and unless otherwise indicated, the term "complementary," when used to describe a first nucleotide sequence in relation to a second nucleotide sequence, refers to the ability of an oligonucleotide or polynucleotide comprising the first nucleotide sequence to hybridize and form a duplex structure under certain conditions with

an oligonucleotide or polynucleotide comprising the second nucleotide sequence, as will be understood by the skilled person. Such conditions can be, for example, "stringent conditions", including but not limited to, 400 mM NaCl, 40 mM PIPES pH 6.4, 1 mM EDTA, 50°C or 70°C for 12-16 hours followed by washing. As used herein, "stringent conditions" or "stringent hybridization conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated. Other conditions, such as physiologically relevant conditions as may be encountered inside an organism, can apply. The skilled person will be able to determine the set of conditions most appropriate for a test of complementarity of two sequences in accordance with the ultimate application of the hybridized nucleotides.

Complementary sequences within an iRNA, *e.g.,* within a dsRNA as described herein, include base-pairing of the oligonucleotide or polynucleotide comprising a first nucleotide sequence to an oligonucleotide or polynucleotide comprising a second nucleotide sequence over the entire length of one or both nucleotide sequences. Such sequences can be referred to as "fully complementary" with respect to each other herein. However, where a first sequence is referred to as "substantially complementary" with respect to a second sequence herein, the two sequences can be fully complementary, or they may form one or more, but generally not more than 5, 4, 3 or 2 mismatched base pairs upon hybridization for a duplex up to 30 base pairs. In some embodiments, the "substantially complementary" sequences disclosed herein comprise a contiguous nucleotide sequence which is at least about 80% complementary over its entire length to the equivalent region of the target GPR146 sequence, such as about 85%, about 90%, about 91%, about 92%, about 93%, about 94%, about 95%, about 96%, about 97%, about 98%, or about 99% complementary. However, where two oligonucleotides are designed to form, upon hybridization, one or more single stranded overhangs, such overhangs shall not be regarded as mismatches with regard to the determination of complementarity. For example, a dsRNA comprising one oligonucleotide 21 nucleotides in length and another oligonucleotide 23 nucleotides in length, wherein the longer oligonucleotide comprises a sequence of 21 nucleotides that is fully complementary to the shorter oligonucleotide, may yet be referred to as "fully complementary" for the purposes described herein.

Complementary sequences, as used herein, may also include, or be formed entirely from, non-Watson-Crick base pairs and/or base pairs formed from non-natural and modified nucleotides, in as far as the above requirements with respect to their ability to hybridize are fulfilled. Such non-Watson-Crick base pairs includes, but are not limited to, G:U Wobble or Hoogsteen base pairing.

The terms "complementary," "fully complementary" and "substantially complementary" herein may be used with respect to the base matching between two oligonucleotides or polynucleotides, such as the sense strand and the antisense strand of a dsRNA, or between the antisense strand of an iRNA agent and a target sequence, as will be understood from the context of their use.

As used herein, a polynucleotide that is "substantially complementary to at least part of" a messenger RNA (mRNA) refers to a polynucleotide that is substantially complementary to a contiguous portion of the mRNA of interest (*e.g.,* an mRNA encoding a CA2 protein). For example, a polynucleotide is complementary to at least a part of a CA2 mRNA if the sequence is substantially complementary to a non-interrupted portion of an mRNA encoding CA2. The term "complementarity" refers to the capacity for pairing between nucleobases of a first nucleic acid and a second nucleic acid.

As used herein, the term "region of complementarity" refers to the region of one nucleotide sequence agent that is substantially complementary to another sequence, *e.g.,* the region of a sense sequence and corresponding antisense sequence of a dsRNA, or the antisense strand of an iRNA and a target sequence, *e.g.,* a CA2 nucleotide sequence, as defined herein. Where the region of complementarity is not fully complementary to the target sequence, the mismatches can be in the internal or terminal regions of the antisense strand of the iRNA. Generally, the most tolerated mismatches are in the terminal regions, *e.g.,* within 5, 4, 3, or 2 nucleotides of the 5'- or 3'-terminus of the iRNA agent.

"Contacting," as used herein, includes directly contacting a cell, as well as indirectly contacting a cell. For example, a cell within a subject may be contacted when a composition comprising an iRNA is administered (*e.g.,* intraocularly, topically, or intravenously) to the subject.

"Introducing into a cell," when referring to an iRNA, means facilitating or effecting uptake or absorption into the cell. Absorption or uptake of an iRNA can occur through unaided diffusive or active cellular processes, or by auxiliary agents or devices. The meaning of this term is not limited to cells *in vitro;* an iRNA may also be "introduced into a cell," wherein the cell is part of a living organism. In such an instance, introduction into the cell will include the delivery to the organism. For example, for *in vivo* delivery, iRNA can be injected into a tissue site or administered systemically. *In vivo* delivery can also be by a β-glucan delivery system, such as those described in U.S. Patent Nos. 5,032,401 and 5,607,677, and U.S. Publication No. 2005/0281781, which are hereby incorporated by reference in their entirety. *In vitro* introduction into a cell includes methods known in the art such as electroporation and lipofection. Further approaches are described herein below or known in the art. As used herein, a "disorder related to CA2 expression," a "disease related to CA2 expression," a "pathological process related to CA2 expression," "a CA2-associated disorder," "a CA2-associated disease," or the like includes any condition, disorder, or disease in which CA2 expression is altered (*e.g.,* decreased

or increased relative to a reference level, e.g., a level characteristic of a non-diseased subject). In some embodiments, CA2 expression is decreased. In some embodiments, CA2 expression is increased. In some embodiments, the decrease or increase in CA2 expression is detectable in a tissue sample from the subject (*e.g.,* in an optic nerve sample). The decrease or increase may be assessed relative the level observed in the same individual prior to the development of the disorder or relative to other individual(s) who do not have the disorder. The decrease or increase may be limited to a particular organ, tissue, or region of the body (*e.g.,* the eye). CA2-associated disorders include, but are not limited to, glaucoma or conditions associated with glaucoma.

The term "condition(s) associated with glaucoma," as used herein, means any disease or condition that is associated with an increase in intraocular pressure. Non-limiting examples of conditions associated with glaucoma that are treatable using methods provided herein include glaucoma, open-angle glaucoma, angle-closure glaucoma, ocular inflammation, systemic inflammation, anterior uveitis, acute retinal necrosis, Sturge-Weber syndrome, Axenfeld-Rieger syndrome, Marfan syndrome, homocystinuria, Weill-Marchesani syndrome, and autoimmune diseases, such as juvenile rheumatoid arthritis and Marie-Strumpell ankylosing spondylitis.

The term "double-stranded RNA," "dsRNA," or "siRNA" as used herein, refers to an iRNA that includes an RNA molecule or complex of molecules having a hybridized duplex region that comprises two anti-parallel and substantially complementary nucleic acid strands, which will be referred to as having "sense" and "antisense" orientations with respect to a target RNA. The duplex region can be of any length that permits specific degradation of a desired target RNA, *e.g.,* through a RISC pathway, but will typically range from 9 to 36 base pairs in length, *e.g.,* 15-30 base pairs in length. Considering a duplex between 9 and 36 base pairs, the duplex can be any length in this range, for example, 9, 10, 11, 12, 13, 14, 15, 16, 17, 18, 19, 20, 21, 22, 23, 24, 25, 26, 27, 28, 29, 30, 31, 32, 33, 34, 35, or 36 and any sub-range therein between, including, but not limited to 15-30 base pairs, 15-26 base pairs, 15-23 base pairs, 15-22 base pairs, 15-21 base pairs, 15-20 base pairs, 15-19 base pairs, 15-18 base pairs, 15-17 base pairs, 18-30 base pairs, 18-26 base pairs, 18-23 base pairs, 18-22 base pairs, 18-21 base pairs, 18-20 base pairs, 19-30 base pairs, 19-26 base pairs, 19-23 base pairs, 19-22 base pairs, 19-21 base pairs, 19-20 base pairs, 20-30 base pairs, 20-26 base pairs, 20-25 base pairs, 20-24 base pairs, 20-23 base pairs, 20-22 base pairs, 20-21 base pairs, 21-30 base pairs, 21-26 base pairs, 21-25 base pairs, 21-24 base pairs, 21-23 base pairs, or 21-22 base pairs. dsRNAs generated in the cell by processing with Dicer and similar enzymes are generally in the range of 19-22 base pairs in length. One strand of the duplex region of a dsDNA comprises a sequence that is substantially complementary to a region of a target RNA. The two strands forming the duplex structure can be from a single RNA molecule having at least one self-complementary region, or can be formed from two or more separate RNA molecules. Where the duplex region is formed from two strands of a single molecule, the molecule can have a duplex region separated by a single stranded chain of nucleotides (herein referred to as a "hairpin loop") between the 3'-end of one strand and the 5'-end of the respective other strand forming the duplex structure. The hairpin loop can comprise at least one unpaired nucleotide; in some embodiments the hairpin loop can comprise at least 3, at least 4, at least 5, at least 6, at least 7, at least 8, at least 9, at least 10, at least 20, at least 23 or more unpaired nucleotides. Where the two substantially complementary strands of a dsRNA are comprised by separate RNA molecules, those molecules need not, but can be covalently connected. In some embodiments, the two strands are connected covalently by means other than a hairpin loop, and the connecting structure is a linker.

In some embodiments, the iRNA agent may be a "single-stranded siRNA" that is introduced into a cell or organism to inhibit a target mRNA. In some embodiments, single-stranded RNAi agents can bind to the RISC endonuclease Argonaute 2, which then cleaves the target mRNA. The single-stranded siRNAs are generally 15-30 nucleotides and are optionally chemically modified. The design and testing of single-stranded siRNAs are described in U.S. Patent No. 8,101,348 and in Lima et al., (2012) Cell 150: 883-894, the entire contents of each of which are hereby incorporated herein by reference. Any of the antisense nucleotide sequences described herein (*e.g.,* sequences provided in Tables 3-10) may be used as a single-stranded siRNA as described herein and optionally as chemically modified, *e.g.,* as described herein, *e.g.,* by the methods described in Lima et al., (2012) Cell 150:883-894.

In some embodiments, an RNA interference agent includes a single stranded RNA that interacts with a target RNA sequence to direct the cleavage of the target RNA. Without wishing to be bound by theory, long double stranded RNA introduced into cells is broken down into siRNA by a Type III endonuclease known as Dicer (Sharp et al., Genes Dev. 2001, 15:485). Dicer, a ribonuclease-III-like enzyme, processes the dsRNA into 19-23 base pair short interfering RNAs with characteristic two base 3' overhangs (Bernstein, et al., (2001) Nature 409:363). The siRNAs are then incorporated into an RNA-induced silencing complex (RISC) where one or more helicases unwind the siRNA duplex, enabling the complementary antisense strand to guide target recognition (Nykanen, et al., (2001) Cell 107:309). Upon binding to the appropriate target mRNA, one or more endonucleases within the RISC cleaves the target to induce silencing (Elbashir, et al., (2001) Genes Dev. 15:188). Thus, in some embodiments, the disclosure relates to a single stranded RNA that promotes the formation of a RISC complex to effect silencing of the target gene.

"G," "C," "A," "T" and "U" each generally stand for a nucleotide that contains guanine, cytosine, adenine, thymidine and uracil as a base, respectively. However, it will be understood that the terms "deoxyribonucleotide," "ribonucleotide," or "nucleotide" can also refer to a modified nucleotide, as further detailed below, or a surrogate

replacement moiety. The skilled person is well aware that guanine, cytosine, adenine, and uracil may be replaced by other moieties without substantially altering the base pairing properties of an oligonucleotide comprising a nucleotide bearing such replacement moiety. For example, without limitation, a nucleotide comprising inosine as its base may base pair with nucleotides containing adenine, cytosine, or uracil. Hence, nucleotides containing uracil, guanine, or adenine may be replaced in the nucleotide sequences of dsRNA featured in the disclosure by a nucleotide containing, for example, inosine. In another example, adenine and cytosine anywhere in the oligonucleotide can be replaced with guanine and uracil, respectively to form G-U Wobble base pairing with the target mRNA. Sequences containing such replacement moieties are suitable for the compositions and methods featured in the disclosure.

As used herein, the term "iRNA," "RNAi", "iRNA agent," or "RNAi agent" or "RNAi molecule" refers to an agent that contains RNA as that term is defined herein, and which mediates the targeted cleavage of an RNA transcript, *e.g.,* via an RNA-induced silencing complex (RISC) pathway. In some embodiments, an iRNA as described herein effects inhibition of CA2 expression, *e.g.,* in a cell or mammal. Inhibition of CA2 expression may be assessed based on a reduction in the level of CA2 mRNA or a reduction in the level of the CA2 protein.

The term "linker" or "linking group" means an organic moiety that connects two parts of a compound, *e.g.,* covalently attaches two parts of a compound.

The term "lipophile" or "lipophilic moiety" broadly refers to any compound or chemical moiety having an affinity for lipids. One way to characterize the lipophilicity of the lipophilic moiety is by the octanol-water partition coefficient, $logK_{ow}$, where $K_{ow}$ is the ratio of a chemical's concentration in the octanol-phase to its concentration in the aqueous phase of a two-phase system at equilibrium. The octanol-water partition coefficient is a laboratory-measured property of a substance. However, it may also be predicted by using coefficients attributed to the structural components of a chemical which are calculated using first-principle or empirical methods (see, for example, Tetko et al., J. Chem. Inf. Comput. Sci. 41:1407-21 (2001), which is incorporated herein by reference in its entirety). It provides a thermodynamic measure of the tendency of the substance to prefer a non-aqueous or oily milieu rather than water (i.e. its hydrophilic/lipophilic balance). In principle, a chemical substance is lipophilic in character when its $logK_{ow}$ exceeds 0. Typically, the lipophilic moiety possesses a $logK_{ow}$ exceeding 1, exceeding 1.5, exceeding 2, exceeding 3, exceeding 4, exceeding 5, or exceeding 10. For instance, the $logK_{ow}$ of 6-amino hexanol, for instance, is predicted to be approximately 0.7. Using the same method, the $logK_{ow}$ of cholesteryl N-(hexan-6-ol) carbamate is predicted to be 10.7.

The lipophilicity of a molecule can change with respect to the functional group it carries. For instance, adding a hydroxyl group or amine group to the end of a lipophilic moiety can increase or decrease the partition coefficient (*e.g.,* $logK_{ow}$) value of the lipophilic moiety.

Alternatively, the hydrophobicity of the double-stranded RNAi agent, conjugated to one or more lipophilic moieties, can be measured by its protein binding characteristics. For instance, in certain embodiments, the unbound fraction in the plasma protein binding assay of the double-stranded RNAi agent could be determined to positively correlate to the relative hydrophobicity of the double-stranded RNAi agent, which could then positively correlate to the silencing activity of the double-stranded RNAi agent.

In some embodiments, the plasma protein binding assay determined is an electrophoretic mobility shift assay (EMSA) using human serum albumin protein. An exemplary protocol of this binding assay is illustrated in detail in, *e.g.,* PCT/US2019/031170. The hydrophobicity of the double-stranded RNAi agent, measured by fraction of unbound siRNA in the binding assay, exceeds 0.15, exceeds 0.2, exceeds 0.25, exceeds 0.3, exceeds 0.35, exceeds 0.4, exceeds 0.45, or exceeds 0.5 for an enhanced *in vivo* delivery of siRNA.

Accordingly, conjugating the lipophilic moieties to the internal position(s) of the double-stranded RNAi agent provides optimal hydrophobicity for the enhanced *in vivo* delivery of siRNA.

The term "lipid nanoparticle" or "LNP" is a vesicle comprising a lipid layer encapsulating a pharmaceutically active molecule, such as a nucleic acid molecule, *e.g.,* a RNAi agent or a plasmid from which a RNAi agent is transcribed. LNPs are described in, for example, U.S. Patent Nos. 6,858,225, 6,815,432, 8,158,601, and 8,058,069, the entire contents of which are hereby incorporated herein by reference.

As used herein, the term "modulate the expression of," refers to an at least partial "inhibition" or partial "activation" of a gene (*e.g.,* CA2 gene) expression in a cell treated with an iRNA composition as described herein compared to the expression of the corresponding gene in a control cell. A control cell includes an untreated cell, or a cell treated with a non-targeting control iRNA.

The skilled artisan will recognize that the term "RNA molecule" or "ribonucleic acid molecule" encompasses not only RNA molecules as expressed or found in nature, but also analogs and derivatives of RNA comprising one or more ribonucleotide/ribonucleoside analogs or derivatives as described herein or as known in the art. Strictly speaking, a "ribonucleoside" includes a nucleoside base and a ribose sugar, and a "ribonucleotide" is a ribonucleoside with one, two or three phosphate moieties or analogs thereof (*e.g.,* phosphorothioate). However, the terms "ribonucleoside" and "ribonucleotide" can be considered to be equivalent as used herein. The RNA can be modified in the nucleobase structure, in the ribose structure, or in the ribose-phosphate backbone structure, *e.g.,* as described herein below. However, the molecules comprising ribonucleoside analogs or derivatives must retain the ability to form a duplex. As non-limiting

examples, an RNA molecule can also include at least one modified ribonucleoside including but not limited to a 2'-O-methyl modified nucleoside, a nucleoside comprising a 5' phosphorothioate group, a terminal nucleoside linked to a cholesteryl derivative or dodecanoic acid bisdecylamide group, a locked nucleoside, an abasic nucleoside, an acyclic nucleoside, a glycol nucleotide, a 2'-deoxy-2'-fluoro modified nucleoside, a 2'-amino-modified nucleoside, 2'-alkyl-modified nucleoside, morpholino nucleoside, a phosphoramidate or a non-natural base comprising nucleoside, or any combination thereof. Alternatively, or in combination, an RNA molecule can comprise at least two modified ribonucleosides, at least 3, at least 4, at least 5, at least 6, at least 7, at least 8, at least 9, at least 10, at least 15, at least 20 or more, up to the entire length of the dsRNA molecule. The modifications need not be the same for each of such a plurality of modified ribonucleosides in an RNA molecule. In some embodiments, modified RNAs contemplated for use in methods and compositions described herein are peptide nucleic acids (PNAs) that have the ability to form the required duplex structure and that permit or mediate the specific degradation of a target RNA, *e.g.,* via a RISC pathway. For clarity, it is understood that the term "iRNA" does not encompass a naturally occurring double stranded DNA molecule or a 100% deoxynucleoside-containing DNA molecule.

In some aspects, a modified ribonucleoside includes a deoxyribonucleoside. In such an instance, an iRNA agent can comprise one or more deoxynucleosides, including, for example, a deoxynucleoside overhang(s), or one or more deoxynucleosides within the double stranded portion of a dsRNA. In certain embodiments, the RNA molecule comprises a percentage of deoxyribonucleosides of at least 5, 10, 15, 20, 25, 30, 35, 40, 45, 50, 55, 60, 65, 70, 75, 80, 85, 90, 95% or higher (but not 100%) deoxyribonucleosides, *e.g.,* in one or both strands.

As used herein, the term "nucleotide overhang" refers to at least one unpaired nucleotide that protrudes from the duplex structure of an iRNA, *e.g.,* a dsRNA. For example, when a 3'-end of one strand of a dsRNA extends beyond the 5'-end of the other strand, or vice versa, there is a nucleotide overhang. A dsRNA can comprise an overhang of at least one nucleotide; alternatively, the overhang can comprise at least two nucleotides, at least three nucleotides, at least four nucleotides, or at least five nucleotides or more. A nucleotide overhang can comprise or consist of a nucleotide/nucleoside analog, including a deoxynucleotide/nucleoside. The overhang(s) may be on the sense strand, the antisense strand or any combination thereof. Furthermore, the nucleotide(s) of an overhang can be present on the 5' end, 3' end or both ends of either an antisense or sense strand of a dsRNA.

In some embodiments, the antisense strand of a dsRNA has a 1-10 nucleotide overhang at the 3' end and/or the 5' end. In some embodiments, the sense strand of a dsRNA has a 1-10 nucleotide overhang at the 3' end and/or the 5' end. In some embodiments, one or more of the nucleotides in the overhang is replaced with a nucleoside thiophosphate.

As used herein, a "pharmaceutical composition" comprises a pharmacologically effective amount of a therapeutic agent (*e.g.,* an iRNA) and a pharmaceutically acceptable carrier. As used herein, "pharmacologically effective amount," "therapeutically effective amount" or simply "effective amount" refers to that amount of an agent (*e.g.,* iRNA) effective to produce the intended pharmacological, therapeutic or preventive result. For example, in a method of treating a disorder related to CA2 expression (*e.g.,* glaucoma or conditions associated with glaucoma), an effective amount includes an amount effective to reduce one or more symptoms associated with the disorder, *e.g.,* an amount effective to (a) inhibit or reduce intraocular pressure; (b) inhibit or reduce the expression or activity of CA2; (c) decrease the amount of aqueous humor; (d) inhibit or reduce optic nerve damage; or (e) inhibit or reduce retinal ganglion cell death or an amount effective to reduce the risk of developing conditions associated with the disorder. For example, if a given clinical treatment is considered effective when there is at least a 10% reduction in a measurable parameter associated with a disease or disorder, a therapeutically effective amount of a drug for the treatment of that disease or disorder is the amount necessary to obtain at least a 10% reduction in that parameter. For example, a therapeutically effective amount of an iRNA targeting CA2 can reduce a level of CA2 mRNA or a level of CA2 protein by any measurable amount, e.g., by at least 10%, 15%, 20%, 25%, 30%, 35%, 40%, 45%, 50%, 55%, 60%, 65%, 70%, 75%, 80%, 85%, 90%, or 95%.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract. Agents included in drug formulations are described further herein below.

As used herein, the term "SNALP" refers to a stable nucleic acid-lipid particle. A SNALP represents a vesicle of lipids coating a reduced aqueous interior comprising a nucleic acid such as an iRNA or a plasmid from which an iRNA is transcribed. SNALPs are described, *e.g.,* in U.S. Patent Application Publication Nos. 2006/0240093, 2007/0135372, and in International Application No. WO 2009/082817. These applications are incorporated herein by reference in their entirety. In some embodiments, the SNALP is a SPLP. As used herein, the term "SPLP" refers to a nucleic acid-lipid particle

comprising plasmid DNA encapsulated within a lipid vesicle.

As used herein, the term "strand comprising a sequence" refers to an oligonucleotide comprising a chain of nucleotides that is described by the sequence referred to using the standard nucleotide nomenclature.

As used herein, a "subject" to be treated according to the methods described herein, includes a human or non-human animal, *e.g.,* a mammal. The mammal may be, for example, a rodent (*e.g.,* a rat or mouse) or a primate (*e.g.,* a monkey). In some embodiments, the subject is a human.

A "subject in need thereof" includes a subject having, suspected of having, or at risk of developing a disorder related to CA2 expression, e.g., overexpression (*e.g.,* glaucoma or conditions associated with glaucoma). In some embodiments, the subject has, or is suspected of having, a disorder related to CA2 expression or overexpression. In some embodiments, the subject is at risk of developing a disorder related to CA2 expression or overexpression.

As used herein, "target sequence" refers to a contiguous portion of the nucleotide sequence of an mRNA molecule formed during the transcription of a gene, *e.g.,* CA2, including mRNA that is a product of RNA processing of a primary transcription product. The target portion of the sequence will be at least long enough to serve as a substrate for iRNA-directed cleavage at or near that portion. For example, the target sequence will generally be from 9-36 nucleotides in length, *e.g.,* 15-30 nucleotides in length, including all sub-ranges therebetween. As non-limiting examples, the target sequence can be from 15-30 nucleotides, 15-26 nucleotides, 15-23 nucleotides, 15-22 nucleotides, 15-21 nucleotides, 15-20 nucleotides, 15-19 nucleotides, 15-18 nucleotides, 15-17 nucleotides, 18-30 nucleotides, 18-26 nucleotides, 18-23 nucleotides, 18-22 nucleotides, 18-21 nucleotides, 18-20 nucleotides, 19-30 nucleotides, 19-26 nucleotides, 19-23 nucleotides, 19-22 nucleotides, 19-21 nucleotides, 19-20 nucleotides, 20-30 nucleotides, 20-26 nucleotides, 20-25 nucleotides, 20-24 nucleotides, 20-23 nucleotides, 20-22 nucleotides, 20-21 nucleotides, 21-30 nucleotides, 21-26 nucleotides, 21-25 nucleotides, 21-24 nucleotides, 21-23 nucleotides, or 21-22 nucleotides.

As used herein, the phrases "therapeutically effective amount" and "prophylactically effective amount" and the like refer to an amount that provides a therapeutic benefit in the treatment, prevention, or management of any disorder or pathological process related to CA2 expression (*e.g.,* glaucoma or conditions associated with glaucoma). The specific amount that is therapeutically effective may vary depending on factors known in the art, such as, for example, the type of disorder or pathological process, the patient's history and age, the stage of the disorder or pathological process, and the administration of other therapies.

In the context of the present disclosure, the terms "treat," "treatment," and the like mean to prevent, delay, relieve or alleviate at least one symptom associated with a disorder related to CA2 expression, or to slow or reverse the progression or anticipated progression of such a disorder. For example, the methods featured herein, when employed to treat glaucoma or conditions associated with glaucoma, may serve to reduce or prevent one or more symptoms of glaucoma or conditions associated with glaucoma, as described herein, or to reduce the risk or severity of associated conditions. Thus, unless the context clearly indicates otherwise, the terms "treat," "treatment," and the like are intended to encompass prophylaxis, *e.g.,* prevention of disorders and/or symptoms of disorders related to CA2 expression. Treatment can also mean prolonging survival as compared to expected survival in the absence of treatment.

By "lower" in the context of a disease marker or symptom is meant any decrease, *e.g.,* a statistically or clinically significant decrease in such level. The decrease can be, for example, at least 10%, at least 20%, at least 30%, at least 40%, at least 40%, at least 50%, at least 60%, at least 70%, at least 80%, or at least 90%. The decrease can be down to a level accepted as within the range of normal for an individual without such disorder.

As used herein, "CA2" refers to "carbonic anhydrase 2" the corresponding mRNA ("CA2 mRNA"), or the corresponding protein ("CA2 protein"). The sequence of a human CA2 mRNA transcript can be found at SEQ ID NO: 1.

The term "substituted" refers to the replacement of one or more hydrogen radicals in a given structure with the radical of a specified substituent including, but not limited to: alkyl, alkenyl, alkynyl, aryl, heterocyclyl, halo, thiol, alkylthio, arylthio, alkylthioalkyl, arylthioalkyl, alkylsulfonyl, alkylsulfonylalkyl, arylsulfonylalkyl, alkoxy, aryloxy, aralkoxy, aminocarbonyl, alkylaminocarbonyl, arylaminocarbonyl, alkoxycarbonyl, aryloxycarbonyl, haloalkyl, amino, trifluoromethyl, cyano, nitro, alkylamino, arylamino, alkylaminoalkyl, arylaminoalkyl, aminoalkylamino, hydroxy, alkoxyalkyl, carboxyalkyl, alkoxycarbonylalkyl, aminocarbonylalkyl, acyl, aralkoxycarbonyl, carboxylic acid, sulfonic acid, sulfonyl, phosphonic acid, aryl, heteroaryl, heterocyclic, and aliphatic. It is understood that the substituent can be further substituted.

The term "alkyl" refers to saturated and unsaturated non-aromatic hydrocarbon chains that may be a straight chain or branched chain, containing the indicated number of carbon atoms (these include without limitation propyl, allyl, or propargyl), which may be optionally inserted with N, O, or S. For example, "(C1-C6) alkyl" means a radical having from 1 6 carbon atoms in a linear or branched arrangement. "(C1-C6) alkyl" includes, for example, methyl, ethyl, propyl, iso-propyl, n-butyl, tert-butyl, pentyl and hexyl. In certain embodiments, a lipophilic moiety of the instant disclosure can include a C6-C18 alkyl hydrocarbon chain.

The term "alkylene" refers to an optionally substituted saturated aliphatic branched or straight chain divalent hydrocarbon radical having the specified number of carbon atoms. For example, "(C1-C6) alkylene" means a divalent saturated aliphatic radical having from 1-6 carbon atoms in a linear arrangement, e.g., $[(CH_2)_n]$, where n is an integer from 1 to 6. "(C1-C6) alkylene" includes methylene, ethylene, propylene, butylene, pentylene and hexylene. Alternatively, "(C1-C6) alkylene" means a divalent saturated radical having from 1-6 carbon atoms in a branched arrangement, for example: $[(CH_2CH_2CH_2CH_2CH(CH_3)]$, $[(CH_2CH_2CH_2CH_2C(CH_3)_2]$, $[(CH_2C(CH_3)_2CH(CH_3))]$, and the like. The term "alkylenedioxo" refers to a divalent species of the structure -O-R-O-, in which R represents an alkylene.

The term "mercapto" refers to an -SH radical. The term "thioalkoxy" refers to an -S-alkyl radical.

The term "halo" refers to any radical of fluorine, chlorine, bromine or iodine. "Halogen" and "halo" are used interchangeably herein.

As used herein, the term "cycloalkyl" means a saturated or unsaturated nonaromatic hydrocarbon ring group having from 3 to 14 carbon atoms, unless otherwise specified. For example, "(C3-C10) cycloalkyl" means a hydrocarbon radical of a (3-10)-membered saturated aliphatic cyclic hydrocarbon ring. Examples of cycloalkyl groups include, but are not limited to, cyclopropyl, methyl-cyclopropyl, 2,2-dimethyl-cyclobutyl, 2-ethyl-cyclopentyl, cyclohexyl, etc. Cycloalkyls may include multiple spiro- or fused rings. Cycloalkyl groups are optionally mono-, di-, tri-, tetra-, or penta-substituted on any position as permitted by normal valency.

As used herein, the term "alkenyl" refers to a non-aromatic hydrocarbon radical, straight or branched, containing at least one carbon-carbon double bond, and having from 2 to 10 carbon atoms unless otherwise specified. Up to five carbon-carbon double bonds may be present in such groups. For example, "C2-C6" alkenyl is defined as an alkenyl radical having from 2 to 6 carbon atoms. Examples of alkenyl groups include, but are not limited to, ethenyl, propenyl, butenyl, and cyclohexenyl. The straight, branched, or cyclic portion of the alkenyl group may contain double bonds and is optionally mono-, di-, tri-, tetra-, or penta-substituted on any position as permitted by normal valency. The term "cycloalkenyl" means a monocyclic hydrocarbon group having the specified number of carbon atoms and at least one carbon-carbon double bond.

As used herein, the term "alkynyl" refers to a hydrocarbon radical, straight or branched, containing from 2 to 10 carbon atoms, unless otherwise specified, and containing at least one carbon-carbon triple bond. Up to 5 carbon-carbon triple bonds may be present. Thus, "C2-C6 alkynyl" means an alkynyl radical having from 2 to 6 carbon atoms. Examples of alkynyl groups include, but are not limited to, ethynyl, 2-propynyl, and 2-butynyl. The straight or branched portion of the alkynyl group may contain triple bonds as permitted by normal valency, and may be optionally mono-, di-, tri-, tetra-, or penta-substituted on any position as permitted by normal valency.

As used herein, "alkoxyl" or "alkoxy" refers to an alkyl group as defined above with the indicated number of carbon atoms attached through an oxygen bridge. For example, "(C1-C3)alkoxy" includes methoxy, ethoxy and propoxy. For example, "(C1-C6)alkoxy", is intended to include C1, C2, C3, C4, C5, and C6 alkoxy groups. For example, "(C1-C8) alkoxy", is intended to include C1, C2, C3, C4, C5, C6, C7, and C8 alkoxy groups. Examples of alkoxy include, but are not limited to, methoxy, ethoxy, n-propoxy, i-propoxy, n-butoxy, s-butoxy, t-butoxy, n-pentoxy, s-pentoxy, n-heptoxy, and n-octoxy. "Alkylthio" means an alkyl radical attached through a sulfur linking atom. The terms "alkylamino" or "aminoalkyl", means an alkyl radical attached through an NH linkage. "Dialkylamino" means two alkyl radical attached through a nitrogen linking atom. The amino groups may be unsubstituted, monosubstituted, or di-substituted. In some embodiments, the two alkyl radicals are the same (e.g., N,N-dimethylamino). In some embodiments, the two alkyl radicals are different (e.g., N-ethyl-N-methylamino).

As used herein, "aryl" or "aromatic" means any stable monocyclic or polycyclic carbon ring of up to 7 atoms in each ring, wherein at least one ring is aromatic. Examples of aryl groups include, but are not limited to, phenyl, naphthyl, anthracenyl, tetrahydronaphthyl, indanyl, and biphenyl. In cases where the aryl substituent is bicyclic and one ring is non-aromatic, it is understood that attachment is via the aromatic ring. Aryl groups are optionally mono-, di-, tri-, tetra-, or penta-substituted on any position as permitted by normal valency. The term "arylalkyl" or the term "aralkyl" refers to alkyl substituted with an aryl. The term "arylalkoxy" refers to an alkoxy substituted with aryl.

"Hetero" refers to the replacement of at least one carbon atom in a ring system with at least one heteroatom selected from N, S and O. "Hetero" also refers to the replacement of at least one carbon atom in an acyclic system. A hetero ring system or a hetero acyclic system may have, for example, 1, 2 or 3 carbon atoms replaced by a heteroatom.

As used herein, the term "heteroaryl" represents a stable monocyclic or polycyclic ring of up to 7 atoms in each ring, wherein at least one ring is aromatic and contains from 1 to 4 heteroatoms selected from the group consisting of O, N and S. Examples of heteroaryl groups include, but are not limited to, acridinyl, carbazolyl, cinnolinyl, quinoxalinyl, pyrrazolyl, indolyl, benzotriazolyl, furanyl, thienyl, benzothienyl, benzofuranyl, benzimidazolonyl, benzoxazolonyl, quinolinyl, isoquinolinyl, dihydroisoindolonyl, imidazopyridinyl, isoindolonyl, indazolyl, oxazolyl, oxadiazolyl, isoxazolyl, indolyl, pyrazinyl, pyridazinyl, pyridinyl, pyrimidinyl, pyrrolyl, tetrahydroquinoline. "Heteroaryl" is also understood to include the N-oxide derivative of any nitrogen-containing heteroaryl. In cases where the heteroaryl substituent is bicyclic and one ring is non-aromatic or contains no heteroatoms, it is understood that attachment is via the aromatic ring or via the heteroatom containing ring. Heteroaryl groups are optionally mono-, di-, tri-, tetra-, or penta-substituted on any position as permitted by normal valency.

As used herein, the term "heterocycle," "heterocyclic," or "heterocyclyl" means a 3- to 14-membered aromatic or nonaromatic heterocycle containing from 1 to 4 heteroatoms selected from the group consisting of O, N and S, including polycyclic groups. As used herein, the term "heterocyclic" is also considered to be synonymous with the terms "heterocycle" and "heterocyclyl" and is understood as also having the same definitions set forth herein. "Heterocyclyl" includes the above mentioned heteroaryls, as well as dihydro and tetrahydro analogs thereof. Examples of heterocyclyl groups include, but are not limited to, azetidinyl, benzoimidazolyl, benzofuranyl, benzofurazanyl, benzopyrazolyl, benzotriazolyl, benzothiophenyl, benzoxazolyl, carbazolyl, carbolinyl, cinnolinyl, furanyl, imidazolyl, indolinyl, indolyl, indolazinyl, indazolyl, isobenzofuranyl, isoindolyl, isoquinolyl, isothiazolyl, isoxazolyl, naphthpyridinyl, oxadiazolyl, oxooxazolidinyl, oxazolyl, oxazoline, oxopiperazinyl, oxopyrrolidinyl, oxomorpholinyl, isoxazoline, oxetanyl, pyranyl, pyrazinyl, pyrazolyl, pyridazinyl, pyridopyridinyl, pyridazinyl, pyridyl, pyridinonyl, pyrimidyl, pyrimidinonyl, pyrrolyl, quinazolinyl, quinolyl, quinoxalinyl, tetrahydropyranyl, tetrahydrofuranyl, tetrahydrothiopyranyl, tetrahydroisoquinolinyl, tetrazolyl, tetrazolopyridyl, thiadiazolyl, thiazolyl, thienyl, triazolyl, 1,4-dioxanyl, hexahydroazepinyl, piperazinyl, piperidinyl, pyridin-2-onyl, pyrrolidinyl, morpholinyl, thiomorpholinyl, dihydrobenzoimidazolyl, dihydrobenzofuranyl, dihydrobenzothiophenyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dioxidothiomorpholinyl, methylenedioxybenzoyl, tetrahydrofuranyl, and tetrahydrothienyl, and N-oxides thereof. Attachment of a heterocyclyl substituent can occur via a carbon atom or via a heteroatom. Heterocyclyl groups are optionally mono-, di-, tri-, tetra-, or penta-substituted on any position as permitted by normal valency.

"Heterocycloalkyl" refers to a cycloalkyl residue in which one to four of the carbons is replaced by a heteroatom such as oxygen, nitrogen or sulfur. Examples of heterocycles whose radicals are heterocyclyl groups include tetrahydropyran, morpholine, pyrrolidine, piperidine, thiazolidine, oxazole, oxazoline, isoxazole, dioxane, tetrahydrofuran and the like.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2, 3, or 4 atoms of each ring may be substituted by a substituent. Examples of heteroaryl groups include pyridyl, furyl or furanyl, imidazolyl, benzimidazolyl, pyrimidinyl, thiophenyl or thienyl, quinolinyl, indolyl, thiazolyl, and the like. The term "heteroarylalkyl" or the term "heteroaralkyl" refers to an alkyl substituted with a heteroaryl. The term "heteroarylalkoxy" refers to an alkoxy substituted with heteroaryl.

The term "cycloalkyl" as employed herein includes saturated and partially unsaturated cyclic hydrocarbon groups having 3 to 12 carbons, for example, 3 to 8 carbons, and, for example, 3 to 6 carbons, wherein the cycloalkyl group additionally may be optionally substituted. Cycloalkyl groups include, without limitation, cyclopropyl, cyclobutyl, cyclopentyl, cyclopentenyl, cyclohexyl, cyclohexenyl, cycloheptyl, and cyclooctyl.

The term "acyl" refers to an alkylcarbonyl, cycloalkylcarbonyl, arylcarbonyl, heterocyclylcarbonyl, or heteroarylcarbonyl substituent, any of which may be further substituted by substituents.

As used herein, "keto" refers to any alkyl, alkenyl, alkynyl, cycloalkyl, cycloalkenyl, heterocyclyl, heteroaryl, or aryl group as defined herein attached through a carbonyl bridge.

Examples of keto groups include, but are not limited to, alkanoyl (e.g., acetyl, propionyl, butanoyl, pentanoyl, hexanoyl), alkenoyl (e.g., acryloyl) alkynoyl (e.g., ethynoyl, propynoyl, butynoyl, pentynoyl, hexynoyl), aryloyl (e.g., benzoyl), heteroaryloyl (e.g., pyrroloyl, imidazoloyl, quinolinoyl, pyridinoyl).

As used herein, "alkoxycarbonyl" refers to any alkoxy group as defined above attached through a carbonyl bridge (i.e., -C(O)O-alkyl). Examples of alkoxycarbonyl groups include, but are not limited to, methoxycarbonyl, ethoxycarbonyl, iso-propoxycarbonyl, n-propoxycarbonyl, t-butoxycarbonyl, benzyloxycarbonyl or n-pentoxycarbonyl.

As used herein, "aryloxycarbonyl" refers to any aryl group as defined herein attached through an oxycarbonyl bridge (i.e., -C(O)O-aryl). Examples of aryloxycarbonyl groups include, but are not limited to, phenoxycarbonyl and naphthyloxycarbonyl.

As used herein, "heteroaryloxycarbonyl" refers to any heteroaryl group as defined herein attached through an oxycarbonyl bridge (i.e., -C(O)O-heteroaryl). Examples of heteroaryloxycarbonyl groups include, but are not limited to, 2-pyridyloxycarbonyl, 2-oxazolyloxycarbonyl, 4-thiazolyloxycarbonyl, or pyrimidinyloxycarbonyl.

The term "oxo" refers to an oxygen atom, which forms a carbonyl when attached to carbon, an N-oxide when attached to nitrogen, and a sulfoxide or sulfone when attached to sulfur.

The person of ordinary skill in the art would readily understand and appreciate that the compounds and compositions disclosed herein may have certain atoms (e.g., N, O, or S atoms) in a protonated or deprotonated state, depending upon the environment in which the compound or composition is placed. Accordingly, as used herein, the structures disclosed herein envisage that certain functional groups, such as, for example, OH, SH, or NH, may be protonated or deprotonated. The disclosure herein is intended to cover the disclosed compounds and compositions regardless of their state of protonation based on the pH of the environment, as would be readily understood by the person of ordinary skill in the art.

## II. iRNA Agents

Described herein are iRNA agents that modulate (*e.g.*, inhibit) the expression of CA2.

In some embodiments, the iRNA agent activates the expression of CA2 in a cell or mammal.

In some embodiments, the iRNA agent includes double-stranded ribonucleic acid (dsRNA) molecules for inhibiting the expression of CA2 in a cell or in a subject (*e.g.,* in a mammal, *e.g.,* in a human), where the dsRNA includes an antisense strand having a region of complementarity which is complementary to at least a part of an mRNA formed in the expression of CA2, and where the region of complementarity is 30 nucleotides or less in length, generally 19-24 nucleotides in length, and where the dsRNA, upon contact with a cell expressing CA2, inhibits the expression of CA2, *e.g.,* by at least 10%, 20%, 30%, 40%, or 50% as compared to a similar cell not contacted with the RNAi agent or an RNAi agent not complimentary to the CA2 gene.

The modulation (*e.g.,* inhibition) of expression of CA2 can be assayed by, for example, a PCR or branched DNA (bDNA)-based method, or by a protein-based method, such as by Western blot. Expression of CA2 in cell culture, such as in COS cells, ARPE-19 cells, hTERT RPE-1 cells, RPE-J cells, HeLa cells, primary hepatocytes, HepG2 cells, primary cultured cells or in a biological sample from a subject can be assayed by measuring CA2 mRNA levels, such as by bDNA or TaqMan assay, or by measuring protein levels, such as by immunofluorescence analysis, using, for example, Western Blotting or flow cytometric techniques.

A dsRNA typically includes two RNA strands that are sufficiently complementary to hybridize to form a duplex structure under conditions in which the dsRNA will be used. One strand of a dsRNA (the antisense strand) typically includes a region of complementarity that is substantially complementary, or fully complementary, to a target sequence, derived from the sequence of an mRNA formed during the expression of CA2. The other strand (the sense strand) typically includes a region that is complementary to the antisense strand, such that the two strands hybridize and form a duplex structure when combined under suitable conditions. Generally, the duplex structure is between 15 and 30 inclusive, more generally between 18 and 25 inclusive, yet more generally between 19 and 24 inclusive, and most generally between 19 and 21 base pairs in length, inclusive. Similarly, the region of complementarity to the target sequence is between 15 and 30 inclusive, more generally between 18 and 25 inclusive, yet more generally between 19 and 24 inclusive, and most generally between 19 and 21 nucleotides in length, inclusive.

In some embodiments, the dsRNA is between 15 and 20 nucleotides in length, inclusive, and in other embodiments, the dsRNA is between 25 and 30 nucleotides in length, inclusive. As the ordinarily skilled person will recognize, the targeted region of an RNA targeted for cleavage will most often be part of a larger RNA molecule, often an mRNA molecule. Where relevant, a "part" of an mRNA target is a contiguous sequence of an mRNA target of sufficient length to be a substrate for RNAi-directed cleavage (*i.e.,* cleavage through a RISC pathway). dsRNAs having duplexes as short as 9 base pairs can, under some circumstances, mediate RNAi-directed RNA cleavage. Most often a target will be at least 15 nucleotides in length, *e.g.,* 15-30 nucleotides in length.

One of skill in the art will also recognize that the duplex region is a primary functional portion of a dsRNA, *e.g.,* a duplex region of 9 to 36, *e.g.,* 15-30 base pairs. Thus, in some embodiments, to the extent that it becomes processed to a functional duplex of *e.g.,* 15-30 base pairs that targets a desired RNA for cleavage, an RNA molecule or complex of RNA molecules having a duplex region greater than 30 base pairs is a dsRNA. Thus, an ordinarily skilled artisan will recognize that in some embodiments, then, an miRNA is a dsRNA. In some embodiments, a dsRNA is not a naturally occurring miRNA. In some embodiments, an iRNA agent useful to target CA2 expression is not generated in the target cell by cleavage of a larger dsRNA.

A dsRNA as described herein may further include one or more single-stranded nucleotide overhangs. The dsRNA can be synthesized by standard methods known in the art as further discussed below, *e.g.,* by use of an automated DNA synthesizer, such as are commercially available from, for example, Biosearch, Applied Biosystems, Inc.

In some embodiments, CA2 is a human CA2.

In specific embodiments, the dsRNA comprises a sense strand that comprises or consists of a sense sequence selected from the sense sequences provided in Tables 3-10 and an antisense strand that comprises or consists of an antisense sequence selected from the antisense sequences provided in Tables 3-10.

In some aspects, a dsRNA will include at least sense and antisense nucleotide sequences, whereby the sense strand is selected from the sequences provided in Tables 3-10 and the corresponding antisense strand is selected from the sequences provided in Tables 3-10.

In these aspects, one of the two sequences is complementary to the other of the two sequences, with one of the sequences being substantially complementary to a sequence of an mRNA generated by the expression of CA2. As such, a dsRNA will include two oligonucleotides, where one oligonucleotide is described as the sense strand, and the second oligonucleotide is described as the corresponding antisense strand. As described elsewhere herein and as known in the art, the complementary sequences of a dsRNA can also be contained as self-complementary regions of a single nucleic acid molecule, as opposed to being on separate oligonucleotides.

The skilled person is well aware that dsRNAs having a duplex structure of between 20 and 23, but specifically 21,

base pairs have been hailed as particularly effective in inducing RNA interference (Elbashir et al., EMBO 2001, 20:6877-6888). However, others have found that shorter or longer RNA duplex structures can be effective as well.

In the embodiments described above, by virtue of the nature of the oligonucleotide sequences provided in Tables 3-10, dsRNAs described herein can include at least one strand of a length of minimally 19 nucleotides. It can be reasonably expected that shorter duplexes having one of the sequences of Tables 3-10 minus only a few nucleotides on one or both ends will be similarly effective as compared to the dsRNAs described above.

In some embodiments, the dsRNA has a partial sequence of at least 15, 16, 17, 18, 19, 20, or more contiguous nucleotides from one of the sequences of Tables 3-10.

In some embodiments, the dsRNA has an antisense sequence that comprises at least 15, 16, 17, 18, or 19 contiguous nucleotides of an antisense sequence provided in Tables 3-10 and a sense sequence that comprises at least 15, 16, 17, 18, or 19 contiguous nucleotides of a corresponding sense sequence provided in Tables 3-10.

In some embodiments, the dsRNA comprises an antisense sequence that comprises at least 15, 16, 17, 18, 19, 20, 21, 22, or 23 contiguous nucleotides of an antisense sequence provided in Tables 3-10 and a sense sequence that comprises at least 15, 16, 17, 18, 19, 20, or 21 contiguous nucleotides of a corresponding sense sequence provided in Tables 3-10.

In some such embodiments, the dsRNA, although it comprises only a portion of the sequences provided in Tables 3-10 is equally effective in inhibiting a level of CA2 expression as is a dsRNA that comprises the full-length sequences provided in Tables 3-10. In some embodiments, the dsRNA differs in its inhibition of a level of expression of CA2 by not more than 5, 10, 15, 20, 25, 30, 35, 40, 45, or 50 % inhibition compared with a dsRNA comprising the full sequence disclosed herein.

The iRNAs of Tables 3-10 were designed based on human CA2 sequence. Without wishing to be bound by theory, CA2 sequence is conserved sufficiently between species such that certain iRNAs designed based on a human sequence have activity against CA2 from primates, such as cynomolgus monkey, and other species, including, for example, mouse, rat, and rabbit.

Consequently, in some embodiments, an iRNA of Tables 3-10 decreases CA2 protein or CA2 mRNA levels in a cell. In some embodiments, the cell is a rodent cell (e.g., a rat cell), or a primate cell (e.g., a cynomolgus monkey cell or a human cell). In some embodiments, CA2 protein or CA2 mRNA levels are reduced by at least 30%, 40%, 50%, 60%, 70%, 80%, 90%, or 95%. In some embodiments, the iRNA of Tables 3-10 that inhibits CA2 in a human cell has less than 5, 4, 3, 2, or 1 mismatches to the corresponding portion of human CA2. In some embodiments, the iRNA of Tables 3-10 that inhibits CA2 in a human cell has no mismatches to the corresponding portion of human CA2.

iRNAs designed based on rodent sequences can have utility, e.g., for inhibiting CA2 in human cells, e.g., for therapeutic purposes, or for inhibiting CA2 in rodent cells, e.g., for research characterizing CA2 in a rodent model.

In some embodiments, an iRNA described herein comprises an antisense strand comprising at least 15 contiguous nucleotides, with 0, 1, 2, or 3 mismatches, of a portion of nucleotide sequence of SEQ ID NO: 2. In some embodiments, an iRNA described herein comprises a sense strand comprising at least 15 contiguous nucleotides, with 0, or 1, 2, or 3 mismatches, of the corresponding portion of the nucleotide sequence of SEQ ID NO: 1. A human CA2 mRNA may have the sequence of SEQ ID NO: 1 provided herein.

In some embodiments, an iRNA described herein includes at least 15 contiguous nucleotides from one of the sequences provided in Tables 3-10, and may optionally be coupled to additional nucleotide sequences taken from the region contiguous to the selected sequence in CA2.

While a target sequence is generally 15-30 nucleotides in length, there is wide variation in the suitability of particular sequences in this range for directing cleavage of any given target RNA. Various software packages and the guidelines set out herein provide guidance for the identification of optimal target sequences for any given gene target, but an empirical approach can also be taken in which a "window" or "mask" of a given size (as a non-limiting example, 21 nucleotides) is literally or figuratively (including, *e.g.,* in silico) placed on the target RNA sequence to identify sequences in the size range that may serve as target sequences. By moving the sequence "window" progressively one nucleotide upstream or downstream of an initial target sequence location, the next potential target sequence can be identified, until the complete set of possible sequences is identified for any given target size selected. This process, coupled with systematic synthesis and testing of the identified sequences (using assays described herein or known in the art) to identify those sequences that perform optimally can identify those RNA sequences that, when targeted with an iRNA agent, mediate the best inhibition of target gene expression. Thus, it is contemplated that further optimization of inhibition efficiency can be achieved by progressively "walking the window" one nucleotide upstream or downstream of the given sequences to identify sequences with equal or better inhibition characteristics.

Further, it is contemplated that for any sequence identified, *e.g.,* in Tables 3-10, further optimization can be achieved by systematically either adding or removing nucleotides to generate longer or shorter sequences and testing those and sequences generated by walking a window of the longer or shorter size up or down the target RNA from that point. Again, coupling this approach to generating new candidate targets with testing for effectiveness of iRNAs based on those target sequences in an inhibition assay as known in the art or as described herein can lead to further improvements in

the efficiency of inhibition. Further still, such optimized sequences can be adjusted by, *e.g.,* the introduction of modified nucleotides as described herein or as known in the art, addition or changes in overhang, or other modifications as known in the art and/or discussed herein to further optimize the molecule (*e.g.,* increasing serum stability or circulating half-life, increasing thermal stability, enhancing transmembrane delivery, targeting to a particular location or cell type, increasing interaction with silencing pathway enzymes, increasing release from endosomes, *etc*.) as an expression inhibitor.

In some embodiments, the disclosure provides an iRNA of any of Tables 3-10 that is unmodified or unconjugated. In some embodiments, an RNAi agent of the disclosure has a nucleotide sequence as provided in any of Tables 3-10, but lacks one or more ligand or moiety shown in the tables. A ligand or moiety (*e.g.,* a lipophilic ligand or moiety) can be included in any of the positions provided in the instant application.

An iRNA as described herein can contain one or more mismatches to the target sequence. In some embodiments, an iRNA as described herein contains no more than 3 mismatches. In some embodiments, when the antisense strand of the iRNA contains mismatches to a target sequence, the area of mismatch is not located in the center of the region of complementarity. In some embodiments, when the antisense strand of the iRNA contains mismatches to the target sequence, the mismatch is restricted to be within the last 5 nucleotides from either the 5' or 3' end of the region of complementarity. For example, for a 23 nucleotide iRNA agent RNA strand which is complementary to a region of CA2, the RNA strand generally does not contain any mismatch within the central 13 nucleotides. The methods described herein, or methods known in the art can be used to determine whether an iRNA containing a mismatch to a target sequence is effective in inhibiting the expression of CA2. For example, Jackson et al. (Nat. Biotechnol. 2003;21: 635-637) described an expression profile study where the expression of a small set of genes with sequence identity to the MAPK14 siRNA only at 12-18 nt of the sense strand, was down-regulated with similar kinetics to MAPK14. Similarly, Lin et al., (Nucleic Acids Res. 2005; 33(14): 4527-4535) using qPCR and reporter assays, showed that a 7 nt complementation between a siRNA and a target is sufficient to cause mRNA degradation of the target. Consideration of the efficacy of iRNAs with mismatches in inhibiting expression of CA2 is important, especially if the particular region of complementarity in a CA2 gene is known to have polymorphic sequence variation within the population.

An RNA target may have regions, or spans of the target RNA's nucleotide sequence, which are relatively more susceptible or amenable than other regions of the RNA target to mediating cleavage of the RNA target via RNA interference induced by the binding of an RNAi agent to that region. The increased susceptibility to RNA interference within such "hotspot regions" (or simply "hotspots") means that iRNA agents targeting the region will likely have higher efficacy in inducing iRNA interference than iRNA agents which target other regions of the target RNA. For example, without being bound by theory, the accessibility of a target region of a target RNA may influence the efficacy of iRNA agents which target that region, with some hotspot regions having increased accessibility. Secondary structures, for instance, that form in the RNA target (e.g., within or proximate to hotspot regions) may affect the ability of the iRNA agent to bind the target region and induce RNA interference.

According to certain aspects of the invention, an iRNA agent may be designed to target a hotspot region of any of the target RNAs described herein, including any identified portions of a target RNA (e.g., a particular exon). As used herein, a hotspot region may refer to an approximately 19-200, 19-150, 19-100, 19-75, 19-50, 21-200, 21-150, 21-100, 21-75, 21-50, 50-200, 50-150, 50-100, 50-75, 75-200, 75-150, 75-100, 100-200, or 100-150 nucleotide region of a target RNA sequence for which targeting using RNAi agents provides an observably higher probability of efficacious silencing relative to targeting other regions of the same target RNA. According to certain aspects of the invention, a hotspot region may comprise a limited region of the target RNA, and in some cases, a substantially limited region of the target, including for example, less than half of the length of the target RNA, such as about 5%, 10%, 15%, 20%, 25%, or 30% of the lenth of the target RNA. Conversely, the other regions against which a hotspot is compared may cumulatively comprise at least a majority of the length of the target RNA. For example, the other regions may cumulatively comprise at least about 60%, or at least about 70%, or at least about 80%, or at least about 90%, or at least about 95% of the length of the target RNA.

Compared regions of the target RNA may be empirically evaluated for identification of hotspots using efficacy data obtained from in vitro or in vivo screening assays. For example, RNAi agents targeting various regions that span a target RNA may be compared for frequency of efficacious iRNA agents (e.g., the amount by which target gene expression is inhibited, such as measured by mRNA expression or protein expression) that bind each region. In general, a hotspot can be recognized by observing clustering of multiple efficacious RNAi agents that bind to a limited region of the RNA target. A hotspot may be sufficiently characterized as such by observing efficacy of iRNA agents which cumulatively span at least about 60% of the target region identified as a hotspot, such as about 70%, about 80%, about 90%, or about 95% or more of the length of the region, including both ends of the region (i.e. at least about 60%, 70%, 80%, 90%, or 95% or more of the nucleotides within the region, including the nucleotides at each end of the region, were targeted by an iRNA agent). According to some aspects of the invention, an iRNA agent which demonstrates at least about 50%, 55%, 60%, 65%, 70%, 75%, 80%, 85%, 90%, or 95% inhibition over the region (e.g., no more than about 50%, 45%, 40%, 35%, 30%, 25%, 20%, 15%, 10%, or 5% mRNA remaining) may be identified as efficacious.

Amenibility to targeting of RNA regions may also be assessed using quantitative comparison of inhibition measurements across different regions of a defined size (e.g, 25, 30, 40, 50, 60, 70, 80, 90, or 100, 110, 120, 130, 140, 150,

160, 170, 180, 190, or 200 nts). For example, an average level of inhibition may be determined for each region and the averages of each region may be compared. The average level of inhibition within a hotspot region may be substantially higher than the average of averages for all evaluated regions. According to some aspects, the average level of inhibition in a hotspot region may be at least about 10%, 20%, 30%, 40%, or 50% higher than the average of averages. According to some aspects, the average level of inhibition in a hotspot region may be at least about 1.0, 1.1, 1.2, 1.3, 1.4, 1.5 1.6, 1.7, 1.8. 1.9, or 2.0 standard deviations above the average of averages. The average level of inhibition may be higher by a statistically significant (e.g., $p < 0.05$) amount. According to some aspects, each inhibition measurement within a hotspot region may be above a threshold amount (e.g., at or below a threshold amount of mRNA remaining). According to some aspects, each inhibition measurement within the region may be substantially higher than an average of all inhibition measurements across all the measured regions. For example, each inhibition measurement in a hotspot region may be at least about 10%, 20%, 30%, 40%, or 50% higher than the average of all inhibition measurements. According to some aspects, each inhibition measurement may be at least about 1.0, 1.1, 1.2, 1.3, 1.4, 1.5 1.6, 1.7, 1.8. 1.9, or 2.0 standard deviations above the average of all inhibition measurements. Each inhibition measurement may be higher by a statistically significant (e.g., $p < 0.05$) amount than the average of all inhibition measurements. A standard for evaluating a hotspot may comprise various combinations of the above standards where compatible (e.g., an average level of inhibition of at least about a first amount and having no inhibition measurements below a threshold level of a second amount, lesser than the first amount).

It is therefore expressly contemplated that any iRNA agent, including the specific exemplary iRNA agents described herein, which targets a hotspot region of a target RNA, may be preferably selected for inducing RNA interference of the target mRNA as targeting such a hotspot region is likely to exhibit a robust inhibitory response relative to targeting a region which is not a hotspot region. RNAi agents targeting target sequences that substantially overlap (e.g., by at least about 70%, 75%, 80%, 85%, 90%, 95% of the target sequence length) or, preferably, that reside fully within the hotspot region may be considered to target the hotspot region. Hotspot regions of the RNA target(s) of the instant invention may include any region for which the data disclosed herein demonstrates higher frequency of targeting by efficacious RNAi agents, including by any of the standards described elsewhere herein, whether or not the range(s) of such hotspot region(s) are explicitly specified.

In various embodiments, a dsRNA agent of the present invention targets a hotspot region of an mRNA encoding CA2.

In some embodiments, at least one end of a dsRNA has a single-stranded nucleotide overhang of 1 to 4, generally 1 or 2 nucleotides. In some embodiments, dsRNAs having at least one nucleotide overhang have superior inhibitory properties relative to their blunt-ended counterparts. In some embodiments, the RNA of an iRNA (*e.g.,* a dsRNA) is chemically modified to enhance stability or other beneficial characteristics. The nucleic acids featured in the disclosure may be synthesized and/or modified by methods well established in the art, such as those described in "Current protocols in nucleic acid chemistry," Beaucage, S.L. et al. (Edrs.), John Wiley & Sons, Inc., New York, NY, USA, which is hereby incorporated herein by reference. Modifications include, for example, (a) end modifications, *e.g.,* 5' end modifications (phosphorylation, conjugation, inverted linkages, *etc.*) 3' end modifications (conjugation, DNA nucleotides, inverted linkages, *etc.*), (b) base modifications, *e.g.,* replacement with stabilizing bases, destabilizing bases, or bases that base pair with an expanded repertoire of partners, removal of bases (abasic nucleotides), or conjugated bases, (c) sugar modifications (*e.g.*, at the 2' position or 4' position, or having an acyclic sugar) or replacement of the sugar, as well as (d) backbone modifications, including modification or replacement of the phosphodiester linkages. Specific examples of RNA compounds useful in this disclosure include, but are not limited to, RNAs containing modified backbones or no natural internucleoside linkages. RNAs having modified backbones include, among others, those that do not have a phosphorus atom in the backbone. For the purposes of this specification, and as sometimes referenced in the art, modified RNAs that do not have a phosphorus atom in their internucleoside backbone can also be considered to be oligonucleosides. In particular embodiments, the modified RNA will have a phosphorus atom in its internucleoside backbone.

Modified RNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5' linked analogs of these, and those) having inverted polarity wherein the adjacent pairs of nucleoside units are linked 3'-5' to 5'-3' or 2'-5' to 5'-2'. Various salts, mixed salts and free acid forms are also included.

Representative U.S. patents that teach the preparation of the above phosphorus-containing linkages include, but are not limited to, U.S. Pat. Nos. 3,687,808; 4,469,863; 4,476,301; 5,023,243; 5,177,195; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,316; 5,550,111; 5,563,253; 5,571,799; 5,587,361; 5,625,050; 6,028,188; 6,124,445; 6,160,109; 6,169,170; 6,172,209; 6, 239,265; 6,277,603; 6,326,199; 6,346,614; 6,444,423; 6,531,590; 6,534,639; 6,608,035; 6,683,167; 6,858,715; 6,867,294; 6,878,805; 7,015,315; 7,041,816; 7,273,933; 7,321,029; and US Pat

RE39464, each of which is herein incorporated by reference.

Modified RNA backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Representative U.S. patents that teach the preparation of the above oligonucleosides include, but are not limited to, U.S. Pat. Nos. 5,034,506; 5,166,315; 5,185,444; 5,214,134; 5,216,141; 5,235,033; 5,64,562; 5,264,564; 5,405,938; 5,434,257; 5,466,677; 5,470,967; 5,489,677; 5,541,307; 5,561,225; 5,596,086; 5,602,240; 5,608,046; 5,610,289; 5,618,704; 5,623,070; 5,663,312; 5,633,360; 5,677,437; and, 5,677,439, each of which is herein incorporated by reference.

In other RNA mimetics suitable or contemplated for use in iRNAs, both the sugar and the internucleoside linkage, *i.e.,* the backbone, of the nucleotide units are replaced with alternate groups. The nucleobase units are maintained for hybridization with an appropriate nucleic acid target compound. One such oligomeric compound, an RNA mimetic that has been shown to have excellent hybridization properties, is referred to as a peptide nucleic acid (PNA). In PNA compounds, the sugar backbone of an RNA is replaced with an amide containing backbone, in particular an aminoethylglycine backbone. The nucleobases are retained and are bound directly or indirectly to aza nitrogen atoms of the amide portion of the backbone. Representative U.S. patents that teach the preparation of PNA compounds include, but are not limited to, U.S. Pat. Nos. 5,539,082; 5,714,331; and 5,719,262, each of which is herein incorporated by reference. Further teaching of PNA compounds can be found, for example, in Nielsen et al., Science, 1991, 254, 1497-1500.

Some embodiments featured in the disclosure include RNAs with phosphorothioate backbones and oligonucleosides with heteroatom backbones, and in particular $--CH_2--NH--CH_2-$, $--CH_2--N(CH_3)--O--CH_2--$[known as a methylene (methylimino) or MMI backbone], $--CH_2--O-N(CH_3)--CH_2--$, $--CH_2--N(CH_3)--N(CH_3)--CH_2--$ and $--N(CH_3)--CH_2--CH_2--$ of the above-referenced U.S. Pat. No. 5,489,677, and the amide backbones of the above-referenced U.S. Pat. No. 5,602,240. In some embodiments, the RNAs featured herein have morpholino backbone structures of the above-referenced U.S. Pat. No. 5,034,506. The native phosphodiester backbone can be represented as $-O-P(O)(OH)-OCH_2-$.

Modified RNAs may also contain one or more substituted sugar moieties. The iRNAs, e.g., dsRNAs, featured herein can include one of the following at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Exemplary suitable modifications include $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. In other embodiments, dsRNAs include one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an iRNA, or a group for improving the pharmacodynamic properties of an iRNA, and other substituents having similar properties. In some embodiments, the modification includes a 2'-methoxyethoxy (2'-O--$CH_2CH_2OCH_3$, also known as 2'-O-(2-methoxyethyl) or 2'-MOE) (Martin et al., Helv. Chim. Acta, 1995, 78:486-504) *i.e.,* an alkoxy-alkoxy group. Another exemplary modification is 2'-dimethylaminooxyethoxy, *i.e.,* a $O(CH_2)_2ON(CH_3)_2$ group, also known as 2'-DMAOE, and 2'-dimethylaminoethoxyethoxy (also known in the art as 2'-O-dimethylaminoethoxyethyl or 2'-DMAEOE), *i.e.*, 2'-O--$CH_2$--O--$CH_2$-$N(CH_3)_2$.

In other embodiments, an iRNA agent comprises one or more (*e.g.,* about 1, 2, 3, 4, 5, 6, 7, 8, 9, 10, or more) acyclic nucleotides (or nucleosides). In certain embodiments, the sense strand or the antisense strand, or both sense strand and antisense strand, include less than five acyclic nucleotides per strand (*e.g.,* four, three, two or one acyclic nucleotides per strand). The one or more acyclic nucleotides can be found, for example, in the double-stranded region, of the sense or antisense strand, or both strands; at the 5'-end, the 3'-end, both of the 5' and 3'-ends of the sense or antisense strand, or both strands, of the iRNA agent. In some embodiments, one or more acyclic nucleotides are present at positions 1 to 8 of the sense or antisense strand, or both. In some embodiments, one or more acyclic nucleotides are found in the antisense strand at positions 4 to 10 (*e.g.,* positions 6-8) from the 5'-end of the antisense strand. In some embodiments, the one or more acyclic nucleotides are found at one or both 3'-terminal overhangs of the iRNA agent.

The term "acyclic nucleotide" or "acyclic nucleoside" as used herein refers to any nucleotide or nucleoside having an acyclic sugar, *e.g.,* an acyclic ribose. An exemplary acyclic nucleotide or nucleoside can include a nucleobase, *e.g.,* a naturally occurring or a modified nucleobase (*e.g.,* a nucleobase as described herein). In certain embodiments, a bond between any of the ribose carbons (C1, C2, C3, C4, or C5), is independently or in combination absent from the nucleotide. In some embodiments, the bond between C2-C3 carbons of the ribose ring is absent, e.g., an acyclic 2'-3'-seco-nucleotide monomer. In other embodiments, the bond between C1-C2, C3-C4, or C4-C5 is absent (*e.g.,* a 1'-2', 3'-4' or 4'-5'-seco nucleotide monomer). Exemplary acyclic nucleotides are disclosed in US 8,314,227, incorporated herein by reference in

its entirely. For example, an acyclic nucleotide can include any of monomers D-J in Figures 1-2 of US 8,314,227. In some embodiments, the acyclic nucleotide includes the following monomer:

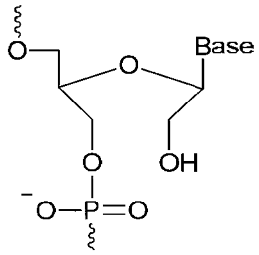

wherein Base is a nucleobase, *e.g.,* a naturally occurring or a modified nucleobase (*e.g.,* a nucleobase as described herein).

In certain embodiments, the acyclic nucleotide can be modified or derivatized, *e.g.,* by coupling the acyclic nucleotide to another moiety, *e.g.,* a ligand (*e.g.,* a GalNAc, a cholesterol ligand), an alkyl, a polyamine, a sugar, a polypeptide, among others.

In other embodiments, the iRNA agent includes one or more acyclic nucleotides and one or more LNAs (*e.g.,* an LNA as described herein). For example, one or more acyclic nucleotides and/or one or more LNAs can be present in the sense strand, the antisense strand, or both. The number of acyclic nucleotides in one strand can be the same or different from the number of LNAs in the opposing strand. In certain embodiments, the sense strand and/or the antisense strand comprises less than five LNAs (*e.g.,* four, three, two or one LNAs) located in the double stranded region or a 3'-overhang. In other embodiments, one or two LNAs are located in the double stranded region or the 3'-overhang of the sense strand. Alternatively, or in combination, the sense strand and/or antisense strand comprises less than five acyclic nucleotides (*e.g.*, four, three, two or one acyclic nucleotides) in the double-stranded region or a 3'-overhang. In some embodiments, the sense strand of the iRNA agent comprises one or two LNAs in the 3'-overhang of the sense strand, and one or two acyclic nucleotides in the double-stranded region of the antisense strand (*e.g.,* at positions 4 to 10 (*e.g.,* positions 6-8) from the 5'-end of the antisense strand) of the iRNA agent.

In other embodiments, inclusion of one or more acyclic nucleotides (alone or in addition to one or more LNAs) in the iRNA agent results in one or more (or all) of: (i) a reduction in an off-target effect; (ii) a reduction in passenger strand participation in RNAi; (iii) an increase in specificity of the guide strand for its target mRNA; (iv) a reduction in a microRNA off-target effect; (v) an increase in stability; or (vi) an increase in resistance to degradation, of the iRNA molecule.

Other modifications include 2'-methoxy (2'-$OCH_3$), 2'-5 aminopropoxy (2'-$OCH_2CH_2CH_2NH_2$) and 2'-fluoro (2'-F). Similar modifications may also be made at other positions on the RNA of an iRNA, particularly the 3' position of the sugar on the 3' terminal nucleotide or in 2'-5' linked dsRNAs and the 5' position of 5' terminal nucleotide. iRNAs may also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, certain of which are commonly owned with the instant application, and each of which is herein incorporated by reference.

An iRNA may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine.

Further modified nucleobases include those disclosed in U.S. Pat. No. 3,687,808, those disclosed in Modified Nucleosides in Biochemistry, Biotechnology and Medicine, Herdewijn, P. ed. Wiley-VCH, 2008; those disclosed in The Concise Encyclopedia of Polymer Science and Engineering, pages 858-859, Kroschwitz, J. L, ed. John Wiley & Sons, 1990, these disclosed by Englisch et al., Angewandte Chemie, International Edition, 1991, 30, 613, and those disclosed by Sanghvi, Y S., Chapter 15, dsRNA Research and Applications, pages 289-302, Crooke, S. T. and Lebleu, B., Ed., CRC Press, 1993. Certain of these modified nucleobases are particularly useful for increasing the binding affinity of the oligomeric compounds featured in the disclosure. These include 5-substituted pyrimidines, 6-azapyrimidines and N-2, N-6 and O-6 substituted purines, including 2-aminopropyladenine, 5-propynyluracil and 5-propynylcytosine. 5-methylcytosine

substitutions have been shown to increase nucleic acid duplex stability by 0.6-1.2°C (Sanghvi, Y. S., Crooke, S. T. and Lebleu, B., Eds., dsRNA Research and Applications, CRC Press, Boca Raton, 1993, pp. 276-278) and are exemplary base substitutions, even more particularly when combined with 2'-O-methoxyethyl sugar modifications.

Representative U.S. patents that teach the preparation of certain of the above noted modified nucleobases as well as other modified nucleobases include, but are not limited to, the above noted U.S. Pat. No. 3,687,808, as well as U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,681,941; 6,015,886; 6,147,200; 6,166,197; 6,222,025; 6,235,887; 6,380,368; 6,528,640; 6,639,062; 6,617,438; 7,045,610; 7,427,672; and 7,495,088, each of which is herein incorporated by reference, and U.S. Pat. No. 5,750,692, also herein incorporated by reference.

The RNA of an iRNA can also be modified to include one or more (e.g., about 1, 2, 3, 4, 5, 6, 7, 8, 9, 10, or more) bicyclic sugar moieties. A "bicyclic sugar" is a furanosyl ring modified by the bridging of two atoms. A "bicyclic nucleoside" ("BNA") is a nucleoside having a sugar moiety comprising a bridge connecting two carbon atoms of the sugar ring, thereby forming a bicyclic ring system. In certain embodiments, the bridge connects the 4'-carbon and the 2'-carbon of the sugar ring. Thus, in some embodiments an agent of the disclosure may include one or more locked nucleic acids (LNAs) (also referred to herein as "locked nucleotides"). In some embodiments, a locked nucleic acid is a nucleotide having a modified ribose moiety in which the ribose moiety comprises an extra bridge connecting, e.g., the 2' and 4' carbons. This structure effectively "locks" the ribose in the 3'-endo structural conformation. The addition of locked nucleic acids to siRNAs has been shown to increase siRNA stability in serum, increase thermal stability, and to reduce off-target effects (Elmen, J. et al., (2005) Nucleic Acids Research 33(1):439-447; Mook, OR. et al., (2007) Mol Canc Ther 6(3):833-843; Grunweller, A. et al., (2003) Nucleic Acids Research 31(12):3185-3193).

Examples of bicyclic nucleosides for use in the polynucleotides of the disclosure include without limitation nucleosides comprising a bridge between the 4' and the 2' ribosyl ring atoms. In certain embodiments, the antisense polynucleotide agents of the disclosure include one or more bicyclic nucleosides comprising a 4' to 2' bridge. Examples of such 4' to 2' bridged bicyclic nucleosides, include but are not limited to 4'-$(CH_2)$-O-2' (LNA); 4'-$(CH_2)$-S-2'; 4'-$(CH_2)$2-O-2' (ENA); 4'-$CH(CH_3)$-O-2' (also referred to as "constrained ethyl" or "cEt") and 4'-$CH(CH_2OCH_3)$-O-2' (and analogs thereof; see, *e.g.,* U.S. Pat. No. 7,399,845); 4'-$C(CH_3)(CH_3)$-O-2' (and analogs thereof; see *e.g.,* US Patent No. 8,278,283); 4'-$CH_2$-$N(OCH_3)$-2' (and analogs thereof; see *e.g.,* US Patent No. 8,278,425); 4'-$CH_2$-O-$N(CH_3)$-2' (see, e.g., U.S. Patent Publication No. 2004/0171570); 4'-$CH_2$-N(R)-O-2', wherein R is H, $C_1$-$C_{12}$ alkyl, or a protecting group (see, *e.g.,* U.S. Pat. No. 7,427,672); 4'-$CH_2$-$C(H)(CH_3)$-2' (see, *e.g.,* Chattopadhyaya et al., J. Org. Chem., 2009, 74, 118-134); and 4'-$CH_2$-$C(=CH_2)$-2' (and analogs thereof; see, *e.g.,* US Patent No. 8,278,426). The contents of each of the foregoing are incorporated herein by reference for the methods provided therein. Representative U.S. Patents that teach the preparation of locked nucleic acids include, but are not limited to, the following: U.S. Pat. Nos. 6,268,490; 6,670,461; 6,794,499; 6,998,484; 7,053,207; 7,084,125; 7,399,845, and 8,314,227, each of which is herein incorporated by reference in its entirety. Exemplary LNAs include but are not limited to, a 2', 4'-C methylene bicyclo nucleotide (see for example Wengel et al., International PCT 5 Publication No. WO 00/66604 and WO 99/14226).

Any of the foregoing bicyclic nucleosides can be prepared having one or more stereochemical sugar configurations including for example $\alpha$-L-ribofuranose and $\beta$-D-ribofuranose (see WO 99/14226).

A RNAi agent of the disclosure can also be modified to include one or more constrained ethyl nucleotides. As used herein, a "constrained ethyl nucleotide" or "cEt" is a locked nucleic acid comprising a bicyclic sugar moiety comprising a 4'-CH(CH3)-0-2' bridge. In some embodiments, a constrained ethyl nucleotide is in the S conformation referred to herein as "S-cEt."

A RNAi agent of the disclosure may also include one or more "conformationally restricted nucleotides" ("CRN"). CRN are nucleotide analogs with a linker connecting the C2'and C4' carbons of ribose or the C3 and -C5' carbons of ribose. CRN lock the ribose ring into a stable conformation and increase the hybridization affinity to mRNA. The linker is of sufficient length to place the oxygen in an optimal position for stability and affinity resulting in less ribose ring puckering.

Representative publications that teach the preparation of certain of the above noted CRN include, but are not limited to, US 2013/0190383; and WO 2013/036868, the contents of each of which are hereby incorporated herein by reference for the methods provided therein.

In some embodiments, a RNAi agent of the disclosure comprises one or more monomers that are UNA (unlocked nucleic acid) nucleotides. UNA is unlocked acyclic nucleic acid, wherein any of the bonds of the sugar has been removed, forming an unlocked "sugar" residue. In one example, UNA also encompasses monomer with bonds between C1'-C4' have been removed (i.e. the covalent carbon-oxygen-carbon bond between the C1' and C4' carbons). In another example, the C2'-C3' bond (i.e. the covalent carbon-carbon bond between the C2' and C3' carbons) of the sugar has been removed (see Nuc. Acids Symp. Series, 52, 133-134 (2008) and Fluiter et al., Mol. Biosyst., 2009, 10, 1039).

Representative U.S. publications that teach the preparation of UNA include, but are not limited to, US8,314,227; and US Patent Publication Nos. 2013/0096289; 2013/0011922; and 2011/0313020, the contents of each of which are hereby incorporated herein by reference for the methods provided therein.

An RNAi agent of the disclosure may also include one or more "cyclohexene nucleic acids" or ("CeNA"). CeNA

are nucleotide analogs with a replacement of the furanose moiety of DNA by a cyclohexene ring. Incorporation of cylcohexenyl nucleosides in a DNA chain increases the stability of a DNA/RNA hybrid. CeNA is stable against degradation in serum and a CeNA/RNA hybrid is able to activate E. Coli RNase H, resulting in cleavage of the RNA strand. (see Wang et al., Am. Chem. Soc. 2000, 122, 36, 8595-8602*,* hereby incorporated by reference).

In other embodiments, the iRNA agents include one or more (*e.g.,* about 1, 2, 3, 4, 5, 6, 7, 8, 9, 10, or more) G-clamp nucleotides. A G-clamp nucleotide is a modified cytosine analog wherein the modifications confer the ability to hydrogen bond both Watson-Crick and Hoogsteen faces of a complementary guanine within a duplex, see for example Lin and Matteucci, 1998, J. Am. Chem. Soc., 120, 8531-8532. A single G-clamp analog substitution within an oligonucleotide can result in substantially enhanced helical thermal stability and mismatch discrimination when hybridized to complementary oligonucleotides. The inclusion of such nucleotides in the iRNA molecules can result in enhanced affinity and specificity to nucleic acid targets, complementary sequences, or template strands.

Potentially stabilizing modifications to the ends of RNA molecules can include N-(acetylaminocaproyl)-4-hydroxyprolinol (Hyp-C6-NHAc), N-(caproyl-4-hydroxyprolinol (Hyp-C6), N-(acetyl-4-hydroxyprolinol (Hyp-NHAc), thymidine-2'-O-deoxythymidine (ether), N-(aminocaproyl)-4-hydroxyprolinol (Hyp-C6-amino), 2-docosanoyl-uridine-3"-phosphate, inverted base dT(idT) and others. Disclosure of this modification can be found in PCT Publication No. WO 2011/005861.

Other modifications of a RNAi agent of the disclosure include a 5' phosphate or 5' phosphate mimic, *e.g.,* a 5'-terminal phosphate or phosphate mimic on the antisense strand of a RNAi agent. Suitable phosphate mimics are disclosed in, for example US 2012/0157511, the contents of which are incorporated herein by reference for the methods provided therein. In one embodiment, the double stranded RNAi agent of the invention further comprises a 5'-phosphate or a 5'-phosphate mimic at the 5' nucleotide of the antisense strand. In another embodiment, the double stranded RNAi agent further comprises a 5'-phosphate mimic at the 5' nucleotide of the antisense strand. In a specific embodiment, the 5'-phosphate mimic is a 5'-vinyl phosphonate (5'-VP). In one embodiment, the phosphate mimic is a 5'-cyclopropyl phosphonate (VP). In some embodiments, the 5'-end of the antisense strand of the double-stranded iRNA agent does not contain a 5'-vinyl phosphonate (VP).

In one embodiment, at least one of the modified nucleotides is selected from the group consisting of a deoxy-nucleotide, a 2'-O-methyl modified nucleotide, a 2'-fluoro modified nucleotide, a 2'-deoxy-modified nucleotide, a glycol modified nucleotide (GNA), e.g., Ggn, Cgn, Tgn, or Agn, a nucleotide with a 2' phosphate, e.g., G2p, C2p, A2p or U2p, and, a vinylphosphonate nucleotide; and combinations thereof. In other embodiments, each of the duplexes of Tables 5, 6, 8, and 10 may be particularly modified to provide another double-stranded iRNA agent of the present disclosure. In one example, the 3'-terminus of each sense duplex may be modified by removing the 3'-terminal L96 ligand and exchanging the two phosphodiester internucleotide linkages between the three 3'-terminal nucleotides with phosphorothioate internucleotide linkages. That is, the three 3'-terminal nucleotides (N) of a sense sequence of the formula:

5'- N1-... -Nn-2Nn-1NnL96 3'

may be replaced with

5'- N1-... -Nn-2sNn-1sNn 3'.

That is, for example, AD-1559459, the sense sequence:
asgsaucgGfuGfCfCfgauuccug**cuL96**
may be replaced with
asgsaucgGfuGfCfCfgauuccu**gscsu**
while the antisense sequence remains unchanged to provide another double-stranded iRNA agent of the present disclosure.

### III. iRNA Motifs

In certain aspects of the disclosure, the double-stranded RNAi agents of the disclosure include agents with chemical modifications as disclosed, for example, in WO 2013/075035, the contents of which are incorporated herein by reference for the methods provided therein. As shown herein and in WO 2013/075035, a superior result may be obtained by introducing one or more motifs of three identical modifications on three consecutive nucleotides into a sense strand or antisense strand of an RNAi agent, particularly at or near the cleavage site. In some embodiments, the sense strand and antisense strand of the RNAi agent may otherwise be completely modified. The introduction of these motifs interrupts the modification pattern, if present, of the sense or antisense strand. The RNAi agent may be optionally conjugated with a lipophilic moiety or ligand, *e.g.,* a C16 moiety or ligand, for instance on the sense strand. The RNAi agent may be optionally modified with a (*S*)-glycol nucleic acid (GNA) modification, for instance on one or more residues of the antisense strand.

The resulting RNAi agents present superior gene silencing activity.

In some embodiments, the sense strand sequence may be represented by formula (I):

$$5'n_p\text{-}N_a\text{-}(XXX)_i\text{-}N_b\text{-}YYY\text{-}N_b\text{-}(ZZZ)_j\text{-}N_a\text{-}n_q\ 3' \qquad (I)$$

wherein:

i and j are each independently 0 or 1;
p and q are each independently 0-6;
each Na independently represents an oligonucleotide sequence comprising 0-25 modified nucleotides, each sequence comprising at least two differently modified nucleotides;
each $N_b$ independently represents an oligonucleotide sequence comprising 0-10 modified nucleotides;
each $n_p$ and $n_q$ independently represent an overhang nucleotide;
wherein Nb and Y do not have the same modification; and
XXX, YYY and ZZZ each independently represent one motif of three identical modifications on three consecutive nucleotides. In some embodiments, YYY is all 2'-F modified nucleotides.

In some embodiments, the $N_a$ and/or $N_b$ comprise modifications of alternating pattern.

In some embodiments, the YYY motif occurs at or near the cleavage site of the sense strand. For example, when the RNAi agent has a duplex region of 17-23 nucleotides in length, the YYY motif can occur at or the vicinity of the cleavage site (*e.g.:* can occur at positions 6, 7, 8; 7, 8, 9; 8, 9, 10; 9, 10, 11; 10, 11,12 or 11, 12, 13) of the sense strand, the count starting from the $1^{st}$ nucleotide, from the 5'-end; or optionally, the count starting at the $1_{st}$ paired nucleotide within the duplex region, from the 5'-end.

In some embodiments, i is 1 and j is 0, or i is 0 and j is 1, or both i and j are 1. The sense strand can therefore be represented by the following formulas:

$$5'n_p\text{-}Na\text{-}YYY\text{-}N_b\text{-}ZZZ\text{-}Na\text{-}n_q\ 3' \qquad (Ib);$$

$$5'n_p\text{-}N_a\text{-}XXX\text{-}N_b\text{-}YYY\text{-}N_a\text{-}n_q\ 3' \qquad (Ic);$$

or

$$5'n_p\text{-}Na\text{-}XXX\text{-}N_b\text{-}YYY\text{-}N_b\text{-}ZZZ\text{-}Na\text{-}n_q\ 3' \qquad (Id).$$

When the sense strand is represented by formula (Ib), $N_b$ represents an oligonucleotide sequence comprising 0-10, 0-7, 0-5, 0-4, 0-2 or 0 modified nucleotides. Each $N_a$ independently can represent an oligonucleotide sequence comprising 2-20, 2-15, or 2-10 modified nucleotides.

When the sense strand is represented as formula (Ic), $N_b$ represents an oligonucleotide sequence comprising 0-10, 0-7, 0-5, 0-4, 0-2 or 0 modified nucleotides. Each $N_a$ can independently represent an oligonucleotide sequence comprising 2-20, 2-15, or 2-10 modified nucleotides.

When the sense strand is represented as formula (Id), each $N_b$ independently represents an oligonucleotide sequence comprising 0-10, 0-7, 0-5, 0-4, 0-2 or 0 modified nucleotides. In some embodiments, $N_b$ is 0, 1, 2, 3, 4, 5 or 6. Each $N_a$ can independently represent an oligonucleotide sequence comprising 2-20, 2-15, or 2-10 modified nucleotides.

Each of X, Y and Z may be the same or different from each other.

In other embodiments, i is 0 and j is 0, and the sense strand may be represented by the formula:

$$5'n_p\text{-}N_a\text{-}YYY\text{-}\ N_a\text{-}n_q\ 3' \qquad (Ia).$$

When the sense strand is represented by formula (Ia), each $N_a$ independently can represent an oligonucleotide sequence comprising 2-20, 2-15, or 2-10 modified nucleotides.

In some embodiments, the antisense strand sequence of the RNAi may be represented by formula (Ie):

$$5'n_{q'}\text{-}N_a'\text{-}(Z'Z'Z')_k\text{-}N_b'\text{-}Y'Y'Y'\text{-}N_b'\text{-}(X'X'X')_l\text{-}N'_a\text{-}n_p'\ 3' \qquad (Ie)$$

wherein:

k and l are each independently 0 or 1;
p' and q' are each independently 0-6;

each $N_a$' independently represents an oligonucleotide sequence comprising 0-25 modified nucleotides, each sequence comprising at least two differently modified nucleotides;
each $N_b$' independently represents an oligonucleotide sequence comprising 0-10 modified nucleotides;
each $n_p$' and $n_q$' independently represent an overhang nucleotide;
wherein $N_b$' and Y' do not have the same modification;
and
X'X'X', Y'Y'Y', and Z'Z'Z' each independently represent one of three identical modification on three consecutive nucleotides.

In some embodiments, the $N_a$' and/or $N_b$' comprise modification of alternating pattern.
The Y'Y'Y' motif occurs at or near the cleavage site of the antisense strand. For example, when the RNAi agent has a duplex region of 17-23 nucleotides in length, the Y'Y'Y' motif can occur at positions 9, 10, 11; 10, 11, 12; 11, 12, 13; 12, 13, 14; or 13, 14, 15 of the antisense strand, with the count starting from the 1st nucleotide, from the 5'-end; or optionally, the count starting at the 1st paired nucleotide within the duplex region, from the 5'- end. In some embodiments, the Y'Y'Y' motif occurs at positions 11, 12, 13.
In some embodiments, Y'Y'Y' motif is all 2'-O-me modified nucleotides.
In on embodiment, k is 1 and l is 0, or k is 0 and l is 1, or both 5 k and l are 1. The antisense strand can therefore be represented by the following formulas:

$$5'n_q'\text{-}N_a'\text{-}Z'Z'Z'\text{-}N_b'\text{-}Y'Y'Y'\text{-}N_a'\text{-}n_p'\ 3' \quad \text{(Ig);}$$

$$5'n_q'\text{-}N_a'\text{-}Y'Y'Y'\text{-}N_b'\text{-}X'X'X'\text{-}n_p'\ 3' \quad \text{(Ih);}$$

or

$$5'n_q'\text{-}N_a'\text{-}\ Z'Z'Z'\text{-}N_b'\text{-}Y'Y'Y'\text{-}N_b'\text{-}\ X'X'X'\text{-}N_a'\text{-}n_p'\ 3' \quad \text{(Ii).}$$

When the antisense strand is represented by formula (IgIb), $N_{b'}$ represents an oligonucleotide sequence comprising 0-10, 0-7, 0-5, 0-4, 0-2 or 0 modified nucleotides. Each $N_{a'}$ independently represents an oligonucleotide sequence comprising 2-20, 2-15, or 2-10 modified nucleotides.
When the antisense strand is represented as formula (Ii), each $N_{b'}$ independently represents an oligonucleotide sequence comprising 0-10, 0-7, 0-5, 0-4, 0-2 or 0 modified nucleotides. Each $N_a$' independently represents an oligonucleotide sequence comprising 2-20, 2-15, or 2-10 modified nucleotides. In some embodiments, $N_b$ is 0, 1, 2, 3, 4, 5 or 6.
In other embodiments, k is 0 and 1 is 0 and the antisense strand may be represented by the formula:

$$5'n_{p'}\text{-}N_{a'}\text{-}Y'Y'Y'\text{-}\ N_{a'}\text{-}n_{q'}\ 3' \quad \text{(If)}$$

.

When the antisense strand is represented as formula (If), each Na' independently represents an oligonucleotide sequence comprising 2-20, 2-15, or 2-10 modified nucleotides.
Each of X', Y' and Z' may be the same or different from each other.
Each nucleotide of the sense strand and antisense strand may be independently modified with LNA, HNA, CeNA, GNA, 2'-methoxyethyl, 2'-O-methyl, 2'-O-allyl, 2'-C- allyl, 2'-hydroxyl, or 2'-fluoro. For example, each nucleotide of the sense strand and antisense strand is independently modified with 2'-O-methyl or 2'-fluoro. Each X, Y, Z, X', Y' and Z', in particular, may represent a 2'-O-methyl modification or a 2'-fluoro modification.
In some embodiments, the sense strand of the RNAi agent may contain YYY motif occurring at 9, 10 and 11 positions of the strand when the duplex region is 21 nt, the count starting from the 1st nucleotide from the 5'-end, or optionally, the count starting at the 1st paired nucleotide within the duplex region, from the 5'- end; and Y represents 2'-F modification. The sense strand may additionally contain XXX motif or ZZZ motifs as wing modifications at the opposite end of the duplex region; and XXX and ZZZ each independently represents a 2'-OMe modification or 2'-F modification.
In some embodiments the antisense strand may Y'Y'Y' motif occurring at positions 11, 12, 13 of the strand, the count starting from the 1st nucleotide from the 5'-end, or optionally, the count starting at the 1st paired nucleotide within the duplex region, from the 5'- end; and Y' represents 2'-O-methyl modification. The antisense strand may additionally contain X'X'X' motif or Z'Z'Z' motifs as wing modifications at the opposite end of the duplex region; and X'X'X' and Z'Z'Z' each independently represents a 2'-OMe modification or 2'-F modification.
The sense strand represented by any one of the above formulas (Ia), (Ib), (Ic), and (Id) forms a duplex with an antisense strand being represented by any one of formulas (If), (Ig), (Ih), and (Ii), respectively.

Accordingly, certain RNAi agents for use in the methods of the disclosure may comprise a sense strand and an antisense strand, each strand having 14 to 30 nucleotides, the RNAi duplex represented by formula (Ij):

sense: 5' $n_p$ -$N_a$-(XXX)i-$N_b$-YYY-$N_b$-(ZZZ)j-$N_a$-$n_q$ 3'
antisense: 3'$n_p$'-Na'-(X'X'X')k-$N_b$'-Y'Y'Y'-$N_b$'-(Z'Z'Z')$_l$-$N_a$'-$n_q$' 5'(Ij)

wherein,

i, j, k, and l are each independently 0 or 1;
p, p', q, and q' are each independently 0-6;
each $N_a$ and $N_a$' independently represents an oligonucleotide sequence comprising 0-25 modified nucleotides, each sequence comprising at least two differently modified nucleotides;
each $N_b$ and $N_b$' independently represents an oligonucleotide sequence comprising 0-10 modified nucleotides; wherein
each $n_p$', $n_p$, $n_q$', and $n_q$, each of which may or may not be present independently represents an overhang nucleotide; and
XXX, YYY, ZZZ, X'X'X', Y'Y'Y', and Z'Z'Z' each independently represent one motif of three identical modifications on three consecutive nucleotides.

In some embodiments, i is 0 and j is 0; or i is 1 and j is 0; or i is 0 and j is 1; or both i and j are 0; or both i and j are 1. In some embodiments, k is 0 and l is 0; or k is 1 and l is 0; k is 0 and l is 1; or both k and 1 are 0; or both k and l are 1.

Exemplary combinations of the sense strand and antisense strand forming a RNAi duplex include the formulas below:

5' $n_p$ -$N_a$-YYY-$N_a$-$n_q$ 3'

3'$n_p$'-$N_a$'-Y'Y'Y'-$N_a$'$n_q$' 5' (Ik)

5' $n_p$ -$N_a$-YYY-$N_b$-ZZZ-$N_a$-$n_q$ 3'

3'$n_p$-$N_a$'-Y'Y'Y'-$N_b$'-Z'Z'Z'-Na'-nq'5' (Il)

5' $n_p$ -$N_a$ - XXX-$N_b$-YYY-$N_a$-$n_q$ 3'

3'$n_p$-$N_a$'-X'X'X'-$N_b$'-Y'Y'Y'-Na'-$n_q$' 5' (Im)

5' $n_p$ -$N_a$ - XXX-$N_b$-YYY-$N_b$-ZZZ-$N_a$-$n_q$ 3'

3' $n_p$ -$N_a$'-X'X'X'-$N_b$'-Y'Y'Y'-$N_b$'-Z'Z'Z'-Na'-$n_q$' 5' (In)

When the RNAi agent is represented by formula (Ik), each $N_a$ independently represents an oligonucleotide sequence comprising 2-20, 2-15, or 2-10 modified nucleotides.

When the RNAi agent is represented by formula (Il), each $N_b$ independently represents an oligonucleotide sequence comprising 1-10, 1-7, 1-5 or 1-4 modified nucleotides. Each $N_a$ independently represents an oligonucleotide sequence comprising 2-20, 2-15, or 2-10 modified nucleotides.

When the RNAi agent is represented as formula (Im), each $N_b$, $N_b$' independently represents an oligonucleotide sequence comprising 0-10, 0-7, 0-5, 0-4, 0-2 or 0 modified nucleotides. Each $N_a$ independently represents an oligonucleotide sequence comprising 2-20, 2-15, or 2-10 modified nucleotides.

When the RNAi agent is represented as formula (In), each $N_b$, $N_b$' independently represents an oligonucleotide sequence comprising 0-10, 0-7, 0-5, 0-4, 0-2 or 0 modified nucleotides. Each $N_a$, $N_a$' independently represents an oligonucleotide sequence comprising 2-20, 2-15, or 2-10 modified nucleotides. Each of $N_a$, $N_a$', $N_b$ and $N_b$' independently comprises modifications of alternating pattern.

Each of X, Y and Z in formulas (Ij), (Ik), (Il), (Im), and (In) may be the same or different from each other.

When the RNAi agent is represented by formula (Ij), (Ik), (Il), (Im), and (In), at least one of the Y nucleotides may form a base pair with one of the Y' nucleotides. Alternatively, at least two of the Y nucleotides form base pairs with the corresponding Y' nucleotides; or all three of the Y nucleotides all form base pairs with the corresponding Y' nucleotides.

When the RNAi agent is represented by formula (Il) or (In), at least one of the Z nucleotides may form a base pair

with one of the Z' nucleotides. Alternatively, at least two of the Z nucleotides form base pairs with the corresponding Z' nucleotides; or all three of the Z nucleotides all form base pairs with the corresponding Z' nucleotides.

When the RNAi agent is represented as formula (Im) or (In), at least one of the X nucleotides may form a base pair with one of the X' nucleotides. Alternatively, at least two of the X nucleotides form base pairs with the corresponding X' nucleotides; or all three of the X nucleotides all form base pairs with the corresponding X' nucleotides.

In some embodiments, the modification on the Y nucleotide is different than the modification on the Y' nucleotide, the modification on the Z nucleotide is different than the modification on the Z' nucleotide, and/or the modification on the X nucleotide is different than the modification on the X' nucleotide.

In some embodiments, when the RNAi agent is represented by formula (IIId), the $N_a$ modifications are 2'-O-methyl or 2'-fluoro modifications. In some embodiments, when the RNAi agent is represented by formula (In), the $N_a$ modifications are 2'-O-methyl or 2'-fluoro modifications and $n_p$' >0 and at least one $n_p$' is linked to a neighboring nucleotide a via phosphorothioate linkage. In some embodiments, when the RNAi agent is represented by formula (In), the $N_a$ modifications are 2'-O-methyl or 2'-fluoro modifications, $n_p$' >0 and at least one $n_p$' is linked to a neighboring nucleotide via phosphorothioate linkage, and the sense strand is conjugated to one or more moieties or ligands (*e.g.,* one or more lipophilic moieties, optionally one or more C16 moieties, or one or more GalNAc moieties) attached through a bivalent or trivalent branched linker. In some embodiments, when the RNAi agent is represented by formula (In), the $N_a$ modifications are 2'-O-methyl or 2'-fluoro modifications, $n_p$' >0 and at least one $n_p$' is linked to a neighboring nucleotide via phosphorothioate linkage, the sense strand comprises at least one phosphorothioate linkage, and the sense strand is conjugated to one or more moieties or ligands (*e.g.*, one or more lipophilic moieties, optionally one or more C16 moieties, or one or more GalNAc moieties) attached through a bivalent or trivalent branched linker.

In some embodiments, when the RNAi agent is represented by formula (Ik), the $N_a$ modifications are 2'-O-methyl or 2'-fluoro modifications, $n_p$' >0 and at least one $n_p$' is linked to a neighboring nucleotide via phosphorothioate linkage, the sense strand comprises at least one phosphorothioate linkage, and the sense strand is conjugated to one or more moieties or ligands (*e.g.,* one or more lipophilic moieties, optionally one or more C16 moieties, or one or more GalNAc moieties) attached through a bivalent or trivalent branched linker.

In some embodiments, the RNAi agent is a multimer containing at least two duplexes represented by formula (Ij), (Ik), (Il), (Im), and (In), wherein the duplexes are connected by a linker. The linker can be cleavable or non-cleavable. Optionally, the multimer further comprises a ligand. Each of the duplexes can target the same gene or two different genes; or each of the duplexes can target same gene at two different target sites.

In some embodiments, the RNAi agent is a multimer containing three, four, five, six or more duplexes represented by formula (Ij), (Ik), (Il), (Im), and (In), wherein the duplexes are connected by a linker. The linker can be cleavable or non-cleavable. Optionally, the multimer further comprises a ligand. Each of the duplexes can target the same gene or two different genes; or each of the duplexes can target same gene at two different target sites.

In some embodiments, two RNAi agents represented by formula (Ij), (Ik), (Il), (Im), and (In) are linked to each other at the 5' end, and one or both of the 3' ends and are optionally conjugated to a ligand. Each of the agents can target the same gene or two different genes; or each of the agents can target same gene at two different target sites.

Various publications describe multimeric RNAi agents that can be used in the methods of the disclosure. Such publications include WO2007/091269, WO2010/141511, WO2007/117686, WO2009/014887, and WO2011/031520; and US 7858769, the contents of each of which are hereby incorporated herein by reference for the methods provided therein. In certain embodiments, the RNAi agents of the disclosure may include GalNAc ligands.

As described in more detail below, the RNAi agent that contains conjugations of one or more carbohydrate moieties to a RNAi agent may improve one or more properties of the RNAi agent. In many cases, the carbohydrate moiety will be attached to a modified subunit of the RNAi agent. For example, the ribose sugar of one or more ribonucleotide subunits of a dsRNA agent can be replaced with another moiety, *e.g.,* a non-carbohydrate (*e.g.,* cyclic) carrier to which is attached a carbohydrate ligand. A ribonucleotide subunit in which the ribose sugar of the subunit has been so replaced is referred to herein as a ribose replacement modification subunit (RRMS). A cyclic carrier may be a carbocyclic ring system, *i.e.,* all ring atoms are carbon atoms, or a heterocyclic ring system, *i.e.,* one or more ring atoms may be a heteroatom, *e.g.,* nitrogen, oxygen, sulfur. The cyclic carrier may be a monocyclic ring system, or may contain two or more rings, *e.g.* fused rings. The cyclic carrier may be a fully saturated ring system, or it may contain one or more double bonds.

The ligand may be attached to the polynucleotide via a carrier. The carriers include (i) at least one "backbone attachment point," such as two "backbone attachment points" and (ii) at least one "tethering attachment point." A "backbone attachment point" as used herein refers to a functional group, *e.g.* a hydroxyl group, or generally, a bond available for, and that is suitable for incorporation of the carrier into the backbone, *e.g.,* the phosphate, or modified phosphate, *e.g.,* sulfur containing, backbone, of a ribonucleic acid. A "tethering attachment point" (TAP) in some embodiments refers to a constituent ring atom of the cyclic carrier, *e.g.,* a carbon atom or a heteroatom (distinct from an atom which provides a backbone attachment point), that connects a selected moiety. The moiety can be, *e.g.,* a carbohydrate, *e.g.* monosaccharide, disaccharide, trisaccharide, tetrasaccharide, oligosaccharide, and polysaccharide. Optionally, the selected moiety is connected by an intervening tether to the cyclic carrier. Thus, the cyclic carrier will often

include a functional group, *e.g.,* an amino group, or generally, provide a bond, that is suitable for incorporation or tethering of another chemical entity, *e.g.,* a ligand to the constituent ring.

The RNAi agents may be conjugated to a ligand *via* a carrier, wherein the carrier can be cyclic group or acyclic group. The cyclic group can be selected from pyrrolidinyl, pyrazolinyl, pyrazolidinyl, imidazolinyl, imidazolidinyl, piperidinyl, piperazinyl, [1,3]dioxolane, oxazolidinyl, isoxazolidinyl, morpholinyl, thiazolidinyl, isothiazolidinyl, quinoxalinyl, pyridazinonyl, tetrahydrofuryl and and decalin. The acyclic group can be a serinol backbone or diethanolamine backbone.

In certain specific embodiments, the RNAi agent for use in the methods of the disclosure is an agent selected from the group of agents listed in any one of Tables 3-10. These agents may further comprise a ligand. The ligand can be attached to the sense strand, antisense strand or both strands, at the 3'-end, 5'-end, or both ends. For instance, the ligand may be conjugated to the sense strand, in particular, the 3'-end of the sense strand.

### IV. iRNA Conjugates

The iRNA agents disclosed herein can be in the form of conjugates. The conjugate may be attached at any suitable location in the iRNA molecule, *e.g.*, at the 3' end or the 5' end of the sense or the antisense strand. The conjugates are optionally attached via a linker.

In some embodiments, an iRNA agent described herein is chemically linked to one or more ligands, moieties or conjugates, which may confer functionality, *e.g.,* by affecting (*e.g.,* enhancing) the activity, cellular distribution or cellular uptake of the iRNA. Such moieties include but are not limited to lipid moieties such as a cholesterol moiety (Letsinger et al., Proc. Natl. Acid. Sci. USA, 1989, 86: 6553-6556), cholic acid (Manoharan et al., Biorg. Med. Chem. Let., 1994, 4:1053-1060), a thioether, *e.g.,* beryl-S-tritylthiol (Manoharan et al., Ann. N.Y. Acad. Sci., 1992, 660:306-309; Manoharan et al., Biorg. Med. Chem. Let., 1993, 3:2765-2770), a thiocholesterol (Oberhauser et al., Nucl. Acids Res., 1992, 20:533-538), an aliphatic chain, *e.g.,* dodecandiol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), a phospholipid, *e.g.*, di-hexadecyl-rac-glycerol or triethyl-ammonium 1,2-di-O-hexadecyl-rac-glycero-3-phosphonate (Manoharan et al., Tetrahedron Lett., 1995, 36:3651-3654; Shea et al., Nucl. Acids Res., 1990, 18:3777-3783), a polyamine or a polyethylene glycol chain (Manoharan et al., Nucleosides & Nucleotides, 1995, 14:969-973), or adamantane acetic acid (Manoharan et al., Tetrahedron Lett., 1995, 36:3651-3654), a palmityl moiety (Mishra et al., Biochim. Biophys. Acta, 1995, 1264:229-237), or an octadecylamine or hexylamino-carbonyloxycholesterol moiety (Crooke et al., J. Pharmacol. Exp. Ther., 1996, 277:923-937).

In some embodiments, a ligand alters the distribution, targeting or lifetime of an iRNA agent into which it is incorporated. In some embodiments, a ligand provides an enhanced affinity for a selected target, *e.g.,* molecule, cell or cell type, compartment, *e.g.,* a cellular or organ compartment, tissue, organ or region of the body, as, *e.g.*, compared to a species absent such a ligand. Typical ligands will not take part in duplex pairing in a duplexed nucleic acid.

Ligands can include a naturally occurring substance, such as a protein (*e.g.,* human serum albumin (HSA), low-density lipoprotein (LDL), or globulin); carbohydrate (*e.g.,* a dextran, pullulan, chitin, chitosan, inulin, cyclodextrin or hyaluronic acid); or a lipid. The ligand may also be a recombinant or synthetic molecule, such as a synthetic polymer, *e.g.*, a synthetic polyamino acid. Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Examples of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an $\alpha$ helical peptide.

Ligands can also include targeting groups, *e.g.,* a cell or tissue targeting agent, *e.g.,* a lectin, glycoprotein, lipid or protein, *e.g.*, an antibody, that binds to a specified cell type such as an ocular cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B 12, biotin, or an RGD peptide or RGD peptide mimetic.

Other examples of ligands include dyes, intercalating agents (*e.g.* acridines), cross-linkers (*e.g.* psoralene, mitomycin C), porphyrins (TPPC4, texaphyrin, Sapphyrin), polycyclic aromatic hydrocarbons (*e.g.,* phenazine, dihydrophenazine), artificial endonucleases (*e.g.* EDTA), lipophilic molecules, *e.g.*, cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid,O3-(oleoyl)lithocholic acid, O3-(oleoyl)cholenic acid, dimethoxytrityl, or phenoxazine)and peptide conjugates (*e.g.*, antennapedia peptide, Tat peptide), alkylating agents, phosphate, amino, mercapto, PEG (*e.g.*, PEG-40K), mPEG, $[mPEG]_2$, polyamino, alkyl, substituted alkyl, radiolabeled markers, enzymes, haptens (*e.g.* biotin), transport/absorption facilitators (*e.g.,* aspirin,

vitamin E, folic acid), synthetic ribonucleases (*e.g.,* imidazole, bisimidazole, histamine, imidazole clusters, acridine-imidazole conjugates, Eu3+ complexes of tetraazamacrocycles), dinitrophenyl, HRP, or AP.

Ligands can be proteins, *e.g.,* glycoproteins, or peptides, *e.g.,* molecules having a specific affinity for a co-ligand, or antibodies *e.g.,* an antibody, that binds to a specified cell type such as an ocular cell. Ligands may also include hormones and hormone receptors. They can also include non-peptidic species, such as lipids, lectins, carbohydrates, vitamins, cofactors, multivalent lactose, multivalent galactose, N-acetyl-galactosamine, N-acetyl-glucosamine multivalent mannose, or multivalent fucose. The ligand can be, for example, a lipopolysaccharide, an activator of p38 MAP kinase, or an activator of NF-κB.

The ligand can be a substance, *e.g.,* a drug, which can increase the uptake of the iRNA agent into the cell, for example, by disrupting the cell's cytoskeleton, *e.g.,* by disrupting the cell's microtubules, microfilaments, and/or intermediate filaments. The drug can be, for example, taxon, vincristine, vinblastine, cytochalasin, nocodazole, japlakinolide, latrunculin A, phalloidin, swinholide A, indanocine, or myoservin.

In some embodiments, a ligand attached to an iRNA as described herein acts as a pharmacokinetic modulator (PK modulator). PK modulators include lipophiles, bile acids, steroids, phospholipid analogues, peptides, protein binding agents, polyethylene glycol (PEG), vitamins *etc.* Exemplary PK modulators include, but are not limited to, cholesterol, fatty acids, cholic acid, lithocholic acid, dialkylglycerides, diacylglyceride, phospholipids, sphingolipids, naproxen, ibuprofen, vitamin E, biotin *etc.* Oligonucleotides that comprise a number of phosphorothioate linkages are also known to bind to serum protein, thus short oligonucleotides, *e.g.,* oligonucleotides of about 5 bases, 10 bases, 15 bases or 20 bases, comprising multiple of phosphorothioate linkages in the backbone are also amenable to the present disclosure as ligands (*e.g.* as PK modulating ligands). In addition, aptamers that bind serum components (*e.g.* serum proteins) are also suitable for use as PK modulating ligands in the embodiments described herein.

Ligand-conjugated oligonucleotides of the disclosure may be synthesized by the use of an oligonucleotide that bears a pendant reactive functionality, such as that derived from the attachment of a linking molecule onto the oligonucleotide (described below). This reactive oligonucleotide may be reacted directly with commercially available ligands, ligands that are synthesized bearing any of a variety of protecting groups, or ligands that have a linking moiety attached thereto.

The oligonucleotides used in the conjugates of the present disclosure may be conveniently and routinely made through the well-known technique of solid-phase synthesis. Equipment for such synthesis is sold by several vendors including, for example, Applied Biosystems (Foster City, Calif.). Any other means for such synthesis known in the art may additionally or alternatively be employed. It is also known to use similar techniques to prepare other oligonucleotides, such as the phosphorothioates and alkylated derivatives.

In the ligand-conjugated oligonucleotides and ligand-molecule bearing sequence-specific linked nucleosides of the present disclosure, the oligonucleotides and oligonucleosides may be assembled on a suitable DNA synthesizer utilizing standard nucleotide or nucleoside precursors, or nucleotide or nucleoside conjugate precursors that already bear the linking moiety, ligand-nucleotide or nucleoside-conjugate precursors that already bear the ligand molecule, or non-nucleoside ligand-bearing building blocks.

When using nucleotide-conjugate precursors that already bear a linking moiety, the synthesis of the sequence-specific linked nucleosides is typically completed, and the ligand molecule is then reacted with the linking moiety to form the ligand-conjugated oligonucleotide. In some embodiments, the oligonucleotides or linked nucleosides of the present disclosure are synthesized by an automated synthesizer using phosphoramidites derived from ligand-nucleoside conjugates in addition to the standard phosphoramidites and non-standard phosphoramidites that are commercially available and routinely used in oligonucleotide synthesis.

*A. Lipophilic Moieties*

In certain embodiments, the lipophilic moiety is an aliphatic, cyclic such as alicyclic, or polycyclic such as polyalicyclic compound, such as a steroid (*e.g.,* sterol) or a linear or branched aliphatic hydrocarbon. The lipophilic moiety may generally comprise a hydrocarbon chain, which may be cyclic or acyclic. The hydrocarbon chain may comprise various substituents or one or more heteroatoms, such as an oxygen or nitrogen atom. Such lipophilic aliphatic moieties include, without limitation, saturated or unsaturated $C_4$-$C_{30}$ hydrocarbon (*e.g.,* $C_6$-$C_{18}$ hydrocarbon), saturated or unsaturated fatty acids, waxes (*e.g.,* monohydric alcohol esters of fatty acids and fatty diamides), terpenes (*e.g.,* $C_{10}$ terpenes, $C_{15}$ sesquiterpenes, $C_{20}$ diterpenes, $C_{30}$ triterpenes, and $C_{40}$ tetraterpenes), and other polyalicyclic hydrocarbons. For instance, the lipophilic moiety may contain a $C_4$-$C_{30}$ hydrocarbon chain (*e.g.,* $C_4$-$C_{30}$ alkyl or alkenyl). In some embodiments the lipophilic moiety contains a saturated or unsaturated $C_6$-$C_{18}$ hydrocarbon chain (*e.g.,* a linear $C_6$-$C_{18}$ alkyl or alkenyl). In some embodiments, the lipophilic moiety contains a saturated or unsaturated C16 hydrocarbon chain (*e.g.,* a linear C16 alkyl or alkenyl).

In some embodiments, the lipophilic moiety is a C6-C30 acid (*e.g.,* hexanoic acid, heptanoic acid, octanoic acid, nonanoic acid, decanoic acid, undecanoic acid, dodcanoic acid, tridecanoic acid, tetradecanoic acid, pentadecanoic acid,

hexadecanoic acid, heptadecanoic acid, octadecanoic acid, oleic acid, linoleic acid, arachidonic acid, cis-4,7,10,13,16,19-docosahexanoic acid, vitamin A, vitamin E, cholesterol etc.) or a C6-C30 alcohol (e.g., hexanol, heptanol, octanol, nonanol, decanol, undecanol, dodcanol, tridecanol, tetradecanol, pentadecanol, hexadecanol, heptadecanol, octadecanol, oleyl alcohol, linoleyl alcohol, arachidonic alcohol, cis-4,7,10,13,16,19-docosahexanol, retinol, vitamin E, cholesterol etc.).

The lipophilic moiety may be attached to the RNAi agent by any method known in the art, including via a functional grouping already present in the lipophilic moiety or introduced into the RNAi agent, such as a hydroxy group (*e.g.,* -CO-$CH_2$-OH). The functional groups already present in the lipophilic moiety or introduced into the RNAi agent include, but are not limited to, hydroxyl, amine, carboxylic acid, sulfonate, phosphate, thiol, azide, and alkyne.

Conjugation of the RNAi agent and the lipophilic moiety may occur, for example, through formation of an ether or a carboxylic or carbamoyl ester linkage between the hydroxy and an alkyl group R-, an alkanoyl group RCO- or a substituted carbamoyl group RNHCO-. The alkyl group R may be cyclic (*e.g.,* cyclohexyl) or acyclic (*e.g.,* straight-chained or branched; and saturated or unsaturated). Alkyl group R may be a butyl, pentyl, hexyl, heptyl, octyl, nonyl, decyl, undecyl, dodecyl, tridecyl, tetradecyl, pentadecyl, hexadecyl, heptadecyl or octadecyl group, or the like.

In some embodiments, the lipophilic moiety is conjugated to the double-stranded RNAi agent via a linker a linker containing an ether, thioether, urea, carbonate, amine, amide, maleimide-thioether, disulfide, phosphodiester, sulfonamide linkage, a product of a click reaction (*e.g.,* a triazole from the azide-alkyne cycloaddition), or carbamate.

In another embodiment, the lipophilic moiety is a steroid, such as sterol. Steroids are polycyclic compounds containing a perhydro-1,2-cyclopentanophenanthrene ring system. Steroids include, without limitation, bile acids (*e.g.,* cholic acid, deoxycholic acid and dehydrocholic acid), cortisone, digoxigenin, testosterone, cholesterol, and cationic steroids, such as cortisone. A "cholesterol derivative" refers to a compound derived from cholesterol, for example by substitution, addition or removal of substituents.

In another embodiment, the lipophilic moiety is an aromatic moiety. In this context, the term "aromatic" refers broadly to mono- and polyaromatic hydrocarbons. Aromatic groups include, without limitation, $C_6$-$C_{14}$ aryl moieties comprising one to three aromatic rings, which may be optionally substituted; "aralkyl" or "arylalkyl" groups comprising an aryl group covalently linked to an alkyl group, either of which may independently be optionally substituted or unsubstituted; and "heteroaryl" groups. As used herein, the term "heteroaryl" refers to groups having 5 to 14 ring atoms, preferably 5, 6, 9, or 10 ring atoms; having 6, 10, or 14$\pi$ electrons shared in a cyclic array, and having, in addition to carbon atoms, one to about three heteroatoms selected from the group consisting of nitrogen (N), oxygen (O), and sulfur (S).

As employed herein, a "substituted" alkyl, cycloalkyl, aryl, heteroaryl, or heterocyclic group is one having one to about four, preferably one to about three, more preferably one or two, non-hydrogen substituents. Suitable substituents include, without limitation, halo, hydroxy, nitro, haloalkyl, alkyl, alkaryl, aryl, aralkyl, alkoxy, aryloxy, amino, acylamino, alkylcarbamoyl, arylcarbamoyl, aminoalkyl, alkoxycarbonyl, carboxy, hydroxyalkyl, alkanesulfonyl, arenesulfonyl, alkanesulfonamido, arenesulfonamido, aralkylsulfonamido, alkylcarbonyl, acyloxy, cyano, and ureido groups.

In some embodiments, the lipophilic moiety is an aralkyl group, *e.g.,* a 2-arylpropanoyl moiety. The structural features of the aralkyl group are selected so that the lipophilic moiety will bind to at least one protein in vivo. In certain embodiments, the structural features of the aralkyl group are selected so that the lipophilic moiety binds to serum, vascular, or cellular proteins. In certain embodiments, the structural features of the aralkyl group promote binding to albumin, an immunoglobulin, a lipoprotein, $\alpha$-2-macroglubulin, or $\alpha$-1-glycoprotein.

In certain embodiments, the ligand is naproxen or a structural derivative of naproxen. Procedures for the synthesis of naproxen can be found in U.S. Pat. No. 3,904,682 and U.S. Pat. No. 4,009,197, which are hereby incorporated by reference in their entirety. Naproxen has the chemical name (S)-6-Methoxy-$\alpha$-methyl-2-naphthaleneacetic acid and the structure is

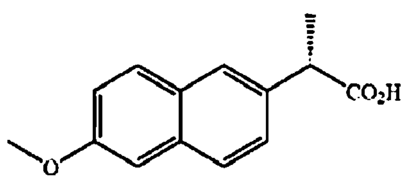

.

In certain embodiments, the ligand is ibuprofen or a structural derivative of ibuprofen. Procedures for the synthesis of ibuprofen can be found in US3,228,831, which is incorporated herein by reference for the methods provided therein. The structure of ibuprofen is

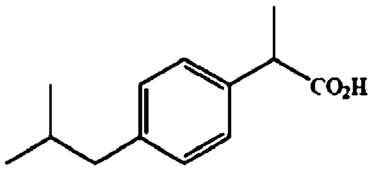

.

Additional exemplary aralkyl groups are illustrated in US 7,626,014, which is incorporated herein by reference for the methods provided therein.

In another embodiment, suitable lipophilic moieties include lipid, cholesterol, retinoic acid, cholic acid, adamantane acetic acid, 1-pyrene butyric acid, dihydrotestosterone, 1,3-bis-O(hexadecyl)glycerol, geranyloxyhexyanol, hexadecylglycerol, borneol, menthol, 1,3-propanediol, heptadecyl group, palmitic acid, myristic acid, O3-(oleoyl)lithocholic acid, O3-(oleoyl)cholenic acid, ibuprofen, naproxen, dimethoxytrityl, or phenoxazine.

In certain embodiments, more than one lipophilic moiety can be incorporated into the double-strand RNAi agent, particularly when the lipophilic moiety has a low lipophilicity or hydrophobicity. In some embodiments, two or more lipophilic moieties are incorporated into the same strand of the double-strand RNAi agent. In some embodiments, each strand of the double-strand RNAi agent has one or more lipophilic moieties incorporated. In some embodiments, two or more lipophilic moieties are incorporated into the same position (i.e., the same nucleobase, same sugar moiety, or same internucleosidic linkage) of the double-strand RNAi agent. This can be achieved by, *e.g.*, conjugating the two or more lipophilic moieties via a carrier, or conjugating the two or more lipophilic moieties via a branched linker, or conjugating the two or more lipophilic moieties via one or more linkers, with one or more linkers linking the lipophilic moieties consecutively.

The lipophilic moiety may be conjugated to the RNAi agent via a direct attachment to the ribosugar of the RNAi agent. Alternatively, the lipophilic moiety may be conjugated to the double-strand RNAi agent via a linker or a carrier.

In certain embodiments, the lipophilic moiety may be conjugated to the RNAi agent via one or more linkers (tethers).

In some embodiments, the lipophilic moiety is conjugated to the double-stranded RNAi agent via a linker containing an ether, thioether, urea, carbonate, amine, amide, maleimide-thioether, disulfide, phosphodiester, sulfonamide linkage, a product of a click reaction (*e.g.*, a triazole from the azide-alkyne cycloaddition), or carbamate.

*B. Lipid Conjugates*

In some embodiments, the ligand is a lipid or lipid-based molecule. Such a lipid or lipid-based molecule can typically bind a serum protein, such as human serum albumin (HSA). An HSA binding ligand allows for vascular distribution of the conjugate to a target tissue. For example, the target tissue can be the eye. Other molecules that can bind HSA can also be used as ligands. For example, neproxin or aspirin can be used. A lipid or lipid-based ligand can (a) increase resistance to degradation of the conjugate, (b) increase targeting or transport into a target cell or cell membrane, and/or (c) can be used to adjust binding to a serum protein, *e.g.*, HSA.

A lipid-based ligand can be used to modulate, *e.g.,* control (*e.g.,* inhibit) the binding of the conjugate to a target tissue. For example, a lipid or lipid-based ligand that binds to HSA more strongly will be less likely to be targeted to the kidney and therefore less likely to be cleared from the body. A lipid or lipid-based ligand that binds to HSA less strongly can be used to target the conjugate to the kidney.

In some embodiments, the lipid-based ligand binds HSA. For example, the ligand can bind HSA with a sufficient affinity such that distribution of the conjugate to a non-kidney tissue is enhanced. However, the affinity is typically not so strong that the HSA-ligand binding cannot be reversed.

In some embodiments, the lipid-based ligand binds HSA weakly or not at all, such that distribution of the conjugate to the kidney is enhanced. Other moieties that target to kidney cells can also be used in place of or in addition to the lipid-based ligand.

In another aspect, the ligand is a moiety, *e.g.,* a vitamin, which is taken up by a target cell, *e.g.,* a proliferating cell. These are particularly useful for treating disorders characterized by unwanted cell proliferation, *e.g.,* of the malignant or non-malignant type, *e.g.,* cancer cells. Exemplary vitamins include vitamin A, E, and K. Other exemplary vitamins include B vitamin, *e.g.,* folic acid, B12, riboflavin, biotin, pyridoxal or other vitamins or nutrients taken up by cancer cells. Also included are HSA and low-density lipoprotein (LDL).

*C. Cell Permeation Agents*

In another aspect, the ligand is a cell-permeation agent, such as a helical cell-permeation agent. In some embodiments, the agent is amphipathic. An exemplary agent is a peptide such as tat or antennopedia. If the agent is a peptide, it can be modified, including a peptidylmimetic, invertomers, non-peptide or pseudo-peptide linkages, and use of

D-amino acids. The helical agent is typically an α-helical agent, and can have a lipophilic and a lipophobic phase.

The ligand can be a peptide or peptidomimetic. A peptidomimetic (also referred to herein as an oligopeptidomimetic) is a molecule capable of folding into a defined three-dimensional structure similar to a natural peptide. The attachment of peptide and peptidomimetics to iRNA agents can affect pharmacokinetic distribution of the iRNA, such as by enhancing cellular recognition and absorption. The peptide or peptidomimetic moiety can be about 5-50 amino acids long, *e.g.*, about 5, 10, 15, 20, 25, 30, 35, 40, 45, or 50 amino acids long.

A peptide or peptidomimetic can be, for example, a cell permeation peptide, cationic peptide, amphipathic peptide, or hydrophobic peptide (*e.g.*, consisting primarily of Tyr, Trp or Phe). The peptide moiety can be a dendrimer peptide, constrained peptide or crosslinked peptide. In another alternative, the peptide moiety can include a hydrophobic membrane translocation sequence (MTS). An exemplary hydrophobic MTS-containing peptide is RFGF having the amino acid sequence AAVALLPAVLLALLAP (SEQ ID NO: 3). An RFGF analogue (*e.g.*, amino acid sequence AALLPVLLAAP (SEQ ID NO: 4)) containing a hydrophobic MTS can also be a targeting moiety. The peptide moiety can be a "delivery" peptide, which can carry large polar molecules including peptides, oligonucleotides, and protein across cell membranes. For example, sequences from the HIV Tat protein (GRKKRRQRRRPPQ (SEQ ID NO: 5)) and the *Drosophila Antennapedia* protein (RQIKIWFQNRRMKWKK (SEQ ID NO: 6)) have been found to be capable of functioning as delivery peptides. A peptide or peptidomimetic can be encoded by a random sequence of DNA, such as a peptide identified from a phage-display library, or one-bead-one-compound (OBOC) combinatorial library (Lam et al., Nature, 354:82-84, 1991). Typically, the peptide or peptidomimetic tethered to a dsRNA agent via an incorporated monomer unit is a cell targeting peptide such as an arginine-glycine-aspartic acid (RGD)-peptide, or RGD mimic. A peptide moiety can range in length from about 5 amino acids to about 40 amino acids. The peptide moieties can have a structural modification, such as to increase stability or direct conformational properties. Any of the structural modifications described below can be utilized.

An RGD peptide for use in the compositions and methods of the disclosure may be linear or cyclic, and may be modified, *e.g.*, glycosylated or methylated, to facilitate targeting to a specific tissue(s). RGD-containing peptides and peptidomimetics may include D-amino acids, as well as synthetic RGD mimics. In addition to RGD, one can use other moieties that target the integrin ligand. In some embodiments, conjugates of this ligand target PECAM-1 or VEGF.

An RGD peptide moiety can be used to target a particular cell type, *e.g.,* an ocular cell, a tumor cell, such as an endothelial tumor cell or a breast cancer tumor cell (Zitzmann et al., Cancer Res., 62:5139-43, 2002). An RGD peptide can facilitate targeting of an dsRNA agent to tumors of a variety of other tissues, including the lung, kidney, spleen, or liver (Aoki et al., Cancer Gene Therapy 8:783-787, 2001). Typically, the RGD peptide will facilitate targeting of an iRNA agent to the eye or kidney. The RGD peptide can be linear or cyclic, and can be modified, *e.g.*, glycosylated or methylated to facilitate targeting to specific tissues. For example, a glycosylated RGD peptide can deliver a iRNA agent to a tumor cell expressing $\alpha_v\beta_3$ (Haubner et al., Jour. Nucl. Med., 42:326-336, 2001).

A "cell permeation peptide" is capable of permeating a cell, *e.g.,* a microbial cell, such as a bacterial or fungal cell, or a mammalian cell, such as a human cell. A microbial cell-permeating peptide can be, for example, an α-helical linear peptide (*e.g.*, LL-37 or Ceropin P1), a disulfide bond-containing peptide (*e.g.,* α -defensin, β-defensin or bactenecin), or a peptide containing only one or two dominating amino acids (*e.g.*, PR-39 or indolicidin). A cell permeation peptide can also include a nuclear localization signal (NLS). For example, a cell permeation peptide can be a bipartite amphipathic peptide, such as MPG, which is derived from the fusion peptide domain of HIV-1 gp41 and the NLS of SV40 large Tantigen (Simeoni et al., Nucl. Acids Res. 31:2717-2724, 2003).

### *D. Carbohydrate Conjugates and Ligands*

In some embodiments of the compositions and methods of the disclosure, an iRNA oligonucleotide further comprises a carbohydrate. The carbohydrate conjugated iRNA are advantageous for the *in vivo* delivery of nucleic acids, as well as compositions suitable for *in vivo* therapeutic use, as described herein. As used herein, "carbohydrate" refers to a compound which is either a carbohydrate *per se* made up of one or more monosaccharide units having at least 6 carbon atoms (which can be linear, branched or cyclic) with an oxygen, nitrogen or sulfur atom bonded to each carbon atom; or a compound having as a part thereof a carbohydrate moiety made up of one or more monosaccharide units each having at least six carbon atoms (which can be linear, branched or cyclic), with an oxygen, nitrogen or sulfur atom bonded to each carbon atom. Representative carbohydrates include the sugars (mono-, di-, tri- and oligosaccharides containing from about 4, 5, 6, 7, 8, or 9 monosaccharide units), and polysaccharides such as starches, glycogen, cellulose and polysaccharide gums. Specific monosaccharides include C5 and above (*e.g.,* C5, C6, C7, or C8) sugars; di- and trisaccharides include sugars having two or three monosaccharide units (*e.g.,* C5, C6, C7, or C8).

In certain embodiments, the compositions and methods of the disclosure include a C16 ligand. In exemplary embodiments, the C16 ligand of the disclosure has the following structure (exemplified here below for a uracil base, yet attachment of the C16 ligand is contemplated for a nucleotide presenting any base (C, G, A, etc.) or possessing any other modification as presented herein, provided that 2' ribo attachment is preserved) and is attached at the 2' position of the ribo within a residue that is so modified:

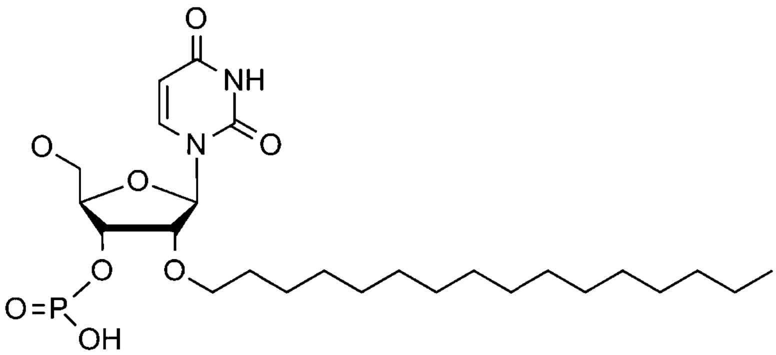

Chemical Formula: $C_{25}H_{43}N_2O_8P$
Exact Mass: 530.2757
Molecular Weight: 530.5913

As shown above, a C16 ligand-modified residue presents a straight chain alkyl at the 2'-ribo position of an exemplary residue (here, a Uracil) that is so modified.

In some embodiments, a carbohydrate conjugate of a RNAi agent of the instant disclosure further comprises one or more additional ligands as described above, such as, but not limited to, a PK modulator or a cell permeation peptide.

Additional carbohydrate conjugates (and linkers) suitable for use in the present disclosure include those described in WO 2014/179620 and WO 2014/179627, the entire contents of each of which are incorporated herein by reference.

In certain embodiments, the compositions and methods of the disclosure include a vinyl phosponate (VP) modification of an RNAi agent as described herein. In exemplary embodiments, a vinyl phosphonate of the disclosure has the following structure:

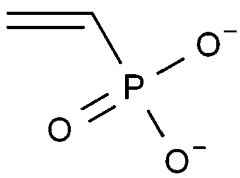

A vinyl phosponate of the instant disclosure may be attached to either the antisense or the sense strand of a dsRNA of the disclosure. In certain embodiments, a vinyl phosphonate of the instant disclosure is attached to the antisense strand of a dsRNA, optionally at the 5' end of the antisense strand of the dsRNA. The dsRNA agent can comprise a phosphorus-containing group at the 5'-end of the sense strand or antisense strand. The 5'-end phosphorus-containing group can be 5'-end phosphate (5'-P), 5'-end phosphorothioate (5'-PS), 5'-end phosphorodithioate (5'-PS2), 5'-end vinylphosphonate (5'-VP), 5'-end methylphosphonate (MePhos), or 5'-deoxy-5'-C-malonyl. When the 5'-end phosphorus-containing group is 5'-end vinylphosphonate (5'-VP), the 5'-VP can be either 5'-E-VP isomer (i.e., trans-vinylphosphonate,

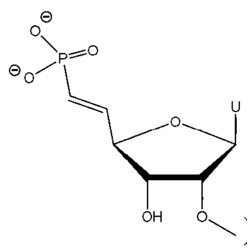

) ,5'-Z-VP isomer (i.e., cis-vinylphosphonate,

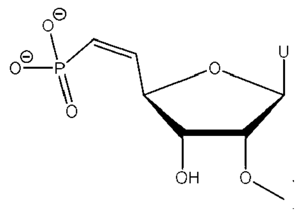

) or mixtures thereof.

Vinyl phosphate modifications are also contemplated for the compositions and methods of the instant disclosure. An exemplary vinyl phosphate structure is:

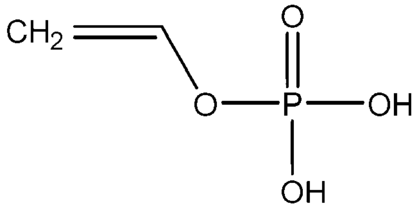

In some embodiments, a carbohydrate conjugate comprises a monosaccharide. In some embodiments, the monosaccharide is an N-acetylgalactosamine (GalNAc). GalNAc conjugates, which comprise one or more N-acetylgalactosamine (GalNAc) derivatives, are described, for example, in U.S. Patent No. 8,106,022, the entire content of which is hereby incorporated herein by reference. In some embodiments, the GalNAc conjugate serves as a ligand that targets the iRNA to particular cells. In some embodiments, the GalNAc conjugate targets the iRNA to liver cells, *e.g.,* by serving as a ligand for the asialoglycoprotein receptor of liver cells (*e.g.,* hepatocytes).

In some embodiments, the carbohydrate conjugate comprises one or more GalNAc derivatives. The GalNAc derivatives may be attached via a linker, *e.g.,* a bivalent or trivalent branched linker. In some embodiments the GalNAc conjugate is conjugated to the 3' end of the sense strand. In some embodiments, the GalNAc conjugate is conjugated to the iRNA agent *(e.g.,* to the 3' end of the sense strand) via a linker, *e.g.,* a linker as described herein.

In some embodiments, the GalNAc conjugate is

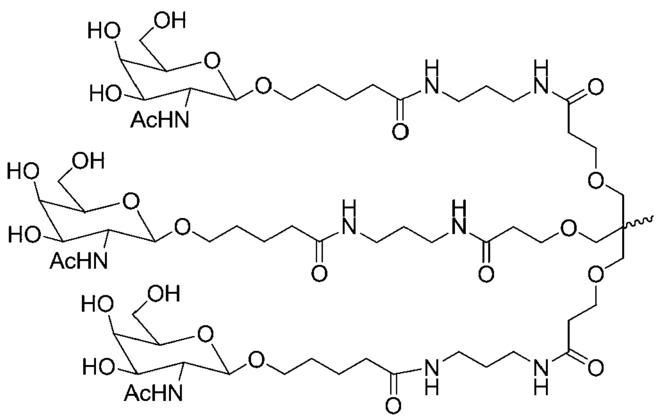

Formula II.

In some embodiments, the RNAi agent is attached to the carbohydrate conjugate via a linker as shown in the following schematic, wherein X is O or S:

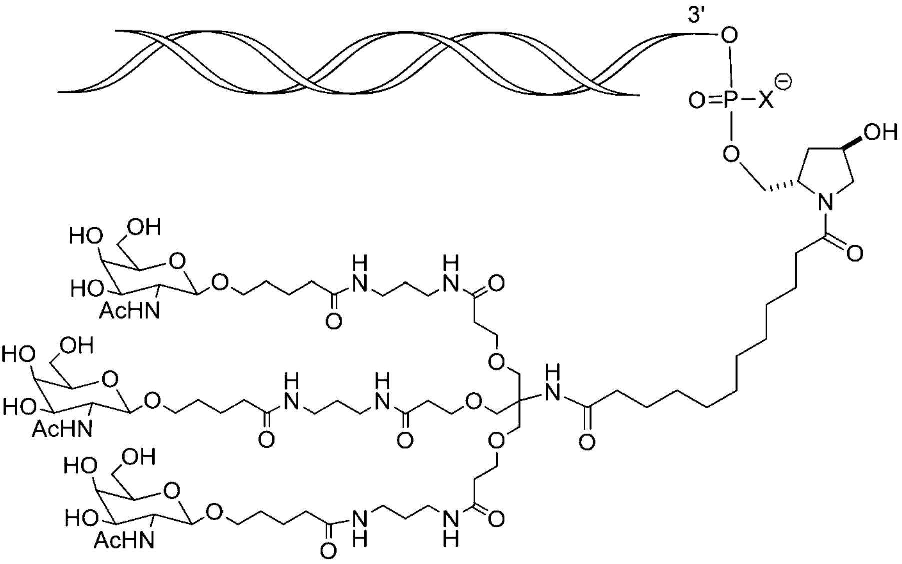

In some embodiments, the RNAi agent is conjugated to L96 as defined in Table 2 and shown below:

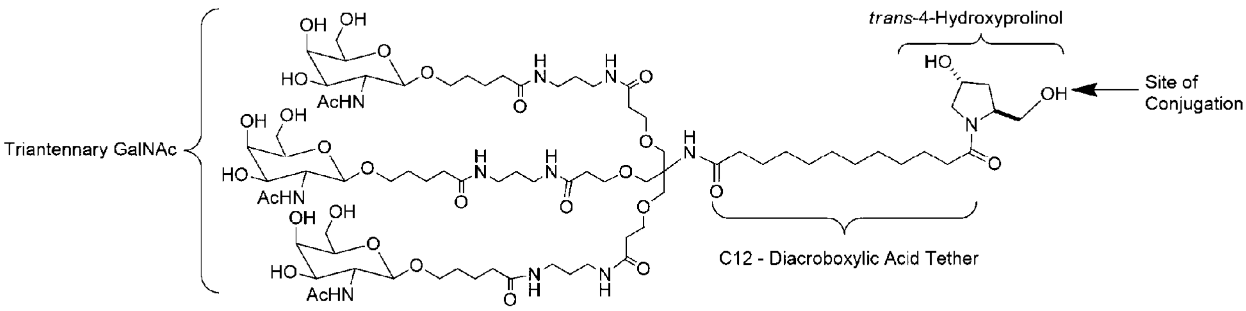


In some embodiments, a carbohydrate conjugate for use in the compositions and methods of the disclosure is selected from the group consisting of:

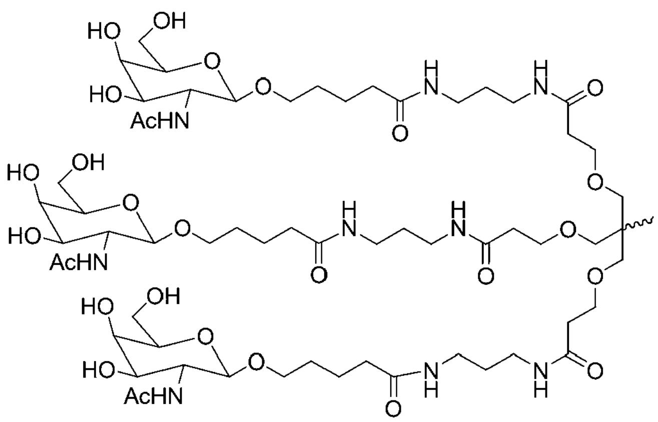

Formula II,

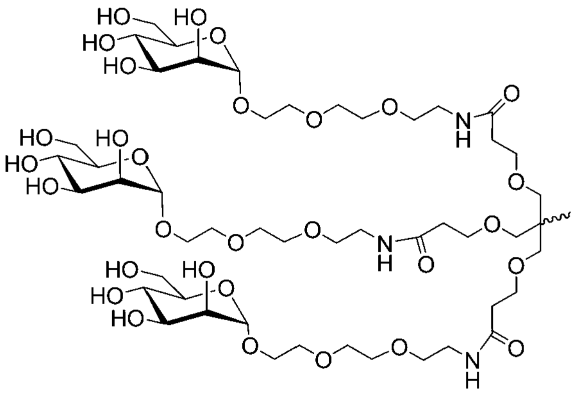

Formula III,

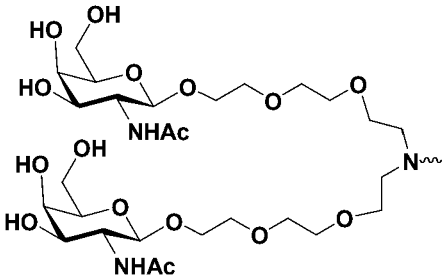

Formula IV,

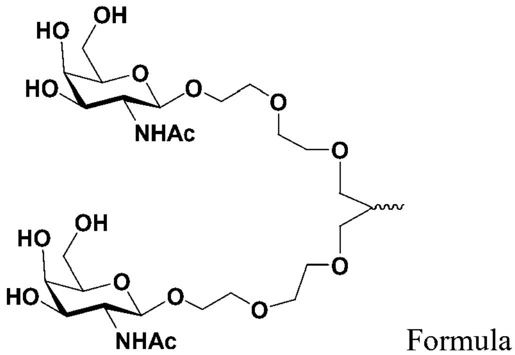

Formula V,

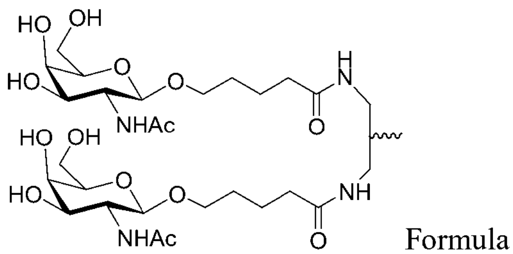

Formula VI,

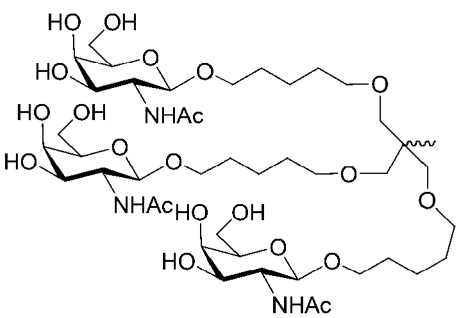

Formula VII,

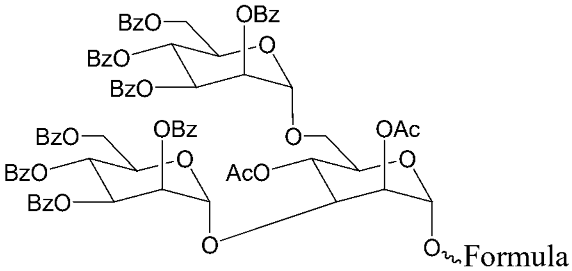

Formula VIII,

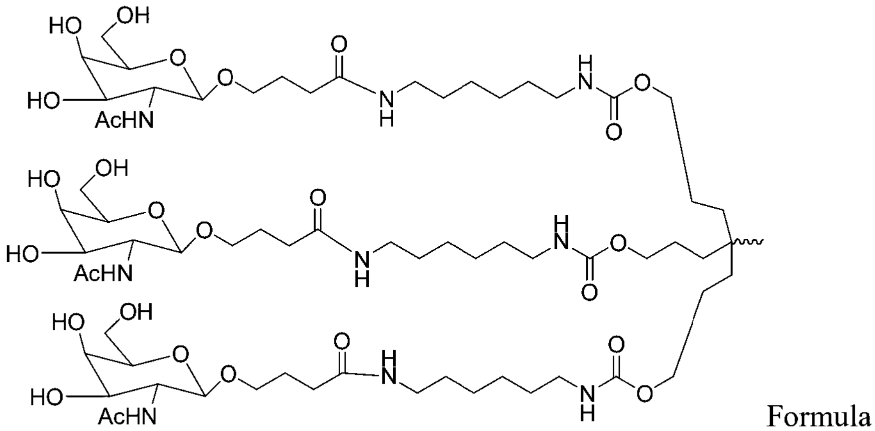

Formula IX,

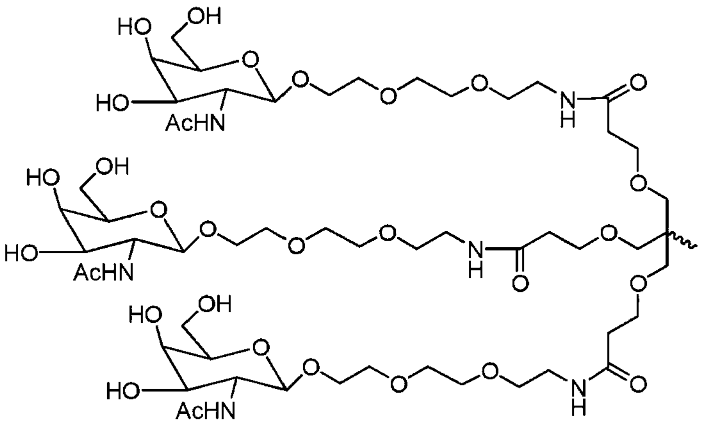

Formula X,

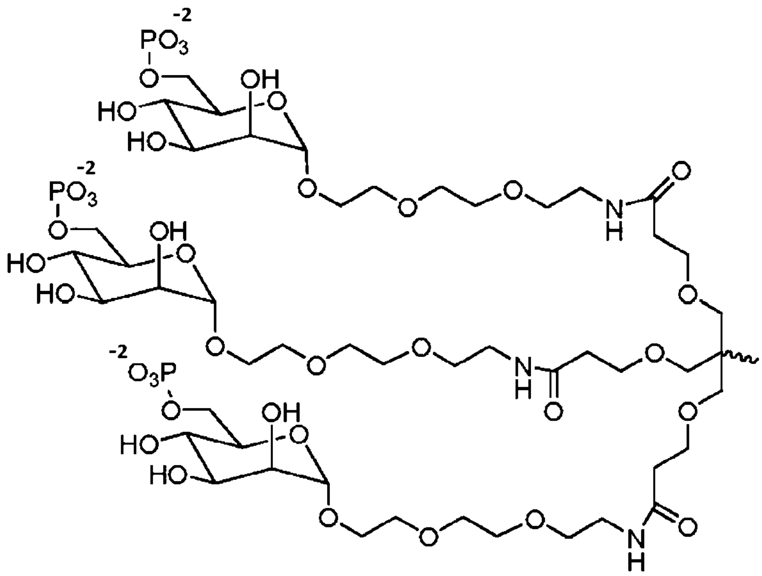

Formula XI,

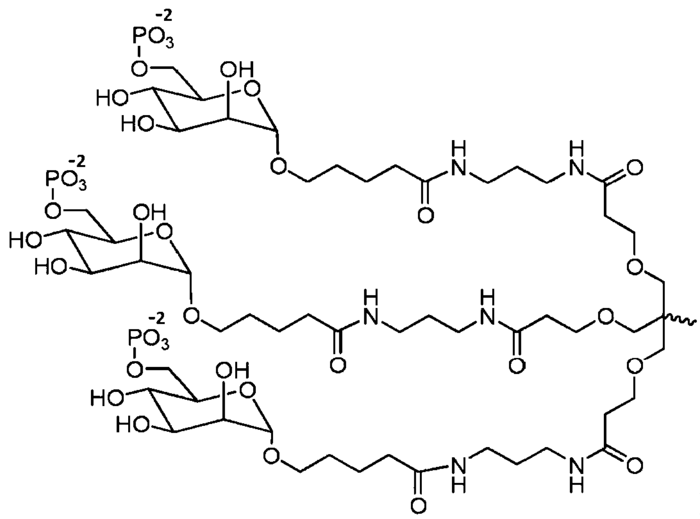

Formula XII,

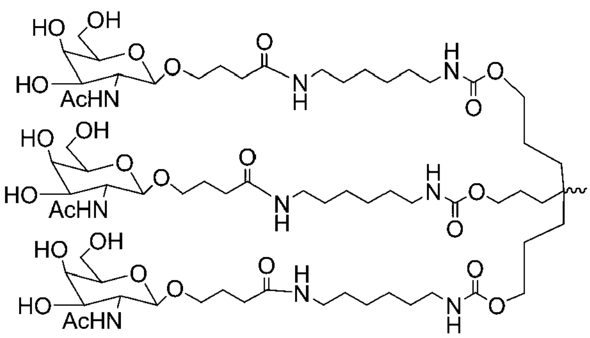

Formula XIII,

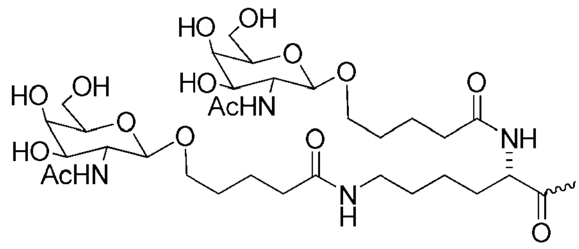
Formula XIV,

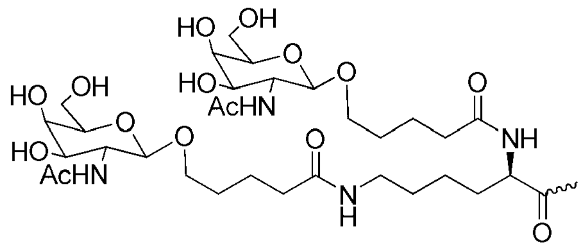
Formula XV,

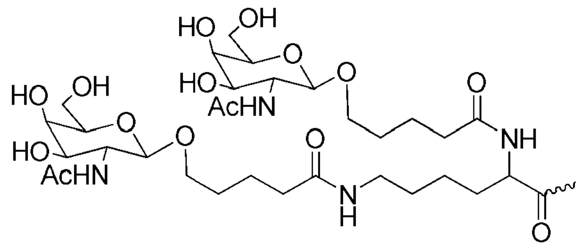
Formula XVI,

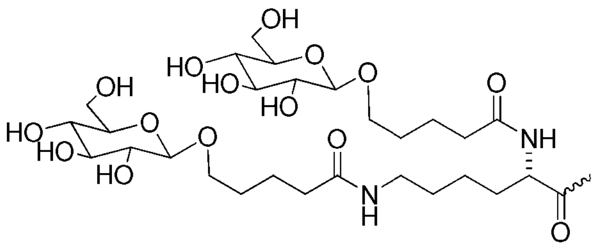
Formula XVII,

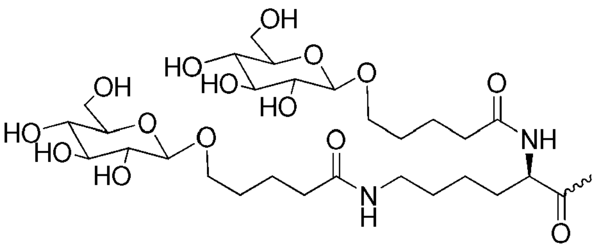
Formula XVIII,

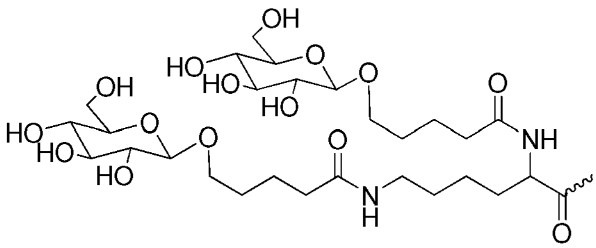
Formula XIX,

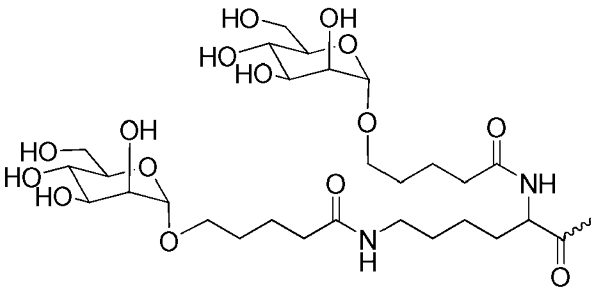

Formula XX,

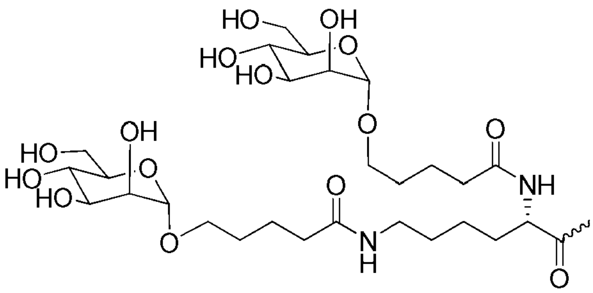

Formula XXI,

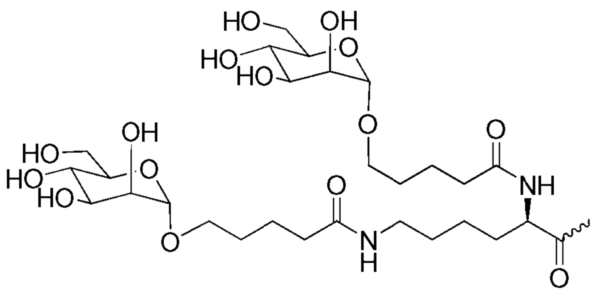

Formula XXII.

Another representative carbohydrate conjugate for use in the embodiments described herein includes, but is not limited to,

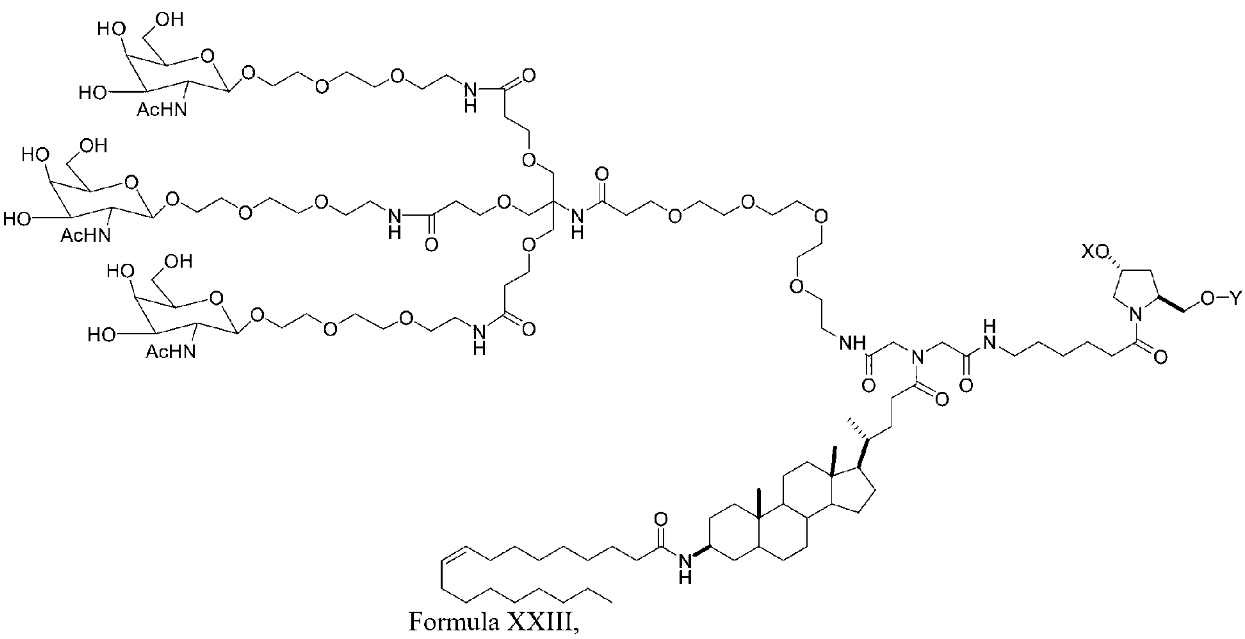

Formula XXIII,

when one of X or Y is an oligonucleotide, the other is a hydrogen.

In some embodiments, the carbohydrate conjugate further comprises one or more additional ligands as

described above, such as, but not limited to, a PK modulator and/or a cell permeation peptide.

In some embodiments, an iRNA of the disclosure is conjugated to a carbohydrate through a linker. Non-limiting examples of iRNA carbohydrate conjugates with linkers of the compositions and methods of the disclosure include, but are not limited to,

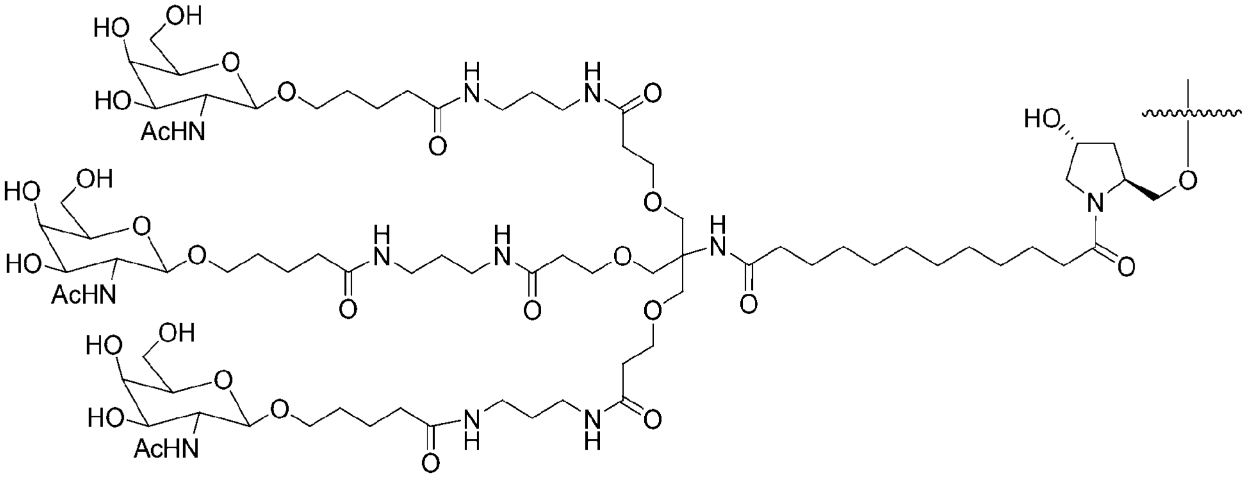

Formula XXIV,

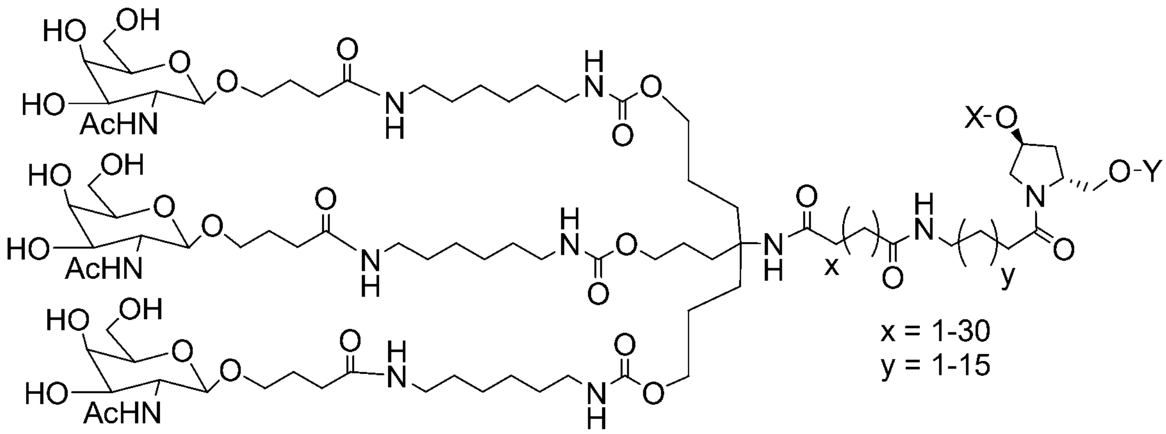

Formula XXV,

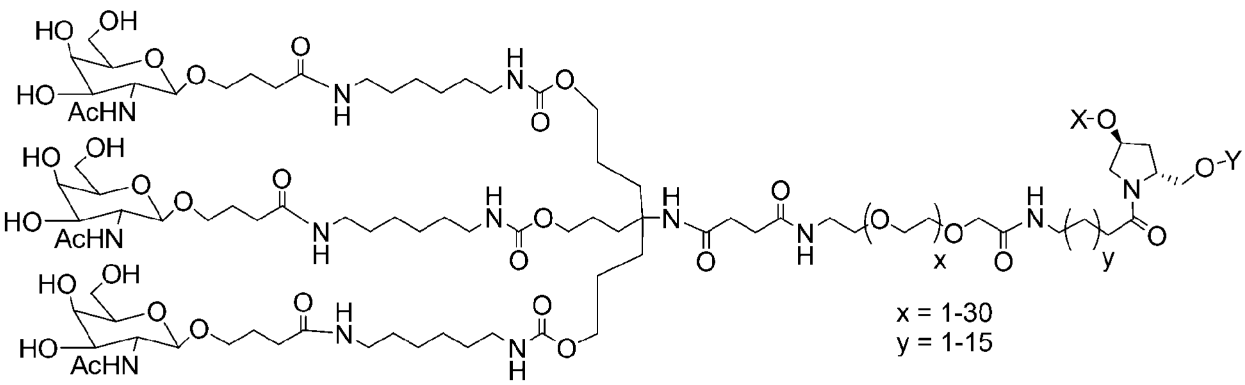

Formula XXVI,

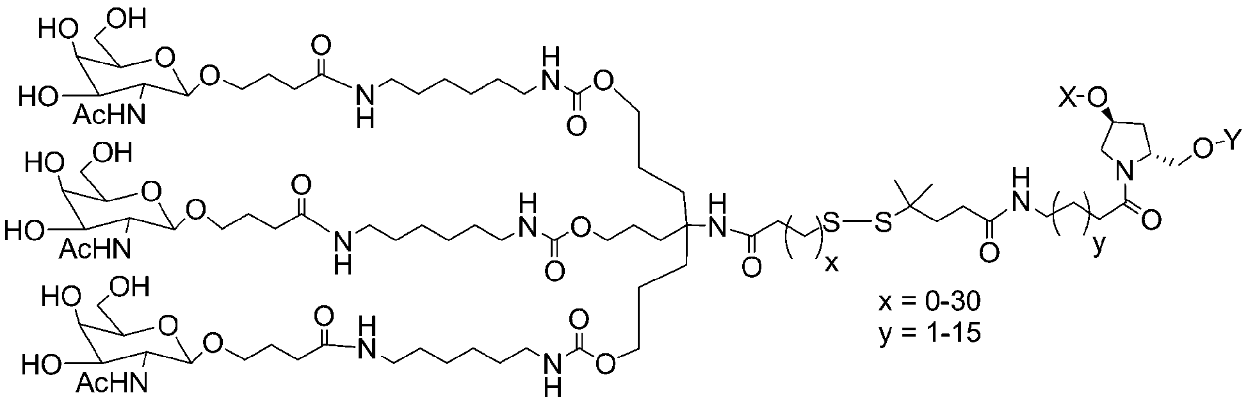

x = 0-30
y = 1-15

Formula XXVII,

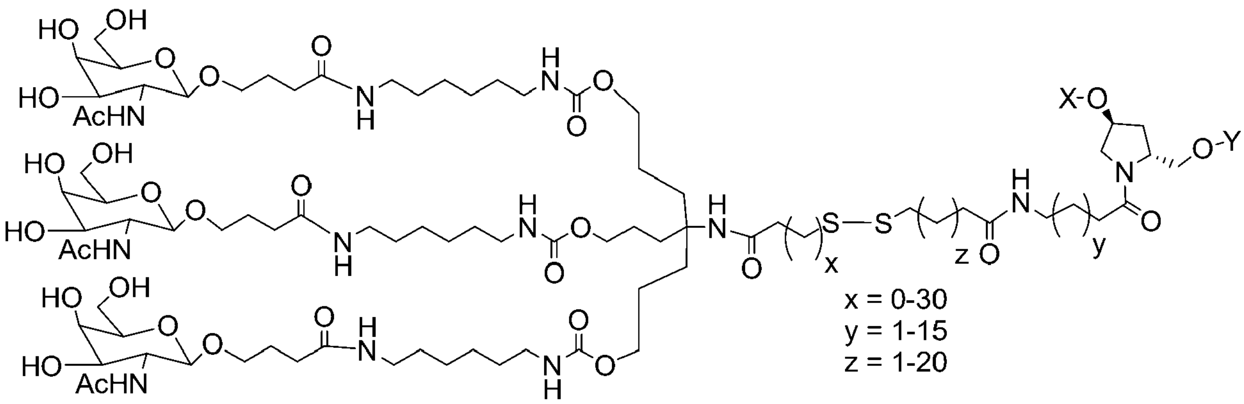

x = 0-30
y = 1-15
z = 1-20

Formula XXVIII,

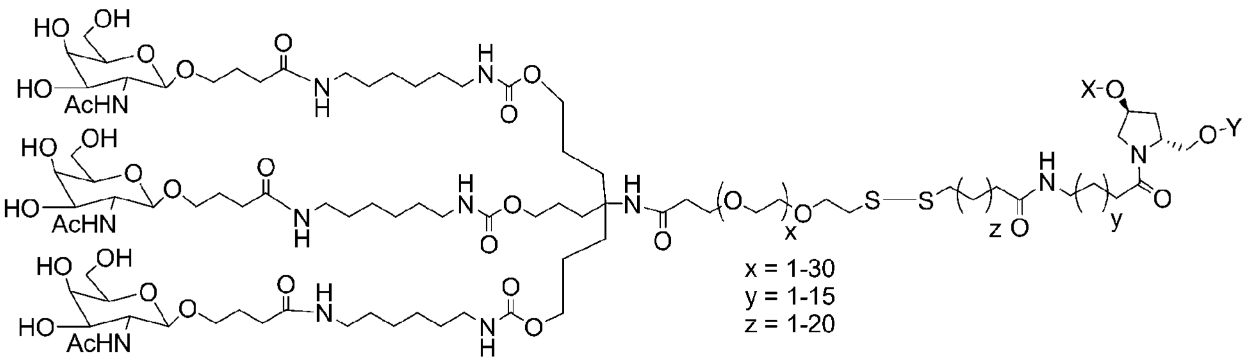

x = 1-30
y = 1-15
z = 1-20

Formula XXIX, and

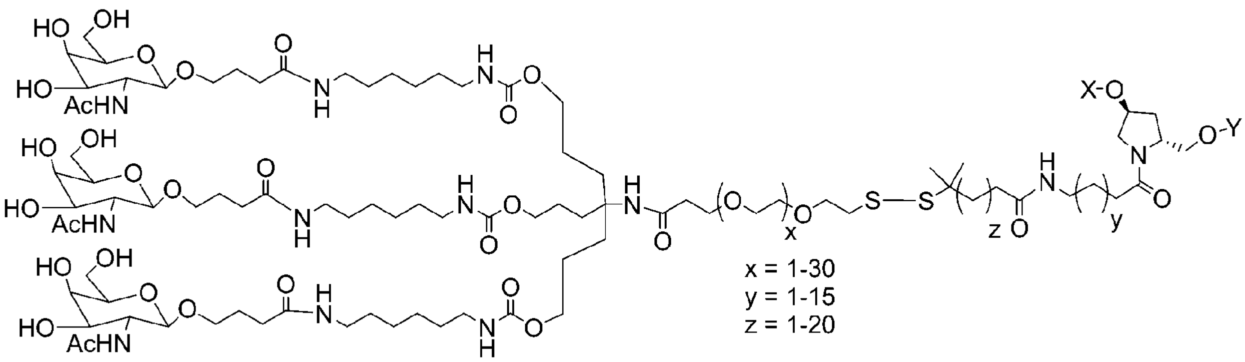

x = 1-30
y = 1-15
z = 1-20

Formula XXX,

when one of X or Y is an oligonucleotide, the other is a hydrogen.

*E. Thermally Destabilizing Modifications*

In certain embodiments, a dsRNA molecule can be optimized for RNA interference by incorporating thermally destabilizing modifications in the seed region of the antisense strand (*i.e.,* at positions 2-9 of the 5'-end of the antisense strand) to reduce or inhibit off-target gene silencing. It has been discovered that dsRNAs with an antisense strand comprising at least one thermally destabilizing modification of the duplex within the first 9 nucleotide positions, counting from the 5' end, of the antisense strand have reduced off-target gene silencing activity. Accordingly, in some embodiments, the antisense strand comprises at least one *(e.g.,* one, two, three, four, five, or more) thermally destabilizing modification of the duplex within the first 9 nucleotide positions of the 5' region of the antisense strand. In some embodiments, one or more thermally destabilizing modification(s) of the duplex is/are located in positions 2-9, or positions 4-8, from the 5'-end of the antisense strand. In some further embodiments, the thermally destabilizing modification(s) of the duplex is/are located at position 6, 7, or 8 from the 5'-end of the antisense strand. In still some further embodiments, the thermally destabilizing modification of the duplex is located at position 7 from the 5'-end of the antisense strand. The term "thermally destabilizing modification(s)" includes modification(s) that would result with a dsRNA with a lower overall melting temperature (Tm), such as a Tm with one, two, three, or four degrees lower than the Tm of the dsRNA without having such modification(s). In some embodiments, the thermally destabilizing modification of the duplex is located at position 2, 3, 4, 5, or 9 from the 5'-end of the antisense strand.

The thermally destabilizing modifications can include, but are not limited to, abasic modification; mismatch with the opposing nucleotide in the opposing strand; and sugar modification such as 2'-deoxy modification or acyclic nucleotide, *e.g.*, unlocked nucleic acids (UNA) or glycol nucleic acid (GNA).

Exemplified abasic modifications include, but are not limited to, the following:

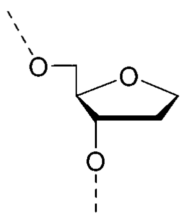
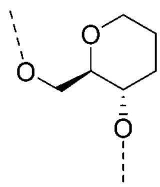
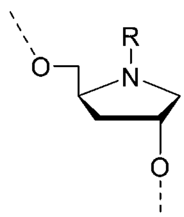
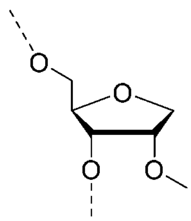

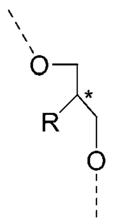
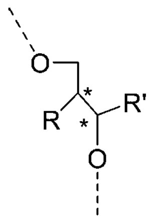
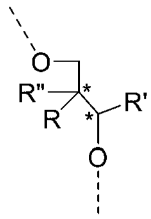

Wherein R = H, Me, Et or OMe; R' = H, Me, Et or OMe; R" = H, Me, Et or OMe

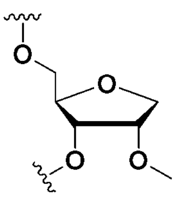

**Mod2 (2'-OMe Abasic Spacer)**

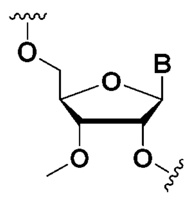

**Mod3 (3'-OMe)**

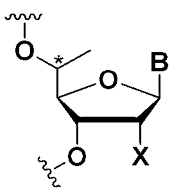

**Mod4 (5'-Me)**

**X = OMe, F**

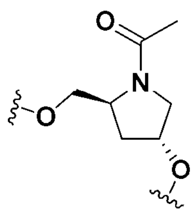

**Mod5 (Hyp-spacer)**

wherein B is a modified or unmodified nucleobase.

Exemplified sugar modifications include, but are not limited to the following:

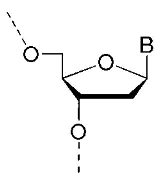

2'-deoxy

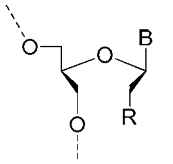

unlocked nucleic acid
R= H, OH, O-alkyl

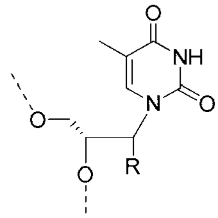

glycol nucleic acid
R= H, OH, O-alkyl

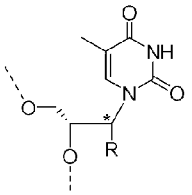

glycol nucleic acid
R= H, OH, O-alkyl

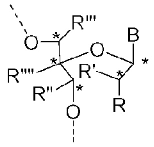

unlocked nucleic acid
R= H, OH, $CH_3$, $CH_2CH_3$, O-alkyl, $NH_2$, NHMe, $NMe_2$
R' = H, OH, $CH_3$, $CH_2CH_3$, O-alkyl, $NH_2$, NHMe, $NMe_2$
R'' = H, OH, $CH_3$, $CH_2CH_3$, O-alkyl, $NH_2$, NHMe, $NMe_2$
R''' = H, OH, $CH_3$, $CH_2CH_3$, O-alkyl, $NH_2$, NHMe, $NMe_2$
R'''' = H, OH, $CH_3$, $CH_2CH_3$, O-alkyl, $NH_2$, NHMe, $NMe_2$

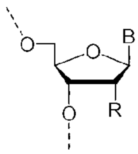

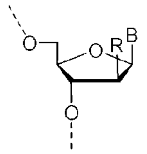

R = H, methyl, ethyl

wherein B is a modified or unmodified nucleobase.

In some embodiments the thermally destabilizing modification of the duplex is selected from the group consisting of:

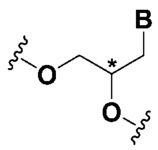,

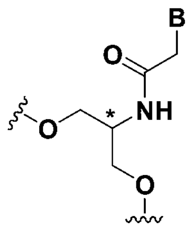,

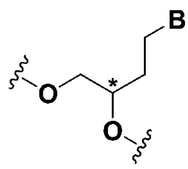,

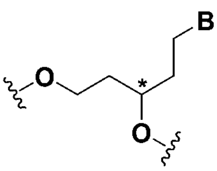,

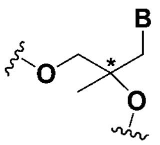, and

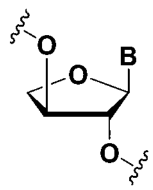

wherein B is a modified or unmodified nucleobase and the asterisk on each structure represents either *R, S* or *racemic.*

The term "acyclic nucleotide" refers to any nucleotide having an acyclic ribose sugar, for example, where any of bonds between the ribose carbons (*e.g.*, C1'-C2', C2'-C3', C3'-C4', C4'-O4', or C1'-O4') is absent or at least one of ribose carbons or oxygen (*e.g.,* C1', C2', C3', C4', or O4') are independently or in combination absent from the nucleotide. In some embodiments, acyclic nucleotide is

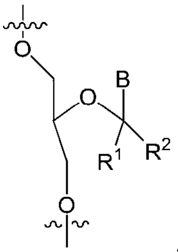,
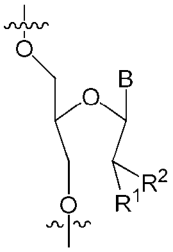,
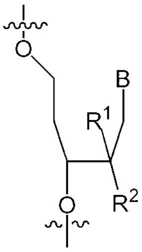,
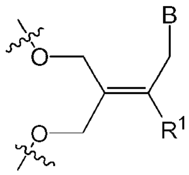

or

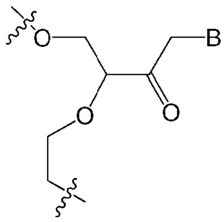,

wherein B is a modified or unmodified nucleobase, $R^1$ and $R^2$ independently are H, halogen, $OR_3$, or alkyl; and $R_3$ is H, alkyl, cycloalkyl, aryl, aralkyl, heteroaryl or sugar). The term "UNA" refers to unlocked acyclic nucleic acid, wherein any of the bonds of the sugar has been removed, forming an unlocked "sugar" residue. In one example, UNA also encompasses monomers with bonds between C1'-C4' being removed (i.e. the covalent carbon-oxygen-carbon bond between the C1' and C4' carbons). In another example, the C2'-C3' bond (i.e. the covalent carbon-carbon bond between the C2' and C3' carbons) of the sugar is removed (see Mikhailov et. al., Tetrahedron Letters, 26 (17): 2059 (1985); and Fluiter et al., Mol. Biosyst., 10: 1039 (2009), which are hereby incorporated by reference in their entirety). The acyclic derivative provides greater backbone flexibility without affecting the Watson-Crick pairings. The acyclic nucleotide can be linked via 2'-5' or 3'-5' linkage.

The term 'GNA' refers to glycol nucleic acid which is a polymer similar to DNA or RNA but differing in the composition of its "backbone" in that is composed of repeating glycerol units linked by phosphodiester bonds:

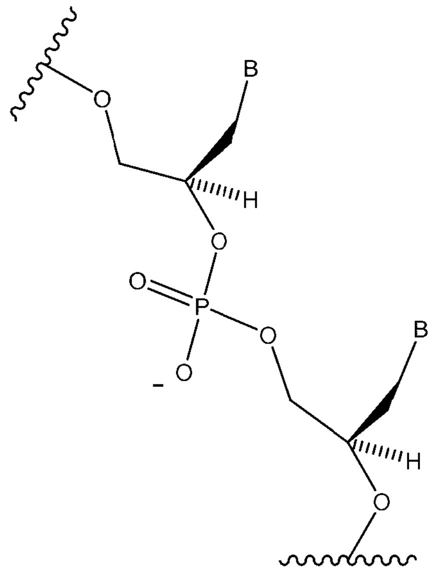

(R) - GNA .

The thermally destabilizing modification of the duplex can be mismatches (i.e., noncomplementary base pairs) between the thermally destabilizing nucleotide and the opposing nucleotide in the opposite strand within the dsRNA duplex. Exemplary mismatch base pairs include G:G, G:A, G:U, G:T, A:A, A:C, C:C, C:U, C:T, U:U, T:T, U:T, or a combination thereof. Other mismatch base pairings known in the art are also amenable to the present invention. A

mismatch can occur between nucleotides that are either naturally occurring nucleotides or modified nucleotides, i.e., the mismatch base pairing can occur between the nucleobases from respective nucleotides independent of the modifications on the ribose sugars of the nucleotides. In certain embodiments, the dsRNA molecule contains at least one nucleobase in the mismatch pairing that is a 2'-deoxy nucleobase; *e.g.*, the 2'-deoxy nucleobase is in the sense strand.

In some embodiments, the thermally destabilizing modification of the duplex in the seed region of the antisense strand includes nucleotides with impaired W-C H-bonding to complementary base on the target mRNA, such as:

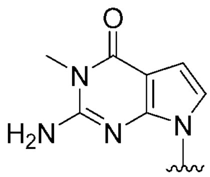
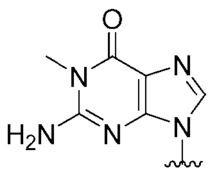
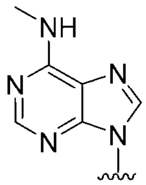
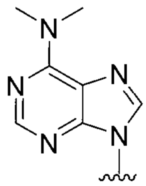
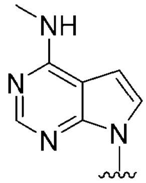
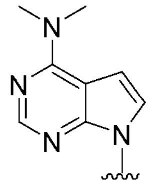
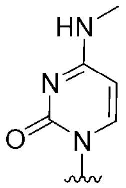
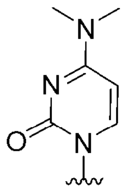
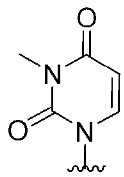
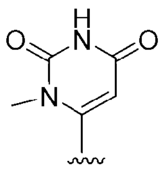
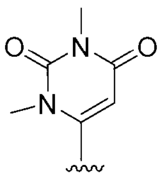
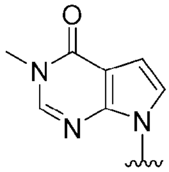
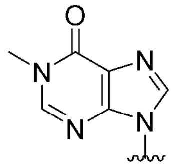
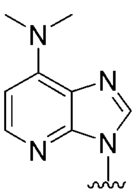
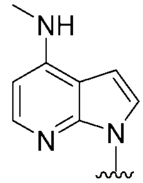
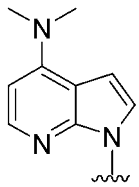
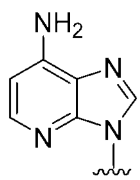
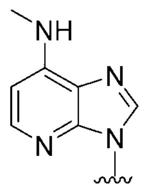
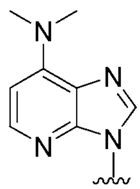

.

More examples of abasic nucleotide, acyclic nucleotide modifications (including UNA and GNA), and mismatch modifications have been described in detail in WO 2011/133876, which is herein incorporated by reference in its entirety.

The thermally destabilizing modifications may also include universal base with reduced or abolished capability to form hydrogen bonds with the opposing bases, and phosphate modifications.

In some embodiments, the thermally destabilizing modification of the duplex includes nucleotides with non-canonical bases such as, but not limited to, nucleobase modifications with impaired or completely abolished capability to form hydrogen bonds with bases in the opposite strand. These nucleobase modifications have been evaluated for destabilization of the central region of the dsRNA duplex as described in WO 2010/0011895, which is herein incorporated by reference in its entirety. Exemplary nucleobase modifications are:

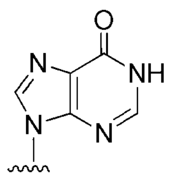
inosine

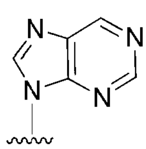
nebularine

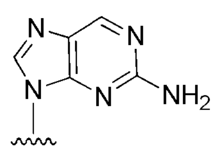
2-aminopurine

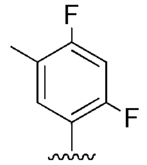
2,4-difluorotoluene

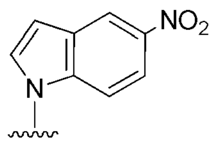
5-nitroindole

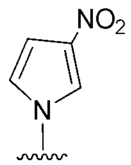
3-nitropyrrole

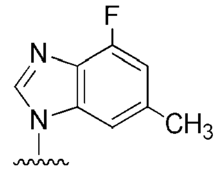
4-Fluoro-6-methylbenzimidazole

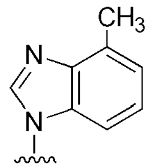
4-Methylbenzimidazole

In some embodiments, the thermally destabilizing modification of the duplex in the seed region of the antisense strand includes one or more α-nucleotide complementary to the base on the target mRNA, such as:

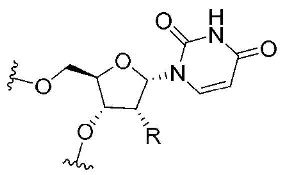
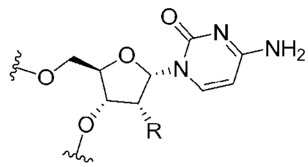
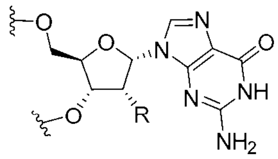
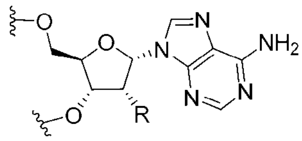

wherein R is H, OH, $OCH_3$, F, $NH_2$, NHMe, $NMe_2$ or O-alkyl.

Exemplary phosphate modifications known to decrease the thermal stability of dsRNA duplexes compared to natural phosphodiester linkages are:

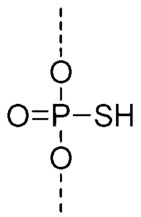
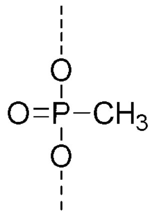
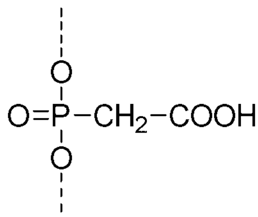
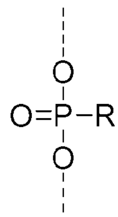
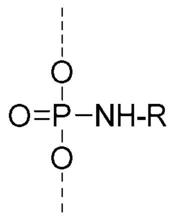
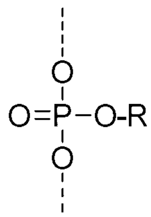

R = alkyl

The alkyl for the R group can be a $C_1$-$C_6$alkyl. Specific alkyls for the R group include, but are not limited to methyl, ethyl, propyl, isopropyl, butyl, pentyl and hexyl.

As the skilled artisan will recognize, in view of the functional role of nucleobases is defining specificity of a RNAi agent of the disclosure, while nucleobase modifications can be performed in the various manners as described herein, *e.g.*, to introduce destabilizing modifications into a RNAi agent of the disclosure, *e.g.*, for purpose of enhancing on-target effect relative to off-target effect, the range of modifications available and, in general, present upon RNAi agents of the disclosure tends to be much greater for non-nucleobase modifications, *e.g.*, modifications to sugar groups or phosphate backbones of polyribonucleotides. Such modifications are described in greater detail in other sections of the instant disclosure and are expressly contemplated for RNAi agents of the disclosure, either possessing native nucleobases or modified nucleobases as described above or elsewhere herein.

In addition to the antisense strand comprising a thermally destabilizing modification, the dsRNA can also comprise one or more stabilizing modifications. For example, the dsRNA can comprise at least two (*e.g.*, two, three, four, five, six, seven, eight, nine, ten, or more) stabilizing modifications. Without limitations, the stabilizing modifications all can be present in one strand. In some embodiments, both the sense and the antisense strands comprise at least two stabilizing modifications. The stabilizing modification can occur on any nucleotide of the sense strand or antisense strand. For instance, the stabilizing modification can occur on every nucleotide on the sense strand or antisense strand; each stabilizing modification can occur in an alternating pattern on the sense strand or antisense strand; or the sense strand or antisense strand comprises both stabilizing modification in an alternating pattern. The alternating pattern of the stabilizing modifications on the sense strand may be the same or different from the antisense strand, and the alternating pattern of the stabilizing modifications on the sense strand can have a shift relative to the alternating pattern of the stabilizing modifications on the antisense strand.

In some embodiments, the antisense strand comprises at least two (*e.g.*, two, three, four, five, six, seven, eight, nine, ten, or more) stabilizing modifications. Without limitations, a stabilizing modification in the antisense strand can be present at any positions.

In some embodiments, the antisense strand comprises stabilizing modifications at positions 2, 6, 8, 9, 14, and 16 from the 5'-end. In some other embodiments, the antisense strand comprises stabilizing modifications at positions 2, 6, 14,

and 16 from the 5'-end. In still some other embodiments, the antisense strand comprises stabilizing modifications at positions 2, 14, and 16 from the 5'-end.

In some embodiments, the antisense strand comprises at least one stabilizing modification adjacent to the destabilizing modification. For example, the stabilizing modification can be the nucleotide at the 5'-end or the 3'-end of the destabilizing modification, *i.e.,* at position -1 or +1 from the position of the destabilizing modification. In some embodiments, the antisense strand comprises a stabilizing modification at each of the 5'-end and the 3'-end of the destabilizing modification, *i.e.,* positions -1 and +1 from the position of the destabilizing modification.

In some embodiments, the antisense strand comprises at least two stabilizing modifications at the 3'-end of the destabilizing modification, *i.e.*, at positions +1 and +2 from the position of the destabilizing modification.

In some embodiments, the sense strand comprises at least two (*e.g.,* two, three, four, five, six, seven, eight, nine, ten or more) stabilizing modifications. Without limitations, a stabilizing modification in the sense strand can be present at any positions. In some embodiments, the sense strand comprises stabilizing modifications at positions 7, 10, and 11 from the 5'-end. In some other embodiments, the sense strand comprises stabilizing modifications at positions 7, 9, 10, and 11 from the 5'-end. In some embodiments, the sense strand comprises stabilizing modifications at positions opposite or complimentary to positions 11, 12, and 15 of the antisense strand, counting from the 5'-end of the antisense strand. In some other embodiments, the sense strand comprises stabilizing modifications at positions opposite or complimentary to positions 11, 12, 13, and 15 of the antisense strand, counting from the 5'-end of the antisense strand. In some embodiments, the sense strand comprises a block of two, three, or four stabilizing modifications.

In some embodiments, the sense strand does not comprise a stabilizing modification in position opposite or complimentary to the thermally destabilizing modification of the duplex in the antisense strand.

Exemplary thermally stabilizing modifications include, but are not limited to, 2'-fluoro modifications. Other thermally stabilizing modifications include, but are not limited to, LNA.

In some embodiments, the dsRNA of the disclosure comprises at least four (e.g., four, five, six, seven, eight, nine, ten, or more) 2'-fluoro nucleotides. Without limitations, the 2'-fluoro nucleotides all can be present in one strand. In some embodiments, both the sense and the antisense strands comprise at least two 2'-fluoro nucleotides. The 2'-fluoro modification can occur on any nucleotide of the sense strand or antisense strand. For instance, the 2'-fluoro modification can occur on every nucleotide on the sense strand or antisense strand; each 2'-fluoro modification can occur in an alternating pattern on the sense strand or antisense strand; or the sense strand or antisense strand comprises both 2'-fluoro modifications in an alternating pattern. The alternating pattern of the 2'-fluoro modifications on the sense strand may be the same or different from the antisense strand, and the alternating pattern of the 2'-fluoro modifications on the sense strand can have a shift relative to the alternating pattern of the 2'-fluoro modifications on the antisense strand.

In some embodiments, the antisense strand comprises at least two (*e.g.,* two, three, four, five, six, seven, eight, nine, ten, or more) 2'-fluoro nucleotides. Without limitations, a 2'-fluoro modification in the antisense strand can be present at any positions. In some embodiments, the antisense comprises 2'-fluoro nucleotides at positions 2, 6, 8, 9, 14, and 16 from the 5'-end. In some other embodiments, the antisense comprises 2'-fluoro nucleotides at positions 2, 6, 14, and 16 from the 5'-end. In still some other embodiments, the antisense comprises 2'-fluoro nucleotides at positions 2, 14, and 16 from the 5'-end.

In some embodiments, the antisense strand comprises at least one 2'-fluoro nucleotide adjacent to the destabilizing modification. For example, the 2'-fluoro nucleotide can be the nucleotide at the 5'-end or the 3'-end of the destabilizing modification, *i.e.,* at position -1 or +1 from the position of the destabilizing modification. In some embodiments, the antisense strand comprises a 2'-fluoro nucleotide at each of the 5'-end and the 3'-end of the destabilizing modification, *i.e.,* positions -1 and +1 from the position of the destabilizing modification.

In some embodiments, the antisense strand comprises at least two 2'-fluoro nucleotides at the 3'-end of the destabilizing modification, *i.e.,* at positions +1 and +2 from the position of the destabilizing modification.

In some embodiments, the sense strand comprises at least two (*e.g.,* two, three, four, five, six, seven, eight, nine, ten, or more) 2'-fluoro nucleotides. Without limitations, a 2'-fluoro modification in the sense strand can be present at any positions. In some embodiments, the antisense comprises 2'-fluoro nucleotides at positions 7, 10, and 11 from the 5'-end. In some other embodiments, the sense strand comprises 2'-fluoro nucleotides at positions 7, 9, 10, and 11 from the 5'-end. In some embodiments, the sense strand comprises 2'-fluoro nucleotides at positions opposite or complimentary to positions 11, 12, and 15 of the antisense strand, counting from the 5'-end of the antisense strand. In some other embodiments, the sense strand comprises 2'-fluoro nucleotides at positions opposite or complimentary to positions 11, 12, 13, and 15 of the antisense strand, counting from the 5'-end of the antisense strand. In some embodiments, the sense strand comprises a block of two, three, or four 2'-fluoro nucleotides.

In some embodiments, the sense strand does not comprise a 2'-fluoro nucleotide in position opposite or complimentary to the thermally destabilizing modification of the duplex in the antisense strand.

In some embodiments, the dsRNA molecule of the disclosure comprises a 21 nucleotides (nt) sense strand and a 23 nucleotides (nt) antisense, wherein the antisense strand contains at least one thermally destabilizing nucleotide, where the at least one thermally destabilizing nucleotide occurs in the seed region of the antisense strand (*i.e.,* at position 2-9 of

the 5'-end of the antisense strand), wherein one end of the dsRNA is blunt, while the other end is comprises a 2 nt overhang, and wherein the dsRNA optionally further has at least one (*e.g.,* one, two, three, four, five, six, or all seven) of the following characteristics: (i) the antisense comprises 2, 3, 4, 5, or 6 2'-fluoro modifications; (ii) the antisense comprises 1, 2, 3, 4, or 5 phosphorothioate internucleotide linkages; (iii) the sense strand is conjugated with a ligand; (iv) the sense strand comprises 2, 3, 4, or 5 2'-fluoro modifications; (v) the sense strand comprises 1, 2, 3, 4, or 5 phosphorothioate internucleotide linkages; (vi) the dsRNA comprises at least four 2'-fluoro modifications; and (vii) the dsRNA comprises a blunt end at 5'-end of the antisense strand. In certain embodiments, the 2 nt overhang is at the 3'-end of the antisense strand.

In some embodiments, every nucleotide in the sense strand and antisense strand of the dsRNA molecule may be modified. Each nucleotide may be modified with the same or different modification which can include one or more alteration of one or both of the non-linking phosphate oxygens or of one or more of the linking phosphate oxygens; alteration of a constituent of the ribose sugar, *e.g.*, of the 2' hydroxyl on the ribose sugar; wholesale replacement of the phosphate moiety with "dephospho" linkers; modification or replacement of a naturally occurring base; and replacement or modification of the ribose-phosphate backbone.

As nucleic acids are polymers of subunits, many of the modifications occur at a position which is repeated within a nucleic acid, *e.g.,* a modification of a base, or a phosphate moiety, or a non-linking O of a phosphate moiety. In some cases, the modification will occur at all of the subject positions in the nucleic acid but in many cases it will not. By way of example, a modification may only occur at a 3' or 5' terminal position, may only occur in a terminal region, *e.g.,* at a position on a terminal nucleotide or in the last 2, 3, 4, 5, or 10 nucleotides of a strand. A modification may occur in a double strand region, a single strand region, or in both. A modification may occur only in the double strand region of an RNA or may only occur in a single strand region of an RNA. *E.g.,* a phosphorothioate modification at a non-linking O position may only occur at one or both termini, may only occur in a terminal region, *e.g.,* at a position on a terminal nucleotide or in the last 2, 3, 4, 5, or 10 nucleotides of a strand, or may occur in double strand and single strand regions, particularly at termini. The 5' end or ends can be phosphorylated.

It may be possible, *e.g.*, to enhance stability, to include particular bases in overhangs, or to include modified nucleotides or nucleotide surrogates, in single strand overhangs, *e.g.*, in a 5' or 3' overhang, or in both. *E.g.,* it can be desirable to include purine nucleotides in overhangs. In some embodiments all or some of the bases in a 3' or 5' overhang may be modified, *e.g.*, with a modification described herein. Modifications can include, *e.g.*, the use of modifications at the 2' position of the ribose sugar with modifications that are known in the art, *e.g.,* the use of deoxyribonucleotides, 2'-deoxy-2'-fluoro (2'-F) or 2'-O-methyl modified instead of the ribosugar of the nucleobase, and modifications in the phosphate group, *e.g.*, phosphorothioate modifications. Overhangs need not be homologous with the target sequence.

In some embodiments, each residue of the sense strand and antisense strand is independently modified with LNA, HNA, CeNA, 2'-methoxyethyl, 2'- O-methyl, 2'-O-allyl, 2'-C- allyl, 2'-deoxy, or 2'-fluoro. The strands can contain more than one modification. In some embodiments, each residue of the sense strand and antisense strand is independently modified with 2'-O-methyl or 2'-fluoro. It is to be understood that these modifications are in addition to the at least one thermally destabilizing modification of the duplex present in the antisense strand.

At least two different modifications are typically present on the sense strand and antisense strand. Those two modifications may be the 2'-deoxy, 2'- O-methyl, or 2'-fluoro modifications, acyclic nucleotides or others. In some embodiments, the sense strand and antisense strand each comprises two differently modified nucleotides selected from 2'-O-methyl or 2'-deoxy. In some embodiments, each residue of the sense strand and antisense strand is independently modified with 2'-O-methyl nucleotide, 2'-deoxy nucleotide, 2'-deoxy-2'-fluoro nucleotide, 2'-O-N-methylacetamido (2'-O-NMA) nucleotide, a 2'-O-dimethylaminoethoxyethyl (2'-O-DMAEOE) nucleotide, 2'-O-aminopropyl (2'-O-AP) nucleotide, or 2'-ara-F nucleotide. Again, it is to be understood that these modifications are in addition to the at least one thermally destabilizing modification of the duplex present in the antisense strand.

In some embodiments, the dsRNA molecule of the disclosure comprises modifications of an alternating pattern, particular in the B1, B2, B3, B1', B2', B3', B4' regions. The term "alternating motif" or "alternative pattern" as used herein refers to a motif having one or more modifications, each modification occurring on alternating nucleotides of one strand. The alternating nucleotide may refer to one per every other nucleotide or one per every three nucleotides, or a similar pattern. For example, if A, B and C each represent one type of modification to the nucleotide, the alternating motif can be "ABABABABABAB...," "AABBAABBAABB...," "AABAABAABAAB...," "AAABAAABAAAB...," "AAABBBAAABBB...," or "ABCABCABCABC...," etc.

The type of modifications contained in the alternating motif may be the same or different. For example, if A, B, C, D each represent one type of modification on the nucleotide, the alternating pattern, i.e., modifications on every other nucleotide, may be the same, but each of the sense strand or antisense strand can be selected from several possibilities of modifications within the alternating motif such as "ABABAB...", "ACACAC..." "BDBDBD..." or "CDCDCD...," etc.

In some embodiments, the dsRNA molecule of the disclosure comprises the modification pattern for the alternating motif on the sense strand relative to the modification pattern for the alternating motif on the antisense strand is shifted. The shift may be such that the modified group of nucleotides of the sense strand corresponds to a differently

modified group of nucleotides of the antisense strand and vice versa. For example, the sense strand when paired with the antisense strand in the dsRNA duplex, the alternating motif in the sense strand may start with "ABABAB" from 5'-3' of the strand and the alternating motif in the antisense strand may start with "BABABA" from 3'-5'of the strand within the duplex region. As another example, the alternating motif in the sense strand may start with "AABBAABB" from 5'-3' of the strand and the alternating motif in the antisense strand may start with "BBAABBAA" from 3'-5' of the strand within the duplex region, so that there is a complete or partial shift of the modification patterns between the sense strand and the antisense strand.

The dsRNA molecule of the disclosure may further comprise at least one phosphorothioate or methylphosphonate internucleotide linkage. The phosphorothioate or methylphosphonate internucleotide linkage modification may occur on any nucleotide of the sense strand or antisense strand or both in any position of the strand. For instance, the internucleotide linkage modification may occur on every nucleotide on the sense strand or antisense strand; each internucleotide linkage modification may occur in an alternating pattern on the sense strand or antisense strand; or the sense strand or antisense strand comprises both internucleotide linkage modifications in an alternating pattern. The alternating pattern of the internucleotide linkage modification on the sense strand may be the same or different from the antisense strand, and the alternating pattern of the internucleotide linkage modification on the sense strand may have a shift relative to the alternating pattern of the internucleotide linkage modification on the antisense strand.

In some embodiments, the dsRNA molecule comprises the phosphorothioate or methylphosphonate internucleotide linkage modification in the overhang region. For example, the overhang region comprises two nucleotides having a phosphorothioate or methylphosphonate internucleotide linkage between the two nucleotides. Internucleotide linkage modifications also may be made to link the overhang nucleotides with the terminal paired nucleotides within duplex region. For example, at least 2, 3, 4, or all the overhang nucleotides may be linked through phosphorothioate or methylphosphonate internucleotide linkage, and optionally, there may be additional phosphorothioate or methylphosphonate internucleotide linkages linking the overhang nucleotide with a paired nucleotide that is next to the overhang nucleotide. For instance, there may be at least two phosphorothioate internucleotide linkages between the terminal three nucleotides, in which two of the three nucleotides are overhang nucleotides, and the third is a paired nucleotide next to the overhang nucleotide. In certain embodiments, these terminal three nucleotides may be at the 3'-end of the antisense strand.

In some embodiments, the sense strand of the dsRNA molecule comprises 1-10 blocks of two to ten phosphorothioate or methylphosphonate internucleotide linkages separated by 1, 2, 3, 4, 5, 6, 7, 8, 9, 10, 11, 12, 13, 14, 15, or 16 phosphate internucleotide linkages, wherein one of the phosphorothioate or methylphosphonate internucleotide linkages is placed at any position in the oligonucleotide sequence and the said sense strand is paired with an antisense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of two phosphorothioate or methylphosphonate internucleotide linkages separated by 1, 2, 3, 4, 5, 6, 7, 8, 9, 10, 11, 12, 13, 14, 15, 16, 17, or 18 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of three phosphorothioate or methylphosphonate internucleotide linkages separated by 1, 2, 3, 4, 5, 6, 7, 8, 9, 10, 11, 12, 13, 14, 15, or 16 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of four phosphorothioate or methylphosphonate internucleotide linkages separated by 1, 2, 3, 4, 5, 6, 7, 8, 9, 10, 11, 12, 13, or 14 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of five phosphorothioate or methylphosphonate internucleotide linkages separated by 1, 2, 3, 4, 5, 6, 7, 8, 9, 10, 11, or 12 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of six phosphorothioate or methylphosphonate internucleotide linkages separated by 1, 2, 3, 4, 5, 6, 7, 8, 9, or 10 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of seven phosphorothioate or methylphosphonate internucleotide linkages separated by 1, 2, 3, 4, 5, 6, 7, or 8 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of eight phosphorothioate or methylphosphonate internucleotide linkages separated by 1, 2, 3, 4, 5, or 6 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the antisense strand of the dsRNA molecule comprises two blocks of nine phosphorothioate or methylphosphonate internucleotide linkages separated by 1, 2, 3, or 4 phosphate internucleotide linkages, wherein one of the phosphorothioate or methylphosphonate internucleotide linkages is placed at any position in the oligonucleotide sequence and the said antisense strand is paired with a sense strand comprising any combination of phosphorothioate, methylphosphonate, and phosphate internucleotide linkages or an antisense strand comprising either phosphorothioate or methylphosphonate or phosphate linkage.

In some embodiments, the dsRNA molecule of the disclosure further comprises one or more phosphorothioate or methylphosphonate internucleotide linkage modification within positions 1-10 of the termini position(s) of the sense or antisense strand. For example, at least 2, 3, 4, 5, 6, 7, 8, 9, or 10 nucleotides may be linked through phosphorothioate or methylphosphonate internucleotide linkage at one end or both ends of the sense or antisense strand.

In some embodiments, the dsRNA molecule of the disclosure further comprises one or more phosphorothioate or methylphosphonate internucleotide linkage modification within positions 1-10 of the internal region of the duplex of each of the sense or antisense strand. For example, at least 2, 3, 4, 5, 6, 7, 8, 9, or 10 nucleotides may be linked through phosphorothioate methylphosphonate internucleotide linkage at position 8-16 of the duplex region counting from the 5'-end of the sense strand; the dsRNA molecule can optionally further comprise one or more phosphorothioate or methylphosphonate internucleotide linkage modification within positions 1-10 of the termini position(s).

In some embodiments, the dsRNA molecule of the disclosure further comprises one to five phosphorothioate or methylphosphonate internucleotide linkage modification(s) within position 1-5 and one to five phosphorothioate or methylphosphonate internucleotide linkage modification(s) within position 18-23 of the sense strand (counting from the 5'-end), and one to five phosphorothioate or methylphosphonate internucleotide linkage modification at positions 1 and 2 and one to five within positions 18-23 of the antisense strand (counting from the 5'-end).

In some embodiments, the dsRNA molecule of the disclosure further comprises one phosphorothioate internucleotide linkage modification within position 1-5 and one phosphorothioate or methylphosphonate internucleotide linkage modification within position 18-23 of the sense strand (counting from the 5'-end), and one phosphorothioate internucleotide linkage modification at positions 1 and 2 and two phosphorothioate or methylphosphonate internucleotide linkage modifications within positions 18-23 of the antisense strand (counting from the 5'-end).

In some embodiments, the dsRNA molecule of the disclosure further comprises two phosphorothioate internucleotide linkage modifications within position 1-5 and one phosphorothioate internucleotide linkage modification within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In some embodiments, the dsRNA molecule of the disclosure further comprises two phosphorothioate internucleotide linkage modifications within position 1-5 and two phosphorothioate internucleotide linkage modifications within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In some embodiments, the dsRNA molecule of the disclosure further comprises two phosphorothioate internucleotide linkage modifications within position 1-5 and two phosphorothioate internucleotide linkage modifications within position 18-23 of the sense strand (counting from the 5'-end), and one phosphorothioate internucleotide linkage modification at positions 1 and 2 and one phosphorothioate internucleotide linkage modification within positions 18-23 of the antisense strand (counting from the 5'-end).

In some embodiments, the dsRNA molecule of the disclosure further comprises one phosphorothioate internucleotide linkage modification within position 1-5 and one phosphorothioate internucleotide linkage modification within position 18-23 of the sense strand (counting from the 5'-end), and two phosphorothioate internucleotide linkage modifications at positions 1 and 2 and two phosphorothioate internucleotide linkage modifications within positions 18-23 of the antisense strand (counting from the 5'-end).

In some embodiments, the dsRNA molecule of the disclosure further comprises one phosphorothioate internucleotide linkage modification within position 1-5 and one within position 18-23 of the sense strand (counting from the 5'-end), and two phosphorothioate internucleotide linkage modification at positions 1 and 2 and one phosphorothioate internucleotide linkage modification within positions 18-23 of the antisense strand (counting from the 5'-end).

In some embodiments, the dsRNA molecule of the disclosure further comprises one phosphorothioate internucleotide linkage modification within position 1-5 (counting from the 5'-end) of the sense strand, and two phosphorothioate internucleotide linkage modifications at positions 1 and 2 and one phosphorothioate internucleotide linkage modification within positions 18-23 of the antisense strand (counting from the 5'-end).

In some embodiments, the dsRNA molecule of the disclosure further comprises two phosphorothioate internucleotide linkage modifications within position 1-5 (counting from the 5'-end) of the sense strand, and one phosphorothioate internucleotide linkage modification at positions 1 and 2 and two phosphorothioate internucleotide linkage modifications within positions 18-23 of the antisense strand (counting from the 5'-end).

In some embodiments, the dsRNA molecule of the disclosure further comprises two phosphorothioate internucleotide linkage modifications within position 1-5 and one within position 18-23 of the sense strand (counting from the 5'-end), and two phosphorothioate internucleotide linkage modifications at positions 1 and 2 and one phosphorothioate internucleotide linkage modification within positions 18-23 of the antisense strand (counting from the 5'-end).

In some embodiments, the dsRNA molecule of the disclosure further comprises two phosphorothioate internucleotide linkage modifications within position 1-5 and one phosphorothioate internucleotide linkage modification within position 18-23 of the sense strand (counting from the 5'-end), and two phosphorothioate internucleotide linkage modifications at positions 1 and 2 and two phosphorothioate internucleotide linkage modifications within positions 18-23 of the antisense strand (counting from the 5'-end).

In some embodiments, the dsRNA molecule of the disclosure further comprises two phosphorothioate internucleotide linkage modifications within position 1-5 and one phosphorothioate internucleotide linkage modification within position 18-23 of the sense strand (counting from the 5'-end), and one phosphorothioate internucleotide linkage modification at positions 1 and 2 and two phosphorothioate internucleotide linkage modifications within positions 18-23 of the antisense strand (counting from the 5'-end).

In some embodiments, the dsRNA molecule of the disclosure further comprises two phosphorothioate internucleotide linkage modifications at position 1 and 2, and two phosphorothioate internucleotide linkage modifications at position 20 and 21 of the sense strand (counting from the 5'-end), and one phosphorothioate internucleotide linkage modification at positions 1 and one at position 21 of the antisense strand (counting from the 5'-end).

In some embodiments, the dsRNA molecule of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions 1 and 2 and two phosphorothioate internucleotide linkage modifications at positions 20 and 21 the antisense strand (counting from the 5'-end).

In some embodiments, the dsRNA molecule of the disclosure further comprises two phosphorothioate internucleotide linkage modifications at position 1 and 2, and two phosphorothioate internucleotide linkage modifications at position 21 and 22 of the sense strand (counting from the 5'-end), and one phosphorothioate internucleotide linkage modification at positions 1 and one phosphorothioate internucleotide linkage modification at position 21 of the antisense strand (counting from the 5'-end).

In some embodiments, the dsRNA molecule of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions 1 and 2 and two phosphorothioate internucleotide linkage modifications at positions 21 and 22 the antisense strand (counting from the 5'-end).

In some embodiments, the dsRNA molecule of the disclosure further comprises two phosphorothioate internucleotide linkage modifications at position 1 and 2, and two phosphorothioate internucleotide linkage modifications at position 22 and 23 of the sense strand (counting from the 5'-end), and one phosphorothioate internucleotide linkage modification at positions 1 and one phosphorothioate internucleotide linkage modification at position 21 of the antisense strand (counting from the 5'-end).

In some embodiments, the dsRNA molecule of the disclosure further comprises one phosphorothioate internucleotide linkage modification at position 1, and one phosphorothioate internucleotide linkage modification at position 21 of the sense strand (counting from the 5'-end), and two phosphorothioate internucleotide linkage modifications at positions

1 and 2 and two phosphorothioate internucleotide linkage modifications at positions 23 and 23 the antisense strand (counting from the 5'-end).

In some embodiments, compound of the disclosure comprises a pattern of backbone chiral centers. In some embodiments, a common pattern of backbone chiral centers comprises at least 5 internucleotidic linkages in the Sp configuration. In some embodiments, a common pattern of backbone chiral centers comprises at least 6 internucleotidic linkages in the Sp configuration. In some embodiments, a common pattern of backbone chiral centers comprises at least 7 internucleotidic linkages in the Sp configuration. In some embodiments, a common pattern of backbone chiral centers comprises at least 8 internucleotidic linkages in the Sp configuration. In some embodiments, a common pattern of backbone chiral centers comprises at least 9 internucleotidic linkages in the Sp configuration. In some embodiments, a common pattern of backbone chiral centers comprises at least 10 internucleotidic linkages in the Sp configuration. In some embodiments, a common pattern of backbone chiral centers comprises at least 11 internucleotidic linkages in the Sp configuration. In some embodiments, a common pattern of backbone chiral centers comprises at least 12 internucleotidic linkages in the Sp configuration. In some embodiments, a common pattern of backbone chiral centers comprises at least 13 internucleotidic linkages in the Sp configuration. In some embodiments, a common pattern of backbone chiral centers comprises at least 14 internucleotidic linkages in the Sp configuration. In some embodiments, a common pattern of backbone chiral centers comprises at least 15 internucleotidic linkages in the Sp configuration. In some embodiments, a common pattern of backbone chiral centers comprises at least 16 internucleotidic linkages in the Sp configuration. In some embodiments, a common pattern of backbone chiral centers comprises at least 17 internucleotidic linkages in the Sp configuration. In some embodiments, a common pattern of backbone chiral centers comprises at least 18 internucleotidic linkages in the Sp configuration. In some embodiments, a common pattern of backbone chiral centers comprises at least 19 internucleotidic linkages in the Sp configuration. In some embodiments, a common pattern of backbone chiral centers comprises no more than 8 internucleotidic linkages in the Rp configuration. In some embodiments, a common pattern of backbone chiral centers comprises no more than 7 internucleotidic linkages in the Rp configuration. In some embodiments, a common pattern of backbone chiral centers comprises no more than 6 internucleotidic linkages in the Rp configuration. In some embodiments, a common pattern of backbone chiral centers comprises no more than 5 internucleotidic linkages in the Rp configuration. In some embodiments, a common pattern of backbone chiral centers comprises no more than 4 internucleotidic linkages in the Rp configuration. In some embodiments, a common pattern of backbone chiral centers comprises no more than 3 internucleotidic linkages in the Rp configuration. In some embodiments, a common pattern of backbone chiral centers comprises no more than 2 internucleotidic linkages in the Rp configuration. In some embodiments, a common pattern of backbone chiral centers comprises no more than 1 internucleotidic linkages in the Rp configuration. In some embodiments, a common pattern of backbone chiral centers comprises no more than 8 internucleotidic linkages which are not chiral (as a non-limiting example, a phosphodiester). In some embodiments, a common pattern of backbone chiral centers comprises no more than 7 internucleotidic linkages which are not chiral. In some embodiments, a common pattern of backbone chiral centers comprises no more than 6 internucleotidic linkages which are not chiral. In some embodiments, a common pattern of backbone chiral centers comprises no more than 5 internucleotidic linkages which are not chiral. In some embodiments, a common pattern of backbone chiral centers comprises no more than 4 internucleotidic linkages which are not chiral. In some embodiments, a common pattern of backbone chiral centers comprises no more than 3 internucleotidic linkages which are not chiral. In some embodiments, a common pattern of backbone chiral centers comprises no more than 2 internucleotidic linkages which are not chiral. In some embodiments, a common pattern of backbone chiral centers comprises no more than 1 internucleotidic linkages which are not chiral. In some embodiments, a common pattern of backbone chiral centers comprises at least 10 internucleotidic linkages in the Sp configuration, and no more than 8 internucleotidic linkages which are not chiral. In some embodiments, a common pattern of backbone chiral centers comprises at least 11 internucleotidic linkages in the Sp configuration, and no more than 7 internucleotidic linkages which are not chiral. In some embodiments, a common pattern of backbone chiral centers comprises at least 12 internucleotidic linkages in the Sp configuration, and no more than 6 internucleotidic linkages which are not chiral. In some embodiments, a common pattern of backbone chiral centers comprises at least 13 internucleotidic linkages in the Sp configuration, and no more than 6 internucleotidic linkages which are not chiral. In some embodiments, a common pattern of backbone chiral centers comprises at least 14 internucleotidic linkages in the Sp configuration, and no more than 5 internucleotidic linkages which are not chiral. In some embodiments, a common pattern of backbone chiral centers comprises at least 15 internucleotidic linkages in the Sp configuration, and no more than 4 internucleotidic linkages which are not chiral. In some embodiments, the internucleotidic linkages in the Sp configuration are optionally contiguous or not contiguous. In some embodiments, the internucleotidic linkages in the Rp configuration are optionally contiguous or not contiguous. In some embodiments, the internucleotidic linkages which are not chiral are optionally contiguous or not contiguous.

In some embodiments, compound of the disclosure comprises a block is a stereochemistry block. In some embodiments, a block is an Rp block in that each internucleotidic linkage of the block is Rp. In some embodiments, a 5'-block is an Rp block. In some embodiments, a 3'-block is an Rp block. In some embodiments, a block is an Sp block in that each internucleotidic linkage of the block is Sp. In some embodiments, a 5'-block is an Sp block. In some embodiments, a

3'-block is an Sp block. In some embodiments, provided oligonucleotides comprise both Rp and Sp blocks. In some embodiments, provided oligonucleotides comprise one or more Rp but no Sp blocks. In some embodiments, provided oligonucleotides comprise one or more Sp but no Rp blocks. In some embodiments, provided oligonucleotides comprise one or more PO blocks wherein each internucleotidic linkage in a natural phosphate linkage.

In some embodiments, compound of the disclosure comprises a 5'-block is an Sp block wherein each sugar moiety comprises a 2'-F modification. In some embodiments, a 5'-block is an Sp block wherein each of internucleotidic linkage is a modified internucleotidic linkage and each sugar moiety comprises a 2'-F modification. In some embodiments, a 5'-block is an Sp block wherein each of internucleotidic linkage is a phosphorothioate linkage and each sugar moiety comprises a 2'-F modification. In some embodiments, a 5'-block comprises 4 or more nucleoside units. In some embodiments, a 5'-block comprises 5 or more nucleoside units. In some embodiments, a 5'-block comprises 6 or more nucleoside units. In some embodiments, a 5'-block comprises 7 or more nucleoside units. In some embodiments, a 3'-block is an Sp block wherein each sugar moiety comprises a 2'-F modification. In some embodiments, a 3'-block is an Sp block wherein each of internucleotidic linkage is a modified internucleotidic linkage and each sugar moiety comprises a 2'-F modification. In some embodiments, a 3'-block is an Sp block wherein each of internucleotidic linkage is a phosphorothioate linkage and each sugar moiety comprises a 2'-F modification. In some embodiments, a 3'-block comprises 4 or more nucleoside units. In some embodiments, a 3'-block comprises 5 or more nucleoside units. In some embodiments, a 3'-block comprises 6 or more nucleoside units. In some embodiments, a 3'-block comprises 7 or more nucleoside units.

In some embodiments, compound of the disclosure comprises a type of nucleoside in a region or an oligonucleotide is followed by a specific type of internucleotidic linkage, *e.g.,* natural phosphate linkage, modified internucleotidic linkage, Rp chiral internucleotidic linkage, Sp chiral internucleotidic linkage, etc. In some embodiments, A is followed by Sp. In some embodiments, A is followed by Rp. In some embodiments, A is followed by natural phosphate linkage (PO). In some embodiments, U is followed by Sp. In some embodiments, U is followed by Rp. In some embodiments, U is followed by natural phosphate linkage (PO). In some embodiments, C is followed by Sp. In some embodiments, C is followed by Rp. In some embodiments, C is followed by natural phosphate linkage (PO). In some embodiments, G is followed by Sp. In some embodiments, G is followed by Rp. In some embodiments, G is followed by natural phosphate linkage (PO). In some embodiments, C and U are followed by Sp. In some embodiments, C and U are followed by Rp. In some embodiments, C and U are followed by natural phosphate linkage (PO). In some embodiments, A and G are followed by Sp. In some embodiments, A and G are followed by Rp.

In some embodiments, the dsRNA molecule of the disclosure comprises mismatch(es) with the target, within the duplex, or combinations thereof. The mismatch can occur in the overhang region or the duplex region. The base pair can be ranked on the basis of their propensity to promote dissociation or melting (e.g., on the free energy of association or dissociation of a particular pairing, the simplest approach is to examine the pairs on an individual pair basis, though next neighbor or similar analysis can also be used). In terms of promoting dissociation: A:U is preferred over G:C; G:U is preferred over G:C; and I:C is preferred over G:C (I=inosine). Mismatches, *e.g.*, non-canonical or other than canonical pairings (as described elsewhere herein) are preferred over canonical (A:T, A:U, G:C) pairings; and pairings which include a universal base are preferred over canonical pairings.

In some embodiments, the dsRNA molecule of the disclosure comprises at least one of the first 1, 2, 3, 4, or 5 base pairs within the duplex regions from the 5'- end of the antisense strand can be chosen independently from the group of: A:U, G:U, I:C, and mismatched pairs, *e.g.*, non-canonical or other than canonical pairings or pairings which include a universal base, to promote the dissociation of the antisense strand at the 5'-end of the duplex.

In some embodiments, the nucleotide at the 1 position within the duplex region from the 5'-end in the antisense strand is selected from the group consisting of A, dA, dU, U, and dT. Alternatively, at least one of the first 1, 2 or 3 base pair within the duplex region from the 5'- end of the antisense strand is an AU base pair. For example, the first base pair within the duplex region from the 5'- end of the antisense strand is an AU base pair.

It was found that introducing 4'-modified or 5'-modified nucleotide to the 3'-end of a phosphodiester (PO), phosphorothioate (PS), or phosphorodithioate (PS2) linkage of a dinucleotide at any position of single stranded or double stranded oligonucleotide can exert steric effect to the internucleotide linkage and, hence, protecting or stabilizing it against nucleases.

In some embodiments, 5'-modified nucleoside is introduced at the 3'-end of a dinucleotide at any position of single stranded or double stranded siRNA. For instance, a 5'-alkylated nucleoside may be introduced at the 3'-end of a dinucleotide at any position of single stranded or double stranded siRNA. The alkyl group at the 5' position of the ribose sugar can be racemic or chirally pure R or S isomer. An exemplary 5'-alkylated nucleoside is 5'-methyl nucleoside. The 5'-methyl can be either racemic or chirally pure R or S isomer.

In some embodiments, 4'-modified nucleoside is introduced at the 3'-end of a dinucleotide at any position of single stranded or double stranded siRNA. For instance, a 4'-alkylated nucleoside may be introduced at the 3'-end of a dinucleotide at any position of single stranded or double stranded siRNA. The alkyl group at the 4' position of the ribose sugar can be racemic or chirally pure *R* or *S* isomer. An exemplary 4'-alkylated nucleoside is 4'-methyl nucleoside. The 4'-methyl can be either racemic or chirally pure *R* or *S* isomer. Alternatively, a 4'-*O*-alkylated nucleoside may be introduced at

the 3'-end of a dinucleotide at any position of single stranded or double stranded siRNA. The 4'-*O*-alkyl of the ribose sugar can be racemic or chirally pure *R* or *S* isomer. An exemplary 4'-*O*-alkylated nucleoside is 4'-*O*-methyl nucleoside. The 4'-*O*-methyl can be either racemic or chirally pure *R* or *S* isomer.

In some embodiments, 5'-alkylated nucleoside is introduced at any position on the sense strand or antisense strand of a dsRNA, and such modification maintains or improves potency of the dsRNA. The 5'-alkyl can be either racemic or chirally pure *R* or *S* isomer. An exemplary 5'-alkylated nucleoside is 5'-methyl nucleoside. The 5'-methyl can be either racemic or chirally pure *R* or *S* isomer.

In some embodiments, 4'-alkylated nucleoside is introduced at any position on the sense strand or antisense strand of a dsRNA, and such modification maintains or improves potency of the dsRNA. The 4'-alkyl can be either racemic or chirally pure *R* or *S* isomer. An exemplary 4'-alkylated nucleoside is 4'-methyl nucleoside. The 4'-methyl can be either racemic or chirally pure *R* or *S* isomer.

In some embodiments, 4'-*O*-alkylated nucleoside is introduced at any position on the sense strand or antisense strand of a dsRNA, and such modification maintains or improves potency of the dsRNA. The 5'-alkyl can be either racemic or chirally pure *R* or *S* isomer. An exemplary 4'-*O*-alkylated nucleoside is 4'-*O*-methyl nucleoside. The 4'-*O*-methyl can be either racemic or chirally pure *R* or *S* isomer.

In some embodiments, the dsRNA molecule of the disclosure can comprise 2'-5' linkages (with 2'-H, 2'-OH, and 2'-OMe and with P=O or P=S). For example, the 2'-5' linkages modifications can be used to promote nuclease resistance or to inhibit binding of the sense to the antisense strand, or can be used at the 5' end of the sense strand to avoid sense strand activation by RISC.

In another embodiment, the dsRNA molecule of the disclosure can comprise L sugars (*e.g.,* L ribose, L-arabinose with 2'-H, 2'-OH and 2'-OMe). For example, these L sugars modifications can be used to promote nuclease resistance or to inhibit binding of the sense to the antisense strand, or can be used at the 5' end of the sense strand to avoid sense strand activation by RISC.

Various publications describe multimeric siRNA which can all be used with the dsRNA of the disclosure. Such publications include WO2007/091269, US 7858769, WO2010/141511, WO2007/117686, WO2009/014887, and WO2011/031520 which are hereby incorporated by their entirely.

In some embodiments dsRNA molecules of the disclosure are 5' phosphorylated or include a phosphoryl analog at the 5' prime terminus. 5'-phosphate modifications include those which are compatible with RISC mediated gene silencing. Suitable modifications include: 5'-monophosphate ($(HO)_2(O)P$-O-5'); 5'-diphosphate ($(HO)_2(O)P$-O-P(HO)(O)-O-5'); *5'*-triphosphate ($(HO)_2(O)P$-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; $(HO)_2(S)P$-O-5'); 5'-monodithiophosphate (phosphorodithioate; (HO)(HS)(S)P-O-5'), 5'-phosphorothiolate ((HO)2(O)P-S-5'); any additional combination of oxygen/sulfur replaced monophosphate, diphosphate and triphosphates (e.g. 5'-alpha-thiotriphosphate, 5'-gamma-thiotriphosphate, etc.), 5'-phosphoramidates ($(HO)_2(O)P$-NH-5', (HO)$(NH_2)$(O)P-O-5'), 5'-alkylphosphonates (R=alkyl=methyl, ethyl, isopropyl, propyl, etc., e.g. RP(OH)(O)-O-5'-, 5'-alkenylphosphonates (i.e. vinyl, substituted vinyl), $(OH)_2(O)P$-5'-CH2-), 5'-alkyletherphosphonates (R=alkylether=methoxymethyl (MeOCH2-), ethoxymethyl, etc., e.g. RP(OH)(O)-O-5'-). In one example, the modification can in placed in the antisense strand of a dsRNA molecule.

### *F. Linkers*

In some embodiments, the conjugate or ligand described herein can be attached to an iRNA oligonucleotide with various linkers that can be cleavable or non-cleavable.

Linkers typically comprise a direct bond or an atom such as oxygen or sulfur, a unit such as NR8, C(O), C(O)NH, SO, $SO_2$, $SO_2NH$ or a chain of atoms, such as, but not limited to, substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, which one or more methylenes can be interrupted or terminated by O, S, S(O), $SO_2$, N(R8), C(O), substituted or unsubstituted aryl, substituted or unsubstituted heteroaryl, substituted or unsubstituted heterocyclic; where R8 is hydrogen, acyl, aliphatic or substituted aliphatic. In some embodiments, the linker is between about 1-24 atoms, 2-24, 3-24, 4-24, 5-24, 6-24, 6-18, 7-18, 8-18 atoms, 7-17, 8-17, 6-16, 7-16, or 8-16 atoms.

In some embodiments, a dsRNA of the disclosure is conjugated to a bivalent or trivalent branched linker selected from the group of structures shown in any of formula (XXXI) - (XXXIV):

Formula XXXI

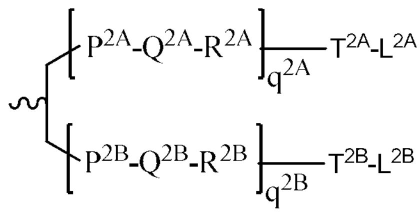

,

Formula XXXII

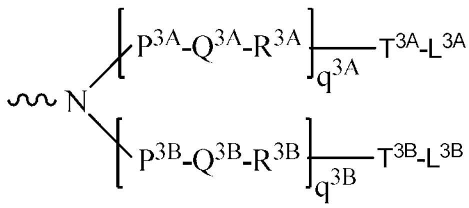

,

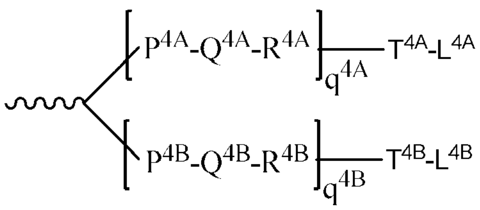

Formula XXXIII

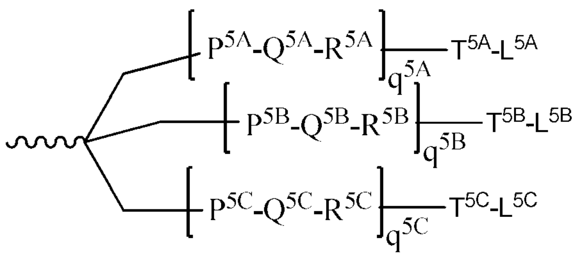

;

Formula XXXIV

wherein:

q2A, q2B, q3A, q3B, q4A, q4B, q5A, q5B and q5C represent independently for each occurrence 0-20 and wherein the repeating unit can be the same or different;

$P^{2A}$, $P^{2B}$, $P^{3A}$, $P^{3B}$, $P^{4A}$, $P^{4B}$, $P^{5A}$, $P^{5B}$, $P^{5C}$, $T^{2A}$, $T^{2B}$, $T^{3A}$, $T^{3B}$, $T^{4A}$, $T^{4B}$, $T^{4A}$, $T^{5B}$, $T^{5C}$ are each independently for each occurrence absent, CO, NH, O, S, OC(O), NHC(O), $CH_2$, $CH_2NH$ or $CH_2O$;

$Q^{2A}$, $Q^{2B}$, $Q^{3A}$, $Q^{3B}$, $Q^{4A}$, $Q^{4B}$, $Q^{5A}$, $Q^{5B}$, $Q^{5C}$ are independently for each occurrence absent, alkylene, substituted alkylene wherein one or more methylenes can be interrupted or terminated by one or more of O, S, S(O), $SO_2$, $N(R^N)$, C(R')=C(R"), C≡C or C(O);

$R^{2A}$, $R^{2B}$, $R^{3A}$, $R^{3B}$, $R^{4A}$, $R^{4B}$, $R^{5A}$, $R^{5B}$, $R^{5C}$ are each independently for each occurrence absent, NH, O, S, $CH_2$, C(O) O, C(O)NH, $NHCH(R^a)C(O)$, $-C(O)-CH(R^a)-NH-$, CO, CH=N-O,

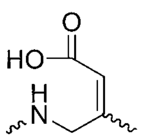, 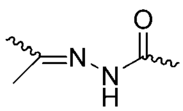, 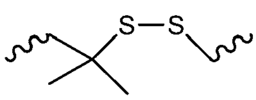, 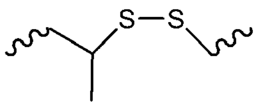,

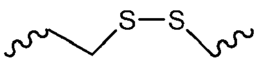

or heterocyclyl;

$L^{2A}$, $L^{2B}$, $L^{3A}$, $L^{3B}$, $L^{4A}$, $L^{4B}$, $L^{5A}$, $L^{5B}$ and $L^{5C}$ represent the ligand; *i.e.* each independently for each occurrence a monosaccharide (such as GalNAc), disaccharide, trisaccharide, tetrasaccharide, oligosaccharide, or polysaccharide; and $R^a$ is H or amino acid side chain. Trivalent conjugating GalNAc derivatives are particularly useful for use with RNAi agents for inhibiting the expression of a target gene, such as those of formula (XXXV):

Formula XXXV

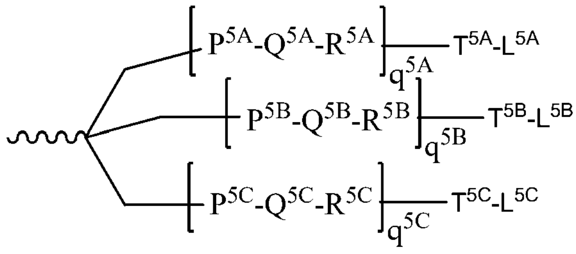

,

wherein $L^{5A}$, $L^{5B}$ and $L^{5C}$ represent a monosaccharide, such as GalNAc derivative.

Examples of suitable bivalent and trivalent branched linker groups conjugating GalNAc derivatives include, but are not limited to, the structures recited above as formulas II, VII, XI, X, and XIII.

A cleavable linking group is one which is sufficiently stable outside the cell, but which upon entry into a target cell is cleaved to release the two parts the linker is holding together. In a some embodiments, the cleavable linking group is cleaved at least about 10 times, 20, times, 30 times, 40 times, 50 times, 60 times, 70 times, 80 times, 90 times or more, or at least about 100 times faster in a target cell or under a first reference condition (which can, *e.g.,* be selected to mimic or represent intracellular conditions) than in the blood of a subject, or under a second reference condition (which can, *e.g.,* be selected to mimic or represent conditions found in the blood or serum).

Cleavable linking groups are susceptible to cleavage agents, *e.g.,* pH, redox potential or the presence of degradative molecules. Generally, cleavage agents are more prevalent or found at higher levels or activities inside cells than in serum or blood. Examples of such degradative agents include: redox agents which are selected for particular substrates or which have no substrate specificity, including, *e.g.*, oxidative or reductive enzymes or reductive agents such as mercaptans, present in cells, that can degrade a redox cleavable linking group by reduction; esterases; endosomes or agents that can create an acidic environment, *e.g.*, those that result in a pH of five or lower; enzymes that can hydrolyze or degrade an acid cleavable linking group by acting as a general acid, peptidases (which can be substrate specific), and phosphatases.

A cleavable linkage group, such as a disulfide bond can be susceptible to pH. The pH of human serum is 7.4, while the average intracellular pH is slightly lower, ranging from about 7.1-7.3. Endosomes have a more acidic pH, in the range of 5.5-6.0, and lysosomes have an even more acidic pH at around 5.0. Some linkers will have a cleavable linking group that is cleaved at a suitable pH, thereby releasing a cationic lipid from the ligand inside the cell, or into the desired compartment of the cell.

A linker can include a cleavable linking group that is cleavable by a particular enzyme. The type of cleavable linking group incorporated into a linker can depend on the cell to be targeted.

In general, the suitability of a candidate cleavable linking group can be evaluated by testing the ability of a degradative agent (or condition) to cleave the candidate linking group. It will also be desirable to also test the candidate cleavable linking group for the ability to resist cleavage in the blood or when in contact with other non-target tissue. Thus, one can determine the relative susceptibility to cleavage between a first and a second condition, where the first is selected to be indicative of cleavage in a target cell and the second is selected to be indicative of cleavage in other tissues or biological fluids, *e.g.,* blood or serum. The evaluations can be carried out in cell free systems, in cells, in cell culture, in organ or tissue culture, or in whole animals. It can be useful to make initial evaluations in cell-free or culture conditions and to confirm by further evaluations in whole animals. In some embodiments, useful candidate compounds are cleaved at least about 2, 4, 10, 20, 30, 40, 50, 60, 70, 80, 90, or about 100 times faster in the cell (or under *in vitro* conditions selected to mimic intracellular conditions) as compared to blood or serum (or under *in vitro* conditions selected to mimic extracellular conditions).

*i. Redox cleavable linking groups*

In some embodiments, a cleavable linking group is a redox cleavable linking group that is cleaved upon reduction or oxidation. An example of reductively cleavable linking group is a disulphide linking group (-S-S-). To determine if a candidate cleavable linking group is a suitable "reductively cleavable linking group," or for example is suitable for use with a particular iRNA moiety and particular targeting agent one can look to methods described herein. For example, a candidate can be evaluated by incubation with dithiothreitol (DTT), or other reducing agent using reagents know in the art, which mimic the rate of cleavage which would be observed in a cell, *e.g.,* a target cell. The candidates can also be evaluated

under conditions which are selected to mimic blood or serum conditions. In one, candidate compounds are cleaved by at most about 10% in the blood. In other embodiments, useful candidate compounds are degraded at least about 2, 4, 10, 20, 30, 40, 50, 60, 70, 80, 90, or about 100 times faster in the cell (or under *in vitro* conditions selected to mimic intracellular conditions) as compared to blood (or under *in vitro* conditions selected to mimic extracellular conditions). The rate of cleavage of candidate compounds can be determined using standard enzyme kinetics assays under conditions chosen to mimic intracellular media and compared to conditions chosen to mimic extracellular media.

*ii. Phosphate-based cleavable linking groups*

In some embodiments, a cleavable linker comprises a phosphate-based cleavable linking group. A phosphate-based cleavable linking group is cleaved by agents that degrade or hydrolyze the phosphate group. An example of an agent that cleaves phosphate groups in cells are enzymes such as phosphatases in cells. Examples of phosphate-based linking groups are -OP(O)(ORk)-O-, -O-P(S)(ORk)-O-, -O-P(S)(SRk)-O-, -S-P(O)(ORk)-O-, -O-P(O)(ORk)-S-, -S-P(O)(ORk)-S-, -O-P(S)(ORk)-S-, -S-P(S)(ORk)-O-, -O-P(O)(Rk)-O-, -O-P(S)(Rk)-O-, -S-P(O)(Rk)-O-, -S-P(S)(Rk)-O-, -S-P(O)(Rk)-S-, -O-P(S)( Rk)-S-. In some embodiments, phosphate-based linking groups are -O-P(O)(OH)-O-, -O-P(S)(OH)-O-, -O-P(S)(SH)-O-, -S-P(O)(OH)-O-, -O-P(O)(OH)-S-, -S-P(O)(OH)-S-, -O-P(S)(OH)-S-, -S-P(S)(OH)-O-, -OP(O)(H)-O-, -O-P(S)(H)-O-, -S-P(O)(H)-O, -S-P(S)(H)-O-, -S-P(O)(H)-S-, -O-P(S)(H)-S--, wherein Rk at each occurrence can be, independently, C1-C20 alkyl, C1-C20 haloalkyl, C6-C10 aryl, or C7-C12 aralkyl. In some embodiments, a phosphate-based linking group is -OP(O)(OH)-O-. These candidates can be evaluated using methods analogous to those described above.

*iii. Acid cleavable linking groups*

In some embodiments, a cleavable linker comprises an acid cleavable linking group. An acid cleavable linking group is a linking group that is cleaved under acidic conditions. In some embodiments acid cleavable linking groups are cleaved in an acidic environment with a pH of about 6.5 or lower (*e.g.*, about 6.0, 5.75, 5.5, 5.25, 5.0, or lower), or by agents such as enzymes that can act as a general acid. In a cell, specific low pH organelles, such as endosomes and lysosomes can provide a cleaving environment for acid cleavable linking groups. Examples of acid cleavable linking groups include but are not limited to hydrazones, esters, and esters of amino acids. Acid cleavable groups can have the general formula -C=NN-, C(O)O, or -OC(O). In some embodiments, the carbon attached to the oxygen of the ester (the alkoxy group) is an aryl group, substituted alkyl group, or tertiary alkyl group such as dimethyl pentyl or t-butyl. These candidates can be evaluated using methods analogous to those described above.

*iv. Ester-based cleavable linking groups*

In some embodiments, a cleavable linker comprises an ester-based cleavable linking group. An ester-based cleavable linking group is cleaved by enzymes such as esterases and amidases in cells. Examples of ester-based cleavable linking groups include but are not limited to esters of alkylene, alkenylene and alkynylene groups. Ester cleavable linking groups have the general formula -C(O)O-, or -OC(O)-. These candidates can be evaluated using methods analogous to those described above.

*v. Peptide-based cleavable linking groups*

In some embodiments, a cleavable linker comprises a peptide-based cleavable linking group. A peptide-based cleavable linking group is cleaved by enzymes such as peptidases and proteases in cells. Peptide-based cleavable linking groups are peptide bonds formed between amino acids to yield oligopeptides (*e.g.,* dipeptides, tripeptides *etc.*) and polypeptides. Peptide-based cleavable groups do not include the amide group (-C(O)NH-). The amide group can be formed between any alkylene, alkenylene or alkynelene. A peptide bond is a special type of amide bond formed between amino acids to yield peptides and proteins. The peptide-based cleavage group is generally limited to the peptide bond (*i.e.,* the amide bond) formed between amino acids yielding peptides and proteins and does not include the entire amide functional group. Peptide-based cleavable linking groups have the general formula - $NHCHR^AC(O)NHCHR^BC(O)$-, where $R^A$ and $R^B$ are the R groups of the two adjacent amino acids. These candidates can be evaluated using methods analogous to those described above. Representative U.S. patents that teach the preparation of RNA conjugates include, but are not limited to, U.S. Pat. Nos. 4,828,979; 4,948,882; 5,218,105; 5,525,465; 5,541,313; 5,545,730; 5,552,538; 5,578,717, 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667;

5,514,785; 5,565,552; 5,567,810; 5,574,142; 5,585,481; 5,587,371; 5,595,726; 5,597,696; 5,599,923; 5,599,928 and 5,688,941; 6,294,664; 6,320,017; 6,576,752; 6,783,931; 6,900,297; 7,037,646; 8,106,022, the entire contents of each of which is herein incorporated by reference.

It is not necessary for all positions in a given compound to be uniformly modified, and in fact more than one of the aforementioned modifications may be incorporated in a single compound or even at a single nucleoside within an iRNA. The present disclosure also includes iRNA compounds that are chimeric compounds.

"Chimeric" iRNA compounds, or "chimeras," in the context of the present disclosure, are iRNA compounds, e.g., dsRNAs, that contain two or more chemically distinct regions, each made up of at least one monomer unit, *i.e.,* a nucleotide in the case of a dsRNA compound. These iRNAs typically contain at least one region wherein the RNA is modified so as to confer upon the iRNA increased resistance to nuclease degradation, increased cellular uptake, and/or increased binding affinity for the target nucleic acid. An additional region of the iRNA may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of iRNA inhibition of gene expression. Consequently, comparable results can often be obtained with shorter iRNAs when chimeric dsRNAs are used, compared to phosphorothioate deoxy dsRNAs hybridizing to the same target region. Cleavage of the RNA target can be routinely detected by gel electrophoresis and, if necessary, associated nucleic acid hybridization techniques known in the art.

In certain instances, the RNA of an iRNA can be modified by a non-ligand group. A number of non-ligand molecules have been conjugated to iRNAs in order to enhance the activity, cellular distribution or cellular uptake of the iRNA, and procedures for performing such conjugations are available in the scientific literature. Such non-ligand moieties have included lipid moieties, such as cholesterol (Kubo, T. et al., Biochem. Biophys. Res. Comm., 2007, 365(1):54-61; Letsinger et al., Proc. Natl. Acad. Sci. USA, 1989, 86:6553), cholic acid (Manoharan et al., Bioorg. Med. Chem. Lett., 1994, 4:1053), a thioether, *e.g.,* hexyl-S-tritylthiol (Manoharan et al., Ann. N.Y. Acad. Sci., 1992, 660:306; Manoharan et al., Bioorg. Med. Chem. Let., 1993, 3:2765), a thiocholesterol (Oberhauser et al., Nucl. Acids Res., 1992, 20:533), an aliphatic chain, *e.g.,* dodecandiol or undecyl residues (Saison-Behmoaras et al., EMBO J., 1991, 10:111; Kabanov et al., FEBS Lett., 1990, 259:327; Svinarchuk et al., Biochimie, 1993, 75:49), a phospholipid, *e.g.*, di-hexadecyl-rac-glycerol or triethylammonium 1,2-di-O-hexadecyl-rac-glycero-3-H-phosphonate (Manoharan et al., Tetrahedron Lett., 1995, 36:3651; Shea et al., Nucl. Acids Res., 1990, 18:3777), a polyamine or a polyethylene glycol chain (Manoharan et al., Nucleosides & Nucleotides, 1995, 14:969), or adamantane acetic acid (Manoharan et al., Tetrahedron Lett., 1995, 36:3651), a palmityl moiety (Mishra et al., Biochim. Biophys. Acta, 1995, 1264:229), or an octadecylamine or hexylamino-carbonyl-oxycholesterol moiety (Crooke et al., J. Pharmacol. Exp. Ther., 1996, 277:923). Representative United States patents that teach the preparation of such RNA conjugates have been listed above. Typical conjugation protocols involve the synthesis of an RNAs bearing an aminolinker at one or more positions of the sequence. The amino group is then reacted with the molecule being conjugated using appropriate coupling or activating reagents. The conjugation reaction may be performed either with the RNA still bound to the solid support or following cleavage of the RNA, in solution phase. Purification of the RNA conjugate by HPLC typically affords the pure conjugate.

## V. Delivery of iRNA

The delivery of an iRNA to a subject in need thereof can be achieved in a number of different ways. *In vivo* delivery can be performed directly by administering a composition comprising an iRNA, *e.g.* a dsRNA, to a subject. Alternatively, delivery can be performed indirectly by administering one or more vectors that encode and direct the expression of the iRNA. These alternatives are discussed further below.

### *A. Direct delivery*

In general, any method of delivering a nucleic acid molecule can be adapted for use with an iRNA (*see e.g.,* Akhtar S. and Julian RL. (1992) Trends Cell. Biol. 2(5):139-144 and WO94/02595, which are incorporated herein by reference in their entireties). However, there are three factors that are important to consider in order to successfully deliver an iRNA molecule *in vivo:* (a) biological stability of the delivered molecule, (2) preventing non-specific effects, and (3) accumulation of the delivered molecule in the target tissue. The non-specific effects of an iRNA can be minimized by local administration, for example by direct injection or implantation into a tissue (as a non-limiting example, the eye) or topically administering the preparation. Local administration to a treatment site maximizes local concentration of the agent, limits the exposure of the agent to systemic tissues that may otherwise be harmed by the agent or that may degrade the agent, and permits a lower total dose of the iRNA molecule to be administered. Several studies have shown successful knockdown of gene products when an iRNA is administered locally. For example, intraocular delivery of a dsRNA by intravitreal injection in cynomolgus monkeys (Tolentino, MJ., et al (2004) Retina 24:132-138) and subretinal injections in mice (Reich, SJ., et al (2003) Mol. Vis. 9:210-216) were both shown to prevent neovascularization in an experimental model of age-related

macular degeneration. In addition, direct intratumoral injection of a dsRNA in mice reduces tumor volume (Pille, J., et al (2005) Mol. Ther.11:267-274) and can prolong survival of tumor-bearing mice (Kim, WJ., et al (2006) Mol. Ther. 14:343-350; Li, S., et al (2007) Mol. Ther. 15:515-523). RNA interference has also shown success with local delivery to the CNS by direct injection (Dorn, G., et al. (2004) Nucleic Acids 32:e49; Tan, PH., et al (2005) Gene Ther. 12:59-66; Makimura, H., et al (2002) BMC Neurosci. 3:18; Shishkina, GT., et al (2004) Neuroscience 129:521-528; Thakker, ER., et al (2004) Proc. Natl. Acad. Sci. U.S.A. 101:17270-17275; Akaneya,Y., et al (2005) J. Neurophysiol. 93:594-602) and to the lungs by intranasal administration (Howard, KA., et al (2006) Mol. Ther. 14:476-484; Zhang, X., et al (2004) J. Biol. Chem. 279:10677-10684; Bitko, V., et al (2005) Nat. Med. 11:50-55). For administering an iRNA systemically for the treatment of a disease, the RNA can be modified or alternatively delivered using a drug delivery system; both methods act to prevent the rapid degradation of the dsRNA by endo- and exo-nucleases *in vivo.*

Modification of the RNA or the pharmaceutical carrier can also permit targeting of the iRNA composition to the target tissue and avoid undesirable off-target effects. iRNA molecules can be modified by chemical conjugation to other groups, *e.g.,* a lipid or carbohydrate group as described herein. Such conjugates can be used to target iRNA to particular cells, *e.g.,* liver cells, *e.g.,* hepatocytes. For example, GalNAc conjugates or lipid *(e.g.,* LNP) formulations can be used to target iRNA to particular cells, *e.g.,* liver cells, *e.g.,* hepatocytes.

iRNA molecules can also be modified by chemical conjugation to lipophilic groups such as cholesterol to enhance cellular uptake and prevent degradation. For example, an iRNA directed against ApoB conjugated to a lipophilic cholesterol moiety was injected systemically into mice and resulted in knockdown of apoB mRNA in both the liver and jejunum (Soutschek, J., et al (2004) Nature 432:173-178). Conjugation of an iRNA to an aptamer has been shown to inhibit tumor growth and mediate tumor regression in a mouse model of prostate cancer (McNamara, JO., et al (2006) Nat. Biotechnol. 24:1005-1015). In an alternative embodiment, the iRNA can be delivered using drug delivery systems such as a nanoparticle, a dendrimer, a polymer, liposomes, or a cationic delivery system. Positively charged cationic delivery systems facilitate binding of an iRNA molecule (negatively charged) and also enhance interactions at the negatively charged cell membrane to permit efficient uptake of an iRNA by the cell. Cationic lipids, dendrimers, or polymers can either be bound to an iRNA, or induced to form a vesicle or micelle *(see e.g.,* Kim SH., et al (2008) Journal of Controlled Release 129(2):107-116) that encases an iRNA. The formation of vesicles or micelles further prevents degradation of the iRNA when administered systemically. Methods for making and administering cationic- iRNA complexes are well within the abilities of one skilled in the art *(see e.g.,* Sorensen, DR., et al (2003) J. Mol. Biol 327:761-766; Verma, UN., et al (2003) Clin. Cancer Res. 9:1291-1300; Arnold, AS et al (2007) J. Hypertens. 25:197-205, which are incorporated herein by reference in their entirety). Some non-limiting examples of drug delivery systems useful for systemic delivery of iRNAs include DOTAP (Sorensen, DR., *et al* (2003), supra; Verma, UN., *et al* (2003), supra), Oligofectamine, "solid nucleic acid lipid particles" (Zimmermann, TS., et al (2006) Nature 441:111-114), cardiolipin (Chien, PY., et al (2005) Cancer Gene Ther. 12:321-328; Pal, A., et al (2005) Int J. Oncol. 26:1087-1091), polyethyleneimine (Bonnet ME., et al (2008) Pharm. Res. Aug 16 Epub ahead of print; Aigner, A. (2006) J. Biomed. Biotechnol. 71659), Arg-Gly-Asp (RGD) peptides (Liu, S. (2006) Mol. Pharm. 3:472-487), and polyamidoamines (Tomalia, DA., et al (2007) Biochem. Soc. Trans. 35:61-67; Yoo, H., et al (1999) Pharm. Res. 16:1799-1804). In some embodiments, an iRNA forms a complex with cyclodextrin for systemic administration. Methods for administration and pharmaceutical compositions of iRNAs and cyclodextrins can be found in U.S. Patent No. 7,427,605, which is herein incorporated by reference in its entirety.

*B. Vector encoded iRNAs*

In another aspect, iRNA targeting CA2 can be expressed from transcription units inserted into DNA or RNA vectors (*see, e.g.,* Couture, A, et al., TIG. (1996), 12:5-10; Skillern, A., et al., International PCT Publication No. WO 00/22113, Conrad, International PCT Publication No. WO 00/22114, and Conrad, U.S. Pat. No. 6,054,299). Expression can be transient (on the order of hours to weeks) or sustained (weeks to months or longer), depending upon the specific construct used and the target tissue or cell type. These transgenes can be introduced as a linear construct, a circular plasmid, or a viral vector, which can be an integrating or non-integrating vector. The transgene can also be constructed to permit it to be inherited as an extrachromosomal plasmid (Gassmann, et al., Proc. Natl. Acad. Sci. USA (1995) 92:1292).

The individual strand or strands of an iRNA can be transcribed from a promoter on an expression vector. Where two separate strands are to be expressed to generate, for example, a dsRNA, two separate expression vectors can be co-introduced (e.g., by transfection or infection) into a target cell. Alternatively, each individual strand of a dsRNA can be transcribed by promoters both of which are located on the same expression plasmid. In some embodiments, a dsRNA is expressed as an inverted repeat joined by a linker polynucleotide sequence such that the dsRNA has a stem and loop structure.

An iRNA expression vector is typically a DNA plasmid or viral vector. An expression vector compatible with eukaryotic cells, *e.g.,* with vertebrate cells, can be used to produce recombinant constructs for the expression of an iRNA as described herein. Eukaryotic cell expression vectors are well known in the art and are available from a number of commercial sources. Typically, such vectors contain convenient restriction sites for insertion of the desired nucleic acid

segment. Delivery of iRNA expressing vectors can be systemic, such as by intravenous or intramuscular administration, by administration to target cells ex-planted from the patient followed by reintroduction into the patient, or by any other means that allows for introduction into a desired target cell.

An iRNA expression plasmid can be transfected into a target cell as a complex with a cationic lipid carrier (*e.g.,* Oligofectamine) or a non-cationic lipid-based carrier (*e.g.,* Transit-TKO™). Multiple lipid transfections for iRNA-mediated knockdowns targeting different regions of a target RNA over a period of a week or more are also contemplated by the disclosure. Successful introduction of vectors into host cells can be monitored using various known methods. For example, transient transfection can be signaled with a reporter, such as a fluorescent marker, such as Green Fluorescent Protein (GFP). Stable transfection of cells *ex vivo* can be ensured using markers that provide the transfected cell with resistance to specific environmental factors (e.g., antibiotics and drugs), such as hygromycin B resistance.

Viral vector systems which can be utilized with the methods and compositions described herein include, but are not limited to, (a) adenovirus vectors; (b) retrovirus vectors, including but not limited to lentiviral vectors, moloney murine leukemia virus, *etc.;* (c) adeno- associated virus vectors; (d) herpes simplex virus vectors; (e) SV40 vectors; (f) polyoma virus vectors; (g) papilloma virus vectors; (h) picornavirus vectors; (i) pox virus vectors such as an orthopox, *e.g.,* vaccinia virus vectors or avipox, *e.g.* canary pox or fowl pox; and (j) a helper-dependent or gutless adenovirus. Replication-defective viruses can also be advantageous. Different vectors will or will not become incorporated into the cells' genome. The constructs can include viral sequences for transfection, if desired. Alternatively, the construct may be incorporated into vectors capable of episomal replication, *e.g* EPV and EBV vectors. Constructs for the recombinant expression of an iRNA will generally require regulatory elements, *e.g.*, promoters, enhancers, *etc.,* to ensure the expression of the iRNA in target cells. Other aspects to consider for vectors and constructs are further described below.

Vectors useful for the delivery of an iRNA will include regulatory elements (promoter, enhancer, *etc.*) sufficient for expression of the iRNA in the desired target cell or tissue. The regulatory elements can be chosen to provide either constitutive or regulated/inducible expression.

Expression of the iRNA can be precisely regulated, for example, by using an inducible regulatory sequence that is sensitive to certain physiological regulators, *e.g.*, circulating glucose levels, or hormones (Docherty et al., 1994, FASEB J. 8:20-24). Such inducible expression systems, suitable for the control of dsRNA expression in cells or in mammals include, for example, regulation by ecdysone, by estrogen, progesterone, tetracycline, chemical inducers of dimerization, and isopropyl-β-D1-thiogalactopyranoside (IPTG). A person skilled in the art would be able to choose the appropriate regulatory/promoter sequence based on the intended use of the iRNA transgene.

In a specific embodiment, viral vectors that contain nucleic acid sequences encoding an iRNA can be used. For example, a retroviral vector can be used (see Miller et al., Meth. Enzymol. 217:581-599 (1993)). These retroviral vectors contain the components necessary for the correct packaging of the viral genome and integration into the host cell DNA. The nucleic acid sequences encoding an iRNA are cloned into one or more vectors, which facilitates delivery of the nucleic acid into a patient. More detail about retroviral vectors can be found, for example, in Boesen et al., Biotherapy 6:291-302 (1994), which describes the use of a retroviral vector to deliver the mdr1 gene to hematopoietic stem cells in order to make the stem cells more resistant to chemotherapy. Other references illustrating the use of retroviral vectors in gene therapy are: Clowes et al., J. Clin. Invest. 93:644-651 (1994); Kiem et al., Blood 83:1467-1473 (1994); Salmons and Gunzberg, Human Gene Therapy 4:129-141 (1993); and Grossman and Wilson, Curr. Opin. in Genetics and Devel. 3:110-114 (1993). Lentiviral vectors contemplated for use include, for example, the HIV based vectors described in U.S. Patent Nos. 6,143,520; 5,665,557; and 5,981,276, which are herein incorporated by reference.

Adenoviruses are also contemplated for use in delivery of iRNAs. Adenoviruses are especially attractive vehicles, *e.g.,* for delivering genes to respiratory epithelia. Adenoviruses naturally infect respiratory epithelia where they cause a mild disease. Other targets for adenovirus-based delivery systems are liver, the central nervous system, endothelial cells, and muscle. Adenoviruses have the advantage of being capable of infecting non-dividing cells. Kozarsky and Wilson, Current Opinion in Genetics and Development 3:499-503 (1993) present a review of adenovirus-based gene therapy. Bout et al., Human Gene Therapy 5:3-10 (1994) demonstrated the use of adenovirus vectors to transfer genes to the respiratory epithelia of rhesus monkeys. Other instances of the use of adenoviruses in gene therapy can be found in Rosenfeld et al., Science 252:431-434 (1991); Rosenfeld et al., Cell 68:143-155 (1992); Mastrangeli et al., J. Clin. Invest. 91:225-234 (1993); PCT Publication WO94/12649; and Wang, et al., Gene Therapy 2:775-783 (1995). A suitable AV vector for expressing an iRNA featured in the disclosure, a method for constructing the recombinant AV vector, and a method for delivering the vector into target cells, are described in Xia H et al. (2002), Nat. Biotech. 20: 1006-1010.

Use of Adeno-associated virus (AAV) vectors is also contemplated (Walsh et al., Proc. Soc. Exp. Biol. Med. 204:289-300 (1993); U.S. Pat. No. 5,436,146). In some embodiments, the iRNA can be expressed as two separate, complementary single-stranded RNA molecules from a recombinant AAV vector having, for example, either the U6 or H1 RNA promoters, or the cytomegalovirus (CMV) promoter. Suitable AAV vectors for expressing the dsRNA featured in the disclosure, methods for constructing the recombinant AV vector, and methods for delivering the vectors into target cells are described in Samulski R et al. (1987), J. Virol. 61: 3096-3101; Fisher K J et al. (1996), J. Virol., 70: 520-532; Samulski R et al. (1989), J. Virol. 63: 3822-3826; U.S. Pat. No. 5,252,479; U.S. Pat. No. 5,139,941; International Patent Application No.

WO 94/13788; and International Patent Application No. WO 93/24641, the entire disclosures of which are herein incorporated by reference.

Another typical viral vector is a pox virus such as a vaccinia virus, for example an attenuated vaccinia such as Modified Virus Ankara (MVA) or NYVAC, an avipox such as fowl pox or canary pox.

The tropism of viral vectors can be modified by pseudotyping the vectors with envelope proteins or other surface antigens from other viruses, or by substituting different viral capsid proteins, as appropriate. For example, lentiviral vectors can be pseudotyped with surface proteins from vesicular stomatitis virus (VSV), rabies, Ebola, Mokola, and the like. AAV vectors can be made to target different cells by engineering the vectors to express different capsid protein serotypes; *see, e.g.,* Rabinowitz J E et al. (2002), J Virol 76:791-801, the entire disclosure of which is herein incorporated by reference.

The pharmaceutical preparation of a vector can include the vector in an acceptable diluent, or can include a slow release matrix in which the gene delivery vehicle is imbedded. Alternatively, where the complete gene delivery vector can be produced intact from recombinant cells, *e.g.*, retroviral vectors, the pharmaceutical preparation can include one or more cells which produce the gene delivery system.

## VI. Pharmaceutical compositions containing iRNA

In some embodiments, the disclosure provides pharmaceutical compositions containing an iRNA, as described herein, and a pharmaceutically acceptable carrier. The pharmaceutical composition containing the iRNA is useful for treating a disease or disorder related to the expression or activity of CA2 (*e.g.,* glaucoma or conditions associated with glaucoma). Such pharmaceutical compositions are formulated based on the mode of delivery. In some embodiments, compositions can be formulated for localized delivery, *e.g.,* by intraocular delivery (*e.g.,* intravitreal administration, e.g., intravitreal injection; transscleral administration, e.g., transscleral injection; subconjunctival administration, e.g., subconjunctival injection; retrobulbar administration, e.g., retrobulbar injection; intracameral administration, e.g., intracameral injection; or subretinal administration, e.g., subretinal injection). In other embodiments, compositions can be formulated for topical delivery. In another example, compositions can be formulated for systemic administration via parenteral delivery, *e.g.,* by intravenous (IV) delivery. In some embodiments, a composition provided herein (*e.g.,* a composition comprising a GalNAc conjugate or an LNP formulation) is formulated for intravenous delivery.

The pharmaceutical compositions featured herein are administered in a dosage sufficient to inhibit expression of CA2. In general, a suitable dose of iRNA will be in the range of 0.01 to 200.0 milligrams per kilogram body weight of the recipient per day. The pharmaceutical composition may be administered once daily, or the iRNA may be administered as two, three, or more sub-doses at appropriate intervals throughout the day or even using continuous infusion or delivery through a controlled release formulation. In that case, the iRNA contained in each sub-dose must be correspondingly smaller in order to achieve the total daily dosage. The dosage unit can also be compounded for delivery over several days, *e.g.*, using a conventional sustained release formulation which provides sustained release of the iRNA over a several day period. Sustained release formulations are well known in the art and are particularly useful for delivery of agents at a particular site, such as can be used with the agents of the present disclosure. In this embodiment, the dosage unit contains a corresponding multiple of the daily dose.

The effect of a single dose on CA2 levels can be long lasting, such that subsequent doses are administered at not more than 3, 4, or 5-day intervals, or at not more than 1, 2, 3, 4, 12, 24, or 36-week intervals.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and *in vivo* half-lives for the individual iRNAs encompassed by the disclosure can be made using conventional methodologies or on the basis of *in vivo* testing using a suitable animal model.

A suitable animal model, *e.g.,* a mouse or a cynomolgus monkey, e.g., an animal containing a transgene expressing human CA2, can be used to determine the therapeutically effective dose and/or an effective dosage regimen administration of CA2 siRNA.

The present disclosure also includes pharmaceutical compositions and formulations that include the iRNA compounds featured herein. The pharmaceutical compositions of the present disclosure may be administered in a number of ways depending upon whether local or systemic treatment is desired and upon the area to be treated. Administration may be local *(e.g.,* by intraocular injection), topical *(e.g.,* by an eye drop solution), or parenteral. Parenteral administration includes intravenous, intraarterial, subcutaneous, intraperitoneal or intramuscular injection or infusion; subdermal, *e.g.,* via an implanted device; or intracranial, *e.g.,* by intraparenchymal, intrathecal, or intraventricular administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Suitable topical formulations include those in which the iRNAs featured in the

disclosure are in admixture with a topical delivery agent such as lipids, liposomes, fatty acids, fatty acid esters, steroids, chelating agents and surfactants. Suitable lipids and liposomes include neutral (e.g., dioleoylphosphatidyl DOPE ethanolamine, dimyristoylphosphatidyl choline DMPC, distearolyphosphatidyl choline) negative (e.g., dimyristoylphosphatidyl glycerol DMPG) and cationic (*e.g.*, dioleoyltetramethylaminopropyl DOTAP and dioleoylphosphatidyl ethanolamine DOTMA). iRNAs featured in the disclosure may be encapsulated within liposomes or may form complexes thereto, in particular to cationic liposomes. Alternatively, iRNAs may be complexed to lipids, in particular to cationic lipids. Suitable fatty acids and esters include but are not limited to arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a $C_{1-20}$ alkyl ester (e.g., isopropylmyristate IPM), monoglyceride, diglyceride or pharmaceutically acceptable salt thereof. Topical formulations are described in detail in U.S. Patent No. 6,747,014, which is incorporated herein by reference.

*A. Liposomal formulations*

There are many organized surfactant structures besides microemulsions that have been studied and used for the formulation of drugs. These include monolayers, micelles, bilayers and vesicles. Vesicles, such as liposomes, have attracted great interest because of their specificity and the duration of action they offer from the standpoint of drug delivery. As used in the present disclosure, the term "liposome" means a vesicle composed of amphiphilic lipids arranged in a spherical bilayer or bilayers.

Liposomes are unilamellar or multilamellar vesicles which have a membrane formed from a lipophilic material and an aqueous interior. The aqueous portion contains the composition to be delivered. Cationic liposomes possess the advantage of being able to fuse to the cell wall. Non-cationic liposomes, although not able to fuse as efficiently with the cell wall, are taken up by macrophages *in vivo.*

In order to traverse intact mammalian skin, lipid vesicles must pass through a series of fine pores, each with a diameter less than 50 nm, under the influence of a suitable transdermal gradient. Therefore, it is desirable to use a liposome which is highly deformable and able to pass through such fine pores.

Further advantages of liposomes include; liposomes obtained from natural phospholipids are biocompatible and biodegradable; liposomes can incorporate a wide range of water and lipid soluble drugs; liposomes can protect encapsulated drugs in their internal compartments from metabolism and degradation (Rosoff, in Pharmaceutical Dosage Forms, Lieberman, Rieger and Banker (Eds.), 1988, Marcel Dekker, Inc., New York, N.Y., volume 1, p. 245). Important considerations in the preparation of liposome formulations are the lipid surface charge, vesicle size and the aqueous volume of the liposomes.

Liposomes are useful for the transfer and delivery of active ingredients to the site of action. Because the liposomal membrane is structurally similar to biological membranes, when liposomes are applied to a tissue, the liposomes start to merge with the cellular membranes and as the merging of the liposome and cell progresses, the liposomal contents are emptied into the cell where the active agent may act.

Liposomal formulations have been the focus of extensive investigation as the mode of delivery for many drugs. There is growing evidence that for topical administration, liposomes present several advantages over other formulations. Such advantages include reduced side-effects related to high systemic absorption of the administered drug, increased accumulation of the administered drug at the desired target, and the ability to administer a wide variety of drugs, both hydrophilic and hydrophobic, into the skin.

Several reports have detailed the ability of liposomes to deliver agents including high-molecular weight DNA into the skin. Compounds including analgesics, antibodies, hormones and high-molecular weight DNAs have been administered to the skin. The majority of applications resulted in the targeting of the upper epidermis

Liposomes fall into two broad classes. Cationic liposomes are positively charged liposomes which interact with the negatively charged DNA molecules to form a stable complex. The positively charged DNA/liposome complex binds to the negatively charged cell surface and is internalized in an endosome. Due to the acidic pH within the endosome, the liposomes are ruptured, releasing their contents into the cell cytoplasm (Wang et al., Biochem. Biophys. Res. Commun., 1987, 147, 980-985).

Liposomes which are pH-sensitive or negatively charged, entrap DNA rather than complex with it. Since both the DNA and the lipid are similarly charged, repulsion rather than complex formation occurs. Nevertheless, some DNA is entrapped within the aqueous interior of these liposomes. pH-sensitive liposomes have been used to deliver DNA encoding the thymidine kinase gene to cell monolayers in culture. Expression of the exogenous gene was detected in the target cells (Zhou et al., Journal of Controlled Release, 1992, 19, 269-274).

One major type of liposomal composition includes phospholipids other than naturally derived phosphatidylcholine. Neutral liposome compositions, for example, can be formed from dimyristoyl phosphatidylcholine (DMPC) or dipalmitoyl phosphatidylcholine (DPPC). Anionic liposome compositions generally are formed from dimyristoyl phosphatidylglycerol, while anionic fusogenic liposomes are formed primarily from dioleoyl phosphatidylethanolamine

(DOPE). Another type of liposomal composition is formed from phosphatidylcholine (PC) such as, for example, soybean PC, and egg PC. Another type is formed from mixtures of phospholipid and/or phosphatidylcholine and/or cholesterol.

Several studies have assessed the topical delivery of liposomal drug formulations to the skin. Application of liposomes containing interferon to guinea pig skin resulted in a reduction of skin herpes sores while delivery of interferon via other means (e.g., as a solution or as an emulsion) were ineffective (Weiner et al., Journal of Drug Targeting, 1992, 2, 405-410). Further, an additional study tested the efficacy of interferon administered as part of a liposomal formulation to the administration of interferon using an aqueous system, and concluded that the liposomal formulation was superior to aqueous administration (du Plessis et al., Antiviral Research, 1992, 18, 259-265).

Non-ionic liposomal systems have also been examined to determine their utility in the delivery of drugs to the skin, in particular systems comprising non-ionic surfactant and cholesterol. Non-ionic liposomal formulations comprising Novasome™ I (glyceryl dilaurate/cholesterol/polyoxyethylene-10-stearyl ether) and Novasome™ II (glyceryl distearate/-cholesterol/polyoxyethylene-10-stearyl ether) were used to deliver cyclosporin-A into the dermis of mouse skin. Results indicated that such non-ionic liposomal systems were effective in facilitating the deposition of cyclosporin-A into different layers of the skin (Hu et al. S.T.P. Pharma. Sci., 1994, 4, 6, 466).

Liposomes also include "sterically stabilized" liposomes, a term which, as used herein, refers to liposomes comprising one or more specialized lipids that, when incorporated into liposomes, result in enhanced circulation lifetimes relative to liposomes lacking such specialized lipids. Examples of sterically stabilized liposomes are those in which part of the vesicle-forming lipid portion of the liposome (A) comprises one or more glycolipids, such as monosialoganglioside $G_{M1}$, or (B) is derivatized with one or more hydrophilic polymers, such as a polyethylene glycol (PEG) moiety. While not wishing to be bound by any particular theory, it is thought in the art that, at least for sterically stabilized liposomes containing gangliosides, sphingomyelin, or PEG-derivatized lipids, the enhanced circulation half-life of these sterically stabilized liposomes derives from a reduced uptake into cells of the reticuloendothelial system (RES) (Allen et al., FEBS Letters, 1987, 223, 42; Wu et al., Cancer Research, 1993, 53, 3765).

Various liposomes comprising one or more glycolipids are known in the art. Papahadjopoulos *et al. (Ann. N.Y. Acad. Sci.,* 1987, 507, 64) reported the ability of monosialoganglioside $G_{M1}$, galactocerebroside sulfate and phosphatidylinositol to improve blood half-lives of liposomes. These findings were expounded upon by Gabizon et al. (Proc. Natl. Acad. Sci. U.S.A., 1988, 85, 6949). U.S. Pat. No. 4,837,028 and WO 88/04924, both to Allen *et al.,* disclose liposomes comprising (1) sphingomyelin and (2) the ganglioside $G_{M1}$ or a galactocerebroside sulfate ester. U.S. Pat. No. 5,543,152 (Webb et al.) discloses liposomes comprising sphingomyelin. Liposomes comprising 1,2-sn-dimyristoylphosphatidylcholine are disclosed in WO 97/13499 (Lim et al).

Many liposomes comprising lipids derivatized with one or more hydrophilic polymers, and methods of preparation thereof, are known in the art. Sunamoto et al. (Bull. Chem. Soc. Jpn., 1980, 53, 2778) described liposomes comprising a nonionic detergent, $2C_{1215G}$, that contains a PEG moiety. Illum et al. (FEBS Lett., 1984, 167, 79) noted that hydrophilic coating of polystyrene particles with polymeric glycols results in significantly enhanced blood half-lives. Synthetic phospholipids modified by the attachment of carboxylic groups of polyalkylene glycols *(e.g.,* PEG) are described by Sears (U.S. Pat. Nos. 4,426,330 and 4,534,899). Klibanov et al. (FEBS Lett., 1990, 268, 235) described experiments demonstrating that liposomes comprising phosphatidylethanolamine (PE) derivatized with PEG or PEG stearate have significant increases in blood circulation half-lives. Blume et al. (Biochimica et Biophysica Acta, 1990, 1029, 91) extended such observations to other PEG-derivatized phospholipids, e.g., DSPE-PEG, formed from the combination of distearoylphosphatidylethanolamine (DSPE) and PEG. Liposomes having covalently bound PEG moieties on their external surface are described in European Patent No. EP 0 445 131 B1 and WO 90/04384 to Fisher. Liposome compositions containing 1-20 mole percent of PE derivatized with PEG, and methods of use thereof, are described by Woodle et al. (U.S. Pat. Nos. 5,013,556 and 5,356,633) and Martin et al. (U.S. Pat. No. 5,213,804 and European Patent No. EP 0 496 813 B1). Liposomes comprising a number of other lipid-polymer conjugates are disclosed in WO 91/05545 and U.S. Pat. No. 5,225,212 (both to Martin et al.) and in WO 94/20073 (Zalipsky et al.). Liposomes comprising PEG-modified ceramide lipids are described in WO 96/10391 (Choi et al). U.S. Pat. No. 5,540,935 (Miyazaki et al.) and U.S. Pat. No. 5,556,948 (Tagawa et al.) describe PEG-containing liposomes that can be further derivatized with functional moieties on their surfaces.

A number of liposomes comprising nucleic acids are known in the art. WO 96/40062 to Thierry *et al*. discloses methods for encapsulating high molecular weight nucleic acids in liposomes. U.S. Pat. No. 5,264,221 to Tagawa et al. discloses protein-bonded liposomes and asserts that the contents of such liposomes may include a dsRNA. U.S. Pat. No. 5,665,710 to Rahman et al. describes certain methods of encapsulating oligodeoxynucleotides in liposomes. WO 97/04787 to Love et al. discloses liposomes comprising dsRNAs targeted to the raf gene.

Transfersomes are yet another type of liposomes, and are highly deformable lipid aggregates which are attractive candidates for drug delivery vehicles. Transfersomes may be described as lipid droplets which are so highly deformable that they are easily able to penetrate through pores which are smaller than the droplet. Transfersomes are adaptable to the environment in which they are used, *e.g.,* they are self-optimizing (adaptive to the shape of pores in the skin), self-repairing, frequently reach their targets without fragmenting, and often self-loading. To make transfersomes it is

possible to add surface edge-activators, usually surfactants, to a standard liposomal composition. Transfersomes have been used to deliver serum albumin to the skin. The transfersome-mediated delivery of serum albumin has been shown to be as effective as subcutaneous injection of a solution containing serum albumin.

Surfactants find wide application in formulations such as emulsions (including microemulsions) and liposomes. The most common way of classifying and ranking the properties of the many different types of surfactants, both natural and synthetic, is by the use of the hydrophile/lipophile balance (HLB). The nature of the hydrophilic group (also known as the "head") provides the most useful means for categorizing the different surfactants used in formulations (Rieger, in Pharmaceutical Dosage Forms, Marcel Dekker, Inc., New York, N.Y., 1988, p. 285).

If the surfactant molecule is not ionized, it is classified as a nonionic surfactant. Nonionic surfactants find wide application in pharmaceutical and cosmetic products and are usable over a wide range of pH values. In general, their HLB values range from 2 to about 18 depending on their structure. Nonionic surfactants include nonionic esters such as ethylene glycol esters, propylene glycol esters, glyceryl esters, polyglyceryl esters, sorbitan esters, sucrose esters, and ethoxylated esters. Nonionic alkanolamides and ethers such as fatty alcohol ethoxylates, propoxylated alcohols, and ethoxylated/propoxylated block polymers are also included in this class. The polyoxyethylene surfactants are the most popular members of the nonionic surfactant class.

If the surfactant molecule carries a negative charge when it is dissolved or dispersed in water, the surfactant is classified as anionic. Anionic surfactants include carboxylates such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates. The most important members of the anionic surfactant class are the alkyl sulfates and the soaps.

If the surfactant molecule carries a positive charge when it is dissolved or dispersed in water, the surfactant is classified as cationic. Cationic surfactants include quaternary ammonium salts and ethoxylated amines. The quaternary ammonium salts are the most used members of this class.

If the surfactant molecule has the ability to carry either a positive or negative charge, the surfactant is classified as amphoteric. Amphoteric surfactants include acrylic acid derivatives, substituted alkylamides, N-alkylbetaines and phosphatides.

The use of surfactants in drug products, formulations and in emulsions has been reviewed (Rieger, in Pharmaceutical Dosage Forms, Marcel Dekker, Inc., New York, N.Y., 1988, p. 285).

*B. Nucleic acid lipid particles*

In some embodiments, a CA2 dsRNA featured in the disclosure is fully encapsulated in the lipid formulation, *e.g.*, to form a SPLP, pSPLP, SNALP, or other nucleic acid-lipid particle. SNALPs and SPLPs typically contain a cationic lipid, a non-cationic lipid, and a lipid that prevents aggregation of the particle (*e.g.,* a PEG-lipid conjugate). SNALPs and SPLPs are extremely useful for systemic applications, as they exhibit extended circulation lifetimes following intravenous (i.v.) injection and accumulate at distal sites (*e.g.,* sites physically separated from the administration site). SPLPs include "pSPLP," which include an encapsulated condensing agent-nucleic acid complex as set forth in PCT Publication No. WO 00/03683. The particles of the present disclosure typically have a mean diameter of about 50 nm to about 150 nm, more typically about 60 nm to about 130 nm, more typically about 70 nm to about 110 nm, most typically about 70 nm to about 90 nm, and are substantially nontoxic. In addition, the nucleic acids when present in the nucleic acid- lipid particles of the present disclosure are resistant in aqueous solution to degradation with a nuclease. Nucleic acid-lipid particles and their method of preparation are disclosed in, *e.g.,* U.S. Patent Nos. 5,976,567; 5,981,501; 6,534,484; 6,586,410; 6,815,432; and PCT Publication No. WO 96/40964.

In some embodiments, the lipid to drug ratio (mass/mass ratio) (*e.g.,* lipid to dsRNA ratio) will be in the range of from about 1:1 to about 50:1, from about 1:1 to about 25:1, from about 3:1 to about 15:1, from about 4:1 to about 10:1, from about 5:1 to about 9:1, or about 6:1 to about 9:1.

The cationic lipid may be, for example, N,N-dioleyl-N,N-dimethylammonium chloride (DODAC), N,N-distearyl-N,N-dimethylammonium bromide (DDAB), N-(I -(2,3-dioleoyloxy)propyl)-N,N,N-trimethylammonium chloride (DOTAP), N-(I -(2,3-dioleyloxy)propyl)-N,N,N-trimethylammonium chloride (DOTMA), N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), 1,2-Dilinoleylcarbamoyloxy-3-dimethylaminopropane (DLin-C-DAP), 1,2-Dilinoleyoxy-3-(dimethylamino) acetoxypropane (DLin-DAC), 1,2-Dilinoleyoxy-3-morpholinopropane (DLin-MA), 1,2-Dilinoleoyl-3-dimethylaminopropane (DLinDAP), 1,2-Dilinoleylthio-3-dimethylaminopropane (DLin-S-DMA), 1-Linoleoyl-2-linoleyloxy-3-dimethylaminopropane (DLin-2-DMAP), 1,2-Dilinoleyloxy-3-trimethylaminopropane chloride salt (DLin-TMA.Cl), 1,2-Dilinoleoyl-3-trimethylaminopropane chloride salt (DLin-TAP.Cl), 1,2-Dilinoleyloxy-3-(N-methylpiperazino)propane (DLin-MPZ), or 3-(N,N-Dilinoleylamino)-1,2-propanediol (DLinAP), 3-(N,N-Dioleylamino)-1,2-propanedio (DOAP), 1,2-Dilinoleyloxo-3-(2-N,N-dimethylamino)ethoxypropane (DLin-EG-DMA), 1,2-Dilinolenyloxy-N,N-dimethylaminopropane (DLinDMA), 2,2-Dilinoleyl-4-dimethylaminomethyl-[1,3]-dioxolane (DLin-K-DMA) or analogs thereof, (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), or a mixture thereof. The cationic lipid may comprise from about 20 mol % to about 50 mol % or about 40 mol % of the total lipid present in the particle.

In some embodiments, the compound 2,2-Dilinoleyl-4-dimethylaminoethyl-[1,3]-dioxolane can be used to prepare lipid-siRNA nanoparticles. Synthesis of 2,2-Dilinoleyl-4-dimethylaminoethyl-[1,3]-dioxolane is described in United States provisional patent application number 61/107,998 filed on October 23, 2008, which is herein incorporated by reference.

In some embodiments, the lipid-siRNA particle includes 40% 2, 2-Dilinoleyl-4-dimethylaminoethyl-[1,3]-dioxolane: 10% DSPC: 40% Cholesterol: 10% PEG-C-DOMG (mole percent) with a particle size of 63.0 $\pm$ 20 nm and a 0.027 siRNA/Lipid Ratio.

The non-cationic lipid may be an anionic lipid or a neutral lipid including, but not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoylphosphatidylethanolamine (POPE), dioleoyl- phosphatidylethanolamine 4-(N-maleimidomethyl)-cyclohexane-1- carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1 -trans PE, 1 -stearoyl-2-oleoyl- phosphatidyethanolamine (SOPE), cholesterol, or a mixture thereof. The non-cationic lipid may be from about 5 mol % to about 90 mol %, about 10 mol %, or about 58 mol % if cholesterol is included, of the total lipid present in the particle.

The conjugated lipid that inhibits aggregation of particles may be, for example, a polyethyleneglycol (PEG)-lipid including, without limitation, a PEG-diacylglycerol (DAG), a PEG-dialkyloxypropyl (DAA), a PEG-phospholipid, a PEG-ceramide (Cer), or a mixture thereof. The PEG-DAA conjugate may be, for example, a PEG-dilauryloxypropyl ($Ci_2$), a PEG-dimyristyloxypropyl ($Ci_4$), a PEG-dipalmityloxypropyl ($Ci_6$), or a PEG- distearyloxypropyl ($[C]_8$). The conjugated lipid that prevents aggregation of particles may be from 0 mol % to about 20 mol % or about 2 mol % of the total lipid present in the particle.

In some embodiments, the nucleic acid-lipid particle further includes cholesterol at, *e.g.,* about 10 mol % to about 60 mol % or about 48 mol % of the total lipid present in the particle.

In some embodiments, the iRNA is formulated in a lipid nanoparticle (LNP). *LNP01*

In some embodiments, the lipidoid ND98·4HC1 (MW 1487) (see U.S. Patent Application No. 12/056,230, filed 3/26/2008, which is herein incorporated by reference), Cholesterol (Sigma-Aldrich), and PEG-Ceramide C16 (Avanti Polar Lipids) can be used to prepare lipid-dsRNA nanoparticles (e.g., LNP01 particles). Stock solutions of each in ethanol can be prepared as follows: ND98, 133 mg/ml; Cholesterol, 25 mg/ml, PEG-Ceramide C16, 100 mg/ml. The ND98, Cholesterol, and PEG-Ceramide C16 stock solutions can then be combined in a, *e.g.,* 42:48:10 molar ratio. The combined lipid solution can be mixed with aqueous dsRNA (e.g., in sodium acetate pH 5) such that the final ethanol concentration is about 35-45% and the final sodium acetate concentration is about 100-300 mM. Lipid-dsRNA nanoparticles typically form spontaneously upon mixing. Depending on the desired particle size distribution, the resultant nanoparticle mixture can be extruded through a polycarbonate membrane (e.g., 100 nm cut-off) using, for example, a thermobarrel extruder, such as Lipex Extruder (Northern Lipids, Inc). In some cases, the extrusion step can be omitted. Ethanol removal and simultaneous buffer exchange can be accomplished by, for example, dialysis or tangential flow filtration. Buffer can be exchanged with, for example, phosphate buffered saline (PBS) at about pH 7, e.g., about pH 6.9, about pH 7.0, about pH 7.1, about pH 7.2, about pH 7.3, or about pH 7.4.

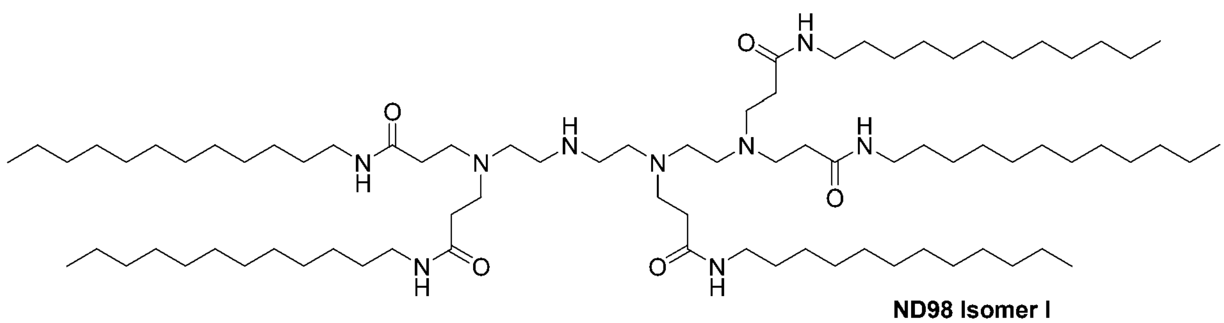

**ND98 Isomer I**

Formula I

LNP01 formulations are described, e.g., in International Application Publication No. WO 2008/042973, which is

hereby incorporated by reference.

Additional exemplary lipid-dsRNA formulations are provided in the following Table 1.

**Table 1: Exemplary lipid formulations**

| | **Cationic Lipid** | **cationic lipid/non-cationic lipid/cholesterol/PEG-lipid conjugate Lipid:siRNA ratio** |
|---|---|---|
| SNALP | 1,2-Dilinolenyloxy-N,N-dimethylaminopropane (DLinDMA) | DLinDMA/DPPC/Cholesterol/PEG-cDMA (57.1/7.1/34.4/1.4) lipid:siRNA ~ 7:1 |
| S-XTC | 2,2-Dilinoleyl-4-dimethylaminoethyl-[1,3]-dioxolane (XTC) | XTC/DPPC/Cholesterol/PEG-cDMA 57.1/7.1/34.4/1.4 lipid:siRNA ~ 7:1 |
| LNP05 | 2,2-Dilinoleyl-4-dimethylaminoethyl-[1,3]-dioxolane (XTC) | XTC/DSPC/Cholesterol/PEG-DMG 57.5/7.5/31.5/3.5 lipid:siRNA ~ 6:1 |
| LNP06 | 2,2-Dilinoleyl-4-dimethylaminoethyl-[1,3]-dioxolane (XTC) | XTC/DSPC/Cholesterol/PEG-DMG 57.5/7.5/31.5/3.5 lipid:siRNA ~ 11:1 |
| LNP07 | 2,2-Dilinoleyl-4-dimethylaminoethyl-[1,3]-dioxolane (XTC) | XTC/DSPC/Cholesterol/PEG-DMG 60/7.5/31/1.5, lipid:siRNA ~ 6:1 |
| LNP08 | 2,2-Dilinoleyl-4-dimethylaminoethyl-[1,3]-dioxolane (XTC) | XTC/DSPC/Cholesterol/PEG-DMG 60/7.5/31/1.5, lipid:siRNA ~ 11:1 |
| LNP09 | 2,2-Dilinoleyl-4-dimethylaminoethyl-[1,3]-dioxolane (XTC) | XTC/DSPC/Cholesterol/PEG-DMG 50/10/38.5/1.5 Lipid:siRNA 10:1 |
| LNP10 | (3aR,5s,6aS)-N,N-dimethyl-2,2-di((9Z,12Z)-octadeca-9,12-dienyl)tetrahydro-3aH-cyclopenta[d][1,3]dioxol-5-amine (ALN100) | ALN100/DSPC/Cholesterol/PEG-DMG 50/10/38.5/1.5 Lipid:siRNA 10:1 |
| LNP11 | (6Z,9Z,28Z,31Z)-heptatriaconta-6,9,28,31-tetraen-19-yl 4-(dimethylamino)butanoate (MC3) | MC-3/DSPC/Cholesterol/PEG-DMG 50/10/38.5/1.5 Lipid:siRNA 10:1 |
| LNP12 | 1,1'-(2-(4-(2-((2-(bis(2-hydroxydodecyl)amino)ethyl)(2-hydroxydodecyl) amino)ethyl)piperazin-1-yl)ethylazanediyl)didodecan-2-ol (C12-200) | C12-200/DSPC/Cholesterol/PEG-DMG 50/10/38.5/1.5 Lipid:siRNA 10:1 |
| LNP13 | XTC | XTC/DSPC/Chol/PEG-DMG 50/10/38.5/1.5 Lipid:siRNA: 33:1 |
| LNP14 | MC3 | MC3/DSPC/Chol/PEG-DMG 40/15/40/5 Lipid:siRNA: 11:1 |
| LNP15 | MC3 | MC3/DSPC/Chol/PEG- DSG/GalNAc-PEG-DSG 50/10/35/4.5/0.5 Lipid:siRNA: 11:1 |
| LNP16 | MC3 | MC3/DSPC/Chol/PEG-DMG 50/10/38.5/1.5 Lipid:siRNA: 7:1 |

(continued)

| | Cationic Lipid | cationic lipid/non-cationic lipid/cholesterol/PEG-lipid conjugate Lipid:siRNA ratio |
|---|---|---|
| LNP17 | MC3 | MC3/DSPC/Chol/PEG-DSG<br>50/10/38.5/1.5<br>Lipid:siRNA: 10:1 |
| LNP18 | MC3 | MC3/DSPC/Chol/PEG-DMG<br>50/10/38.5/1.5<br>Lipid:siRNA: 12:1 |
| LNP19 | MC3 | MC3/DSPC/Chol/PEG-DMG<br>50/10/35/5<br>Lipid:siRNA: 8:1 |
| LNP20 | MC3 | MC3/DSPC/Chol/PEG-DPG<br>50/10/38.5/1.5<br>Lipid:siRNA: 10:1 |
| LNP21 | C12-200 | C12-200/DSPC/Chol/PEG-DSG<br>50/10/38.5/1.5<br>Lipid:siRNA: 7:1 |
| LNP22 | XTC | XTC/DSPC/Chol/PEG-DSG<br>50/10/38.5/1.5<br>Lipid:siRNA: 10:1 |
| DSPC: distearoylphosphatidylcholine<br>DPPC: dipalmitoylphosphatidylcholine<br>PEG-DMG: PEG-didimyristoyl glycerol (C14-PEG, or PEG-C14) (PEG with avg mol wt of 2000)<br>PEG-DSG: PEG-distyryl glycerol (C18-PEG, or PEG-C18) (PEG with avg mol wt of 2000)<br>PEG-cDMA: PEG-carbamoyl-1,2-dimyristyloxypropylamine (PEG with avg mol wt of 2000) | | |

SNALP (1,2-Dilinolenyloxy-N,N-dimethylaminopropane (DLinDMA)) comprising formulations are described in International Publication No. WO2009/127060, filed April 15, 2009, which is hereby incorporated by reference.

XTC comprising formulations are described, *e.g.,* in U.S. Provisional Serial No. 61/148,366, filed January 29, 2009; U.S. Provisional Serial No. 61/156,851, filed March 2, 2009; U.S. Provisional Serial No. 61/185,712, filed June 10, 2009; U.S. Provisional Serial No. 61/228,373, filed July 24, 2009; U.S. Provisional Serial No. 61/239,686, filed September 3, 2009, and International Application No. PCT/US2010/022614, filed January 29, 2010, which are hereby incorporated by reference.

MC3 comprising formulations are described, *e.g.,* in U.S. Provisional Serial No. 61/244,834, filed September 22, 2009, U.S. Provisional Serial No. 61/185,800, filed June 10, 2009, and International Application No. PCT/US10/28224, filed June 10, 2010, which are hereby incorporated by reference.

ALNY-100 comprising formulations are described, *e.g.,* International patent application number PCT/US09/63933, filed on November 10, 2009, which is hereby incorporated by reference.

C12-200 comprising formulations are described in U.S. Provisional Serial No. 61/175,770, filed May 5, 2009 and International Application No. PCT/US10/33777, filed May 5, 2010, which are hereby incorporated by reference.

*C. Synthesis of cationic lipids*

Any of the compounds, *e.g.,* cationic lipids and the like, used in the nucleic acid-lipid particles featured in the disclosure may be prepared by known organic synthesis techniques. All substituents are as defined below unless indicated otherwise.

"Alkyl" means a straight chain or branched, noncyclic or cyclic, saturated aliphatic hydrocarbon containing from 1 to 24 carbon atoms. Representative saturated straight chain alkyls include methyl, ethyl, n-propyl, n-butyl, n-pentyl, n-hexyl, and the like; while saturated branched alkyls include isopropyl, *sec*-butyl, isobutyl, *tert*-butyl, isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like.

"Alkenyl" means an alkyl, as defined above, containing at least one double bond between adjacent carbon atoms.

Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Acyl" means any alkyl, alkenyl, or alkynyl wherein the carbon at the point of attachment is substituted with an oxo group, as defined below. For example, -C(=O)alkyl, -C(=O)alkenyl, and -C(=O)alkynyl are acyl groups.

"Heterocycle" means a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined below. Heterocycles include morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" means that, when substituted, at least one hydrogen atom is replaced with a substituent. In the case of an oxo substituent (=O) two hydrogen atoms are replaced. In this regard, substituents include oxo, halogen, heterocycle, -CN, - $OR^x$, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^y$, wherein n is 0, 1 or 2, $R^x$ and $R^y$ are the same or different and independently hydrogen, alkyl or heterocycle, and each of said alkyl and heterocycle substituents may be further substituted with one or more of oxo, halogen, -OH, -CN, alkyl, $-OR^x$ heterocycle, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^y$.

"Halogen" means fluoro, chloro, bromo and iodo.

In some embodiments, the methods featured in the disclosure may require the use of protecting groups. Protecting group methodology is well known to those skilled in the art (*see, for example,* PROTECTIVE GROUPS IN ORGANIC SYNTHESIS, Green, T.W. et al., Wiley-Interscience, New York City, 1999). Briefly, protecting groups within the context of this disclosure are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

Synthesis of Formula A

In some embodiments, nucleic acid-lipid particles featured in the disclosure are formulated using a cationic lipid of formula A:

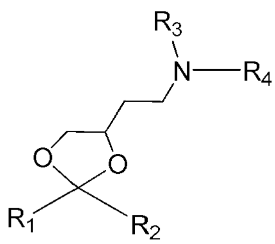

,

where R1 and R2 are independently alkyl, alkenyl or alkynyl, each can be optionally substituted, and R3 and R4 are independently lower alkyl or R3 and R4 can be taken together to form an optionally substituted heterocyclic ring. In some embodiments, the cationic lipid is XTC (2,2-Dilinoleyl-4-dimethylaminoethyl-[1,3]-dioxolane). In general, the lipid of formula A above may be made by the following Reaction Schemes 1 or 2, wherein all substituents are as defined above unless indicated otherwise.

Scheme 1

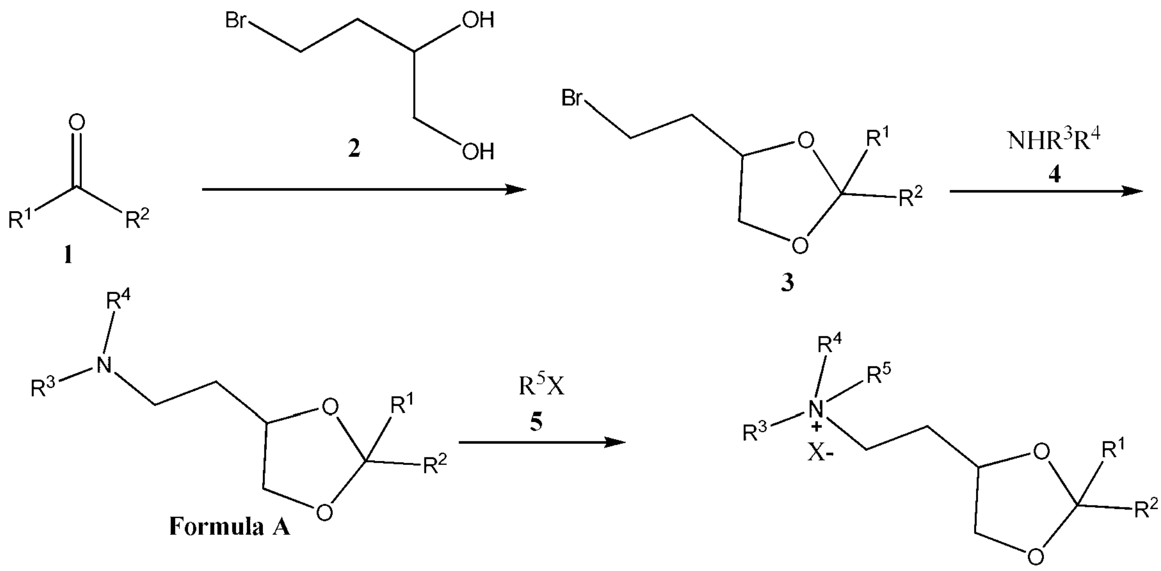


Lipid A, where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally substituted heterocyclic ring, can be prepared according to Scheme 1. Ketone 1 and bromide 2 can be purchased or prepared according to methods known to those of ordinary skill in the art. Reaction of 1 and 2 yields ketal 3. Treatment of ketal 3 with amine 4 yields lipids of formula A. The lipids of formula A can be converted to the corresponding ammonium salt with an organic salt of formula 5, where X is anion counter ion selected from halogen, hydroxide, phosphate, sulfate, or the like.

Scheme 2

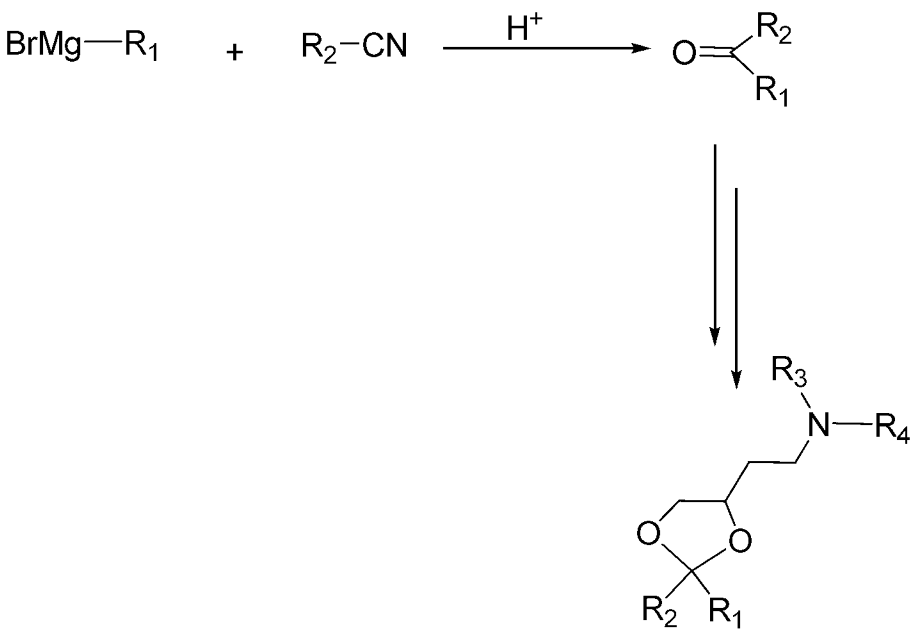


Alternatively, the ketone 1 starting material can be prepared according to Scheme 2. Grignard reagent 6 and cyanide 7 can be purchased or prepared according to methods known to those of ordinary skill in the art. Reaction of 6 and 7 yields ketone 1. Conversion of ketone 1 to the corresponding lipids of formula A is as described in Scheme 1.

Synthesis of MC3:

Preparation of DLin-M-C3-DMA (*i.e.,* (6Z,9Z,28Z,31Z)-heptatriaconta-6,9,28,31-tetraen-19-yl 4-(dimethylamino)butanoate) was as follows. A solution of (6Z,9Z,28Z,31Z)-heptatriaconta-6,9,28,31-tetraen-19-ol (0.53 g), 4-N,N-dimethylaminobutyric acid hydrochloride (0.51 g), 4-N,N-dimethylaminopyridine (0.61g) and 1-ethyl-3-(3-dimethylaminopropyl)carbodiimide hydrochloride (0.53 g) in dichloromethane (5 mL) was stirred at room temperature overnight. The solution was washed with dilute hydrochloric acid followed by dilute aqueous sodium bicarbonate. The organic fractions were dried over anhydrous magnesium sulphate, filtered and the solvent removed on a rotovap. The residue was passed down a silica gel column (20 g) using a 1-5% methanol/dichloromethane elution gradient. Fractions containing the purified product were combined and the solvent removed, yielding a colorless oil (0.54 g).

Synthesis of ALNY-100:

Synthesis of ketal 519 [ALNY-100] was performed using the following scheme 3:

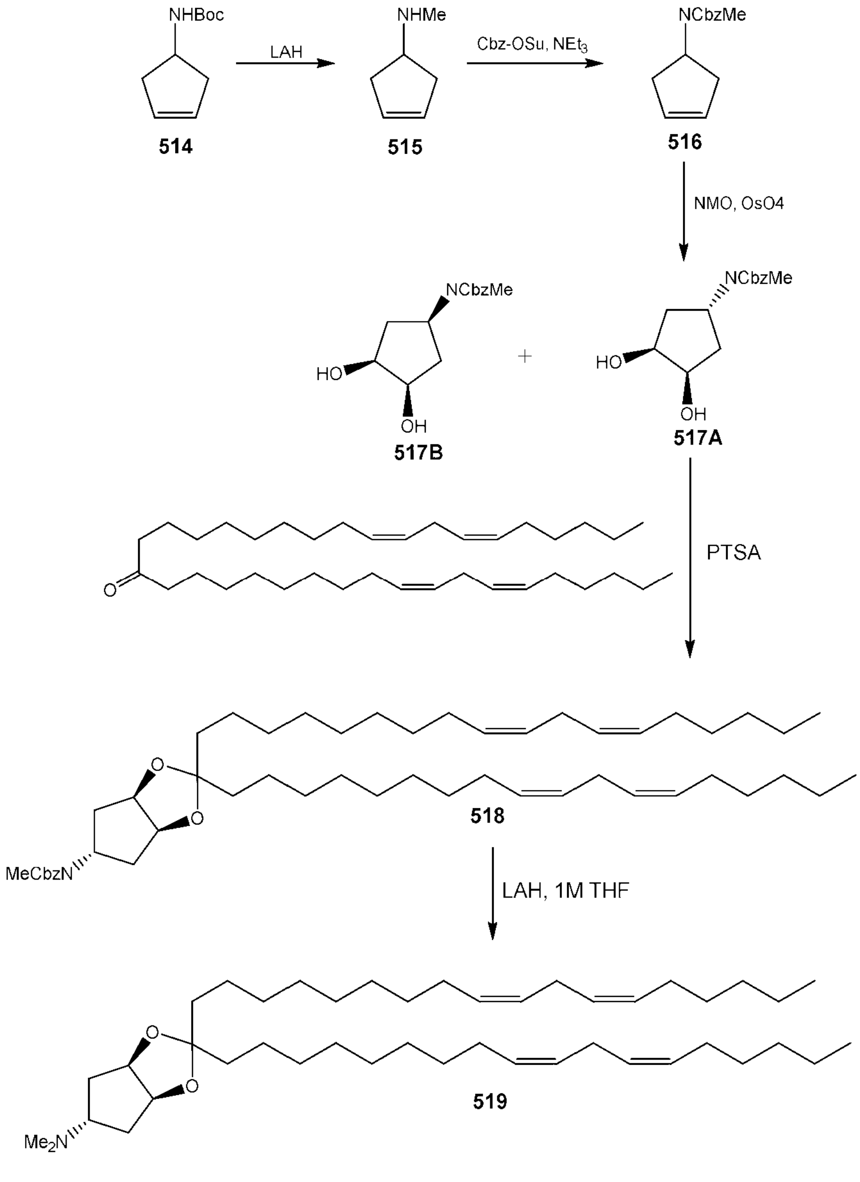


Synthesis of 515:

To a stirred suspension of LiAlH4 (3.74 g, 0.09852 mol) in 200 ml anhydrous THF in a two neck RBF (1L), was added a solution of 514 (10g, 0.04926mol) in 70 mL of THF slowly at 0 0C under nitrogen atmosphere. After complete addition, reaction mixture was warmed to room temperature and then heated to reflux for 4 h. Progress of the reaction was monitored by TLC. After completion of reaction (by TLC) the mixture was cooled to 0 0C and quenched with careful addition of saturated Na2SO4 solution. Reaction mixture was stirred for 4 h at room temperature and filtered off. Residue was washed well with THF. The filtrate and washings were mixed and diluted with 400 mL dioxane and 26 mL conc. HCl and

stirred for 20 minutes at room temperature. The volatilities were stripped off under vacuum to furnish the hydrochloride salt of 515 as a white solid. Yield: 7.12 g 1H-NMR (DMSO, 400MHz): $\delta$= 9.34 (broad, 2H), 5.68 (s, 2H), 3.74 (m, 1H), 2.66-2.60 (m, 2H), 2.50-2.45 (m, 5H).

Synthesis of 516:

To a stirred solution of compound 515 in 100 mL dry DCM in a 250 mL two neck RBF, was added NEt3 (37.2 mL, 0.2669 mol) and cooled to 0 0C under nitrogen atmosphere. After a slow addition of N-(benzyloxy-carbonyloxy)-succinimide (20 g, 0.08007 mol) in 50 mL dry DCM, reaction mixture was allowed to warm to room temperature. After completion of the reaction (2-3 h by TLC) mixture was washed successively with 1N HCl solution (1 x 100 mL) and saturated NaHCO3 solution (1 x 50 mL). The organic layer was then dried over anhyd. Na2SO4 and the solvent was evaporated to give crude material which was purified by silica gel column chromatography to get 516 as sticky mass. Yield: 11g (89%). 1H-NMR (CDCl3, 400MHz): $\delta$ = 7.36-7.27(m, 5H), 5.69 (s, 2H), 5.12 (s, 2H), 4.96 (br., 1H) 2.74 (s, 3H), 2.60(m, 2H), 2.30-2.25(m, 2H). LC-MS [M+H] -232.3 (96.94%).

Synthesis of 517A and 517B:

The cyclopentene 516 (5 g, 0.02164 mol) was dissolved in a solution of 220 mL acetone and water (10: 1) in a single neck 500 mL RBF and to it was added N-methyl morpholine-N-oxide (7.6 g, 0.06492 mol) followed by 4.2 mL of 7.6% solution of OsO4 (0.275 g, 0.00108 mol) in tert-butanol at room temperature. After completion of the reaction (~ 3 h), the mixture was quenched with addition of solid Na2SO3 and resulting mixture was stirred for 1.5 h at room temperature. Reaction mixture was diluted with DCM (300 mL) and washed with water (2 x 100 mL) followed by saturated NaHCO3 (1 x 50 mL) solution, water (1 x 30 mL) and finally with brine (1x 50 mL). Organic phase was dried over an.Na2SO4 and solvent was removed in vacuum. Silica gel column chromatographic purification of the crude material was afforded a mixture of diastereomers, which were separated by prep HPLC. Yield: - 6 g crude 517A - Peak-1 (white solid), 5.13 g (96%). 1H-NMR (DMSO, 400MHz): $\delta$= 7.39-7.31(m, 5H), 5.04(s, 2H), 4.78-4.73 (m, 1H), 4.48-4.47(d, 2H), 3.94-3.93(m, 2H), 2.71(s, 3H), 1.72- 1.67(m, 4H). LC-MS - [M+H]-266.3, [M+NH4 +]-283.5 present, HPLC-97.86%. Stereochemistry confirmed by X-ray.

Synthesis of 518:

Using a procedure analogous to that described for the synthesis of compound 505, compound 518 (1.2 g, 41%) was obtained as a colorless oil. 1H-NMR (CDCl3, 400MHz): $\delta$= 7.35-7.33(m, 4H), 7.30-7.27(m, 1H), 5.37-5.27(m, 8H), 5.12(s, 2H), 4.75(m,1H), 4.58-4.57(m,2H), 2.78-2.74(m,7H), 2.06-2.00(m,8H), 1.96-1.91(m, 2H), 1.62(m, 4H), 1.48(m, 2H), 1.37-1.25(br m, 36H), 0.87(m, 6H). HPLC-98.65%.

General Procedure for the Synthesis of Compound 519:

A solution of compound 518 (1 eq) in hexane (15 mL) was added in a drop-wise fashion to an ice-cold solution of LAH in THF (1 M, 2 eq). After complete addition, the mixture was heated at 40°C over 0.5 h then cooled again on an ice bath. The mixture was carefully hydrolyzed with saturated aqueous Na2SO4 then filtered through celite and reduced to an oil. Column chromatography provided the pure 519 (1.3 g, 68%) which was obtained as a colorless oil. 13C NMR = 130.2, 130.1 (x2), 127.9 (x3), 112.3, 79.3, 64.4, 44.7, 38.3, 35.4, 31.5, 29.9 (x2), 29.7, 29.6 (x2), 29.5 (x3), 29.3 (x2), 27.2 (x3), 25.6, 24.5, 23.3, 226, 14.1; Electrospray MS (+ve): Molecular weight for C44H80NO2 (M + H)+ Calc. 654.6, Found 654.6.

Formulations prepared by either the standard or extrusion-free method can be characterized in similar manners. For example, formulations are typically characterized by visual inspection. They should be whitish translucent solutions free from aggregates or sediment. Particle size and particle size distribution of lipid-nanoparticles can be measured by light scattering using, for example, a Malvern Zetasizer Nano ZS (Malvern, USA). Particles should be about 20-300 nm, such as 40-100 nm in size. The particle size distribution should be unimodal. The total dsRNA concentration in the formulation, as well as the entrapped fraction, is estimated using a dye exclusion assay. A sample of the formulated dsRNA can be incubated with an RNA-binding dye, such as Ribogreen (Molecular Probes) in the presence or absence of a formulation disrupting surfactant, *e.g.,* 0.5% Triton-X100. The total dsRNA in the formulation can be determined by the signal from the sample containing the surfactant, relative to a standard curve. The entrapped fraction is determined by subtracting the "free" dsRNA content (as measured by the signal in the absence of surfactant) from the total dsRNA content. Percent entrapped dsRNA is typically >85%. For SNALP formulation, the particle size is at least 30 nm, at least 40 nm, at least 50 nm, at least 60 nm, at least 70 nm, at least 80 nm, at least 90 nm, at least 100 nm, at least 110 nm, and at least 120 nm. The suitable range is typically about at least 50 nm to about at least 110 nm, about at least 60 nm to about at least 100 nm, or about at least 80 nm to about at least 90 nm.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. In some embodiments, oral formulations are those in which dsRNAs featured in the disclosure are administered in conjunction with one or more penetration enhancers surfactants and chelators. Suitable surfactants include fatty acids and/or esters or salts thereof, bile acids and/or salts thereof. Suitable bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Suitable fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (e.g., sodium). In some embodiments, combinations of penetration enhancers are used, for example, fatty acids/salts in combination with bile acids/salts. One exemplary combination is the sodium salt of lauric acid, capric acid and UDCA. Further penetration enhancers include polyoxyethylene-9-lauryl ether, polyoxyethylene-20-cetyl ether. DsRNAs featured in the disclosure may be delivered orally, in granular form including sprayed dried particles, or complexed to form micro or nanoparticles. DsRNA complexing agents include poly-amino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Suitable complexing agents include chitosan, N-trimethylchitosan, poly-L-lysine, polyhistidine, polyornithine, polyspermines, protamine, polyvinylpyridine, polythiodiethylaminomethylethylene P(TDAE), polyaminostyrene (e.g., p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. Patent 6,887,906, US Publn. No. 20030027780, and U.S. Patent No. 6,747,014, each of which is incorporated herein by reference.

Compositions and formulations for parenteral, intraparenchymal (into the brain), intrathecal, intravitreal, subretinal, transscleral, subconjunctival, retrobulbar, intracameral, intraventricular, or intrahepatic administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present disclosure include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations featured in the present disclosure, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions featured in the present disclosure may be formulated into any of many possible dosage forms such as, but not limited to, tablets, capsules, gel capsules, liquid syrups, soft gels, suppositories, and enemas. The compositions may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

*D. Additional Formulations*

*i. Emulsions*

The compositions of the present disclosure may be prepared and formulated as emulsions. Emulsions are typically heterogeneous systems of one liquid dispersed in another in the form of droplets usually exceeding 0.1μm in diameter (*see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199; Rosoff, in Pharmaceutical Dosage Forms, Lieberman, Rieger and Banker (Eds.), 1988, Marcel Dekker, Inc., New York, N.Y., Volume 1, p. 245; Block in Pharmaceutical Dosage Forms, Lieberman, Rieger and Banker (Eds.), 1988, Marcel Dekker, Inc., New York, N.Y., volume 2, p. 335; Higuchi et al., in Remington's Pharmaceutical Sciences, Mack

Publishing Co., Easton, Pa., 1985, p. 301). Emulsions are often biphasic systems comprising two immiscible liquid phases intimately mixed and dispersed with each other. In general, emulsions may be of either the water-in-oil (w/o) or the oil-in-water (o/w) variety. When an aqueous phase is finely divided into and dispersed as minute droplets into a bulk

oily phase, the resulting composition is called a water-in-oil (w/o) emulsion. Alternatively, when an oily phase is finely divided into and dispersed as minute droplets into a bulk aqueous phase, the resulting composition is called an oil-in-water (o/w) emulsion. Emulsions may contain additional components in addition to the dispersed phases, and the active drug which may be present as a solution in either the aqueous phase, oily phase or itself as a separate phase. Pharmaceutical excipients such as emulsifiers, stabilizers, dyes, and anti-oxidants may also be present in emulsions as needed. Pharmaceutical emulsions may also be multiple emulsions that are comprised of more than two phases such as, for example, in the case of oil-in-water-in-oil (o/w/o) and water-in-oil-in-water (w/o/w) emulsions. Such complex formulations often provide certain advantages that simple binary emulsions do not. Multiple emulsions in which individual oil droplets of an o/w emulsion enclose small water droplets constitute a w/o/w emulsion. Likewise, a system of oil droplets enclosed in globules of water stabilized in an oily continuous phase provides an o/w/o emulsion.

Emulsions are characterized by little or no thermodynamic stability. Often, the dispersed or discontinuous phase of the emulsion is well dispersed into the external or continuous phase and maintained in this form through the means of emulsifiers or the viscosity of the formulation. Either of the phases of the emulsion may be a semisolid or a solid, as is the case of emulsion-style ointment bases and creams. Other means of stabilizing emulsions entail the use of emulsifiers that may be incorporated into either phase of the emulsion. Emulsifiers may broadly be classified into four categories: synthetic surfactants, naturally occurring emulsifiers, absorption bases, and finely dispersed solids *(see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199).

Synthetic surfactants, also known as surface active agents, have found wide applicability in the formulation of emulsions and have been reviewed in the literature (*see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Rieger, in Pharmaceutical Dosage Forms, Lieberman, Rieger and Banker (Eds.), 1988, Marcel Dekker, Inc., New York, N.Y., volume 1, p. 285; Idson, in Pharmaceutical Dosage Forms, Lieberman, Rieger and Banker (Eds.), Marcel Dekker, Inc., New York, N.Y., 1988, volume 1, p. 199). Surfactants are typically amphiphilic and comprise a hydrophilic and a hydrophobic portion. The ratio of the hydrophilic to the hydrophobic nature of the surfactant has been termed the hydrophile/lipophile balance (HLB) and is a valuable tool in categorizing and selecting surfactants in the preparation of formulations. Surfactants may be classified into different classes based on the nature of the hydrophilic group: nonionic, anionic, cationic and amphoteric *(see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY Rieger, in Pharmaceutical Dosage Forms, Lieberman, Rieger and Banker (Eds.), 1988, Marcel Dekker, Inc., New York, N.Y., volume 1, p. 285).

Naturally occurring emulsifiers used in emulsion formulations include lanolin, beeswax, phosphatides, lecithin and acacia. Absorption bases possess hydrophilic properties such that they can soak up water to form w/o emulsions yet retain their semisolid consistencies, such as anhydrous lanolin and hydrophilic petrolatum. Finely divided solids have also been used as good emulsifiers especially in combination with surfactants and in viscous preparations. These include polar inorganic solids, such as heavy metal hydroxides, nonswelling clays such as bentonite, attapulgite, hectorite, kaolin, montmorillonite, colloidal aluminum silicate and colloidal magnesium aluminum silicate, pigments and nonpolar solids such as carbon or glyceryl tristearate.

A large variety of non-emulsifying materials are also included in emulsion formulations and contribute to the properties of emulsions. These include fats, oils, waxes, fatty acids, fatty alcohols, fatty esters, humectants, hydrophilic colloids, preservatives and antioxidants (Block, in Pharmaceutical Dosage Forms, Lieberman, Rieger and Banker (Eds.), 1988, Marcel Dekker, Inc., New York, N.Y., volume 1, p. 335; Idson, in Pharmaceutical Dosage Forms, Lieberman, Rieger and Banker (Eds.), 1988, Marcel Dekker, Inc., New York, N.Y., volume 1, p. 199).

Hydrophilic colloids or hydrocolloids include naturally occurring gums and synthetic polymers such as polysaccharides (for example, acacia, agar, alginic acid, carrageenan, guar gum, karaya gum, and tragacanth), cellulose derivatives (for example, carboxymethylcellulose and carboxypropylcellulose), and synthetic polymers (for example, carbomers, cellulose ethers, and carboxyvinyl polymers). These disperse or swell in water to form colloidal solutions that stabilize emulsions by forming strong interfacial films around the dispersed-phase droplets and by increasing the viscosity of the external phase.

Since emulsions often contain a number of ingredients such as carbohydrates, proteins, sterols and phosphatides that may readily support the growth of microbes, these formulations often incorporate preservatives. Commonly used preservatives included in emulsion formulations include methyl paraben, propyl paraben, quaternary ammonium salts, benzalkonium chloride, esters of p-hydroxybenzoic acid, and boric acid. Antioxidants are also commonly added to emulsion formulations to prevent deterioration of the formulation. Antioxidants used may be free radical scavengers such as tocopherols, alkyl gallates, butylated hydroxyanisole, butylated hydroxytoluene, or reducing agents such as ascorbic acid and sodium metabisulfite, and antioxidant synergists such as citric acid, tartaric acid, and lecithin.

The application of emulsion formulations via dermatological, oral and parenteral routes and methods for their manufacture have been reviewed in the literature *(see e.g.,* Ansel's Pharmaceutical Dosage Forms and Drug Delivery

Systems, Allen, LV., Popovich NG., and Ansel HC., 2004, Lippincott Williams & Wilkins (8th ed.), New York, NY; Idson, in Pharmaceutical Dosage Forms, Lieberman, Rieger and Banker (Eds.), 1988, Marcel Dekker, Inc., New York, N.Y., volume 1, p. 199). Emulsion formulations for oral delivery have been very widely used because of ease of formulation, as well as efficacy from an absorption and bioavailability standpoint (*see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Idson, in Pharmaceutical Dosage Forms, Lieberman, Rieger and Banker (Eds.), 1988, Marcel Dekker, Inc., New York, N.Y., volume 1, p. 199). Mineral-oil base laxatives, oil-soluble vitamins and high fat nutritive preparations are among the materials that have commonly been administered orally as o/w emulsions.

*ii. Microemulsions*

In some embodiments of the present disclosure, the compositions of iRNAs and nucleic acids are formulated as microemulsions. A microemulsion may be defined as a system of water, oil and amphiphile which is a single optically isotropic and thermodynamically stable liquid solution *(see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245). Typically, microemulsions are systems that are prepared by first dispersing an oil in an aqueous surfactant solution and then adding a sufficient amount of a fourth component, generally an intermediate chain-length alcohol to form a transparent system. Therefore, microemulsions have also been described as thermodynamically stable, isotopically clear dispersions of two immiscible liquids that are stabilized by interfacial films of surface-active molecules (Leung and Shah, in: Controlled Release of Drugs: Polymers and Aggregate Systems, Rosoff, M., Ed., 1989, VCH Publishers, New York, pages 185-215). Microemulsions commonly are prepared via a combination of three to five components that include oil, water, surfactant, cosurfactant and electrolyte. Whether the microemulsion is of the water-in-oil (w/o) or an oil-in-water (o/w) type is dependent on the properties of the oil and surfactant used and on the structure and geometric packing of the polar heads and hydrocarbon tails of the surfactant molecules (Schott, in Remington's Pharmaceutical Sciences, Mack Publishing Co., Easton, Pa., 1985, p. 271).

The phenomenological approach utilizing phase diagrams has been extensively studied and has yielded a comprehensive knowledge, to one skilled in the art, of how to formulate microemulsions (*see e.g.,* Ansel's Pharmaceutical Dosage Forms and Drug Delivery Systems, Allen, LV., Popovich NG., and Ansel HC., 2004, Lippincott Williams & Wilkins (8th ed.), New York, NY; Rosoff, in Pharmaceutical Dosage Forms, Lieberman, Rieger and Banker (Eds.), 1988, Marcel Dekker, Inc., New York, N.Y., volume 1, p. 245; Block, in Pharmaceutical Dosage Forms, Lieberman, Rieger and Banker (Eds.), 1988, Marcel Dekker, Inc., New York, N.Y., volume 1, p. 335). Compared to conventional emulsions, microemulsions offer the advantage of solubilizing water-insoluble drugs in a formulation of thermodynamically stable droplets that are formed spontaneously.

Surfactants used in the preparation of microemulsions include, but are not limited to, ionic surfactants, non-ionic surfactants, Brij 96, polyoxyethylene oleyl ethers, polyglycerol fatty acid esters, tetraglycerol monolaurate (ML310), tetraglycerol monooleate (MO310), hexaglycerol monooleate (PO310), hexaglycerol pentaoleate (PO500), decaglycerol monocaprate (MCA750), decaglycerol monooleate (MO750), decaglycerol sequioleate (SO750), decaglycerol decaoleate (DAO750), alone or in combination with cosurfactants. The cosurfactant, usually a short-chain alcohol such as ethanol, 1-propanol, and 1-butanol, serves to increase the interfacial fluidity by penetrating into the surfactant film and consequently creating a disordered film because of the void space generated among surfactant molecules. Microemulsions may, however, be prepared without the use of cosurfactants and alcohol-free self-emulsifying microemulsion systems are known in the art. The aqueous phase may typically be, but is not limited to, water, an aqueous solution of the drug, glycerol, PEG300, PEG400, polyglycerols, propylene glycols, and derivatives of ethylene glycol. The oil phase may include, but is not limited to, materials such as Captex 300, Captex 355, Capmul MCM, fatty acid esters, medium chain (C8-C12) mono, di, and tri-glycerides, polyoxyethylated glyceryl fatty acid esters, fatty alcohols, polyglycolized glycerides, saturated polyglycolized C8-C10 glycerides, vegetable oils and silicone oil.

Microemulsions are particularly of interest from the standpoint of drug solubilization and the enhanced absorption of drugs. Lipid based microemulsions (both o/w and w/o) have been proposed to enhance the oral bioavailability of drugs, including peptides *(see e.g.,* U.S. Patent Nos. 6,191,105; 7,063,860; 7,070,802; 7,157,099; Constantinides et al., Pharmaceutical Research, 1994, 11, 1385-1390; Ritschel, Meth. Find. Exp. Clin. Pharmacol., 1993, 13, 205). Microemulsions afford advantages of improved drug solubilization, protection of drug from enzymatic hydrolysis, possible enhancement of drug absorption due to surfactant-induced alterations in membrane fluidity and permeability, ease of preparation, ease of oral administration over solid dosage forms, improved clinical potency, and decreased toxicity *(see e.g.,* U.S. Patent Nos. 6,191,105; 7,063,860; 7,070,802; 7,157,099; Constantinides et al., Pharmaceutical Research, 1994, 11, 1385; Ho et al., J. Pharm. Sci., 1996, 85, 138-143). Often microemulsions may form spontaneously when their components are brought together at ambient temperature. This may be particularly advantageous when formulating

thermolabile drugs, peptides or iRNAs. Microemulsions have also been effective in the transdermal delivery of active components in both cosmetic and pharmaceutical applications. It is expected that the microemulsion compositions and formulations of the present disclosure will facilitate the increased systemic absorption of iRNAs and nucleic acids from the gastrointestinal tract, as well as improve the local cellular uptake of iRNAs and nucleic acids.

Microemulsions of the present disclosure may also contain additional components and additives such as sorbitan monostearate (Grill 3), Labrasol, and penetration enhancers to improve the properties of the formulation and to enhance the absorption of the iRNAs and nucleic acids of the present disclosure. Penetration enhancers used in the microemulsions of the present disclosure may be classified as belonging to one of five broad categories--surfactants, fatty acids, bile salts, chelating agents, and non-chelating non-surfactants (Lee et al., Critical Reviews in Therapeutic Drug Carrier Systems, 1991, p. 92). Each of these classes has been discussed above.

*iii. Penetration Enhancers*

In some embodiments, the present disclosure employs various penetration enhancers to effect the efficient delivery of nucleic acids, particularly iRNAs, to the skin of animals. Most drugs are present in solution in both ionized and nonionized forms. However, usually only lipid soluble or lipophilic drugs readily cross cell membranes. It has been discovered that even non-lipophilic drugs may cross cell membranes if the membrane to be crossed is treated with a penetration enhancer. In addition to aiding the diffusion of non-lipophilic drugs across cell membranes, penetration enhancers also enhance the permeability of lipophilic drugs.

Penetration enhancers may be classified as belonging to one of five broad categories, *i.e.,* surfactants, fatty acids, bile salts, chelating agents, and non-chelating non-surfactants (*see e.g.,* Malmsten, M. Surfactants and polymers in drug delivery, Informa Health Care, New York, NY, 2002; Lee et al., Critical Reviews in Therapeutic Drug Carrier Systems, 1991, p.92). Each of the above-mentioned classes of penetration enhancers are described below in greater detail.

*Surfactants:* In connection with the present disclosure, surfactants (or "surface-active agents") are chemical entities which, when dissolved in an aqueous solution, reduce the surface tension of the solution or the interfacial tension between the aqueous solution and another liquid, with the result that absorption of iRNAs through the mucosa is enhanced. In addition to bile salts and fatty acids, these penetration enhancers include, for example, sodium lauryl sulfate, polyoxyethylene-9-lauryl ether and polyoxyethylene-20-cetyl ether) *(see e.g.,* Malmsten, M. Surfactants and polymers in drug delivery, Informa Health Care, New York, NY, 2002; Lee et al., Critical Reviews in Therapeutic Drug Carrier Systems, 1991, p.92); and perfluorochemical emulsions, such as FC-43. Takahashi et al., J. Pharm. Pharmacol., 1988, 40, 252).

*Fatty acids:* Various fatty acids and their derivatives which act as penetration enhancers include, for example, oleic acid, lauric acid, capric acid (n-decanoic acid), myristic acid, palmitic acid, stearic acid, linoleic acid, linolenic acid, dicaprate, tricaprate, monoolein (1-monooleoyl-rac-glycerol), dilaurin, caprylic acid, arachidonic acid, glycerol 1-monocaprate, 1-dodecylazacycloheptan-2-one, acylcarnitines, acylcholines, $C_{1-20}$ alkyl esters thereof (e.g., methyl, isopropyl and t-butyl), and mono- and di-glycerides thereof (*i.e.,* oleate, laurate, caprate, myristate, palmitate, stearate, linoleate, *etc.*) (*see e.g.,* Touitou, E., et al. Enhancement in Drug Delivery, CRC Press, Danvers, MA, 2006; Lee et al., Critical Reviews in Therapeutic Drug Carrier Systems, 1991, p.92; Muranishi, Critical Reviews in Therapeutic Drug Carrier Systems, 1990, 7, 1-33; El Hariri et al., J. Pharm. Pharmacol., 1992, 44, 651-654).

*Bile salts:* The physiological role of bile includes the facilitation of dispersion and absorption of lipids and fat-soluble vitamins *(see e.g.,* Malmsten, M. Surfactants and polymers in drug delivery, Informa Health Care, New York, NY, 2002; Brunton, Chapter 38 in: Goodman & Gilman's The Pharmacological Basis of Therapeutics, 9th Ed., Hardman et al. Eds., McGraw-Hill, New York, 1996, pp. 934-935). Various natural bile salts, and their synthetic derivatives, act as penetration enhancers. Thus, the term "bile salts" includes any of the naturally occurring components of bile as well as any of their synthetic derivatives. Suitable bile salts include, for example, cholic acid (or its pharmaceutically acceptable sodium salt, sodium cholate), dehydrocholic acid (sodium dehydrocholate), deoxycholic acid (sodium deoxycholate), glucholic acid (sodium glucholate), glycholic acid (sodium glycocholate), glycodeoxycholic acid (sodium glycodeoxycholate), taurocholic acid (sodium taurocholate), taurodeoxycholic acid (sodium taurodeoxycholate), chenodeoxycholic acid (sodium chenodeoxycholate), ursodeoxycholic acid (UDCA), sodium tauro-24,25-dihydro-fusidate (STDHF), sodium glycodihydrofusidate and polyoxyethylene-9-lauryl ether (POE) (*see e.g.,* Malmsten, M. Surfactants and polymers in drug delivery, Informa Health Care, New York, NY, 2002; Lee et al., Critical Reviews in Therapeutic Drug Carrier Systems, 1991, page 92; Swinyard, Chapter 39 In: Remington's Pharmaceutical Sciences, 18th Ed., Gennaro, ed., Mack Publishing Co., Easton, Pa., 1990, pages 782-783; Muranishi, Critical Reviews in Therapeutic Drug Carrier Systems, 1990, 7, 1-33; Yamamoto et al., J. Pharm. Exp. Ther., 1992, 263, 25; Yamashita et al., J. Pharm. Sci., 1990, 79, 579-583).

*Chelating Agents:* Chelating agents, as used in connection with the present disclosure, can be defined as compounds that remove metallic ions from solution by forming complexes therewith, with the result that absorption of iRNAs through the mucosa is enhanced. With regards to their use as penetration enhancers in the present disclosure, chelating agents have the added advantage of also serving as DNase inhibitors, as most characterized DNA nucleases

require a divalent metal ion for catalysis and are thus inhibited by chelating agents (Jarrett, J. Chromatogr., 1993, 618, 315-339). Suitable chelating agents include but are not limited to disodium ethylenediaminetetraacetate (EDTA), citric acid, salicylates (e.g., sodium salicylate, 5-methoxysalicylate and homovanilate), N-acyl derivatives of collagen, laureth-9 and N-amino acyl derivatives of β-diketones (enamines)(see *e.g.,* Katdare, A. et al., Excipient development for pharmaceutical, biotechnology, and drug delivery, CRC Press, Danvers, MA, 2006; Lee et al., Critical Reviews in Therapeutic Drug Carrier Systems, 1991, page 92; Muranishi, Critical Reviews in Therapeutic Drug Carrier Systems, 1990, 7, 1-33; Buur et al., J. Control Rel., 1990, 14, 43-51).

*Non-chelating non-surfactants:* As used herein, non-chelating non-surfactant penetration enhancing compounds can be defined as compounds that demonstrate insignificant activity as chelating agents or as surfactants but that nonetheless enhance absorption of iRNAs through the alimentary mucosa (*see e.g.,* Muranishi, Critical Reviews in Therapeutic Drug Carrier Systems, 1990, 7, 1-33). This class of penetration enhancers include, for example, unsaturated cyclic ureas, 1-alkyl- and 1-alkenylazacyclo-alkanone derivatives (Lee et al., Critical Reviews in Therapeutic Drug Carrier Systems, 1991, page 92); and non-steroidal anti-inflammatory agents such as diclofenac sodium, indomethacin and phenylbutazone (Yamashita et al., J. Pharm. Pharmacol., 1987, 39, 621-626).

Agents that enhance uptake of iRNAs at the cellular level may also be added to the pharmaceutical and other compositions of the present disclosure. For example, cationic lipids, such as lipofectin (Junichi et al, U.S. Pat. No. 5,705,188), cationic glycerol derivatives, and polycationic molecules, such as polylysine (Lollo et al., PCT Application WO 97/30731), are also known to enhance the cellular uptake of dsRNAs. Examples of commercially available transfection reagents include, for example Lipofectamine™ (Invitrogen; Carlsbad, CA), Lipofectamine 2000™ (Invitrogen; Carlsbad, CA), 293fectin™ (Invitrogen; Carlsbad, CA), Cellfectin™ (Invitrogen; Carlsbad, CA), DMRIE-C™ (Invitrogen; Carlsbad, CA), FreeStyle™ MAX (Invitrogen; Carlsbad, CA), Lipofectamine™ 2000 CD (Invitrogen; Carlsbad, CA), Lipofectamine™ (Invitrogen; Carlsbad, CA), RNAiMAX (Invitrogen; Carlsbad, CA), Oligofectamine™ (Invitrogen; Carlsbad, CA), Optifect™ (Invitrogen; Carlsbad, CA), X-tremeGENE Q2 Transfection Reagent (Roche; Grenzacherstrasse, Switzerland), DOTAP Liposomal Transfection Reagent (Grenzacherstrasse, Switzerland), DOSPER Liposomal Transfection Reagent (Grenzacherstrasse, Switzerland), or Fugene (Grenzacherstrasse, Switzerland), Transfectam® Reagent (Promega; Madison, WI), TransFast™ Transfection Reagent (Promega; Madison, WI), Tfx™-20 Reagent (Promega; Madison, WI), Tfx™-50 Reagent (Promega; Madison, WI), DreamFect™ (OZ Biosciences; Marseille, France), EcoTransfect (OZ Biosciences; Marseille, France), TransPassª D1 Transfection Reagent (New England Biolabs; Ipswich, MA, USA), LyoVec™/LipoGen™ (Invivogen; San Diego, CA, USA), PerFectin Transfection Reagent (Genlantis; San Diego, CA, USA), NeuroPORTER Transfection Reagent (Genlantis; San Diego, CA, USA), GenePORTER Transfection reagent (Genlantis; San Diego, CA, USA), GenePORTER 2 Transfection reagent (Genlantis; San Diego, CA, USA), Cytofectin Transfection Reagent (Genlantis; San Diego, CA, USA), BaculoPORTER Transfection Reagent (Genlantis; San Diego, CA, USA), TroganPORTER™ transfection Reagent (Genlantis; San Diego, CA, USA), RiboFect (Bioline; Taunton, MA, USA), PlasFect (Bioline; Taunton, MA, USA), UniFECTOR (B-Bridge International; Mountain View, CA, USA), SureFECTOR (B-Bridge International; Mountain View, CA, USA), or HiFect™ (B-Bridge International, Mountain View, CA, USA), among others.

Other agents may be utilized to enhance the penetration of the administered nucleic acids, including glycols such as ethylene glycol and propylene glycol, pyrrols such as 2-pyrrol, azones, and terpenes such as limonene and menthone.

*iv. Carriers*

Certain compositions of the present disclosure also incorporate carrier compounds in the formulation. As used herein, "carrier compound" can refer to a nucleic acid, or analog thereof, which is inert (*i.e.,* does not possess biological activity per se) but is recognized as a nucleic acid by *in vivo* processes that reduce the bioavailability of a nucleic acid having biological activity by, for example, degrading the biologically active nucleic acid or promoting its removal from circulation. The coadministration of a nucleic acid and a carrier compound, typically with an excess of the latter substance, can result in a substantial reduction of the amount of nucleic acid recovered in the liver, kidney or other extracirculatory reservoirs, presumably due to competition between the carrier compound and the nucleic acid for a common receptor. For example, the recovery of a partially phosphorothioate dsRNA in hepatic tissue can be reduced when it is coadministered with polyinosinic acid, dextran sulfate, polycytidic acid or 4-acetamido-4'isothiocyano-stilbene-2,2'-disulfonic acid (Miyao et al., DsRNA Res. Dev., 1995, 5, 115-121; Takakura et al., DsRNA & Nucl. Acid Drug Dev., 1996, 6, 177-183).

*v. Excipients*

In contrast to a carrier compound, a pharmaceutical carrier or excipient may comprise, *e.g.,* a pharmaceutically acceptable solvent, suspending agent or any other pharmacologically inert vehicle for delivering one or more nucleic acids to an animal. The excipient may be liquid or solid and is selected, with the planned manner of administration in mind, so as to provide for the desired bulk, consistency, *etc.,* when combined with a nucleic acid and the other components of a given pharmaceutical composition. Typical pharmaceutical carriers include, but are not limited to, binding agents (*e.g.*,

pregelatinized maize starch, polyvinylpyrrolidone or hydroxypropyl methylcellulose, *etc*.); fillers (*e.g.,* lactose and other sugars, microcrystalline cellulose, pectin, gelatin, calcium sulfate, ethyl cellulose, polyacrylates or calcium hydrogen phosphate, *etc.*); lubricants (*e.g.,* magnesium stearate, talc, silica, colloidal silicon dioxide, stearic acid, metallic stearates, hydrogenated vegetable oils, corn starch, polyethylene glycols, sodium benzoate, sodium acetate, *etc*.); disintegrants (*e.g.,* starch, sodium starch glycolate, *etc*.); and wetting agents (*e.g.,* sodium lauryl sulphate, *etc*).

Pharmaceutically acceptable organic or inorganic excipients suitable for non-parenteral administration which do not deleteriously react with nucleic acids can also be used to formulate the compositions of the present disclosure. Suitable pharmaceutically acceptable carriers include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone and the like.

Formulations for topical administration of nucleic acids may include sterile and non-sterile aqueous solutions, non-aqueous solutions in common solvents such as alcohols, or solutions of the nucleic acids in liquid or solid oil bases. The solutions may also contain buffers, diluents and other suitable additives. Pharmaceutically acceptable organic or inorganic excipients suitable for non-parenteral administration which do not deleteriously react with nucleic acids can be used.

Suitable pharmaceutically acceptable excipients include, but are not limited to, water, salt solutions, alcohol, polyethylene glycols, gelatin, lactose, amylose, magnesium stearate, talc, silicic acid, viscous paraffin, hydroxymethylcellulose, polyvinylpyrrolidone and the like.

*vi. Other Components*

The compositions of the present disclosure may additionally contain other adjunct components conventionally found in pharmaceutical compositions, *e.g.,* at their art-established usage levels. Thus, for example, the compositions may contain additional, compatible, pharmaceutically-active materials such as, for example, antipruritics, astringents, local anesthetics or anti-inflammatory agents, or may contain additional materials useful in physically formulating various dosage forms of the compositions of the present disclosure, such as dyes, flavoring agents, preservatives, antioxidants, opacifiers, thickening agents and stabilizers. However, such materials, when added, should not unduly interfere with the biological activities of the components of the compositions of the present disclosure. The formulations can be sterilized and, if desired, mixed with auxiliary agents, *e.g.,* lubricants, preservatives, stabilizers, wetting agents, emulsifiers, salts for influencing osmotic pressure, buffers, colorings, flavorings and/or aromatic substances and the like which do not deleteriously interact with the nucleic acid(s) of the formulation.

Aqueous suspensions may contain substances that increase the viscosity of the suspension including, for example, sodium carboxymethylcellulose, sorbitol and/or dextran. The suspension may also contain stabilizers.

In some embodiments, pharmaceutical compositions featured in the disclosure include (a) one or more iRNA compounds and (b) one or more biologic agents which function by a non-RNAi mechanism. Examples of such biologic agents include agents that interfere with an interaction of CA2 and at least one CA2 binding partner.

Toxicity and therapeutic efficacy of such compounds can be determined by standard pharmaceutical procedures in cell cultures or experimental animals, *e.g.,* for determining the

LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit high therapeutic indices are typical.

The data obtained from cell culture assays and animal studies can be used in formulating a range of dosage for use in humans. The dosage of compositions featured in the disclosure lies generally within a range of circulating concentrations that include the ED50 with little or no toxicity. The dosage may vary within this range depending upon the dosage form employed and the route of administration utilized. For any compound used in the methods featured in the disclosure, the therapeutically effective dose can be estimated initially from cell culture assays. A dose may be formulated in animal models to achieve a circulating plasma concentration range of the compound or, when appropriate, of the polypeptide product of a target sequence (*e.g.*, achieving a decreased concentration of the polypeptide) that includes the IC50 (*i.e.,* the concentration of the test compound which achieves a half-maximal inhibition of symptoms) as determined in cell culture. Such information can be used to more accurately determine useful doses in humans. Levels in plasma may be measured, for example, by high performance liquid chromatography.

In addition to their administration, as discussed above, the iRNAs featured in the disclosure can be administered in combination with other known agents effective in treatment of diseases or disorders related to CA2 expression (*e.g.*, glaucoma or conditions associated with glaucoma). In any event, the administering physician can adjust the amount and timing of iRNA administration on the basis of results observed using standard measures of efficacy known in the art or described herein.

## VII. Methods of treating disorders related to expression of CA2

The present disclosure relates to the use of an iRNA targeting CA2 to inhibit CA2 expression and/or to treat a disease, disorder, or pathological process that is related to CA2 expression (*e.g.*, glaucoma or conditions associated with glaucoma).

In some aspects, a method of treatment of a disorder related to expression of CA2 is provided, the method comprising administering an iRNA (*e.g.,* a dsRNA) disclosed herein to a subject in need thereof. In some embodiments, the iRNA inhibits (decreases) CA2 expression.

In some embodiments, the subject is an animal that serves as a model for a disorder related to CA2 expression, *e.g.,* glaucoma or conditions associated with glaucoma.

### *A. Glaucoma or Conditions Associated with Glaucoma*

In some embodiments, the disorder related to CA2 expression is glaucoma or conditions associated with glaucoma. Non-limiting examples of glaucoma or conditions associated with glaucoma that are treatable using the methods described herein include glaucoma, open-angle glaucoma, angle-closure glaucoma, ocular inflammation, systemic inflammation, anterior uveitis, acute retinal necrosis, Sturge-Weber syndrome, Axenfeld-Rieger syndrome, Marfan syndrome, homocystinuria, Weill-Marchesani syndrome, and autoimmune diseases, such as juvenile rheumatoid arthritis and Marie-Strumpell ankylosing spondylitis.

Clinical and pathological features of glaucoma or conditions associated with glaucoma include, but are not limited to, intraocular pressure, vision loss, a reduction in visual acuity (*e.g.*, characterized by floating spots, blurriness around the edges or center of field of vision (*e.g.,* scotoma), ocular inflammation, and/or optic nerve damage.

In some embodiments, the subject with glaucoma or conditions associated with glaucoma is less than 18 years old. In some embodiments, the subject with glaucoma or conditions associated with glaucoma is an adult. In some embodiments, the subject has, or is identified as having, elevated levels of CA2 mRNA or protein relative to a reference level (e.g., a level of CA2 that is greater than a reference level).

In some embodiments, the glaucoma or conditions associated with glaucoma is diagnosed using analysis of a sample from the subject (*e.g.,* a ciliary epithelium sample). In some embodiments, the sample is analyzed using a method selected from one or more of: fluorescent in situ hybridization (FISH), immunohistochemistry, CA2 immunoassay, electron microscopy, laser microdissection, and mass spectrometry. In some embodiments, glaucoma or conditions associated with glaucoma is diagnosed using any suitable diagnostic test or technique, *e.g.,* tonometry, pachymetry, evaluation of the retina, gonioscopy, angiography *(e.g.,* fluorescein angiography or indocyanine green angiography), electroretinography, ultrasonography, optical coherence tomography (OCT), computed tomography (CT) and magnetic resonance imaging (MRI), color vision testing, visual field testing, slit-lamp examination, ophthalmoscopy, and physical examination (*e.g.*, to assess visual acuity (*e.g.*, by fundoscopy or optical coherence tomography (OCT)).

### *B. Combination Therapies*

In some embodiments, an iRNA (*e.g.,* a dsRNA) disclosed herein is administered in combination with a second therapy (*e.g.*, one or more additional therapies) known to be effective in treating a disorder related to CA2 expression (*e.g.*, glaucoma) or a symptom of such a disorder. The iRNA may be administered before, after, or concurrent with the second therapy. In some embodiments, the iRNA is administered before the second therapy. In some embodiments, the iRNA is administered after the second therapy. In some embodiments, the iRNA is administered concurrent with the second therapy.

The second therapy may be an additional therapeutic agent. The iRNA and the additional therapeutic agent can be administered in combination in the same composition or the additional therapeutic agent can be administered as part of a separate composition.

In some embodiments, the second therapy is a non-iRNA therapeutic agent that is effective to treat the disorder or symptoms of the disorder.

In some embodiments, the iRNA is administered in conjunction with a therapy. Exemplary combination therapies include, but are not limited to, medication to reduce intraocular pressure, laser treatment, surgery or trabeculectomy. In some embodiments, the additional therapeutic agent comprises a prostaglandin analog, a beta blocker, an alpha-adrenergic agonist, a carbonic anhydrase inhibitor, or an anti-CA2 agent.

In some embodiments, the additional therapeutic is a prostaglandin analog. In some embodiments, the prostaglandin analog comprises Bimatoprost (Lumigan®), Latanoprost (Xalatan®), Tafluprost (Zioptan™), latanoprostene bunod (Vyzulta™) or Travoprost (Travatan Z®).

In some embodiments, the additional therapeutic agent is a beta blocker. In some embodiments, the beta blocker comprises Betaxolol (Betoptic S®) or Timolol (Betimol®, Timoptic).

In some embodiments, the additional therapeutic agent is an alpha-adrenergic agonist. In some embodiments, the alpha-adrenergic agonist comprises brimonidine (Alphagan®P) or apraclonidine (Iopidine®).
In some embodiments, the additional therapeutic agent is a carbonic anhydrase inhibitor. In some embodiments, the carbonic anhydrase inhibitor comprises dorzolamide (Trusopt®), brinzolamide (Azopt®), acetazolamide (Diamox) or methazolamide (Neptazane®).
In some embodiments, the anti-CA2 agent is an antibody molecule. In some embodiments the antibody is a monoclonal antibody.

*C. Administration dosages, routes, and timing*

A subject (*e.g.,* a human subject, *e.g.,* a patient) can be administered a therapeutic amount of iRNA. The therapeutic amount can be, *e.g.,* 0.05-50 mg/kg.
In some embodiments, the iRNA is formulated for delivery to a target organ, *e.g.,* to the eye.
In some embodiments, the iRNA is formulated as a lipid formulation, *e.g.,* an LNP formulation as described herein. In some such embodiments, the therapeutic amount is 0.05-5 mg/kg dsRNA. In some embodiments, the lipid formulation, *e.g.,* LNP formulation, is administered intravenously.
In some embodiments, the iRNA is in the form of a GalNAc conjugate *e.g.,* as described herein. In some such embodiments, the therapeutic amount is 0.5-50 mg dsRNA. In some embodiments, the *e.g.,* GalNAc conjugate is administered subcutaneously.
In some embodiments, the iRNA is in the form of a C16 conjugate *e.g.,* as described herein.
In certain embodiments, subjects can be administered a therapeutic amount of dsRNA, such as about 0.01 mg/kg to about 200 mg/kg. In other embodiments, subjects can be administered a therapeutic amount of dsRNA, such as about 0.01 mg/kg to about 500 mg/kg. In yet other embodiments, subjects can be administered a therapeutic amount of dsRNA of about 500 mg/kg or more.
In some embodiments, the administration is repeated, for example, on a regular basis, such as, daily, biweekly (*i.e.,* every two weeks) for one month, two months, three months, four months, six months or longer. After an initial treatment regimen, the treatments can be administered on a less frequent basis. For example, after administration biweekly for three months, administration can be repeated once per month, for six months or a year or longer.
In some embodiments, the iRNA agent is administered in two or more doses. In some embodiments, the number or amount of subsequent doses is dependent on the achievement of a desired effect, *e.g.,* to (a) inhibit or reduce intraocular pressure; (b) inhibit or reduce the expression or activity of CA2; (c) decrease the amount of aqueous humor; (d) inhibit or reduce optic nerve damage; or (e) inhibit or reduce retinal ganglion cell death, or the achievement of a therapeutic or prophylactic effect, *e.g.,* reduction or prevention of one or more symptoms associated with the disorder.
In some embodiments, the iRNA agent is administered according to a schedule. For example, the iRNA agent may be administered once per week, twice per week, three times per week, four times per week, or five times per week. In some embodiments, the schedule involves regularly spaced administrations, *e.g.,* hourly, every four hours, every six hours, every eight hours, every twelve hours, daily, every 2 days, every 3 days, every 4 days, every 5 days, weekly, biweekly, or monthly. In some embodiments, the iRNA agent is administered at the frequency required to achieve a desired effect.
In some embodiments, the schedule involves closely spaced administrations followed by a longer period of time during which the agent is not administered. For example, the schedule may involve an initial set of doses that are administered in a relatively short period of time (*e.g.,* about every 6 hours, about every 12 hours, about every 24 hours, about every 48 hours, or about every 72 hours) followed by a longer time period (*e.g.,* about 1 week, about 2 weeks, about 3 weeks, about 4 weeks, about 5 weeks, about 6 weeks, about 7 weeks, or about 8 weeks) during which the iRNA agent is not administered. In some embodiments, the iRNA agent is initially administered hourly and is later administered at a longer interval (*e.g.,* daily, weekly, biweekly, or monthly). In some embodiments, the iRNA agent is initially administered daily and is later administered at a longer interval (*e.g.,* weekly, biweekly, or monthly). In certain embodiments, the longer interval increases over time or is determined based on the achievement of a desired effect.
Before administration of a full dose of the iRNA, patients can be administered a smaller dose, such as a 5% infusion dose, and monitored for adverse effects, such as an allergic reaction, or for elevated lipid levels or blood pressure. In another example, the patient can be monitored for unwanted effects.

## VIII. Methods for modulating expression of CA2

In some aspects, the disclosure provides a method for modulating (*e.g.,* inhibiting or activating) the expression of CA2, *e.g.,* in a cell, in a tissue, or in a subject. In some embodiments, the cell or tissue is *ex vivo, in vitro,* or *in vivo.* In some embodiments, the cell or tissue is in the eye (*e.g.,* a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an

astrocyte, a pericyte, a Müller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel). In some embodiments, the cell or tissue is in a subject (*e.g.,* a mammal, such as, for example, a human). In some embodiments, the subject (*e.g.,* the human) is at risk, or is diagnosed with a disorder related to expression of CA2 expression, as described herein.

In some embodiments, the method includes contacting the cell with an iRNA as described herein, in an amount effective to decrease the expression of CA2 in the cell. In some embodiments, contacting a cell with an RNAi agent includes contacting a cell *in vitro* with the RNAi agent or contacting a cell *in vivo* with the RNAi agent. In some embodiments, the RNAi agent is put into physical contact with the cell by the individual performing the method, or the RNAi agent may be put into a situation that will permit or cause it to subsequently come into contact with the cell. Contacting a cell in vitro may be done, for example, by incubating the cell with the RNAi agent. Contacting a cell in vivo may be done, for example, by injecting the RNAi agent into or near the tissue where the cell is located, or by injecting the RNAi agent into another area, *e.g.,* ocular tissue. For example, the RNAi agent may contain or be coupled to a ligand, *e.g.,* a lipophilic moiety or moieties as described below and further detailed, *e.g.,* in PCT/US2019/031170 which is incorporated herein by reference in its entirety, including the passages therein describing lipophilic moieties, that directs or otherwise stabilizes the RNAi agent at a site of interest. Combinations of in vitro and in vivo methods of contacting are also possible. For example, a cell may also be contacted in vitro with an RNAi agent and subsequently transplanted into a subject.

The expression of CA2 may be assessed based on the level of expression of CA2 mRNA, CA2 protein, or the level of another parameter functionally linked to the level of expression of CA2. In some embodiments, the expression of CA2 is inhibited by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95%. In some embodiments, the iRNA has an $IC_{50}$ in the range of 0.001-0.01 nM, 0.001-0.10 nM, 0.001-1.0 nM, 0.001-10 nM, 0.01-0.05 nM, 0.01-0.50 nM, 0.02-0.60 nM, 0.01-1.0 nM, 0.01-1.5 nM, 0.01-10 nM. The $IC_{50}$ value may be normalized relative to an appropriate control value, *e.g.,* the $IC_{50}$ of a non-targeting iRNA.

In some embodiments, the method includes introducing into the cell or tissue an iRNA as described herein and maintaining the cell or tissue for a time sufficient to obtain degradation of the mRNA transcript of CA2, thereby inhibiting the expression of CA2 in the cell or tissue.

In some embodiments, the method includes administering a composition described herein, *e.g.,* a composition comprising an iRNA that binds CA2, to the mammal such that expression of the target CA2 is decreased, such as for an extended duration, *e.g.,* at least two, three, four days or more, *e.g.,* one week, two weeks, three weeks, or four weeks or longer. In some embodiments, the decrease in expression of CA2 is detectable within 1 hour, 2 hours, 4 hours, 8 hours, 12 hours, or 24 hours of the first administration.

In some embodiments, the method includes administering a composition as described herein to a mammal such that expression of the target CA2 is increased by e.g., at least 10% compared to an untreated animal. In some embodiments, the activation of CA2 occurs over an extended duration, e.g., at least two, three, four days or more, e.g., one week, two weeks, three weeks, four weeks, or more. Without wishing to be bound by theory, an iRNA can activate CA2 expression by stabilizing the CA2 mRNA transcript, interacting with a promoter in the genome, or inhibiting an inhibitor of CA2 expression.

The iRNAs useful for the methods and compositions featured in the disclosure specifically target RNAs (primary or processed) of CA2. Compositions and methods for inhibiting the expression of CA2 using iRNAs can be prepared and performed as described elsewhere herein.

In some embodiments, the method includes administering a composition containing an iRNA, where the iRNA includes a nucleotide sequence that is complementary to at least a part of an RNA transcript of CA2 of the subject, *e.g.,* the mammal, *e.g.,* the human, to be treated. The composition may be administered by any appropriate means known in the art including, but not limited to ocular (*e.g.,* intraocular), topical, and intravenous administration.

In certain embodiments, the composition is administered intraocularly (*e.g.*, by intravitreal administration, e.g., intravitreal injection; transscleral administration, e.g., transscleral injection; subconjunctival administration, e.g., subconjunctival injection; retrobulbar administration, e.g., retrobulbar injection; intracameral administration, e.g., intracameral injection; or subretinal administration, e.g., subretinal injection. In other embodiments, the composition is administered topically. In other embodiments, the composition is administered by intravenous infusion or injection.

In certain embodiments, the composition is administered by intravenous infusion or injection. In some such embodiments, the composition comprises a lipid formulated siRNA (*e.g.,* an LNP formulation, such as an LNP11 formulation) for intravenous infusion.

Unless otherwise defined, all technical and scientific terms used herein have the same meaning as commonly understood by one of ordinary skill in the art to which this disclosure belongs. Although methods and materials similar or equivalent to those described herein can be used in the practice or testing of the iRNAs and methods featured in the disclosure, suitable methods and materials are described below. All publications, patent applications, patents, and other references mentioned herein are incorporated by reference in their entirety. In case of conflict, the present specification,

including definitions, will control. In addition, the materials, methods, and examples are illustrative only and not intended to be limiting.

## Specific Embodiments

In an embodiment the disclosure provides a double stranded ribonucleic acid (dsRNA) agent for inhibiting expression of carbonic anhydrase 2 (CA2), wherein the dsRNA agent comprises a sense strand and an antisense strand forming a double stranded region, wherein the sense strand comprises a nucleotide sequence comprising at least 15 contiguous nucleotides, with 0, 1, 2, or 3 mismatches, of a portion of a coding strand of human CA2 and the antisense strand comprises a nucleotide sequence comprising at least 15 contiguous nucleotides, with 0, 1, 2, or 3 mismatches, of the corresponding portion of a non-coding strand of human CA2 such that the sense strand is complementary to the at least 15 contiguous nucleotides in the antisense strand.

In some embodiments the disclosure provides a double stranded ribonucleic acid (dsRNA) agent for inhibiting expression of carbonic anhydrase 2 (CA2), wherein the dsRNA agent comprises a sense strand and an antisense strand forming a double stranded region, wherein the sense strand comprises a nucleotide sequence comprising at least 15 contiguous nucleotides, with 0, 1, 2, or 3 mismatches, of a portion of a coding strand of human CA2 and the antisense strand comprises a nucleotide sequence comprising at least 15 contiguous nucleotides, with 0, 1, 2, or 3 mismatches, of the corresponding portion of a non-coding strand of human CA2 such that the sense strand is complementary to the at least 15 contiguous nucleotides in the antisense strand wherein the dsRNA agent comprises at least one modified nucleotide.

In some embodiments the coding strand of human CA2 comprises the sequence SEQ ID NO: 1. In some embodiments the non-coding strand of human CA2 comprises the sequence of SEQ ID NO: 2

In some embodiments the double stranded ribonucleic acid (dsRNA) agent for inhibiting expression of CA2 comprises a sense strand and an antisense strand forming a double stranded region, wherein the antisense strand comprises a nucleotide sequence comprising at least 15 contiguous nucleotides, with 0, 1, 2, or 3 mismatches, of a portion of nucleotide sequence of SEQ ID NO: 2 such that the sense strand is complementary to the at least 15 contiguous nucleotides in the antisense strand.

In some embodiments the double stranded ribonucleic acid (dsRNA) agent for inhibiting expression of CA2 comprises a sense strand and an antisense strand forming a double stranded region, wherein the sense strand comprises a nucleotide sequence comprising at least 15 contiguous nucleotides, with 0, or 1, 2, or 3 mismatches, of the corresponding portion of the nucleotide sequence of SEQ ID NO: 1.

In some embodiments the dsRNA agent comprises a sense strand and an antisense strand, wherein the antisense strand comprises a nucleotide sequence comprising at least 17 contiguous nucleotides, with 0, 1, 2, or 3 mismatches, of a portion of nucleotide sequence of SEQ ID NO: 2 such that the sense strand is complementary to the at least 17 contiguous nucleotides in the antisense strand.

In some embodiments the dsRNA agent comprises a sense strand and an antisense strand, wherein the sense strand comprises a nucleotide sequence comprising at least 17 contiguous nucleotides, with 0, or 1, 2, or 3 mismatches, of the corresponding portion of the nucleotide sequence of SEQ ID NO: 1.

In some embodiments the dsRNA agent comprises a sense strand and an antisense strand, wherein the antisense strand comprises a nucleotide sequence comprising at least 19 contiguous nucleotides, with 0, 1, 2, or 3 mismatches, of a portion of nucleotide sequence of SEQ ID NO: 2 such that the sense strand is complementary to the at least 19 contiguous nucleotides in the antisense strand.

In some embodiments the sense strand comprises a nucleotide sequence comprising at least 19 contiguous nucleotides, with 0, 1, 2, or 3 mismatches, of the corresponding portion of the nucleotide sequence of SEQ ID NO: 1.

In some embodiments the dsRNA agent comprises a sense strand and an antisense strand, wherein the antisense strand comprises a nucleotide sequence comprising at least 21 contiguous nucleotides, with 0, 1, 2, or 3 mismatches, of a portion of nucleotide sequence of SEQ ID NO: 2 such that the sense strand is complementary to the at least 21 contiguous nucleotides in the antisense strand.

In some embodiments the sense strand of the dsRNA agent comprises a nucleotide sequence comprising at least 21 contiguous nucleotides, with 0, or 1, 2, or 3 mismatches, of the corresponding portion of the nucleotide sequence of SEQ ID NO: 1.

In some embodiments the portion of the sense strand of the dsRNA agent is a portion within a sense strand in any one of Tables 3-10. In some embodiments the portion of the antisense strand of the dsRNA agent is a portion within an antisense strand in any one of Tables 3-10.

In some embodiments the antisense strand of the dsRNA agent comprises a nucleotide sequence comprising at least 15 contiguous nucleotides, with 0, 1, 2, or 3 mismatches, from one of the antisense sequences listed in any one of Tables 3-10.

In some embodiments the sense strand of the dsRNA agent comprises a nucleotide sequence comprising at least 15 contiguous nucleotides, with 0, 1, 2, or 3 mismatches, from a sense sequence listed in any one of Tables 3-10 that

corresponds to the antisense sequence.

In some embodiments the antisense strand of the dsRNA agent comprises a nucleotide sequence comprising at least 17 contiguous nucleotides, with 0, 1, 2, or 3 mismatches, from one of the antisense sequences listed in any one of Tables 3-10.

In some embodiments the sense strand of the dsRNA agent comprises a nucleotide sequence comprising at least 17 contiguous nucleotides, with 0, 1, 2, or 3 mismatches, from a sense sequence listed in any one of Tables 3-10 that corresponds to the antisense sequence. In some embodiments the antisense strand of the dsRNA agent comprises a nucleotide sequence comprising at least 19 contiguous nucleotides, with 0,1, 2, or 3 mismatches, from one of the antisense sequences listed in any one of Tables 3-10.

In some embodiments the sense strand of the dsRNA agent comprises a nucleotide sequence comprising at least 19 contiguous nucleotides, with 0, 1, 2, or 3 mismatches, from a sense sequence listed in any one of Tables 3-10 that corresponds to the antisense sequence.

In some embodiments the antisense strand of the dsRNA agent comprises a nucleotide sequence comprising at least 21 contiguous nucleotides, with 0, 1, 2, or 3 mismatches, from one of the antisense sequences listed in any one of Tables 3-10.

In some embodiments the sense strand of the dsRNA agent comprises a nucleotide sequence comprising at least 21 contiguous nucleotides, with 0, 1, 2, or 3 mismatches, from a sense sequence listed in any one of Tables 3-10 that corresponds to the antisense sequence.

In some embodiments the sense strand of the dsRNA agent is at least 23 nucleotides in length, e.g., 23-30 nucleotides in length.

In some embodiments at least one of the sense strand and the antisense strand of the dsRNA agent is conjugated to one or more lipophilic moieties.

In some embodiments the lipophilic moiety is conjugated to one or more positions in the double stranded region of the dsRNA agent. In some embodiments the lipophilic moiety is conjugated via a linker or carrier.

In some embodiments the lipophilicity of the lipophilic moiety, measured by logKow, exceeds 0.

In some embodiments the hydrophobicity of the double-stranded RNAi agent, measured by the unbound fraction in a plasma protein binding assay of the double-stranded RNAi agent, exceeds 0.2.

In an embodiment the plasma protein binding assay is an electrophoretic mobility shift assay using human serum albumin protein.

In a particular embodiment the dsRNA agent comprises at least one modified nucleotide.

In some embodiments no more than five of the sense strand nucleotides and not more than five of the nucleotides of the antisense strand of the dsRNA agent are unmodified nucleotides.

In some embodiments all of the nucleotides of the sense strand and all of the nucleotides of the antisense strand of the dsRNA agent comprise a modification.

In some embodiments at least one of the modified nucleotides of the dsRNA agent is selected from the group consisting of a deoxy-nucleotide, a 3'-terminal deoxythimidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-C-alkyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, a nucleotide comprising a 5'-phosphate mimic, a glycol modified nucleotide, and a 2-O-(N-methylacetamide) modified nucleotide; and combinations thereof.

In some embodiments no more than five of the sense strand nucleotides and not more than five of the nucleotides of the antisense strand of the dsRNA agent include modifications other than 2'-O-methyl modified nucleotide, a 2'-fluoro modified nucleotide, a 2'-deoxy-modified nucleotide, unlocked nucleic acids (UNA) or glycerol nucleic acid (GNA).

In some embodiments the dsRNA agent comprises a non-nucleotide spacer (wherein optionally the non-nucleotide spacer comprises a C3-C6 alkyl) between two of the contiguous nucleotides of the sense strand or between two of the contiguous nucleotides of the antisense strand.

In some embodiments each strand of the dsRNA agent is no more than 30 nucleotides in length.

In some embodiments at least one strand of the dsRNA agent comprises a 3' overhang of at least 1 nucleotide.

In some embodiments at least one strand of the dsRNA agent comprises a 3' overhang of at least 2 nucleotides.

In some embodiments the double stranded region of the dsRNA agent is 15-30 nucleotide pairs in length. In some embodiments the double stranded region of the dsRNA agent is 17-23 nucleotide pairs in length. In some embodiments the double stranded region of the dsRNA agent is 17-25 nucleotide pairs in length. In some embodiments the double stranded region of the dsRNA agent is 23-27 nucleotide pairs in length. In some embodiments the double stranded region of the dsRNA agent is 19-21 nucleotide pairs in length. In some embodiments the double stranded region is 21-23 nucleotide

pairs in length. In some embodiments the positions in the double stranded region exclude a cleavage site region of the sense strand of the dsRNA agent.

In some embodiments each strand of the dsRNA agent has 19-30 nucleotides. In some embodiments each strand of the dsRNA agent has 19-23 nucleotides. In some embodiments each strand of the dsRNA agent has 21-23 nucleotides.

In some embodiments the dsRNA agent comprises at least one phosphorothioate or methylphosphonate internucleotide linkage. In some embodiments the phosphorothioate or methylphosphonate internucleotide linkage is at the 3'-terminus of one strand of the dsRNA agent. In some embodiments the phosphorothioate or methylphosphonate internucleotide linkage is at the 3'-terminus of the antisense strand of the dsRNA agent. In some embodiments the phosphorothioate or methylphosphonate internucleotide linkage is at the 3'-terminus of the sense strand of the dsRNA agent. In some embodiments the phosphorothioate or methylphosphonate internucleotide linkage is at the 5'-terminus of one strand of the dsRNA agent. In some embodiments the phosphorothioate or methylphosphonate internucleotide linkage is at the 5'-terminus of the antisense strand of the dsRNA agent. In some embodiments the phosphorothioate or methylphosphonate internucleotide linkage is at the 5'-terminus of the sense strand of the dsRNA agent. In some embodiments the 5'- and 3'-terminus of one strand of the dsRNA agent comprises a phosphorothioate or methylphosphonate internucleotide linkage.

In some embodiments the 5'- and 3'-terminus of the antisense strand of the dsRNA agent comprises a phosphorothioate or methylphosphonate internucleotide linkage.

In some embodiments the base pair at the 1 position of the 5'-end of the antisense strand of the duplex is an AU base pair.

In some embodiments the sense strand of the dsRNA agent has a total of 21 nucleotides and the antisense strand has a total of 23 nucleotides.

In some embodiments one or more lipophilic moieties are conjugated to one or more internal positions on at least one strand of the dsRNA agent. In some embodiments one or more lipophilic moieties are conjugated to one or more internal positions on at least one strand of the dsRNA agent via a linker or carrier. In some embodiments the internal positions include all positions except the terminal two positions from each end of at least one strand of the dsRNA agent. In some embodiments the internal positions include all positions except the terminal three positions from each end of the at least one strand of the dsRNA agent. In some embodiments the internal positions exclude a cleavage site region of the sense strand of the dsRNA agent.

In some embodiments the internal positions include all positions except positions 9-12, counting from the 5'-end of the sense strand of the dsRNA agent.

In some embodiments the internal positions include all positions except positions 11-13, counting from the 3'-end of the sense strand of the dsRNA agent.

In some embodiments the internal positions exclude a cleavage site region of the antisense strand of the dsRNA agent.

In some embodiments the internal positions include all positions except positions 12-14, counting from the 5'-end of the antisense strand of the dsRNA agent.

In some embodiments the internal positions include all positions except positions 11-13 on the sense strand of the dsRNA agent, counting from the 3'-end, and positions 12-14 on the antisense strand of the dsRNA agent, counting from the 5'-end.

In some embodiments the lipophilic moieties are conjugated to one or more of the internal positions selected from the group consisting of positions 4-8 and 13-18 on the sense strand, and positions 6-10 and 15-18 on the antisense strand, counting from the 5'end of each strand of the dsRNA agent.

In some embodiments the lipophilic moieties are conjugated to one or more of the internal positions selected from the group consisting of positions 5, 6, 7, 15, and 17 on the sense strand, and positions 15 and 17 on the antisense strand, counting from the 5'-end of each strand of the dsRNA agent.

In some embodiments the sense strand is 21 nucleotides in length, the antisense strand is 23 nucleotides in length, and the lipophilic moiety is conjugated to position 21, position 20, position 15, position 1, position 7, position 6, or position 2 of the sense strand or position 16 of the antisense strand of the dsRNA agent.

In some embodiments the lipophilic moiety is conjugated to position 21, position 20, position 15, position 1, or position 7 of the sense strand of the dsRNA agent.

In some embodiments the lipophilic moiety is conjugated to position 21, position 20, or position 15 of the sense strand of the dsRNA agent.

In some embodiments the lipophilic moiety is conjugated to position 20 or position 15 of the sense strand of the dsRNA agent.

In some embodiments the lipophilic moiety is conjugated to position 16 of the antisense strand of the dsRNA agent.

In some embodiments the lipophilic moiety is conjugated to position 6, counting from the 5'-end of the sense

strand of the dsRNA agent.

In some embodiments the lipophilic moiety is an aliphatic, alicyclic, or polyalicyclic compound.

In some embodiments the lipophilic moiety is selected from the group consisting of lipid, cholesterol, retinoic acid, cholic acid, adamantane acetic acid, 1-pyrene butyric acid, dihydrotestosterone, 1,3-bis-O(hexadecyl)glycerol, geranyloxyhexyanol, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine.

In some embodiments the lipophilic moiety contains a saturated or unsaturated C4-C30 hydrocarbon chain, and an optional functional group selected from the group consisting of hydroxyl, amine, carboxylic acid, sulfonate, phosphate, thiol, azide, and alkyne.

In some embodiments the lipophilic moiety contains a saturated or unsaturated C6-C18 hydrocarbon chain.

In some embodiments the lipophilic moiety contains a saturated or unsaturated C16 hydrocarbon chain.

In some embodiments the lipophilic moiety is conjugated via a carrier that replaces one or more nucleotide(s) in the internal position(s) or the double stranded region.

In some embodiments the carrier is a cyclic group selected from the group consisting of pyrrolidinyl, pyrazolinyl, pyrazolidinyl, imidazolinyl, imidazolidinyl, piperidinyl, piperazinyl, [1,3]dioxolanyl, oxazolidinyl, isoxazolidinyl, morpholinyl, thiazolidinyl, isothiazolidinyl, quinoxalinyl, pyridazinonyl, tetrahydrofuranyl, and decalinyl; or is an acyclic moiety based on a serinol backbone or a diethanolamine backbone.

In some embodiments the lipophilic moiety is conjugated to the double-stranded iRNA agent via a linker containing an ether, thioether, urea, carbonate, amine, amide, maleimide-thioether, disulfide, phosphodiester, sulfonamide linkage, a product of a click reaction, or carbamate.

In some embodiments the lipophilic moiety is conjugated to a nucleobase, sugar moiety, or internucleosidic linkage.

In some embodiments the lipophilic moiety is conjugated via a bio-cleavable linker selected from the group consisting of DNA, RNA, disulfide, amide, functionalized monosaccharides or oligosaccharides of galactosamine, glucosamine, glucose, galactose, mannose, and combinations thereof.

In some embodiments the 3' end of the sense strand of the dsRNA agent is protected via an end cap which is a cyclic group having an amine, said cyclic group being selected from the group consisting of pyrrolidinyl, pyrazolinyl, pyrazolidinyl, imidazolinyl, imidazolidinyl, piperidinyl, piperazinyl, [1,3]dioxolanyl, oxazolidinyl, isoxazolidinyl, morpholinyl, thiazolidinyl, isothiazolidinyl, quinoxalinyl, pyridazinonyl, tetrahydrofuranyl, and decalinyl.

In some embodiments the dsRNA agent further comprising a targeting ligand, e.g., a ligand that targets an ocular tissue or a liver tissue. In some embodiments the ligand is conjugated to the sense strand of the dsRNA agent. In some embodiments the ligand is conjugated to the 3' end or the 5' end of the sense strand of the dsRNA agent. In some embodiments the ligand is conjugated to the 3' end of the sense strand of the dsRNA agent.

In some embodiments the dsRNA agent further comprising a ligand that targets an ocular tissue wherein the ocular tissue is ciliary epithelium, an optic nerve, a trabecular meshwork, a juxtacanalicular tissue, a ganglion (e.g., including a retinal ganglion), episcleral veins or a Schlemm's canal (e.g., including an endothelial cell).

In some embodiments the targeting ligand of the dsRNA agent comprises N-acetylgalactosamine (GalNAc). In some embodiments the targeting ligand of the dsRNA agent is one or more GalNAc conjugates or one or more or GalNAc derivatives. In some embodiments the GalNAc conjugates or one or more GalNAc derivatives are attached through a monovalent linker, or a bivalent, trivalent, or tetravalent branched linker.

In some embodiments the targeting ligand of the dsRNA agent is

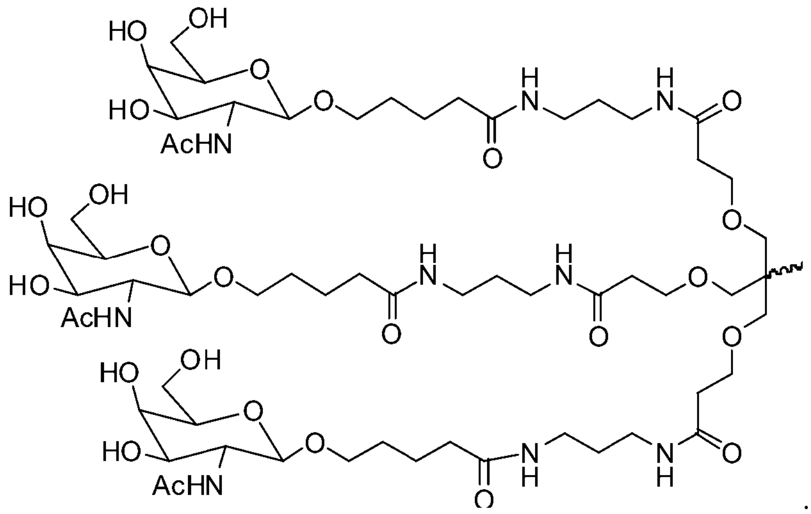

In some embodiments the dsRNA agent is conjugated to the ligand as shown in the following schematic

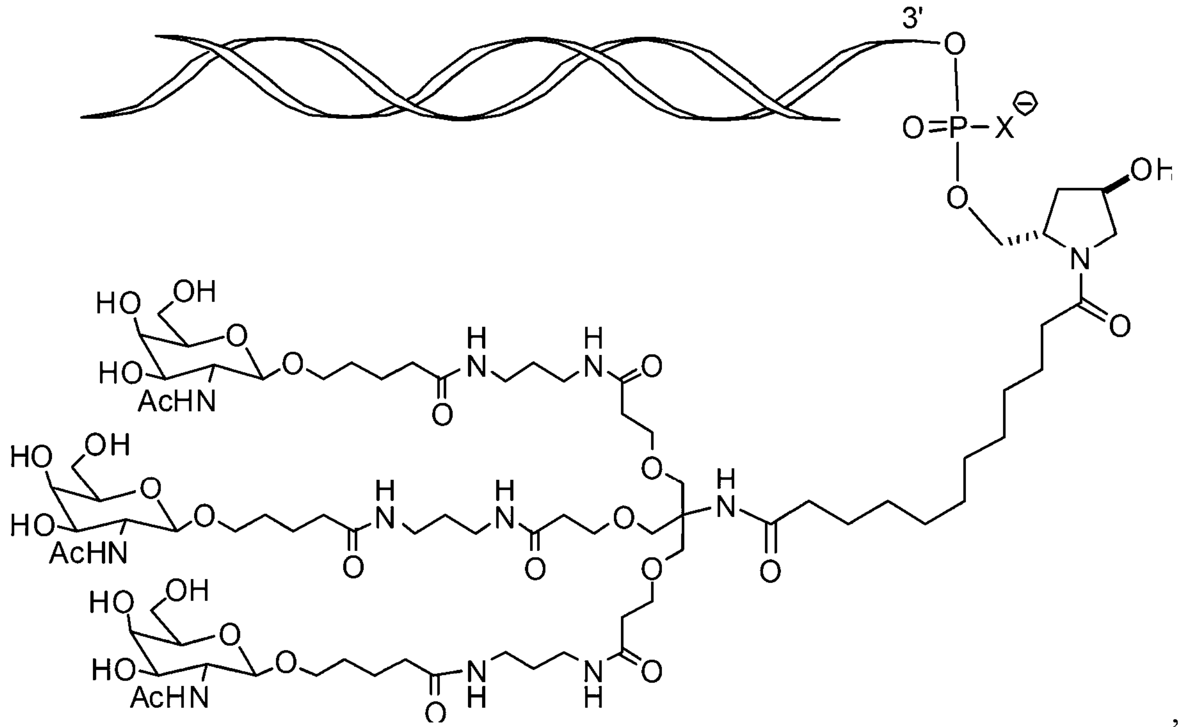

wherein X is O or S.

In some embodiments the dsRNA agent further comprises a terminal, chiral modification occurring at the first internucleotide linkage at the 3' end of the antisense strand, having the linkage phosphorus atom in Sp configuration, a terminal, chiral modification occurring at the first internucleotide linkage at the 5' end of the antisense strand, having the linkage phosphorus atom in Rp configuration, and a terminal, chiral modification occurring at the first internucleotide linkage at the 5' end of the sense strand, having the linkage phosphorus atom in either Rp configuration or Sp configuration.

In some embodiments the dsRNA agent further comprises a terminal, chiral modification occurring at the first and second internucleotide linkages at the 3' end of the antisense strand, having the linkage phosphorus atom in Sp configuration; a terminal, chiral modification occurring at the first internucleotide linkage at the 5' end of the antisense strand, having the linkage phosphorus atom in Rp configuration; and a terminal, chiral modification occurring at the first internucleotide linkage at the 5' end of the sense strand, having the linkage phosphorus atom in either Rp or Sp configuration.

In some embodiments the dsRNA agent further comprises a terminal, chiral modification occurring at the first, second and third internucleotide linkages at the 3' end of the antisense strand, having the linkage phosphorus atom in Sp

configuration; a terminal, chiral modification occurring at the first internucleotide linkage at the 5' end of the antisense strand, having the linkage phosphorus atom in Rp configuration; and a terminal, chiral modification occurring at the first internucleotide linkage at the 5' end of the sense strand, having the linkage phosphorus atom in either Rp or Sp configuration.

In some embodiments the dsRNA agent further comprises a terminal, chiral modification occurring at the first, and second internucleotide linkages at the 3' end of the antisense strand, having the linkage phosphorus atom in Sp configuration; a terminal, chiral modification occurring at the third internucleotide linkages at the 3' end of the antisense strand, having the linkage phosphorus atom in Rp configuration; a terminal, chiral modification occurring at the first internucleotide linkage at the 5' end of the antisense strand, having the linkage phosphorus atom in Rp configuration; and a terminal, chiral modification occurring at the first internucleotide linkage at the 5' end of the sense strand, having the linkage phosphorus atom in either Rp or Sp configuration.

In some embodiments the dsRNA agent further comprises a terminal, chiral modification occurring at the first, and second internucleotide linkages at the 3' end of the antisense strand, having the linkage phosphorus atom in Sp configuration; a terminal, chiral modification occurring at the first, and second internucleotide linkages at the 5' end of the antisense strand, having the linkage phosphorus atom in Rp configuration; and a terminal, chiral modification occurring at the first internucleotide linkage at the 5' end of the sense strand, having the linkage phosphorus atom in either Rp or Sp configuration.

In some embodiments the dsRNA agent further comprises a phosphate or phosphate mimic at the 5'-end of the antisense strand. In some embodiments the phosphate mimic is a 5'-vinyl phosphonate (VP).

In some embodiments the disclosure provides a cell containing the dsRNA agent of any one of the preceding embodiments.

In some embodiments the cell containing the dsRNA agent is a human ocular cell, e.g., (a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an astrocyte, a pericyte, a Miiller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel) comprising a reduced level of CA2 mRNA or a level of CA2 protein as compared to an otherwise similar untreated cell, wherein optionally the level is reduced by at least 10%, 15%, 20%, 25%, 30%, 35%, 40%, 45%, 50%, 55%, 60%, 65%, 70%, 75%, 80%, 85%, 90%, or 95%.

In some embodiments the human cell containing the dsRNA agent is produced by a process comprising contacting a human cell with the dsRNA agent of any one of preceding embodiments.

In some embodiments the disclosure provides a pharmaceutical composition for inhibiting expression of CA2, comprising the dsRNA agent of any one of preceding embodiments.

In a particular embodiment the disclosure provides a pharmaceutical composition comprising the dsRNA agent of any one of preceding embodiments and a lipid formulation.

In some embodiments the disclosure provides a method of inhibiting expression of CA2 in a cell, the method comprising:

(a) contacting the cell with the dsRNA agent of any one of preceding embodiments or a pharmaceutical composition comprising the dsRNA agent of any one of preceding embodiments; and
(b) maintaining the cell produced in step (a) for a time sufficient to obtain degradation of the mRNA transcript of CA2, thereby inhibiting expression of CA2 in the cell.

In other embodiments the disclosure provides a method of inhibiting expression of CA2 in a cell, the method comprising:

(a) contacting the cell with the dsRNA agent of any one of preceding embodiments or a pharmaceutical composition comprising the dsRNA agent of any one of preceding embodiments; and
(b) maintaining the cell produced in step (a) for a time sufficient to reduce levels of CA2 mRNA, CA2 protein, or both of CA2 mRNA and protein, thereby inhibiting expression of CA2 in the cell.

In some embodiments the disclosure provides a method of inhibiting expression of CA2 in a cell wherein the cell is within a subject.

In some embodiments the disclosure provides a method of inhibiting expression of CA2 in a cell, wherein the cell is within a human subject.

In some embodiments the disclosure provides a method of inhibiting expression of CA2 in a cell wherein the level of CA2 mRNA is inhibited by at least 50%.

In some embodiments the disclosure provides a method of inhibiting expression of CA2 in a cell wherein the level of CA2 protein is inhibited by at least 50%.

In some embodiments the disclosure provides a method of inhibiting expression of CA2 in a cell wherein inhibiting expression of CA2 decreases a CA2 protein level in a biological sample *(e.g.,* a ciliary epithelium sample) from the subject by at least 30%, 40%, 50%, 60%, 70%, 80%, 90%, or 95%.

In particular embodiments the disclosure provides a method of inhibiting expression of CA2 in a cell wherein the subject has been diagnosed with a CA2-associated disorder, e.g., glaucoma.

In some embodiments the method of inhibiting expression of CA2 in an ocular cell or tissue comprises

(a) contacting the cell or tissue with a dsRNA agent that binds CA2; and

(b) maintaining the cell or tissue produced in step (a) for a time sufficient to reduce levels of CA2 mRNA, CA2 protein, or both of CA2 mRNA and protein, thereby inhibiting expression of CA2 in the cell or tissue. In some embodiments the ocular cell or tissue comprises a ciliary epithelium cell, an optic nerve cell, a trabecular meshwork cell, a Schlemm's canal cell (e.g., including an endothelial cell), a juxtacanalicular tissue cell, a ciliary muscle cell, a retinal cell, an astrocyte, a pericyte, a Miiller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel.

In some embodiments the disclosure provides a method of treating a subject diagnosed with a CA2-associated disorder comprising administering to the subject a therapeutically effective amount of the dsRNA agent of any one of preceding embodiments or a pharmaceutical composition thereof, thereby treating the disorder. In some embodiments the disclosure provides a method of treating a subject diagnosed with a CA2-associated disorder wherein the CA2-associated disorder is glaucoma or a glaucoma associated condition.

In particular embodiments the disclosure provides a method of treating a subject diagnosed with a CA2-associated disorder wherein treating comprises amelioration of at least one sign or symptom of the disorder. In particular embodiments the disclosure provides a method of treating a subject diagnosed with a CA2-associated disorder where treating comprises prevention of progression of the disorder. In some embodiments the treating comprises one or more of (a) inhibiting or reducing intraocular pressure; (b) inhibiting or reducing the expression or activity of CA2; (c) decreasing the amount of aqueous humor; (d) inhibiting or reducing optic nerve damage; (e) inhibiting or reducing retinal ganglion cell death; (f) medication to reduce intraocular pressure; (g) laser treatment; (h) surgery; (i) or trabeculectomy.

In some embodiments the disclosure provides a method of treating a subject diagnosed with glaucoma wherein at least one sign or symptom of glaucoma comprises a measure of one or more of intraocular pressure, vision loss, optic nerve damage, ocular inflammation, visual acuity, or presence, level, or activity of CA2 (*e.g.,* CA2 gene, CA2 mRNA, or CA2 protein).

In particular embodiments the disclosure provides a method of treating a subject diagnosed with a CA2-associated disorder wherein the treating results in at least a 30% mean reduction from baseline of CA2 mRNA in a ciliary epithelium cell, an optic nerve cell, a trabecular meshwork cell, a Schlemm's canal cell (e.g., including an endothelial cell), a juxtacanalicular tissue cell, a ciliary muscle cell, retinal pigment epithelium (RPE), a retinal cell, an astrocyte, a pericyte, a Miiller cell, a ganglion cell (e.g., including a retinal ganglion cell), an endothelial cell, a photoreceptor cell, a retinal blood vessel (e.g., including endothelial cells and vascular smooth muscle cells), episcleral veins or choroid tissue, e.g., a choroid vessel.

In some embodiments the disclosure provides a method of treating a subject diagnosed with a CA2-associated disorder wherein the treating results in at least a 60% mean reduction from baseline of CA2 mRNA in the ciliary epithelium cell, optic nerve cell, trabecular meshwork cell, Schlemm's canal cell (e.g., including an endothelial cell), juxtacanalicular tissue cell, ciliary muscle cell, retinal pigment epithelium (RPE), retinal cell, astrocyte, pericyte, Miiller cell, ganglion cell (e.g., including retinal ganglion cell), endothelial cell, photoreceptor cell, retinal blood vessel (e.g., including endothelial cells and vascular smooth muscle cells), episcleral veins or choroid tissue, e.g., choroid vessel.

In other embodiments the disclosure provides a method of treating a subject diagnosed with a CA2-associated disorder wherein the treating results in at least a 90% mean reduction from baseline of CA2 mRNA in the ciliary epithelium cell, optic nerve cell, trabecular meshwork cell, Schlemm's canal cell (e.g., including an endothelial cell), juxtacanalicular tissue cell, ciliary muscle cell, retinal pigment epithelium (RPE), retinal cell, astrocyte, pericyte, Miiller cell, ganglion cell (e.g., including retinal ganglion cell), endothelial cell, photoreceptor cell, retinal blood vessel (e.g., including endothelial cells and vascular smooth muscle cells), episcleral veins or choroid tissue, e.g., choroid vessel.

In some embodiments the disclosure provides a method of treating a subject diagnosed with a CA2-associated disorder wherein after treatment the subject experiences at least an 8-week duration of knockdown following a single dose of dsRNA as assessed by CA2 protein in the ciliary epithelium.

In particular embodiments the disclosure provides a method of treating a subject diagnosed with a CA2-associated disorder wherein treating results in at least a 12-week duration of knockdown following a single dose of dsRNA as assessed by CA2 protein in the ciliary epithelium.

In particular embodiments the disclosure provides a method of treating a subject diagnosed with a CA2-associated disorder wherein treating results in at least a 16-week duration of knockdown following a single dose of dsRNA as assessed by CA2 protein in the ciliary epithelium.

In some embodiments the disclosure provides a method of treating a subject diagnosed with a CA2-associated disorder wherein the subject is human.

In some embodiments the disclosure provides a method of treating a subject diagnosed with a CA2-associated disorder wherein the dsRNA agent is administered at a dose of about 0.01 mg/kg to about 50 mg/kg. In some embodiments the disclosure provides a method of treating a subject diagnosed with a CA2-associated disorder wherein the dsRNA agent is administered to the subject intraocularly, intravenously, or topically. In some embodiments the intraocular administration comprises intravitreal administration (e.g., intravitreal injection), transscleral administration (e.g., transscleral injection), subconjunctival administration (e.g., subconjunctival injection), retrobulbar administration (e.g., retrobulbar injection), intracameral administration (e.g., intracameral injection), or subretinal administration (e.g., subretinal injection).

In some embodiments the method of treating a subject diagnosed with a CA2-associated disorder further comprising measuring level of CA2 (*e.g.,* CA2 gene, CA2 mRNA, or CA2 protein) in the subject.

In some embodiments measuring the level of CA2 in the subject comprises measuring the level of CA2 gene, CA2 protein or CA2 mRNA in a biological sample from the subject (*e.g.,* a ciliary epithelium sample). In some embodiments measuring level of CA2 (*e.g.,* CA2 gene, CA2 mRNA, or CA2 protein) in the subject is performed prior to treatment with the dsRNA agent or the pharmaceutical composition. In other embodiments measuring level of CA2 (*e.g.,* CA2 gene, CA2 mRNA, or CA2 protein) in the subject is performed after treatment with the dsRNA agent or the pharmaceutical composition. In some embodiments upon determination that a subject has a level of CA2 (*e.g.,* CA2 gene, CA2 mRNA, or CA2 protein) that is greater than a reference level, the dsRNA agent or the pharmaceutical composition is administered to the subject. In some embodiments, measuring level of CA2 (*e.g.,* CA2 gene, CA2 mRNA, or CA2 protein) in the subject is performed after treatment with the dsRNA agent or the pharmaceutical composition.

In some embodiments the disclosure provides a method of treating a subject diagnosed with a CA2-associated disorder further comprising performing a blood test, an imaging test, a tonometry test or a ciliary epithelium biopsy.

In some embodiments the disclosure provides a method of treating a subject diagnosed with a CA2-associated disorder, the method further comprising administering to the subject an additional agent and/or therapy suitable for treatment or prevention of an CA2-associated disorder. In some embodiments the additional agent and/or therapy comprises one or more of a prostaglandin analog, a beta blocker, an alpha-adrenergic agonist, a carbonic anhydrase inhibitor, or an anti-CA2 agent.

## EXAMPLES

### Example 1. CA2 siRNA

Nucleic acid sequences provided herein are represented using standard nomenclature. *See* the abbreviations of Table 2.

**Table 2. Abbreviations of nucleotide monomers used in nucleic acid sequence** representation

| It will be understood that these monomers, when present in an oligonucleotide, are mutually linked by 5'-3'-phosphodiester bonds. | |
|---|---|
| Abbreviation | Nucleotide(s) |
| A | Adenosine-3'-phosphate |
| Ab | beta-L-adenosine-3'-phosphate |
| Abs | beta-L-adenosine-3'-phosphorothioate |
| Af | 2'-fluoroadenosine-3'-phosphate |
| Afs | 2'-fluoroadenosine-3'-phosphorothioate |
| (Ahd) | 2'-O-hexadecyl-adenosine-3'-phosphate |
| (Ahds) | 2'-O-hexadecyl-adenosine-3'-phosphorothioate |
| As | adenosine-3'-phosphorothioate |
| (A2p) | adenosine-2'-phosphate |
| (A2ps) | adenosine-2'-phosphorothioate |
| C | cytidine-3'-phosphate |
| Cb | beta-L-cytidine-3'-phosphate |
| Cbs | beta-L-cytidine-3'-phosphorothioate |
| Cf | 2'-fluorocytidine-3'-phosphate |
| Cfs | 2'-fluorocytidine-3'-phosphorothioate |
| (Chd) | 2'-O-hexadecyl-cytidine-3'-phosphate |
| (Chds) | 2'-O-hexadecyl-cytidine-3'-phosphorothioate |
| Cs | cytidine-3'-phosphorothioate |
| (C2p) | cytidine-2'-phosphate |
| (C2ps) | cytidine-2'-phosphorothioate |
| G | guanosine-3'-phosphate |
| Gb | beta-L-guanosine-3'-phosphate |
| Gbs | beta-L-guanosine-3'-phosphorothioate |
| Gf | 2'-fluoroguanosine-3'-phosphate |
| Gfs | 2'-fluoroguanosine-3'-phosphorothioate |

(continued)

| It will be understood that these monomers, when present in an oligonucleotide, are mutually linked by 5'-3'-phosphodiester bonds. | |
|---|---|
| Abbreviation | Nucleotide(s) |
| (Ghd) | 2'-O-hexadecyl-guanosine-3'-phosphate |
| (Ghds) | 2'-O-hexadecyl-guanosine-3'-phosphorothioate |
| Gs | guanosine-3'-phosphorothioate |
| (G2p) | guanosine-2'-phosphate |
| (G2ps) | guanosine-2'-phosphorothioate |
| T | 5'-methyluridine-3'-phosphate |
| Tb | beta-L-thymidine-3'-phosphate |
| Tbs | beta-L-thymidine-3'-phosphorothioate |
| Tf | 2'-fluoro-5-methyluridine-3'-phosphate |
| Tfs | 2'-fluoro-5-methyluridine-3'-phosphorothioate |
| Tgn | thymidine-glycol nucleic acid (GNA) S-Isomer |
| Agn | adenosine- glycol nucleic acid (GNA) S-Isomer |
| Cgn | cytidine-glycol nucleic acid (GNA) S-Isomer |
| Ggn | guanosine-glycol nucleic acid (GNA) S-Isomer |
| Ts | 5-methyluridine-3'-phosphorothioate |
| U | Uridine-3'-phosphate |
| Ub | beta-L-uridine-3'-phosphate |
| Ubs | beta-L-uridine-3'-phosphorothioate |
| Uf | 2'-fluorouridine-3'-phosphate |
| Ufs | 2'-fluorouridine -3'-phosphorothioate |
| (Uhd) | 2'-O-hexadecyl-uridine-3'-phosphate |
| (Uhds) | 2'-O-hexadecyl-uridine-3'-phosphorothioate |
| Us | uridine -3'-phosphorothioate |
| (U2p) | uridine-2'-phosphate |
| (U2ps) | uridine-2'-phosphorothioate |

(continued)

| It will be understood that these monomers, when present in an oligonucleotide, are mutually linked by 5'-3'-phosphodiester bonds. | |
|---|---|
| Abbreviation | Nucleotide(s) |
| N | any nucleotide (G, A, C, T or U) |
| VP | Vinyl phosphonate |
| a | 2'-O-methyladenosine-3'-phosphate |
| as | 2'-O-methyladenosine-3'- phosphorothioate |
| c | 2'-O-methylcytidine-3'-phosphate |
| cs | 2'-O-methylcytidine-3'- phosphorothioate |
| g | 2'-O-methylguanosine-3'-phosphate |
| gs | 2'-O-methylguanosine-3'- phosphorothioate |
| t | 2'-O-methyl-5-methyluridine-3'-phosphate |
| ts | 2'-O-methyl-5-methyluridine-3'-phosphorothioate |
| u | 2'-O-methyluridine-3'-phosphate |
| us | 2'-O-methyluridine-3'-phosphorothioate |
| dA | 2'-deoxyadenosine-3'-phosphate |
| dAs | 2'-deoxyadenosine-3'-phosphorothioate |
| dC | 2'-deoxycytidine-3'-phosphate |
| dCs | 2'-deoxycytidine-3'-phosphorothioate |
| dG | 2'-deoxyuanosine-3'-phosphate |
| dGs | 2'-deoxyguanosine-3'-phosphorothioate |
| dT | 2'-deoxythymidine |
| dTs | 2'-deoxythymidine-3'-phosphorothioate |
| dU | 2'-deoxyuridine |
| s | phosphorothioate linkage |
| L96[1] | N-[tris(GalNAc-alkyl)-amidodecanoyl)]-4-hydroxyprolinol Hyp-(GalNAc-alkyl)3 |
| (Aeo) | 2'-O-methoxyethyladenosine-3'-phosphate |
| (Aeos) | 2'-O-methoxyethyladenosine-3'-phosphorothioate |

(continued)

| It will be understood that these monomers, when present in an oligonucleotide, are mutually linked by 5'-3'-phosphodiester bonds. | |
|---|---|
| Abbreviation | Nucleotide(s) |
| (Geo) | 2'-O-methoxyethylguanosine-3'-phosphate |
| (Geos) | 2'-O-methoxyethylguanosine-3'- phosphorothioate |
| (Teo) | 2'-O-methoxyethyl-5-methyluridine-3'-phosphate |
| (Teos) | 2'-O-methoxyethyl-5-methyluridine-3'-phosphorothioate |
| (m5Ceo) | 2'-O-methoxyethyl-5-methylcytidine-3'-phosphate |
| (m5Ceos) | 2'-O-methoxyethyl-5-methylcytidine-3'-phosphorothioate |

[1]The chemical structure of L96 is as follows:

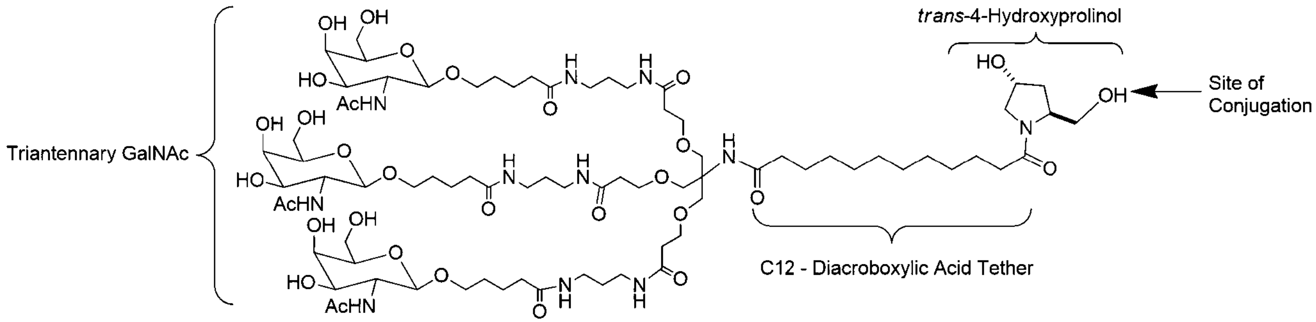

## Experimental Methods

### Bioinformatics

Transcripts

siRNAs targeting the human CA2, "carbonic anhydrase 2" (human: NCBI refseqID NM_000067.3; NCBI GeneID: 760) were generated. The human NM_000067.3 REFSEQ mRNA, version 1, has a length of 1562 bases. Pairs of oligos were generated using bioinformatic methods and ranked, and exemplary pairs of oligos are shown in Tables 3 and 4, Tables 7 and 8, and Tables 9 and 10. Modified sequences are presented in Tables 5, 6, 8, and 10. Unmodified sequences are presented in Tables 3, 4, 7, and 9. The oligos in Tables 3, 5, and 9 were designed for C16 modification and the oligos in Tables 4 and 6 were designed for GalNAc modification.

It is to be understood that, throughout the application, a duplex name without a decimal is equivalent to a duplex name with a decimal which merely references the batch number of the duplex. For example, AD-1560600 is equivalent to AD-1560600.1.

*siRNA Synthesis*

siRNAs were synthesized and annealed using routine methods known in the art.

Briefly, siRNA sequences were synthesized at 1 μmol scale on a Mermade 192 synthesizer (BioAutomation) using the solid support mediated phosphoramidite chemistry. The solid support was controlled pore glass (500 A) loaded with custom GalNAc ligand or universal solid support (AM biochemical). Ancillary synthesis reagents, 2'-F and 2'-O-Methyl RNA and deoxy phosphoramidites were obtained from Thermo-Fisher (Milwaukee, WI) and Hongene (China). 2'F 2'-O-Methyl, GNA (glycol nucleic acids), 5'phosphate and other modifications were introduced using the corresponding phosphoramidites. Synthesis of 3' GalNAc conjugated single strands was performed on a GalNAc modified CPG support. Custom CPG universal solid support was used for the synthesis of antisense single strands. Coupling time for all phosphoramidites (100 mM in acetonitrile) was 5 minutes employing 5-Ethylthio-1H-tetrazole (ETT) as activator (0.6 M in acetonitrile). Phosphorothioate linkages were generated using a 50 mM solution of 3-((Dimethylamino-methylidene) amino)-3H-1,2,4-dithiazole-3-thione (DDTT, obtained from Chemgenes (Wilmington, MA, USA)) in anhydrous acetonitrile/pyridine (1:1 v/v). Oxidation time was 3 minutes. All sequences were synthesized with final removal of the DMT group ("DMT off").

Upon completion of the solid phase synthesis, oligoribonucleotides were cleaved from the solid support and deprotected in sealed 96 deep well plates using 200 μL Aqueous Methylamine reagents at 60°C for 20 minutes. For sequences containing 2' ribo residues (2'-OH) that are protected with a tert-butyl dimethyl silyl (TBDMS) group, a second step deprotection was performed using TEA.3HF (triethylamine trihydro fluoride) reagent. To the methylamine deprotection solution, 200 μL of dimethyl sulfoxide (DMSO) and 300 μL TEA.3HF reagent was added and the solution was incubated for additional 20 minutes at 60°C. At the end of cleavage and deprotection step, the synthesis plate was allowed to come to room temperature and was precipitated by addition of 1mL of acetontile: ethanol mixture (9:1). The plates were cooled at - 80 °C for 2 hours, supernatant decanted carefully with the aid of a multi-channel pipette. The oligonucleotide pellet was re-suspended in 20mM NaOAc buffer and were desalted using a 5 mL HiTrap size exclusion column (GE Healthcare) on an AKTA Purifier System equipped with an A905 autosampler and a Frac 950 fraction collector. Desalted samples were collected in 96-well plates. Samples from each sequence were analyzed by LC-MS to confirm the identity, UV (260 nm) for quantification and a selected set of samples by IEX chromatography to determine purity.

Annealing of single strands was performed on a Tecan liquid handling robot. Equimolar mixture of sense and antisense single strands were combined and annealed in 96 well plates. After combining the complementary single strands, the 96-well plate was sealed tightly and heated in an oven at 100°C for 10 minutes and allowed to come slowly to room temperature over a period 2-3 hours. The concentration of each duplex was normalized to 10 μM in 1X PBS and then submitted for *in vitro* screening assays.

**Table 3. Unmodified Sense and Antisense Strand Sequences of CA2 dsRNA Agents for C16 Modification**

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1560600 | AGAUCGGUGCC GAUUCCUGCA | 7 | 32-52 | UGCAGGAAUCGG CACCGAUCUGG | 142 | 30-52 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1560617 | CGCGACCAUGU CCCAUCACUA | 8 | 69-89 | UAGUGAUGGGAC AUGGUCGCGCU | 143 | 67-89 |
| AD-1560622 | GUACGGCAAAC ACAACGGACA | 9 | 93-113 | UGUCCGUUGUGU UUGCCGUACCC | 144 | 91-113 |
| AD-1560628 | CAAACACAACG GACCUGAGCA | 10 | 99-119 | UGCUCAGGUCCG UUGUGUUUGCC | 145 | 97-119 |
| AD-1560638 | GGACCUGAGCA CUGGCAUAAA | 11 | 109-129 | UUUAUGCCAGUG CUCAGGUCCGU | 146 | 107-129 |
| AD-1560644 | GAGCACUGGCA UAAGGACUUA | 12 | 115-135 | UAAGUCCUUAUG CCAGUGCUCAG | 147 | 113-135 |
| AD-1560655 | GUUGACAUCGA CACUCAUACA | 13 | 166-186 | UGUAUGAGUGUC GAUGUCAACAG | 148 | 164-186 |
| AD-1560665 | ACACUCAUACA GCCAAGUAUA | 14 | 176-196 | UAUACUUGGCUG UAUGAGUGUCG | 149 | 174-196 |
| AD-1560672 | UACAGCCAAGU AUGACCCUUA | 15 | 183-203 | UAAGGGUCAUAC UUGGCUGUAUG | 150 | 181-203 |
| AD-1560678 | CAAGUAUGACC CUUCCCUGAA | 16 | 189-209 | UUCAGGGAAGGG UCAUACUUGGC | 151 | 187-209 |
| AD-1560684 | UGUCUGUUUCC UAUGAUCAAA | 17 | 215-235 | UUUGAUCAUAGG AAACAGACAGG | 152 | 213-235 |
| AD-1560693 | CCUAUGAUCAA GCAACUUCCA | 18 | 224-244 | UGGAAGUUGCUU GAUCAUAGGAA | 153 | 222-244 |
| AD-1560701 | CAAGCAACUUC CCUGAGGAUA | 19 | 232-252 | UAUCCUCAGGGA AGUUGCUUGAU | 154 | 230-252 |
| AD-1560711 | CCCUGAGGAUC CUCAACAAUA | 20 | 242-262 | UAUUGUUGAGGA UCCUCAGGGAA | 155 | 240-262 |
| AD-1560720 | UCCUCAACAAU GGUCAUGCUA | 21 | 251-271 | UAGCAUGACCAU UGUUGAGGAUC | 156 | 249-271 |
| AD-1560726 | ACAAUGGUCAU GCUUUCAACA | 22 | 257-277 | UGUUGAAAGCAU GACCAUUGUUG | 157 | 255-277 |
| AD-1560735 | AUGCUUUCAAC GUGGAGUUUA | 23 | 266-286 | UAAACUCCACGU UGAAAGCAUGA | 158 | 264-286 |
| AD-1560745 | AACGUGGAGUU UGAUGACUCA | 24 | 274-294 | UGAGUCAUCAAA CUCCACGUUGA | 159 | 272-294 |
| AD-1560752 | UGAUGACUCUC AGGACAAAGA | 25 | 285-305 | UCUUUGUCCUGA GAGUCAUCAAA | 160 | 283-305 |
| AD-1560759 | UCUCAGGACAA AGCAGUGCUA | 26 | 292-312 | UAGCACUGCUUU GUCCUGAGAGU | 161 | 290-312 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1560765 | GACAAAGCAGU GCUCAAGGGA | 27 | 298-318 | UCCCUUGAGCAC UGCUUUGUCCU | 162 | 296-318 |
| AD-1560777 | UGGCACUUACA GAUUGAUUCA | 28 | 330-350 | UGAAUCAAUCUG UAAGUGCCAUC | 163 | 328-350 |
| AD-1560783 | UUACAGAUUGA UUCAGUUUCA | 29 | 336-356 | UGAAACUGAAUC AAUCUGUAAGU | 164 | 334-356 |
| AD-1560792 | AUUCAGUUUCA CUUUCACUGA | 30 | 346-366 | UCAGUGAAAGUG AAACUGAAUCA | 165 | 344-366 |
| AD-1560798 | UCACUUGAUGG ACAAGGUUCA | 31 | 370-390 | UGAACCUUGUCC AUCAAGUGAAC | 166 | 368-390 |
| AD-1560804 | GAUGGACAAGG UUCAGAGCAA | 32 | 376-396 | UUGCUCUGAACC UUGUCCAUCAA | 167 | 374-396 |
| AD-1560810 | CAAGGUUCAGA GCAUACUGUA | 33 | 382-402 | UACAGUAUGCUC UGAACCUUGUC | 168 | 380-402 |
| AD-1560816 | UCAGAGCAUAC UGUGGAUAAA | 34 | 388-408 | UUUAUCCACAGU AUGCUCUGAAC | 169 | 386-408 |
| AD-1560837 | AAGAAAUAUGC UGCAGAACUA | 35 | 409-429 | UAGUUCUGCAGC AUAUUUCUUUU | 170 | 407-429 |
| AD-1560845 | UAUGCUGCAGA ACUUCACUUA | 36 | 415-435 | UAAGUGAAGUUC UGCAGCAUAUU | 171 | 413-435 |
| AD-1560851 | GCAGAACUUCA CUUGGUUCAA | 37 | 421-441 | UUGAACCAAGUG AAGUUCUGCAG | 172 | 419-441 |
| AD-1560843 | CUUCACUUGGU UCACUGGAAA | 38 | 817-837 | UUUCCAGUGAAC CAAGUGAAGUU | 173 | |
| AD-1560862 | UUUGGGAAAGC UGUGCAGCAA | 39 | 463-483 | UUGCUGCACAGC UUUCCCAAAAU | 174 | 461-483 |
| AD-1560874 | GUGCAGCAACC UGAUGGACUA | 40 | 475-495 | UAGUCCAUCAGG UUGCUGCACAG | 175 | 473-495 |
| AD-1560880 | CAACCUGAUGG ACUGGCCGUA | 41 | 481-501 | UACGGCCAGUCC AUCAGGUUGCU | 176 | 479-501 |
| AD-1560892 | CUGGCCGUUCU AGGUAUUUUA | 42 | 493-513 | UAAAAUACCUAG AACGGCCAGUC | 177 | 491-513 |
| AD-1560895 | UGAAGGUUGGC AGCGCUAAAA | 43 | 515-535 | UUUUAGCGCUGC CAACCUUCAAA | 178 | 513-535 |
| AD-1560904 | GCAGCGCUAAA CCGGGCCUUA | 44 | 524-544 | UAAGGCCCGGUU UAGCGCUGCCA | 179 | 522-544 |
| AD-1560915 | CCGGGCCUUCA GAAAGUUGUA | 45 | 535-555 | UACAACUUUCUG AAGGCCCGGUU | 180 | 533-555 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1560921 | CUUCAGAAAGU UGUUGAUGUA | 46 | 541-561 | UACAUCAACAAC UUUCUGAAGGC | 181 | 539-561 |
| AD-1560930 | GUUGUUGAUGU GCUGGAUUCA | 47 | 550-570 | UGAAUCCAGCAC AUCAACAACUU | 182 | 548-570 |
| AD-1560941 | GCUGGAUUCCA UUAAAACAAA | 48 | 561-581 | UUUGUUUUAAUG GAAUCCAGCAC | 183 | 559-581 |
| AD-1560948 | UCCAUUAAAAC AAAGGGCAAA | 49 | 568-588 | UUUGCCCUUUGU UUUAAUGGAAU | 184 | 566-588 |
| AD-1560954 | AAAACAAAGGG CAAGAGUGCA | 50 | 574-594 | UGCACUCUUGCC CUUUGUUUUAA | 185 | 572-594 |
| AD-1560963 | GGCAAGAGUGC UGACUUCACA | 51 | 583-603 | UGUGAAGUCAGC ACUCUUGCCCU | 186 | 581-603 |
| AD-1560970 | GUGCUGACUUC ACUAACUUCA | 52 | 590-610 | UGAAGUUAGUGA AGUCAGCACUC | 187 | 588-610 |
| AD-1560976 | ACUUCACUAAC UUCGAUCCUA | 53 | 596-616 | UAGGAUCGAAGU UAGUGAAGUCA | 188 | 594-616 |
| AD-1560989 | CGAUCCUCGUG GCCUCCUUCA | 54 | 609-629 | UGAAGGAGGCCA CGAGGAUCGAA | 189 | 607-629 |
| AD-1560996 | UCGUGGCCUCC UUCCUGAAUA | 55 | 615-635 | UAUUCAGGAAGG AGGCCACGAGG | 190 | 613-635 |
| AD-1561002 | CCUCCUUCCUG AAUCCUUGGA | 56 | 621-641 | UCCAAGGAUUCA GGAAGGAGGCC | 191 | 619-641 |
| AD-1561009 | CCUGAAUCCUU GGAUUACUGA | 57 | 628-648 | UCAGUAAUCCAA GGAUUCAGGAA | 192 | 626-648 |
| AD-1561015 | UCCUUGGAUUA CUGGACCUAA | 58 | 634-654 | UUAGGUCCAGUA AUCCAAGGAUU | 193 | 632-654 |
| AD-1561031 | CCUACCCAGGC UCACUGACCA | 59 | 650-670 | UGGUCAGUGAGC CUGGGUAGGUC | 194 | 648-670 |
| AD-1561037 | CCUCUUCUGGA AUGUGUGACA | 60 | 676-696 | UGUCACACAUUC CAGAAGAGGAG | 195 | 674-696 |
| AD-1561043 | UGGAAUGUGUG ACCUGGAUUA | 61 | 683-703 | UAAUCCAGGUCA CACAUUCCAGA | 196 | 681-703 |
| AD-1561050 | UGUGACCUGGA UUGUGCUCAA | 62 | 690-710 | UUGAGCACAAUC CAGGUCACACA | 197 | 688-710 |
| AD-1561056 | CUGGAUUGUGC UCAAGGAACA | 63 | 696-716 | UGUUCCUUGAGC ACAAUCCAGGU | 198 | 694-716 |
| AD-1561066 | CUCAAGGAACC CAUCAGCGUA | 64 | 706-726 | UACGCUGAUGGG UUCCUUGAGCA | 199 | 704-726 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1561072 | GAACCCAUCAG CGUCAGCAGA | 65 | 712-732 | UCUGCUGACGCU GAUGGGUUCCU | 200 | 710-732 |
| AD-1475424 | AGAACUGAUGG UGGACAACUA | 66 | 719-739 | UAGUUGUCCACC AUCAGUUCUUC | 201 | 717-739 |
| AD-1561092 | CGAGCAGGUGU UGAAAUUCCA | 67 | 732-752 | UGGAAUUUCAAC ACCUGCUCGCU | 202 | 730-752 |
| AD-1561100 | GGUGUUGAAAU UCCGUAAACA | 68 | 738-758 | UGUUUACGGAAU UUCAACACCUG | 203 | 736-758 |
| AD-1561106 | GAAAUUCCGUA AACUUAACUA | 69 | 744-764 | UAGUUAAGUUUA CGGAAUUUCAA | 204 | 742-764 |
| AD-1561112 | CCGUAAACUUA ACUUCAAUGA | 70 | 750-770 | UCAUUGAAGUUA AGUUUACGGAA | 205 | 748-770 |
| AD-1561116 | GAGGGUGAACC CGAAGAACUA | 71 | 772-792 | UAGUUCUUCGGG UUCACCCUCCC | 206 | 770-792 |
| AD-1561122 | GAACCCGAAGA ACUGAUGGUA | 72 | 778-798 | UACCAUCAGUUC UUCGGGUUCAC | 207 | 776-798 |
| AD-1561130 | AUGGUGGACAA CUGGCGCCCA | 73 | 793-813 | UGGGCGCCAGUU GUCCACCAUCA | 208 | 791-813 |
| AD-1561146 | CCAGCUCAGCC ACUGAAGAAA | 74 | 811-831 | UUUCUUCAGUGG CUGAGCUGGGC | 209 | 809-831 |
| AD-1561152 | CAGCCACUGAA GAACAGGCAA | 75 | 817-837 | UUGCCUGUUCUU CAGUGGCUGAG | 210 | 815-837 |
| AD-1561158 | CUGAAGAACAG GCAAAUCAAA | 76 | 823-843 | UUUGAUUUGCCU GUUCUUCAGUG | 211 | 821-843 |
| AD-1446763 | UCACUGGAACA CCAAAUAUGA | 77 | 828-848 | UCAUAUUUGGUG UUCCAGUGAAC | 212 | 826-848 |
| AD-1561168 | GGCAAAUCAAA GCUUCCUUCA | 78 | 833-853 | UGAAGGAAGCUU UGAUUUGCCUG | 213 | 831-853 |
| AD-1561175 | CAAAGCUUCCU UCAAAUAAGA | 79 | 840-860 | UCUUAUUUGAAG GAAGCUUUGAU | 214 | 838-860 |
| AD-1561181 | UUCCUUCAAAU AAGAUGGUCA | 80 | 846-866 | UGACCAUCUUAU UUGAAGGAAGC | 215 | 844-866 |
| AD-1561190 | AUAAGAUGGUC CCAUAGUCUA | 81 | 855-875 | UAGACUAUGGGA CCAUCUUAUUU | 216 | 853-875 |
| AD-1561196 | UGGUCCCAUAG UCUGUAUCCA | 82 | 861-881 | UGGAUACAGACU AUGGGACCAUC | 217 | 859-881 |
| AD-1561203 | AUAGUCUGUAU CCAAAUAAUA | 83 | 868-888 | UAUUAUUUGGAU ACAGACUAUGG | 218 | 866-888 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1561210 | GUAUCCAAAUA AUGAAUCUUA | 84 | 875-895 | UAAGAUUCAUUA UUUGGAUACAG | 219 | 873-895 |
| AD-1561218 | AUAAUGAAUCU UCGGGUGUUA | 85 | 883-903 | UAACACCCGAAG AUUCAUUAUUU | 220 | 881-903 |
| AD-1561225 | AUCUUCGGGUG UUUCCCUUUA | 86 | 890-910 | UAAAGGGAAACA CCCGAAGAUUC | 221 | 888-910 |
| AD-1561231 | GGGUGUUUCCC UUUAGCUAAA | 87 | 896-916 | UUUAGCUAAAGG GAAACACCCGA | 222 | 894-916 |
| AD-1561239 | CCCUUUAGCUA AGCACAGAUA | 88 | 904-924 | UAUCUGUGCUUA GCUAAAGGGAA | 223 | 902-924 |
| AD-1561245 | AGCUAAGCACA GAUCUACCUA | 89 | 910-930 | UAGGUAGAUCUG UGCUUAGCUAA | 224 | 908-930 |
| AD-1561254 | CAGAUCUACCU UGGUGAUUUA | 90 | 919-939 | UAAAUCACCAAG GUAGAUCUGUG | 225 | 917-939 |
| AD-1561261 | ACCUUGGUGAU UUGGACCCUA | 91 | 926-946 | UAGGGUCCAAAU CACCAAGGUAG | 226 | 924-946 |
| AD-1561272 | UUGGACCCUGG UUGCUUUGUA | 92 | 937-957 | UACAAAGCAACC AGGGUCCAAAU | 227 | 935-957 |
| AD-1561279 | CUGGUUGCUUU GUGUCUAGUA | 93 | 944-964 | UACUAGACACAA AGCAACCAGGG | 228 | 942-964 |
| AD-1561285 | GCUUUGUGUCU AGUUUUCUAA | 94 | 950-970 | UUAGAAAACUAG ACACAAAGCAA | 229 | 948-970 |
| AD-1561294 | CUAGUUUUCUA GACCCUUCAA | 95 | 959-979 | UUGAAGGGUCUA GAAAACUAGAC | 230 | 957-979 |
| AD-1561300 | UUCUAGACCCU UCAUCUCUUA | 96 | 965-985 | UAAGAGAUGAAG GGUCUAGAAAA | 231 | 963-985 |
| AD-1561306 | ACCCUUCAUCU CUUACUUGAA | 97 | 971-991 | UUCAAGUAAGAG AUGAAGGGUCU | 232 | 969-991 |
| AD-1561313 | AUCUCUUACUU GAUAGACUUA | 98 | 978-998 | UAAGUCUAUCAA GUAAGAGAUGA | 233 | 976-998 |
| AD-1561319 | UACUUGAUAGA CUUACUAAUA | 99 | 984-1004 | UAUUAGUAAGUC UAUCAAGUAAG | 234 | 982-1004 |
| AD-1561327 | CUUACUAAUAA AAUGUGAAGA | 100 | 995-1015 | UCUUCACAUUUU AUUAGUAAGUC | 235 | 993-1015 |
| AD-1561336 | AAAAUGUGAAG ACUAGACCAA | 101 | 1004-1024 | UUGGUCUAGUCU UCACAUUUUAU | 236 | 1002-1024 |
| AD-1561342 | UGAAGACUAGA CCAAUUGUCA | 102 | 1010-1030 | UGACAAUUGGUC UAGUCUUCACA | 237 | 1008-1030 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1561349 | UAGACCAAUUG UCAUGCUUGA | 103 | 1017-1037 | UCAAGCAUGACA AUUGGUCUAGU | 238 | 1015-1037 |
| AD-1561360 | UCAUGCUUGAC ACAACUGCUA | 104 | 1028-1048 | UAGCAGUUGUGU CAAGCAUGACA | 239 | 1026-1048 |
| AD-1561366 | UUGACACAACU GCUGUGGCUA | 105 | 1034-1054 | UAGCCACAGCAG UUGUGUCAAGC | 240 | 1032-1054 |
| AD-1561378 | CUGUGGCUGGU UGGUGCUUUA | 106 | 1046-1066 | UAAAGCACCAAC CAGCCACAGCA | 241 | 1044-1066 |
| AD-1561384 | CUGGUUGGUGC UUUGUUUAUA | 107 | 1052-1072 | UAUAAACAAAGC ACCAACCAGCC | 242 | 1050-1072 |
| AD-1561390 | GGUGCUUUGUU UAUGGUAGUA | 108 | 1058-1078 | UACUACCAUAAA CAAAGCACCAA | 243 | 1056-1078 |
| AD-1561396 | UUGUUUAUGGU AGUAGUUUUA | 109 | 1064-1084 | UAAAACUACUAC CAUAAACAAAG | 244 | 1062-1084 |
| AD-1561402 | UGGUAGUAGUU UUUCUGUAAA | 110 | 1071-1091 | UUUACAGAAAAA CUACUACCAUA | 245 | 1069-1091 |
| AD-1561408 | UAGUUUUUCUG UAACACAGAA | 111 | 1077-1097 | UUCUGUGUUACA GAAAAACUACU | 246 | 1075-1097 |
| AD-1561414 | UUCUGUAACAC AGAAUAUAGA | 112 | 1083-1103 | UCUAUAUUCUGU GUUACAGAAAA | 247 | 1081-1103 |
| AD-1561422 | CACAGAAUAUA GGAUAAGAAA | 113 | 1091-1111 | UUUCUUAUCCUA UAUUCUGUGUU | 248 | 1089-1111 |
| AD-1561433 | AGAAUAAAGUA CCUUGACUUA | 114 | 1114-1134 | UAAGUCAAGGUA CUUUAUUCUUA | 249 | 1112-1134 |
| AD-1561444 | CUUGACUUUGU UCACAGCAUA | 115 | 1126-1146 | UAUGCUGUGAAC AAAGUCAAGGU | 250 | 1124-1146 |
| AD-1561450 | UUUGUUCACAG CAUGUAGGGA | 116 | 1132-1152 | UCCCUACAUGCU GUGAACAAAGU | 251 | 1130-1152 |
| AD-1561456 | CACAGCAUGUA GGGUGAUGAA | 117 | 1138-1158 | UUCAUCACCCUA CAUGCUGUGAA | 252 | 1136-1158 |
| AD-1561465 | UAGGGUGAUGA GCACUCACAA | 118 | 1147-1167 | UUGUGAGUGCUC AUCACCCUACA | 253 | 1145-1167 |
| AD-1561471 | GAUGAGCACUC ACAAUUGUUA | 119 | 1153-1173 | UAACAAUUGUGA GUGCUCAUCAC | 254 | 1151-1173 |
| AD-1561478 | ACUCACAAUUG UUGACUAAAA | 120 | 1160-1180 | UUUUAGUCAACA AUUGUGAGUGC | 255 | 1158-1180 |
| AD-1561489 | UUGACUAAAAU GCUGCUUUUA | 121 | 1171-1191 | UAAAAGCAGCAU UUUAGUCAACA | 256 | 1169-1191 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1561498 | AUGCUGCUUUUAAAACAUAGA | 122 | 1180-1200 | UCUAUGUUUUAAAGCAGCAUUU | 257 | 1178-1200 |
| AD-1561504 | CUUUUAAAACAUAGGAAAGUA | 123 | 1186-1206 | UACUUUCCUAUGUUUUAAAAGCA | 258 | 1184-1206 |
| AD-1561513 | CAUAGGAAAGUAGAAUGGUUA | 124 | 1195-1215 | UAACCAUUCUACUUUCCUAUGUU | 259 | 1193-1215 |
| AD-1561521 | AGUAGAAUGGUUGAGUGCAAA | 125 | 1203-1223 | UUUGCACUCAACCAUUCUACUUU | 260 | 1201-1223 |
| AD-1561527 | AUGGUUGAGUGCAAAUCCAUA | 126 | 1209-1229 | UAUGGAUUUGCACUCAACCAUUC | 261 | 1207-1229 |
| AD-1561534 | AGUGCAAAUCCAUAGCACAAA | 127 | 1216-1236 | UUUGUGCUAUGGAUUUGCACUCA | 262 | 1214-1236 |
| AD-1561542 | UCCAUAGCACAAGAUAAAUUA | 128 | 1224-1244 | UAAUUUAUCUUGUGCUAUGGAUU | 263 | 1222-1244 |
| AD-1561551 | CAAGAUAAAUUGAGCUAGUUA | 129 | 1233-1253 | UAACUAGCUCAAUUUAUCUUGUG | 264 | 1231-1253 |
| AD-1561562 | GAGCUAGUUAAGGCAAAUCAA | 130 | 1244-1264 | UUGAUUUGCCUUAACUAGCUCAA | 265 | 1242-1264 |
| AD-1561570 | UAAGGCAAAUCAGGUAAAAUA | 131 | 1252-1272 | UAUUUUACCUGAUUUGCCUUAAC | 266 | 1250-1272 |
| AD-1561581 | AGGUAAAAUAGUCAUGAUUCA | 132 | 1263-1283 | UGAAUCAUGACUAUUUUACCUGA | 267 | 1261-1283 |
| AD-1561591 | GUCAUGAUUCUAUGUAAUGUA | 133 | 1273-1293 | UACAUUACAUAGAAUCAUGACUA | 268 | 1271-1293 |
| AD-1561601 | UAUGUAAUGUAAACCAGAAAA | 134 | 1283-1303 | UUUUCUGGUUUACAUUACAUAGA | 269 | 1281-1303 |
| AD-1561613 | UCAUGAUUUCAAGAUGUUAUA | 135 | 1313-1333 | UAUAACAUCUUGAAAUCAUGAAC | 270 | 1311-1333 |
| AD-1561651 | CUUUUGAAUUACAGAGAUAUA | 136 | 1411-1431 | UAUAUCUCUGUAAUUCAAAAGUC | 271 | 1409-1431 |
| AD-1561679 | UUAGAGUUGUGAUACAGAGUA | 137 | 1463-1483 | UACUCUGUAUCACAACUCUAAUU | 272 | 1461-1483 |
| AD-1561686 | UACAGAGUAUAUUUCCAUUCA | 138 | 1475-1495 | UGAAUGGAAAUAUACUCUGUAUC | 273 | 1473-1495 |
| AD-1561694 | AUAUUUCCAUUCAGACAAUAA | 139 | 1483-1503 | UUAUUGUCUGAAUGGAAAUAUAC | 274 | 1481-1503 |
| AD-1561703 | UUCAGACAAUAUAUCAUAACA | 140 | 1492-1512 | UGUUAUGAUAUAUUGUCUGAAUG | 275 | 1490-1512 |

(continued)

| Duplex Name | Sense Sequence 5' to 3' | SEQ ID NO: | Range in NM_0000 67.3 | Antisense Sequence 5' to 3' | SEQ ID NO: | Range in NM_000 067.3 |
|---|---|---|---|---|---|---|
| AD-1447598 | UUGUGAUACAG AGUAUAUUUA | 141 | 1835-1855 | UAAAUAUACUCU GUAUCACAACU | 276 | 1833-1855 |

**Table 4. Unmodified Sense and Antisense Strand Sequences of CA2 dsRNA Agents for GalNAc Modification**

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1559459 | AGAUCGGUGCC GAUUCCUGCU | 277 | 32-52 | AGCAGGAAUCGG CACCGAUCUGG | 412 | 30-52 |
| AD-1559476 | CGCGACCAUGU CCCAUCACUU | 278 | 69-89 | AAGUGAUGGGAC AUGGUCGCGCU | 413 | 67-89 |
| AD-1559481 | GUACGGCAAAC ACAACGGACU | 279 | 93-113 | AGUCCGUUGUGU UUGCCGUACCC | 414 | 91-113 |
| AD-1559487 | CAAACACAACG GACCUGAGCU | 280 | 99-119 | AGCUCAGGUCCG UUGUGUUUGCC | 415 | 97-119 |
| AD-1559497 | GGACCUGAGCA CUGGCAUAAU | 281 | 109-129 | AUUAUGCCAGUG CUCAGGUCCGU | 416 | 107-129 |
| AD-1559503 | GAGCACUGGCA UAAGGACUUU | 282 | 115-135 | AAAGUCCUUAUG CCAGUGCUCAG | 417 | 113-135 |
| AD-1559514 | GUUGACAUCGA CACUCAUACU | 283 | 166-186 | AGUAUGAGUGUC GAUGUCAACAG | 418 | 164-186 |
| AD-1559524 | ACACUCAUACA GCCAAGUAUU | 284 | 176-196 | AAUACUUGGCUG UAUGAGUGUCG | 419 | 174-196 |
| AD-1559531 | UACAGCCAAGU AUGACCCUUU | 285 | 183-203 | AAAGGGUCAUAC UUGGCUGUAUG | 420 | 181-203 |
| AD-1559537 | CAAGUAUGACC CUUCCCUGAU | 286 | 189-209 | AUCAGGGAAGGG UCAUACUUGGC | 421 | 187-209 |
| AD-1559543 | UGUCUGUUUCC UAUGAUCAAU | 287 | 215-235 | AUUGAUCAUAGG AAACAGACAGG | 422 | 213-235 |
| AD-1559552 | CCUAUGAUCAA GCAACUUCCU | 288 | 224-244 | AGGAAGUUGCUU GAUCAUAGGAA | 423 | 222-244 |
| AD-1559560 | CAAGCAACUUC CCUGAGGAUU | 289 | 232-252 | AAUCCUCAGGGA AGUUGCUUGAU | 424 | 230-252 |
| AD-1559570 | CCCUGAGGAUC CUCAACAAUU | 290 | 242-262 | AAUUGUUGAGGA UCCUCAGGGAA | 425 | 240-262 |
| AD-1559579 | UCCUCAACAAU GGUCAUGCUU | 291 | 251-271 | AAGCAUGACCAU UGUUGAGGAUC | 426 | 249-271 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1559585 | ACAAUGGUCAUGCUUUCAACU | 292 | 257-277 | AGUUGAAAGCAUGACCAUUGUUG | 427 | 255-277 |
| AD-1559594 | AUGCUUUCAACGUGGAGUUUU | 293 | 266-286 | AAAACUCCACGUUGAAAGCAUGA | 428 | 264-286 |
| AD-1559602 | AACGUGGAGUUUGAUGACUCU | 294 | 274-294 | AGAGUCAUCAAACUCCACGUUGA | 429 | 272-294 |
| AD-1559613 | UGAUGACUCUCAGGACAAAGU | 295 | 285-305 | ACUUUGUCCUGAGAGUCAUCAAA | 430 | 283-305 |
| AD-1559620 | UCUCAGGACAAAGCAGUGCUU | 296 | 292-312 | AAGCACUGCUUUGUCCUGAGAGU | 431 | 290-312 |
| AD-1559626 | GACAAAGCAGUGCUCAAGGGU | 297 | 298-318 | ACCCUUGAGCACUGCUUUGUCCU | 432 | 296-318 |
| AD-1559638 | UGGCACUUACAGAUUGAUUCU | 298 | 330-350 | AGAAUCAAUCUGUAAGUGCCAUC | 433 | 328-350 |
| AD-1559644 | UUACAGAUUGAUUCAGUUUCU | 299 | 336-356 | AGAAACUGAAUCAAUCUGUAAGU | 434 | 334-356 |
| AD-1559654 | AUUCAGUUUCACUUUCACUGU | 300 | 346-366 | ACAGUGAAAGUGAAACUGAAUCA | 435 | 344-366 |
| AD-1559660 | UCACUUGAUGGACAAGGUUCU | 301 | 370-390 | AGAACCUUGUCCAUCAAGUGAAC | 436 | 368-390 |
| AD-1559666 | GAUGGACAAGGUUCAGAGCAU | 302 | 376-396 | AUGCUCUGAACCUUGUCCAUCAA | 437 | 374-396 |
| AD-1559672 | CAAGGUUCAGAGCAUACUGUU | 303 | 382-402 | AACAGUAUGCUCUGAACCUUGUC | 438 | 380-402 |
| AD-1559678 | UCAGAGCAUACUGUGGAUAAU | 304 | 388-408 | AUUAUCCACAGUAUGCUCUGAAC | 439 | 386-408 |
| AD-1559699 | AAGAAAUAUGCUGCAGAACUU | 305 | 409-429 | AAGUUCUGCAGCAUAUUUCUUUU | 440 | 407-429 |
| AD-1559705 | UAUGCUGCAGAACUUCACUUU | 306 | 415-435 | AAAGUGAAGUUCUGCAGCAUAUU | 441 | 413-435 |
| AD-1559711 | GCAGAACUUCACUUGGUUCAU | 307 | 421-441 | AUGAACCAAGUGAAGUUCUGCAG | 442 | 419-441 |
| AD-1559717 | CUUCACUUGGUUCACUGGAAU | 308 | 427-447 | AUUCCAGUGAACCAAGUGAAGUU | 443 | 425-447 |
| AD-1559728 | UCACUGGAACACCAAAUAUGU | 309 | 438-458 | ACAUAUUUGGUGUUCCAGUGAAC | 444 | 436-458 |
| AD-1559735 | UUUGGGAAAGCUGUGCAGCAU | 310 | 463-483 | AUGCUGCACAGCUUUCCCAAAAU | 445 | 461-483 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1559747 | GUGCAGCAACC UGAUGGACUU | 311 | 475-495 | AAGUCCAUCAGG UUGCUGCACAG | 446 | 473-495 |
| AD-1559753 | CAACCUGAUGG ACUGGCCGUU | 312 | 481-501 | AACGGCCAGUCC AUCAGGUUGCU | 447 | 479-501 |
| AD-1559765 | CUGGCCGUUCU AGGUAUUUUU | 313 | 493-513 | AAAAAUACCUAG AACGGCCAGUC | 448 | 491-513 |
| AD-1559768 | UGAAGGUUGGC AGCGCUAAAU | 314 | 515-535 | AUUUAGCGCUGC CAACCUUCAAA | 449 | 513-535 |
| AD-1559777 | GCAGCGCUAAA CCGGGCCUUU | 315 | 524-544 | AAAGGCCCGGUU UAGCGCUGCCA | 450 | 522-544 |
| AD-1559788 | CCGGGCCUUCA GAAAGUUGUU | 316 | 535-555 | AACAACUUUCUG AAGGCCCGGUU | 451 | 533-555 |
| AD-1559794 | CUUCAGAAAGU UGUUGAUGUU | 317 | 541-561 | AACAUCAACAAC UUUCUGAAGGC | 452 | 539-561 |
| AD-1559803 | GUUGUUGAUGU GCUGGAUUCU | 318 | 550-570 | AGAAUCCAGCAC AUCAACAACUU | 453 | 548-570 |
| AD-1559814 | GCUGGAUUCCA UUAAAACAAU | 319 | 561-581 | AUUGUUUUAAUG GAAUCCAGCAC | 454 | 559-581 |
| AD-1559821 | UCCAUUAAAAC AAAGGGCAAU | 320 | 568-588 | AUUGCCCUUUGU UUUAAUGGAAU | 455 | 566-588 |
| AD-1559827 | AAAACAAAGGG CAAGAGUGCU | 321 | 574-594 | AGCACUCUUGCC CUUUGUUUUAA | 456 | 572-594 |
| AD-1559836 | GGCAAGAGUGC UGACUUCACU | 322 | 583-603 | AGUGAAGUCAGC ACUCUUGCCCU | 457 | 581-603 |
| AD-1559843 | GUGCUGACUUC ACUAACUUCU | 323 | 590-610 | AGAAGUUAGUGA AGUCAGCACUC | 458 | 588-610 |
| AD-1559849 | ACUUCACUAAC UUCGAUCCUU | 324 | 596-616 | AAGGAUCGAAGU UAGUGAAGUCA | 459 | 594-616 |
| AD-1559862 | CGAUCCUCGUG GCCUCCUUCU | 325 | 609-629 | AGAAGGAGGCCA CGAGGAUCGAA | 460 | 607-629 |
| AD-1559868 | UCGUGGCCUCC UUCCUGAAUU | 326 | 615-635 | AAUUCAGGAAGG AGGCCACGAGG | 461 | 613-635 |
| AD-1559874 | CCUCCUUCCUG AAUCCUUGGU | 327 | 621-641 | ACCAAGGAUUCA GGAAGGAGGCC | 462 | 619-641 |
| AD-1559881 | CCUGAAUCCUU GGAUUACUGU | 328 | 628-648 | ACAGUAAUCCAA GGAUUCAGGAA | 463 | 626-648 |
| AD-1559887 | UCCUUGGAUUA CUGGACCUAU | 329 | 634-654 | AUAGGUCCAGUA AUCCAAGGAUU | 464 | 632-654 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1559903 | CCUACCCAGGCUCACUGACCU | 330 | 650-670 | AGGUCAGUGAGCCUGGGUAGGUC | 465 | 648-670 |
| AD-1559909 | CCUCUUCUGGAAUGUGUGACU | 331 | 676-696 | AGUCACACAUUCCAGAAGAGGAG | 466 | 674-696 |
| AD-1559916 | UGGAAUGUGUGACCUGGAUUU | 332 | 683-703 | AAAUCCAGGUCACACAUUCCAGA | 467 | 681-703 |
| AD-1559923 | UGUGACCUGGAUUGUGCUCAU | 333 | 690-710 | AUGAGCACAAUCCAGGUCACACA | 468 | 688-710 |
| AD-1559929 | CUGGAUUGUGCUCAAGGAACU | 334 | 696-716 | AGUUCCUUGAGCACAAUCCAGGU | 469 | 694-716 |
| AD-1559939 | CUCAAGGAACCCAUCAGCGUU | 335 | 706-726 | AACGCUGAUGGGUUCCUUGAGCA | 470 | 704-726 |
| AD-1559945 | GAACCCAUCAGCGUCAGCAGU | 336 | 712-732 | ACUGCUGACGCUGAUGGGUUCCU | 471 | 710-732 |
| AD-1559965 | CGAGCAGGUGUUGAAAUUCCU | 337 | 732-752 | AGGAAUUUCAACACCUGCUCGCU | 472 | 730-752 |
| AD-1559971 | GGUGUUGAAAUUCCGUAAACU | 338 | 738-758 | AGUUUACGGAAUUUCAACACCUG | 473 | 736-758 |
| AD-1559977 | GAAAUUCCGUAAACUUAACUU | 339 | 744-764 | AAGUUAAGUUUACGGAAUUUCAA | 474 | 742-764 |
| AD-1559983 | CCGUAAACUUAACUUCAAUGU | 340 | 750-770 | ACAUUGAAGUUAAGUUUACGGAA | 475 | 748-770 |
| AD-1559987 | GAGGGUGAACCCGAAGAACUU | 341 | 772-792 | AAGUUCUUCGGGUUCACCCUCCC | 476 | 770-792 |
| AD-1559993 | GAACCCGAAGAACUGAUGGUU | 342 | 778-798 | AACCAUCAGUUCUUCGGGUUCAC | 477 | 776-798 |
| AD-1560001 | AGAACUGAUGGUGGACAACUU | 343 | 786-806 | AAGUUGUCCACCAUCAGUUCUUC | 478 | 784-806 |
| AD-1560008 | AUGGUGGACAACUGGCGCCCU | 344 | 793-813 | AGGGCGCCAGUUGUCCACCAUCA | 479 | 791-813 |
| AD-1560024 | CCAGCUCAGCCACUGAAGAAU | 345 | 811-831 | AUUCUUCAGUGGCUGAGCUGGGC | 480 | 809-831 |
| AD-1560030 | CAGCCACUGAAGAACAGGCAU | 346 | 817-837 | AUGCCUGUUCUUCAGUGGCUGAG | 481 | 815-837 |
| AD-1560036 | CUGAAGAACAGGCAAAUCAAU | 347 | 823-843 | AUUGAUUUGCCUGUUCUUCAGUG | 482 | 821-843 |
| AD-1560046 | GGCAAAUCAAAGCUUCCUUCU | 348 | 833-853 | AGAAGGAAGCUUUGAUUUGCCUG | 483 | 831-853 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1560053 | CAAAGCUUCCU UCAAAUAAGU | 349 | 840-860 | ACUUAUUUGAAG GAAGCUUUGAU | 484 | 838-860 |
| AD-1560059 | UUCCUUCAAAU AAGAUGGUCU | 350 | 846-866 | AGACCAUCUUAU UUGAAGGAAGC | 485 | 844-866 |
| AD-1560068 | AUAAGAUGGUC CCAUAGUCUU | 351 | 855-875 | AAGACUAUGGGA CCAUCUUAUUU | 486 | 853-875 |
| AD-1560074 | UGGUCCCAUAG UCUGUAUCCU | 352 | 861-881 | AGGAUACAGACU AUGGGACCAUC | 487 | 859-881 |
| AD-1560081 | AUAGUCUGUAU CCAAAUAAUU | 353 | 868-888 | AAUUAUUUGGAU ACAGACUAUGG | 488 | 866-888 |
| AD-1560088 | GUAUCCAAAUA AUGAAUCUUU | 354 | 875-895 | AAAGAUUCAUUA UUUGGAUACAG | 489 | 873-895 |
| AD-1560096 | AUAAUGAAUCU UCGGGUGUUU | 355 | 883-903 | AAACACCCGAAG AUUCAUUAUUU | 490 | 881-903 |
| AD-1560103 | AUCUUCGGGUG UUUCCCUUUU | 356 | 890-910 | AAAAGGGAAACA CCCGAAGAUUC | 491 | 888-910 |
| AD-1560109 | GGGUGUUUCCC UUUAGCUAAU | 357 | 896-916 | AUUAGCUAAAGG GAAACACCCGA | 492 | 894-916 |
| AD-1560117 | CCCUUUAGCUA AGCACAGAUU | 358 | 904-924 | AAUCUGUGCUUA GCUAAAGGGAA | 493 | 902-924 |
| AD-1560123 | AGCUAAGCACA GAUCUACCUU | 359 | 910-930 | AAGGUAGAUCUG UGCUUAGCUAA | 494 | 908-930 |
| AD-1560132 | CAGAUCUACCU UGGUGAUUUU | 360 | 919-939 | AAAAUCACCAAG GUAGAUCUGUG | 495 | 917-939 |
| AD-1560139 | ACCUUGGUGAU UUGGACCCUU | 361 | 926-946 | AAGGGUCCAAAU CACCAAGGUAG | 496 | 924-946 |
| AD-1560150 | UUGGACCCUGG UUGCUUUGUU | 362 | 937-957 | AACAAAGCAACC AGGGUCCAAAU | 497 | 935-957 |
| AD-1560157 | CUGGUUGCUUU GUGUCUAGUU | 363 | 944-964 | AACUAGACACAA AGCAACCAGGG | 498 | 942-964 |
| AD-1560163 | GCUUUGUGUCU AGUUUUCUAU | 364 | 950-970 | AUAGAAAACUAG ACACAAAGCAA | 499 | 948-970 |
| AD-1560172 | CUAGUUUUCUA GACCCUUCAU | 365 | 959-979 | AUGAAGGGUCUA GAAAACUAGAC | 500 | 957-979 |
| AD-1560178 | UUCUAGACCCU UCAUCUCUUU | 366 | 965-985 | AAAGAGAUGAAG GGUCUAGAAAA | 501 | 963-985 |
| AD-1560184 | ACCCUUCAUCU CUUACUUGAU | 367 | 971-991 | AUCAAGUAAGAG AUGAAGGGUCU | 502 | 969-991 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1560191 | AUCUCUUACUU GAUAGACUUU | 368 | 978-998 | AAAGUCUAUCAA GUAAGAGAUGA | 503 | 976-998 |
| AD-1560197 | UACUUGAUAGA CUUACUAAUU | 369 | 984-1004 | AAUUAGUAAGUC UAUCAAGUAAG | 504 | 982-1004 |
| AD-1560205 | CUUACUAAUAA AAUGUGAAGU | 370 | 995-1015 | ACUUCACAUUUU AUUAGUAAGUC | 505 | 993-1015 |
| AD-1560214 | AAAAUGUGAAG ACUAGACCAU | 371 | 1004-1024 | AUGGUCUAGUCU UCACAUUUUAU | 506 | 1002-1024 |
| AD-1560220 | UGAAGACUAGA CCAAUUGUCU | 372 | 1010-1030 | AGACAAUUGGUC UAGUCUUCACA | 507 | 1008-1030 |
| AD-1560227 | UAGACCAAUUG UCAUGCUUGU | 373 | 1017-1037 | ACAAGCAUGACA AUUGGUCUAGU | 508 | 1015-1037 |
| AD-1560238 | UCAUGCUUGAC ACAACUGCUU | 374 | 1028-1048 | AAGCAGUUGUGU CAAGCAUGACA | 509 | 1026-1048 |
| AD-1560244 | UUGACACAACU GCUGUGGCUU | 375 | 1034-1054 | AAGCCACAGCAG UUGUGUCAAGC | 510 | 1032-1054 |
| AD-1560256 | CUGUGGCUGGU UGGUGCUUUU | 376 | 1046-1066 | AAAAGCACCAAC CAGCCACAGCA | 511 | 1044-1066 |
| AD-1560262 | CUGGUUGGUGC UUUGUUUAUU | 377 | 1052-1072 | AAUAAACAAAGC ACCAACCAGCC | 512 | 1050-1072 |
| AD-1560268 | GGUGCUUUGUU UAUGGUAGUU | 378 | 1058-1078 | AACUACCAUAAA CAAAGCACCAA | 513 | 1056-1078 |
| AD-1560274 | UUGUUUAUGGU AGUAGUUUUU | 379 | 1064-1084 | AAAAACUACUAC CAUAAACAAAG | 514 | 1062-1084 |
| AD-1560280 | UGGUAGUAGUU UUUCUGUAAU | 380 | 1071-1091 | AUUACAGAAAAA CUACUACCAUA | 515 | 1069-1091 |
| AD-1560286 | UAGUUUUUCUG UAACACAGAU | 381 | 1077-1097 | AUCUGUGUUACA GAAAAACUACU | 516 | 1075-1097 |
| AD-1560292 | UUCUGUAACAC AGAAUAUAGU | 382 | 1083-1103 | ACUAUAUUCUGU GUUACAGAAAA | 517 | 1081-1103 |
| AD-1560300 | CACAGAAUAUA GGAUAAGAAU | 383 | 1091-1111 | AUUCUUAUCCUA UAUUCUGUGUU | 518 | 1089-1111 |
| AD-1560311 | AGAAUAAAGUA CCUUGACUUU | 384 | 1114-1134 | AAAGUCAAGGUA CUUUAUUCUUA | 519 | 1112-1134 |
| AD-1560323 | CUUGACUUUGU UCACAGCAUU | 385 | 1126-1146 | AAUGCUGUGAAC AAAGUCAAGGU | 520 | 1124-1146 |
| AD-1560329 | UUUGUUCACAG CAUGUAGGGU | 386 | 1132-1152 | ACCCUACAUGCU GUGAACAAAGU | 521 | 1130-1152 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1560335 | CACAGCAUGUAGGGUGAUGAU | 387 | 1138-1158 | AUCAUCACCCUACAUGCUGUGAA | 522 | 1136-1158 |
| AD-1560344 | UAGGGUGAUGAGCACUCACAU | 388 | 1147-1167 | AUGUGAGUGCUCAUCACCCUACA | 523 | 1145-1167 |
| AD-1560350 | GAUGAGCACUCACAAUUGUUU | 389 | 1153-1173 | AAACAAUUGUGAGUGCUCAUCAC | 524 | 1151-1173 |
| AD-1560357 | ACUCACAAUUGUUGACUAAAU | 390 | 1160-1180 | AUUUAGUCAACAAUUGUGAGUGC | 525 | 1158-1180 |
| AD-1560368 | UUGACUAAAAUGCUGCUUUUU | 391 | 1171-1191 | AAAAAGCAGCAUUUUAGUCAACA | 526 | 1169-1191 |
| AD-1560377 | AUGCUGCUUUUAAAACAUAGU | 392 | 1180-1200 | ACUAUGUUUUAAAGCAGCAUUU | 527 | 1178-1200 |
| AD-1560383 | CUUUUAAAACAUAGGAAAGUU | 393 | 1186-1206 | AACUUUCCUAUGUUUUAAAAGCA | 528 | 1184-1206 |
| AD-1560392 | CAUAGGAAAGUAGAAUGGUUU | 394 | 1195-1215 | AAACCAUUCUACUUUCCUAUGUU | 529 | 1193-1215 |
| AD-1560400 | AGUAGAAUGGUUGAGUGCAAU | 395 | 1203-1223 | AUUGCACUCAACCAUUCUACUUU | 530 | 1201-1223 |
| AD-1560406 | AUGGUUGAGUGCAAAUCCAUU | 396 | 1209-1229 | AAUGGAUUUGCACUCAACCAUUC | 531 | 1207-1229 |
| AD-1560413 | AGUGCAAAUCCAUAGCACAAU | 397 | 1216-1236 | AUUGUGCUAUGGAUUUGCACUCA | 532 | 1214-1236 |
| AD-1560421 | UCCAUAGCACAAGAUAAAUUU | 398 | 1224-1244 | AAAUUUAUCUUGUGCUAUGGAUU | 533 | 1222-1244 |
| AD-1560430 | CAAGAUAAAUUGAGCUAGUUU | 399 | 1233-1253 | AAACUAGCUCAAUUUAUCUUGUG | 534 | 1231-1253 |
| AD-1560441 | GAGCUAGUUAAGGCAAAUCAU | 400 | 1244-1264 | AUGAUUUGCCUUAACUAGCUCAA | 535 | 1242-1264 |
| AD-1560449 | UAAGGCAAAUCAGGUAAAAUU | 401 | 1252-1272 | AAUUUUACCUGAUUUGCCUUAAC | 536 | 1250-1272 |
| AD-1560460 | AGGUAAAAUAGUCAUGAUUCU | 402 | 1263-1283 | AGAAUCAUGACUAUUUUACCUGA | 537 | 1261-1283 |
| AD-1560470 | GUCAUGAUUCUAUGUAAUGUU | 403 | 1273-1293 | AACAUUACAUAGAAUCAUGACUA | 538 | 1271-1293 |
| AD-1560480 | UAUGUAAUGUAAACCAGAAAU | 404 | 1283-1303 | AUUUCUGGUUUACAUUACAUAGA | 539 | 1281-1303 |
| AD-1560492 | UCAUGAUUUCAAGAUGUUAUU | 405 | 1313-1333 | AAUAACAUCUUGAAAUCAUGAAC | 540 | 1311-1333 |

(continued)

| Duplex Name | Sense Sequence 5' to 3' | SEQ ID NO: | Range in NM_000 067.3 | Antisense Sequence 5' to 3' | SEQ ID NO: | Range in NM_000 067.3 |
|---|---|---|---|---|---|---|
| AD-1560528 | CUUUUGAAUUACAGAGAUAUU | 406 | 1411-1431 | AAUAUCUCUGUAAUUCAAAAGUC | 541 | 1409-1431 |
| AD-1560556 | UUAGAGUUGUGAUACAGAGUU | 407 | 1463-1483 | AACUCUGUAUCACAACUCUAAUU | 542 | 1461-1483 |
| AD-1560562 | UUGUGAUACAGAGUAUAUUUU | 408 | 1469-1489 | AAAAUAUACUCUGUAUCACAACU | 543 | 1467-1489 |
| AD-1560568 | UACAGAGUAUAUUUCCAUUCU | 409 | 1475-1495 | AGAAUGGAAAUAUACUCUGUAUC | 544 | 1473-1495 |
| AD-1560576 | AUAUUUCCAUUCAGACAAUAU | 410 | 1483-1503 | AUAUUGUCUGAAUGGAAAUAUAC | 545 | 1481-1503 |
| AD-1560585 | UUCAGACAAUAUAUCAUAACU | 411 | 1492-1512 | AGUUAUGAUAUAUUGUCUGAAUG | 546 | 1490-1512 |

**Table 5. Modified Sense and Antisense Strand Sequences of CA2 dsRNA Agents with C16 Modification**

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560600 | asgsauc(Ghd)GfuGf CfCfgauuccugscsa | 547 | VPusGfscagGfaAfUfc ggcAfcCfgaucusgsg | 682 | CCAGAUCGGUGC CGAUUCCUGCC | 817 |
| AD-1560617 | csgscga(Chd)CfaUf GfUfcccaucacsusa | 548 | VPusAfsgugAfuGfGf gacaUfgGfucgcgscsu | 683 | AGCGCGACCAUG UCCCAUCACUG | 818 |
| AD-1560622 | gsusacg(Ghd)CfaAf AfCfacaacggascsa | 549 | VPusGfsuccGfuUfGfu guuUfgCfcguacscsc | 684 | GGGUACGGCAAA CACAACGGACC | 819 |
| AD-1560628 | csasaac(Ahd)CfaAf CfGfgaccugagscsa | 550 | VPusGfscucAfgGfUfc cguUfgUfguuugscsc | 685 | GGCAAACACAAC GGACCUGAGCA | 820 |
| AD-1560638 | gsgsacc(Uhd)GfaGf CfAfcuggcauasasa | 551 | VPusUfsuauGfcCfAfg ugcUfcAfgguccsgsu | 686 | ACGGACCUGAGC ACUGGCAUAAG | 821 |
| AD-1560644 | gsasgca(Chd)UfgGf CfAfuaaggacususa | 552 | VPusAfsaguCfcUfUfa ugcCfaGfugcucsasg | 687 | CUGAGCACUGGC AUAAGGACUUC | 822 |
| AD-1560655 | gsusuga(Chd)AfuCf GfAfcacucauascsa | 553 | VPusGfsuauGfaGfUfg ucgAfuGfucaacsasg | 688 | CUGUUGACAUCG ACACUCAUACA | 823 |
| AD-1560665 | ascsacu(Chd)AfuAf CfAfgccaaguasusa | 554 | VPusAfsuacUfuGfGfc uguAfuGfaguguscsg | 689 | CGACACUCAUAC AGCCAAGUAUG | 824 |
| AD-1560672 | usascag(Chd)CfaAf GfUfaugacccususa | 555 | VPusAfsaggGfuCfAfu acuUfgGfcuguasusg | 690 | CAUACAGCCAAG UAUGACCCUUC | 825 |
| AD-1560678 | csasagu(Ahd)UfgAf CfCfcuucccugsasa | 556 | VPusUfscagGfgAfAfg gguCfaUfacuugsgsc | 691 | GCCAAGUAUGAC CCUUCCCUGAA | 826 |
| AD-1560684 | usgsucu(Ghd)UfuUf CfCfuaugaucasasa | 557 | VPusUfsugaUfcAfUfa ggaAfaCfagacasgsg | 692 | CCUGUCUGUUUC CUAUGAUCAAG | 827 |
| AD-1560693 | cscsuau(Ghd)AfuCf AfAfgcaacuucscsa | 558 | VPusGfsgaaGfuUfGfc uugAfuCfauaggsasa | 693 | UUCCUAUGAUCA AGCAACUUCCC | 828 |
| AD-1560701 | csasagc(Ahd)AfcUf UfCfccugaggasusa | 559 | VPusAfsuccUfcAfGfg gaaGfuUfgcuugsasu | 694 | AUCAAGCAACUU CCCUGAGGAUC | 829 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560711 | cscscug(Ahd)GfgAf UfCfcucaacaasusa | 560 | VPusAfsuugUfuGfAf ggauCfcUfcagggsasa | 695 | UUCCCUGAGGAU CCUCAACAAUG | 830 |
| AD-1560720 | uscscuc(Ahd)AfcAf AfUfggucaugcsusa | 561 | VPusAfsgcaUfgAfCfc auuGfuUfgaggasusc | 696 | GAUCCUCAACAA UGGUCAUGCUU | 831 |
| AD-1560726 | ascsaau(Ghd)GfuCf AfUfgcuuucaascsa | 562 | VPusGfsuugAfaAfGfc augAfcCfauugususg | 697 | CAACAAUGGUCA UGCUUUCAACG | 832 |
| AD-1560735 | asusgcu(Uhd)UfcAf AfCfguggaguususa | 563 | VPusAfsaacUfcCfAfc guuGfaAfagcausgsa | 698 | UCAUGCUUUCAA CGUGGAGUUUG | 833 |
| AD-1560745 | asascgu(Ghd)GfaGf UfUfugaugacuscsa | 564 | VPusGfsaguCfaUfCfa aacUfcCfacguusgsa | 699 | UCAACGUGGAGU UUGAUGACUCU | 834 |
| AD-1560752 | usgsaug(Ahd)CfuCf UfCfaggacaaasgsa | 565 | VPusCfsuuuGfuCfCfu gagAfgUfcaucasasa | 700 | UUUGAUGACUCU CAGGACAAAGC | 835 |
| AD-1560759 | uscsuca(Ghd)GfaCf AfAfagcagugcsusa | 566 | VPusAfsgcaCfuGfCfu uugUfcCfugagasgsu | 701 | ACUCUCAGGACA AAGCAGUGCUC | 836 |
| AD-1560765 | gsascaa(Ahd)GfcAf GfUfgcucaaggsgsa | 567 | VPusCfsccuUfgAfGfc acuGfcUfuugucscsu | 702 | AGGACAAAGCAG UGCUCAAGGGA | 837 |
| AD-1560777 | usgsgca(Chd)UfuAf CfAfgauugauuscsa | 568 | VPusGfsaauCfaAfUfc uguAfaGfugccasusc | 703 | GAUGGCACUUAC AGAUUGAUUCA | 838 |
| AD-1560783 | ususaca(Ghd)AfuUf GfAfuucaguuuscsa | 569 | VPusGfsaaaCfuGfAfa ucaAfuCfuguaasgsu | 704 | ACUUACAGAUUG AUUCAGUUUCA | 839 |
| AD-1560792 | asusuca(Ghd)UfuUf CfAfcuuucacusgsa | 570 | VPusCfsaguGfaAfAfg ugaAfaCfugaauscsa | 705 | UGAUUCAGUUUC ACUUUCACUGG | 840 |
| AD-1560798 | uscsacu(Uhd)GfaUf GfGfacaagguuscsa | 571 | VPusGfsaacCfuUfGfu ccaUfcAfagugasasc | 706 | GUUCACUUGAUG GACAAGGUUCA | 841 |
| AD-1560804 | gsasugg(Ahd)CfaAf GfGfuucagagcsasa | 572 | VPusUfsgcuCfuGfAfa ccuUfgUfccaucsasa | 707 | UUGAUGGACAAG GUUCAGAGCAU | 842 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560810 | csasagg(Uhd)UfcAf GfAfgcauacugsusa | 573 | VPusAfscagUfaUfGfc ucuGfaAfccuugsusc | 708 | GACAAGGUUCAG AGCAUACUGUG | 843 |
| AD-1560816 | uscsaga(Ghd)CfaUf AfCfuguggauasasa | 574 | VPusUfsuauCfcAfCfa guaUfgCfucugasasc | 709 | GUUCAGAGCAUA CUGUGGAUAAA | 844 |
| AD-1560837 | asasgaa(Ahd)UfaUf GfCfugcagaacsusa | 575 | VPusAfsguuCfuGfCfa gcaUfaUfuucuusus u | 710 | AAAAGAAAUAUG CUGCAGAACUU | 845 |
| AD-1560845 | usasugc(Uhd)GfcAf GfAfacuucacususa | 576 | VPusAfsaguGfaAfGfu ucuGfcAfgcauasusu | 711 | AAUAUGCUGCAG AACUUCACUUG | 846 |
| AD-1560851 | gscsaga(Ahd)CfuUf CfAfcuugguucsasa | 577 | VPusUfsgaaCfcAfAfg ugaAfgUfucugcsasg | 712 | CUGCAGAACUUC ACUUGGUUCAC | 847 |
| AD-1560843 | csusuca(Chd)UfuGf GfUfucacuggasasa | 578 | VPusUfsuccAfgUfGfa accAfaGfugaagsusu | 713 | AACUUCACUUGG UUCACUGGAAC | 848 |
| AD-1560862 | ususugg(Ghd)AfaAf GfCfugugcagcsasa | 579 | VPusUfsgcuGfcAfCfa gcuUfuCfccaaasasu | 714 | AUUUUGGGAAAG CUGUGCAGCAA | 849 |
| AD-1560874 | gsusgca(Ghd)CfaAf CfCfugauggacsusa | 580 | VPusAfsgucCfaUfCfa gguUfgCfugcacsasg | 715 | CUGUGCAGCAAC CUGAUGGACUG | 850 |
| AD-1560880 | csasacc(Uhd)GfaUf GfGfacuggccgsusa | 581 | VPusAfscggCfcAfGfu ccaUfcAfgguugscsu | 716 | AGCAACCUGAUG GACUGGCCGUU | 851 |
| AD-1560892 | csusggc(Chd)GfuUf CfUfagguauuususa | 582 | VPusAfsaaaUfaCfCfu agaAfcGfgccagsusc | 717 | GACUGGCCGUUC UAGGUAUUUUU | 852 |
| AD-1560895 | usgsaag(Ghd)UfuGf GfCfagcgcuaasasa | 583 | VPusUfsuuaGfcGfCfu gccAfaCfcuucasasa | 718 | UUUGAAGGUUGG CAGCGCUAAAC | 853 |
| AD-1560904 | gscsagc(Ghd)CfuAf AfAfccgggccususa | 584 | VPusAfsaggCfcCfGfg uuuAfgCfgcugcscsa | 719 | UGGCAGCGCUAA ACCGGGCCUUC | 854 |
| AD-1560915 | cscsggg(Chd)CfuUf CfAfgaaaguugsusa | 585 | VPusAfscaaCfuUfUfc ugaAfgGfcccggsusu | 720 | AACCGGGCCUUC AGAAAGUUGUU | 855 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560921 | csusuca(Ghd)AfaAf GfUfuguugaugsusa | 586 | VPusAfscauCfaAfCfa acuUfuCfugaagsgsc | 721 | GCCUUCAGAAAG UUGUUGAUGUG | 856 |
| AD-1560930 | gsusugu(Uhd)GfaUf GfUfgcuggauuscsa | 587 | VPusGfsaauCfcAfGfc acaUfcAfacaacsusu | 722 | AAGUUGUUGAUG UGCUGGAUUCC | 857 |
| AD-1560941 | gscsugg(Ahd)UfuCf CfAfuuaaaacasasa | 588 | VPusUfsuguUfuUfAf auggAfaUfccagcsasc | 723 | GUGCUGGAUUCC AUUAAAACAAA | 858 |
| AD-1560948 | uscscau(Uhd)AfaAf AfCfaaagggcasasa | 589 | VPusUfsugcCfcUfUfu guuUfuAfauggasasu | 724 | AUUCCAUUAAAA CAAAGGGCAAG | 859 |
| AD-1560954 | asasaac(Ahd)AfaGf GfGfcaagagugscsa | 590 | VPusGfscacUfcUfUfg cccUfuUfguuuusasa | 725 | UUAAAACAAAGG GCAAGAGUGCU | 860 |
| AD-1560963 | gsgscaa(Ghd)AfgUf GfCfugacuucascsa | 591 | VPusGfsugaAfgUfCfa gcaCfuCfuugccscsu | 726 | AGGGCAAGAGUG CUGACUUCACU | 861 |
| AD-1560970 | gsusgcu(Ghd)AfcUf UfCfacuaacuuscsa | 592 | VPusGfsaagUfuAfGfu gaaGfuCfagcacsusc | 727 | GAGUGCUGACUU CACUAACUUCG | 862 |
| AD-1560976 | ascsuuc(Ahd)CfuAf AfCfuucgauccsusa | 593 | VPusAfsggaUfcGfAfa guuAfgUfgaaguscsa | 728 | UGACUUCACUAA CUUCGAUCCUC | 863 |
| AD-1560989 | csgsauc(Chd)UfcGf UfGfgccuccuuscsa | 594 | VPusGfsaagGfaGfGfc cacGfaGfgaucgsasa | 729 | UUCGAUCCUCGU GGCCUCCUUCC | 864 |
| AD-1560996 | uscsgug(Ghd)CfcUf CfCfuuccugaasusa | 595 | VPusAfsuucAfgGfAfa ggaGfgCfcacgasgsg | 730 | CCUCGUGGCCUC CUUCCUGAAUC | 865 |
| AD-1561002 | cscsucc(Uhd)UfcCf UfGfaauccuugsgsa | 596 | VPusCfscaaGfgAfUfu cagGfaAfggaggscsc | 731 | GGCCUCCUUCCU GAAUCCUUGGA | 866 |
| AD-1561009 | cscsuga(Ahd)UfcCf UfUfggauuacusgsa | 597 | VPusCfsaguAfaUfCfc aagGfaUfucaggsasa | 732 | UUCCUGAAUCCU UGGAUUACUGG | 867 |
| AD-1561015 | uscscuu(Ghd)GfaUf UfAfcuggaccusasa | 598 | VPusUfsaggUfcCfAfg uaaUfcCfaaggasusu | 733 | AAUCCUUGGAUU ACUGGACCUAC | 868 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561031 | cscsuac(Chd)CfaGf GfCfucacugacscsa | 599 | VPusGfsgucAfgUfGfa gccUfgGfguaggsusc | 734 | GACCUACCCAGG CUCACUGACCA | 869 |
| AD-1561037 | cscsucu(Uhd)CfuGf GfAfaugugugascsa | 600 | VPusGfsucaCfaCfAfu uccAfgAfagaggsasg | 735 | CUCCUCUUCUGG AAUGUGUGACC | 870 |
| AD-1561043 | usgsgaa(Uhd)GfuGf UfGfaccuggaususa | 601 | VPusAfsaucCfaGfGfu cacAfcAfuuccasgsa | 736 | UCUGGAAUGUGU GACCUGGAUUG | 871 |
| AD-1561050 | usgsuga(Chd)CfuGf GfAfuugugcucsasa | 602 | VPusUfsgagCfaCfAfa uccAfgGfucacascsa | 737 | UGUGUGACCUGG AUUGUGCUCAA | 872 |
| AD-1561056 | csusgga(Uhd)UfgUf GfCfucaaggaascsa | 603 | VPusGfsuucCfuUfGfa gcaCfaAfuccagsgsu | 738 | ACCUGGAUUGUG CUCAAGGAACC | 873 |
| AD-1561066 | csuscaa(Ghd)GfaAf CfCfcaucagcgsusa | 604 | VPusAfscgcUfgAfUfg gguUfcCfuugagscsa | 739 | UGCUCAAGGAAC CCAUCAGCGUC | 874 |
| AD-1561072 | gsasacc(Chd)AfuCf AfGfcgucagcasgsa | 605 | VPusCfsugcUfgAfCfg cugAfuGfgguucscsu | 740 | AGGAACCCAUCA GCGUCAGCAGC | 875 |
| AD-1475424 | asgsaac(Uhd)GfaUf GfGfuggacaacsusa | 606 | VPusAfsguuGfuCfCfa ccaUfcAfguucususc | 741 | GAAGAACUGAUG GUGGACAACUG | 876 |
| AD-1561092 | csgsagc(Ahd)GfgUf GfUfugaaauucscsa | 607 | VPusGfsgaaUfuUfCfa acaCfcUfgcucgscsu | 742 | AGCGAGCAGGUG UUGAAAUUCCG | 877 |
| AD-1561100 | gsgsugu(Uhd)GfaAf AfUfuccguaaascsa | 608 | VPusGfsuuuAfcGfGfa auuUfcAfacaccsusg | 743 | CAGGUGUUGAAA UUCCGUAAACU | 878 |
| AD-1561106 | gsasaau(Uhd)CfcGf UfAfaacuuaacsusa | 609 | VPusAfsguuAfaGfUf uuacGfgAfauuucsasa | 744 | UUGAAAUUCCGU AAACUUAACUU | 879 |
| AD-1561112 | cscsgua(Ahd)AfcUf UfAfacuucaausgsa | 610 | VPusCfsauuGfaAfGfu uaaGfuUfuacggsasa | 745 | UUCCGUAAACUU AACUUCAAUGG | 880 |
| AD-1561116 | gsasggg(Uhd)GfaAf CfCfcgaagaacsusa | 611 | VPusAfsguuCfuUfCfg gguUfcAfcccucscsc | 746 | GGGAGGGUGAAC CCGAAGAACUG | 881 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561122 | gsasacc(Chd)GfaAf GfAfacugauggsusa | 612 | VPusAfsccaUfcAfGfu ucuUfcGfgguucsasc | 747 | GUGAACCCGAAG AACUGAUGGUG | 882 |
| AD-1561130 | asusggu(Ghd)GfaCf AfAfcuggcgccscsa | 613 | VPusGfsggcGfcCfAfg uugUfcCfaccauscsa | 748 | UGAUGGUGGACA ACUGGCGCCCA | 883 |
| AD-1561146 | cscsagc(Uhd)CfaGf CfCfacugaagasasa | 614 | VPusUfsucuUfcAfGfu ggcUfgAfgcuggsgsc | 749 | GCCCAGCUCAGC CACUGAAGAAC | 884 |
| AD-1561152 | csasgcc(Ahd)CfuGf AfAfgaacaggcsasa | 615 | VPusUfsgccUfgUfUfc uucAfgUfggcugsasg | 750 | CUCAGCCACUGA AGAACAGGCAA | 885 |
| AD-1561158 | csusgaa(Ghd)AfaCf AfGfgcaaaucasasa | 616 | VPusUfsugaUfuUfGfc cugUfuCfuucagsusg | 751 | CACUGAAGAACA GGCAAAUCAAA | 886 |
| AD-1446763 | uscsacu(Ghd)GfaAf CfAfccaaauausgsa | 617 | VPusCfsauaUfuUfGfg uguUfcCfagugasasc | 752 | GUUCACUGGAAC ACCAAAUAUGG | 887 |
| AD-1561168 | gsgscaa(Ahd)UfcAf AfAfgcuuccuuscsa | 618 | VPusGfsaagGfaAfGfc uuuGfaUfuugccsusg | 753 | CAGGCAAAUCAA AGCUUCCUUCA | 888 |
| AD-1561175 | csasaag(Chd)UfuCf CfUfucaaauaasgsa | 619 | VPusCfsuuaUfuUfGfa aggAfaGfcuuugsasu | 754 | AUCAAAGCUUCC UUCAAAUAAGA | 889 |
| AD-1561181 | ususccu(Uhd)CfaAf AfUfaagauggsuscsa | 620 | VPusGfsaccAfuCfUfu auuUfgAfaggaasgsc | 755 | GCUUCCUUCAAA UAAGAUGGUCC | 890 |
| AD-1561190 | asusaag(Ahd)UfgGf UfCfccauagucsusa | 621 | VPusAfsgacUfaUfGfg gacCfaUfcuuaususu | 756 | AAAUAAGAUGGU CCCAUAGUCUG | 891 |
| AD-1561196 | usgsguc(Chd)CfaUf AfGfucuguaucscsa | 622 | VPusGfsgauAfcAfGfa cuaUfgGfgaccasusc | 757 | GAUGGUCCCAUA GUCUGUAUCCA | 892 |
| AD-1561203 | asusagu(Chd)UfgUf AfUfccaaauaasusa | 623 | VPusAfsuuaUfuUfGf gauaCfaGfacuausgsg | 758 | CCAUAGUCUGUA UCCAAAUAAUG | 893 |
| AD-1561210 | gsusauc(Chd)AfaAf UfAfaugaaucususa | 624 | VPusAfsagaUfuCfAfu uauUfuGfgauacsasg | 759 | CUGUAUCCAAAU AAUGAAUCUUC | 894 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561218 | asusaau(Ghd)AfaUf CfUfucgggugususa | 625 | VPusAfsacaCfcCfGfa agaUfuCfauuaususu | 760 | AAAUAAUGAAUC UUCGGGUGUUU | 895 |
| AD-1561225 | asuscuu(Chd)GfgGf UfGfuuucccuususa | 626 | VPusAfsaagGfgAfAfa cacCfcGfaagaususc | 761 | GAAUCUUCGGGU GUUUCCCUUUA | 896 |
| AD-1561231 | gsgsgug(Uhd)UfuCf CfCfuuuagcuasasa | 627 | VPusUfsuagCfuAfAfa gggAfaAfcacccsgsa | 762 | UCGGGUGUUUCC CUUUAGCUAAG | 897 |
| AD-1561239 | cscscuu(Uhd)AfgCf UfAfagcacagasusa | 628 | VPusAfsucuGfuGfCfu uagCfuAfaagggsasa | 763 | UUCCCUUUAGCU AAGCACAGAUC | 898 |
| AD-1561245 | asgscua(Ahd)GfcAf CfAfgaucuaccsusa | 629 | VPusAfsgguAfgAfUf cuguGfcUfuagcusasa | 764 | UUAGCUAAGCAC AGAUCUACCUU | 899 |
| AD-1561254 | csasgau(Chd)UfaCf CfUfuggugauususa | 630 | VPusAfsaauCfaCfCfa aggUfaGfaucugsusg | 765 | CACAGAUCUACC UUGGUGAUUUG | 900 |
| AD-1561261 | ascscuu(Ghd)GfuGf AfUfuuggacccsusa | 631 | VPusAfsgggUfcCfAfa aucAfcCfaaggusasg | 766 | CUACCUUGGUGA UUUGGACCCUG | 901 |
| AD-1561272 | ususgga(Chd)CfcUf GfGfuugcuuugsusa | 632 | VPusAfscaaAfgCfAfa ccaGfgGfuccaasasu | 767 | AUUUGGACCCUG GUUGCUUUGUG | 902 |
| AD-1561279 | csusggu(Uhd)GfcUf UfUfgugucuagsusa | 633 | VPusAfscuaGfaCfAfc aaaGfcAfaccagsgsg | 768 | CCCUGGUUGCUU UGUGUCUAGUU | 903 |
| AD-1561285 | gscsuuu(Ghd)UfgUf CfUfaguuuucusasa | 634 | VPusUfsagaAfaAfCfu agaCfaCfaaagcsasa | 769 | UUGCUUUGUGUC UAGUUUUCUAG | 904 |
| AD-1561294 | csusagu(Uhd)UfuCf UfAfgacccuucsasa | 635 | VPusUfsgaaGfgGfUfc uagAfaAfacuagsasc | 770 | GUCUAGUUUUCU AGACCCUUCAU | 905 |
| AD-1561300 | ususcua(Ghd)AfcCf CfUfucaucucususa | 636 | VPusAfsagaGfaUfGfa aggGfuCfuagaasasa | 771 | UUUUCUAGACCC UUCAUCUCUUA | 906 |
| AD-1561306 | ascsccu(Uhd)CfaUf CfUfcuuacuugsasa | 637 | VPusUfscaaGfuAfAfg agaUfgAfaggguscsu | 772 | AGACCCUUCAUC UCUUACUUGAU | 907 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561313 | asuscuc(Uhd)UfaCfUfUfgauagacususa | 638 | VPusAfsaguCfuAfUfcaagUfaAfgagausgsa | 773 | UCAUCUCUUACUUGAUAGACUUA | 908 |
| AD-1561319 | usascuu(Ghd)AfuAfGfAfcuuacuaasusa | 639 | VPusAfsuuaGfuAfAfgucuAfuCfaaguasasg | 774 | CUUACUUGAUAGACUUACUAAUA | 909 |
| AD-1561327 | csusuac(Uhd)AfaUfAfAfaaugugaasgsa | 640 | VPusCfsuucAfcAfUfuuuaUfuAfguaagsusc | 775 | GACUUACUAAUAAAAUGUGAAGA | 910 |
| AD-1561336 | asasaau(Ghd)UfgAfAfGfacuagaccsasa | 641 | VPusUfsgguCfuAfGfucuuCfaCfauuuusasu | 776 | AUAAAAUGUGAAGACUAGACCAA | 911 |
| AD-1561342 | usgsaag(Ahd)CfuAfGfAfccaauuguscsa | 642 | VPusGfsacaAfuUfGfgucuAfgUfcuucascsa | 777 | UGUGAAGACUAGACCAAUUGUCA | 912 |
| AD-1561349 | usasgac(Chd)AfaUfUfGfucaugcuusgsa | 643 | VPusCfsaagCfaUfGfacaaUfuGfgucuasgsu | 778 | ACUAGACCAAUUGUCAUGCUUGA | 913 |
| AD-1561360 | uscsaug(Chd)UfuGfAfCfacaacugcsusa | 644 | VPusAfsgcaGfuUfGfugucAfaGfcaugascsa | 779 | UGUCAUGCUUGACACAACUGCUG | 914 |
| AD-1561366 | ususgac(Ahd)CfaAfCfUfgcuguggcsusa | 645 | VPusAfsgccAfcAfGfcaguUfgUfgucaasgsc | 780 | GCUUGACACAACUGCUGUGGCUG | 915 |
| AD-1561378 | csusgug(Ghd)CfuGfGfUfuggugcuususa | 646 | VPusAfsaagCfaCfCfaaccAfgCfcacagscsa | 781 | UGCUGUGGCUGGUUGGUGCUUUG | 916 |
| AD-1561384 | csusggu(Uhd)GfgUfGfCfuuuguuuasusa | 647 | VPusAfsuaaAfcAfAfagcaCfcAfaccagscsc | 782 | GGCUGGUUGGUGCUUUGUUUAUG | 917 |
| AD-1561390 | gsgsugc(Uhd)UfuGfUfUfuaugguagsusa | 648 | VPusAfscuaCfcAfUfaaacAfaAfgcaccsasa | 783 | UUGGUGCUUUGUUUAUGGUAGUA | 918 |
| AD-1561396 | ususguu(Uhd)AfuGfGfUfaguaguuususa | 649 | VPusAfsaaaCfuAfCfuaccAfuAfaacaasasg | 784 | CUUUGUUUAUGGUAGUAGUUUUU | 919 |
| AD-1561402 | usgsgua(Ghd)UfaGfUfUfuuucuguasasa | 650 | VPusUfsuacAfgAfAfaaacUfaCfuaccasusa | 785 | UAUGGUAGUAGUUUUUCUGUAAC | 920 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561408 | usasguu(Uhd)UfuCf UfGfuaacacagsasa | 651 | VPusUfscugUfgUfUfa cagAfaAfaacuascsu | 786 | AGUAGUUUUCU GUAACACAGAA | 921 |
| AD-1561414 | usu










scug(Uhd)AfaCf AfCfagaauauasgsa | 652 | VPusCfsuauAfuUfCfu gugUfuAfcagaasasa | 787 | UUUUCUGUAACA CAGAAUAUAGG | 922 |
| AD-1561422 | csascag(Ahd)AfuAf UfAfggauaagasasa | 653 | VPusUfsucuUfaUfCfc uauAfuUfcugugsusu | 788 | AACACAGAAUAU AGGAUAAGAAA | 923 |
| AD-1561433 | asgsaau(Ahd)AfaGf UfAfccuugacususa | 654 | VPusAfsaguCfaAfGfg uacUfuUfauucususa | 789 | UAAGAAUAAAGU ACCUUGACUUU | 924 |
| AD-1561444 | csusuga(Chd)UfuUf GfUfucacagcasusa | 655 | VPusAfsugcUfgUfGfa acaAfaGfucaagsgsu | 790 | ACCUUGACUUUG UUCACAGCAUG | 925 |
| AD-1561450 | ususugu(Uhd)CfaCf AfGfcauguaggsgsa | 656 | VPusCfsccuAfcAfUfg cugUfgAfacaaasgsu | 791 | ACUUUGUUCACA GCAUGUAGGGU | 926 |
| AD-1561456 | csascag(Chd)AfuGf UfAfgggugaugsasa | 657 | VPusUfscauCfaCfCfc uacAfuGfcugugsasa | 792 | UUCACAGCAUGU AGGGUGAUGAG | 927 |
| AD-1561465 | usasggg(Uhd)GfaUf GfAfgcacucacsasa | 658 | VPusUfsgugAfgUfGf cucaUfcAfcccuascsa | 793 | UGUAGGGUGAUG AGCACUCACAA | 928 |
| AD-1561471 | gsasuga(Ghd)CfaCf UfCfacaauugususa | 659 | VPusAfsacaAfuUfGfu gagUfgCfucaucsasc | 794 | GUGAUGAGCACU CACAAUUGUUG | 929 |
| AD-1561478 | ascsuca(Chd)AfaUf UfGfuugacuaasasa | 660 | VPusUfsuuaGfuCfAfa caaUfuGfugagusgsc | 795 | GCACUCACAAUU GUUGACUAAAA | 930 |
| AD-1561489 | ususgac(Uhd)AfaAf AfUfgcugcuuususa | 661 | VPusAfsaaaGfcAfGfc auuUfuAfgucaascsa | 796 | UGUUGACUAAAA UGCUGCUUUUA | 931 |
| AD-1561498 | asusgcu(Ghd)CfuUf UfUfaaaacauasgsa | 662 | VPusCfsuauGfuUfUfu aaaAfgCfagcaususu | 797 | AAAUGCUGCUUU UAAAACAUAGG | 932 |
| AD-1561504 | csusuuu(Ahd)AfaAf CfAfuaggaaagsusa | 663 | VPusAfscuuUfcCfUfa uguUfuUfaaaagscsa | 798 | UGCUUUUAAAAC AUAGGAAAGUA | 933 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561513 | csasuag(Ghd)AfaAf GfUfagaauggususa | 664 | VPusAfsaccAfuUfCfu acuUfuCfcuaugsusu | 799 | AACAUAGGAAG UAGAAUGGUUG | 934 |
| AD-1561521 | asgsuag(Ahd)AfuGf GfUfugagugcasasa | 665 | VPusUfsugcAfcUfCfa accAfuUfcuacususu | 800 | AAAGUAGAAUGG UUGAGUGCAAA | 935 |
| AD-1561527 | asusggu(Uhd)GfaGf UfGfcaaauccasusa | 666 | VPusAfsuggAfuUfUf gcacUfcAfaccaususc | 801 | GAAUGGUUGAGU GCAAAUCCAUA | 936 |
| AD-1561534 | asgsugc(Ahd)AfaUf CfCfauagcacasasa | 667 | VPusUfsuguGfcUfAf uggaUfuUfgcacuscsa | 802 | UGAGUGCAAAUC CAUAGCACAAG | 937 |
| AD-1561542 | uscscau(Ahd)GfcAf CfAfagauaaaususa | 668 | VPusAfsauuUfaUfCfu uguGfcUfauggasusu | 803 | AAUCCAUAGCAC AAGAUAAAUUG | 938 |
| AD-1561551 | csasaga(Uhd)AfaAf UfUfgagcuagususa | 669 | VPusAfsacuAfgCfUfc aauUfuAfucuugsusg | 804 | CACAAGAUAAAU UGAGCUAGUUA | 939 |
| AD-1561562 | gsasgcu(Ahd)GfuUf AfAfggcaaaucsasa | 670 | VPusUfsgauUfuGfCfc uuaAfcUfagcucsasa | 805 | UUGAGCUAGUUA AGGCAAAUCAG | 940 |
| AD-1561570 | usasagg(Chd)AfaAf UfCfagguaaaasusa | 671 | VPusAfsuuuUfaCfCfu gauUfuGfccuuasasc | 806 | GUUAAGGCAAAU CAGGUAAAAUA | 941 |
| AD-1561581 | asgsgua(Ahd)AfaUf AfGfucaugauuscsa | 672 | VPusGfsaauCfaUfGfa cuaUfuUfuaccusgsa | 807 | UCAGGUAAAAUA GUCAUGAUUCU | 942 |
| AD-1561591 | gsuscau(Ghd)AfuUf CfUfauguaaugsusa | 673 | VPusAfscauUfaCfAfu agaAfuCfaugacsusa | 808 | UAGUCAUGAUUC UAUGUAAUGUA | 943 |
| AD-1561601 | usasugu(Ahd)AfuGf UfAfaaccagaasasa | 674 | VPusUfsuucUfgGfUf uuacAfuUfacauasgsa | 809 | UCUAUGUAAUGU AAACCAGAAAA | 944 |
| AD-1561613 | uscsaug(Ahd)UfuUf CfAfagauguuasusa | 675 | VPusAfsuaaCfaUfCfu ugaAfaUfcaugasasc | 810 | GUUCAUGAUUUC AAGAUGUUAUA | 945 |
| AD-1561651 | csusuuu(Ghd)AfaUf UfAfcagagauasusa | 676 | VPusAfsuauCfuCfUfg uaaUfuCfaaaagsusc | 811 | GACUUUUGAAUU ACAGAGAUAUA | 946 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1561679 | ususaga(Ghd)UfuGf UfGfauacagagsusa | 677 | VPusAfscucUfgUfAfu cacAfaCfucuaasusu | 812 | AAUUAGAGUUGU GAUACAGAGUA | 947 |
| AD-1561686 | usascag(Ahd)GfuAf UfAfuuuccauuscsa | 678 | VPusGfsaauGfgAfAfa uauAfcUfcuguasusc | 813 | GAUACAGAGUAU AUUUCCAUUCA | 948 |
| AD-1561694 | asusauu(Uhd)CfcAf UfUfcagacaausasa | 679 | VPusUfsauuGfuCfUfg aauGfgAfaauausasc | 814 | GUAUAUUUCCAU UCAGACAAUAU | 949 |
| AD-1561703 | ususcag(Ahd)CfaAf UfAfuaucauaascsa | 680 | VPusGfsuuaUfgAfUfa uauUfgUfcugaasusg | 815 | CAUUCAGACAAU AUAUCAUAACU | 950 |
| AD-1447598 | ususgug(Ahd)UfaCf AfGfaguauauususa | 681 | VPusAfsaauAfuAfCfu cugUfaUfcacaascsu | 816 | AGUUGUGAUACA GAGUAUAUUUC | 951 |

**Table 6. Modified Sense and Antisense Strand Sequences of CA2 dsRNA Agents with GalNAc Modification**

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559459 | asgsaucgGfuGfCfCfgauuccugcuL96 | 952 | asGfscagGfaAfUfcggcAfcCfgaucusgsg | 1087 | CCAGAUCGGUGCCGAUUCCUGCC | 817 |
| AD-1559476 | csgscgacCfaUfGfUfcccaucacuuL96 | 953 | asAfsgugAfuGfGfgacaUfgGfucgcgscsu | 1088 | AGCGCGACCAUGUCCCAUCACUG | 818 |
| AD-1559481 | gsusacggCfaAfAfCfacaacggacuL96 | 954 | asGfsuccGfuUfGfuguuUfgCfcguacscsc | 1089 | GGGUACGGCAAACACAACGGACC | 819 |
| AD-1559487 | csasaacaCfaAfCfGfgaccugagcuL96 | 955 | asGfscucAfgGfUfccguUfgUfguuugscsc | 1090 | GGCAAACACAACGGACCUGAGCA | 820 |
| AD-1559497 | gsgsaccuGfaGfCfAfcuggcauaauL96 | 956 | asUfsuauGfcCfAfgugcUfcAfgguccsgsu | 1091 | ACGGACCUGAGCACUGGCAUAAG | 821 |
| AD-1559503 | gsasgcacUfgGfCfAfuaaggacuuuL96 | 957 | asAfsaguCfcUfUfaugcCfaGfugcucsasg | 1092 | CUGAGCACUGGCAUAAGGACUUC | 822 |
| AD-1559514 | gsusugacAfuCfGfAfcacucauacuL96 | 958 | asGfsuauGfaGfUfgucgAfuGfucaacsasg | 1093 | CUGUUGACAUCGACACUCAUACA | 823 |
| AD-1559524 | ascsacucAfuAfCfAfgccaaguauuL96 | 959 | asAfsuacUfuGfGfcuguAfuGfagugucsg | 1094 | CGACACUCAUACAGCCAAGUAUG | 824 |
| AD-1559531 | usascagcCfaAfGfUfaugacccuuuL96 | 960 | asAfsaggGfuCfAfuacuUfgGfcuguasusg | 1095 | CAUACAGCCAAGUAUGACCCUUC | 825 |
| AD-1559537 | csasaguaUfgAfCfCfcuucccugauL96 | 961 | asUfscagGfgAfAfggguCfaUfacuugsgsc | 1096 | GCCAAGUAUGACCCUUCCCUGAA | 826 |
| AD-1559543 | usgsucugUfuUfCfCfuaugaucaauL96 | 962 | asUfsugaUfcAfUfaggaAfaCfagacasgsg | 1097 | CCUGUCUGUUUCCUAUGAUCAAG | 827 |
| AD-1559552 | cscsuaugAfuCfAfAfgcaacuuccuL96 | 963 | asGfsgaaGfuUfGfcuugAfuCfauaggsasa | 1098 | UUCCUAUGAUCAAGCAACUUCCC | 828 |
| AD-1559560 | csasagcaAfcUfUfCfccugaggauuL96 | 964 | asAfsuccUfcAfGfggaaGfuUfgcuugsasu | 1099 | AUCAAGCAACUUCCCUGAGGAUC | 829 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559570 | cscscugaGfgAfUfCfcucaacaauuL96 | 965 | asAfsuugUfuGfAfggauCfcUfcagggsasa | 1100 | UUCCCUGAGGAUCCUCAACAAUG | 830 |
| AD-1559579 | uscscucaAfcAfAfUfggucaugcuuL96 | 966 | asAfsgcaUfgAfCfcauuGfuUfgaggasusc | 1101 | GAUCCUCAACAAUGGUCAUGCUU | 831 |
| AD-1559585 | ascsaaugGfuCfAfUfgcuuucaacuL96 | 967 | asGfsuugAfaAfGfcaugAfcCfauugususg | 1102 | CAACAAUGGUCAUGCUUUCAACG | 832 |
| AD-1559594 | asusgcuuUfcAfAfCfguggaguuuuL96 | 968 | asAfsaacUfcCfAfcguuGfaAfagcausgsa | 1103 | UCAUGCUUUCAACGUGGAGUUUG | 833 |
| AD-1559602 | asascgugGfaGfUfUfugaugacucuL96 | 969 | asGfsaguCfaUfCfaaacUfcCfacguusgsa | 1104 | UCAACGUGGAGUUUGAUGACUCU | 834 |
| AD-1559613 | usgsaugaCfuCfUfCfaggacaaaguL96 | 970 | asCfsuuuGfuCfCfugagAfgUfcaucasasa | 1105 | UUUGAUGACUCUCAGGACAAAGC | 835 |
| AD-1559620 | uscsucagGfaCfAfAfagcagugcuuL96 | 971 | asAfsgcaCfuGfCfuuugUfcCfugagasgsu | 1106 | ACUCUCAGGACAAAGCAGUGCUC | 836 |
| AD-1559626 | gsascaaaGfcAfGfUfgcucaaggguL96 | 972 | asCfsccuUfgAfGfcacuGfcUfuugucscsu | 1107 | AGGACAAAGCAGUGCUCAAGGGA | 837 |
| AD-1559638 | usgsgcacUfuAfCfAfgauugauucuL96 | 973 | asGfsaauCfaAfUfcuguAfaGfugccasusc | 1108 | GAUGGCACUUACAGAUUGAUUCA | 838 |
| AD-1559644 | ususacagAfuUfGfAfuucaguuucuL96 | 974 | asGfsaaaCfuGfAfaucaAfuCfuguaasgsu | 1109 | ACUUACAGAUUGAUUCAGUUUCA | 839 |
| AD-1559654 | asusucagUfuUfCfAfcuuucacuguL96 | 975 | asCfsaguGfaAfAfgugaAfaCfugaauscsa | 1110 | UGAUUCAGUUUCACUUUCACUGG | 840 |
| AD-1559660 | uscsacuuGfaUfGfGfacaagguucuL96 | 976 | asGfsaacCfuUfGfuccaUfcAfagugasasc | 1111 | GUUCACUUGAUGGACAAGGUUCA | 841 |
| AD-1559666 | gsasuggaCfaAfGfGfuucagagcauL96 | 977 | asUfsgcuCfuGfAfaccuUfgUfccaucsasa | 1112 | UUGAUGGACAAGGUUCAGAGCAU | 842 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559672 | csasagguUfcAfGfA fgcauacuguuL96 | 978 | asAfscagUfaUfGfcucu GfaAfccuugsusc | 1113 | GACAAGGUUCAG AGCAUACUGUG | 843 |
| AD-1559678 | uscsagagCfaUfAfC fuguggauaauL96 | 979 | asUfsuauCfcAfCfagua UfgCfucugasasc | 1114 | GUUCAGAGCAUA CUGUGGAUAAA | 844 |
| AD-1559699 | asasgaaaUfaUfGfC fugcagaacuuL96 | 980 | asAfsguuCfuGfCfagca UfaUfuucuususu | 1115 | AAAAGAAAUAUG CUGCAGAACUU | 845 |
| AD-1559705 | usasugcuGfcAfGf AfacuucacuuuL96 | 981 | asAfsaguGfaAfGfuucu GfcAfgcauasusu | 1116 | AAUAUGCUGCAG AACUUCACUUG | 846 |
| AD-1559711 | gscsagaaCfuUfCfA fcuugguucauL96 | 982 | asUfsgaaCfcAfAfguga AfgUfucugcsasg | 1117 | CUGCAGAACUUC ACUUGGUUCAC | 847 |
| AD-1559717 | csusucacUfuGfGfU fucacuggaauL96 | 983 | asUfsuccAfgUfGfaacc AfaGfugaagsusu | 1118 | AACUUCACUUGG UUCACUGGAAC | 848 |
| AD-1559728 | uscsacugGfaAfCfA fccaaauauguL96 | 984 | asCfsauaUfuUfGfgugu UfcCfagugasasc | 1119 | GUUCACUGGAAC ACCAAAUAUGG | 849 |
| AD-1559735 | ususugggAfaAfGf CfugugcagcauL96 | 985 | asUfsgcuGfcAfCfagcu UfuCfccaaasasu | 1120 | AUUUUGGGAAAG CUGUGCAGCAA | 850 |
| AD-1559747 | gsusgcagCfaAfCfC fugauggacuuL96 | 986 | asAfsgucCfaUfCfaggu UfgCfugcacsasg | 1121 | CUGUGCAGCAAC CUGAUGGACUG | 851 |
| AD-1559753 | csasaccuGfaUfGfG facuggccguuL96 | 987 | asAfscggCfcAfGfucca UfcAfgguugscsu | 1122 | AGCAACCUGAUG GACUGGCCGUU | 852 |
| AD-1559765 | csusggccGfuUfCfU fagguauuuuuL96 | 988 | asAfsaaaUfaCfCfuaga AfcGfgccagsusc | 1123 | GACUGGCCGUUC UAGGUAUUUUU | 853 |
| AD-1559768 | usgsaaggUfuGfGf CfagcgcuaaauL96 | 989 | asUfsuuaGfcGfCfugcc AfaCfcuucasasa | 1124 | UUUGAAGGUUGG CAGCGCUAAAC | 854 |
| AD-1559777 | gscsagcgCfuAfAfA fccgggccuuuL96 | 990 | asAfsaggCfcCfGfguuu AfgCfgcugcscsa | 1125 | UGGCAGCGCUAA ACCGGGCCUUC | 855 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559788 | cscsgggcCfuUfCfAfgaaaguuguuL96 | 991 | asAfscaaCfuUfUfcugaAfgGfcccggsusu | 1126 | AACCGGGCCUUCAGAAAGUUGUU | 856 |
| AD-1559794 | csusucagAfaAfGfUfuguugauguuL96 | 992 | asAfscauCfaAfCfaacuUfuCfugaagsgsc | 1127 | GCCUUCAGAAAGUUGUUGAUGUG | 857 |
| AD-1559803 | gsusuguuGfaUfGfUfgcuggauucuL96 | 993 | asGfsaauCfcAfGfcacaUfcAfacaacsusu | 1128 | AAGUUGUUGAUGUGCUGGAUUCC | 858 |
| AD-1559814 | gscsuggaUfuCfCfAfuuaaaacaauL96 | 994 | asUfsuguUfuUfAfauggAfaUfccagcsasc | 1129 | GUGCUGGAUUCCAUUAAAACAAA | 859 |
| AD-1559821 | uscscauuAfaAfAfCfaaagggcaauL96 | 995 | asUfsugcCfcUfUfuguuUfuAfauggasasu | 1130 | AUUCCAUUAAAACAAAGGGCAAG | 860 |
| AD-1559827 | asasaacaAfaGfGfGfcaagagugcuL96 | 996 | asGfscacUfcUfUfgcccUfuUfguuuusasa | 1131 | UUAAAACAAAGGGCAAGAGUGCU | 861 |
| AD-1559836 | gsgscaagAfgUfGfCfugacuucacuL96 | 997 | asGfsugaAfgUfCfagcaCfuCfuugccscsu | 1132 | AGGGCAAGAGUGCUGACUUCACU | 862 |
| AD-1559843 | gsusgcugAfcUfUfCfacuaacuucuL96 | 998 | asGfsaagUfuAfGfugaaGfuCfagcacsusc | 1133 | GAGUGCUGACUUCACUAACUUCG | 863 |
| AD-1559849 | ascsuucaCfuAfAfCfuucgauccuuL96 | 999 | asAfsggaUfcGfAfaguuAfgUfgaaguscsa | 1134 | UGACUUCACUAACUUCGAUCCUC | 864 |
| AD-1559862 | csgsauccUfcGfUfGfgccuccuucuL96 | 1000 | asGfsaagGfaGfGfccacGfaGfgaucgsasa | 1135 | UUCGAUCCUCGUGGCCUCCUUCC | 865 |
| AD-1559868 | uscsguggCfcUfCfCfuuccugaauuL96 | 1001 | asAfsuucAfgGfAfaggaGfgCfcacgasgsg | 1136 | CCUCGUGGCCUCCUUCCUGAAUC | 866 |
| AD-1559874 | cscsuccuUfcCfUfGfaauccugguL96 | 1002 | asCfscaaGfgAfUfucagGfaAfggaggscsc | 1137 | GGCCUCCUUCCUGAAUCCUUGGA | 867 |
| AD-1559881 | cscsugaaUfcCfUfUfggauuacuguL96 | 1003 | asCfsaguAfaUfCfcaagGfaUfucaggsasa | 1138 | UUCCUGAAUCCUUGGAUUACUGG | 868 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559887 | uscscuugGfaUfUf AfcuggaccuauL96 | 1004 | asUfsaggUfcCfAfguaa UfcCfaaggasusu | 1139 | AAUCCUUGGAUU ACUGGACCUAC | 869 |
| AD-1559903 | cscsuaccCfaGfGfC fucacugaccuL96 | 1005 | asGfsgucAfgUfGfagcc UfgGfguaggsusc | 1140 | GACCUACCCAGG CUCACUGACCA | 870 |
| AD-1559909 | cscsucuuCfuGfGfA faugugugacuL96 | 1006 | asGfsucaCfaCfAfuucc AfgAfagaggsasg | 1141 | CUCCUCUUCUGG AAUGUGUGACC | 871 |
| AD-1559916 | usgsgaauGfuGfUf GfaccuggauuuL96 | 1007 | asAfsaucCfaGfGfucac AfcAfuuccasgsa | 1142 | UCUGGAAUGUGU GACCUGGAUUG | 872 |
| AD-1559923 | usgsugacCfuGfGf AfuugugcucauL96 | 1008 | asUfsgagCfaCfAfaucc AfgGfucacascsa | 1143 | UGUGUGACCUGG AUUGUGCUCAA | 873 |
| AD-1559929 | csusggauUfgUfGf CfucaaggaacuL96 | 1009 | asGfsuucCfuUfGfagca CfaAfuccagsgsu | 1144 | ACCUGGAUUGUG CUCAAGGAACC | 874 |
| AD-1559939 | csuscaagGfaAfCfC fcaucagcguuL96 | 1010 | asAfscgcUfgAfUfgggu UfcCfuugagscsa | 1145 | UGCUCAAGGAAC CCAUCAGCGUC | 875 |
| AD-1559945 | gsasacccAfuCfAfG fcgucagcaguL96 | 1011 | asCfsugcUfgAfCfgcug AfuGfgguucscsu | 1146 | AGGAACCCAUCA GCGUCAGCAGC | 876 |
| AD-1559965 | csgsagcaGfgUfGfU fugaaauuccuL96 | 1012 | asGfsgaaUfuUfCfaaca CfcUfgcucgscsu | 1147 | AGCGAGCAGGUG UUGAAAUUCCG | 877 |
| AD-1559971 | gsgsuguuGfaAfAf UfuccguaaacuL96 | 1013 | asGfsuuuAfcGfGfaauu UfcAfacaccsusg | 1148 | CAGGUGUUGAAA UUCCGUAAACU | 878 |
| AD-1559977 | gsasaauuCfcGfUfA faacuuaacuuL96 | 1014 | asAfsguuAfaGfUfuuac GfgAfauuucsasa | 1149 | UUGAAAUUCCGU AAACUUAACUU | 879 |
| AD-1559983 | cscsguaaAfcUfUfA facuucaauguL96 | 1015 | asCfsauuGfaAfGfuuaa GfuUfuacggsasa | 1150 | UUCCGUAAACUU AACUUCAAUGG | 880 |
| AD-1559987 | gsasggguGfaAfCfC fcgaagaacuuL96 | 1016 | asAfsguuCfuUfCfgggu UfcAfcccucscsc | 1151 | GGGAGGGUGAAC CCGAAGAACUG | 881 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1559993 | gsasacccGfaAfGfA facugaugguuL96 | 1017 | asAfsccaUfcAfGfuucu UfcGfgguucsasc | 1152 | GUGAACCCGAAG AACUGAUGGUG | 882 |
| AD-1560001 | asgsaacuGfaUfGfG fuggacaacuuL96 | 1018 | asAfsguuGfuCfCfacca UfcAfguucususc | 1153 | GAAGAACUGAUG GUGGACAACUG | 883 |
| AD-1560008 | asusggugGfaCfAf AfcuggcgcccuL96 | 1019 | asGfsggcGfcCfAfguug UfcCfaccauscsa | 1154 | UGAUGGUGGACA ACUGGCGCCCA | 884 |
| AD-1560024 | cscsagcuCfaGfCfC facugaagaauL96 | 1020 | asUfsucuUfcAfGfuggc UfgAfgcuggsgsc | 1155 | GCCCAGCUCAGC CACUGAAGAAC | 885 |
| AD-1560030 | csasgccaCfuGfAfA fgaacaggcauL96 | 1021 | asUfsgccUfgUfUfcuuc AfgUfggcugsasg | 1156 | CUCAGCCACUGA AGAACAGGCAA | 886 |
| AD-1560036 | csusgaagAfaCfAfG fgcaaaucaauL96 | 1022 | asUfsugaUfuUfGfccug UfuCfuucagsusg | 1157 | CACUGAAGAACA GGCAAAUCAAA | 887 |
| AD-1560046 | gsgscaaaUfcAfAfA fgcuuccuucuL96 | 1023 | asGfsaagGfaAfGfcuuu GfaUfuugccsusg | 1158 | CAGGCAAAUCAA AGCUUCCUUCA | 888 |
| AD-1560053 | csasaagcUfuCfCfU fucaaauaaguL96 | 1024 | asCfsuuaUfuUfGfaagg AfaGfcuuugsasu | 1159 | AUCAAAGCUUCC UUCAAAUAAGA | 889 |
| AD-1560059 | ususccuuCfaAfAfU faagaugggucuL96 | 1025 | asGfsaccAfuCfUfuauu UfgAfaggaasgsc | 1160 | GCUUCCUUCAAA UAAGAUGGUCC | 890 |
| AD-1560068 | asusaagaUfgGfUfC fccauagucuuL96 | 1026 | asAfsgacUfaUfGfggac CfaUfcuuaususu | 1161 | AAAUAAGAUGGU CCCAUAGUCUG | 891 |
| AD-1560074 | usgsguccCfaUfAfG fucuguaucccuL96 | 1027 | asGfsgauAfcAfGfacua UfgGfgaccasusc | 1162 | GAUGGUCCCAUA GUCUGUAUCCA | 892 |
| AD-1560081 | asusagucUfgUfAf UfccaaauaauuL96 | 1028 | asAfsuuaUfuUfGfgaua CfaGfacuausgsg | 1163 | CCAUAGUCUGUA UCCAAAUAAUG | 893 |
| AD-1560088 | gsusauccAfaAfUfA faugaaucuuuL96 | 1029 | asAfsagaUfuCfAfuuau UfuGfgauacsasg | 1164 | CUGUAUCCAAAU AAUGAAUCUUC | 894 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560096 | asusaaugAfaUfCfU fucggguguuuL96 | 1030 | asAfsacaCfcCfGfaaga UfuCfauuaususu | 1165 | AAAUAAUGAAUC UUCGGGUGUUU | 895 |
| AD-1560103 | asuscuucGfgGfUf GfuuucccuuuL96 | 1031 | asAfsaagGfgAfAfacac CfcGfaagaususc | 1166 | GAAUCUUCGGGU GUUUCCCUUUA | 896 |
| AD-1560109 | gsgsguguUfuCfCf CfuuuagcuaauL96 | 1032 | asUfsuagCfuAfAfaggg AfaAfcacccsgsa | 1167 | UCGGGUGUUUCC CUUUAGCUAAG | 897 |
| AD-1560117 | cscscuuuAfgCfUfA fagcacagauuL96 | 1033 | asAfsucuGfuGfCfuuag CfuAfaagggsasa | 1168 | UUCCCUUUAGCU AAGCACAGAUC | 898 |
| AD-1560123 | asgscuaaGfcAfCfA fgaucuaccuuL96 | 1034 | asAfsgguAfgAfUfcugu GfcUfuagcusasa | 1169 | UUAGCUAAGCAC AGAUCUACCUU | 899 |
| AD-1560132 | csasgaucUfaCfCfU fuggugauuuuL96 | 1035 | asAfsaauCfaCfCfaagg UfaGfaucugsusg | 1170 | CACAGAUCUACC UUGGUGAUUUG | 900 |
| AD-1560139 | ascscuugGfuGfAf UfuuggacccuuL96 | 1036 | asAfsgggUfcCfAfaauc AfcCfaaggusasg | 1171 | CUACCUUGGUGA UUUGGACCCUG | 901 |
| AD-1560150 | ususggacCfcUfGfG fuugcuuuguuL96 | 1037 | asAfscaaAfgCfAfacca GfgGfuccaasasu | 1172 | AUUUGGACCCUG GUUGCUUUGUG | 902 |
| AD-1560157 | csusgguuGfcUfUf UfgugucuaguuL96 | 1038 | asAfscuaGfaCfAfcaaa GfcAfaccagsgsg | 1173 | CCCUGGUUGCUU UGUGUCUAGUU | 903 |
| AD-1560163 | gscsuuugUfgUfCf UfaguuuucuauL96 | 1039 | asUfsagaAfaAfCfuaga CfaCfaaagcsasa | 1174 | UUGCUUUGUGUC UAGUUUUCUAG | 904 |
| AD-1560172 | csusaguuUfuCfUf AfgacccuucauL96 | 1040 | asUfsgaaGfgGfUfcuag AfaAfacuagsasc | 1175 | GUCUAGUUUUCU AGACCCUUCAU | 905 |
| AD-1560178 | ususcuagAfcCfCfU fucaucucuuuL96 | 1041 | asAfsagaGfaUfGfaagg GfuCfuagaasasa | 1176 | UUUUCUAGACCC UUCAUCUCUUA | 906 |
| AD-1560184 | ascsccuuCfaUfCfU fcuuacuugauL96 | 1042 | asUfscaaGfuAfAfgaga UfgAfaggguscsu | 1177 | AGACCCUUCAUC UCUUACUUGAU | 907 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560191 | asuscucuUfaCfUfUfgauagacuuuL96 | 1043 | asAfsaguCfuAfUfcaagUfaAfgagausgsa | 1178 | UCAUCUCUUACUUGAUAGACUUA | 908 |
| AD-1560197 | usascuugAfuAfGfAfcuuacuaauuL96 | 1044 | asAfsuuaGfuAfAfgucuAfuCfaaguasasg | 1179 | CUUACUUGAUAGACUUACUAAUA | 909 |
| AD-1560205 | csusuacuAfaUfAfAfaaugugaaguL96 | 1045 | asCfsuucAfcAfUfuuuaUfuAfguaagsusc | 1180 | GACUUACUAAUAAAAUGUGAAGA | 910 |
| AD-1560214 | asasaaugUfgAfAfGfacuagaccauL96 | 1046 | asUfsgguCfuAfGfucuuCfaCfauuuusasu | 1181 | AUAAAAUGUGAAGACUAGACCAA | 911 |
| AD-1560220 | usgsaagaCfuAfGfAfccaauugucuL96 | 1047 | asGfsacaAfuUfGfgucuAfgUfcuucascsa | 1182 | UGUGAAGACUAGACCAAUUGUCA | 912 |
| AD-1560227 | usasgaccAfaUfUfGfucaugcuuguL96 | 1048 | asCfsaagCfaUfGfacaaUfuGfgucuasgsu | 1183 | ACUAGACCAAUUGUCAUGCUUGA | 913 |
| AD-1560238 | uscsaugcUfuGfAfCfacaacugcuuL96 | 1049 | asAfsgcaGfuUfGfugucAfaGfcaugascsa | 1184 | UGUCAUGCUUGACACAACUGCUG | 914 |
| AD-1560244 | ususgacaCfaAfCfUfgcuguggcuuL96 | 1050 | asAfsgccAfcAfGfcaguUfgUfgucaasgsc | 1185 | GCUUGACACAACUGCUGUGGCUG | 915 |
| AD-1560256 | csusguggCfuGfGfUfuggugcuuuuL96 | 1051 | asAfsaagCfaCfCfaaccAfgCfcacagscsa | 1186 | UGCUGUGGCUGGUUGGUGCUUUG | 916 |
| AD-1560262 | csusgguuGfgUfGfCfuuuguuuauuL96 | 1052 | asAfsuaaAfcAfAfagcaCfcAfaccagscsc | 1187 | GGCUGGUUGGUGCUUUGUUUAUG | 917 |
| AD-1560268 | gsgsugcuUfuGfUfUfuaugguaguuL96 | 1053 | asAfscuaCfcAfUfaaacAfaAfgcaccsasa | 1188 | UUGGUGCUUUGUUUAUGGUAGUA | 918 |
| AD-1560274 | ususguuuAfuGfGfUfaguaguuuuuL96 | 1054 | asAfsaaaCfuAfCfuaccAfuAfaacaasasg | 1189 | CUUUGUUUAUGGUAGUAGUUUUU | 919 |
| AD-1560280 | usgsguagUfaGfUfUfuuucuguaauL96 | 1055 | asUfsuacAfgAfAfaaacUfaCfuaccasusa | 1190 | UAUGGUAGUAGUUUUUCUGUAAC | 920 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560286 | usasguuuUfuCfUf GfuaacacagauL96 | 1056 | asUfscugUfgUfUfacag AfaAfaacuascsu | 1191 | AGUAGUUUUCU GUAACACAGAA | 921 |
| AD-1560292 | ususcuguAfaCfAfC fagaauauaguL96 | 1057 | asCfsuauAfuUfCfugug UfuAfcagaasasa | 1192 | UUUUCUGUAACA CAGAAUAUAGG | 922 |
| AD-1560300 | csascagaAfuAfUfA fggauaagaauL96 | 1058 | asUfsucuUfaUfCfcuau AfuUfcugugsusu | 1193 | AACACAGAAUAU AGGAUAAGAAA | 923 |
| AD-1560311 | asgsaauaAfaGfUfA fccuugacuuuL96 | 1059 | asAfsaguCfaAfGfguac UfuUfauucususa | 1194 | UAAGAAUAAAGU ACCUUGACUUU | 924 |
| AD-1560323 | csusugacUfuUfGf UfucacagcauuL96 | 1060 | asAfsugcUfgUfGfaaca AfaGfucaagsgsu | 1195 | ACCUUGACUUUG UUCACAGCAUG | 925 |
| AD-1560329 | ususuguuCfaCfAf GfcauguaggguL96 | 1061 | asCfsccuAfcAfUfgcug UfgAfacaaasgsu | 1196 | ACUUUGUUCACA GCAUGUAGGGU | 926 |
| AD-1560335 | csascagcAfuGfUfA fggugaugauL96 | 1062 | asUfscauCfaCfCfcuac AfuGfcugugsasa | 1197 | UUCACAGCAUGU AGGGUGAUGAG | 927 |
| AD-1560344 | usasggguGfaUfGf AfgcacucacauL96 | 1063 | asUfsgugAfgUfGfcuca UfcAfcccuascsa | 1198 | UGUAGGGUGAUG AGCACUCACAA | 928 |
| AD-1560350 | gsasugagCfaCfUfC facaauuguuuL96 | 1064 | asAfsacaAfuUfGfugag UfgCfucaucsasc | 1199 | GUGAUGAGCACU CACAAUUGUUG | 929 |
| AD-1560357 | ascsucacAfaUfUfG fuugacuaaauL96 | 1065 | asUfsuuaGfuCfAfacaa UfuGfugagusgsc | 1200 | GCACUCACAAUU GUUGACUAAAA | 930 |
| AD-1560368 | ususgacuAfaAfAf UfgcugcuuuuuL96 | 1066 | asAfsaaaGfcAfGfcauu UfuAfgucaascsa | 1201 | UGUUGACUAAAA UGCUGCUUUUA | 931 |
| AD-1560377 | asusgcugCfuUfUf UfaaaacauaguL96 | 1067 | asCfsuauGfuUfUfuaaa AfgCfagcaususu | 1202 | AAAUGCUGCUUU UAAAACAUAGG | 932 |
| AD-1560383 | csusuuuaAfaAfCfA fuaggaaaguuL96 | 1068 | asAfscuuUfcCfUfaugu UfuUfaaaagscsa | 1203 | UGCUUUUAAAAC AUAGGAAAGUA | 933 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560392 | csasuaggAfaAfGfUfagaaugguuuL96 | 1069 | asAfsaccAfuUfCfuacuUfuCfcuaugsusu | 1204 | AACAUAGGAAAGUAGAAUGGUUG | 934 |
| AD-1560400 | asgsuagaAfuGfGfUfugagugcaauL96 | 1070 | asUfsugcAfcUfCfaaccAfuUfcuacususu | 1205 | AAAGUAGAAUGGUUGAGUGCAAA | 935 |
| AD-1560406 | asusgguuGfaGfUfGfcaaauccauuL96 | 1071 | asAfsuggAfuUfUfgcacUfcAfaccaususc | 1206 | GAAUGGUUGAGUGCAAAUCCAUA | 936 |
| AD-1560413 | asgsugcaAfaUfCfCfauagcacaauL96 | 1072 | asUfsuguGfcUfAfuggaUfuUfgcacuscsa | 1207 | UGAGUGCAAAUCCAUAGCACAAG | 937 |
| AD-1560421 | uscscauaGfcAfCfAfagauaaauuuL96 | 1073 | asAfsauuUfaUfCfuuguGfcUfauggasusu | 1208 | AAUCCAUAGCACAAGAUAAAUUG | 938 |
| AD-1560430 | csasagauAfaAfUfUfgagcuaguuuL96 | 1074 | asAfsacuAfgCfUfcaauUfuAfucuugsusg | 1209 | CACAAGAUAAAUUGAGCUAGUUA | 939 |
| AD-1560441 | gsasgcuaGfuUfAfAfggcaaaucauL96 | 1075 | asUfsgauUfuGfCfcuuaAfcUfagcucsasa | 1210 | UUGAGCUAGUUAAGGCAAAUCAG | 940 |
| AD-1560449 | usasaggcAfaAfUfCfagguaaaauuL96 | 1076 | asAfsuuuUfaCfCfugauUfuGfccuuasasc | 1211 | GUUAAGGCAAAUCAGGUAAAAUA | 941 |
| AD-1560460 | asgsguaaAfaUfAfGfucaugauucuL96 | 1077 | asGfsaauCfaUfGfacuaUfuUfuaccusgsa | 1212 | UCAGGUAAAAUAGUCAUGAUUCU | 942 |
| AD-1560470 | gsuscaugAfuUfCfUfauguaauguuL96 | 1078 | asAfscauUfaCfAfuagaAfuCfaugacsusa | 1213 | UAGUCAUGAUUCUAUGUAAUGUA | 943 |
| AD-1560480 | usasuguaAfuGfUfAfaaccagaaauL96 | 1079 | asUfsuucUfgGfUfuuacAfuUfacauasgsa | 1214 | UCUAUGUAAUGUAAACCAGAAAA | 944 |
| AD-1560492 | uscsaugaUfuUfCfAfagauguuauuL96 | 1080 | asAfsuaaCfaUfCfuugaAfaUfcaugasasc | 1215 | GUUCAUGAUUUCAAGAUGUUAUA | 945 |
| AD-1560528 | csusuuugAfaUfUfAfcagagauauuL96 | 1081 | asAfsuauCfuCfUfguaaUfuCfaaaagsusc | 1216 | GACUUUUGAAUUACAGAGAUAUA | 946 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1560556 | ususagagUfuGfUf GfauacagaguuL96 | 1082 | asAfscucUfgUfAfucac AfaCfucuaasusu | 1217 | AAUUAGAGUUGU GAUACAGAGUA | 947 |
| AD-1560562 | ususgugaUfaCfAf GfaguauauuuuL96 | 1083 | asAfsaauAfuAfCfucug UfaUfcacaascsu | 1218 | AGUUGUGAUACA GAGUAUAUUUC | 948 |
| AD-1560568 | usascagaGfuAfUfA fuuuccauucuL96 | 1084 | asGfsaauGfgAfAfauau AfcUfcuguasusc | 1219 | GAUACAGAGUAU AUUUCCAUUCA | 949 |
| AD-1560576 | asusauuuCfcAfUfU fcagacaauauL96 | 1085 | asUfsauuGfuCfUfgaau GfgAfaauausasc | 1220 | GUAUAUUUCCAU UCAGACAAUAU | 950 |
| AD-1560585 | ususcagaCfaAfUfA fuaucauaacuL96 | 1086 | asGfsuuaUfgAfUfauau UfgUfcugaasusg | 1221 | CAUUCAGACAAU AUAUCAUAACU | 951 |

**Table 7. Unmodified Sense and Antisense Strand Sequences of CA2 dsRNA Agents**

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784188.1 | UGUUUCCUAUGA UCAAGCAAA | 1222 | 219-239 | UUUGCUTGAUCA UAGGAAACAGA | 1533 | 217-239 |
| AD-1784189.1 | UGACUUCACUAA CUUCGAUCA | 1223 | 594-614 | UGAUCGAAGUUA GUGAAGUCAGC | 1534 | 592-614 |
| AD-1784190.1 | CAAAGCUUCCUU CAAAUAAGA | 1224 | 840-860 | UCUUAUUUGAAG GAAGCUUUGAU | 1535 | 838-860 |
| AD-1784191.1 | UCAAAGCUUCCU UCAAAUAAA | 1225 | 839-859 | UUUATUTGAAGG AAGCUUUGAUU | 1536 | 837-859 |
| AD-1784192.1 | GUCUGUAUCCAA AUAAUGAAA | 1226 | 871-891 | UUUCAUUAUUUG GAUACAGACUA | 1537 | 869-891 |
| AD-1784193.1 | GUCUGUAUCCAA AUAAUGAAA | 1226 | 871-891 | UUUCAUTAUUUG GAUACAGACUA | 1538 | 869-891 |
| AD-1784194.1 | AUUCCGUAAACU UAACUUCAA | 1227 | 747-767 | UUGAAGTUAAGU UUACGGAAUUU | 1539 | 745-767 |
| AD-1784195.1 | UCCUAUGAUCAA GCAACUUCA | 1228 | 223-243 | UGAAGUTGCUUG AUCAUAGGAAA | 1540 | 221-243 |
| AD-1784196.1 | GUUUCCUAUGAU CAAGCAACA | 1229 | 220-240 | UGUUGCTUGAUC AUAGGAAACAG | 1541 | 218-240 |
| AD-1784197.1 | AUGCUGCUUUUA AAACAUAGA | 1230 | 1180-1200 | UCUATGTUUUAA AAGCAGCAUUU | 1542 | 1178-1200 |
| AD-1784198.1 | CAUUCAGACAAU AUAUCAUAA | 1231 | 1490-1510 | UUAUGATAUAUU GUCUGAAUGGA | 1543 | 1488-1510 |
| AD-1784199.1 | GACUUCACUAAC UUCGAUCCA | 1232 | 595-615 | UGGAUCGAAGUU AGUGAAGUCAG | 1544 | 593-615 |
| AD-1784200.1 | CCAUUCAGACAA UAUAUCAUA | 1233 | 1489-1509 | UAUGAUAUAUUG UCUGAAUGGAA | 1545 | 1487-1509 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784201.1 | UCUGUAUCCAAAUAAUGAAUA | 1234 | 872-892 | UAUUCATUAUUUGGAUACAGACU | 1546 | 870-892 |
| AD-1784202.1 | AAUCAAAGCUUCCUUCAAAUA | 1235 | 837-857 | UAUUTGAAGGAAGCUUUGAUUUG | 1547 | 835-857 |
| AD-1784203.1 | AUUCAGACAAUAUAUCAUAAA | 1236 | 1491-1511 | UUUATGAUAUAUUGUCUGAAUGG | 1548 | 1489-1511 |
| AD-1784204.1 | CCGUAAACUUAACUUCAAUGA | 1237 | 750-770 | UCAUUGAAGUUAAGUUUACGGAA | 1549 | 748-770 |
| AD-1784205.1 | CCGUAAACUUAACUUCAAUGA | 1237 | 750-770 | UCAUTGAAGUUAAGUUUACGGAA | 1550 | 748-770 |
| AD-1784206.1 | GUGCUGACUUCACUAACUUCA | 1238 | 590-610 | UGAAGUTAGUGAAGUCAGCACUC | 1551 | 588-610 |
| AD-1784207.1 | AAGCUUCCUUCAAAUAAGAUA | 1239 | 842-862 | UAUCUUAUUUGAAGGAAGCUUUG | 1552 | 840-862 |
| AD-1784208.1 | AAGCUUCCUUCAAAUAAGAUA | 1239 | 842-862 | UAUCTUAUUUGAAGGAAGCUUUG | 1553 | 840-862 |
| AD-1784209.1 | AAAUUCCGUAAACUUAACUUA | 1240 | 745-765 | UAAGUUAAGUUUACGGAAUUUCA | 1554 | 743-765 |
| AD-1784210.1 | CUGUCUGUUUCCUAUGAUCAA | 1241 | 214-234 | UUGATCAUAGGAAACAGACAGGG | 1555 | 212-234 |
| AD-1784211.1 | GUAUCCAAAUAAUGAAUCUUA | 1242 | 875-895 | UAAGAUUCAUUAUUUGGAUACAG | 1556 | 873-895 |
| AD-1784212.1 | GUAUCCAAAUAAUGAAUCUUA | 1242 | 875-895 | UAAGAUTCAUUAUUUGGAUACAG | 1557 | 873-895 |
| AD-1784213.1 | CUGACUUCACUAACUUCGAUA | 1243 | 593-613 | UAUCGAAGUUAGUGAAGUCAGCA | 1558 | 591-613 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784214.1 | GCUUCCUUCAAA UAAGAUGGA | 1244 | 844-864 | UCCAUCUUAUUU GAAGGAAGCUU | 1559 | 842-864 |
| AD-1784215.1 | GCUUCCUUCAAA UAAGAUGGA | 1244 | 844-864 | UCCATCTUAUUU GAAGGAAGCUU | 1560 | 842-864 |
| AD-1784216.1 | AAAUCAAAGCUU CCUUCAAAA | 1245 | 836-856 | UUUUGAAGGAAG CUUUGAUUUGC | 1561 | 834-856 |
| AD-1784217.1 | AGCUUCCUUCAA AUAAGAUGA | 1246 | 843-863 | UCAUCUUAUUUG AAGGAAGCUUU | 1562 | 841-863 |
| AD-1784218.1 | UGCUGCUUUUAA AACAUAGGA | 1247 | 1181-1201 | UCCUAUGUUUUA AAAGCAGCAUU | 1563 | 1179-1201 |
| AD-1784219.1 | AGGCAAAUCAAA GCUUCCUUA | 1248 | 832-852 | UAAGGAAGCUUU GAUUUGCCUGU | 1564 | 830-852 |
| AD-1784220.1 | AGGCAAAUCAAA GCUUCCUUA | 1248 | 832-852 | UAAGGAAGCUUU GAUUUGCCUGU | 1564 | 830-852 |
| AD-1784221.1 | GGCAAAUCAAAG CUUCCUUCA | 1249 | 833-853 | UGAAGGAAGCUU UGAUUUGCCUG | 1565 | 831-853 |
| AD-1784222.1 | AAAGCUUCCUUC AAAUAAGAA | 1250 | 841-861 | UUCUUAUUUGAA GGAAGCUUUGA | 1566 | 839-861 |
| AD-1784223.1 | AAAGCUUCCUUC AAAUAAGAA | 1250 | 841-861 | UUCUTATUUGAA GGAAGCUUUGA | 1567 | 839-861 |
| AD-1784224.1 | UAAAAUGCUGCU UUUAAAACA | 1251 | 1176-1196 | UGUUUUAAAAGC AGCAUUUUAGU | 1568 | 1174-1196 |
| AD-1784225.1 | AAGAAUAAAGUA CCUUGACUA | 1252 | 1113-1133 | UAGUCAAGGUAC UUUAUUCUUAU | 1569 | 1111-1133 |
| AD-1784226.1 | AGAAUAAAGUAC CUUGACUUA | 1253 | 1114-1134 | UAAGUCAAGGUA CUUUAUUCUUA | 1570 | 1112-1134 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784227.1 | AGAAUAAAGUAC CUUGACUUA | 1253 | 1114-1134 | UAAGTCAAGGUA CUUUAUUCUUA | 1571 | 1112-1134 |
| AD-1784228.1 | GUCUGUUUCCUA UGAUCAAGA | 1254 | 216-236 | UCUUGATCAUAG GAAACAGACAG | 1572 | 214-236 |
| AD-1784229.1 | UCCGUAAACUUA ACUUCAAUA | 1255 | 749-769 | UAUUGAAGUUAA GUUUACGGAAU | 1573 | 747-769 |
| AD-1784230.1 | CCUCUUCUGGAA UGUGUGACA | 1256 | 676-696 | UGUCACACAUUC CAGAAGAGGAG | 1574 | 674-696 |
| AD-1784231.1 | UAUCCAAAUAAU GAAUCUUCA | 1257 | 876-896 | UGAAGATUCAUU AUUUGGAUACA | 1575 | 874-896 |
| AD-1784232.1 | UCUGUUUCCUAU GAUCAAGCA | 1258 | 217-237 | UGCUTGAUCAUA GGAAACAGACA | 1576 | 215-237 |
| AD-1784233.1 | GUUGACAUCGAC ACUCAUACA | 1259 | 166-186 | UGUATGAGUGUC GAUGUCAACAG | 1577 | 164-186 |
| AD-1784234.1 | AAGUACCUUGAC UUUGUUCAA | 1260 | 1120-1140 | UUGAACAAAGUC AAGGUACUUUA | 1578 | 1118-1140 |
| AD-1784235.1 | AAGUACCUUGAC UUUGUUCAA | 1260 | 1120-1140 | UUGAACAAAGUC AAGGUACUUUA | 1578 | 1118-1140 |
| AD-1784236.1 | CAGAUCUACCUU GGUGAUUUA | 1261 | 919-939 | UAAATCACCAAG GUAGAUCUGUG | 1579 | 917-939 |
| AD-1784237.1 | CUGGAUUGUGCU CAAGGAACA | 1262 | 696-716 | UGUUCCUGAGC ACAAUCCAGGU | 1580 | 694-716 |
| AD-1784238.1 | UGCUUUUAAAAC AUAGGAAAA | 1263 | 1184-1204 | UUUUCCTAUGUU UUAAAAGCAGC | 1581 | 1182-1204 |
| AD-1784239.1 | UGCUGACUUCAC UAACUUCGA | 1264 | 591-611 | UCGAAGUUAGUG AAGUCAGCACU | 1582 | 589-611 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784240.1 | UGCUGACUUCAC UAACUUCGA | 1264 | 591-611 | UCGAAGTUAGUG AAGUCAGCACU | 1583 | 589-611 |
| AD-1784241.1 | GAAAUUCCGUAA ACUUAACUA | 1265 | 744-764 | UAGUUAAGUUUA CGGAAUUUCAA | 1584 | 742-764 |
| AD-1784242.1 | GAAAUUCCGUAA ACUUAACUA | 1265 | 744-764 | UAGUTAAGUUUA CGGAAUUUCAA | 1585 | 742-764 |
| AD-1784243.1 | UAAGGCAAAUCA GGUAAAAUA | 1266 | 1252-1272 | UAUUUUACCUGA UUUGCCUUAAC | 1586 | 1250-1272 |
| AD-1784244.1 | UAAGGCAAAUCA GGUAAAAUA | 1266 | 1252-1272 | UAUUTUACCUGA UUUGCCUUAAC | 1587 | 1250-1272 |
| AD-1784245.1 | GUUCUAGGUAUU UUUUUGAAA | 1267 | 499-519 | UUUCAAAAAAAU ACCUAGAACGG | 1588 | 497-519 |
| AD-1784246.1 | AAGAUAAAUUGA GCUAGUUAA | 1268 | 1234-1254 | UUAACUAGCUCA AUUUAUCUUGU | 1589 | 1232-1254 |
| AD-1784247.1 | UUAGCUAAGCAC AGAUCUACA | 1269 | 908-928 | UGUAGATCUGUG CUUAGCUAAAG | 1590 | 906-928 |
| AD-1784248.1 | CUUCACUAACUU CGAUCCUCA | 1270 | 597-617 | UGAGGATCGAAG UUAGUGAAGUC | 1591 | 595-617 |
| AD-1784249.1 | AAUUCCGUAAAC UUAACUUCA | 1271 | 746-766 | UGAAGUTAAGUU UACGGAAUUUC | 1592 | 744-766 |
| AD-1784250.1 | CUGCUUUUAAAA CAUAGGAAA | 1272 | 1183-1203 | UUUCCUAUGUUU UAAAAGCAGCA | 1593 | 1181-1203 |
| AD-1784251.1 | CUGUUGACAUCG ACACUCAUA | 1273 | 164-184 | UAUGAGTGUCGA UGUCAACAGGG | 1594 | 162-184 |
| AD-1784252.1 | UUCACUAACUUC GAUCCUCGA | 1274 | 598-618 | UCGAGGAUCGAA GUUAGUGAAGU | 1595 | 596-618 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784253.1 | GCUAAGCACAGA UCUACCUUA | 1275 | 911-931 | UAAGGUAGAUCU GUGCUUAGCUA | 1596 | 909-931 |
| AD-1784254.1 | UAAAGUACCUUG ACUUUGUUA | 1276 | 1118-1138 | UAACAAAGUCAA GGUACUUUAUU | 1597 | 1116-1138 |
| AD-1784255.1 | AAAAUGCUGCUU UUAAAACAA | 1277 | 1177-1197 | UUGUTUTAAAAG CAGCAUUUUAG | 1598 | 1175-1197 |
| AD-1784256.1 | GCUGCUUUUAAA ACAUAGGAA | 1278 | 1182-1202 | UUCCUAUGUUUU AAAAGCAGCAU | 1599 | 1180-1202 |
| AD-1784257.1 | GCUGCUUUUAAA ACAUAGGAA | 1278 | 1182-1202 | UUCCTATGUUUU AAAAGCAGCAU | 1600 | 1180-1202 |
| AD-1784258.1 | UCAUGAUUCUAU GUAAUGUAA | 1279 | 1274-1294 | UUACAUUACAUA GAAUCAUGACU | 1601 | 1272-1294 |
| AD-1784259.1 | UCAUGAUUCUAU GUAAUGUAA | 1279 | 1274-1294 | UUACAUTACAUA GAAUCAUGACU | 1602 | 1272-1294 |
| AD-1784260.1 | AGUGCUGACUUC ACUAACUUA | 1280 | 589-609 | UAAGUUAGUGAA GUCAGCACUCU | 1603 | 587-609 |
| AD-1784261.1 | CUAAGCACAGAU CUACCUUGA | 1281 | 912-932 | UCAAGGTAGAUC UGUGCUUAGCU | 1604 | 910-932 |
| AD-1784262.1 | CACUAACUUCGA UCCUCGUGA | 1282 | 600-620 | UCACGAGGAUCG AAGUUAGUGAA | 1605 | 598-620 |
| AD-1784263.1 | CUGAAGAACAGG CAAAUCAAA | 1283 | 823-843 | UUUGAUTUGCCU GUUCUUCAGUG | 1606 | 821-843 |
| AD-1784264.1 | AAAGUACCUUGA CUUUGUUCA | 1284 | 1119-1139 | UGAACAAAGUCA AGGUACUUUAU | 1607 | 1117-1139 |
| AD-1784265.1 | GCUUUGUUUAUG GUAGUAGUA | 1285 | 1061-1081 | UACUACTACCAU AAACAAAGCAC | 1608 | 1059-1081 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784266.1 | CAUGAUUCUAUG UAAUGUAAA | 1286 | 1275-1295 | UUUACAUUACAU AGAAUCAUGAC | 1609 | 1273-1295 |
| AD-1784267.1 | CGUUCUAGGUAU UUUUUUGAA | 1287 | 498-518 | UUCAAAAAAAUA CCUAGAACGGC | 1610 | 496-518 |
| AD-1784268.1 | UUCUAGGUAUUU UUUUGAAGA | 1288 | 500-520 | UCUUCAAAAAAA UACCUAGAACG | 1611 | 498-520 |
| AD-1784269.1 | UCCUUCCUGAAU CCUUGGAUA | 1289 | 623-643 | UAUCCAAGGAUU CAGGAAGGAGG | 1612 | 621-643 |
| AD-1784270.1 | UCCUUCCUGAAU CCUUGGAUA | 1289 | 623-643 | UAUCCAAGGAUU CAGGAAGGAGG | 1612 | 621-643 |
| AD-1784271.1 | GACUAAAAUGCU GCUUUUAAA | 1290 | 1173-1193 | UUUAAAAGCAGC AUUUUAGUCAA | 1613 | 1171-1193 |
| AD-1784272.1 | GACUAAAAUGCU GCUUUUAAA | 1290 | 1173-1193 | UUUAAAAGCAGC AUUUUAGUCAA | 1613 | 1171-1193 |
| AD-1784273.1 | AACAGGCAAAUC AAAGCUUCA | 1291 | 829-849 | UGAAGCTUUGAU UUGCCUGUUCU | 1614 | 827-849 |
| AD-1784274.1 | CCUUCCUGAAUC CUUGGAUUA | 1292 | 624-644 | UAAUCCAAGGAU UCAGGAAGGAG | 1615 | 622-644 |
| AD-1784275.1 | UGAUGACUCUCA GGACAAAGA | 1293 | 285-305 | UCUUTGTCCUGA GAGUCAUCAAA | 1616 | 283-305 |
| AD-1784276.1 | UGGAGUUUGAUG ACUCUCAGA | 1294 | 278-298 | UCUGAGAGUCAU CAAACUCCACG | 1617 | 276-298 |
| AD-1784277.1 | AUCCAAAUAAUG AAUCUUCGA | 1295 | 877-897 | UCGAAGAUUCAU UAUUUGGAUAC | 1618 | 875-897 |
| AD-1784278.1 | AUCCAAAUAAUG AAUCUUCGA | 1295 | 877-897 | UCGAAGAUUCAU UAUUUGGAUAC | 1618 | 875-897 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784279.1 | UUGACUUUGUUC ACAGCAUGA | 1296 | 1127-1147 | UCAUGCTGUGAA CAAAGUCAAGG | 1619 | 1125-1147 |
| AD-1784280.1 | AGAUCUACCUUG GUGAUUUGA | 1297 | 920-940 | UCAAAUCACCAA GGUAGAUCUGU | 1620 | 918-940 |
| AD-1784281.1 | AUGGUAGUAGUU UUUCUGUAA | 1298 | 1070-1090 | UUACAGAAAAAC UACUACCAUAA | 1621 | 1068-1090 |
| AD-1784282.1 | AUGGUAGUAGUU UUUCUGUAA | 1298 | 1070-1090 | UUACAGAAAAAC UACUACCAUAA | 1621 | 1068-1090 |
| AD-1784283.1 | CCUUGACUUUGU UCACAGCAA | 1299 | 1125-1145 | UUGCTGTGAACA AAGUCAAGGUA | 1622 | 1123-1145 |
| AD-1784284.1 | CCUGGAUUGUGC UCAAGGAAA | 1300 | 695-715 | UUUCCUTGAGCA CAAUCCAGGUC | 1623 | 693-715 |
| AD-1784285.1 | GAGCUAGUUAAG GCAAAUCAA | 1301 | 1244-1264 | UUGATUTGCCUU AACUAGCUCAA | 1624 | 1242-1264 |
| AD-1784286.1 | ACUGAAGAACAG GCAAAUCAA | 1302 | 822-842 | UUGATUTGCCUG UUCUUCAGUGG | 1625 | 820-842 |
| AD-1784287.1 | UGAAGAACAGGC AAAUCAAAA | 1303 | 824-844 | UUUUGATUUGCC UGUUCUUCAGU | 1626 | 822-844 |
| AD-1784288.1 | CUCCUCUUCUGG AAUGUGUGA | 1304 | 674-694 | UCACACAUUCCA GAAGAGGAGGG | 1627 | 672-694 |
| AD-1784289.1 | CUCCUCUUCUGG AAUGUGUGA | 1304 | 674-694 | UCACACAUUCCA GAAGAGGAGGG | 1627 | 672-694 |
| AD-1784290.1 | GCUUUCAACGUG GAGUUUGAA | 1305 | 268-288 | UUCAAACUCCAC GUUGAAAGCAU | 1628 | 266-288 |
| AD-1784291.1 | UGCUUUCAACGU GGAGUUUGA | 1306 | 267-287 | UCAAACUCCACG UUGAAAGCAUG | 1629 | 265-287 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784292.1 | UGCUUUCAACGU GGAGUUUGA | 1306 | 267-287 | UCAAACTCCACG UUGAAAGCAUG | 1630 | 265-287 |
| AD-1784293.1 | CAGGUAAAAUAG UCAUGAUUA | 1307 | 1262-1282 | UAAUCATGACUA UUUUACCUGAU | 1631 | 1260-1282 |
| AD-1784294.1 | CUGUAUCCAAAU AAUGAAUCA | 1308 | 873-893 | UGAUTCAUUAUU UGGAUACAGAC | 1632 | 871-893 |
| AD-1784295.1 | AAGGCAAAUCAG GUAAAAUAA | 1309 | 1253-1273 | UUAUTUTACCUG AUUUGCCUUAA | 1633 | 1251-1273 |
| AD-1784296.1 | CCUCCUUCCUGA AUCCUUGGA | 1310 | 621-641 | UCCAAGGAUUCA GGAAGGAGGCC | 1634 | 619-641 |
| AD-1784297.1 | UUCCUUCAAAUA AGAUGGUCA | 1311 | 846-866 | UGACCATCUUAU UUGAAGGAAGC | 1635 | 844-866 |
| AD-1784298.1 | UUGAAAUUCCGU AAACUUAAA | 1312 | 742-762 | UUUAAGTUUACG GAAUUUCAACA | 1636 | 740-762 |
| AD-1784299.1 | ACACUCAUACAG CCAAGUAUA | 1313 | 176-196 | UAUACUTGGCUG UAUGAGUGUCG | 1637 | 174-196 |
| AD-1784300.1 | GCACAGAUCUAC CUUGGUGAA | 1314 | 916-936 | UUCACCAAGGUA GAUCUGUGCUU | 1638 | 914-936 |
| AD-1784301.1 | CUUUCAACGUGG AGUUUGAUA | 1315 | 269-289 | UAUCAAACUCCA CGUUGAAAGCA | 1639 | 267-289 |
| AD-1784302.1 | UAGCUAAGCACA GAUCUACCA | 1316 | 909-929 | UGGUAGAUCUGU GCUUAGCUAAA | 1640 | 907-929 |
| AD-1784303.1 | UUGUGAUACAGA GUAUAUUUA | 1317 | 1469-1489 | UAAAUAUACUCU GUAUCACAACU | 1641 | 1467-1489 |
| AD-1784304.1 | ACUCAUACAGCC AAGUAUGAA | 1318 | 178-198 | UUCAUACUUGGC UGUAUGAGUGU | 1642 | 176-198 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784305.1 | AGUUAAGGCAAAUCAGGUAAA | 1319 | 1249-1269 | UUUACCTGAUUUGCCUUAACUAG | 1643 | 1247-1269 |
| AD-1784306.1 | GUUGUGAUACAGAGUAUAUUA | 1320 | 1468-1488 | UAAUAUACUCUGUAUCACAACUC | 1644 | 1466-1488 |
| AD-1784307.1 | GUUGUGAUACAGAGUAUAUUA | 1320 | 1468-1488 | UAAUAUACUCUGUAUCACAACUC | 1644 | 1466-1488 |
| AD-1784308.1 | UGACAUCGACACUCAUACAGA | 1321 | 168-188 | UCUGUAUGAGUGUCGAUGUCAAC | 1645 | 166-188 |
| AD-1784309.1 | AGAUAAAUUGAGCUAGUUAAA | 1322 | 1235-1255 | UUUAACTAGCUCAAUUUAUCUUG | 1646 | 1233-1255 |
| AD-1784310.1 | UAGGUAUUUUUUUGAAGGUUA | 1323 | 503-523 | UAACCUUCAAAAAAAUACCUAGA | 1647 | 501-523 |
| AD-1784311.1 | UAGGUAUUUUUUUGAAGGUUA | 1324 | 503-523 | UAACCUTCAAAAAAAUACCUAGA | 1648 | 501-523 |
| AD-1784312.1 | UGGUGCUUUGUUUAUGGUAGA | 1325 | 1057-1077 | UCUACCAUAAACAAAGCACCAAC | 1649 | 1055-1077 |
| AD-1784313.1 | UGUGAUACAGAGUAUAUUUCA | 1326 | 1470-1490 | UGAAAUAUACUCUGUAUCACAAC | 1650 | 1468-1490 |
| AD-1784314.1 | UCUUCUGGAAUGUGUGACCUA | 1327 | 678-698 | UAGGTCACACAUUCCAGAAGAGG | 1651 | 676-698 |
| AD-1784315.1 | CUGGCCGUUCUAGGUAUUUUA | 1328 | 493-513 | UAAAAUACCUAGAACGGCCAGUC | 1652 | 491-513 |
| AD-1784316.1 | AUCAGGUAAAAUAGUCAUGAA | 1329 | 1260-1280 | UUCATGACUAUUUUACCUGAUUU | 1653 | 1258-1280 |
| AD-1784317.1 | UUCCAUUAAAACAAAGGGCAA | 1330 | 567-587 | UUGCCCTUUGUUUUAAUGGAAUC | 1654 | 565-587 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784318.1 | CAAGAGUGCUGA CUUCACUAA | 1331 | 585-605 | UUAGTGAAGUCA GCACUCUUGCC | 1655 | 583-605 |
| AD-1784319.1 | UUUCAACGUGGA GUUUGAUGA | 1332 | 270-290 | UCAUCAAACUCC ACGUUGAAAGC | 1656 | 268-290 |
| AD-1784320.1 | UUGGUGCUUUGU UUAUGGUAA | 1333 | 1056-1076 | UUACCAUAAACA AAGCACCAACC | 1657 | 1054-1076 |
| AD-1784321.1 | UUGGUGCUUUGU UUAUGGUAA | 1333 | 1056-1076 | UUACCATAAACA AAGCACCAACC | 1658 | 1054-1076 |
| AD-1784322.1 | CACUCAUACAGC CAAGUAUGA | 1334 | 177-197 | UCAUACUUGGCU GUAUGAGUGUC | 1659 | 175-197 |
| AD-1784323.1 | AUAAAGUACCUU GACUUUGUA | 1335 | 1117-1137 | UACAAAGUCAAG GUACUUUAUUC | 1660 | 1115-1137 |
| AD-1784324.1 | AUGACUUUUGAA UUACAGAGA | 1336 | 1407-1427 | UCUCTGTAAUUC AAAAGUCAUUA | 1661 | 1405-1427 |
| AD-1784325.1 | GUCAUGAUUCUA UGUAAUGUA | 1337 | 1273-1293 | UACAUUACAUAG AAUCAUGACUA | 1662 | 1271-1293 |
| AD-1784326.1 | GACCUGGAUUGU GCUCAAGGA | 1338 | 693-713 | UCCUTGAGCACA AUCCAGGUCAC | 1663 | 691-713 |
| AD-1784327.1 | GACAUCGACACU CAUACAGCA | 1339 | 169-189 | UGCUGUAUGAGU GUCGAUGUCAA | 1664 | 167-189 |
| AD-1784328.1 | GAAGAACAGGCA AAUCAAAGA | 1340 | 825-845 | UCUUTGAUUUGC CUGUUCUUCAG | 1665 | 823-845 |
| AD-1784329.1 | CGUAAACUUAAC UUCAAUGGA | 1341 | 751-771 | UCCAUUGAAGUU AAGUUUACGGA | | 749-771 |
| AD-1784330.1 | AGGUAAAAUAGU CAUGAUUCA | 1342 | 1263-1283 | UGAATCAUGACU AUUUUACCUGA | 1666 | 1261-1283 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784331.1 | GUACCUUGACUU UGUUCACAA | 1343 | 1122-1142 | UUGUGAACAAAG UCAAGGUACUU | 1667 | 1120-1142 |
| AD-1784332.1 | CCGUUCUAGGUA UUUUUUUGA | 1344 | 497-517 | UCAAAAAAAUAC CUAGAACGGCC | 1668 | 495-517 |
| AD-1784333.1 | UUUAUGGUAGUA GUUUUUCUA | 1345 | 1067-1087 | UAGAAAAACUAC UACCAUAAACA | 1669 | 1065-1087 |
| AD-1784334.1 | CGUGGAGUUUGA UGACUCUCA | 1346 | 276-296 | UGAGAGTCAUCA AACUCCACGUU | 1670 | 274-296 |
| AD-1784335.1 | UUCAACGUGGAG UUUGAUGAA | 1347 | 271-291 | UUCATCAAACUC CACGUUGAAAG | 1671 | 269-291 |
| AD-1784336.1 | GAGUUGUGAUAC AGAGUAUAA | 1348 | 1466-1486 | UUAUACTCUGUA UCACAACUCUA | 1672 | 1464-1486 |
| AD-1784337.1 | UACCUUGACUUU GUUCACAGA | 1349 | 1123-1143 | UCUGUGAACAAA GUCAAGGUACU | 1673 | 1121-1143 |
| AD-1784338.1 | UACCUUGACUUU GUUCACAGA | 1349 | 1123-1143 | UCUGTGAACAAA GUCAAGGUACU | 1674 | 1121-1143 |
| AD-1784339.1 | UAGAGUUGUGAU ACAGAGUAA | 1350 | 1464-1484 | UUACTCTGUAUC ACAACUCUAAU | 1675 | 1462-1484 |
| AD-1784340.1 | UGAGUGCAAAUC CAUAGCACA | 1351 | 1214-1234 | UGUGCUAUGGAU UUGCACUCAAC | 1676 | 1212-1234 |
| AD-1784341.1 | CAAAUCAGGUAA AAUAGUCAA | 1352 | 1257-1277 | UUGACUAUUUUA CCUGAUUUGCC | 1677 | 1255-1277 |
| AD-1784342.1 | AAGCACAGAUCU ACCUUGGUA | 1353 | 914-934 | UACCAAGGUAGA UCUGUGCUUAG | 1678 | 912-934 |
| AD-1784343.1 | ACUUUGUUCACA GCAUGUAGA | 1354 | 1130-1150 | UCUACAUGCUGU GAACAAAGUCA | 1679 | 1128-1150 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784344.1 | UGGCCGUUCUAG GUAUUUUUA | 1355 | 494-514 | UAAAAAUACCUA GAACGGCCAGU | 1680 | 492-514 |
| AD-1784345.1 | GCCAAGUAUGAC CCUUCCCUA | 1356 | 187-207 | UAGGGAAGGGUC AUACUUGGCUG | 1681 | 185-207 |
| AD-1784346.1 | UAUGGUAGUAGU UUUUCUGUA | 1357 | 1069-1089 | UACAGAAAAACU ACUACCAUAAA | 1682 | 1067-1089 |
| AD-1784347.1 | AAUUGAGCUAGU UAAGGCAAA | 1358 | 1240-1260 | UUUGCCUUAACU AGCUCAAUUUA | 1683 | 1238-1260 |
| AD-1784348.1 | ACUAAAAUGCUG CUUUUAAAA | 1359 | 1174-1194 | UUUUAAAAGCAG CAUUUUAGUCA | 1684 | 1172-1194 |
| AD-1784349.1 | ACUUCACUUGGU UCACUGGAA | 1360 | 426-446 | UUCCAGTGAACC AAGUGAAGUUC | 1685 | 424-446 |
| AD-1784350.1 | UCUAGGUAUUUU UUUGAAGGA | 1361 | 501-521 | UCCUUCAAAAAA AUACCUAGAAC | 1686 | 499-521 |
| AD-1784351.1 | AGCACAGAUCUA CCUUGGUGA | 1362 | 915-935 | UCACCAAGGUAG AUCUGUGCUUA | 1687 | 913-935 |
| AD-1784352.1 | GCCGUUCUAGGU AUUUUUUUA | 1363 | 496-516 | UAAAAAAAUACC UAGAACGGCCA | 1688 | 494-516 |
| AD-1784353.1 | CUAAAAUGCUGC UUUUAAAAA | 1364 | 1175-1195 | UUUUUAAAAGCA GCAUUUUAGUC | 1689 | 1173-1195 |
| AD-1784354.1 | GAACAGGCAAAU CAAAGCUUA | 1365 | 828-848 | UAAGCUTUGAUU UGCCUGUUCUU | 1690 | 826-848 |
| AD-1784355.1 | UGCUUUGUUUAU GGUAGUAGA | 1366 | 1060-1080 | UCUACUACCAUA AACAAAGCACC | 1691 | 1058-1080 |
| AD-1784356.1 | AAUUAGAGUUGU GAUACAGAA | 1367 | 1461-1481 | UUCUGUAUCACA ACUCUAAUUAU | 1692 | 1459-1481 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784357.1 | CUGGUUGGUGCU UUGUUUAUA | 1368 | 1052-1072 | UAUAAACAAAGC ACCAACCAGCC | 1693 | 1050-1072 |
| AD-1784358.1 | UCCUUCAAAUAA GAUGGUCCA | 1369 | 847-867 | UGGACCAUCUUA UUUGAAGGAAG | 1694 | 845-867 |
| AD-1784359.1 | GCCUCCUUCCUG AAUCCUUGA | 1370 | 620-640 | UCAAGGAUUCAG GAAGGAGGCCA | 1695 | 618-640 |
| AD-1784360.1 | GAUUCUAUGUAA UGUAAACCA | 1371 | 1278-1298 | UGGUTUACAUUA CAUAGAAUCAU | 1696 | 1276-1298 |
| AD-1784361.1 | UGGUUGGUGCUU UGUUUAUGA | 1372 | 1053-1073 | UCAUAAACAAAG CACCAACCAGC | 1697 | 1051-1073 |
| AD-1784362.1 | CUCAUACAGCCA AGUAUGACA | 1373 | 179-199 | UGUCAUACUUGG CUGUAUGAGUG | 1698 | 177-199 |
| AD-1784363.1 | CUCAUACAGCCA AGUAUGACA | 1373 | 179-199 | UGUCAUACUUGG CUGUAUGAGUG | 1698 | 177-199 |
| AD-1784364.1 | AUCGACACUCAU ACAGCCAAA | 1374 | 172-192 | UUUGGCTGUAUG AGUGUCGAUGU | 1699 | 170-192 |
| AD-1784365.1 | GCACUGGCAUAA GGACUUCCA | 1375 | 117-137 | UGGAAGTCCUUA UGCCAGUGCUC | 1700 | 115-137 |
| AD-1784366.1 | AACGUGGAGUUU GAUGACUCA | 1376 | 274-294 | UGAGTCAUCAAA CUCCACGUUGA | 1701 | 272-294 |
| AD-1784367.1 | GCAAAUCAGGUA AAAUAGUCA | 1377 | 1256-1276 | UGACUAUUUUAC CUGAUUUGCCU | 1702 | 1254-1276 |
| AD-1784368.1 | GCAAAUCAGGUA AAAUAGUCA | 1377 | 1256-1276 | UGACTAUUUUAC CUGAUUUGCCU | 1703 | 1254-1276 |
| AD-1784369.1 | CUUCAGAAAGUU GUUGAUGUA | 1378 | 541-561 | UACAUCAACAAC UUUCUGAAGGC | 1704 | 539-561 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784370.1 | CUUCAGAAAGUU GUUGAUGUA | 1378 | 541-561 | UACATCAACAAC UUUCUGAAGGC | 1705 | 539-561 |
| AD-1784371.1 | AAAUCAGGUAAA AUAGUCAUA | 1379 | 1258-1278 | UAUGACTAUUUU ACCUGAUUUGC | 1706 | 1256-1278 |
| AD-1784372.1 | AGGCAAAUCAGG UAAAAUAGA | 1380 | 1254-1274 | UCUAUUUUACCU GAUUUGCCUUA | 1707 | 1252-1274 |
| AD-1784373.1 | GGGCAAGAGUGC UGACUUCAA | 1381 | 582-602 | UUGAAGTCAGCA CUCUUGCCCUU | 1708 | 580-602 |
| AD-1784375.1 | GGCCGUUCUAGG UAUUUUUUA | 1382 | 495-515 | UAAAAAAUACCU AGAACGGCCAG | 1709 | 493-515 |
| AD-1784377.1 | CGGGCCUUCAGA AAGUUGUUA | 1383 | 536-556 | UAACAACUUUCU GAAGGCCCGGU | 1710 | 534-556 |
| AD-1784378.1 | GGCAAAUCAGGU AAAAUAGUA | 1384 | 1255-1275 | UACUAUUUUACC UGAUUUGCCUU | 1711 | 1253-1275 |
| AD-1784379.1 | GAGGAUCCUCAA CAAUGGUCA | 1385 | 246-266 | UGACCAUUGUUG AGGAUCCUCAG | 1712 | 244-266 |
| AD-1784380.1 | GAGGAUCCUCAA CAAUGGUCA | 1385 | 246-266 | UGACCATUGUUG AGGAUCCUCAG | 1713 | 244-266 |
| AD-1784381.1 | UUCACUUGGUUC ACUGGAACA | 1386 | 428-448 | UGUUCCAGUGAA CCAAGUGAAGU | 1714 | 426-448 |
| AD-1784382.1 | AGAACUGAUGGU GGACAACUA | 1387 | 786-806 | UAGUTGTCCACC AUCAGUUCUUC | 1715 | 784-806 |
| AD-1784383.1 | AAUAAAGUACCU UGACUUUGA | 1388 | 1116-1136 | UCAAAGUCAAGG UACUUUAUUCU | 1716 | 1114-1136 |
| AD-1784384.1 | AAUAAAGUACCU UGACUUUGA | 1388 | 1116-1136 | UCAAAGTCAAGG UACUUUAUUCU | 1717 | 1114-1136 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784385.1 | CUUUGUUCACAG CAUGUAGGA | 1389 | 1131-1151 | UCCUACAUGCUG UGAACAAAGUC | 1718 | 1129-1151 |
| AD-1784386.1 | CUUUGUUCACAG CAUGUAGGA | 1389 | 1131-1151 | UCCUACAUGCUG UGAACAAAGUC | 1718 | 1129-1151 |
| AD-1784387.1 | AAUAAGAAUAAA GUACCUUGA | 1390 | 1110-1130 | UCAAGGTACUUU AUUCUUAUUUC | 1719 | 1108-1130 |
| AD-1784388.1 | AGUAGUUUUUCU GUAACACAA | 1391 | 1075-1095 | UUGUGUTACAGA AAAACUACUAC | 1720 | 1073-1095 |
| AD-1784389.1 | CCAAGUAUGACC CUUCCCUGA | 1392 | 188-208 | UCAGGGAAGGGU CAUACUUGGCU | 1721 | 186-208 |
| AD-1784390.1 | UUGAGUGCAAAU CCAUAGCAA | 1393 | 1213-1233 | UUGCTATGGAUU UGCACUCAACC | 1722 | 1211-1233 |
| AD-1784391.1 | GGCCUUCAGAAA GUUGUUGAA | 1394 | 538-558 | UUCAACAACUUU CUGAAGGCCCG | 1723 | 536-558 |
| AD-1784392.1 | AGGAUCCUCAAC AAUGGUCAA | 1395 | 247-267 | UUGACCAUUGUU GAGGAUCCUCA | 1724 | 245-267 |
| AD-1784393.1 | AUUAGAGUUGUG AUACAGAGA | 1396 | 1462-1482 | UCUCTGTAUCAC AACUCUAAUUA | 1725 | 1460-1482 |
| AD-1784394.1 | CAACGUGGAGUU UGAUGACUA | 1397 | 273-293 | UAGUCATCAAAC UCCACGUUGAA | 1726 | 271-293 |
| AD-1784395.1 | GACUUUUGAAUU ACAGAGAUA | 1398 | 1409-1429 | UAUCTCTGUAAU UCAAAAGUCAU | 1727 | 1407-1429 |
| AD-1784396.1 | UGAGGAUCCUCA ACAAUGGUA | 1399 | 245-265 | UACCAUUGUUGA GGAUCCUCAGG | 1728 | 243-265 |
| AD-1784397.1 | GAGUUUGAUGAC UCUCAGGAA | 1400 | 280-300 | UUCCTGAGAGUC AUCAAACUCCA | 1729 | 278-300 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784398.1 | UUUUAAAACAUA GGAAAGUAA | 1401 | 1187-1207 | UUACTUTCCUAU GUUUUAAAAGC | 1730 | 1185-1207 |
| AD-1784399.1 | UUAUGGUAGUAG UUUUUCUGA | 1402 | 1068-1088 | UCAGAAAAACUA CUACCAUAAAC | 1731 | 1066-1088 |
| AD-1784400.1 | AACUUCACUUGG UUCACUGGA | 1403 | 425-445 | UCCAGUGAACCA AGUGAAGUUCU | 1732 | 423-445 |
| AD-1784401.1 | AAAUUGAGCUAG UUAAGGCAA | 1404 | 1239-1259 | UUGCCUTAACUA GCUCAAUUUAU | 1733 | 1237-1259 |
| AD-1784402.1 | UUUGUUUAUGGU AGUAGUUUA | 1405 | 1063-1083 | UAAACUACUACC AUAAACAAAGC | 1734 | 1061-1083 |
| AD-1784403.1 | UUUGUUUAUGGU AGUAGUUUA | 1405 | 1063-1083 | UAAACUACUACC AUAAACAAAGC | 1734 | 1061-1083 |
| AD-1784404.1 | AAGGGCAAGAGU GCUGACUUA | 1406 | 580-600 | UAAGTCAGCACU CUUGCCCUUUG | 1735 | 578-600 |
| AD-1784405.1 | GGAGUUUGAUGA CUCUCAGGA | 1407 | 279-299 | UCCUGAGAGUCA UCAAACUCCAC | 1736 | 277-299 |
| AD-1784406.1 | GGGCCUUCAGAA AGUUGUUGA | 1408 | 537-557 | UCAACAACUUUC UGAAGGCCCGG | 1737 | 535-557 |
| AD-1784407.1 | GCAAGAGUGCUG ACUUCACUA | 1409 | 584-604 | UAGUGAAGUCAG CACUCUUGCCC | 1738 | 582-604 |
| AD-1784408.1 | AGCCACUGAAGA ACAGGCAAA | 1410 | 818-838 | UUUGCCTGUUCU UCAGUGGCUGA | 1739 | 816-838 |
| AD-1784409.1 | AUUCCAUUAAAA CAAAGGGCA | 1411 | 566-586 | UGCCCUUUGUUU UAAUGGAAUCC | 1740 | 564-586 |
| AD-1784410.1 | AUUCCAUUAAAA CAAAGGGCA | 1411 | 566-586 | UGCCCUTUGUUU UAAUGGAAUCC | 1741 | 564-586 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784411.1 | GUAGUUUUUCUG UAACACAGA | 1412 | 1076-1096 | UCUGTGTUACAG AAAAACUACUA | 1742 | 1074-1096 |
| AD-1784412.1 | GGUAUUUUUUUG AAGGUUGGA | 1413 | 505-525 | UCCAACCUUCAA AAAAAUACCUA | 1743 | 503-525 |
| AD-1784413.1 | UCCAAAUAAUGA AUCUUCGGA | 1414 | 878-898 | UCCGAAGAUUCA UUAUUUGGAUA | 1744 | 876-898 |
| AD-1784414.1 | AAACAUAGGAAA GUAGAAUGA | 1415 | 1192-1212 | UCAUTCTACUUU CCUAUGUUUUA | 1745 | 1190-1212 |
| AD-1784415.1 | AUGACUCUCAGG ACAAAGCAA | 1416 | 287-307 | UUGCTUTGUCCU GAGAGUCAUCA | 1746 | 285-307 |
| AD-1784416.1 | AGCUAGUUAAGG CAAAUCAGA | 1417 | 1245-1265 | UCUGAUUUGCCU UAACUAGCUCA | 1747 | 1243-1265 |
| AD-1784417.1 | CCUGAGGAUCCU CAACAAUGA | 1418 | 243-263 | UCAUTGTUGAGG AUCCUCAGGGA | 1748 | 241-263 |
| AD-1784418.1 | GGUUGGUGCUUU GUUUAUGGA | 1419 | 1054-1074 | UCCAUAAACAAA GCACCAACCAG | 1749 | 1052-1074 |
| AD-1784419.1 | AGGUAUUUUUUU GAAGGUUGA | 1420 | 504-524 | UCAACCUUCAAA AAAAUACCUAG | 1750 | 502-524 |
| AD-1784420.1 | UGAAUCUUCGGG UGUUUCCCA | 1421 | 887-907 | UGGGAAACACCC GAAGAUUCAUU | 1751 | 885-907 |
| AD-1784421.1 | UGAAUCUUCGGG UGUUUCCCA | 1421 | 887-907 | UGGGAAACACCC GAAGAUUCAUU | 1751 | 885-907 |
| AD-1784422.1 | UAGUAGUUUUUC UGUAACACA | 1422 | 1074-1094 | UGUGUUACAGAA AAACUACUACC | 1752 | 1072-1094 |
| AD-1784423.1 | UAGUAGUUUUUC UGUAACACA | 1422 | 1074-1094 | UGUGTUACAGAA AAACUACUACC | 1753 | 1072-1094 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784424.1 | GUUUGAUGACUC UCAGGACAA | 1423 | 282-302 | UUGUCCTGAGAG UCAUCAAACUC | 1754 | 280-302 |
| AD-1784425.1 | AAUGAAUCUUCG GGUGUUUCA | 1424 | 885-905 | UGAAACACCCGA AGAUUCAUUAU | 1755 | 883-905 |
| AD-1784426.1 | CAAAUAAUGAAU CUUCGGGUA | 1425 | 880-900 | UACCCGAAGAUU CAUUAUUUGGA | 1756 | 878-900 |
| AD-1784427.1 | UUUGUUCACAGC AUGUAGGGA | 1426 | 1132-1152 | UCCCUACAUGCU GUGAACAAAGU | 1757 | 1130-1152 |
| AD-1784428.1 | AUUGUGCUCAAG GAACCCAUA | 1427 | 700-720 | UAUGGGTUCCUU GAGCACAAUCC | 1758 | 698-720 |
| AD-1784429.1 | GUUGGUGCUUUG UUUAUGGUA | 1428 | 1055-1075 | UACCAUAAACAA AGCACCAACCA | 1759 | 1053-1075 |
| AD-1784430.1 | GAAUCUUCGGGU GUUUCCCUA | 1429 | 888-908 | UAGGGAAACACC CGAAGAUUCAU | 1760 | 886-908 |
| AD-1784431.1 | GGUAGUAGUUUU UCUGUAACA | 1430 | 1072-1092 | UGUUACAGAAAA ACUACUACCAU | 1761 | 1070-1092 |
| AD-1784432.1 | AGAACAGGCAAA UCAAAGCUA | 1431 | 827-847 | UAGCUUUGAUUU GCCUGUUCUUC | 1762 | 825-847 |
| AD-1784433.1 | AGAACAGGCAAA UCAAAGCUA | 1431 | 827-847 | UAGCTUTGAUUU GCCUGUUCUUC | 1763 | 825-847 |
| AD-1784434.1 | AAAAUAGUCAUG AUUCUAUGA | 1432 | 1267-1287 | UCAUAGAAUCAU GACUAUUUUAC | 1764 | 1265-1287 |
| AD-1784435.1 | ACUGGCCGUUCU AGGUAUUUA | 1433 | 492-512 | UAAAUACCUAGA ACGGCCAGUCC | 1765 | 490-512 |
| AD-1784436.1 | CCCUGAGGAUCC UCAACAAUA | 1434 | 242-262 | UAUUGUTGAGGA UCCUCAGGGAA | 1766 | 240-262 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784437.1 | GCUGGUUGGUGC UUUGUUUAA | 1435 | 1051-1071 | UUAAACAAAGCA CCAACCAGCCA | 1767 | 1049-1071 |
| AD-1784438.1 | CAGAAAGUUGUU GAUGUGCUA | 1436 | 544-564 | UAGCACAUCAAC AACUUUCUGAA | 1768 | 542-564 |
| AD-1784439.1 | ACUAACUUCGAU CCUCGUGGA | 1437 | 601-621 | UCCACGAGGAUC GAAGUUAGUGA | 1769 | 599-621 |
| AD-1784440.1 | CCUUCAGAAAGU UGUUGAUGA | 1438 | 540-560 | UCAUCAACAACU UUCUGAAGGCC | 1770 | 538-560 |
| AD-1784441.1 | UGAGCACUGGCA UAAGGACUA | 1439 | 114-134 | UAGUCCUUAUGC CAGUGCUCAGG | 1771 | 112-134 |
| AD-1784442.1 | CUAGUUAAGGCA AAUCAGGUA | 1440 | 1247-1267 | UACCUGAUUUGC CUUAACUAGCU | 1772 | 1245-1267 |
| AD-1784443.1 | CUGAGGAUCCUC AACAAUGGA | 1441 | 244-264 | UCCAUUGUUGAG GAUCCUCAGGG | 1773 | 242-264 |
| AD-1784444.1 | GUUUAUGGUAGU AGUUUUUCA | 1442 | 1066-1086 | UGAAAAACUACU ACCAUAAACAA | 1774 | 1064-1086 |
| AD-1784445.1 | UGUGACCUGGAU UGUGCUCAA | 1443 | 690-710 | UUGAGCACAAUC CAGGUCACACA | 1775 | 688-710 |
| AD-1784446.1 | ACAUCGACACUC AUACAGCCA | 1444 | 170-190 | UGGCTGTAUGAG UGUCGAUGUCA | 1776 | 168-190 |
| AD-1784447.1 | GAUUGUGCUCAA GGAACCCAA | 1445 | 699-719 | UUGGGUTCCUUG AGCACAAUCCA | 1777 | 697-719 |
| AD-1784448.1 | UCAUACAGCCAA GUAUGACCA | 1446 | 180-200 | UGGUCATACUUG GCUGUAUGAGU | 1778 | 178-200 |
| AD-1784449.1 | UAAAAUAGUCAU GAUUCUAUA | 1447 | 1266-1286 | UAUAGAAUCAUG ACUAUUUUACC | 1779 | 1264-1286 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784450.1 | UAAAAUAGUCAUGAUUCUAUA | 1447 | 1266-1286 | UAUAGAAUCAUGACUAUUUUACC | 1779 | 1264-1286 |
| AD-1784451.1 | GUGCUCAAGGAACCCAUCAGA | 1448 | 703-723 | UCUGAUGGGUUCCUUGAGCACAA | 1780 | 701-723 |
| AD-1784452.1 | CGAAGAACUGAUGGUGGACAA | 1449 | 783-803 | UUGUCCACCAUCAGUUCUUCGGG | 1781 | 781-803 |
| AD-1784453.1 | AAUAAAAUGUGAAGACUAGAA | 1450 | 1001-1021 | UUCUAGTCUUCACAUUUUAUUAG | 1782 | 999-1021 |
| AD-1784454.1 | GGCUGGUUGGUGCUUUGUUUA | 1451 | 1050-1070 | UAAACAAAGCACCAACCAGCCAC | 1783 | 1048-1070 |
| AD-1784455.1 | UUGUUCACAGCAUGUAGGGUA | 1452 | 1133-1153 | UACCCUACAUGCUGUGAACAAAG | 1784 | 1131-1153 |
| AD-1784456.1 | UUCAAAUAAGAUGGUCCCAUA | 1453 | 850-870 | UAUGGGACCAUCUUAUUUGAAGG | 1785 | 848-870 |
| AD-1784457.1 | UUUAAAACAUAGGAAAGUAGA | 1454 | 1188-1208 | UCUACUUUCCUAUGUUUUAAAAG | 1786 | 1186-1208 |
| AD-1784458.1 | UUGUUUAUGGUAGUAGUUUUA | 1455 | 1064-1084 | UAAAACUACUACCAUAAACAAAG | 1787 | 1062-1084 |
| AD-1784459.1 | UUGUUUAUGGUAGUAGUUUUA | 1455 | 1064-1084 | UAAAACTACUACCAUAAACAAAG | 1788 | 1062-1084 |
| AD-1784460.1 | GCUAGUUAAGGCAAAUCAGGA | 1456 | 1246-1266 | UCCUGAUUUGCCUUAACUAGCUC | 1789 | 1244-1266 |
| AD-1784461.1 | GUAAAAUAGUCAUGAUUCUAA | 1457 | 1265-1285 | UUAGAAUCAUGACUAUUUUACCU | 1790 | 1263-1285 |
| AD-1784462.1 | GUAAAAUAGUCAUGAUUCUAA | 1457 | 1265-1285 | UUAGAATCAUGACUAUUUUACCU | 1791 | 1263-1285 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784463.1 | GACUGGCCGUUC UAGGUAUUA | 1458 | 491-511 | UAAUACCUAGAA CGGCCAGUCCA | 1792 | 489-511 |
| AD-1784464.1 | UCAGCCACUGAA GAACAGGCA | 1459 | 816-836 | UGCCTGTUCUUC AGUGGCUGAGC | 1793 | 814-836 |
| AD-1784465.1 | UGGAAUGUGUGA CCUGGAUUA | 1460 | 683-703 | UAAUCCAGGUCA CACAUUCCAGA | 1794 | 681-703 |
| AD-1784466.1 | UCACAGCAUGUA GGGUGAUGA | 1461 | 1137-1157 | UCAUCACCCUAC AUGCUGUGAAC | 1795 | 1135-1157 |
| AD-1784467.1 | UACAGCCAAGUA UGACCCUUA | 1462 | 183-203 | UAAGGGTCAUAC UUGGCUGUAUG | 1796 | 181-203 |
| AD-1784468.1 | CUGAGCACUGGC AUAAGGACA | 1463 | 113-133 | UGUCCUUAUGCC AGUGCUCAGGU | 1797 | 111-133 |
| AD-1784469.1 | CUGAGCACUGGC AUAAGGACA | 1463 | 113-133 | UGUCCUTAUGCC AGUGCUCAGGU | 1798 | 111-133 |
| AD-1784470.1 | GCUCAAGGAACC CAUCAGCGA | 1464 | 705-725 | UCGCTGAUGGGU UCCUUGAGCAC | 1799 | 703-725 |
| AD-1784471.1 | UUCUGGAAUGUG UGACCUGGA | 1465 | 680-700 | UCCAGGTCACAC AUUCCAGAAGA | 1800 | 678-700 |
| AD-1784472.1 | UAAAUUGAGCUA GUUAAGGCA | 1466 | 1238-1258 | UGCCUUAACUAG CUCAAUUUAUC | 1801 | 1236-1258 |
| AD-1784473.1 | UAUUUUUUUGAA GGUUGGCAA | 1467 | 507-527 | UUGCCAACCUUC AAAAAAAUACC | 1802 | 505-527 |
| AD-1784474.1 | UAAUUAGAGUUG UGAUACAGA | 1468 | 1460-1480 | UCUGUAUCACAA CUCUAAUUAUA | 1803 | 1458-1480 |
| AD-1784475.1 | UAAUUAGAGUUG UGAUACAGA | 1468 | 1460-1480 | UCUGTATCACAA CUCUAAUUAUA | 1804 | 1458-1480 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784476.1 | UGACUCUCAGGA CAAAGCAGA | 1469 | 288-308 | UCUGCUUUGUCC UGAGAGUCAUC | 1805 | 286-308 |
| AD-1784477.1 | UGACUCUCAGGA CAAAGCAGA | 1469 | 288-308 | UCUGCUTUGUCC UGAGAGUCAUC | 1806 | 286-308 |
| AD-1784478.1 | CAUACAGCCAAG UAUGACCCA | 1470 | 181-201 | UGGGTCAUACUU GGCUGUAUGAG | 1807 | 179-201 |
| AD-1784479.1 | GUAUUUUUUUGA AGGUUGGCA | 1471 | 506-526 | UGCCAACCUUCA AAAAAAUACCU | 1808 | 504-526 |
| AD-1784480.1 | CACAGCAUGUAG GGUGAUGAA | 1472 | 1138-1158 | UUCATCACCCUA CAUGCUGUGAA | 1809 | 1136-1158 |
| AD-1784481.1 | UAUAAUUAGAGU UGUGAUACA | 1473 | 1458-1478 | UGUATCACAACU CUAAUUAUAAC | 1810 | 1456-1478 |
| AD-1784482.1 | GAUUUUGGGAAA GCUGUGCAA | 1474 | 460-480 | UUGCACAGCUUU CCCAAAAUCCC | 1811 | 458-480 |
| AD-1784483.1 | UAAAACAAAGGG CAAGAGUGA | 1475 | 573-593 | UCACTCTUGCCC UUUGUUUUAAU | 1812 | 571-593 |
| AD-1784484.1 | UCAAGGAACCCA UCAGCGUCA | 1476 | 707-727 | UGACGCUGAUGG GUUCCUUGAGC | 1813 | 705-727 |
| AD-1784485.1 | UCAAGGAACCCA UCAGCGUCA | 1476 | 707-727 | UGACGCTGAUGG GUUCCUUGAGC | 1814 | 705-727 |
| AD-1784486.1 | UCAGAAAGUUGU UGAUGUGCA | 1477 | 543-563 | UGCACAUCAACA ACUUUCUGAAG | 1815 | 541-563 |
| AD-1784487.1 | UCAGAAAGUUGU UGAUGUGCA | 1477 | 543-563 | UGCACATCAACA ACUUUCUGAAG | 1816 | 541-563 |
| AD-1784488.1 | CCAAAUAAUGAA UCUUCGGGA | 1478 | 879-899 | UCCCGAAGAUUC AUUAUUUGGAU | 1817 | 877-899 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784489.1 | AGCAUGUAGGGU GAUGAGCAA | 1479 | 1141-1161 | UUGCTCAUCACC CUACAUGCUGU | 1818 | 1139-1161 |
| AD-1784490.1 | GAUAAAUUGAGC UAGUUAAGA | 1480 | 1236-1256 | UCUUAACUAGCU CAAUUUAUCUU | 1819 | 1234-1256 |
| AD-1784491.1 | CUCAGCCACUGA AGAACAGGA | 1481 | 815-835 | UCCUGUUCUUCA GUGGCUGAGCU | 1820 | 813-835 |
| AD-1784492.1 | CUCAGCCACUGA AGAACAGGA | 1481 | 815-835 | UCCUGUTCUUCA GUGGCUGAGCU | 1821 | 813-835 |
| AD-1784493.1 | CAGCAUGUAGGG UGAUGAGCA | 1482 | 1140-1160 | UGCUCATCACCC UACAUGCUGUG | 1822 | 1138-1160 |
| AD-1784494.1 | AUAAUGAAUCUU CGGGUGUUA | 1483 | 883-903 | UAACACCCGAAG AUUCAUUAUUU | 1823 | 881-903 |
| AD-1784495.1 | GGAACCCAUCAG CGUCAGCAA | 1484 | 711-731 | UUGCTGACGCUG AUGGGUUCCUU | 1824 | 709-731 |
| AD-1784496.1 | UGUUCACAGCAU GUAGGGUGA | 1485 | 1134-1154 | UCACCCUACAUG CUGUGAACAAA | 1825 | 1132-1154 |
| AD-1784497.1 | AAACAAAGGGCA AGAGUGCUA | 1486 | 575-595 | UAGCACTCUUGC CCUUUGUUUUA | 1826 | 573-595 |
| AD-1784498.1 | UGUGUGACCUGG AUUGUGCUA | 1487 | 688-708 | UAGCACAAUCCA GGUCACACAUU | 1827 | 686-708 |
| AD-1784499.1 | UGUGUGACCUGG AUUGUGCUA | 1487 | 688-708 | UAGCACAAUCCA GGUCACACAUU | 1827 | 686-708 |
| AD-1784500.1 | AAUAAUGAAUCU UCGGGUGUA | 1488 | 882-902 | UACACCCGAAGA UUCAUUAUUUG | 1828 | 880-902 |
| AD-1784501.1 | AUUUUUUUGAAG GUUGGCAGA | 1489 | 508-528 | UCUGCCAACCUU CAAAAAAAUAC | 1829 | 506-528 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784502.1 | AUUUUUUUGAAG GUUGGCAGA | 1489 | 508-528 | UCUGCCAACCUU CAAAAAAAUAC | 1829 | 506-528 |
| AD-1784503.1 | UGCUCAAGGAAC CCAUCAGCA | 1490 | 704-724 | UGCUGATGGGUU CCUUGAGCACA | 1830 | 702-724 |
| AD-1784504.1 | UAAUGAAUCUUC GGGUGUUUA | 1491 | 884-904 | UAAACACCCGAA GAUUCAUUAUU | 1831 | 882-904 |
| AD-1784505.1 | GUGGCUGGUUGG UGCUUUGUA | 1492 | 1048-1068 | UACAAAGCACCA ACCAGCCACAG | 1832 | 1046-1068 |
| AD-1784506.1 | AAGGAACCCAUC AGCGUCAGA | 1493 | 709-729 | UCUGACGCUGAU GGGUUCCUUGA | 1833 | 707-729 |
| AD-1784507.1 | UUAAAACAAAGG GCAAGAGUA | 1494 | 572-592 | UACUCUTGCCCU UUGUUUUAAUG | 1834 | 570-592 |
| AD-1784508.1 | CUGUGGCUGGUU GGUGCUUUA | 1495 | 1046-1066 | UAAAGCACCAAC CAGCCACAGCA | 1835 | 1044-1066 |
| AD-1784509.1 | CAGCUCAGCCAC UGAAGAACA | 1496 | 812-832 | UGUUCUTCAGUG GCUGAGCUGGG | 1836 | 810-832 |
| AD-1784510.1 | AAAUAAUGAAUC UUCGGGUGA | 1497 | 881-901 | UCACCCGAAGAU UCAUUAUUUGG | 1837 | 879-901 |
| AD-1784511.1 | GAAUGUGUGACC UGGAUUGUA | 1498 | 685-705 | UACAAUCCAGGU CACACAUUCCA | 1838 | 683-705 |
| AD-1784512.1 | CAUGUAGGGUGA UGAGCACUA | 1499 | 1143-1163 | UAGUGCTCAUCA CCCUACAUGCU | 1839 | 1141-1163 |
| AD-1784513.1 | GGACUGGCCGUU CUAGGUAUA | 1500 | 490-510 | UAUACCTAGAAC GGCCAGUCCAU | 1840 | 488-510 |
| AD-1784514.1 | AUAAAUUGAGCU AGUUAAGGA | 1501 | 1237-1257 | UCCUUAACUAGC UCAAUUUAUCU | 1841 | 1235-1257 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784515.1 | GAAAGUUGUUGA UGUGCUGGA | 1502 | 546-566 | UCCAGCACAUCA ACAACUUUCUG | 1842 | 544-566 |
| AD-1784516.1 | GAAAGUUGUUGA UGUGCUGGA | 1502 | 546-566 | UCCAGCACAUCA ACAACUUUCUG | 1842 | 544-566 |
| AD-1784517.1 | AACAAAGGGCAA GAGUGCUGA | 1503 | 576-596 | UCAGCACUCUUG CCCUUUGUUUU | 1843 | 574-596 |
| AD-1784518.1 | CCUGAGCACUGG CAUAAGGAA | 1504 | 112-132 | UUCCTUAUGCCA GUGCUCAGGUC | 1844 | 110-132 |
| AD-1784519.1 | UGAUGGUGGACA ACUGGCGCA | 1505 | 791-811 | UGCGCCAGUUGU CCACCAUCAGU | 1845 | 789-811 |
| AD-1784520.1 | ACAAAGGGCAAG AGUGCUGAA | 1506 | 577-597 | UUCAGCACUCUU GCCCUUUGUUU | 1846 | 575-597 |
| AD-1784521.1 | ACGGACCUGAGC ACUGGCAUA | 1507 | 107-127 | UAUGCCAGUGCU CAGGUCCGUUG | 1847 | 105-127 |
| AD-1784522.1 | UGGGAAAGCUGU GCAGCAACA | 1508 | 465-485 | UGUUGCTGCACA GCUUUCCCAAA | 1848 | 463-485 |
| AD-1784523.1 | AACCCAUCAGCG UCAGCAGCA | 1509 | 713-733 | UGCUGCTGACGC UGAUGGGUUCC | 1849 | 711-733 |
| AD-1784524.1 | AGCUCAGCCACU GAAGAACAA | 1510 | 813-833 | UUGUTCTUCAGU GGCUGAGCUGG | 1850 | 811-833 |
| AD-1784525.1 | UUUUGAAGGUUG GCAGCGCUA | 1511 | 512-532 | UAGCGCTGCCAA CCUUCAAAAAA | 1851 | 510-532 |
| AD-1784526.1 | GUAUGACCCUUC CCUGAAGCA | 1512 | 192-212 | UGCUTCAGGGAA GGGUCAUACUU | 1852 | 190-212 |
| AD-1784527.1 | CUACCCAGGCUC ACUGACCAA | 1513 | 651-671 | UUGGTCAGUGAG CCUGGGUAGGU | 1853 | 649-671 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784528.1 | ACCCAGGCUCAC UGACCACCA | 1514 | 653-673 | UGGUGGTCAGUG AGCCUGGGUAG | 1854 | 651-673 |
| AD-1784529.1 | AUGGACUGGCCG UUCUAGGUA | 1515 | 488-508 | UACCUAGAACGG CCAGUCCAUCA | 1855 | 486-508 |
| AD-1784530.1 | ACCUGAGCACUG GCAUAAGGA | 1516 | 111-131 | UCCUUAUGCCAG UGCUCAGGUCC | 1856 | 109-131 |
| AD-1784531.1 | CCAUCAGCGUCA GCAGCGAGA | 1517 | 716-736 | UCUCGCUGCUGA CGCUGAUGGGU | 1857 | 714-736 |
| AD-1784532.1 | UGGACUGGCCGU UCUAGGUAA | 1518 | 489-509 | UUACCUAGAACG GCCAGUCCAUC | 1858 | 487-509 |
| AD-1784533.1 | UUUUGGGAAAGC UGUGCAGCA | 1519 | 462-482 | UGCUGCACAGCU UUCCCAAAAUC | 1859 | 460-482 |
| AD-1784534.1 | CUGAUGGACUGG CCGUUCUAA | 1520 | 485-505 | UUAGAACGGCCA GUCCAUCAGGU | 1860 | 483-505 |
| AD-1784535.1 | ACCUACCCAGGC UCACUGACA | 1521 | 649-669 | UGUCAGTGAGCC UGGGUAGGUCC | 1861 | 647-669 |
| AD-1784536.1 | GGACCUACCCAG GCUCACUGA | 1522 | 647-667 | UCAGTGAGCCUG GGUAGGUCCAG | 1862 | 645-667 |
| AD-1784537.1 | GAUGGACUGGCC GUUCUAGGA | 1523 | 487-507 | UCCUAGAACGGC CAGUCCAUCAG | 1863 | 485-507 |
| AD-1784538.1 | AAGGUUGGCAGC GCUAAACCA | 1524 | 517-537 | UGGUTUAGCGCU GCCAACCUUCA | 1864 | 515-537 |
| AD-1784539.1 | UGAUGGACUGGC CGUUCUAGA | 1525 | 486-506 | UCUAGAACGGCC AGUCCAUCAGG | 1865 | 484-506 |
| AD-1784540.1 | AACUGAUGGUGG ACAACUGGA | 1526 | 788-808 | UCCAGUUGUCCA CCAUCAGUUCU | 1866 | 786-808 |

(continued)

| Duplex Name | Sense Sequence 5' to 3' | SEQ ID NO: | Range in NM_000067.3 | Antisense Sequence 5' to 3' | SEQ ID NO: | Range in NM_000067.3 |
|---|---|---|---|---|---|---|
| AD-1784541.1 | AACUGAUGGUGG ACAACUGGA | 1526 | 788-808 | UCCAGUTGUCCA CCAUCAGUUCU | 1867 | 786-808 |
| AD-1784542.1 | ACAACUGCUGUG GCUGGUUGA | 1527 | 1039-1059 | UCAACCAGCCAC AGCAGUUGUGU | 1868 | 1037-1059 |
| AD-1784543.1 | ACAACUGCUGUG GCUGGUUGA | 1527 | 1039-1059 | UCAACCAGCCAC AGCAGUUGUGU | 1868 | 1037-1059 |
| AD-1784544.1 | CAACGGACCUGA GCACUGGCA | 1528 | 105-125 | UGCCAGTGCUCA GGUCCGUUGUG | 1869 | 103-125 |
| AD-1784545.1 | CAACUGCUGUGG CUGGUUGGA | 1529 | 1040-1060 | UCCAACCAGCCA CAGCAGUUGUG | 1870 | 1038-1060 |
| AD-1784546.1 | ACUGAUGGUGGA CAACUGGCA | 1530 | 789-809 | UGCCAGTUGUCC ACCAUCAGUUC | 1871 | 787-809 |
| AD-1784547.1 | CUGCUGUGGCUG GUUGGUGCA | 1531 | 1043-1063 | UGCACCAACCAG CCACAGCAGUU | 1872 | 1041-1063 |

Table 8. Modified Sense and Antisense Strand Sequences of CA2 dsRNA Agents

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784188.1 | usgsuuucCfuAfUf Gfaucaagcasasa | 1873 | VPusUfsugdCu(Tgn)ga ucauAfgGfaaacasgsa | 2183 | UCUGUUUCCUAU GAUCAAGCAAC | 2541 |
| AD-1784189.1 | usgsacuuCfaCfUf Afacuucgauscsa | 1874 | VPusGfsaucGfaaguuag UfgAfagucasgsc | 2184 | GCUGACUUCACU AACUUCGAUCC | 2542 |
| AD-1784190.1 | csasaagcUfuCfCf Ufucaaauaasgsa | 1875 | VPusCfsuuaUfuugaagg AfaGfcuuugsasu | 2185 | AUCAAAGCUUCC UUCAAAUAAGA | 2543 |
| AD-1784191.1 | uscsaaagCfuUfCf Cfuucaaauasasa | 1876 | VPusUfsuadTu(Tgn)ga aggaAfgCfuuugasusu | 2186 | AAUCAAAGCUUC CUUCAAAUAAG | 2544 |
| AD-1784192.1 | gsuscuguAfuCfCf Afaauaaugasasa | 1877 | VPusUfsucaUfuauuugg AfuAfcagacsusa | 2187 | UAGUCUGUAUCC AAAUAAUGAAU | 2545 |
| AD-1784193.1 | gsuscuguAfuCfCf Afaauaaugasasa | 1877 | VPusUfsucdAu(Tgn)au uuggAfuAfcagacsusa | 2188 | UAGUCUGUAUCC AAAUAAUGAAU | 2545 |
| AD-1784194.1 | asusuccgUfaAfAf Cfuuaacuucsasa | 1878 | VPusUfsgadAg(Tgn)ua aguuUfaCfggaaususu | 2189 | AAAUUCCGUAAA CUUAACUUCAA | 2546 |
| AD-1784195.1 | uscscuauGfaUfCf Afagcaacuuscsa | 1879 | VPusGfsaadGu(Tgn)gc uugaUfcAfuaggasasa | 2190 | UUUCCUAUGAUC AAGCAACUUCC | 2547 |
| AD-1784196.1 | gsusuuccUfaUfGf Afucaagcaascsa | 1880 | VPusGfsuudGc(Tgn)ug aucaUfaGfgaaacsasg | 2191 | CUGUUUCCUAUG AUCAAGCAACU | 2548 |
| AD-1784197.1 | asusgcugCfuUfUf Ufaaaacauasgsa | 1881 | VPusCfsuadTg(Tgn)uu uaaaAfgCfagcaususu | 2192 | AAAUGCUGCUUU UAAAACAUAGG | 2549 |
| AD-1784198.1 | csasuucaGfaCfAf Afuauaucausasa | 1882 | VPusUfsaudGa(Tgn)au auugUfcUfgaaugsgsa | 2193 | UCCAUUCAGACA AUAUAUCAUAA | 2550 |
| AD-1784199.1 | gsascuucAfcUfAf Afcuucgaucscsa | 1883 | VPusGfsgauCfgaaguua GfuGfaagucsasg | 2194 | CUGACUUCACUA ACUUCGAUCCU | 2551 |
| AD-1784200.1 | cscsauucAfgAfCf Afauauaucasusa | 1884 | VPusAfsugaUfauauugu CfuGfaauggsasa | 2195 | UUCCAUUCAGAC AAUAUAUCAUA | 2552 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784201.1 | uscsuguaUfcCfAf Afauaaugaasusa | 1885 | VPusAfsuudCa(Tgn)ua uuugGfaUfacagascsu | 2196 | AGUCUGUAUCCA AAUAAUGAAUC | 2553 |
| AD-1784202.1 | asasucaaAfgCfUf Ufccuucaaasusa | 1886 | VPusAfsuudTg(Agn)ag gaagCfuUfugauusysg | 2197 | CAAAUCAAAGCU UCCUUCAAAUA | 2554 |
| AD-1784203.1 | asusucagAfcAfAf Ufauaucauasasa | 1887 | VPusUfsuadTg(Agn)ua uauuGfuCfugaausgsg | 2198 | CCAUUCAGACAA UAUAUCAUAAC | 2555 |
| AD-1784204.1 | cscsguaaAfcUfUf Afacuucaausgsa | 1888 | VPusCfsauuGfaaguuaa GfuUfuacggsasa | 2199 | UUCCGUAAACUU AACUUCAAUGG | 2556 |
| AD-1784205.1 | cscsguaaAfcUfUf Afacuucaausgsa | 1888 | VPusCfsaudTg(Agn)ag uuaaGfuUfuacggsasa | 2200 | UUCCGUAAACUU AACUUCAAUGG | 2556 |
| AD-1784206.1 | gsusgcugAfcUfUf Cfacuaacuuscsa | 1890 | VPusGfsaadGu(Tgn)ag ugaaGfuCfagcacsusc | 2201 | GAGUGCUGACUU CACUAACUUCG | 2557 |
| AD-1784207.1 | asasgcuuCfcUfUf Cfaaauaagasusa | 1891 | VPusAfsucuUfauuugaa GfgAfagcuususg | 2202 | CAAAGCUUCCUU CAAAUAAGAUG | 2558 |
| AD-1784208.1 | asasgcuuCfcUfUf Cfaaauaagasusa | 1891 | VPusAfsucdTu(Agn)uu ugaaGfgAfagcuususg | 2203 | CAAAGCUUCCUU CAAAUAAGAUG | 2558 |
| AD-1784209.1 | asasauucCfgUfAf Afacuuaacususa | 1892 | VPusAfsaguUfaaguuua CfgGfaauuuscsa | 2204 | UGAAAUUCCGUA AACUUAACUUC | 2559 |
| AD-1784210.1 | csusgucuGfuUfUf Cfcuaugaucsasa | 1893 | VPusUfsgadTc(Agn)ua ggaaAfcAfgacagsgsg | 2205 | CCCUGUCUGUUU CCUAUGAUCAA | 2560 |
| AD-1784211.1 | gsusauccAfaAfUf Afaugaaucususa | 1894 | VPusAfsagaUfucauuau UfuGfgauacsasg | 2206 | CUGUAUCCAAAU AAUGAAUCUUC | 2561 |
| AD-1784212.1 | gsusauccAfaAfUf Afaugaaucususa | 1894 | VPusAfsagdAu(Tgn)ca uuauUfuGfgauacsasg | 2207 | CUGUAUCCAAAU AAUGAAUCUUC | 2561 |
| AD-1784213.1 | csusgacuUfcAfCf Ufaacuucgasusa | 1895 | VPusAfsucgAfaguuagu GfaAfgucagscsa | 2208 | UGCUGACUUCAC UAACUUCGAUC | 2562 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784214.1 | gscsuuccUfuCfAf Afauaagaugsgsa | 1896 | VPusCfscauCfuuauuug AfaGfgaagcsusu | 2209 | AAGCUUCCUUCA AAUAAGAUGGU | 2563 |
| AD-1784215.1 | gscsuuccUfuCfAf Afauaagaugsgsa | 1896 | VPusCfscadTc(Tgn)ua uuugAfaGfgaagcsusu | 2210 | AAGCUUCCUUCA AAUAAGAUGGU | 2563 |
| AD-1784216.1 | asasaucaAfaGfCf Ufuccuucaasasa | 1897 | VPusUfsuudGa(Agn)g gaagcUfuUfgauuusgsc | 2211 | GCAAAUCAAAGC UUCCUUCAAAU | 2564 |
| AD-1784217.1 | asgscuucCfuUfCf Afaauaagausgsa | 1898 | VPusCfsaucUfuauuuga AfgGfaagcusu | 2212 | AAAGCUUCCUUC AAAUAAGAUGG | 2565 |
| AD-1784218.1 | usgscugcUfuUfUf Afaaacauagsgsa | 1899 | VPusCfscuaUfguuuuaa AfaGfcagcasusu | 2213 | AAUGCUGCUUUU AAAACAUAGGA | 2566 |
| AD-1784219.1 | asgsgcaaAfuCfAf Afagcuuccususa | 1900 | VPusAfsaggAfagcuuug AfuUfugccusgsu | 2214 | ACAGGCAAAUCA AAGCUUCCUUC | 2567 |
| AD-1784220.1 | asgsgcaaAfuCfAf Afagcuuccususa | 1900 | VPusAfsagdGa(Agn)gc uuugAfuUfugccusgsu | 2215 | ACAGGCAAAUCA AAGCUUCCUUC | 2567 |
| AD-1784221.1 | gsgscaaaUfcAfAf Afgcuuccuuscsa | 1901 | VPusGfsaadGg(Agn)ag cuuuGfaUfuugccsusg | 2216 | CAGGCAAAUCAA AGCUUCCUUCA | 2568 |
| AD-1784222.1 | asasagcuUfcCfUf Ufcaaauaagsasa | 1902 | VPusUfscuuAfuuugaag GfaAfgcuuusgsa | 2217 | UCAAAGCUUCCU UCAAAUAAGAU | 2569 |
| AD-1784223.1 | asasagcuUfcCfUf Ufcaaauaagsasa | 1902 | VPusUfscudTa(Tgn)uu gaagGfaAfgcuuusgsa | 2218 | UCAAAGCUUCCU UCAAAUAAGAU | 2569 |
| AD-1784224.1 | usasaaauGfcUfGf Cfuuuuaaaascsa | 1903 | VPusGfsuuuUfaaaagca GfcAfuuuuasgsu | 2219 | ACUAAAAUGCUG CUUUUAAAACA | 2570 |
| AD-1784225.1 | asasgaauAfaAfGf Ufaccuugacsusa | 1904 | VPusAfsgudCa(Agn)g guacuUfuAfuucuusasu | 2220 | AUAAGAAUAAAG UACCUUGACUU | 2571 |
| AD-1784226.1 | asgsaauaAfaGfUf Afccuugacususa | 1905 | VPusAfsaguCfaagguac UfuUfauucususa | 2221 | UAAGAAUAAAGU ACCUUGACUUU | 2572 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784227.1 | asgsaauaAfaGfUf Afccuugacususa | 1905 | VPusAfsagdTc(Agn)ag guacUfuUfauucususa | 2222 | UAAGAAUAAAGU ACCUUGACUUU | 2572 |
| AD-1784228.1 | gsuscuguUfuCfCf Ufaugaucaasgsa | 1906 | VPusCfsuudGa(Tgn)ca uaggAfaAfcagacsasg | 2223 | CUGUCUGUUUCC UAUGAUCAAGC | 2573 |
| AD-1784229.1 | uscscguaAfaCfUf Ufaacuucaasusa | 1907 | VPusAfsuudGa(Agn)g uuaagUfuUfacggasasu | 2224 | AUUCCGUAAACU UAACUUCAAUG | 2574 |
| AD-1784230.1 | cscsucuuCfuGfGf Afaugugugascsa | 1908 | VPusGfsucdAc(Agn)ca uuccAfgAfagaggsasg | 2225 | CUCCUCUUCUGG AAUGUGUGACC | 2575 |
| AD-1784231.1 | usasuccaAfaUfAf Afugaaucuuscsa | 1909 | VPusGfsaadGa(Tgn)uc auuaUfuUfggauascsa | 2226 | UGUAUCCAAAUA AUGAAUCUUCG | 2576 |
| AD-1784232.1 | uscsuguuUfcCfUf Afugaucaagscsa | 1910 | VPusGfscudTg(Agn)uc auagGfaAfacagascsa | 2227 | UGUCUGUUUCCU AUGAUCAAGCA | 2577 |
| AD-1784233.1 | gsusugacAfuCfGf Afcacucauascsa | 1911 | VPusGfsuadTg(Agn)gu gucgAfuGfucaacsasg | 2228 | CUGUUGACAUCG ACACUCAUACA | 2578 |
| AD-1784234.1 | asasguacCfuUfGf Afcuuuguucsasa | 1912 | VPusUfsgaaCfaaaguca AfgGfuacuususa | 2229 | UAAAGUACCUUG ACUUUGUUCAC | 2579 |
| AD-1784235.1 | asasguacCfuUfGf Afcuuuguucsasa | 1912 | VPusUfsgadAc(Agn)aa gucaAfgGfuacuususa | 2230 | UAAAGUACCUUG ACUUUGUUCAC | 2579 |
| AD-1784236.1 | csasgaucUfaCfCf Ufuggugauususa | 1913 | VPusAfsaadTc(Agn)cc aaggUfaGfaucugsusg | 2231 | CACAGAUCUACC UUGGUGAUUUG | 2580 |
| AD-1784237.1 | csusggauUfgUfGf Cfucaaggaascsa | 1914 | VPusGfsuudCc(Tgn)ug agcaCfaAfuccagsgsu | 2232 | ACCUGGAUUGUG CUCAAGGAACC | 2581 |
| AD-1784238.1 | usgscuuuUfaAfAf Afcauaggaasasa | 1915 | VPusUfsuudCc(Tgn)au guuuUfaAfaagcasgsc | 2233 | GCUGCUUUUAAA ACAUAGGAAAG | 2582 |
| AD-1784239.1 | usgscugaCfuUfCf Afcuaacuucsgsa | 1916 | VPusCfsgaaGfuuaguga AfgUfcagcascsu | 2234 | AGUGCUGACUUC ACUAACUUCGA | 2583 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784240.1 | usgscugaCfuUfCf Afcuaacuucsgsa | 1916 | VPusCfsgadAg(Tgn)ua gugaAfgUfcagcascsu | 2235 | AGUGCUGACUUC ACUAACUUCGA | 2583 |
| AD-1784241.1 | gsasaauuCfcGfUf Afaacuuaacsusa | 1917 | VPusAfsguuAfaguuuac GfgAfauuucsasa | 2236 | UUGAAAUUCCGU AAACUUAACUU | 2584 |
| AD-1784242.1 | gsasaauuCfcGfUf Afaacuuaacsusa | 1917 | VPusAfsgudTa(Agn)gu uuacGfgAfauuucsasa | 2237 | UUGAAAUUCCGU AAACUUAACUU | 2584 |
| AD-1784243.1 | usasaggcAfaAfUf Cfagguaaaasusa | 1918 | VPusAfsuuuUfaccugau UfuGfccuuasasc | 2238 | GUUAAGGCAAAU CAGGUAAAAUA | 2585 |
| AD-1784244.1 | usasaggcAfaAfUf Cfagguaaaasusa | 1918 | VPusAfsuudTu(Agn)cc ugauUfuGfccuuasasc | 2239 | GUUAAGGCAAAU CAGGUAAAAUA | 2585 |
| AD-1784245.1 | gsusucuaGfgUfAf Ufuuuuuugasasa | 1919 | VPusUfsucaAfaaaaaua CfcUfagaacsgsg | 2240 | CCGUUCUAGGUA UUUUUUUGAAG | 2586 |
| AD-1784246.1 | asasgauaAfaUfUf Gfagcuaguusasa | 1920 | VPusUfsaadCu(Agn)gc ucaaUfuUfaucuusgsu | 2241 | ACAAGAUAAAUU GAGCUAGUUAA | 2587 |
| AD-1784247.1 | ususagcuAfaGfCf Afcagaucuascsa | 1921 | VPusGfsuadGa(Tgn)cu gugcUfuAfgcuaasasg | 2242 | CUUUAGCUAAGC ACAGAUCUACC | 2588 |
| AD-1784248.1 | csusucacUfaAfCf Ufucgauccuscsa | 1922 | VPusGfsagdGa(Tgn)cg aaguUfaGfugaagsusc | 2243 | GACUUCACUAAC UUCGAUCCUCG | 2589 |
| AD-1784249.1 | asasuuccGfuAfAf Afcuuaacuuscsa | 1923 | VPusGfsaadGu(Tgn)aa guuuAfcGfgaauusus c | 2244 | GAAAUUCCGUAA ACUUAACUUCA | 2590 |
| AD-1784250.1 | csusgcuuUfuAfAf Afacauaggasasa | 1924 | VPusUfsucdCu(Agn)u guuuuAfaAfagcagscsa | 2245 | UGCUGCUUUUAA AACAUAGGAAA | 2591 |
| AD-1784251.1 | csusguugAfcAfUf Cfgacacucasusa | 1925 | VPusAfsugdAg(Tgn)g ucgauGfuCfaacagsgsg | 2246 | CCCUGUUGACAU CGACACUCAUA | 2592 |
| AD-1784252.1 | ususcacuAfaCfUf Ufcgauccucsgsa | 1926 | VPusCfsgadGg(Agn)uc gaagUfuAfgugaasgsu | 2247 | ACUUCACUAACU UCGAUCCUCGU | 2593 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784253.1 | gscsuaagCfaCfAf Gfaucuaccususa | 1927 | VPusAfsagdGu(Agn)g aucugUfgCfuuagcsusa | 2248 | UAGCUAAGCACA GAUCUACCUUG | 2594 |
| AD-1784254.1 | usasaaguAfcCfUf Ufgacuuugususa | 1928 | VPusAfsacaAfagucaag GfuAfcuuuasusu | 2249 | AAUAAAGUACCU UGACUUUGUUC | 2595 |
| AD-1784255.1 | asasaaugCfuGfCf Ufuuuaaaacsasa | 1929 | VPusUfsgudTu(Tgn)aa aagcAfgCfauuuusasg | 2250 | CUAAAAUGCUGC UUUUAAAACAU | 2596 |
| AD-1784256.1 | gscsugcuUfuUfAf Afaacauaggsasa | 1930 | VPusUfsccuAfuguuuua AfaAfgcagcsasu | 2251 | AUGCUGCUUUUA AAACAUAGGAA | 2597 |
| AD-1784257.1 | gscsugcuUfuUfAf Afaacauaggsasa | 1930 | VPusUfsccdTa(Tgn)gu uuuaAfaAfgcagcsasu | 2252 | AUGCUGCUUUUA AAACAUAGGAA | 2597 |
| AD-1784258.1 | uscsaugaUfuCfUf Afuguaaugusasa | 1931 | VPusUfsacaUfuacauag AfaUfcaugascsu | 2253 | AGUCAUGAUUCU AUGUAAUGUAA | 2598 |
| AD-1784259.1 | uscsaugaUfuCfUf Afuguaaugusasa | 1931 | VPusUfsacdAu(Tgn)ac auagAfaUfcaugascsu | 2254 | AGUCAUGAUUCU AUGUAAUGUAA | 2598 |
| AD-1784260.1 | asgsugcuGfaCfUf Ufcacuaacususa | 1932 | VPusAfsaguUfagugaag UfcAfgcacuscsu | 2255 | AGAGUGCUGACU UCACUAACUUC | 2599 |
| AD-1784261.1 | csusaagcAfcAfGf Afucuaccuugsgsa | 1933 | VPusCfsaadGg(Tgn)ag aucuGfuGfcuuagscsu | 2256 | AGCUAAGCACAG AUCUACCUUGG | 2600 |
| AD-1784262.1 | csascuaaCfuUfCf Gfauccucgusgsa | 1934 | VPusCfsacgAfggaucga AfgUfuagugsasa | 2257 | UUCACUAACUUC GAUCCUCGUGG | 2601 |
| AD-1784263.1 | csusgaagAfaCfAf Gfgcaaaucasasa | 1935 | VPusUfsugdAu(Tgn)u gccugUfuCfuucagsusg | 2258 | CACUGAAGAACA GGCAAAUCAAA | 2602 |
| AD-1784264.1 | asasaguaCfcUfUf Gfacuuuguuscsa | 1936 | VPusGfsaacAfaagucaa GfgUfacuuusasu | 2259 | AUAAAGUACCUU GACUUUGUUCA | 2603 |
| AD-1784265.1 | gscsuuugUfuUfAf Ufgguaguagsusa | 1937 | VPusAfscudAc(Tgn)ac cauaAfaCfaaagcsasc | 2260 | GUGCUUUGUUUA UGGUAGUAGUU | 2604 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784266.1 | csasugauUfcUfAf Ufguaauguasasa | 1938 | VPusUfsuadCa(Tgn)ua cauaGfaAfucaugsasc | 2261 | GUCAUGAUUCUA UGUAAUGUAAA | 2605 |
| AD-1784267.1 | csgsuucuAfgGfUf Afuuuuuuugsasa | 1939 | VPusUfscaaAfaaaauac CfuAfgaacgsgsc | 2262 | GCCGUUCUAGGU AUUUUUUUGAA | 2606 |
| AD-1784268.1 | ususcuagGfuAfUf Ufuuuuugaasgsa | 1940 | VPusCfsuucAfaaaaaau AfcCfuagaascsg | 2263 | CGUUCUAGGUAU UUUUUUGAAGG | 2607 |
| AD-1784269.1 | uscscuucCfuGfAf Afuccuuggasusa | 1941 | VPusAfsuccAfaggauuc AfgGfaaggasgsg | 2264 | CCUCCUUCCUGA AUCCUUGGAUU | 2608 |
| AD-1784270.1 | uscscuucCfuGfAf Afuccuuggasusa | 1941 | VPusAfsucdCa(Agn)gg auucAfgGfaaggasgsg | 2265 | CCUCCUUCCUGA AUCCUUGGAUU | 2608 |
| AD-1784271.1 | gsascuaaAfaUfGf Cfugcuuuuasasa | 1942 | VPusUfsuaaAfagcagca UfuUfuagucsasa | 2266 | UUGACUAAAAUG CUGCUUUUAAA | 2609 |
| AD-1784272.1 | gsascuaaAfaUfGf Cfugcuuuuasasa | 1942 | VPusUfsuadAa(Agn)gc agcaUfuUfuagucsasa | 2267 | UUGACUAAAAUG CUGCUUUUAAA | 2609 |
| AD-1784273.1 | asascaggCfaAfAf Ufcaaagcuuscsa | 1943 | VPusGfsaadGc(Tgn)uu gauuUfgCfcuguuscsu | 2268 | AGAACAGGCAAA UCAAAGCUUCC | 2610 |
| AD-1784274.1 | cscsuuccUfgAfAf Ufccuuggaususa | 1944 | VPusAfsaucCfaaggauu CfaGfgaaggsasg | 2269 | CUCCUUCCUGAA UCCUUGGAUUA | 2611 |
| AD-1784275.1 | usgsaugaCfuCfUf Cfaggacaaasgsa | 1945 | VPusCfsuudTg(Tgn)cc ugagAfgUfcaucasasa | 2270 | UUUGAUGACUCU CAGGACAAAGC | 2612 |
| AD-1784276.1 | usgsgaguUfuGfAf Ufgacucucasgsa | 1946 | VPusCfsugdAg(Agn)g ucaucAfaAfcuccascsg | 2271 | CGUGGAGUUUGA UGACUCUCAGG | 2613 |
| AD-1784277.1 | asusccaaAfuAfAf Ufgaaucuucsgsa | 1947 | VPusCfsgaaGfauucauu AfuUfuggausasc | 2272 | GUAUCCAAAUAA UGAAUCUUCGG | 2614 |
| AD-1784278.1 | asusccaaAfuAfAf Ufgaaucuucsgsa | 1947 | VPusCfsgadAg(Agn)u ucauuAfuUfuggausasc | 2273 | GUAUCCAAAUAA UGAAUCUUCGG | 2614 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784279.1 | ususgacuUfuGfUf Ufcacagcausgsa | 1948 | VPusCfsaudGc(Tgn)gu gaacAfaAfgucaasgsg | 2274 | CCUUGACUUUGU UCACAGCAUGU | 2615 |
| AD-1784280.1 | asgsaucuAfcCfUf Ufggugauuusgsa | 1949 | VPusCfsaaaUfcaccaag GfuAfgaucusgsu | 2275 | ACAGAUCUACCU UGGUGAUUUGG | 2616 |
| AD-1784281.1 | asusgguaGfuAfGf Ufuuuucugusasa | 1950 | VPusUfsacaGfaaaaacu AfcUfaccausasa | 2276 | UUAUGGUAGUAG UUUUUCUGUAA | 2617 |
| AD-1784282.1 | asusgguaGfuAfGf Ufuuuucugusasa | 1950 | VPusUfsacdAg(Agn)aa aacuAfcUfaccausasa | 2277 | UUAUGGUAGUAG UUUUUCUGUAA | 2617 |
| AD-1784283.1 | cscsuugaCfuUfUf Gfuucacagcsasa | 1951 | VPusUfsgcdTg(Tgn)ga acaaAfgUfcaaggsusa | 2278 | UACCUUGACUUU GUUCACAGCAU | 2618 |
| AD-1784284.1 | cscsuggaUfuGfUf Gfcucaaggasasa | 1952 | VPusUfsucdCu(Tgn)ga gcacAfaUfccaggsusc | 2279 | GACCUGGAUUGU GCUCAAGGAAC | 2619 |
| AD-1784285.1 | gsasgcuaGfuUfAf Afggcaaaucsasa | 1953 | VPusUfsgadTu(Tgn)gc cuuaAfcUfagcucsasa | 2280 | UUGAGCUAGUUA AGGCAAAUCAG | 2620 |
| AD-1784286.1 | ascsugaaGfaAfCf Afggcaaaucsasa | 1954 | VPusUfsgadTu(Tgn)gc cuguUfcUfucagusgsg | 2281 | CCACUGAAGAAC AGGCAAAUCAA | 2621 |
| AD-1784287.1 | usgsaagaAfcAfGf Gfcaaaucaasasa | 1955 | VPusUfsuudGa(Tgn)uu gccuGfuUfcuucasgsu | 2282 | ACUGAAGAACAG GCAAAUCAAAG | 2622 |
| AD-1784288.1 | csusccucUfuCfUf Gfgaaugugusgsa | 1956 | VPusCfsacaCfauuccag AfaGfaggagsgsg | 2283 | CCCUCCUCUUCU GGAAUGUGUGA | 2623 |
| AD-1784289.1 | csusccucUfuCfUf Gfgaaugugusgsa | 1956 | VPusCfsacdAc(Agn)uu ccagAfaGfaggagsgsg | 2284 | CCCUCCUCUUCU GGAAUGUGUGA | 2623 |
| AD-1784290.1 | gscsuuucAfaCfGf Ufggaguuugsasa | 1957 | VPusUfscaaAfcuccacg UfuGfaaagcsasu | 2285 | AUGCUUUCAACG UGGAGUUUGAU | 2624 |
| AD-1784291.1 | usgscuuuCfaAfCf Gfuggaguuusgsa | 1958 | VPusCfsaaaCfuccacgu UfgAfaagcasusg | 2286 | CAUGCUUUCAAC GUGGAGUUUGA | 2625 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784292.1 | usgscuuuCfaAfCf Gfuggaguuusgsa | 1958 | VPusCfsaadAc(Tgn)cc acguUfgAfaagcasusg | 2287 | CAUGCUUUCAAC GUGGAGUUUGA | 2625 |
| AD-1784293.1 | csasgguaAfaAfUf Afgucaugaususa | 1959 | VPusAfsaudCa(Tgn)ga cuauUfuUfaccugsasu | 2288 | AUCAGGUAAAAU AGUCAUGAUUC | 2626 |
| AD-1784294.1 | csusguauCfcAfAf Afuaaugaauscsa | 1960 | VPusGfsaudTc(Agn)uu auuuGfgAfuacagsasc | 2289 | GUCUGUAUCCAA AUAAUGAAUCU | 2627 |
| AD-1784295.1 | asasggcaAfaUfCf Afgguaaaausasa | 1961 | VPusUfsaudTu(Tgn)ac cugaUfuUfgccuusasa | 2290 | UUAAGGCAAAUC AGGUAAAAUAG | 2628 |
| AD-1784296.1 | cscsuccuUfcCfUf Gfaauccuugsgsa | 1962 | VPusCfscaaGfgauucag GfaAfggaggscsc | 2291 | GGCCUCCUUCCU GAAUCCUUGGA | 2629 |
| AD-1784297.1 | ususccuuCfaAfAf Ufaagaugguscsa | 1963 | VPusGfsacdCa(Tgn)cu uauuUfgAfaggaasgsc | 2292 | GCUUCCUUCAAA UAAGAUGGUCC | 2630 |
| AD-1784298.1 | ususgaaaUfuCfCf Gfuaaacuuasasa | 1964 | VPusUfsuadAg(Tgn)uu acggAfaUfuucaascsa | 2293 | UGUUGAAAUUCC GUAAACUUAAC | 2631 |
| AD-1784299.1 | ascsacucAfuAfCf Afgccaaguasusa | 1965 | VPusAfsuadCu(Tgn)gg cuguAfuGfaguguscsg | 2294 | CGACACUCAUAC AGCCAAGUAUG | 2632 |
| AD-1784300.1 | gscsacagAfuCfUf Afccuuggugsasa | 1966 | VPusUfscacCfaagguag AfuCfugugcsusu | 2295 | AAGCACAGAUCU ACCUUGGUGAU | 2633 |
| AD-1784301.1 | csusuucaAfcGfUf Gfgaguuugasusa | 1967 | VPusAfsucaAfacuccac GfuUfgaaagscsa | 2296 | UGCUUUCAACGU GGAGUUUGAUG | 2634 |
| AD-1784302.1 | usasgcuaAfgCfAf Cfagaucuacscsa | 1968 | VPusGfsgudAg(Agn)u cugugCfuUfagcuasasa | 2297 | UUUAGCUAAGCA CAGAUCUACCU | 2635 |
| AD-1784303.1 | ususgugaUfaCfAf Gfaguauaaususa | 1969 | VPusAfsaauAfuacucug UfaUfcacaascsu | 2298 | AGUUGUGAUACA GAGUAUAUUUC | 2636 |
| AD-1784304.1 | ascsucauAfcAfGf Cfcaaguaugsasa | 1970 | VPusUfscauAfcuuggcu GfuAfugagusgsu | 2299 | ACACUCAUACAG CCAAGUAUGAC | 2637 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784305.1 | asgsuuaaGfgCfAf Afaucagguasasa | 1971 | VPusUfsuadCc(Tgn)ga uuugCfcUfuaacusasg | 2300 | CUAGUUAAGGCA AAUCAGGUAAA | 2638 |
| AD-1784306.1 | gsusugugAfuAfCf Afgaguauaususa | 1972 | VPusAfsauaUfacucugu AfuCfacaacsusc | 2301 | GAGUUGUGAUAC AGAGUAUAUUU | 2639 |
| AD-1784307.1 | gsusugugAfuAfCf Afgaguauaususa | 1972 | VPusAfsaudAu(Agn)c ucuguAfuCfacaacsusc | 2302 | GAGUUGUGAUAC AGAGUAUAUUU | 2639 |
| AD-1784308.1 | usgsacauCfgAfCf Afcucauacasgsa | 1973 | VPusCfsuguAfugagugu CfgAfugucasasc | 2303 | GUUGACAUCGAC ACUCAUACAGC | 2640 |
| AD-1784309.1 | asgsauaaAfuUfGf Afgcuaguuasasa | 1974 | VPusUfsuadAc(Tgn)ag cucaAfuUfuaucususg | 2304 | CAAGAUAAAUUG AGCUAGUUAAG | 2641 |
| AD-1784310.1 | usasgguaUfuUfUf Ufuugaaggususa | 1975 | VPusAfsaccUfucaaaaa AfaUfaccuasgsa | 2305 | UCUAGGUAUUUU UUUGAAGGUUG | 2642 |
| AD-1784311.1 | usasgguaUfuUfUf Ufuugaaggususa | 1975 | VPusAfsacdCu(Tgn)ca aaaaAfaUfaccuasgsa | 2306 | UCUAGGUAUUUU UUUGAAGGUUG | 2642 |
| AD-1784312.1 | usgsgugcUfuUfGf Ufuuaugguasgsa | 1976 | VPusCfsuacCfauaaaca AfaGfcaccasasc | 2307 | GUUGGUGCUUUG UUUAUGGUAGU | 2643 |
| AD-1784313.1 | usgsugauAfcAfGf Afguauauuuscsa | 1977 | VPusGfsaaaUfauacucu GfuAfucacasasc | 2308 | GUUGUGAUACAG AGUAUAUUUCC | 2644 |
| AD-1784314.1 | uscsuucuGfgAfAf Ufgugugaccsusa | 1978 | VPusAfsggdTc(Agn)ca cauuCfcAfgaagasgsg | 2309 | CCUCUUCUGGAA UGUGUGACCUG | 2645 |
| AD-1784315.1 | csusggccGfuUfCf Ufagguauuususa | 1979 | VPusAfsaaaUfaccuaga AfcGfgccagsusc | 2310 | GACUGGCCGUUC UAGGUAUUUUU | 2646 |
| AD-1784316.1 | asuscaggUfaAfAf Afuagucaugsasa | 1980 | VPusUfscadTg(Agn)cu auuuUfaCfcugaususu | 2311 | AAAUCAGGUAAA AUAGUCAUGAU | 2647 |
| AD-1784317.1 | ususccauUfaAfAf Afcaaagggcsasa | 1981 | VPusUfsgcdCc(Tgn)uu guuuUfaAfuggaasusc | 2312 | GAUUCCAUUAAA ACAAAGGGCAA | 2648 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784318.1 | csasagagUfgCfUf Gfacuucacusasa | 1982 | VPusUfsagdTg(Agn)ag ucagCfaCfucuugscsc | 2313 | GGCAAGAGUGCU GACUUCACUAA | 2649 |
| AD-1784319.1 | ususucaaCfgUfGf Gfaguuugausgsa | 1983 | VPusCfsaucAfaacucca CfgUfugaaasgsc | 2314 | GCUUUCAACGUG GAGUUUGAUGA | 2650 |
| AD-1784320.1 | ususggugCfuUfUf Gfuuuauggusasa | 1984 | VPusUfsaccAfuaaacaa AfgCfaccaascsc | 2315 | GGUUGGUGCUUU GUUUAUGGUAG | 2651 |
| AD-1784321.1 | ususggugCfuUfUf Gfuuuauggusasa | 1984 | VPusUfsacdCa(Tgn)aa acaaAfgCfaccaascsc | 2316 | GGUUGGUGCUUU GUUUAUGGUAG | 2651 |
| AD-1784322.1 | csascucaUfaCfAf Gfccaaguausgsa | 1985 | VPusCfsauaCfuuggcug UfaUfgagugsusc | 2317 | GACACUCAUACA GCCAAGUAUGA | 2652 |
| AD-1784323.1 | asusaaagUfaCfCf Ufugacuuugsusa | 1986 | VPusAfscaaAfgucaagg UfaCfuuuaususc | 2318 | GAAUAAAGUACC UUGACUUUGUU | 2653 |
| AD-1784324.1 | asusgacuUfuUfGf Afauuacagasgsa | 1987 | VPusCfsucdTg(Tgn)aa uucaAfaAfgucaususa | 2319 | UAAUGACUUUUG AAUUACAGAGA | 2654 |
| AD-1784325.1 | gsuscaugAfuUfCf Ufauguaaugsusa | 1988 | VPusAfscauUfacauaga AfuCfaugacsusa | 2320 | UAGUCAUGAUUC UAUGUAAUGUA | 2655 |
| AD-1784326.1 | gsasccugGfaUfUf Gfugcucaagsgsa | 1989 | VPusCfscudTg(Agn)gc acaaUfcCfaggucsasc | 2321 | GUGACCUGGAUU GUGCUCAAGGA | 2656 |
| AD-1784327.1 | gsascaucGfaCfAf Cfucauacagscsa | 1990 | VPusGfscudGu(Agn)u gagugUfcGfaugucsasa | 2322 | UUGACAUCGACA CUCAUACAGCC | 2657 |
| AD-1784328.1 | gsasagaaCfaGfGf Cfaaaucaaasgsa | 1991 | VPusCfsuudTg(Agn)uu ugccUfgUfucuucsasg | 2323 | CUGAAGAACAGG CAAAUCAAAGC | 2658 |
| AD-1784329.1 | csgsuaaaCfuUfAf Afcuucaaugsgsa | 1992 | VPusCfscauUfgaaguua AfgUfuuacgsgsa | 2324 | UCCGUAAACUUA ACUUCAAUGGG | 2659 |
| AD-1784330.1 | asgsguaaAfaUfAf Gfucaugauucsa | 1993 | VPusGfsaadTc(Agn)ug acuaUfuUfuaccusgsa | 2325 | UCAGGUAAAAUA GUCAUGAUUCU | 2660 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784331.1 | gsusaccuUfgAfCf Ufuuguucacsasa | 1994 | VPusUfsgudGa(Agn)ca aaguCfaAfgguacsusu | 2326 | AAGUACCUUGAC UUUGUUCACAG | 2661 |
| AD-1784332.1 | cscsguucUfaGfGf Ufauuuuuuusgsa | 1995 | VPusCfsaaaAfaaauacc UfaGfaacggscsc | 2327 | GGCCGUUCUAGG UAUUUUUUUGA | 2662 |
| AD-1784333.1 | ususuaugGfuAfGf Ufaguuuucsusa | 1996 | VPusAfsgaaAfaacuacu AfcCfauaaascsa | 2328 | UGUUUAUGGUAG UAGUUUUUCUG | 2663 |
| AD-1784334.1 | csgsuggaGfuUfUf Gfaugacucuscsa | 1997 | VPusGfsagdAg(Tgn)ca ucaaAfcUfccacgsusu | 2329 | AACGUGGAGUUU GAUGACUCUCA | 2664 |
| AD-1784335.1 | ususcaacGfuGfGf Afguuugaugsasa | 1998 | VPusUfscadTc(Agn)aa cuccAfcGfuugaasasg | 2330 | CUUUCAACGUGG AGUUUGAUGAC | 2665 |
| AD-1784336.1 | gsasguugUfgAfUf Afcagaguausasa | 1999 | VPusUfsaudAc(Tgn)cu guauCfaCfaacucsusa | 2331 | UAGAGUUGUGAU ACAGAGUAUAU | 2666 |
| AD-1784337.1 | usasccuuGfaCfUf Ufuguucacasgsa | 2000 | VPusCfsuguGfaacaaag UfcAfagguascsu | 2332 | AGUACCUUGACU UUGUUCACAGC | 2667 |
| AD-1784338.1 | usasccuuGfaCfUf Ufuguucacasgsa | 2000 | VPusCfsugdTg(Agn)ac aaagUfcAfagguascsu | 2333 | AGUACCUUGACU UUGUUCACAGC | 2667 |
| AD-1784339.1 | usasgaguUfgUfGf Afuacagagusasa | 2001 | VPusUfsacdTc(Tgn)gu aucaCfaAfcucuasasu | 2334 | AUUAGAGUUGUG AUACAGAGUAU | 2668 |
| AD-1784340.1 | usgsagugCfaAfAf Ufccauagcascsa | 2002 | VPusGfsugdCu(Agn)u ggauuUfgCfacucasasc | 2335 | GUUGAGUGCAAA UCCAUAGCACA | 2669 |
| AD-1784341.1 | csasaaucAfgGfUf Afaaauagucsasa | 2003 | VPusUfsgadCu(Agn)u uuuacCfuGfauuugscsc | 2336 | GGCAAAUCAGGU AAAAUAGUCAU | 2670 |
| AD-1784342.1 | asasgcacAfgAfUf Cfuaccuuggsusa | 2004 | VPusAfsccaAfgguagau CfuGfugcuusasg | 2337 | CUAAGCACAGAU CUACCUUGGUG | 2671 |
| AD-1784343.1 | ascsuuugUfuCfAf Cfagcauguasgsa | 2005 | VPusCfsuacAfugcugug AfaCfaaaguscsa | 2338 | UGACUUUGUUCA CAGCAUGUAGG | 2672 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784344.1 | usgsgccgUfuCfUf Afgguauuuususa | 2006 | VPusAfsaaaAfuaccuag AfaCfggccasgsu | 2339 | ACUGGCCGUUCU AGGUAUUUUUU | 2673 |
| AD-1784345.1 | gscscaagUfaUfGf Afcccuucccsusa | 2007 | VPusAfsggdGa(Agn)g ggucaUfaCfuuggcsusg | 2340 | CAGCCAAGUAUG ACCCUUCCCUG | 2674 |
| AD-1784346.1 | usasugguAfgUfAf Gfuuuuucugsusa | 2008 | VPusAfscagAfaaaacua CfuAfccauasasa | 2341 | UUUAUGGUAGUA GUUUUUCUGUA | 2675 |
| AD-1784347.1 | asasuugaGfcUfAf Gfuuaaggcasasa | 2009 | VPusUfsugdCc(Tgn)ua acuaGfcUfcaauusua | 2342 | UAAAUUGAGCUA GUUAAGGCAAA | 2676 |
| AD-1784348.1 | ascsuaaaAfuGfCf Ufgcuuuuaasasa | 2010 | VPusUfsuuaAfaagcagc AfuUfuuaguscsa | 2343 | UGACUAAAAUGC UGCUUUUAAAA | 2677 |
| AD-1784349.1 | ascsuucaCfuUfGf Gfuucacuggsasa | 2011 | VPusUfsccdAg(Tgn)ga accaAfgUfgaagususc | 2344 | GAACUUCACUUG GUUCACUGGAA | 2678 |
| AD-1784350.1 | uscsuaggUfaUfUf Ufuuuugaagsgsa | 2012 | VPusCfscuuCfaaaaaaa UfaCfcuagasasc | 2345 | GUUCUAGGUAUU UUUUUGAAGGU | 2679 |
| AD-1784351.1 | asgscacaGfaUfCf Ufaccuuggusgsa | 2013 | VPusCfsaccAfagguaga UfcUfgugcususa | 2346 | UAAGCACAGAUC UACCUUGGUGA | 2680 |
| AD-1784352.1 | gscscguuCfuAfGf Gfuauuuuuusa | 2014 | VPusAfsaaaAfaauaccu AfgAfacggcscsa | 2347 | UGGCCGUUCUAG GUAUUUUUUUG | 2681 |
| AD-1784353.1 | csusaaaaUfgCfUf Gfcuuuuaaasasa | 2015 | VPusUfsuuuAfaaagcag CfaUfuuuagsusc | 2348 | GACUAAAAUGCU GCUUUUAAAAC | 2682 |
| AD-1784354.1 | gsasacagGfcAfAf Afucaaagcususa | 2016 | VPusAfsagdCu(Tgn)ug auuuGfcCfuguucsusu | 2349 | AAGAACAGGCAA AUCAAAGCUUC | 2683 |
| AD-1784355.1 | usgscuuuGfuUfUf Afugguaguasgsa | 2017 | VPusCfsuacUfaccauaa AfcAfaagcascsc | 2350 | GGUGCUUUGUUU AUGGUAGUAGU | 2684 |
| AD-1784356.1 | asasuuagAfgUfUf Gfugauacagsasa | 2018 | VPusUfscudGu(Agn)u cacaaCfuCfuaauusasu | 2351 | AUAAUUAGAGUU GUGAUACAGAG | 2685 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784357.1 | csusgguuGfgUfGf Cfuuuguuuasusa | 2019 | VPusAfsuaaAfcaaagca CfcAfaccagscsc | 2352 | GGCUGGUUGGUG CUUUGUUUAUG | 2686 |
| AD-1784358.1 | uscscuucAfaAfUf Afagauggucscsa | 2020 | VPusGfsgadCc(Agn)uc uuauUfuGfaaggasasg | 2353 | CUUCCUUCAAAU AAGAUGGUCCC | 2687 |
| AD-1784359.1 | gscscuccUfuCfCf Ufgaauccuusgsa | 2021 | VPusCfsaadGg(Agn)uu caggAfaGfgaggcscsa | 2354 | UGGCCUCCUUCC UGAAUCCUUGG | 2688 |
| AD-1784360.1 | gsasuucuAfuGfUf Afauguaaacscsa | 2022 | VPusGfsgudTu(Agn)ca uuacAfuAfgaaucsasu | 2355 | AUGAUUCUAUGU AAUGUAAACCA | 2689 |
| AD-1784361.1 | usgsguugGfuGfCf Ufuuguuuausgsa | 2023 | VPusCfsauaAfacaaagc AfcCfaaccasgsc | 2356 | GCUGGUUGGUGC UUUGUUUAUGG | 2690 |
| AD-1784362.1 | csuscauaCfaGfCf Cfaaguaugascsa | 2024 | VPusGfsucaUfacuuggc UfgUfaugagsusg | 2357 | CACUCAUACAGC CAAGUAUGACC | 2691 |
| AD-1784363.1 | csuscauaCfaGfCf Cfaaguaugascsa | 2024 | VPusGfsucdAu(Agn)c uuggcUfgUfaugagsusg | 2358 | CACUCAUACAGC CAAGUAUGACC | 2691 |
| AD-1784364.1 | asuscgacAfcUfCf Afuacagccasasa | 2025 | VPusUfsugdGc(Tgn)gu augaGfuGfucgausgsu | 2359 | ACAUCGACACUC AUACAGCCAAG | 2692 |
| AD-1784365.1 | gscsacugGfcAfUf Afaggacuucscsa | 2026 | VPusGfsgadAg(Tgn)cc uuauGfcCfagugcsusc | 2360 | GAGCACUGGCAU AAGGACUUCCC | 2693 |
| AD-1784366.1 | asascgugGfaGfUf Ufugaugacuscsa | 2027 | VPusGfsagdTc(Agn)uc aaacUfcCfacguusgsa | 2361 | UCAACGUGGAGU UUGAUGACUCU | 2694 |
| AD-1784367.1 | gscsaaauCfaGfGf Ufaaaauaguscsa | 2028 | VPusGfsacuAfuuuuacc UfgAfuuugcscsu | 2362 | AGGCAAAUCAGG UAAAAUAGUCA | 2695 |
| AD-1784368.1 | gscsaaauCfaGfGf Ufaaaauaguscsa | 2028 | VPusGfsacdTa(Tgn)uu uaccUfgAfuuugcscsu | 2363 | AGGCAAAUCAGG UAAAAUAGUCA | 2695 |
| AD-1784369.1 | csusucagAfaAfGf Ufuguugaugsusa | 2029 | VPusAfscauCfaacaacu UfuCfugaagsgsc | 2364 | GCCUUCAGAAAG UUGUUGAUGUG | 2696 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784370.1 | csusucagAfaAfGf Ufuguugaugsusa | 2029 | VPusAfscadTc(Agn)ac aacuUfuCfugaagsgsc | 2365 | GCCUUCAGAAG UUGUUGAUGUG | 2696 |
| AD-1784371.1 | asasaucaGfgUfAf Afaauagucasusa | 2030 | VPusAfsugdAc(Tgn)au uuuaCfcUfgauuusgsc | 2366 | GCAAAUCAGGUA AAAUAGUCAUG | 2697 |
| AD-1784372.1 | asgsgcaaAfuCfAf Gfguaaaauasgsa | 2031 | VPusCfsuauUfuuaccug AfuUfugccususa | 2367 | UAAGGCAAAUCA GGUAAAAUAGU | 2698 |
| AD-1784373.1 | gsgsgcaaGfaGfUf Gfcugacuucsasa | 2032 | VPusUfsgadAg(Tgn)ca gcacUfcUfugcccsusu | 2368 | AAGGGCAAGAGU GCUGACUUCAC | 2699 |
| AD-1784375.1 | gsgsccguUfcUfAf Gfguauuuuusus a | 2033 | VPusAfsaaaAfauaccua GfaAfcggccsasg | 2369 | CUGGCCGUUCUA GGUAUUUUUUU | 2700 |
| AD-1784377.1 | csgsggccUfuCfAf Gfaaaguugususa | 2034 | VPusAfsacaAfcuuucug AfaGfgcccgsgsu | 2370 | ACCGGGCCUUCA GAAAGUUGUUG | 2701 |
| AD-1784378.1 | gsgscaaaUfcAfGf Gfuaaaauagsusa | 2035 | VPusAfscuaUfuuuaccu GfaUfuugccsusu | 2371 | AAGGCAAAUCAG GUAAAAUAGUC | 2702 |
| AD-1784379.1 | gsasggauCfcUfCf Afacaaugguscsa | 2036 | VPusGfsaccAfuuguuga GfgAfuccucsasg | 2372 | CUGAGGAUCCUC AACAAUGGUCA | 2703 |
| AD-1784380.1 | gsasggauCfcUfCf Afacaaugguscsa | 2036 | VPusGfsacdCa(Tgn)ug uugaGfgAfuccucsasg | 2373 | CUGAGGAUCCUC AACAAUGGUCA | 2703 |
| AD-1784381.1 | ususcacuUfgGfUf Ufcacuggaascsa | 2037 | VPusGfsuudCc(Agn)g ugaacCfaAfgugaasgsu | 2374 | ACUUCACUUGGU UCACUGGAACA | 2704 |
| AD-1784382.1 | asgsaacuGfaUfGf Gfuggacaacsusa | 2038 | VPusAfsgudTg(Tgn)cc accaUfcAfguucususc | 2375 | GAAGAACUGAUG GUGGACAACUG | 2705 |
| AD-1784383.1 | asasuaaaGfuAfCf Cfuugacuuusgsa | 2039 | VPusCfsaaaGfucaaggu AfcUfuuauuscsu | 2376 | AGAAUAAAGUAC CUUGACUUUGU | 2706 |
| AD-1784384.1 | asasuaaaGfuAfCf Cfuugacuuusgsa | 2039 | VPusCfsaadAg(Tgn)ca agguAfcUfuuauuscsu | 2377 | AGAAUAAAGUAC CUUGACUUUGU | 2706 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784385.1 | csusuuguUfcAfCf Afgcauguagsgsa | 2040 | VPusCfscuaCfaugcugu GfaAfcaaagsusc | 2378 | GACUUUGUUCAC AGCAUGUAGGG | 2707 |
| AD-1784386.1 | csusuuguUfcAfCf Afgcauguagsgsa | 2040 | VPusCfscudAc(Agn)ug cuguGfaAfcaaagsusc | 2379 | GACUUUGUUCAC AGCAUGUAGGG | 2707 |
| AD-1784387.1 | asasuaagAfaUfAf Afaguaccuusgsa | 2041 | VPusCfsaadGg(Tgn)ac uuuaUfuCfuuauusuc | 2380 | GAAAUAAGAAUA AAGUACCUUGA | 2708 |
| AD-1784388.1 | asgsuaguUfuUfUf Cfuguaacacsasa | 2042 | VPusUfsgudGu(Tgn)ac agaaAfaAfcuacusasc | 2381 | GUAGUAGUUUUU CUGUAACACAG | 2709 |
| AD-1784389.1 | cscsaaguAfuGfAf Cfccuucccusgsa | 2043 | VPusCfsaggGfaaggguc AfuAfcuuggscsu | 2382 | AGCCAAGUAUGA CCCUUCCCUGA | 2710 |
| AD-1784390.1 | ususgaguGfcAfAf Afuccauagcsasa | 2044 | VPusUfsgcdTa(Tgn)gg auuuGfcAfcucaascsc | 2383 | GGUUGAGUGCAA AUCCAUAGCAC | 2711 |
| AD-1784391.1 | gsgsccuuCfaGfAf Afaguuguugsasa | 2045 | VPusUfscaaCfaacuuuc UfgAfaggccscsg | 2384 | CGGGCCUUCAGA AAGUUGUUGAU | 2712 |
| AD-1784392.1 | asgsgaucCfuCfAf Afcaauggucsasa | 2046 | VPusUfsgadCc(Agn)uu guugAfgGfauccuscsa | 2385 | UGAGGAUCCUCA ACAAUGGUCAU | 2713 |
| AD-1784393.1 | asusuagaGfuUfGf Ufgauacagasgsa | 2047 | VPusCfsucdTg(Tgn)au cacaAfcUfcuaaususa | 2386 | UAAUUAGAGUUG UGAUACAGAGU | 2714 |
| AD-1784394.1 | csasacguGfgAfGf Ufuugaugacsusa | 2048 | VPusAfsgudCa(Tgn)ca aacuCfcAfcguugsasa | 2387 | UUCAACGUGGAG UUUGAUGACUC | 2715 |
| AD-1784395.1 | gsascuuuUfgAfAf Ufuacagagasusa | 2049 | VPusAfsucdTc(Tgn)gu aauuCfaAfaagucsasu | 2388 | AUGACUUUUGAA UUACAGAGAUA | 2716 |
| AD-1784396.1 | usgsaggaUfcCfUf Cfaacaauggsusa | 2050 | VPusAfsccaUfuguugag GfaUfccucasgsg | 2389 | CCUGAGGAUCCU CAACAAUGGUC | 2717 |
| AD-1784397.1 | gsasguuuGfaUfGf Afcucucaggsasa | 2051 | VPusUfsccdTg(Agn)ga gucaUfcAfaacucscsa | 2390 | UGGAGUUUGAUG ACUCUCAGGAC | 2718 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784398.1 | ususuuaaAfaCfAf Ufaggaaagusasa | 2052 | VPusUfsacdTu(Tgn)cc uaugUfuUfuaaaasgsc | 2391 | GCUUUUAAAACA UAGGAAAGUAG | 2719 |
| AD-1784399.1 | ususauggUfaGfUf Afguuuuucusgsa | 2053 | VPusCfsagaAfaaacuac UfaCfcauaasasc | 2392 | GUUUAUGGUAGU AGUUUUUCUGU | 2720 |
| AD-1784400.1 | asascuucAfcUfUf Gfguucacugsgsa | 2054 | VPusCfscagUfgaaccaa GfuGfaaguuscsu | 2393 | AGAACUUCACUU GGUUCACUGGA | 2721 |
| AD-1784401.1 | asasauugAfgCfUf Afguuaaggcsasa | 2055 | VPusUfsgcdCu(Tgn)aa cuagCfuCfaauuusasu | 2394 | AUAAAUUGAGCU AGUUAAGGCAA | 2722 |
| AD-1784402.1 | ususuguuUfaUfGf Gfuaguaguususa | 2056 | VPusAfsaacUfacuacca UfaAfacaaasgsc | 2395 | GCUUUGUUUAUG GUAGUAGUUUU | 2723 |
| AD-1784403.1 | ususuguuUfaUfGf Gfuaguaguususa | 2056 | VPusAfsaadCu(Agn)cu accaUfaAfacaaasgsc | 2396 | GCUUUGUUUAUG GUAGUAGUUUU | 2723 |
| AD-1784404.1 | asasgggcAfaGfAf Gfugcugacususa | 2057 | VPusAfsagdTc(Agn)gc acucUfuGfcccuusug | 2397 | CAAAGGGCAAGA GUGCUGACUUC | 2724 |
| AD-1784405.1 | gsgsaguuUfgAfUf Gfacucucagsgsa | 2058 | VPusCfscugAfgagucau CfaAfacuccsasc | 2398 | GUGGAGUUUGAU GACUCUCAGGA | 2725 |
| AD-1784406.1 | gsgsgccuUfcAfGf Afaaguuguusgsa | 2059 | VPusCfsaacAfacuuucu GfaAfggcccsgsg | 2399 | CCGGGCCUUCAG AAAGUUGUUGA | 2726 |
| AD-1784407.1 | gscsaagaGfuGfCf Ufgacuucacsusa | 2060 | VPusAfsgudGa(Agn)g ucagcAfcUfcuugcscsc | 2400 | GGGCAAGAGUGC UGACUUCACUA | 2727 |
| AD-1784408.1 | asgsccacUfgAfAf Gfaacaggcasasa | 2061 | VPusUfsugdCc(Tgn)gu ucuuCfaGfuggcusgsa | 2401 | UCAGCCACUGAA GAACAGGCAAA | 2728 |
| AD-1784409.1 | asusuccaUfuAfAf Afacaaagggscsa | 2062 | VPusGfscccUfuuguuuu AfaUfggaauscsc | 2402 | GGAUUCCAUUAA AACAAAGGGCA | 2729 |
| AD-1784410.1 | asusuccaUfuAfAf Afacaaagggscsa | 2062 | VPusGfsccdCu(Tgn)ug uuuuAfaUfggaauscsc | 2403 | GGAUUCCAUUAA AACAAAGGGCA | 2729 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784411.1 | gsusaguuUfuUfCf Ufguaacacasgsa | 2063 | VPusCfsugdTg(Tgn)ua cagaAfaAfacuacsusa | 2404 | UAGUAGUUUUC UGUAACACAGA | 2730 |
| AD-1784412.1 | gsgsuauuUfuUfUf Ufgaagguugsgsa | 2064 | VPusCfscaaCfcuucaaa AfaAfauaccsusa | 2405 | UAGGUAUUUUU UGAAGGUUGGC | 2731 |
| AD-1784413.1 | uscscaaaUfaAfUf Gfaaucuucgsgsa | 2065 | VPusCfscgaAfgauucau UfaUfuuggasusa | 2406 | UAUCCAAAUAAU GAAUCUUCGGG | 2732 |
| AD-1784414.1 | asasacauAfgGfAf Afaguagaausgsa | 2066 | VPusCfsaudTc(Tgn)ac uuucCfuAfuguuususa | 2407 | UAAAACAUAGGA AAGUAGAAUGG | 2733 |
| AD-1784415.1 | asusgacuCfuCfAf Gfgacaaagcsasa | 2067 | VPusUfsgcdTu(Tgn)gu ccugAfgAfgucauscsa | 2408 | UGAUGACUCUCA GGACAAAGCAG | 2734 |
| AD-1784416.1 | asgscuagUfuAfAf Gfgcaaaucasgsa | 2068 | VPusCfsugaUfuugccuu AfaCfuagcuscsa | 2409 | UGAGCUAGUUAA GGCAAAUCAGG | 2735 |
| AD-1784417.1 | cscsugagGfaUfCf Cfucaacaausgsa | 2069 | VPusCfsaudTg(Tgn)ug aggaUfcCfucaggsgsa | 2410 | UCCCUGAGGAUC CUCAACAAUGG | 2736 |
| AD-1784418.1 | gsgsuuggUfgCfUf Ufuguuuaugsgsa | 2070 | VPusCfscauAfaacaaag CfaCfcaaccsasg | 2411 | CUGGUUGGUGCU UUGUUUAUGGU | 2737 |
| AD-1784419.1 | asgsguauUfuUfUf Ufugaagguusgsa | 2071 | VPusCfsaacCfuucaaaa AfaAfuaccusasg | 2412 | CUAGGUAUUUUU UUGAAGGUUGG | 2738 |
| AD-1784420.1 | usgsaaucUfuCfGf Gfguguuuccscsa | 2072 | VPusGfsggaAfacacccg AfaGfauucasusu | 2413 | AAUGAAUCUUCG GGUGUUUCCCU | 2739 |
| AD-1784421.1 | usgsaaucUfuCfGf Gfguguuuccscsa | 2072 | VPusGfsggdAa(Agn)ca cccgAfaGfauucasusu | 2414 | AAUGAAUCUUCG GGUGUUUCCCU | 2739 |
| AD-1784422.1 | usasguagUfuUfUf Ufcuguaacascsa | 2073 | VPusGfsuguUfacagaaa AfaCfuacuascsc | 2415 | GGUAGUAGUUUU UCUGUAACACA | 2740 |
| AD-1784423.1 | usasguagUfuUfUf Ufcuguaacascsa | 2073 | VPusGfsugdTu(Agn)ca gaaaAfaCfuacuascsc | 2416 | GGUAGUAGUUUU UCUGUAACACA | 2740 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784424.1 | gsusuugaUfgAfCf Ufcucaggacsasa | 2074 | VPusUfsgudCc(Tgn)ga gaguCfaUfcaaacsusc | 2417 | GAGUUUGAUGAC UCUCAGGACAA | 2741 |
| AD-1784425.1 | asasugaaUfcUfUf Cfggguguuuscsa | 2075 | VPusGfsaadAc(Agn)cc cgaaGfaUfucauusasu | 2418 | AUAAUGAAUCUU CGGGUGUUUCC | 2742 |
| AD-1784426.1 | csasaauaAfuGfAf Afucuucgggsusa | 2076 | VPusAfscccGfaagauuc AfuUfauuugsgsa | 2419 | UCCAAAUAAUGA AUCUUCGGGUG | 2743 |
| AD-1784427.1 | ususuguuCfaCfAf Gfcauguaggsgsa | 2077 | VPusCfsccuAfcaugcug UfgAfacaaasgsu | 2420 | ACUUUGUUCACA GCAUGUAGGGU | 2744 |
| AD-1784428.1 | asusugugCfuCfAf Afggaacccasusa | 2078 | VPusAfsugdGg(Tgn)uc cuugAfgCfacaauscsc | 2421 | GGAUUGUGCUCA AGGAACCCAUC | 2745 |
| AD-1784429.1 | gsusugguGfcUfUf Ufguuuauggsusa | 2079 | VPusAfsccaUfaaacaaa GfcAfccaacscsa | 2422 | UGGUUGGUGCUU UGUUUAUGGUA | 2746 |
| AD-1784430.1 | gsasaucuUfcGfGf Gfuguuucccsusa | 2080 | VPusAfsgggAfaacaccc GfaAfgauucsasu | 2423 | AUGAAUCUUCGG GUGUUUCCCUU | 2747 |
| AD-1784431.1 | gsgsuaguAfgUfUf Ufuucuguaascsa | 2081 | VPusGfsuudAc(Agn)g aaaaaCfuAfcuaccsasu | 2424 | AUGGUAGUAGUU UUUCUGUAACA | 2748 |
| AD-1784432.1 | asgsaacaGfgCfAf Afaucaaagcsusa | 2082 | VPusAfsgcuUfugauuug CfcUfguucususc | 2425 | GAAGAACAGGCA AAUCAAAGCUU | 2749 |
| AD-1784433.1 | asgsaacaGfgCfAf Afaucaaagcsusa | 2082 | VPusAfsgcdTu(Tgn)ga uuugCfcUfguucususc | 2426 | GAAGAACAGGCA AAUCAAAGCUU | 2749 |
| AD-1784434.1 | asasaauaGfuCfAf Ufgauucuausgsa | 2083 | VPusCfsauaGfaaucaug AfcUfauuuusasc | 2427 | GUAAAAUAGUCA UGAUUCUAUGU | 2750 |
| AD-1784435.1 | ascsuggcCfgUfUf Cfuagguauususa | 2084 | VPusAfsaauAfccuagaa CfgGfccaguscsc | 2428 | GGACUGGCCGUU CUAGGUAUUUU | 2751 |
| AD-1784436.1 | cscscugaGfgAfUf Cfcucaacaasusa | 2085 | VPusAfsuudGu(Tgn)ga ggauCfcUfcagggsasa | 2429 | UUCCCUGAGGAU CCUCAACAAUG | 2752 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784437.1 | gscsugguUfgGfUf Gfcuuuguuusasa | 2086 | VPusUfsaaaCfaaagcac CfaAfccagcscsa | 2430 | UGGCUGGUUGGU GCUUUGUUUAU | 2753 |
| AD-1784438.1 | csasgaaaGfuUfGf Ufugaugugcsusa | 2087 | VPusAfsgcdAc(Agn)uc aacaAfcUfuucugsasa | 2431 | UUCAGAAAGUUG UUGAUGUGCUG | 2754 |
| AD-1784439.1 | ascsuaacUfuCfGf Afuccucgugsgsa | 2088 | VPusCfscacGfaggaucg AfaGfuuagusgsa | 2432 | UCACUAACUUCG AUCCUCGUGGC | 2755 |
| AD-1784440.1 | cscsuucaGfaAfAf Gfuuguugausgsa | 2089 | VPusCfsaucAfacaacuu UfcUfgaaggscsc | 2433 | GGCCUUCAGAAA GUUGUUGAUGU | 2756 |
| AD-1784441.1 | usgsagcaCfuGfGf Cfauaaggacsusa | 2090 | VPusAfsgudCc(Tgn)ua ugccAfgUfgcucasgsg | 2434 | CCUGAGCACUGG CAUAAGGACUU | 2757 |
| AD-1784442.1 | csusaguuAfaGfGf Cfaaaucaggsusa | 2091 | VPusAfsccdTg(Agn)uu ugccUfuAfacuagscsu | 2435 | AGCUAGUUAAGG CAAAUCAGGUA | 2758 |
| AD-1784443.1 | csusgaggAfuCfCf Ufcaacaaugsgsa | 2092 | VPusCfscauUfguugagg AfuCfcucagsgsg | 2436 | CCCUGAGGAUCC UCAACAAUGGU | 2759 |
| AD-1784444.1 | gsusuuauGfgUfAf Gfuaguuuuuscsa | 2093 | VPusGfsaaaAfacuacua CfcAfuaaacsasa | 2437 | UUGUUUAUGGUA GUAGUUUUUCU | 2760 |
| AD-1784445.1 | usgsugacCfuGfGf Afuugugcucsasa | 2094 | VPusUfsgadGc(Agn)ca auccAfgGfucacascsa | 2438 | UGUGUGACCUGG AUUGUGCUCAA | 2761 |
| AD-1784446.1 | ascsaucgAfcAfCf Ufcauacagcscsa | 2095 | VPusGfsgcdTg(Tgn)au gaguGfuCfgauguscsa | 2439 | UGACAUCGACAC UCAUACAGCCA | 2762 |
| AD-1784447.1 | gsasuuguGfcUfCf Afaggaacccsasa | 2096 | VPusUfsggdGu(Tgn)cc uugaGfcAfcaaucscsa | 2440 | UGGAUUGUGCUC AAGGAACCCAU | 2763 |
| AD-1784448.1 | uscsauacAfgCfCf Afaguaugacscsa | 2097 | VPusGfsgudCa(Tgn)ac uuggCfuGfuaugasgsu | 2441 | ACUCAUACAGCC AAGUAUGACCC | 2764 |
| AD-1784449.1 | usasaaauAfgUfCf Afugauucuasusa | 2098 | VPusAfsuagAfaucauga CfuAfuuuuascsc | 2442 | GGUAAAAUAGUC AUGAUUCUAUG | 2765 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784450.1 | usasaaauAfgUfCf Afugauucuasusa | 2098 | VPusAfsuadGa(Agn)uc augaCfuAfuuuuascsc | 2443 | GGUAAAAUAGUC AUGAUUCUAUG | 2765 |
| AD-1784451.1 | gsusgcucAfaGfGf Afacccaucasgsa | 2099 | VPusCfsugaUfgguucc UfuGfagcacsasa | 2444 | UUGUGCUCAAGG AACCCAUCAGC | 2766 |
| AD-1784452.1 | csgsaagaAfcUfGf Afugguggacsasa | 2100 | VPusUfsgudCc(Agn)cc aucaGfuUfcuucgsgsg | 2445 | CCCGAAGAACUG AUGGUGGACAA | 2767 |
| AD-1784453.1 | asasuaaaAfuGfUf Gfaagacuagsasa | 2101 | VPusUfscudAg(Tgn)cu ucacAfuUfuuauusasg | 2446 | CUAAUAAAAUGU GAAGACUAGAC | 2768 |
| AD-1784454.1 | gsgscuggUfuGfGf Ufgcuuuguusua | 2102 | VPusAfsaacAfaagcacc AfaCfcagccsasc | 2447 | GUGGCUGGUUGG UGCUUUGUUUA | 2769 |
| AD-1784455.1 | ususguucAfcAfGf Cfauguagggsusa | 2103 | VPusAfscccUfacaugcu GfuGfaacaasasg | 2448 | CUUUGUUCACAG CAUGUAGGGUG | 2770 |
| AD-1784456.1 | ususcaaaUfaAfGf Afuggucccasusa | 2104 | VPusAfsugdGg(Agn)c caucuUfaUfuugaasgsg | 2449 | CCUUCAAAUAAG AUGGUCCCAUA | 2771 |
| AD-1784457.1 | ususuaaaAfcAfUf Afggaaaguasgsa | 2105 | VPusCfsuacUfuuccuau GfuUfuuaaasasg | 2450 | CUUUUAAAACAU AGGAAAGUAGA | 2772 |
| AD-1784458.1 | ususguuuAfuGfGf Ufaguaguuususa | 2106 | VPusAfsaaaCfuacuacc AfuAfaacaasasg | 2451 | CUUUGUUUAUGG UAGUAGUUUUU | 2773 |
| AD-1784459.1 | ususguuuAfuGfGf Ufaguaguuususa | 2106 | VPusAfsaadAc(Tgn)ac uaccAfuAfaacaasasg | 2452 | CUUUGUUUAUGG UAGUAGUUUUU | 2773 |
| AD-1784460.1 | gscsuaguUfaAfGf Gfcaaaucagsgsa | 2107 | VPusCfscugAfuuugccu UfaAfcuagcsusc | 2453 | GAGCUAGUUAAG GCAAAUCAGGU | 2774 |
| AD-1784461.1 | gsusaaaaUfaGfUf Cfaugauucusasa | 2108 | VPusUfsagaAfucaugac UfaUfuuuacscsu | 2454 | AGGUAAAAUAGU CAUGAUUCUAU | 2775 |
| AD-1784462.1 | gsusaaaaUfaGfUf Cfaugauucusasa | 2108 | VPusUfsagdAa(Tgn)ca ugacUfaUfuuuacscsu | 2455 | AGGUAAAAUAGU CAUGAUUCUAU | 2775 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784463.1 | gsascuggCfcGfUf Ufcuagguaususa | 2109 | VPusAfsauaCfcuagaac GfgCfcagucscsa | 2456 | UGGACUGGCCGU UCUAGGUAUUU | 2776 |
| AD-1784464.1 | uscsagccAfcUfGf Afagaacaggscsa | 2110 | VPusGfsccdTg(Tgn)uc uucaGfuGfgcugasgsc | 2457 | GCUCAGCCACUG AAGAACAGGCA | 2777 |
| AD-1784465.1 | usgsgaauGfuGfUf Gfaccuggaususa | 2111 | VPusAfsaudCc(Agn)gg ucacAfcAfuuccasgsa | 2458 | UCUGGAAUGUGU GACCUGGAUUG | 2778 |
| AD-1784466.1 | uscsacagCfaUfGf Ufagggugausgsa | 2112 | VPusCfsaucAfcccuaca UfgCfugugasasc | 2459 | GUUCACAGCAUG UAGGGUGAUGA | 2779 |
| AD-1784467.1 | usascagcCfaAfGf Ufaugacccususa | 2113 | VPusAfsagdGg(Tgn)ca uacuUfgGfcuguasusg | 2460 | CAUACAGCCAAG UAUGACCCUUC | 2780 |
| AD-1784468.1 | csusgagcAfcUfGf Gfcauaaggascsa | 2114 | VPusGfsuccUfuaugcca GfuGfcucagsgsu | 2461 | ACCUGAGCACUG GCAUAAGGACU | 2781 |
| AD-1784469.1 | csusgagcAfcUfGf Gfcauaaggascsa | 2114 | VPusGfsucdCu(Tgn)au gccaGfuGfcucagsgsu | 2462 | ACCUGAGCACUG GCAUAAGGACU | 2781 |
| AD-1784470.1 | gscsucaaGfgAfAf Cfccaucagcsgsa | 2115 | VPusCfsgcdTg(Agn)ug gguuCfcUfugagcsasc | 2463 | GUGCUCAAGGAA CCCAUCAGCGU | 2782 |
| AD-1784471.1 | ususcuggAfaUfGf Ufgugaccugsgsa | 2116 | VPusCfscadGg(Tgn)ca cacaUfuCfcagaasgsa | 2464 | UCUUCUGGAAUG UGUGACCUGGA | 2783 |
| AD-1784472.1 | usasaauuGfaGfCf Ufaguuaaggscsa | 2117 | VPusGfsccuUfaacuagc UfcAfauuuasusc | 2465 | GAUAAAUUGAGC UAGUUAAGGCA | 2784 |
| AD-1784473.1 | usasuuuuUfuUfGf Afagguuggcsasa | 2118 | VPusUfsgcdCa(Agn)cc uucaAfaAfaaauascsc | 2466 | GGUAUUUUUUUG AAGGUUGGCAG | 2785 |
| AD-1784474.1 | usasauuaGfaGfUf Ufgugauacasgsa | 2119 | VPusCfsuguAfucacaac UfcUfaauuasusa | 2467 | UAUAAUUAGAGU UGUGAUACAGA | 2786 |
| AD-1784475.1 | usasauuaGfaGfUf Ufgugauacasgsa | 2119 | VPusCfsugdTa(Tgn)ca caacUfcUfaauuasusa | 2468 | UAUAAUUAGAGU UGUGAUACAGA | 2786 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784476.1 | usgsacucUfcAfGfGfacaaagcasgsa | 2120 | VPusCfsugcUfuuguccuGfaGfagucasusc | 2469 | GAUGACUCUCAGGACAAAGCAGU | 2787 |
| AD-1784477.1 | usgsacucUfcAfGfGfacaaagcasgsa | 2120 | VPusCfsugdCu(Tgn)uguccuGfaGfagucasusc | 2470 | GAUGACUCUCAGGACAAAGCAGU | 2787 |
| AD-1784478.1 | csasuacaGfcCfAfAfguaugaccscsa | 2121 | VPusGfsggdTc(Agn)uacuugGfcUfguaugsasg | 2471 | CUCAUACAGCCAAGUAUGACCCU | 2788 |
| AD-1784479.1 | gsusauuuUfuUfUfGfaagguuggscsa | 2122 | VPusGfsccaAfccuucaaAfaAfaauacscsu | 2472 | AGGUAUUUUUUUGAAGGUUGGCA | 2789 |
| AD-1784480.1 | csascagcAfuGfUfAfgggugaugsasa | 2123 | VPusUfscadTc(Agn)cccuacAfuGfcugugsasa | 2473 | UUCACAGCAUGUAGGGUGAUGAG | 2790 |
| AD-1784481.1 | usasuaauUfaGfAfGfuugugauascsa | 2124 | VPusGfsuadTc(Agn)caacucUfaAfuuauasasc | 2474 | GUUAUAAUUAGAGUUGUGAUACA | 2791 |
| AD-1784482.1 | gsasuuuuGfgGfAfAfagcugugcsasa | 2125 | VPusUfsgcdAc(Agn)gcuuucCfcAfaaaucscsc | 2475 | GGGAUUUUGGGAAAGCUGUGCAG | 2792 |
| AD-1784483.1 | usasaaacAfaAfGfGfgcaagagusgsa | 2126 | VPusCfsacdTc(Tgn)ugcccuUfuGfuuuuasasu | 2476 | AUUAAAACAAAGGGCAAGAGUGC | 2793 |
| AD-1784484.1 | uscsaaggAfaCfCfCfaucagcguscsa | 2127 | VPusGfsacgCfugaugggUfuCfcuugasgsc | 2477 | GCUCAAGGAACCCAUCAGCGUCA | 2794 |
| AD-1784485.1 | uscsaaggAfaCfCfCfaucagcguscsa | 2127 | VPusGfsacdGc(Tgn)gaugggUfuCfcuugasgsc | 2478 | GCUCAAGGAACCCAUCAGCGUCA | 2794 |
| AD-1784486.1 | uscsagaaAfgUfUfGfuugaugugscsa | 2128 | VPusGfscacAfucaacaaCfuUfucugasasg | 2479 | CUUCAGAAAGUUGUUGAUGUGCU | 2795 |
| AD-1784487.1 | uscsagaaAfgUfUfGfuugaugugscsa | 2128 | VPusGfscadCa(Tgn)caacaaCfuUfucugasasg | 2480 | CUUCAGAAAGUUGUUGAUGUGCU | 2795 |
| AD-1784488.1 | cscsaaauAfaUfGfAfaucuucggsgsa | 2129 | VPusCfsccgAfagauucaUfuAfuuuggsasu | 2481 | AUCCAAAUAAUGAAUCUUCGGGU | 2796 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784489.1 | asgscaugUfaGfGf Gfugaugagcsasa | 2130 | VPusUfsgcdTc(Agn)uc acccUfaCfaugcusgsu | 2482 | ACAGCAUGUAGG GUGAUGAGCAC | 2797 |
| AD-1784490.1 | gsasuaaaUfuGfAf Gfcuaguuaasgsa | 2131 | VPusCfsuuaAfcuagcuc AfaUfuuaucsusu | 2483 | AAGAUAAAUUGA GCUAGUUAAGG | 2798 |
| AD-1784491.1 | csuscagcCfaCfUf Gfaagaacagsgsa | 2132 | VPusCfscugUfucuucag UfgGfcugagscsu | 2484 | AGCUCAGCCACU GAAGAACAGGC | 2799 |
| AD-1784492.1 | csuscagcCfaCfUf Gfaagaacagsgsa | 2132 | VPusCfscudGu(Tgn)cu ucagUfgGfcugagscsu | 2485 | AGCUCAGCCACU GAAGAACAGGC | 2799 |
| AD-1784493.1 | csasgcauGfuAfGf Gfgugaugagscsa | 2133 | VPusGfscudCa(Tgn)ca cccuAfcAfugcugsusg | 2486 | CACAGCAUGUAG GGUGAUGAGCA | 2800 |
| AD-1784494.1 | asusaaugAfaUfCf Ufucgggugususa | 2134 | VPusAfsacaCfccgaaga UfuCfauuaususu | 2487 | AAAUAAUGAAUC UUCGGGUGUUU | 2801 |
| AD-1784495.1 | gsgsaaccCfaUfCf Afgcgucagcsasa | 2135 | VPusUfsgcdTg(Agn)cg cugaUfgGfguuccsusu | 2488 | AAGGAACCCAUC AGCGUCAGCAG | 2802 |
| AD-1784496.1 | usgsuucaCfaGfCf Afuguaggugsgsa | 2136 | VPusCfsaccCfuacaugc UfgUfgaacasasa | 2489 | UUUGUUCACAGC AUGUAGGGUGA | 2803 |
| AD-1784497.1 | asasacaaAfgGfGf Cfaagagugcsusa | 2137 | VPusAfsgcdAc(Tgn)cu ugccCfuUfuguuususa | 2490 | UAAAACAAAGGG CAAGAGUGCUG | 2804 |
| AD-1784498.1 | usgsugugAfcCfUf Gfgauugugcsusa | 2138 | VPusAfsgcaCfaauccag GfuCfacacasusu | 2491 | AAUGUGUGACCU GGAUUGUGCUC | 2805 |
| AD-1784499.1 | usgsugugAfcCfUf Gfgauugugcsusa | 2138 | VPusAfsgcdAc(Agn)au ccagGfuCfacacasusu | 2492 | AAUGUGUGACCU GGAUUGUGCUC | 2805 |
| AD-1784500.1 | asasuaauGfaAfUf Cfuucgggugsusa | 2139 | VPusAfscacCfcgaagau UfcAfuuauususg | 2493 | CAAAUAAUGAAU CUUCGGGUGUU | 2806 |
| AD-1784501.1 | asusuuuuUfuGfAf Afgguuggcasgsa | 2140 | VPusCfsugcCfaaccuuc AfaAfaaaausasc | 2494 | GUAUUUUUUUGA AGGUUGGCAGC | 2807 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784502.1 | asusuuuuUfuGfAf Afgguuggcasgsa | 2140 | VPusCfsugdCc(Agn)ac cuucAfaAfaaaausasc | 2495 | GUAUUUUUUUGA AGGUUGGCAGC | 2807 |
| AD-1784503.1 | usgscucaAfgGfAf Afcccaucagscsa | 2141 | VPusGfscudGa(Tgn)gg guucCfuUfgagcascsa | 2496 | UGUGCUCAAGGA ACCCAUCAGCG | 2808 |
| AD-1784504.1 | usasaugaAfuCfUf Ufcggguguususa | 2142 | VPusAfsaacAfcccgaag AfuUfcauuasusu | 2497 | AAUAAUGAAUCU UCGGGUGUUUC | 2809 |
| AD-1784505.1 | gsusggcuGfgUfUf Gfgugcuuugsusa | 2143 | VPusAfscaaAfgcaccaa CfcAfgccacsasg | 2498 | CUGUGGCUGGUU GGUGCUUUGUU | 2810 |
| AD-1784506.1 | asasggaaCfcCfAf Ufcagcgucasgsa | 2144 | VPusCfsugaCfgcugaug GfgUfuccuusgsa | 2499 | UCAAGGAACCCA UCAGCGUCAGC | 2811 |
| AD-1784507.1 | ususaaaaCfaAfAf Gfggcaagagsusa | 2145 | VPusAfscudCu(Tgn)gc ccuuUfgUfuuuaasusg | 2500 | CAUUAAAACAAA GGGCAAGAGUG | 2812 |
| AD-1784508.1 | csusguggCfuGfGf Ufuggugcuususa | 2146 | VPusAfsaadGc(Agn)cc aaccAfgCfcacagscsa | 2501 | UGCUGUGGCUGG UUGGUGCUUUG | 2813 |
| AD-1784509.1 | csasgcucAfgCfCf Afcugaagaascsa | 2147 | VPusGfsuudCu(Tgn)ca guggCfuGfagcugsgsg | 2502 | CCCAGCUCAGCC ACUGAAGAACA | 2814 |
| AD-1784510.1 | asasauaaUfgAfAf Ufcuucgggusgsa | 2148 | VPusCfsaccCfgaagauu CfaUfuauuusgsg | 2503 | CCAAAUAAUGAA UCUUCGGGUGU | 2815 |
| AD-1784511.1 | gsasauguGfuGfAf Cfcuggauugsusa | 2149 | VPusAfscaaUfccagguc AfcAfcauucscsa | 2504 | UGGAAUGUGUGA CCUGGAUUGUG | 2816 |
| AD-1784512.1 | csasuguaGfgGfUf Gfaugagcacsusa | 2150 | VPusAfsgudGc(Tgn)ca ucacCfcUfacaugscsu | 2505 | AGCAUGUAGGGU GAUGAGCACUC | 2817 |
| AD-1784513.1 | gsgsacugGfcCfGf Ufucuagguasusa | 2151 | VPusAfsuadCc(Tgn)ag aacgGfcCfaguccsasu | 2506 | AUGGACUGGCCG UUCUAGGUAUU | 2818 |
| AD-1784514.1 | asusaaauUfgAfGf Cfuaguuaagsgsa | 2152 | VPusCfscuuAfacuagcu CfaAfuuuauscsu | 2507 | AGAUAAAUUGAG CUAGUUAAGGC | 2819 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784515.1 | gsasaaguUfgUfUfGfaugugcugsgsa | 2153 | VPusCfscagCfacaucaaCfaAfcuuucsusg | 2508 | CAGAAAGUUGUUGAUGUGCUGGA | 2820 |
| AD-1784516.1 | gsasaaguUfgUfUfGfaugugcugsgsa | 2153 | VPusCfscadGc(Agn)caucaaCfaAfcuuucsusg | 2509 | CAGAAAGUUGUUGAUGUGCUGGA | 2820 |
| AD-1784517.1 | asascaaaGfgGfCfAfagagugcusgsa | 2154 | VPusCfsagcAfcucuugcCfcUfuuguususu | 2510 | AAAACAAAGGGCAAGAGUGCUGA | 2821 |
| AD-1784518.1 | cscsugagCfaCfUfGfgcauaaggsasa | 2155 | VPusUfsccdTu(Agn)ugccagUfgCfucaggsusc | 2511 | GACCUGAGCACUGGCAUAAGGAC | 2822 |
| AD-1784519.1 | usgsauggUfgGfAfCfaacuggcgscsa | 2156 | VPusGfscgdCc(Agn)guugucCfaCfcaucasgsu | 2512 | ACUGAUGGUGGACAACUGGCGCC | 2823 |
| AD-1784520.1 | ascsaaagGfgCfAfAfgagugcugsasa | 2157 | VPusUfscadGc(Agn)cucuugCfcCfuuugususu | 2513 | AAACAAAGGGCAAGAGUGCUGAC | 2824 |
| AD-1784521.1 | ascsggacCfuGfAfGfcacuggcasusa | 2158 | VPusAfsugdCc(Agn)gugcucAfgGfuccgusug | 2514 | CAACGGACCUGAGCACUGGCAUA | 2825 |
| AD-1784522.1 | usgsggaaAfgCfUfGfugcagcaascsa | 2159 | VPusGfsuudGc(Tgn)gcacagCfuUfucccasasa | 2515 | UUUGGGAAAGCUGUGCAGCAACC | 2826 |
| AD-1784523.1 | asascccaUfcAfGfCfgucagcagscsa | 2160 | VPusGfscudGc(Tgn)gacgcuGfaUfggguuscsc | 2516 | GGAACCCAUCAGCGUCAGCAGCG | 2827 |
| AD-1784524.1 | asgscucaGfcCfAfCfugaagaacsasa | 2161 | VPusUfsgudTc(Tgn)ucagugGfcUfgagcusgsg | 2517 | CCAGCUCAGCCACUGAAGAACAG | 2828 |
| AD-1784525.1 | ususuugaAfgGfUfUfggcagcgcsusa | 2162 | VPusAfsgcdGc(Tgn)gccaacCfuUfcaaaasasa | 2518 | UUUUUUGAAGGUUGGCAGCGCUA | 2829 |
| AD-1784526.1 | gsusaugaCfcCfUfUfcccugaagscsa | 2163 | VPusGfscudTc(Agn)gggaagGfgUfcauacsusu | 2519 | AAGUAUGACCCUUCCCUGAAGCC | 2830 |
| AD-1784527.1 | csusacccAfgGfCfUfcacugaccsasa | 2164 | VPusUfsggdTc(Agn)gugagcCfuGfgguagsgsu | 2520 | ACCUACCCAGGCUCACUGACCAC | 2831 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784528.1 | ascsccagGfcUfCfAfcugaccacscsa | 2165 | VPusGfsgudGg(Tgn)cagugaGfcCfugggusasg | 2521 | CUACCCAGGCUCACUGACCACCC | 2832 |
| AD-1784529.1 | asusggacUfgGfCfCfguucuaggsusa | 2166 | VPusAfsccuAfgaacggcCfaGfuccauscsa | 2522 | UGAUGGACUGGCCGUUCUAGGUA | 2833 |
| AD-1784530.1 | ascscugaGfcAfCfUfggcauaagsgsa | 2167 | VPusCfscuuAfugccaguGfcUfcaggusesc | 2523 | GGACCUGAGCACUGGCAUAAGGA | 2834 |
| AD-1784531.1 | cscsaucaGfcGfUfCfagcagcgasgsa | 2168 | VPusCfsucgCfugcugacGfcUfgauggsgsu | 2524 | ACCCAUCAGCGUCAGCAGCGAGC | 2835 |
| AD-1784532.1 | usgsgacuGfgCfCfGfuucuaggusasa | 2169 | VPusUfsacdCu(Agn)gaacggCfcAfguccasusc | 2525 | GAUGGACUGGCCGUUCUAGGUAU | 2836 |
| AD-1784533.1 | ususuuggGfaAfAfGfcugugcagscsa | 2170 | VPusGfscudGc(Agn)cagcuuUfcCfcaaaasusc | 2526 | GAUUUUGGGAAAGCUGUGCAGCA | 2837 |
| AD-1784534.1 | csusgaugGfaCfUfGfgccguucusasa | 2171 | VPusUfsagaAfcggccagUfcCfaucagsgsu | 2527 | ACCUGAUGGACUGGCCGUUCUAG | 2838 |
| AD-1784535.1 | ascscuacCfcAfGfGfcucacugascsa | 2172 | VPusGfsucdAg(Tgn)gagccuGfgGfuaggusesc | 2528 | GGACCUACCCAGGCUCACUGACC | 2839 |
| AD-1784536.1 | gsgsaccuAfcCfCfAfggcucacusgsa | 2173 | VPusCfsagdTg(Agn)gccuggGfuAfgguccsasg | 2529 | CUGGACCUACCCAGGCUCACUGA | 2840 |
| AD-1784537.1 | gsasuggaCfuGfGfCfcguucuagsgsa | 2174 | VPusCfscuaGfaacggccAfgUfccaucsasg | 2530 | CUGAUGGACUGGCCGUUCUAGGU | 2841 |
| AD-1784538.1 | asasgguuGfgCfAfGfcgcuaaacscsa | 2175 | VPusGfsgudTu(Agn)gcgcugCfcAfaccuuscsa | 2531 | UGAAGGUUGGCAGCGCUAAACCG | 2842 |
| AD-1784539.1 | usgsauggAfcUfGfGfccguucuasgsa | 2176 | VPusCfsuagAfacggccaGfuCfcaucasgsg | 2532 | CCUGAUGGACUGGCCGUUCUAGG | 2843 |
| AD-1784540.1 | asascugaUfgGfUfGfgacaacugsgsa | 2177 | VPusCfscagUfuguccacCfaUfcaguuscsu | 2533 | AGAACUGAUGGUGGACAACUGGC | 2844 |

(continued)

| Duplex Name | Sense Sequence 5' to 3' | SEQ ID NO: | Antisense Sequence 5' to 3' | SEQ ID NO: | mRNA Target Sequence 5' to 3' | SEQ ID NO: |
|---|---|---|---|---|---|---|
| AD-1784541.1 | asascugaUfgGfUf Gfgacaacugsgsa | 2177 | VPusCfscadGu(Tgn)gu ccacCfaUfcaguuscsu | 2534 | AGAACUGAUGGU GGACAACUGGC | 2844 |
| AD-1784542.1 | ascsaacuGfcUfGf Ufggcugguusgsa | 2178 | VPusCfsaacCfagccaca GfcAfguugusgsu | 2535 | ACACAACUGCUG UGGCUGGUUGG | 2845 |
| AD-1784543.1 | ascsaacuGfcUfGf Ufggcugguusgsa | 2178 | VPusCfsaadCc(Agn)gc cacaGfcAfguugusgsu | 2536 | ACACAACUGCUG UGGCUGGUUGG | 2845 |
| AD-1784544.1 | csasacggAfcCfUf Gfagcacuggscsa | 2179 | VPusGfsccdAg(Tgn)gc ucagGfuCfcguugsusg | 2537 | CACAACGGACCU GAGCACUGGCA | 2846 |
| AD-1784545.1 | csasacugCfuGfUf Gfgcugguugsgsa | 2180 | VPusCfscaaCfcagccac AfgCfaguugsusg | 2538 | CACAACUGCUGU GGCUGGUUGGU | 2847 |
| AD-1784546.1 | ascsugauGfgUfGf Gfacaacuggscsa | 2181 | VPusGfsccdAg(Tgn)ug uccaCfcAfucagususc | 2539 | GAACUGAUGGUG GACAACUGGCG | 2848 |
| AD-1784547.1 | csusgcugUfgGfCf Ufgguuggugscsa | 2182 | VPusGfscacCfaaccagc CfaCfagcagsusu | 2540 | AACUGCUGUGGC UGGUUGGUGCU | 2849 |

Table 9. Unmodified Sense and Antisense Strand Sequences of CA2 dsRNA Agents for C16

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962343 | ACCUGAGCACUGGCAUAAGGU | 2850 | 111-131 | ACCUUAUGCCAGUGCUCAGGUCC | 3210 | 109-131 |
| AD-1962345 | CUGAGCACUGGCAUAAGGACU | 2851 | 113-133 | AGUCCUUAUGCCAGUGCUCAGGU | 3211 | 111-133 |
| AD-1962360 | UGACAUCGACACUCAUACAGU | 2852 | 168-188 | ACUGUAUGAGUGUCGAUGUCAAC | 3212 | 166-188 |
| AD-1962369 | CACUCAUACAGCCAAGUAUGU | 2853 | 177-197 | ACAUACUUGGCUGUAUGAGUGUC | 3213 | 175-197 |
| AD-1962370 | ACUCAUACAGCCAAGUAUGAU | 2854 | 178-198 | AUCAUACUUGGCUGUAUGAGUGU | 3214 | 176-198 |
| AD-1962371 | CUCAUACAGCCAAGUAUGACU | 2855 | 179-199 | AGUCAUACUUGGCUGUAUGAGUG | 3215 | 177-199 |
| AD-1962380 | CCAAGUAUGACCCUUCCCUGU | 2856 | 188-208 | ACAGGGAAGGGUCAUACUUGGCU | 3216 | 186-208 |
| AD-1962416 | CUGAGGAUCCUCAACAAUGGU | 2857 | 244-264 | ACCAUUGUUGAGGAUCCUCAGGG | 3217 | 242-264 |
| AD-1962417 | UGAGGAUCCUCAACAAUGGUU | 2858 | 245-265 | AACCAUUGUUGAGGAUCCUCAGG | 3218 | 243-265 |
| AD-1962418 | GAGGAUCCUCAACAAUGGUCU | 2859 | 246-266 | AGACCAUUGUUGAGGAUCCUCAG | 3219 | 244-266 |
| AD-1962439 | UGCUUUCAACGUGGAGUUUGU | 2860 | 267-287 | ACAAACUCCACGUUGAAAGCAUG | 3220 | 265-287 |
| AD-1962440 | GCUUUCAACGUGGAGUUUGAU | 2861 | 268-288 | AUCAAACUCCACGUUGAAAGCAU | 3221 | 266-288 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962441 | CUUUCAACGUGG AGUUUGAUU | 2862 | 269-289 | AAUCAAACUCCAC GUUGAAAGCA | 3222 | 267-289 |
| AD-1962442 | UUUCAACGUGGA GUUUGAUGU | 2863 | 270-290 | ACAUCAAACUCCA CGUUGAAAGC | 3223 | 268-290 |
| AD-1962451 | GGAGUUUGAUGA CUCUCAGGU | 2864 | 279-299 | ACCUGAGAGUCAU CAAACUCCAC | 3224 | 277-299 |
| AD-1962460 | UGACUCUCAGGA CAAAGCAGU | 2865 | 288-308 | ACUGCUUUGUCCU GAGAGUCAUC | 3225 | 286-308 |
| AD-1962557 | AACUUCACUUGG UUCACUGGU | 2866 | 425-445 | ACCAGUGAACCAA GUGAAGUUCU | 3226 | 423-445 |
| AD-1962597 | CUGAUGGACUGG CCGUUCUAU | 2867 | 485-505 | AUAGAACGGCCAG UCCAUCAGGU | 3227 | 483-505 |
| AD-1962598 | UGAUGGACUGGC CGUUCUAGU | 2868 | 486-506 | ACUAGAACGGCCA GUCCAUCAGG | 3228 | 484-506 |
| AD-1962599 | GAUGGACUGGCC GUUCUAGGU | 2869 | 487-507 | ACCUAGAACGGCC AGUCCAUCAG | 3229 | 485-507 |
| AD-1962600 | AUGGACUGGCCG UUCUAGGUU | 2870 | 488-508 | AACCUAGAACGGC CAGUCCAUCA | 3230 | 486-508 |
| AD-1962603 | GACUGGCCGUUC UAGGUAUUU | 2871 | 491-511 | AAAUACCUAGAAC GGCCAGUCCA | 3231 | 489-511 |
| AD-1962604 | ACUGGCCGUUCU AGGUAUUUU | 2872 | 492-512 | AAAAUACCUAGAA CGGCCAGUCC | 3232 | 490-512 |
| AD-1962605 | CUGGCCGUUCUA GGUAUUUUU | 2873 | 493-513 | AAAAAUACCUAGA ACGGCCAGUC | 3233 | 491-513 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962606 | UGGCCGUUCUAG GUAUUUUUU | 2874 | 494-514 | AAAAAAUACCUAG AACGGCCAGU | 3234 | 492-514 |
| AD-1962607 | GGCCGUUCUAGG UAUUUUUUU | 2875 | 495-515 | AAAAAAAUACCUA GAACGGCCAG | 3235 | 493-515 |
| AD-1962608 | GCCGUUCUAGGU AUUUUUUUU | 2876 | 496-516 | AAAAAAAAUACCU AGAACGGCCA | 3236 | 494-516 |
| AD-1962609 | CCGUUCUAGGUA UUUUUUUGU | 2877 | 497-517 | ACAAAAAAAUACC UAGAACGGCC | 3237 | 495-517 |
| AD-1962610 | CGUUCUAGGUAU UUUUUUGAU | 2878 | 498-518 | AUCAAAAAAAUAC CUAGAACGGC | 3238 | 496-518 |
| AD-1962611 | GUUCUAGGUAUU UUUUUGAAU | 2879 | 499-519 | AUUCAAAAAAAUA CCUAGAACGG | 3239 | 497-519 |
| AD-1962612 | UUCUAGGUAUUU UUUUGAAGU | 2880 | 500-520 | ACUUCAAAAAAAU ACCUAGAACG | 3240 | 498-520 |
| AD-1962613 | UCUAGGUAUUUU UUUGAAGGU | 2881 | 501-521 | ACCUUCAAAAAAA UACCUAGAAC | 3241 | 499-521 |
| AD-1962615 | UAGGUAUUUUUU UGAAGGUUU | 2882 | 503-523 | AAACCUUCAAAAA AAUACCUAGA | 3242 | 501-523 |
| AD-1962616 | AGGUAUUUUUUU GAAGGUUGU | 2883 | 504-524 | ACAACCUUCAAAA AAAUACCUAG | 3243 | 502-524 |
| AD-1962617 | GGUAUUUUUUUG AAGGUUGGU | 2884 | 505-525 | ACCAACCUUCAAA AAAAUACCUA | 3244 | 503-525 |
| AD-1962618 | GUAUUUUUUUGA AGGUUGGCU | 2885 | 506-526 | AGCCAACCUUCAA AAAAAUACCU | 3245 | 504-526 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962620 | AUUUUUUUGAAGGUUGGCAGU | 2886 | 508-528 | ACUGCCAACCUUCAAAAAAAUAC | 3246 | 506-528 |
| AD-1962648 | CGGGCCUUCAGAAAGUUGUUU | 2887 | 536-556 | AAACAACUUUCUGAAGGCCCGGU | 3247 | 534-556 |
| AD-1962649 | GGGCCUUCAGAAAGUUGUUGU | 2888 | 537-557 | ACAACAACUUUCUGAAGGCCCGG | 3248 | 535-557 |
| AD-1962650 | GGCCUUCAGAAAGUUGUUGAU | 2889 | 538-558 | AUCAACAACUUUCUGAAGGCCCG | 3249 | 536-558 |
| AD-1962652 | CCUUCAGAAAGUUGUUGAUGU | 2890 | 540-560 | ACAUCAACAACUUUCUGAAGGCC | 3250 | 538-560 |
| AD-1962653 | CUUCAGAAAGUUGUUGAUGUU | 2891 | 541-561 | AACAUCAACAACUUUCUGAAGGC | 3251 | 539-561 |
| AD-1962655 | UCAGAAAGUUGUUGAUGUGCU | 2892 | 543-563 | AGCACAUCAACAACUUUCUGAAG | 3252 | 541-563 |
| AD-1962658 | GAAAGUUGUUGAUGUGCUGGU | 2893 | 546-566 | ACCAGCACAUCAACAACUUUCUG | 3253 | 544-566 |
| AD-1962678 | AUUCCAUUAAAACAAAGGGCU | 2894 | 566-586 | AGCCCUUUGUUUUAAUGGAAUCC | 3254 | 564-586 |
| AD-1962688 | AACAAAGGGCAAGAGUGCUGU | 2895 | 576-596 | ACAGCACUCUUGCCCUUUGUUUU | 3255 | 574-596 |
| AD-1962701 | AGUGCUGACUUCACUAACUUU | 2896 | 589-609 | AAAGUUAGUGAAGUCAGCACUCU | 3256 | 587-609 |
| AD-1962703 | UGCUGACUUCACUAACUUCGU | 2897 | 591-611 | ACGAAGUUAGUGAAGUCAGCACU | 3257 | 589-611 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962705 | CUGACUUCACUA ACUUCGAUU | 2898 | 593-613 | AAUCGAAGUUAGU GAAGUCAGCA | 3258 | 591-613 |
| AD-1962706 | UGACUUCACUAA CUUCGAUCU | 2899 | 594-614 | AGAUCGAAGUUAG UGAAGUCAGC | 3259 | 592-614 |
| AD-1962707 | GACUUCACUAAC UUCGAUCCU | 2900 | 595-615 | AGGAUCGAAGUUA GUGAAGUCAG | 3260 | 593-615 |
| AD-1962712 | CACUAACUUCGA UCCUCGUGU | 2901 | 600-620 | ACACGAGGAUCGA AGUUAGUGAA | 3261 | 598-620 |
| AD-1962713 | ACUAACUUCGAU CCUCGUGGU | 2902 | 601-621 | ACCACGAGGAUCG AAGUUAGUGA | 3262 | 599-621 |
| AD-1962733 | CCUCCUUCCUGA AUCCUUGGU | 2903 | 621-641 | ACCAAGGAUUCAG GAAGGAGGCC | 3263 | 619-641 |
| AD-1962735 | UCCUUCCUGAAU CCUUGGAUU | 2904 | 623-643 | AAUCCAAGGAUUC AGGAAGGAGG | 3264 | 621-643 |
| AD-1962736 | CCUUCCUGAAUC CUUGGAUUU | 2905 | 624-644 | AAAUCCAAGGAUU CAGGAAGGAG | 3265 | 622-644 |
| AD-1962766 | CUCCUCUUCUGG AAUGUGUGU | 2906 | 674-694 | ACACACAUUCCAG AAGAGGAGGG | 3266 | 672-694 |
| AD-1962777 | GAAUGUGUGACC UGGAUUGUU | 2907 | 685-705 | AACAAUCCAGGUC ACACAUUCCA | 3267 | 683-705 |
| AD-1962780 | UGUGUGACCUGG AUUGUGCUU | 2908 | 688-708 | AAGCACAAUCCAG GUCACACAUU | 3268 | 686-708 |
| AD-1962795 | GUGCUCAAGGAA CCCAUCAGU | 2909 | 703-723 | ACUGAUGGGUUCC UUGAGCACAA | 3269 | 701-723 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962799 | UCAAGGAACCCA UCAGCGUCU | 2910 | 707-727 | AGACGCUGAUGGG UUCCUUGAGC | 3270 | 705-727 |
| AD-1962801 | AAGGAACCCAUC AGCGUCAGU | 2911 | 709-729 | ACUGACGCUGAUG GGUUCCUUGA | 3271 | 707-729 |
| AD-1962808 | CCAUCAGCGUCA GCAGCGAGU | 2912 | 716-736 | ACUCGCUGCUGAC GCUGAUGGGU | 3272 | 714-736 |
| AD-1962836 | GAAAUUCCGUAA ACUUAACUU | 2913 | 744-764 | AAGUUAAGUUUAC GGAAUUUCAA | 3273 | 742-764 |
| AD-1962837 | AAAUUCCGUAAA CUUAACUUU | 2914 | 745-765 | AAAGUUAAGUUUA CGGAAUUUCA | 3274 | 743-765 |
| AD-1962842 | CCGUAAACUUAA CUUCAAUGU | 2915 | 750-770 | ACAUUGAAGUUAA GUUUACGGAA | 3275 | 748-770 |
| AD-1962843 | CGUAAACUUAAC UUCAAUGGU | 2916 | 751-771 | ACCAUUGAAGUUA AGUUUACGGA | 3276 | 749-771 |
| AD-1962860 | AACUGAUGGUGG ACAACUGGU | 2917 | 788-808 | ACCAGUUGUCCAC CAUCAGUUCU | 3277 | 786-808 |
| AD-1962885 | CUCAGCCACUGA AGAACAGGU | 2918 | 815-835 | ACCUGUUCUUCAG UGGCUGAGCU | 3278 | 813-835 |
| AD-1962897 | AGAACAGGCAAA UCAAAGCUU | 2919 | 827-847 | AAGCUUUGAUUUG CCUGUUCUUC | 3279 | 825-847 |
| AD-1962902 | AGGCAAAUCAAA GCUUCCUUU | 2920 | 832-852 | AAAGGAAGCUUUG AUUUGCCUGU | 3280 | 830-852 |
| AD-1962910 | CAAAGCUUCCUU CAAAUAAGU | 2921 | 840-860 | ACUUAUUUGAAGG AAGCUUUGAU | 3281 | 838-860 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962911 | AAAGCUUCCUUC AAAUAAGAU | 2922 | 841-861 | AUCUUAUUUGAAG GAAGCUUUGA | 3282 | 839-861 |
| AD-1962912 | AAGCUUCCUUCA AAUAAGAUU | 2923 | 842-862 | AAUCUUAUUUGAA GGAAGCUUUG | 3283 | 840-862 |
| AD-1962913 | AGCUUCCUUCAA AUAAGAUGU | 2924 | 843-863 | ACAUCUUAUUUGA AGGAAGCUUU | 3284 | 841-863 |
| AD-1962914 | GCUUCCUUCAAA UAAGAUGGU | 2925 | 844-864 | ACCAUCUUAUUUG AAGGAAGCUU | 3285 | 842-864 |
| AD-1962941 | GUCUGUAUCCAA AUAAUGAAU | 2926 | 871-891 | AUUCAUUAUUUGG AUACAGACUA | 3286 | 869-891 |
| AD-1962945 | GUAUCCAAAUAA UGAAUCUUU | 2927 | 875-895 | AAAGAUUCAUUAU UUGGAUACAG | 3287 | 873-895 |
| AD-1962947 | AUCCAAAUAAUG AAUCUUCGU | 2928 | 877-897 | ACGAAGAUUCAUU AUUUGGAUAC | 3288 | 875-897 |
| AD-1962948 | UCCAAAUAAUGA AUCUUCGGU | 2929 | 878-898 | ACCGAAGAUUCAU UAUUUGGAUA | 3289 | 876-898 |
| AD-1962949 | CCAAAUAAUGAA UCUUCGGGU | 2930 | 879-899 | ACCCGAAGAUUCA UUAUUUGGAU | 3290 | 877-899 |
| AD-1962950 | CAAAUAAUGAAU CUUCGGGUU | 2931 | 880-900 | AACCCGAAGAUUC AUUAUUUGGA | 3291 | 878-900 |
| AD-1962951 | AAAUAAUGAAUC UUCGGGUGU | 2932 | 881-901 | ACACCCGAAGAUU CAUUAUUUGG | 3292 | 879-901 |
| AD-1962952 | AAUAAUGAAUCU UCGGGUGUU | 2933 | 882-902 | AACACCCGAAGAU UCAUUAUUUG | 3293 | 880-902 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1962953 | AUAAUGAAUCUU CGGGUGUUU | 2934 | 883-903 | AAACACCCGAAGA UUCAUUAUUU | 3294 | 881-903 |
| AD-1962954 | UAAUGAAUCUUC GGGUGUUUU | 2935 | 884-904 | AAAACACCCGAAG AUUCAUUAUU | 3295 | 882-904 |
| AD-1962957 | UGAAUCUUCGGG UGUUUCCCU | 2936 | 887-907 | AGGGAAACACCCG AAGAUUCAUU | 3296 | 885-907 |
| AD-1962958 | GAAUCUUCGGGU GUUUCCCUU | 2937 | 888-908 | AAGGGAAACACCC GAAGAUUCAU | 3297 | 886-908 |
| AD-1962984 | AAGCACAGAUCU ACCUUGGUU | 2938 | 914-934 | AACCAAGGUAGAU CUGUGCUUAG | 3298 | 912-934 |
| AD-1962985 | AGCACAGAUCUA CCUUGGUGU | 2939 | 915-935 | ACACCAAGGUAGA UCUGUGCUUA | 3299 | 913-935 |
| AD-1962986 | GCACAGAUCUAC CUUGGUGAU | 2940 | 916-936 | AUCACCAAGGUAG AUCUGUGCUU | 3300 | 914-936 |
| AD-1962990 | AGAUCUACCUUG GUGAUUUGU | 2941 | 920-940 | ACAAAUCACCAAG GUAGAUCUGU | 3301 | 918-940 |
| AD-1963114 | ACAACUGCUGUG GCUGGUUGU | 2942 | 1039-1059 | ACAACCAGCCACA GCAGUUGUGU | 3302 | 1037-1059 |
| AD-1963115 | CAACUGCUGUGG CUGGUUGGU | 2943 | 1040-1060 | ACCAACCAGCCAC AGCAGUUGUG | 3303 | 1038-1060 |
| AD-1963118 | CUGCUGUGGCUG GUUGGUGCU | 2944 | 1043-1063 | AGCACCAACCAGC CACAGCAGUU | 3304 | 1041-1063 |
| AD-1963123 | GUGGCUGGUUGG UGCUUUGUU | 2945 | 1048-1068 | AACAAAGCACCAA CCAGCCACAG | 3305 | 1046-1068 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963125 | GGCUGGUUGGUG CUUUGUUUU | 2946 | 1050-1070 | AAAACAAAGCACC AACCAGCCAC | 3306 | 1048-1070 |
| AD-1963126 | GCUGGUUGGUGC UUUGUUUAU | 2947 | 1051-1071 | AUAAACAAAGCAC CAACCAGCCA | 3307 | 1049-1071 |
| AD-1963127 | CUGGUUGGUGCU UUGUUUAUU | 2948 | 1052-1072 | AAUAAACAAAGCA CCAACCAGCC | 3308 | 1050-1072 |
| AD-1963128 | UGGUUGGUGCUU UGUUUAUGU | 2949 | 1053-1073 | ACAUAAACAAAGC ACCAACCAGC | 3309 | 1051-1073 |
| AD-1963129 | GGUUGGUGCUUU GUUUAUGGU | 2950 | 1054-1074 | ACCAUAAACAAAG CACCAACCAG | 3310 | 1052-1074 |
| AD-1963130 | GUUGGUGCUUUG UUUAUGGUU | 2951 | 1055-1075 | AACCAUAAACAAA GCACCAACCA | 3311 | 1053-1075 |
| AD-1963131 | UUGGUGCUUUGU UUAUGGUAU | 2952 | 1056-1076 | AUACCAUAAACAA AGCACCAACC | 3312 | 1054-1076 |
| AD-1963132 | UGGUGCUUUGUU UAUGGUAGU | 2953 | 1057-1077 | ACUACCAUAAACA AAGCACCAAC | 3313 | 1055-1077 |
| AD-1963135 | UGCUUUGUUUAU GGUAGUAGU | 2954 | 1060-1080 | ACUACUACCAUAA ACAAAGCACC | 3314 | 1058-1080 |
| AD-1963138 | UUUGUUUAUGGU AGUAGUUUU | 2955 | 1063-1083 | AAAACUACUACCA UAAACAAAGC | 3315 | 1061-1083 |
| AD-1963139 | UUGUUUAUGGUA GUAGUUUUU | 2956 | 1064-1084 | AAAAACUACUACC AUAAACAAAG | 3316 | 1062-1084 |
| AD-1963140 | GUUUAUGGUAGU AGUUUUUCU | 2957 | 1066-1086 | AGAAAAACUACUA CCAUAAACAA | 3317 | 1064-1086 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963141 | UUUAUGGUAGUA GUUUUUCUU | 2958 | 1067-1087 | AAGAAAAACUACU ACCAUAAACA | 3318 | 1065-1087 |
| AD-1963142 | UUAUGGUAGUAG UUUUUCUGU | 2959 | 1068-1088 | ACAGAAAAACUAC UACCAUAAAC | 3319 | 1066-1088 |
| AD-1963143 | UAUGGUAGUAGU UUUUCUGUU | 2960 | 1069-1089 | AACAGAAAAACUA CUACCAUAAA | 3320 | 1067-1089 |
| AD-1963144 | AUGGUAGUAGUU UUUCUGUAU | 2961 | 1070-1090 | AUACAGAAAAACU ACUACCAUAA | 3321 | 1068-1090 |
| AD-1963148 | UAGUAGUUUUUC UGUAACACU | 2962 | 1074-1094 | AGUGUUACAGAAA AACUACUACC | 3322 | 1072-1094 |
| AD-1963202 | AGAAUAAAGUAC CUUGACUUU | 2963 | 1114-1134 | AAAGUCAAGGUAC UUUAUUCUUA | 3323 | 1112-1134 |
| AD-1963204 | AAUAAAGUACCU UGACUUUGU | 2964 | 1116-1136 | ACAAAGUCAAGGU ACUUUAUUCU | 3324 | 1114-1136 |
| AD-1963205 | AUAAAGUACCUU GACUUUGUU | 2965 | 1117-1137 | AACAAAGUCAAGG UACUUUAUUC | 3325 | 1115-1137 |
| AD-1963206 | UAAAGUACCUUG ACUUUGUUU | 2966 | 1118-1138 | AAACAAAGUCAAG GUACUUUAUU | 3326 | 1116-1138 |
| AD-1963207 | AAAGUACCUUGA CUUUGUUCU | 2967 | 1119-1139 | AGAACAAAGUCAA GGUACUUUAU | 3327 | 1117-1139 |
| AD-1963208 | AAGUACCUUGAC UUUGUUCAU | 2968 | 1120-1140 | AUGAACAAAGUCA AGGUACUUUA | 3328 | 1118-1140 |
| AD-1963211 | UACCUUGACUUU GUUCACAGU | 2969 | 1123-1143 | ACUGUGAACAAAG UCAAGGUACU | 3329 | 1121-1143 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963218 | ACUUUGUUCACA GCAUGUAGU | 2970 | 1130-1150 | ACUACAUGCUGUG AACAAAGUCA | 3330 | 1128-1150 |
| AD-1963219 | CUUUGUUCACAG CAUGUAGGU | 2971 | 1131-1151 | ACCUACAUGCUGU GAACAAAGUC | 3331 | 1129-1151 |
| AD-1963220 | UUUGUUCACAGC AUGUAGGGU | 2972 | 1132-1152 | ACCCUACAUGCUG UGAACAAAGU | 3332 | 1130-1152 |
| AD-1963221 | UUGUUCACAGCA UGUAGGGUU | 2973 | 1133-1153 | AACCCUACAUGCU GUGAACAAAG | 3333 | 1131-1153 |
| AD-1963222 | UGUUCACAGCAU GUAGGGUGU | 2974 | 1134-1154 | ACACCCUACAUGC UGUGAACAAA | 3334 | 1132-1154 |
| AD-1963225 | UCACAGCAUGUA GGGUGAUGU | 2975 | 1137-1157 | ACAUCACCCUACA UGCUGUGAAC | 3335 | 1135-1157 |
| AD-1963237 | CAACGGACCUGA GCACUGGCU | 2976 | 105-125 | AGCCAGTGCUCAG GUCCGUUGUG | 3336 | 103-125 |
| AD-1963239 | ACGGACCUGAGC ACUGGCAUU | 2977 | 107-127 | AAUGCCAGUGCUC AGGUCCGUUG | 3337 | 105-127 |
| AD-1963244 | CCUGAGCACUGG CAUAAGGAU | 2978 | 112-132 | AUCCTUAUGCCAG UGCUCAGGUC | 3338 | 110-132 |
| AD-1963245 | CUGAGCACUGGC AUAAGGACU | 2979 | 113-133 | AGUCCUTAUGCCA GUGCUCAGGU | 3339 | 111-133 |
| AD-1963246 | UGAGCACUGGCA UAAGGACUU | 2980 | 114-134 | AAGUCCTUAUGCC AGUGCUCAGG | 3340 | 112-134 |
| AD-1963249 | GCACUGGCAUAA GGACUUCCU | 2981 | 117-137 | AGGAAGTCCUUAU GCCAGUGCUC | 3341 | 115-137 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963287 | GACUAAAAUGCU GCUUUUAAU | 2982 | 1173-1193 | AUUAAAAGCAGCA UUUUAGUCAA | 3342 | 1171-1193 |
| AD-1963288 | ACUAAAAUGCUG CUUUUAAAU | 2983 | 1174-1194 | AUUUAAAAGCAGC AUUUUAGUCA | 3343 | 1172-1194 |
| AD-1963289 | CUAAAAUGCUGC UUUUAAAAU | 2984 | 1175-1195 | AUUUUAAAAGCAG CAUUUUAGUC | 3344 | 1173-1195 |
| AD-1963290 | UAAAAUGCUGCU UUUAAAACU | 2985 | 1176-1196 | AGUUUUAAAAGCA GCAUUUUAGU | 3345 | 1174-1196 |
| AD-1963295 | UGCUGCUUUUAA AACAUAGGU | 2986 | 1181-1201 | ACCUAUGUUUUAA AAGCAGCAUU | 3346 | 1179-1201 |
| AD-1963296 | GCUGCUUUUAAA ACAUAGGAU | 2987 | 1182-1202 | AUCCUAUGUUUUA AAAGCAGCAU | 3347 | 1180-1202 |
| AD-1963302 | UUUAAAACAUAG GAAAGUAGU | 2988 | 1188-1208 | ACUACUUUCCUAU GUUUUAAAAG | 3348 | 1186-1208 |
| AD-1963306 | CUGUUGACAUCG ACACUCAUU | 2989 | 164-184 | AAUGAGTGUCGAU GUCAACAGGG | 3349 | 162-184 |
| AD-1963308 | GUUGACAUCGAC ACUCAUACU | 2990 | 166-186 | AGUATGAGUGUCG AUGUCAACAG | 3350 | 164-186 |
| AD-1963311 | GACAUCGACACU CAUACAGCU | 2991 | 169-189 | AGCUGUAUGAGUG UCGAUGUCAA | 3351 | 167-189 |
| AD-1963312 | ACAUCGACACUC AUACAGCCU | 2992 | 170-190 | AGGCTGTAUGAGU GUCGAUGUCA | 3352 | 168-190 |
| AD-1963314 | AUCGACACUCAU ACAGCCAAU | 2993 | 172-192 | AUUGGCTGUAUGA GUGUCGAUGU | 3353 | 170-192 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963318 | ACACUCAUACAG CCAAGUAUU | 2994 | 176-196 | AAUACUTGGCUGU AUGAGUGUCG | 3354 | 174-196 |
| AD-1963321 | CUCAUACAGCCA AGUAUGACU | 2995 | 179-199 | AGUCAUACUUGGC UGUAUGAGUG | 3355 | 177-199 |
| AD-1963322 | UCAUACAGCCAA GUAUGACCU | 2996 | 180-200 | AGGUCATACUUGG CUGUAUGAGU | 3356 | 178-200 |
| AD-1963323 | CAUACAGCCAAG UAUGACCCU | 2997 | 181-201 | AGGGTCAUACUUG GCUGUAUGAG | 3357 | 179-201 |
| AD-1963325 | UACAGCCAAGUA UGACCCUUU | 2998 | 183-203 | AAAGGGTCAUACU UGGCUGUAUG | 3358 | 181-203 |
| AD-1963329 | GCCAAGUAUGAC CCUUCCCUU | 2999 | 187-207 | AAGGGAAGGGUCA UACUUGGCUG | 3359 | 185-207 |
| AD-1963375 | GAUAAAUUGAGC UAGUUAAGU | 3000 | 1236-1256 | ACUUAACUAGCUC AAUUUAUCUU | 3360 | 1234-1256 |
| AD-1963376 | AUAAAUUGAGCU AGUUAAGGU | 3001 | 1237-1257 | ACCUUAACUAGCU CAAUUUAUCU | 3361 | 1235-1257 |
| AD-1963377 | UAAAUUGAGCUA GUUAAGGCU | 3002 | 1238-1258 | AGCCUUAACUAGC UCAAUUUAUC | 3362 | 1236-1258 |
| AD-1963384 | GUAUGACCCUUC CCUGAAGCU | 3003 | 192-212 | AGCUTCAGGGAAG GGUCAUACUU | 3363 | 190-212 |
| AD-1963386 | CUGUCUGUUUCC UAUGAUCAU | 3004 | 214-234 | AUGATCAUAGGAA ACAGACAGGG | 3364 | 212-234 |
| AD-1963388 | GUCUGUUUCCUA UGAUCAAGU | 3005 | 216-236 | ACUUGATCAUAGG AAACAGACAG | 3365 | 214-236 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963389 | UCUGUUUCCUAU GAUCAAGCU | 3006 | 217-237 | AGCUTGAUCAUAG GAAACAGACA | 3366 | 215-237 |
| AD-1963391 | UGUUUCCUAUGA UCAAGCAAU | 3007 | 219-239 | AUUGCUTGAUCAU AGGAAACAGA | 3367 | 217-239 |
| AD-1963392 | GUUUCCUAUGAU CAAGCAACU | 3008 | 220-240 | AGUUGCTUGAUCA UAGGAAACAG | 3368 | 218-240 |
| AD-1963395 | UCCUAUGAUCAA GCAACUUCU | 3009 | 223-243 | AGAAGUTGCUUGA UCAUAGGAAA | 3369 | 221-243 |
| AD-1963410 | AGCUAGUUAAGG CAAAUCAGU | 3010 | 1245-1265 | ACUGAUUUGCCUU AACUAGCUCA | 3370 | 1243-1265 |
| AD-1963411 | GCUAGUUAAGGC AAAUCAGGU | 3011 | 1246-1266 | ACCUGAUUUGCCU UAACUAGCUC | 3371 | 1244-1266 |
| AD-1963417 | UAAGGCAAAUCA GGUAAAAUU | 3012 | 1252-1272 | AAUUUUACCUGAU UUGCCUUAAC | 3372 | 1250-1272 |
| AD-1963419 | AGGCAAAUCAGG UAAAAUAGU | 3013 | 1254-1274 | ACUAUUUUACCUG AUUUGCCUUA | 3373 | 1252-1274 |
| AD-1963420 | GGCAAAUCAGGU AAAAUAGUU | 3014 | 1255-1275 | AACUAUUUUACCU GAUUUGCCUU | 3374 | 1253-1275 |
| AD-1963421 | GCAAAUCAGGUA AAAUAGUCU | 3015 | 1256-1276 | AGACUAUUUUACC UGAUUUGCCU | 3375 | 1254-1276 |
| AD-1963430 | GUAAAAUAGUCA UGAUUCUAU | 3016 | 1265-1285 | AUAGAAUCAUGAC UAUUUUACCU | 3376 | 1263-1285 |
| AD-1963431 | UAAAAUAGUCAU GAUUCUAUU | 3017 | 1266-1286 | AAUAGAAUCAUGA CUAUUUUACC | 3377 | 1264-1286 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963432 | AAAAUAGUCAUG AUUCUAUGU | 3018 | 1267-1287 | ACAUAGAAUCAUG ACUAUUUUAC | 3378 | 1265-1287 |
| AD-1963437 | AGUCAUGAUUCU AUGUAAUGU | 3019 | 1272-1292 | ACAUUACAUAGAA UCAUGACUAU | 3379 | 1270-1292 |
| AD-1963438 | GUCAUGAUUCUA UGUAAUGUU | 3020 | 1273-1293 | AACAUUACAUAGA AUCAUGACUA | 3380 | 1271-1293 |
| AD-1963439 | UCAUGAUUCUAU GUAAUGUAU | 3021 | 1274-1294 | AUACAUUACAUAG AAUCAUGACU | 3381 | 1272-1294 |
| AD-1963464 | CCCUGAGGAUCC UCAACAAUU | 3022 | 242-262 | AAUUGUTGAGGAU CCUCAGGGAA | 3382 | 240-262 |
| AD-1963465 | CCUGAGGAUCCU CAACAAUGU | 3023 | 243-263 | ACAUTGUTGAGGA UCCUCAGGGA | 3383 | 241-263 |
| AD-1963468 | GAGGAUCCUCAA CAAUGGUCU | 3024 | 246-266 | AGACCATUGUUGA GGAUCCUCAG | 3384 | 244-266 |
| AD-1963469 | AGGAUCCUCAAC AAUGGUCAU | 3025 | 247-267 | AUGACCAUUGUUG AGGAUCCUCA | 3385 | 245-267 |
| AD-1963539 | UGCUUUCAACGU GGAGUUUGU | 3026 | 267-287 | ACAAACTCCACGU UGAAAGCAUG | 3386 | 265-287 |
| AD-1963543 | UUCAACGUGGAG UUUGAUGAU | 3027 | 271-291 | AUCATCAAACUCC ACGUUGAAAG | 3387 | 269-291 |
| AD-1963545 | CAACGUGGAGUU UGAUGACUU | 3028 | 273-293 | AAGUCATCAAACU CCACGUUGAA | 3388 | 271-293 |
| AD-1963546 | AACGUGGAGUUU GAUGACUCU | 3029 | 274-294 | AGAGTCAUCAAAC UCCACGUUGA | 3389 | 272-294 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963548 | CGUGGAGUUUGA UGACUCUCU | 3030 | 276-296 | AGAGAGTCAUCAA ACUCCACGUU | 3390 | 274-296 |
| AD-1963550 | UGGAGUUUGAUG ACUCUCAGU | 3031 | 278-298 | ACUGAGAGUCAUC AAACUCCACG | 3391 | 276-298 |
| AD-1963552 | GAGUUUGAUGAC UCUCAGGAU | 3032 | 280-300 | AUCCTGAGAGUCA UCAAACUCCA | 3392 | 278-300 |
| AD-1963554 | GUUUGAUGACUC UCAGGACAU | 3033 | 282-302 | AUGUCCTGAGAGU CAUCAAACUC | 3393 | 280-302 |
| AD-1963557 | UGAUGACUCUCA GGACAAAGU | 3034 | 285-305 | ACUUTGTCCUGAG AGUCAUCAAA | 3394 | 283-305 |
| AD-1963582 | UAAUUAGAGUUG UGAUACAGU | 3035 | 1460-1480 | ACUGUAUCACAAC UCUAAUUAUA | 3395 | 1458-1480 |
| AD-1963590 | GUUGUGAUACAG AGUAUAUUU | 3036 | 1468-1488 | AAAUAUACUCUGU AUCACAACUC | 3396 | 1466-1488 |
| AD-1963591 | UUGUGAUACAGA GUAUAUUUU | 3037 | 1469-1489 | AAAAUAUACUCUG UAUCACAACU | 3397 | 1467-1489 |
| AD-1963592 | UGUGAUACAGAG UAUAUUUCU | 3038 | 1470-1490 | AGAAAUAUACUCU GUAUCACAAC | 3398 | 1468-1490 |
| AD-1963609 | AUGACUCUCAGG ACAAAGCAU | 3039 | 287-307 | AUGCTUTGUCCUG AGAGUCAUCA | 3399 | 285-307 |
| AD-1963610 | UGACUCUCAGGA CAAAGCAGU | 3040 | 288-308 | ACUGCUTUGUCCU GAGAGUCAUC | 3400 | 286-308 |
| AD-1963618 | CCAUUCAGACAA UAUAUCAUU | 3041 | 1489-1509 | AAUGAUAUAUUGU CUGAAUGGAA | 3401 | 1487-1509 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963719 | ACUUCACUUGGU UCACUGGAU | 3042 | 426-446 | AUCCAGTGAACCA AGUGAAGUUC | 3402 | 424-446 |
| AD-1963721 | UUCACUUGGUUC ACUGGAACU | 3043 | 428-448 | AGUUCCAGUGAAC CAAGUGAAGU | 3403 | 426-448 |
| AD-1963733 | GAUUUUGGGAAA GCUGUGCAU | 3044 | 460-480 | AUGCACAGCUUUC CCAAAAUCCC | 3404 | 458-480 |
| AD-1963735 | UUUUGGGAAAGC UGUGCAGCU | 3045 | 462-482 | AGCUGCACAGCUU UCCCAAAAUC | 3405 | 460-482 |
| AD-1963738 | UGGGAAAGCUGU GCAGCAACU | 3046 | 465-485 | AGUUGCTGCACAG CUUUCCCAAA | 3406 | 463-485 |
| AD-1963762 | UGGACUGGCCGU UCUAGGUAU | 3047 | 489-509 | AUACCUAGAACGG CCAGUCCAUC | 3407 | 487-509 |
| AD-1963763 | GGACUGGCCGUU CUAGGUAUU | 3048 | 490-510 | AAUACCTAGAACG GCCAGUCCAU | 3408 | 488-510 |
| AD-1963776 | UAGGUAUUUUUU UGAAGGUUU | 3049 | 503-523 | AAACCUTCAAAAA AAUACCUAGA | 3409 | 501-523 |
| AD-1963780 | UAUUUUUUUGAA GGUUGGCAU | 3050 | 507-527 | AUGCCAACCUUCA AAAAAAUACC | 3410 | 505-527 |
| AD-1963781 | AUUUUUUUGAAG GUUGGCAGU | 3051 | 508-528 | ACUGCCAACCUUC AAAAAAAUAC | 3411 | 506-528 |
| AD-1963785 | UUUUGAAGGUUG GCAGCGCUU | 3052 | 512-532 | AAGCGCTGCCAAC CUUCAAAAAA | 3412 | 510-532 |
| AD-1963790 | AAGGUUGGCAGC GCUAAACCU | 3053 | 517-537 | AGGUTUAGCGCUG CCAACCUUCA | 3413 | 515-537 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963814 | CUUCAGAAAGUU GUUGAUGUU | 3054 | 541-561 | AACAUCAACAACU UUCUGAAGGC | 3414 | 539-561 |
| AD-1963816 | UCAGAAAGUUGU UGAUGUGCU | 3055 | 543-563 | AGCACAUCAACAA CUUUCUGAAG | 3415 | 541-563 |
| AD-1963817 | CAGAAAGUUGUU GAUGUGCUU | 3056 | 544-564 | AAGCACAUCAACA ACUUUCUGAA | 3416 | 542-564 |
| AD-1963819 | GAAAGUUGUUGA UGUGCUGGU | 3057 | 546-566 | ACCAGCACAUCAA CAACUUUCUG | 3417 | 544-566 |
| AD-1963839 | AUUCCAUUAAAA CAAAGGGCU | 3058 | 566-586 | AGCCCUTUGUUUU AAUGGAAUCC | 3418 | 564-586 |
| AD-1963840 | UUCCAUUAAAAC AAAGGGCAU | 3059 | 567-587 | AUGCCCTUUGUUU UAAUGGAAUC | 3419 | 565-587 |
| AD-1963845 | UUAAAACAAAGG GCAAGAGUU | 3060 | 572-592 | AACUCUTGCCCUU UGUUUUAAUG | 3420 | 570-592 |
| AD-1963846 | UAAAACAAAGGG CAAGAGUGU | 3061 | 573-593 | ACACTCTUGCCCUU UGUUUUAAU | 3421 | 571-593 |
| AD-1963848 | AAACAAAGGGCA AGAGUGCUU | 3062 | 575-595 | AAGCACTCUUGCC CUUUGUUUUA | 3422 | 573-595 |
| AD-1963850 | ACAAAGGGCAAG AGUGCUGAU | 3063 | 577-597 | AUCAGCACUCUUG CCCUUUGUUU | 3423 | 575-597 |
| AD-1963853 | AAGGGCAAGAGU GCUGACUUU | 3064 | 580-600 | AAAGTCAGCACUC UUGCCCUUUG | 3424 | 578-600 |
| AD-1963855 | GGGCAAGAGUGC UGACUUCAU | 3065 | 582-602 | AUGAAGTCAGCAC UCUUGCCCUU | 3425 | 580-602 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963857 | GCAAGAGUGCUG ACUUCACUU | 3066 | 584-604 | AAGUGAAGUCAGC ACUCUUGCCC | 3426 | 582-604 |
| AD-1963858 | CAAGAGUGCUGA CUUCACUAU | 3067 | 585-605 | AUAGTGAAGUCAG CACUCUUGCC | 3427 | 583-605 |
| AD-1963860 | AGAGUGCUGACU UCACUAACU | 3068 | 587-607 | AGUUAGTGAAGUC AGCACUCUUG | 3428 | 585-607 |
| AD-1963863 | GUGCUGACUUCA CUAACUUCU | 3069 | 590-610 | AGAAGUTAGUGAA GUCAGCACUC | 3429 | 588-610 |
| AD-1963864 | UGCUGACUUCAC UAACUUCGU | 3070 | 591-611 | ACGAAGTUAGUGA AGUCAGCACU | 3430 | 589-611 |
| AD-1963870 | CUUCACUAACUU CGAUCCUCU | 3071 | 597-617 | AGAGGATCGAAGU UAGUGAAGUC | 3431 | 595-617 |
| AD-1963871 | UUCACUAACUUC GAUCCUCGU | 3072 | 598-618 | ACGAGGAUCGAAG UUAGUGAAGU | 3432 | 596-618 |
| AD-1963893 | GCCUCCUUCCUG AAUCCUUGU | 3073 | 620-640 | ACAAGGAUUCAGG AAGGAGGCCA | 3433 | 618-640 |
| AD-1963896 | UCCUUCCUGAAU CCUUGGAUU | 3074 | 623-643 | AAUCCAAGGAUUC AGGAAGGAGG | 3434 | 621-643 |
| AD-1963920 | GGACCUACCCAG GCUCACUGU | 3075 | 647-667 | ACAGTGAGCCUGG GUAGGUCCAG | 3435 | 645-667 |
| AD-1963922 | ACCUACCCAGGC UCACUGACU | 3076 | 649-669 | AGUCAGTGAGCCU GGGUAGGUCC | 3436 | 647-669 |
| AD-1963924 | CUACCCAGGCUC ACUGACCAU | 3077 | 651-671 | AUGGTCAGUGAGC CUGGGUAGGU | 3437 | 649-671 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963926 | ACCCAGGCUCAC UGACCACCU | 3078 | 653-673 | AGGUGGTCAGUGA GCCUGGGUAG | 3438 | 651-673 |
| AD-1963927 | CUCCUCUUCUGG AAUGUGUGU | 3079 | 674-694 | ACACACAUUCCAG AAGAGGAGGG | 3439 | 672-694 |
| AD-1963929 | CCUCUUCUGGAA UGUGUGACU | 3080 | 676-696 | AGUCACACAUUCC AGAAGAGGAG | 3440 | 674-696 |
| AD-1963931 | UCUUCUGGAAUG UGUGACCUU | 3081 | 678-698 | AAGGTCACACAUU CCAGAAGAGG | 3441 | 676-698 |
| AD-1963933 | UUCUGGAAUGUG UGACCUGGU | 3082 | 680-700 | ACCAGGTCACACA UUCCAGAAGA | 3442 | 678-700 |
| AD-1963936 | UGGAAUGUGUGA CCUGGAUUU | 3083 | 683-703 | AAAUCCAGGUCAC ACAUUCCAGA | 3443 | 681-703 |
| AD-1963941 | UGUGUGACCUGG AUUGUGCUU | 3084 | 688-708 | AAGCACAAUCCAG GUCACACAUU | 3444 | 686-708 |
| AD-1963943 | UGUGACCUGGAU UGUGCUCAU | 3085 | 690-710 | AUGAGCACAAUCC AGGUCACACA | 3445 | 688-710 |
| AD-1963946 | GACCUGGAUUGU GCUCAAGGU | 3086 | 693-713 | ACCUTGAGCACAA UCCAGGUCAC | 3446 | 691-713 |
| AD-1963948 | CCUGGAUUGUGC UCAAGGAAU | 3087 | 695-715 | AUUCCUTGAGCAC AAUCCAGGUC | 3447 | 693-715 |
| AD-1963949 | CUGGAUUGUGCU CAAGGAACU | 3088 | 696-716 | AGUUCCTGAGCA CAAUCCAGGU | 3448 | 694-716 |
| AD-1963952 | GAUUGUGCUCAA GGAACCCAU | 3089 | 699-719 | AUGGGUTCCUUGA GCACAAUCCA | 3449 | 697-719 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1963953 | AUUGUGCUCAAG GAACCCAUU | 3090 | 700-720 | AAUGGGTUCCUUG AGCACAAUCC | 3450 | 698-720 |
| AD-1963957 | UGCUCAAGGAAC CCAUCAGCU | 3091 | 704-724 | AGCUGATGGGUUC CUUGAGCACA | 3451 | 702-724 |
| AD-1963958 | GCUCAAGGAACC CAUCAGCGU | 3092 | 705-725 | ACGCTGAUGGGUU CCUUGAGCAC | 3452 | 703-725 |
| AD-1963960 | UCAAGGAACCCA UCAGCGUCU | 3093 | 707-727 | AGACGCTGAUGGG UUCCUUGAGC | 3453 | 705-727 |
| AD-1963964 | GGAACCCAUCAG CGUCAGCAU | 3094 | 711-731 | AUGCTGACGCUGA UGGGUUCCUU | 3454 | 709-731 |
| AD-1963966 | AACCCAUCAGCG UCAGCAGCU | 3095 | 713-733 | AGCUGCTGACGCU GAUGGGUUCC | 3455 | 711-733 |
| AD-1963995 | UUGAAAUUCCGU AAACUUAAU | 3096 | 742-762 | AUUAAGTUUACGG AAUUUCAACA | 3456 | 740-762 |
| AD-1963997 | GAAAUUCCGUAA ACUUAACUU | 3097 | 744-764 | AAGUTAAGUUUAC GGAAUUUCAA | 3457 | 742-764 |
| AD-1963999 | AAUUCCGUAAAC UUAACUUCU | 3098 | 746-766 | AGAAGUTAAGUUU ACGGAAUUUC | 3458 | 744-766 |
| AD-1964000 | AUUCCGUAAACU UAACUUCAU | 3099 | 747-767 | AUGAAGTUAAGUU UACGGAAUUU | 3459 | 745-767 |
| AD-1964002 | UCCGUAAACUUA ACUUCAAUU | 3100 | 749-769 | AAUUGAAGUUAAG UUUACGGAAU | 3460 | 747-769 |
| AD-1964003 | CCGUAAACUUAA CUUCAAUGU | 3101 | 750-770 | ACAUTGAAGUUAA GUUUACGGAA | 3461 | 748-770 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964016 | CGAAGAACUGAU GGUGGACAU | 3102 | 783-803 | AUGUCCACCAUCA GUUCUUCGGG | 3462 | 781-803 |
| AD-1964019 | AGAACUGAUGGU GGACAACUU | 3103 | 786-806 | AAGUTGTCCACCA UCAGUUCUUC | 3463 | 784-806 |
| AD-1964021 | AACUGAUGGUGG ACAACUGGU | 3104 | 788-808 | ACCAGUTGUCCAC CAUCAGUUCU | 3464 | 786-808 |
| AD-1964022 | ACUGAUGGUGGA CAACUGGCU | 3105 | 789-809 | AGCCAGTUGUCCA CCAUCAGUUC | 3465 | 787-809 |
| AD-1964024 | UGAUGGUGGACA ACUGGCGCU | 3106 | 791-811 | AGCGCCAGUUGUC CACCAUCAGU | 3466 | 789-811 |
| AD-1964043 | CAGCUCAGCCAC UGAAGAACU | 3107 | 812-832 | AGUUCUTCAGUGG CUGAGCUGGG | 3467 | 810-832 |
| AD-1964044 | AGCUCAGCCACU GAAGAACAU | 3108 | 813-833 | AUGUTCTUCAGUG GCUGAGCUGG | 3468 | 811-833 |
| AD-1964046 | CUCAGCCACUGA AGAACAGGU | 3109 | 815-835 | ACCUGUTCUUCAG UGGCUGAGCU | 3469 | 813-835 |
| AD-1964047 | UCAGCCACUGAA GAACAGGCU | 3110 | 816-836 | AGCCTGTUCUUCA GUGGCUGAGC | 3470 | 814-836 |
| AD-1964049 | AGCCACUGAAGA ACAGGCAAU | 3111 | 818-838 | AUUGCCTGUUCUU CAGUGGCUGA | 3471 | 816-838 |
| AD-1964053 | ACUGAAGAACAG GCAAAUCAU | 3112 | 822-842 | AUGATUTGCCUGU UCUUCAGUGG | 3472 | 820-842 |
| AD-1964054 | CUGAAGAACAGG CAAAUCAAU | 3113 | 823-843 | AUUGAUTUGCCUG UUCUUCAGUG | 3473 | 821-843 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964055 | UGAAGAACAGGC AAAUCAAAU | 3114 | 824-844 | AUUUGATUUGCCU GUUCUUCAGU | 3474 | 822-844 |
| AD-1964056 | GAAGAACAGGCA AAUCAAAGU | 3115 | 825-845 | ACUUTGAUUUGCC UGUUCUUCAG | 3475 | 823-845 |
| AD-1964058 | AGAACAGGCAAA UCAAAGCUU | 3116 | 827-847 | AAGCTUTGAUUUG CCUGUUCUUC | 3476 | 825-847 |
| AD-1964059 | GAACAGGCAAAU CAAAGCUUU | 3117 | 828-848 | AAAGCUTUGAUUU GCCUGUUCUU | 3477 | 826-848 |
| AD-1964060 | AACAGGCAAAUC AAAGCUUCU | 3118 | 829-849 | AGAAGCTUUGAUU UGCCUGUUCU | 3478 | 827-849 |
| AD-1964063 | AGGCAAAUCAAA GCUUCCUUU | 3119 | 832-852 | AAAGGAAGCUUUG AUUUGCCUGU | 3479 | 830-852 |
| AD-1964064 | GGCAAAUCAAAG CUUCCUUCU | 3120 | 833-853 | AGAAGGAAGCUUU GAUUUGCCUG | 3480 | 831-853 |
| AD-1964067 | AAAUCAAAGCUU CCUUCAAAU | 3121 | 836-856 | AUUUGAAGGAAGC UUUGAUUUGC | 3481 | 834-856 |
| AD-1964068 | AAUCAAAGCUUC CUUCAAAUU | 3122 | 837-857 | AAUUTGAAGGAAG CUUUGAUUUG | 3482 | 835-857 |
| AD-1964070 | UCAAAGCUUCCU UCAAAUAAU | 3123 | 839-859 | AUUATUTGAAGGA AGCUUUGAUU | 3483 | 837-859 |
| AD-1964072 | AAAGCUUCCUUC AAAUAAGAU | 3124 | 841-861 | AUCUTATUUGAAG GAAGCUUUGA | 3484 | 839-861 |
| AD-1964073 | AAGCUUCCUUCA AAUAAGAUU | 3125 | 842-862 | AAUCTUAUUUGAA GGAAGCUUUG | 3485 | 840-862 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964075 | GCUUCCUUCAAA UAAGAUGGU | 3126 | 844-864 | ACCATCTUAUUUG AAGGAAGCUU | 3486 | 842-864 |
| AD-1964077 | UUCCUUCAAAUA AGAUGGUCU | 3127 | 846-866 | AGACCATCUUAUU UGAAGGAAGC | 3487 | 844-866 |
| AD-1964078 | UCCUUCAAAUAA GAUGGUCCU | 3128 | 847-867 | AGGACCAUCUUAU UUGAAGGAAG | 3488 | 845-867 |
| AD-1964081 | UUCAAAUAAGAU GGUCCCAUU | 3129 | 850-870 | AAUGGGACCAUCU UAUUUGAAGG | 3489 | 848-870 |
| AD-1964102 | GUCUGUAUCCAA AUAAUGAAU | 3130 | 871-891 | AUUCAUTAUUUGG AUACAGACUA | 3490 | 869-891 |
| AD-1964103 | UCUGUAUCCAAA UAAUGAAUU | 3131 | 872-892 | AAUUCATUAUUUG GAUACAGACU | 3491 | 870-892 |
| AD-1964104 | CUGUAUCCAAAU AAUGAAUCU | 3132 | 873-893 | AGAUTCAUUAUUU GGAUACAGAC | 3492 | 871-893 |
| AD-1964106 | GUAUCCAAAUAA UGAAUCUUU | 3133 | 875-895 | AAAGAUTCAUUAU UUGGAUACAG | 3493 | 873-895 |
| AD-1964107 | UAUCCAAAUAAU GAAUCUUCU | 3134 | 876-896 | AGAAGATUCAUUA UUUGGAUACA | 3494 | 874-896 |
| AD-1964108 | AUCCAAAUAAUG AAUCUUCGU | 3135 | 877-897 | ACGAAGAUUCAUU AUUUGGAUAC | 3495 | 875-897 |
| AD-1964116 | AAUGAAUCUUCG GGUGUUUCU | 3136 | 885-905 | AGAAACACCCGAA GAUUCAUUAU | 3496 | 883-905 |
| AD-1964118 | UGAAUCUUCGGG UGUUUCCCU | 3137 | 887-907 | AGGGAAACACCCG AAGAUUCAUU | 3497 | 885-907 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964139 | UUAGCUAAGCAC AGAUCUACU | 3138 | 908-928 | AGUAGATCUGUGC UUAGCUAAAG | 3498 | 906-928 |
| AD-1964140 | UAGCUAAGCACA GAUCUACCU | 3139 | 909-929 | AGGUAGAUCUGUG CUUAGCUAAA | 3499 | 907-929 |
| AD-1964142 | GCUAAGCACAGA UCUACCUUU | 3140 | 911-931 | AAAGGUAGAUCUG UGCUUAGCUA | 3500 | 909-931 |
| AD-1964143 | CUAAGCACAGAU CUACCUUGU | 3141 | 912-932 | ACAAGGTAGAUCU GUGCUUAGCU | 3501 | 910-932 |
| AD-1964150 | CAGAUCUACCUU GGUGAUUUU | 3142 | 919-939 | AAAATCACCAAGG UAGAUCUGUG | 3502 | 917-939 |
| AD-1964229 | AAUAAAAUGUGA AGACUAGAU | 3143 | 1001-1021 | AUCUAGTCUUCAC AUUUUAUUAG | 3503 | 999-1021 |
| AD-1964267 | ACAACUGCUGUG GCUGGUUGU | 3144 | 1039-1059 | ACAACCAGCCACA GCAGUUGUGU | 3504 | 1037-1059 |
| AD-1964274 | CUGUGGCUGGUU GGUGCUUUU | 3145 | 1046-1066 | AAAAGCACCAACC AGCCACAGCA | 3505 | 1044-1066 |
| AD-1964284 | UUGGUGCUUUGU UUAUGGUAU | 3146 | 1056-1076 | AUACCATAAACAA AGCACCAACC | 3506 | 1054-1076 |
| AD-1964289 | GCUUUGUUUAUG GUAGUAGUU | 3147 | 1061-1081 | AACUACTACCAUA AACAAAGCAC | 3507 | 1059-1081 |
| AD-1964291 | UUUGUUUAUGGU AGUAGUUUU | 3148 | 1063-1083 | AAAACUACUACCA UAAACAAAGC | 3508 | 1061-1083 |
| AD-1964292 | UUGUUUAUGGUA GUAGUUUUU | 3149 | 1064-1084 | AAAAACTACUACC AUAAACAAAG | 3509 | 1062-1084 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964297 | AUGGUAGUAGUU UUUCUGUAU | 3150 | 1070-1090 | AUACAGAAAAACU ACUACCAUAA | 3510 | 1068-1090 |
| AD-1964299 | GGUAGUAGUUUU UCUGUAACU | 3151 | 1072-1092 | AGUUACAGAAAAA CUACUACCAU | 3511 | 1070-1092 |
| AD-1964301 | UAGUAGUUUUUC UGUAACACU | 3152 | 1074-1094 | AGUGTUACAGAAA AACUACUACC | 3512 | 1072-1094 |
| AD-1964302 | AGUAGUUUUUCU GUAACACAU | 3153 | 1075-1095 | AUGUGUTACAGAA AAACUACUAC | 3513 | 1073-1095 |
| AD-1964303 | GUAGUUUUUCUG UAACACAGU | 3154 | 1076-1096 | ACUGTGUACAGA AAAACUACUA | 3514 | 1074-1096 |
| AD-1964325 | AAUAAGAAUAAA GUACCUUGU | 3155 | 1110-1130 | ACAAGGTACUUUA UUCUUAUUUC | 3515 | 1108-1130 |
| AD-1964328 | AAGAAUAAAGUA CCUUGACUU | 3156 | 1113-1133 | AAGUCAAGGUACU UUAUUCUUAU | 3516 | 1111-1133 |
| AD-1964329 | AGAAUAAAGUAC CUUGACUUU | 3157 | 1114-1134 | AAAGTCAAGGUAC UUUAUUCUUA | 3517 | 1112-1134 |
| AD-1964331 | AAUAAAGUACCU UGACUUUGU | 3158 | 1116-1136 | ACAAAGTCAAGGU ACUUUAUUCU | 3518 | 1114-1136 |
| AD-1964335 | AAGUACCUUGAC UUUGUUCAU | 3159 | 1120-1140 | AUGAACAAAGUCA AGGUACUUUA | 3519 | 1118-1140 |
| AD-1964337 | GUACCUUGACUU UGUUCACAU | 3160 | 1122-1142 | AUGUGAACAAAGU CAAGGUACUU | 3520 | 1120-1142 |
| AD-1964338 | UACCUUGACUUU GUUCACAGU | 3161 | 1123-1143 | ACUGTGAACAAAG UCAAGGUACU | 3521 | 1121-1143 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964340 | CCUUGACUUUGU UCACAGCAU | 3162 | 1125-1145 | AUGCTGTGAACAA AGUCAAGGUA | 3522 | 1123-1145 |
| AD-1964342 | UUGACUUUGUUC ACAGCAUGU | 3163 | 1127-1147 | ACAUGCTGUGAAC AAAGUCAAGG | 3523 | 1125-1147 |
| AD-1964346 | CUUUGUUCACAG CAUGUAGGU | 3164 | 1131-1151 | ACCUACAUGCUGU GAACAAAGUC | 3524 | 1129-1151 |
| AD-1964353 | CACAGCAUGUAG GGUGAUGAU | 3165 | 1138-1158 | AUCATCACCCUAC AUGCUGUGAA | 3525 | 1136-1158 |
| AD-1964355 | CAGCAUGUAGGG UGAUGAGCU | 3166 | 1140-1160 | AGCUCATCACCCU ACAUGCUGUG | 3526 | 1138-1160 |
| AD-1964356 | AGCAUGUAGGGU GAUGAGCAU | 3167 | 1141-1161 | AUGCTCAUCACCC UACAUGCUGU | 3527 | 1139-1161 |
| AD-1964358 | CAUGUAGGGUGA UGAGCACUU | 3168 | 1143-1163 | AAGUGCTCAUCAC CCUACAUGCU | 3528 | 1141-1163 |
| AD-1964388 | GACUAAAAUGCU GCUUUUAAU | 3169 | 1173-1193 | AUUAAAAGCAGCA UUUUAGUCAA | 3529 | 1171-1193 |
| AD-1964392 | AAAAUGCUGCUU UUAAAACAU | 3170 | 1177-1197 | AUGUTUTAAAAGC AGCAUUUUAG | 3530 | 1175-1197 |
| AD-1964395 | AUGCUGCUUUUA AAACAUAGU | 3171 | 1180-1200 | ACUATGTUUUAAA AGCAGCAUUU | 3531 | 1178-1200 |
| AD-1964397 | GCUGCUUUUAAA ACAUAGGAU | 3172 | 1182-1202 | AUCCTATGUUUUA AAAGCAGCAU | 3532 | 1180-1202 |
| AD-1964398 | CUGCUUUUAAAA CAUAGGAAU | 3173 | 1183-1203 | AUUCCUAUGUUUU AAAAGCAGCA | 3533 | 1181-1203 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964399 | UGCUUUUAAAAC AUAGGAAAU | 3174 | 1184-1204 | AUUUCCTAUGUUU UAAAAGCAGC | 3534 | 1182-1204 |
| AD-1964402 | UUUUAAAACAUA GGAAAGUAU | 3175 | 1187-1207 | AUACTUTCCUAUG UUUUAAAAGC | 3535 | 1185-1207 |
| AD-1964407 | AAACAUAGGAAA GUAGAAUGU | 3176 | 1192-1212 | ACAUTCTACUUUC CUAUGUUUUA | 3536 | 1190-1212 |
| AD-1964428 | UUGAGUGCAAAU CCAUAGCAU | 3177 | 1213-1233 | AUGCTATGGAUUU GCACUCAACC | 3537 | 1211-1233 |
| AD-1964429 | UGAGUGCAAAUC CAUAGCACU | 3178 | 1214-1234 | AGUGCUAUGGAUU UGCACUCAAC | 3538 | 1212-1234 |
| AD-1964449 | AAGAUAAAUUGA GCUAGUUAU | 3179 | 1234-1254 | AUAACUAGCUCAA UUUAUCUUGU | 3539 | 1232-1254 |
| AD-1964450 | AGAUAAAUUGAG CUAGUUAAU | 3180 | 1235-1255 | AUUAACTAGCUCA AUUUAUCUUG | 3540 | 1233-1255 |
| AD-1964454 | AAAUUGAGCUAG UUAAGGCAU | 3181 | 1239-1259 | AUGCCUTAACUAG CUCAAUUUAU | 3541 | 1237-1259 |
| AD-1964455 | AAUUGAGCUAGU UAAGGCAAU | 3182 | 1240-1260 | AUUGCCTUAACUA GCUCAAUUUA | 3542 | 1238-1260 |
| AD-1964459 | GAGCUAGUUAAG GCAAAUCAU | 3183 | 1244-1264 | AUGATUTGCCUUA ACUAGCUCAA | 3543 | 1242-1264 |
| AD-1964462 | CUAGUUAAGGCA AAUCAGGUU | 3184 | 1247-1267 | AACCTGAUUUGCC UUAACUAGCU | 3544 | 1245-1267 |
| AD-1964464 | AGUUAAGGCAAA UCAGGUAAU | 3185 | 1249-1269 | AUUACCTGAUUUG CCUUAACUAG | 3545 | 1247-1269 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964467 | UAAGGCAAAUCA GGUAAAAUU | 3186 | 1252-1272 | AAUUTUACCUGAU UUGCCUUAAC | 3546 | 1250-1272 |
| AD-1964468 | AAGGCAAAUCAG GUAAAAUAU | 3187 | 1253-1273 | AUAUTUTACCUGA UUUGCCUUAA | 3547 | 1251-1273 |
| AD-1964471 | GCAAAUCAGGUA AAAUAGUCU | 3188 | 1256-1276 | AGACTATUUUACC UGAUUUGCCU | 3548 | 1254-1276 |
| AD-1964472 | CAAAUCAGGUAA AAUAGUCAU | 3189 | 1257-1277 | AUGACUAUUUUAC CUGAUUUGCC | 3549 | 1255-1277 |
| AD-1964473 | AAAUCAGGUAAA AUAGUCAUU | 3190 | 1258-1278 | AAUGACTAUUUUA CCUGAUUUGC | 3550 | 1256-1278 |
| AD-1964475 | AUCAGGUAAAAU AGUCAUGAU | 3191 | 1260-1280 | AUCATGACUAUUU UACCUGAUUU | 3551 | 1258-1280 |
| AD-1964477 | CAGGUAAAAUAG UCAUGAUUU | 3192 | 1262-1282 | AAAUCATGACUAU UUUACCUGAU | 3552 | 1260-1282 |
| AD-1964478 | AGGUAAAAUAGU CAUGAUUCU | 3193 | 1263-1283 | AGAATCAUGACUA UUUUACCUGA | 3553 | 1261-1283 |
| AD-1964480 | GUAAAAUAGUCA UGAUUCUAU | 3194 | 1265-1285 | AUAGAATCAUGAC UAUUUUACCU | 3554 | 1263-1285 |
| AD-1964481 | UAAAAUAGUCAU GAUUCUAUU | 3195 | 1266-1286 | AAUAGAAUCAUGA CUAUUUUACC | 3555 | 1264-1286 |
| AD-1964489 | UCAUGAUUCUAU GUAAUGUAU | 3196 | 1274-1294 | AUACAUTACAUAG AAUCAUGACU | 3556 | 1272-1294 |
| AD-1964490 | CAUGAUUCUAUG UAAUGUAAU | 3197 | 1275-1295 | AUUACATUACAUA GAAUCAUGAC | 3557 | 1273-1295 |

(continued)

| Modification | | | | | | |
|---|---|---|---|---|---|---|
| Duplex Name | Sense Sequence 5' to 3' | SEQ ID NO: | Range in NM_000067.3 | Antisense Sequence 5' to 3' | SEQ ID NO: | Range in NM_000 067.3 |
| AD-1964493 | GAUUCUAUGUAA UGUAAACCU | 3198 | 1278-1298 | AGGUTUACAUUAC AUAGAAUCAU | 3558 | 1276-1298 |
| AD-1964551 | AUGACUUUUGAA UUACAGAGU | 3199 | 1407-1427 | ACUCTGTAAUUCA AAAGUCAUUA | 3559 | 1405-1427 |
| AD-1964553 | GACUUUUGAAUU ACAGAGAUU | 3200 | 1409-1429 | AAUCTCTGUAAUU CAAAAGUCAU | 3560 | 1407-1429 |
| AD-1964578 | UAUAAUUAGAGU UGUGAUACU | 3201 | 1458-1478 | AGUATCACAACUC UAAUUAUAAC | 3561 | 1456-1478 |
| AD-1964580 | UAAUUAGAGUUG UGAUACAGU | 3202 | 1460-1480 | ACUGTATCACAAC UCUAAUUAUA | 3562 | 1458-1480 |
| AD-1964581 | AAUUAGAGUUGU GAUACAGAU | 3203 | 1461-1481 | AUCUGUAUCACAA CUCUAAUUAU | 3563 | 1459-1481 |
| AD-1964582 | AUUAGAGUUGUG AUACAGAGU | 3204 | 1462-1482 | ACUCTGTAUCACA ACUCUAAUUA | 3564 | 1460-1482 |
| AD-1964584 | UAGAGUUGUGAU ACAGAGUAU | 3205 | 1464-1484 | AUACTCTGUAUCA CAACUCUAAU | 3565 | 1462-1484 |
| AD-1964586 | GAGUUGUGAUAC AGAGUAUAU | 3206 | 1466-1486 | AUAUACTCUGUAU CACAACUCUA | 3566 | 1464-1486 |
| AD-1964588 | GUUGUGAUACAG AGUAUAUUU | 3207 | 1468-1488 | AAAUAUACUCUGU AUCACAACUC | 3567 | 1466-1488 |
| AD-1964610 | CAUUCAGACAAU AUAUCAUAU | 3208 | 1490-1510 | AUAUGATAUAUUG UCUGAAUGGA | 3568 | 1488-1510 |
| AD-1964611 | AUUCAGACAAUA UAUCAUAAU | 3209 | 1491-1511 | AUUATGAUAUAUU GUCUGAAUGG | 3569 | 1489-1511 |

Table 10. Modified Sense and Antisense Strand Sequences of CA2 dsRNA Agents with C16

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962343 | ascscug(Ahd)GfcAf CfUfggcauaagsgsu | 3570 | asCfscuuAfugccagu GfcUfcagguscsc | 3930 | GGACCUGAGCAC UGGCAUAAGGA | 2834 |
| AD-1962345 | csusgag(Chd)AfcUf GfGfcauaaggascsu | 3571 | asGfsuccUfuaugccaG fuGfcucagsgsu | 3931 | ACCUGAGCACUG GCAUAAGGACU | 2781 |
| AD-1962360 | usgsaca(Uhd)CfgAf CfAfcucauacasgsu | 3572 | asCfsuguAfugagugu CfgAfugucasasc | 3932 | GUUGACAUCGAC ACUCAUACAGC | 2640 |
| AD-1962369 | csascuc(Ahd)UfaCf AfGfccaaguausgsu | 3573 | asCfsauaCfuuggcug UfaUfgagugsusc | 3933 | GACACUCAUACA GCCAAGUAUGA | 2652 |
| AD-1962370 | ascsuca(Uhd)AfcAf GfCfcaaguaugsasu | 3574 | asUfscauAfcuuggcu GfuAfugagusgsu | 3934 | ACACUCAUACAG CCAAGUAUGAC | 2637 |
| AD-1962371 | csuscau(Ahd)CfaGf CfCfaaguaugascsu | 3575 | asGfsucaUfacuuggc UfgUfaugagsusg | 3935 | CACUCAUACAGC CAAGUAUGACC | 2691 |
| AD-1962380 | cscsaag(Uhd)AfuGf AfCfccuucccusgsu | 3576 | asCfsaggGfaagggucAfuAfcuuggscsu | 3936 | AGCCAAGUAUGA CCCUUCCCUGA | 2710 |
| AD-1962416 | csusgag(Ghd)AfuCf CfUfcaacaaugsgsu | 3577 | asCfscauUfguugagg AfuCfcucagsgsg | 3937 | CCCUGAGGAUCC UCAACAAUGGU | 2759 |
| AD-1962417 | usgsagg(Ahd)UfcCf UfCfaacaauggsusu | 3578 | asAfsccaUfuguugag GfaUfccucasgsg | 3938 | CCUGAGGAUCCU CAACAAUGGUC | 2717 |
| AD-1962418 | gsasgga(Uhd)CfcUf CfAfacaaugguscsu | 3579 | asGfsaccAfuuguuga GfgAfuccucsasg | 3939 | CUGAGGAUCCUC AACAAUGGUCA | 2703 |
| AD-1962439 | usgscuu(Uhd)CfaAf CfGfuggaguuusgsu | 3580 | asCfsaaaCfuccacguU fgAfaagcasusg | 3940 | CAUGCUUUCAAC GUGGAGUUUGA | 2625 |
| AD-1962440 | gscsuuu(Chd)AfaCf GfUfggaguuugsasu | 3581 | asUfscaaAfcuccacgU fuGfaaagcsasu | 3941 | AUGCUUUCAACG UGGAGUUUGAU | 2624 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962441 | csusuuc(Ahd)AfcGf UfGfgaguuugasusu | 3582 | asAfsucaAfacuccacG fuUfgaaagscsa | 3942 | UGCUUUCAACGU GGAGUUUGAUG | 2634 |
| AD-1962442 | ususuca(Ahd)CfgUf GfGfaguuugausgsu | 3583 | asCfsaucAfaacuccaC fgUfugaaasgsc | 3943 | GCUUUCAACGUG GAGUUUGAUGA | 2650 |
| AD-1962451 | gsgsagu(Uhd)UfgAf UfGfacucucagsgsu | 3584 | asCfscugAfgagucauC faAfacuccsasc | 3944 | GUGGAGUUUGAU GACUCUCAGGA | 2725 |
| AD-1962460 | usgsacu(Chd)UfcAf GfGfacaaagcasgsu | 3585 | asCfsugcUfuguccu GfaGfagucasusc | 3945 | GAUGACUCUCAG GACAAAGCAGU | 2787 |
| AD-1962557 | asascuu(Chd)AfcUf UfGfguucacugsgsu | 3586 | asCfscagUfgaaccaaG fuGfaaguuscsu | 3946 | AGAACUUCACUU GGUUCACUGGA | 2721 |
| AD-1962597 | csusgau(Ghd)GfaCf UfGfgccguucusasu | 3587 | asUfsagaAfcggccagU fcCfaucagsgsu | 3947 | ACCUGAUGGACU GGCCGUUCUAG | 2838 |
| AD-1962598 | usgsaug(Ghd)AfcUf GfGfccguucuasgsu | 3588 | asCfsuagAfacggccaG fuCfcaucasgsg | 3948 | CCUGAUGGACUG GCCGUUCUAGG | 2843 |
| AD-1962599 | gsasugg(Ahd)CfuGf GfCfcguucuagsgsu | 3589 | asCfscuaGfaacggccA fgUfccaucsasg | 3949 | CUGAUGGACUGG CCGUUCUAGGU | 2841 |
| AD-1962600 | asusgga(Chd)UfgGf CfCfguucuaggsusu | 3590 | asAfsccuAfgaacggcC faGfuccauscsa | 3950 | UGAUGGACUGGC CGUUCUAGGUA | 2833 |
| AD-1962603 | gsascug(Ghd)CfcGf UfUfcuagguaususu | 3591 | asAfsauaCfcuagaacG fgCfcagucscsa | 3951 | UGGACUGGCCGU UCUAGGUAUUU | 2776 |
| AD-1962604 | ascsugg(Chd)CfgUf UfCfuagguauusus u | 3592 | asAfsaauAfccuagaaC fgGfccaguscsc | 3952 | GGACUGGCCGUU CUAGGUAUUUU | 2751 |
| AD-1962605 | csusggc(Chd)GfuUf CfUfagguauuususu | 3593 | asAfsaaaUfaccuagaA fcGfgccagsusc | 3953 | GACUGGCCGUUC UAGGUAUUUUU | 2646 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962606 | usgsgcc(Ghd)UfuCfUfAfgguauuuususu | 3594 | asAfsaaaAfuaccuagAfaCfggccasgsu | 3954 | ACUGGCCGUUCUAGGUAUUUUUU | 2673 |
| AD-1962607 | gsgsccg(Uhd)UfcUfAfGfguauuuuususu | 3595 | asAfsaaaAfauaccuaGfaAfcggccsasg | 3955 | CUGGCCGUUCUAGGUAUUUUUUU | 2700 |
| AD-1962608 | gscscgu(Uhd)CfuAfGfGfuauuuuuususu | 3596 | asAfsaaaAfaauaccuAfgAfacggcscsa | 3956 | UGGCCGUUCUAGGUAUUUUUUUG | 2681 |
| AD-1962609 | cscsguu(Chd)UfaGfGfUfauuuuuuusgsu | 3597 | asCfsaaaAfaaauaccUfaGfaacggscsc | 3957 | GGCCGUUCUAGGUAUUUUUUUGA | 2662 |
| AD-1962610 | csgsuuc(Uhd)AfgGfUfAfuuuuuuugsasu | 3598 | asUfscaaAfaaaauacCfuAfgaacgsgsc | 3958 | GCCGUUCUAGGUAUUUUUUUGAA | 2606 |
| AD-1962611 | gsusucu(Ahd)GfgUfAfUfuuuuuugasasu | 3599 | asUfsucaAfaaaaauaCfcUfagaacsgsg | 3959 | CCGUUCUAGGUAUUUUUUUGAAG | 2586 |
| AD-1962612 | ususcua(Ghd)GfuAfUfUfuuuuugaasgsu | 3600 | asCfsuucAfaaaaaauAfcCfuagaascsg | 3960 | CGUUCUAGGUAUUUUUUGAAGG | 2607 |
| AD-1962613 | uscsuag(Ghd)UfaUfUfUfuuuugaagsgsu | 3601 | asCfscuuCfaaaaaaaUfaCfcuagasasc | 3961 | GUUCUAGGUAUUUUUUUGAAGGU | 2679 |
| AD-1962615 | usasggu(Ahd)UfuUfUfUfuugaaggusus | 3602 | asAfsaccUfucaaaaaAfaUfaccuasgsa | 3962 | UCUAGGUAUUUUUUUGAAGGUUG | 2642 |
| AD-1962616 | asgsgua(Uhd)UfuUfUfUfugaagguusgsu | 3603 | asCfsaacCfuucaaaaAfaAfuaccusasg | 3963 | CUAGGUAUUUUUUUGAAGGUUGG | 2738 |
| AD-1962617 | gsgsuau(Uhd)UfuUfUfUfgaagguugsgsu | 3604 | asCfscaaCfcuucaaaAfaAfauaccsusa | 3964 | UAGGUAUUUUUUUGAAGGUUGGC | 2731 |
| AD-1962618 | gsusauu(Uhd)UfuUfUfGfaagguuggscsu | 3605 | asGfsccaAfccuucaaAfaAfaauacscsu | 3965 | AGGUAUUUUUUUGAAGGUUGGCA | 2789 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962620 | asusuuu(Uhd)UfuGf AfAfgguuggcasgsu | 3606 | asCfsugcCfaaccuucA faAfaaaausasc | 3966 | GUAUUUUUUUGA AGGUUGGCAGC | 2807 |
| AD-1962648 | csgsggc(Chd)UfuCf AfGfaaaguugususu | 3607 | asAfsacaAfcuuucug AfaGfgcccgsgsu | 3967 | ACCGGGCCUUCA GAAAGUUGUUG | 2701 |
| AD-1962649 | gsgsgcc(Uhd)UfcAf GfAfaaguuguusgsu | 3608 | asCfsaacAfacuuucuG faAfggcccsgsg | 3968 | CCGGGCCUUCAG AAAGUUGUUGA | 2726 |
| AD-1962650 | gsgsccu(Uhd)CfaGf AfAfaguuguugsasu | 3609 | asUfscaaCfaacuuucU fgAfaggccscsg | 3969 | CGGGCCUUCAGA AAGUUGUUGAU | 2712 |
| AD-1962652 | cscsuuc(Ahd)GfaAf AfGfuuguugausgsu | 3610 | asCfsaucAfacaacuuU fcUfgaaggscsc | 3970 | GGCCUUCAGAAA GUUGUUGAUGU | 2756 |
| AD-1962653 | csusuca(Ghd)AfaAf GfUfuguugaugsusu | 3611 | asAfscauCfaacaacuU fuCfugaagsgsc | 3971 | GCCUUCAGAAAG UUGUUGAUGUG | 2696 |
| AD-1962655 | uscsaga(Ahd)AfgUf UfGfuugaugugscsu | 3612 | asGfscacAfucaacaaC fuUfucugasasg | 3972 | CUUCAGAAAGUU GUUGAUGUGCU | 2795 |
| AD-1962658 | gsasaag(Uhd)UfgUf UfGfaugugcugsgsu | 3613 | asCfscagCfacaucaaC faAfcuuucsusg | 3973 | CAGAAAGUUGUU GAUGUGCUGGA | 2820 |
| AD-1962678 | asusucc(Ahd)UfuAf AfAfacaaagggscsu | 3614 | asGfscccUfuuguuuu AfaUfggaauscsc | 3974 | GGAUUCCAUUAA AACAAAGGGCA | 2729 |
| AD-1962688 | asascaa(Ahd)GfgGf CfAfagagugcusgsu | 3615 | asCfsagcAfcucuugcC fcUfuuguususu | 3975 | AAAACAAAGGGC AAGAGUGCUGA | 2821 |
| AD-1962701 | asgsugc(Uhd)GfaCf UfUfcacuaacususu | 3616 | asAfsaguUfagugaag UfcAfgcacuscsu | 3976 | AGAGUGCUGACU UCACUAACUUC | 2599 |
| AD-1962703 | usgscug(Ahd)CfuUf CfAfcuaacuucsgsu | 3617 | asCfsgaaGfuuaguga AfgUfcagcascsu | 3977 | AGUGCUGACUUC ACUAACUUCGA | 2583 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962705 | csusgac(Uhd)UfcAfCfUfaacuucgasusu | 3618 | asAfsucgAfaguuaguGfaAfgucagscsa | 3978 | UGCUGACUUCACUAACUUCGAUC | 2562 |
| AD-1962706 | usgsacu(Uhd)CfaCfUfAfacuucgauscsu | 3619 | asGfsaucGfaaguuagUfgAfagucasgsc | 3979 | GCUGACUUCACUAACUUCGAUCC | 2542 |
| AD-1962707 | gsascuu(Chd)AfcUfAfAfcuucgaucscsu | 3620 | asGfsgauCfgaaguuaGfuGfaagucsasg | 3980 | CUGACUUCACUAACUUCGAUCCU | 2551 |
| AD-1962712 | csascua(Ahd)CfuUfCfGfauccucgusgsu | 3621 | asCfsacgAfggaucgaAfgUfuagugsasa | 3981 | UUCACUAACUUCGAUCCUCGUGG | 2601 |
| AD-1962713 | ascsuaa(Chd)UfuCfGfAfuccucgugsgsu | 3622 | asCfscacGfaggaucgAfaGfuuagusgsa | 3982 | UCACUAACUUCGAUCCUCGUGGC | 2755 |
| AD-1962733 | cscsucc(Uhd)UfcCfUfGfaauccuugsgsu | 3623 | asCfscaaGfgauucagGfaAfggaggscsc | 3983 | GGCCUCCUUCCUGAAUCCUUGGA | 2629 |
| AD-1962735 | uscscuu(Chd)CfuGfAfAfuccuuggasusu | 3624 | asAfsuccAfaggauucAfgGfaaggasgsg | 3984 | CCUCCUUCCUGAAUCCUUGGAUU | 2608 |
| AD-1962736 | cscsuuc(Chd)UfgAfAfUfccuuggaususu | 3625 | asAfsaucCfaaggauuCfaGfgaaggsasg | 3985 | CUCCUUCCUGAAUCCUUGGAUUA | 2611 |
| AD-1962766 | csusccu(Chd)UfuCfUfGfgaaugugusgsu | 3626 | asCfsacaCfauuccagAfaGfaggagsgsg | 3986 | CCCUCCUCUUCUGGAAUGUGUGA | 2623 |
| AD-1962777 | gsasaug(Uhd)GfuGfAfCfcuggauugsusu | 3627 | asAfscaaUfccaggucAfcAfcauucscsa | 3987 | UGGAAUGUGUGACCUGGAUUGUG | 2816 |
| AD-1962780 | usgsugu(Ghd)AfcCfUfGfgauugugcsusu | 3628 | asAfsgcaCfaauccagGfuCfacacasusu | 3988 | AAUGUGUGACCUGGAUUGUGCUC | 2805 |
| AD-1962795 | gsusgcu(Chd)AfaGfGfAfacccaucasgsu | 3629 | asCfsugaUfggguuccUfuGfagcacsasa | 3989 | UUGUGCUCAAGGAACCCAUCAGC | 2766 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962799 | uscsaag(Ghd)AfaCf CfCfaucagcguscsu | 3630 | asGfsacgCfugauggg UfuCfcuugasgsc | 3990 | GCUCAAGGAACC CAUCAGCGUCA | 2794 |
| AD-1962801 | asasgga(Ahd)CfcCf AfUfcagcgucasgsu | 3631 | asCfsugaCfgcugaug GfgUfuccuusgsa | 3991 | UCAAGGAACCCA UCAGCGUCAGC | 2811 |
| AD-1962808 | cscsauc(Ahd)GfcGf UfCfagcagcgasgsu | 3632 | asCfsucgCfugcugacG fcUfgauggsgsu | 3992 | ACCCAUCAGCGU CAGCAGCGAGC | 2835 |
| AD-1962836 | gsasaau(Uhd)CfcGf UfAfaacuuaacsusu | 3633 | asAfsguuAfaguuuac GfgAfauuucsasa | 3993 | UUGAAAUUCCGU AAACUUAACUU | 2584 |
| AD-1962837 | asasauu(Chd)CfgUf AfAfacuuaacusus u | 3634 | asAfsaguUfaaguuua CfgGfaauuuscsa | 3994 | UGAAAUUCCGUA AACUUAACUUC | 2559 |
| AD-1962842 | cscsgua(Ahd)AfcUf UfAfacuucaausgsu | 3635 | asCfsauuGfaaguuaaG fuUfuacggsasa | 3995 | UUCCGUAAACUU AACUUCAAUGG | 2556 |
| AD-1962843 | csgsuaa(Ahd)CfuUf AfAfcuucaaugsgsu | 3636 | asCfscauUfgaaguuaA fgUfuuacgsgsa | 3996 | UCCGUAAACUUA ACUUCAAUGGG | 2659 |
| AD-1962860 | asascug(Ahd)UfgGf UfGfgacaacugsgsu | 3637 | asCfscagUfuguccacC faUfcaguuscsu | 3997 | AGAACUGAUGGU GGACAACUGGC | 2844 |
| AD-1962885 | csuscag(Chd)CfaCf UfGfaagaacagsgsu | 3638 | asCfscugUfucuucag UfgGfcugagscsu | 3998 | AGCUCAGCCACU GAAGAACAGGC | 2799 |
| AD-1962897 | asgsaac(Ahd)GfgCf AfAfaucaaagcsusu | 3639 | asAfsgcuUfugauuug CfcUfguucususc | 3999 | GAAGAACAGGCA AAUCAAAGCUU | 2749 |
| AD-1962902 | asgsgca(Ahd)AfuCf AfAfagcuuccususu | 3640 | asAfsaggAfagcuuug AfuUfugccusgsu | 4000 | ACAGGCAAAUCA AAGCUUCCUUC | 2567 |
| AD-1962910 | csasaag(Chd)UfuCf CfUfucaaauaasgsu | 3641 | asCfsuuaUfuugaagg AfaGfcuuugsasu | 4001 | AUCAAAGCUUCC UUCAAAUAAGA | 2543 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962911 | asasagc(Uhd)UfcCf UfUfcaaauaagsasu | 3642 | asUfscuuAfuuugaag GfaAfgcuuusgsa | 4002 | UCAAAGCUUCCU UCAAAUAAGAU | 2569 |
| AD-1962912 | asasgcu(Uhd)CfcUf UfCfaaauaagasusu | 3643 | asAfsucuUfauuugaa GfgAfagcuususg | 4003 | CAAAGCUUCCUU CAAAUAAGAUG | 2558 |
| AD-1962913 | asgscuu(Chd)CfuUf CfAfaauaagausgsu | 3644 | asCfsaucUfuauuuga AfgGfaagcususu | 4004 | AAAGCUUCCUUC AAAUAAGAUGG | 2565 |
| AD-1962914 | gscsuuc(Chd)UfuCf AfAfauaagaugsgsu | 3645 | asCfscauCfuuauuug AfaGfgaagcsusu | 4005 | AAGCUUCCUUCA AAUAAGAUGGU | 2563 |
| AD-1962941 | gsuscug(Uhd)AfuCf CfAfaauaaugasasu | 3646 | asUfsucaUfuauuugg AfuAfcagacsusa | 4006 | UAGUCUGUAUCC AAAUAAUGAAU | 2545 |
| AD-1962945 | gsusauc(Chd)AfaAf UfAfaugaaucususu | 3647 | asAfsagaUfucauuau UfuGfgauacsasg | 4007 | CUGUAUCCAAAU AAUGAAUCUUC | 2561 |
| AD-1962947 | asuscca(Ahd)AfuAf AfUfgaaucuucsgsu | 3648 | asCfsgaaGfauucauuA fuUfuggausasc | 4008 | GUAUCCAAAUAA UGAAUCUUCGG | 2614 |
| AD-1962948 | uscscaa(Ahd)UfaAf UfGfaaucuucgsgsu | 3649 | asCfscgaAfgauucauU faUfuuggasusa | 4009 | UAUCCAAAUAAU GAAUCUUCGGG | 2732 |
| AD-1962949 | cscsaaa(Uhd)AfaUf GfAfaucuucggsgsu | 3650 | asCfsccgAfagauucaU fuAfuuuggsasu | 4010 | AUCCAAAUAAUG AAUCUUCGGGU | 2796 |
| AD-1962950 | csasaau(Ahd)AfuGf AfAfucuucgggsusu | 3651 | asAfscccGfaagauucA fuUfauuugsgsa | 4011 | UCCAAAUAAUGA AUCUUCGGGUG | 2743 |
| AD-1962951 | asasaua(Ahd)UfgAf AfUfcuucgggusgsu | 3652 | asCfsaccCfgaagauuC faUfuauuusgsg | 4012 | CCAAAUAAUGAA UCUUCGGGUGU | 2815 |
| AD-1962952 | asasuaa(Uhd)GfaAf UfCfuucgggugsusu | 3653 | asAfscacCfcgaagauU fcAfuuauusus g | 4013 | CAAAUAAUGAAU CUUCGGGUGUU | 2806 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1962953 | asusaau(Ghd)AfaUfCfUfucgggugususu | 3654 | asAfsacaCfccgaagaUfuCfauuaususu | 4014 | AAAUAAUGAAUCUUCGGGUGUUU | 2801 |
| AD-1962954 | usasaug(Ahd)AfuCfUfUfcggguguususu | 3655 | asAfsaacAfcccgaagAfuUfcauuasusu | 4015 | AAUAAUGAAUCUUCGGGUGUUUC | 2809 |
| AD-1962957 | usgsaau(Chd)UfuCfGfGfguguuuccscsu | 3656 | asGfsggaAfacacccgAfaGfauucasusu | 4016 | AAUGAAUCUUCGGGUGUUUCCCU | 2739 |
| AD-1962958 | gsasauc(Uhd)UfcGfGfGfuguuucccsusu | 3657 | asAfsgggAfaacacccGfaAfgauucsasu | 4017 | AUGAAUCUUCGGGUGUUUCCCUU | 2747 |
| AD-1962984 | asasgca(Chd)AfgAfUfCfuaccuuggsusu | 3658 | asAfsccaAfgguagauCfuGfugcuusasg | 4018 | CUAAGCACAGAUCUACCUUGGUG | 2671 |
| AD-1962985 | asgscac(Ahd)GfaUfCfUfaccuuggusgsu | 3659 | asCfsaccAfagguagaUfcUfgugcususa | 4019 | UAAGCACAGAUCUACCUUGGUGA | 2680 |
| AD-1962986 | gscsaca(Ghd)AfuCfUfAfccuuggugsasu | 3660 | asUfscacCfaagguagAfuCfugugcsusu | 4020 | AAGCACAGAUCUACCUUGGUGAU | 2633 |
| AD-1962990 | asgsauc(Uhd)AfcCfUfUfggugauuusgsu | 3661 | asCfsaaaUfcaccaagGfuAfgaucusgsu | 4021 | ACAGAUCUACCUUGGUGAUUUGG | 2616 |
| AD-1963114 | ascsaac(Uhd)GfcUfGfUfggcugguusgsu | 3662 | asCfsaacCfagccacaGfcAfguugusgsu | 4022 | ACACAACUGCUGUGGCUGGUUGG | 2845 |
| AD-1963115 | csasacu(Ghd)CfuGfUfGfgcugguugsgsu | 3663 | asCfscaaCfcagccacAfgCfaguugsusg | 4023 | CACAACUGCUGUGGCUGGUUGGU | 2847 |
| AD-1963118 | csusgcu(Ghd)UfgGfCfUfgguuggugscsu | 3664 | asGfscacCfaaccagcCfaCfagcagsusu | 4024 | AACUGCUGUGGCUGGUUGGUGCU | 2849 |
| AD-1963123 | gsusggc(Uhd)GfgUfUfGfgugcuuugsusu | 3665 | asAfscaaAfgcaccaaCfcAfgccacsasg | 4025 | CUGUGGCUGGUUGGUGCUUUGUU | 2810 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963125 | gsgscug(Ghd)UfuGf GfUfgcuuuguususu | 3666 | asAfsaacAfaagcaccA faCfcagccsasc | 4026 | GUGGCUGGUUGG UGCUUUGUUUA | 2769 |
| AD-1963126 | gscsugg(Uhd)UfgGf UfGfcuuuguuusasu | 3667 | asUfsaaaCfaaagcacCf aAfccagcscsa | 4027 | UGGCUGGUUGGU GCUUUGUUUAU | 2753 |
| AD-1963127 | csusggu(Uhd)GfgUf GfCfuuuguuuasusu | 3668 | asAfsuaaAfcaaagcaC fcAfaccagscsc | 4028 | GGCUGGUUGGUG CUUUGUUUAUG | 2686 |
| AD-1963128 | usgsguu(Ghd)GfuGf CfUfuuguuuausgsu | 3669 | asCfsauaAfacaaagcA fcCfaaccasgsc | 4029 | GCUGGUUGGUGC UUUGUUUAUGG | 2690 |
| AD-1963129 | gsgsuug(Ghd)UfgCf UfUfuguuuaugsgsu | 3670 | asCfscauAfaacaaagC faCfcaaccsasg | 4030 | CUGGUUGGUGCU UUGUUUAUGGU | 2737 |
| AD-1963130 | gsusugg(Uhd)GfcUf UfUfguuuauggsusu | 3671 | asAfsccaUfaaacaaaG fcAfccaacscsa | 4031 | UGGUUGGUGCUU UGUUUAUGGUA | 2746 |
| AD-1963131 | ususggu(Ghd)CfuUf UfGfuuuauggusasu | 3672 | asUfsaccAfuaaacaaA fgCfaccaascsc | 4032 | GGUUGGUGCUUU GUUUAUGGUAG | 2651 |
| AD-1963132 | usgsgug(Chd)UfuUf GfUfuuaugguasgsu | 3673 | asCfsuacCfauaaacaA faGfcaccasasc | 4033 | GUUGGUGCUUUG UUUAUGGUAGU | 2643 |
| AD-1963135 | usgscuu(Uhd)GfuUf UfAfugguaguasgsu | 3674 | asCfsuacUfaccauaaA fcAfaagcascsc | 4034 | GGUGCUUUGUUU AUGGUAGUAGU | 2684 |
| AD-1963138 | ususugu(Uhd)UfaUf GfGfuaguaguususu | 3675 | asAfsaacUfacuaccaU faAfacaaasgsc | 4035 | GCUUUGUUUAUG GUAGUAGUUUU | 2723 |
| AD-1963139 | ususguu(Uhd)AfuGf GfUfaguaguuususu | 3676 | asAfsaaaCfuacuaccA fuAfaacaasasg | 4036 | CUUUGUUUAUGG UAGUAGUUUUU | 2773 |
| AD-1963140 | gsusuua(Uhd)GfgUf AfGfuaguuuuscsu | 3677 | asGfsaaaAfacuacuaC fcAfuaaacsasa | 4037 | UUGUUUAUGGUA GUAGUUUUUCU | 2760 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963141 | ususuau(Ghd)GfuAf GfUfaguuuuucsusu | 3678 | asAfsgaaAfaacuacuA fcCfauaaascsa | 4038 | UGUUUAUGGUAG UAGUUUUUCUG | 2663 |
| AD-1963142 | ususaug(Ghd)UfaGf UfAfguuuuucusgsu | 3679 | asCfsagaAfaaacuacU faCfcauaasasc | 4039 | GUUUAUGGUAGU AGUUUUUCUGU | 2720 |
| AD-1963143 | usasugg(Uhd)AfgUf AfGfuuuuucugsusu | 3680 | asAfscagAfaaaacuaC fuAfccauasasa | 4040 | UUUAUGGUAGUA GUUUUUCUGUA | 2675 |
| AD-1963144 | asusggu(Ahd)GfuAf GfUfuuuucugusasu | 3681 | asUfsacaGfaaaaacuA fcUfaccausasa | 4041 | UUAUGGUAGUAG UUUUUCUGUAA | 2617 |
| AD-1963148 | usasgua(Ghd)UfuUf UfUfcuguaacascsu | 3682 | asGfsuguUfacagaaaA faCfuacuascsc | 4042 | GGUAGUAGUUUU UCUGUAACACA | 2740 |
| AD-1963202 | asgsaau(Ahd)AfaGf UfAfccuugacususu | 3683 | asAfsaguCfaagguacU fuUfauucususa | 4043 | UAAGAAUAAAGU ACCUUGACUUU | 2572 |
| AD-1963204 | asasuaa(Ahd)GfuAf CfCfuugacuuusgsu | 3684 | asCfsaaaGfucaagguA fcUfuuauuscsu | 4044 | AGAAUAAAGUAC CUUGACUUUGU | 2706 |
| AD-1963205 | asusaaa(Ghd)UfaCf CfUfugacuuugsusu | 3685 | asAfscaaAfgucaaggU faCfuuuaususc | 4045 | GAAUAAAGUACC UUGACUUUGUU | 2653 |
| AD-1963206 | usasaag(Uhd)AfcCf UfUfgacuuugususu | 3686 | asAfsacaAfagucaagG fuAfcuuuasusu | 4046 | AAUAAAGUACCU UGACUUUGUUC | 2595 |
| AD-1963207 | asasagu(Ahd)CfcUf UfGfacuuuguuscsu | 3687 | asGfsaacAfaagucaaG fgUfacuuusasu | 4047 | AUAAAGUACCUU GACUUUGUUCA | 2603 |
| AD-1963208 | asasgua(Chd)CfuUf GfAfcuuuguucsasu | 3688 | asUfsgaaCfaaagucaA fgGfuacuususa | 4048 | UAAAGUACCUUG ACUUUGUUCAC | 2579 |
| AD-1963211 | usasccu(Uhd)GfaCf UfUfuguucacasgsu | 3689 | asCfsuguGfaacaaagU fcAfagguascsu | 4049 | AGUACCUUGACU UUGUUCACAGC | 2667 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963218 | ascsuuu(Ghd)UfuCf AfCfagcauguasgsu | 3690 | asCfsuacAfugcugug AfaCfaaaguscsa | 4050 | UGACUUUGUUCA CAGCAUGUAGG | 2672 |
| AD-1963219 | csusuug(Uhd)UfcAf CfAfgcauguagsgsu | 3691 | asCfscuaCfaugcuguG faAfcaaagsusc | 4051 | GACUUUGUUCAC AGCAUGUAGGG | 2707 |
| AD-1963220 | ususugu(Uhd)CfaCf AfGfcauguaggsgsu | 3692 | asCfsccuAfcaugcugU fgAfacaaasgsu | 4052 | ACUUUGUUCACA GCAUGUAGGGU | 2744 |
| AD-1963221 | ususguu(Chd)AfcAf GfCfauguagggsusu | 3693 | asAfscccUfacaugcuG fuGfaacaasasg | 4053 | CUUUGUUCACAG CAUGUAGGGUG | 2770 |
| AD-1963222 | usgsuuc(Ahd)CfaGf CfAfuguaggguss gsu | 3694 | asCfsaccCfuacaugcU fgUfgaacasasa | 4054 | UUUGUUCACAGC AUGUAGGGUGA | 2803 |
| AD-1963225 | uscsaca(Ghd)CfaUf GfUfagggugausgsu | 3695 | asCfsaucAfcccuacaU fgCfugugasasc | 4055 | GUUCACAGCAUG UAGGGUGAUGA | 2779 |
| AD-1963237 | csasacg(Ghd)AfcCf UfGfagcacuggscsu | 3696 | asGfsccdAg(Tgn)gcu cagGfuCfcguugsusg | 4056 | CACAACGGACCU GAGCACUGGCA | 2846 |
| AD-1963239 | ascsgga(Chd)CfuGf AfGfcacuggcasusu | 3697 | asAfsugdCc(Agn)gu gcucAfgGfuccgusGsg | 4057 | CAACGGACCUGA GCACUGGCAUA | 2825 |
| AD-1963244 | cscsuga(Ghd)CfaCf UfGfgcauaaggsasu | 3698 | asUfsccdTu(Agn)ugc cagUfgCfucaggsusc | 4058 | GACCUGAGCACU GGCAUAAGGAC | 2822 |
| AD-1963245 | csusgag(Chd)AfcUf GfGfcauaaggascsu | 3699 | asGfsucdCu(Tgn)aug ccaGfuGfcucagsgsu | 4059 | ACCUGAGCACUG GCAUAAGGACU | 2781 |
| AD-1963246 | usgsagc(Ahd)CfuGf GfCfauaaggacsusu | 3700 | asAfsgudCc(Tgn)uau gccAfgUfgcucasgsg | 4060 | CCUGAGCACUGG CAUAAGGACUU | 2757 |
| AD-1963249 | gscsacu(Ghd)GfcAf UfAfaggacuucscsu | 3701 | asGfsgadAg(Tgn)ccu uauGfcCfagugcsusc | 4061 | GAGCACUGGCAU AAGGACUUCCC | 2693 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963287 | gsascua(Ahd)AfaUf GfCfugcuuuuasasu | 3702 | asUfsuaaAfagcagcaU fuUfuagucsasa | 4062 | UUGACUAAAAUG CUGCUUUUAAA | 2609 |
| AD-1963288 | ascsuaa(Ahd)AfuGf CfUfgcuuuuaasasu | 3703 | asUfsuuaAfaagcagcA fuUfuuaguscsa | 4063 | UGACUAAAAUGC UGCUUUUAAAA | 2677 |
| AD-1963289 | csusaaa(Ahd)UfgCf UfGfcuuuuaaasasu | 3704 | asUfsuuuAfaaagcagC faUfuuuagsusc | 4064 | GACUAAAAUGCU GCUUUUAAAAC | 2682 |
| AD-1963290 | usasaaa(Uhd)GfcUf GfCfuuuuaaaascsu | 3705 | asGfsuuuUfaaaagcaG fcAfuuuuasgsu | 4065 | ACUAAAAUGCUG CUUUUAAAACA | 2570 |
| AD-1963295 | usgscug(Chd)UfuUf UfAfaaacauagsgsu | 3706 | asCfscuaUfguuuuaa AfaGfcagcasusu | 4066 | AAUGCUGCUUUU AAAACAUAGGA | 2566 |
| AD-1963296 | gscsugc(Uhd)UfuUf AfAfaacauaggsasu | 3707 | asUfsccuAfuguuuua AfaAfgcagcsasu | 4067 | AUGCUGCUUUUA AAACAUAGGAA | 2597 |
| AD-1963302 | ususuaa(Ahd)AfcAf UfAfggaaaguasgsu | 3708 | asCfsuacUfuuccuauG fuUfuuaaasasg | 4068 | CUUUUAAAACAU AGGAAAGUAGA | 2772 |
| AD-1963306 | csusguu(Ghd)AfcAf UfCfgacacucasusu | 3709 | asAfsugdAg(Tgn)guc gauGfuCfaacagsgsg | 4069 | CCCUGUUGACAU CGACACUCAUA | 2592 |
| AD-1963308 | gsusuga(Chd)AfuCf GfAfcacucauascsu | 3710 | asGfsuadTg(Agn)gug ucgAfuGfucaacsasg | 4070 | CUGUUGACAUCG ACACUCAUACA | 2578 |
| AD-1963311 | gsascau(Chd)GfaCf AfCfucauacagscsu | 3711 | asGfscudGu(Agn)ug agugUfcGfaugucsasa | 4071 | UUGACAUCGACA CUCAUACAGCC | 2657 |
| AD-1963312 | ascsauc(Ghd)AfcAf CfUfcauacagcscsu | 3712 | asGfsgcdTg(Tgn)aug aguGfuCfgauguscsa | 4072 | UGACAUCGACAC UCAUACAGCCA | 2762 |
| AD-1963314 | asuscga(Chd)AfcUf CfAfuacagccasasu | 3713 | asUfsugdGc(Tgn)gua ugaGfuGfucgausgsu | 4073 | ACAUCGACACUC AUACAGCCAAG | 2692 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963318 | ascsacu(Chd)AfuAf CfAfgccaaguasusu | 3714 | asAfsuadCu(Tgn)ggc uguAfuGfaguguscsg | 4074 | CGACACUCAUAC AGCCAAGUAUG | 2632 |
| AD-1963321 | csuscau(Ahd)CfaGf CfCfaaguaugascsu | 3715 | asGfsucdAu(Agn)cu uggcUfgUfaugagsusg | 4075 | CACUCAUACAGC CAAGUAUGACC | 2691 |
| AD-1963322 | uscsaua(Chd)AfgCf CfAfaguaugacscsu | 3716 | asGfsgudCa(Tgn)acu uggCfuGfuaugasgsu | 4076 | ACUCAUACAGCC AAGUAUGACCC | 2764 |
| AD-1963323 | csasuac(Ahd)GfcCf AfAfguaugaccscsu | 3717 | asGfsggdTc(Agn)uac uugGfcUfguaugsasg | 4077 | CUCAUACAGCCA AGUAUGACCCU | 2788 |
| AD-1963325 | usascag(Chd)CfaAf GfUfaugacccusus u | 3718 | asAfsagdGg(Tgn)cau acuUfgGfcuguasusg | 4078 | CAUACAGCCAAG UAUGACCCUUC | 2780 |
| AD-1963329 | gscscaa(Ghd)UfaUf GfAfcccuucccsusu | 3719 | asAfsggdGa(Agn)gg gucaUfaCfuuggcsusg | 4079 | CAGCCAAGUAUG ACCCUUCCCUG | 2674 |
| AD-1963375 | gsasuaa(Ahd)UfuGf AfGfcuaguuaasgsu | 3720 | asCfsuuaAfcuagcucA faUfuuaucsusu | 4080 | AAGAUAAAUUGA GCUAGUUAAGG | 2798 |
| AD-1963376 | asusaaa(Uhd)UfgAf GfCfuaguuaagsgsu | 3721 | asCfscuuAfacuagcuC faAfuuuauscsu | 4081 | AGAUAAAUUGAG CUAGUUAAGGC | 2819 |
| AD-1963377 | usasaau(Uhd)GfaGf CfUfaguuaaggscsu | 3722 | asGfsccuUfaacuagcU fcAfauuuasusc | 4082 | GAUAAAUUGAGC UAGUUAAGGCA | 2784 |
| AD-1963384 | gsusaug(Ahd)CfcCf UfUfcccugaagscsu | 3723 | asGfscudTc(Agn)ggg aagGfgUfcauacsusu | 4083 | AAGUAUGACCCU UCCCUGAAGCC | 2830 |
| AD-1963386 | csusguc(Uhd)GfuUf UfCfcuaugaucsasu | 3724 | asUfsgadTc(Agn)uag gaaAfcAfgacagsgsg | 4084 | CCCUGUCUGUUU CCUAUGAUCAA | 2560 |
| AD-1963388 | gsuscug(Uhd)UfuCf CfUfaugaucaasgsu | 3725 | asCfsuudGa(Tgn)cau aggAfaAfcagacsasg | 4085 | CUGUCUGUUUCC UAUGAUCAAGC | 2573 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963389 | uscsugu(Uhd)UfcCfUfAfugaucaagscsu | 3726 | asGfscudTg(Agn)ucauagGfaAfacagascsa | 4086 | UGUCUGUUUCCUAUGAUCAAGCA | 2577 |
| AD-1963391 | usgsuuu(Chd)CfuAfUfGfaucaagcasasu | 3727 | asUfsugdCu(Tgn)gaucauAfgGfaaacasgsa | 4087 | UCUGUUUCCUAUGAUCAAGCAAC | 2541 |
| AD-1963392 | gsusuuc(Chd)UfaUfGfAfucaagcaascsu | 3728 | asGfsuudGc(Tgn)ugaucaUfaGfgaaacsasg | 4088 | CUGUUUCCUAUGAUCAAGCAACU | 2548 |
| AD-1963395 | uscscua(Uhd)GfaUfCfAfagcaacuuscsu | 3729 | asGfsaadGu(Tgn)gcuugaUfcAfuaggasasa | 4089 | UUUCCUAUGAUCAAGCAACUUCC | 2547 |
| AD-1963410 | asgscua(Ghd)UfuAfAfGfgcaaaucasgsu | 3730 | asCfsugaUfuugccuuAfaCfuagcuscsa | 4090 | UGAGCUAGUUAAGGCAAAUCAGG | 2735 |
| AD-1963411 | gscsuag(Uhd)UfaAfGfGfcaaaucagsgsu | 3731 | asCfscugAfuuugccuUfaAfcuagcsusc | 4091 | GAGCUAGUUAAGGCAAAUCAGGU | 2774 |
| AD-1963417 | usasagg(Chd)AfaAfUfCfagguaaaasusu | 3732 | asAfsuuuUfaccugauUfuGfccuuasasc | 4092 | GUUAAGGCAAAUCAGGUAAAAUA | 2585 |
| AD-1963419 | asgsgca(Ahd)AfuCfAfGfguaaaauasgsu | 3733 | asCfsuauUfuuaccugAfuUfugccusosa | 4093 | UAAGGCAAAUCAGGUAAAAUAGU | 2698 |
| AD-1963420 | gsgscaa(Ahd)UfcAfGfGfuaaaauagsusu | 3734 | asAfscuaUfuuuaccuGfaUfuugccsusu | 4094 | AAGGCAAAUCAGGUAAAAUAGUC | 2702 |
| AD-1963421 | gscsaaa(Uhd)CfaGfGfUfaaaauaguscsu | 3735 | asGfsacuAfuuuuaccUfgAfuuugcscsu | 4095 | AGGCAAAUCAGGUAAAAUAGUCA | 2695 |
| AD-1963430 | gsusaaa(Ahd)UfaGfUfCfaugauucusasu | 3736 | asUfsagaAfucaugacUfaUfuuuacscsu | 4096 | AGGUAAAAUAGUCAUGAUUCUAU | 2775 |
| AD-1963431 | usasaaa(Uhd)AfgUfCfAfugauucuasusu | 3737 | asAfsuagAfaucaugaCfuAfuuuuascsc | 4097 | GGUAAAAUAGUCAUGAUUCUAUG | 2765 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963432 | asasaau(Ahd)GfuCf AfUfgauucuausgsu | 3738 | asCfsauaGfaaucaugA fcUfauuuusasc | 4098 | GUAAAAUAGUCA UGAUUCUAUGU | 2750 |
| AD-1963437 | asgsuca(Uhd)GfaUf UfCfuauguaausgsu | 3739 | asCfsauuAfcauagaaU fcAfugacusasu | 4099 | AUAGUCAUGAUU CUAUGUAAUGU | 4290 |
| AD-1963438 | gsuscau(Ghd)AfuUf CfUfauguaaugsusu | 3740 | asAfscauUfacauagaA fuCfaugacsusa | 4100 | UAGUCAUGAUUC UAUGUAAUGUA | 2655 |
| AD-1963439 | uscsaug(Ahd)UfuCf UfAfuguaaugusasu | 3741 | asUfsacaUfuacauagA faUfcaugascsu | 4101 | AGUCAUGAUUCU AUGUAAUGUAA | 2598 |
| AD-1963464 | cscscug(Ahd)GfgAf UfCfcucaacaasusu | 3742 | asAfsuudGu(Tgn)gag gauCfcUfcagggsasa | 4102 | UUCCCUGAGGAU CCUCAACAAUG | 2752 |
| AD-1963465 | cscsuga(Ghd)GfaUf CfCfucaacaausgsu | 3743 | asCfsaudTg(Tgn)uga ggaUfcCfucaggsgsa | 4103 | UCCCUGAGGAUC CUCAACAAUGG | 2736 |
| AD-1963468 | gsasgga(Uhd)CfcUf CfAfacaaugguscsu | 3744 | asGfsacdCa(Tgn)ugu ugaGfgAfuccucsasg | 4104 | CUGAGGAUCCUC AACAAUGGUCA | 2703 |
| AD-1963469 | asgsgau(Chd)CfuCf AfAfcaauggucsasu | 3745 | asUfsgadCc(Agn)uug uugAfgGfauccuscsa | 4105 | UGAGGAUCCUCA ACAAUGGUCAU | 2713 |
| AD-1963539 | usgscuu(Uhd)CfaAf CfGfuggaguuusgsu | 3746 | asCfsaadAc(Tgn)cca cguUfgAfaagcasusg | 4106 | CAUGCUUUCAAC GUGGAGUUUGA | 2625 |
| AD-1963543 | ususcaa(Chd)GfuGf GfAfguuugaugsasu | 3747 | asUfscadTc(Agn)aac uccAfcGfuugaasasg | 4107 | CUUUCAACGUGG AGUUUGAUGAC | 2665 |
| AD-1963545 | csasacg(Uhd)GfgAf GfUfuugaugacsusu | 3748 | asAfsgudCa(Tgn)caa acuCfcAfcguugsasa | 4108 | UUCAACGUGGAG UUUGAUGACUC | 2715 |
| AD-1963546 | asascgu(Ghd)GfaGf UfUfugaugacuscsu | 3749 | asGfsagdTc(Agn)uca aacUfcCfacguusgsa | 4109 | UCAACGUGGAGU UUGAUGACUCU | 2694 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963548 | csgsugg(Ahd)GfuUfUfGfaugacucuscsu | 3750 | asGfsagdAg(Tgn)caucaaAfcUfccacgsusu | 4110 | AACGUGGAGUUUGAUGACUCUCA | 2664 |
| AD-1963550 | usgsgag(Uhd)UfuGfAfUfgacucucasgsu | 3751 | asCfsugdAg(Agn)gucaucAfaAfcuccascsg | 4111 | CGUGGAGUUUGAUGACUCUCAGG | 2613 |
| AD-1963552 | gsasguu(Uhd)GfaUfGfAfcucucaggsasu | 3752 | asUfsccdTg(Agn)gagucaUfcAfaacucscsa | 4112 | UGGAGUUUGAUGACUCUCAGGAC | 2718 |
| AD-1963554 | gsusuug(Ahd)UfgAfCfUfcucaggacsasu | 3753 | asUfsgudCc(Tgn)gagaguCfaUfcaaacsusc | 4113 | GAGUUUGAUGACUCUCAGGACAA | 2741 |
| AD-1963557 | usgsaug(Ahd)CfuCfUfCfaggacaaasgsu | 3754 | asCfsuudTg(Tgn)ccugagAfgUfcaucasasa | 4114 | UUUGAUGACUCUCAGGACAAAGC | 2612 |
| AD-1963582 | usasauu(Ahd)GfaGfUfUfgugauacasgsu | 3755 | asCfsuguAfucacaacUfcUfaauuasusa | 4115 | UAUAAUUAGAGUUGUGAUACAGA | 2786 |
| AD-1963590 | gsusugu(Ghd)AfuAfCfAfgaguauaususu | 3756 | asAfsauaUfacucuguAfuCfacaacsusc | 4116 | GAGUUGUGAUACAGAGUAUAUUU | 2639 |
| AD-1963591 | ususgug(Ahd)UfaCfAfGfaguauauususu | 3757 | asAfsaauAfuacucugUfaUfcacaascsu | 4117 | AGUUGUGAUACAGAGUAUAUUUC | 2636 |
| AD-1963592 | usgsuga(Uhd)AfcAfGfAfguauauuuscsu | 3758 | asGfsaaaUfauacucuGfuAfucacasasc | 4118 | GUUGUGAUACAGAGUAUAUUUCC | 2644 |
| AD-1963609 | asusgac(Uhd)CfuCfAfGfgacaaagcsasu | 3759 | asUfsgcdTu(Tgn)guccugAfgAfgucauscsa | 4119 | UGAUGACUCUCAGGACAAAGCAG | 2734 |
| AD-1963610 | usgsacu(Chd)UfcAfGfGfacaaagcasgsu | 3760 | asCfsugdCu(Tgn)uguccuGfaGfagucasusc | 4120 | GAUGACUCUCAGGACAAAGCAGU | 2787 |
| AD-1963618 | cscsauu(Chd)AfgAfCfAfauauaucasusu | 3761 | asAfsugaUfauauuguCfuGfaauggsasa | 4121 | UUCCAUUCAGACAAUAUAUCAUA | 2552 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963719 | ascsuuc(Ahd)CfuUf GfGfuucacuggsasu | 3762 | asUfsccdAg(Tgn)gaa ccaAfgUfgaagususc | 4122 | GAACUUCACUUG GUUCACUGGAA | 2678 |
| AD-1963721 | ususcac(Uhd)UfgGf UfUfcacuggaascsu | 3763 | asGfsuudCc(Agn)gu gaacCfaAfgugaasgsu | 4123 | ACUUCACUUGGU UCACUGGAACA | 2704 |
| AD-1963733 | gsasuuu(Uhd)GfgGf AfAfagcugugcsasu | 3764 | asUfsgcdAc(Agn)gcu uucCfcAfaaaucscsc | 4124 | GGGAUUUUGGGA AAGCUGUGCAG | 2792 |
| AD-1963735 | ususuug(Ghd)GfaAf AfGfcugugcagscsu | 3765 | asGfscudGc(Agn)cag cuuUfcCfcaaaasusc | 4125 | GAUUUUGGGAAA GCUGUGCAGCA | 2837 |
| AD-1963738 | usgsgga(Ahd)AfgCf UfGfugcagcaascsu | 3766 | asGfsuudGc(Tgn)gca cagCfuUfucccasasa | 4126 | UUUGGGAAAGCU GUGCAGCAACC | 2826 |
| AD-1963762 | usgsgac(Uhd)GfgCf CfGfuucuaggusasu | 3767 | asUfsacdCu(Agn)gaa cggCfcAfguccasusc | 4127 | GAUGGACUGGCC GUUCUAGGUAU | 2836 |
| AD-1963763 | gsgsacu(Ghd)GfcCf GfUfucuagguasusu | 3768 | asAfsuadCc(Tgn)aga acgGfcCfaguccsasu | 4128 | AUGGACUGGCCG UUCUAGGUAUU | 2818 |
| AD-1963776 | usasggu(Ahd)UfuUf UfUfuugaaggususu | 3769 | asAfsacdCu(Tgn)caa aaaAfaUfaccuasgsa | 4129 | UCUAGGUAUUUU UUUGAAGGUUG | 2642 |
| AD-1963780 | usasuuu(Uhd)UfuUf GfAfagguuggcsasu | 3770 | asUfsgcdCa(Agn)ccu ucaAfaAfaaauascsc | 4130 | GGUAUUUUUUUG AAGGUUGGCAG | 2785 |
| AD-1963781 | asusuuu(Uhd)UfuGf AfAfgguuggcasgsu | 3771 | asCfsugdCc(Agn)acc uucAfaAfaaaausasc | 4131 | GUAUUUUUUUGA AGGUUGGCAGC | 2807 |
| AD-1963785 | ususuug(Ahd)AfgGf UfUfggcagcgcsusu | 3772 | asAfsgcdGc(Tgn)gcc aacCfuUfcaaaasasa | 4132 | UUUUUUGAAGGU UGGCAGCGCUA | 2829 |
| AD-1963790 | asasggu(Uhd)GfgCf AfGfcgcuaaacscsu | 3773 | asGfsgudTu(Agn)gcg cugCfcAfaccuuscsa | 4133 | UGAAGGUUGGCA GCGCUAAACCG | 2842 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963814 | csusuca(Ghd)AfaAf GfUfuguugaugsusu | 3774 | asAfscadTc(Agn)aca acuUfuCfugaagsgsc | 4134 | GCCUUCAGAAAG UUGUUGAUGUG | 2696 |
| AD-1963816 | uscsaga(Ahd)AfgUf UfGfuugaugugscsu | 3775 | asGfscadCa(Tgn)caa caaCfuUfucugasasg | 4135 | CUUCAGAAAGUU GUUGAUGUGCU | 2795 |
| AD-1963817 | csasgaa(Ahd)GfuUf GfUfugaugugcsusu | 3776 | asAfsgcdAc(Agn)uca acaAfcUfuucugsasa | 4136 | UUCAGAAAGUUG UUGAUGUGCUG | 2754 |
| AD-1963819 | gsasaag(Uhd)UfgUf UfGfaugugcugsgsu | 3777 | asCfscadGc(Agn)cau caaCfaAfcuuucsusg | 4137 | CAGAAAGUUGUU GAUGUGCUGGA | 2820 |
| AD-1963839 | asusucc(Ahd)UfuAf AfAfacaaagggscsu | 3778 | asGfsccdCu(Tgn)ugu uuuAfaUfggaauscsc | 4138 | GGAUUCCAUUAA AACAAAGGGCA | 2729 |
| AD-1963840 | ususcca(Uhd)UfaAf AfAfcaaagggcsasu | 3779 | asUfsgcdCc(Tgn)uug uuuUfaAfuggaasusc | 4139 | GAUUCCAUUAAA ACAAAGGGCAA | 2648 |
| AD-1963845 | ususaaa(Ahd)CfaAf AfGfggcaagagsusu | 3780 | asAfscudCu(Tgn)gcc cuuUfgUfuuuaasusg | 4140 | CAUUAAAACAAA GGGCAAGAGUG | 2812 |
| AD-1963846 | usasaaa(Chd)AfaAf GfGfgcaagagusgsu | 3781 | asCfsacdTc(Tgn)ugc ccuUfuGfuuuuasasu | 4141 | AUUAAAACAAAG GGCAAGAGUGC | 2793 |
| AD-1963848 | asasaca(Ahd)AfgGf GfCfaagagugcsusu | 3782 | asAfsgcdAc(Tgn)cuu gccCfuUfuguuususa | 4142 | UAAAACAAAGGG CAAGAGUGCUG | 2804 |
| AD-1963850 | ascsaaa(Ghd)GfgCf AfAfgagugcugsasu | 3783 | asUfscadGc(Agn)cuc uugCfcCfuuugususu | 4143 | AAACAAAGGGCA AGAGUGCUGAC | 2824 |
| AD-1963853 | asasggg(Chd)AfaGf AfGfugcugacususu | 3784 | asAfsagdTc(Agn)gca cucUfuGfcccuususg | 4144 | CAAAGGGCAAGA GUGCUGACUUC | 2724 |
| AD-1963855 | gsgsgca(Ahd)GfaGf UfGfcugacuucsasu | 3785 | asUfsgadAg(Tgn)cag cacUfcUfugcccsusu | 4145 | AAGGGCAAGAGU GCUGACUUCAC | 2699 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963857 | gscsaag(Ahd)GfuGf CfUfgacuucacsusu | 3786 | asAfsgudGa(Agn)gu cagcAfcUfcuugcscsc | 4146 | GGGCAAGAGUGC UGACUUCACUA | 2727 |
| AD-1963858 | csasaga(Ghd)UfgCf UfGfacuucacusasu | 3787 | asUfsagdTg(Agn)agu cagCfaCfucuugscsc | 4147 | GGCAAGAGUGCU GACUUCACUAA | 2649 |
| AD-1963860 | asgsagu(Ghd)CfuGf AfCfuucacuaascsu | 3788 | asGfsuudAg(Tgn)gaa gucAfgCfacucusug | 4148 | CAAGAGUGCUGA CUUCACUAACU | 4291 |
| AD-1963863 | gsusgcu(Ghd)AfcUf UfCfacuaacuuscsu | 3789 | asGfsaadGu(Tgn)agu gaaGfuCfagcacsusc | 4149 | GAGUGCUGACUU CACUAACUUCG | 2557 |
| AD-1963864 | usgscug(Ahd)CfuUf CfAfcuaacuucsgsu | 3790 | asCfsgadAg(Tgn)uag ugaAfgUfcagcascsu | 4150 | AGUGCUGACUUC ACUAACUUCGA | 2583 |
| AD-1963870 | csusuca(Chd)UfaAf CfUfucgauccuscsu | 3791 | asGfsagdGa(Tgn)cga aguUfaGfugaagsusc | 4151 | GACUUCACUAAC UUCGAUCCUCG | 2589 |
| AD-1963871 | ususcac(Uhd)AfaCf UfUfcgauccucsgsu | 3792 | asCfsgadGg(Agn)ucg aagUfuAfgugaasgsu | 4152 | ACUUCACUAACU UCGAUCCUCGU | 2593 |
| AD-1963893 | gscscuc(Chd)UfuCf CfUfgaauccuusgsu | 3793 | asCfsaadGg(Agn)uuc aggAfaGfgaggcscsa | 4153 | UGGCCUCCUUCC UGAAUCCUUGG | 2688 |
| AD-1963896 | uscscuu(Chd)CfuGf AfAfuccuuggasusu | 3794 | asAfsucdCa(Agn)gga uucAfgGfaaggasgsg | 4154 | CCUCCUUCCUGA AUCCUUGGAUU | 2608 |
| AD-1963920 | gsgsacc(Uhd)AfcCf CfAfggcucacusgsu | 3795 | asCfsagdTg(Agn)gcc uggGfuAfgguccsasg | 4155 | CUGGACCUACCC AGGCUCACUGA | 2840 |
| AD-1963922 | ascscua(Chd)CfcAf GfGfcucacugascsu | 3796 | asGfsucdAg(Tgn)gag ccuGfgGfuaggu scsc | 4156 | GGACCUACCCAG GCUCACUGACC | 2839 |
| AD-1963924 | csusacc(Chd)AfgGf CfUfcacugaccsasu | 3797 | asUfsggdTc(Agn)gug agcCfuGfgguagsgsu | 4157 | ACCUACCCAGGC UCACUGACCAC | 2831 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963926 | ascscca(Ghd)GfcUf CfAfcugaccacscsu | 3798 | asGfsgudGg(Tgn)cag ugaGfcCfugggusasg | 4158 | CUACCCAGGCUC ACUGACCACCC | 2832 |
| AD-1963927 | csusccu(Chd)UfuCf UfGfgaaugugusgsu | 3799 | asCfsacdAc(Agn)uuc cagAfaGfaggagsgsg | 4159 | CCCUCCUCUUCU GGAAUGUGUGA | 2623 |
| AD-1963929 | cscsucu(Uhd)CfuGf GfAfaugugugascsu | 3800 | asGfsucdAc(Agn)cau uccAfgAfagaggsasg | 4160 | CUCCUCUUCUGG AAUGUGUGACC | 2575 |
| AD-1963931 | uscsuuc(Uhd)GfgAf AfUfgugugaccsusu | 3801 | asAfsggdTc(Agn)cac auuCfcAfgaagasgsg | 4161 | CCUCUUCUGGAA UGUGUGACCUG | 2645 |
| AD-1963933 | ususcug(Ghd)AfaUf GfUfgugaccugsgsu | 3802 | asCfscadGg(Tgn)cac acaUfuCfcagaasgsa | 4162 | UCUUCUGGAAUG UGUGACCUGGA | 2783 |
| AD-1963936 | usgsgaa(Uhd)GfuGf UfGfaccuggaususu | 3803 | asAfsaudCc(Agn)ggu cacAfcAfuuccasgsa | 4163 | UCUGGAAUGUGU GACCUGGAUUG | 2778 |
| AD-1963941 | usgsugu(Ghd)AfcCf UfGfgauugugcsusu | 3804 | asAfsgcdAc(Agn)auc cagGfuCfacacasusu | 4164 | AAUGUGUGACCU GGAUUGUGCUC | 2805 |
| AD-1963943 | usgsuga(Chd)CfuGf GfAfuugugcucsasu | 3805 | asUfsgadGc(Agn)caa uccAfgGfucacascsa | 4165 | UGUGUGACCUGG AUUGUGCUCAA | 2761 |
| AD-1963946 | gsasccu(Ghd)GfaUf UfGfugcucaagsgsu | 3806 | asCfscudTg(Agn)gca caaUfcCfaggucsasc | 4166 | GUGACCUGGAUU GUGCUCAAGGA | 2656 |
| AD-1963948 | cscsugg(Ahd)UfuGf UfGfcucaaggasasu | 3807 | asUfsucdCu(Tgn)gag cacAfaUfccaggsusc | 4167 | GACCUGGAUUGU GCUCAAGGAAC | 2619 |
| AD-1963949 | csusgga(Uhd)UfgUf GfCfucaaggaascsu | 3808 | asGfsuudCc(Tgn)uga gcaCfaAfuccagsgsu | 4168 | ACCUGGAUUGUG CUCAAGGAACC | 2581 |
| AD-1963952 | gsasuug(Uhd)GfcUf CfAfaggaacccsasu | 3809 | asUfsggdGu(Tgn)ccu ugaGfcAfcaaucscsa | 4169 | UGGAUUGUGCUC AAGGAACCCAU | 2763 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1963953 | asusugu(Ghd)CfuCf AfAfggaacccasusu | 3810 | asAfsugdGg(Tgn)ucc uugAfgCfacaauscsc | 4170 | GGAUUGUGCUCA AGGAACCCAUC | 2745 |
| AD-1963957 | usgscuc(Ahd)AfgGf AfAfcccaucagscsu | 3811 | asGfscudGa(Tgn)ggg uucCfuUfgagcascsa | 4171 | UGUGCUCAAGGA ACCCAUCAGCG | 2808 |
| AD-1963958 | gscsuca(Ahd)GfgAf AfCfccaucagcsgsu | 3812 | asCfsgcdTg(Agn)ugg guuCfcUfugagcsasc | 4172 | GUGCUCAAGGAA CCCAUCAGCGU | 2782 |
| AD-1963960 | uscsaag(Ghd)AfaCf CfCfaucagcguscsu | 3813 | asGfsacdGc(Tgn)gau gggUfuCfcuugasgsc | 4173 | GCUCAAGGAACC CAUCAGCGUCA | 2794 |
| AD-1963964 | gsgsaac(Chd)CfaUf CfAfgcgucagcsasu | 3814 | asUfsgcdTg(Agn)cgc ugaUfgGfguuccsusu | 4174 | AAGGAACCCAUC AGCGUCAGCAG | 2802 |
| AD-1963966 | asasccc(Ahd)UfcAf GfCfgucagcagscsu | 3815 | asGfscudGc(Tgn)gac gcuGfaUfggguuscsc | 4175 | GGAACCCAUCAG CGUCAGCAGCG | 2827 |
| AD-1963995 | ususgaa(Ahd)UfuCf CfGfuaaacuuasasu | 3816 | asUfsuadAg(Tgn)uua cggAfaUfuucaascsa | 4176 | UGUUGAAAUUCC GUAAACUUAAC | 2631 |
| AD-1963997 | gsasaau(Uhd)CfcGf UfAfaacuuaacsusu | 3817 | asAfsgudTa(Agn)guu uacGfgAfauuucsasa | 4177 | UUGAAAUUCCGU AAACUUAACUU | 2584 |
| AD-1963999 | asasuuc(Chd)GfuAf AfAfcuuaacuuscsu | 3818 | asGfsaadGu(Tgn)aag uuuAfcGfgaauusuc | 4178 | GAAAUUCCGUAA ACUUAACUUCA | 2590 |
| AD-1964000 | asusucc(Ghd)UfaAf AfCfuuaacuucsasu | 3819 | asUfsgadAg(Tgn)uaa guuUfaCfggaaususu | 4179 | AAAUUCCGUAAA CUUAACUUCAA | 2546 |
| AD-1964002 | uscscgu(Ahd)AfaCf UfUfaacuucaasusu | 3820 | asAfsuudGa(Agn)gu uaagUfuUfacggasasu | 4180 | AUUCCGUAAACU UAACUUCAAUG | 2574 |
| AD-1964003 | cscsgua(Ahd)AfcUf UfAfacuucaausgsu | 3821 | asCfsaudTg(Agn)agu uaaGfuUfuacggsasa | 4181 | UUCCGUAAACUU AACUUCAAUGG | 2556 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964016 | csgsaag(Ahd)AfcUf GfAfugguggacsasu | 3822 | asUfsgudCc(Agn)cca ucaGfuUfcuucgsgsg | 4182 | CCCGAAGAACUG AUGGUGGACAA | 2767 |
| AD-1964019 | asgsaac(Uhd)GfaUf GfGfuggacaacsusu | 3823 | asAfsgudTg(Tgn)cca ccaUfcAfguucususc | 4183 | GAAGAACUGAUG GUGGACAACUG | 2705 |
| AD-1964021 | asascug(Ahd)UfgGf UfGfgacaacugsgsu | 3824 | asCfscadGu(Tgn)guc cacCfaUfcaguuscsu | 4184 | AGAACUGAUGGU GGACAACUGGC | 2844 |
| AD-1964022 | ascsuga(Uhd)GfgUf GfGfacaacuggscsu | 3825 | asGfsccdAg(Tgn)ugu ccaCfcAfucagususc | 4185 | GAACUGAUGGUG GACAACUGGCG | 2848 |
| AD-1964024 | usgsaug(Ghd)UfgGf AfCfaacuggcgscsu | 3826 | asGfscgdCc(Agn)guu gucCfaCfcaucasgsu | 4186 | ACUGAUGGUGGA CAACUGGCGCC | 2823 |
| AD-1964043 | csasgcu(Chd)AfgCf CfAfcugaagaascsu | 3827 | asGfsuudCu(Tgn)cag uggCfuGfagcugsgsg | 4187 | CCCAGCUCAGCC ACUGAAGAACA | 2814 |
| AD-1964044 | asgscuc(Ahd)GfcCf AfCfugaagaacsasu | 3828 | asUfsgudTc(Tgn)uca gugGfcUfgagcusgsg | 4188 | CCAGCUCAGCCA CUGAAGAACAG | 2828 |
| AD-1964046 | csuscag(Chd)CfaCf UfGfaagaacagsgsu | 3829 | asCfscudGu(Tgn)cuu cagUfgGfcugagscsu | 4189 | AGCUCAGCCACU GAAGAACAGGC | 2799 |
| AD-1964047 | uscsagc(Chd)AfcUf GfAfagaacaggscsu | 3830 | asGfsccdTg(Tgn)ucu ucaGfuGfgcugasgsc | 4190 | GCUCAGCCACUG AAGAACAGGCA | 2777 |
| AD-1964049 | asgscca(Chd)UfgAf AfGfaacaggcasasu | 3831 | asUfsugdCc(Tgn)guu cuuCfaGfuggcusgsa | 4191 | UCAGCCACUGAA GAACAGGCAAA | 2728 |
| AD-1964053 | ascsuga(Ahd)GfaAf CfAfggcaaaucsasu | 3832 | asUfsgadTu(Tgn)gcc uguUfcUfucagusgsg | 4192 | CCACUGAAGAAC AGGCAAAUCAA | 2621 |
| AD-1964054 | csusgaa(Ghd)AfaCf AfGfgcaaaucasasu | 3833 | asUfsugdAu(Tgn)ugc cugUfuCfuucagsusg | 4193 | CACUGAAGAACA GGCAAAUCAAA | 2602 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964055 | usgsaag(Ahd)AfcAf GfGfcaaaucaasasu | 3834 | asUfsuudGa(Tgn)uug ccuGfuUfcuucasgsu | 4194 | ACUGAAGAACAG GCAAAUCAAAG | 2622 |
| AD-1964056 | gsasaga(Ahd)CfaGf GfCfaaaucaaasgsu | 3835 | asCfsuudTg(Agn)uuu gccUfgUfucuucsasg | 4195 | CUGAAGAACAGG CAAAUCAAAGC | 2658 |
| AD-1964058 | asgsaac(Ahd)GfgCf AfAfaucaaagcsusu | 3836 | asAfsgcdTu(Tgn)gau uugCfcUfguucusc | 4196 | GAAGAACAGGCA AAUCAAAGCUU | 2749 |
| AD-1964059 | gsasaca(Ghd)GfcAf AfAfucaaagcusus u | 3837 | asAfsagdCu(Tgn)uga uuuGfcCfuguucsusu | 4197 | AAGAACAGGCAA AUCAAAGCUUC | 2683 |
| AD-1964060 | asascag(Ghd)CfaAf AfUfcaaagcuuscsu | 3838 | asGfsaadGc(Tgn)uug auuUfgCfcuguuscsu | 4198 | AGAACAGGCAAA UCAAAGCUUCC | 2610 |
| AD-1964063 | asgsgca(Ahd)AfuCf AfAfagcuuccusus u | 3839 | asAfsagdGa(Agn)gcu uugAfuUfugccusgsu | 4199 | ACAGGCAAAUCA AAGCUUCCUUC | 2567 |
| AD-1964064 | gsgscaa(Ahd)UfcAf AfAfgcuuccuuscsu | 3840 | asGfsaadGg(Agn)agc uuuGfaUfuugccsusg | 4200 | CAGGCAAAUCAA AGCUUCCUUCA | 2568 |
| AD-1964067 | asasauc(Ahd)AfaGf CfUfuccuucaasasu | 3841 | asUfsuudGa(Agn)gg aagcUfuUfgauuusgsc | 4201 | GCAAAUCAAAGC UUCCUUCAAAU | 2564 |
| AD-1964068 | asasuca(Ahd)AfgCf UfUfccuucaaasusu | 3842 | asAfsuudTg(Agn)agg aagCfuUfugauususg | 4202 | CAAAUCAAAGCU UCCUUCAAAUA | 2554 |
| AD-1964070 | uscsaaa(Ghd)CfuUf CfCfuucaaauasasu | 3843 | asUfsuadTu(Tgn)gaa ggaAfgCfuuugasusu | 4203 | AAUCAAAGCUUC CUUCAAAUAAG | 2544 |
| AD-1964072 | asasagc(Uhd)UfcCf UfUfcaaauaagsasu | 3844 | asUfscudTa(Tgn)uug aagGfaAfgcuuusgsa | 4204 | UCAAAGCUUCCU UCAAAUAAGAU | 2569 |
| AD-1964073 | asasgcu(Uhd)CfcUf UfCfaaauaagasusu | 3845 | asAfsucdTu(Agn)uuu gaaGfgAfagcuususg | 4205 | CAAAGCUUCCUU CAAAUAAGAUG | 2558 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964075 | gscsuuc(Chd)UfuCf AfAfauaagaugsgsu | 3846 | asCfscadTc(Tgn)uau uugAfaGfgaagcsusu | 4206 | AAGCUUCCUUCA AAUAAGAUGGU | 2563 |
| AD-1964077 | ususccu(Uhd)CfaAf AfUfaagaugguscsu | 3847 | asGfsacdCa(Tgn)cuu auuUfgAfaggaasgsc | 4207 | GCUUCCUUCAAA UAAGAUGGUCC | 2630 |
| AD-1964078 | uscsuu(Chd)AfaAf UfAfagauggucscsu | 3848 | asGfsgadCc(Agn)ucu uauUfuGfaaggasasg | 4208 | CUUCCUUCAAAU AAGAUGGUCCC | 2687 |
| AD-1964081 | ususcaa(Ahd)UfaAf GfAfuggucccasusu | 3849 | asAfsugdGg(Agn)cca ucuUfaUfuugaasgsg | 4209 | CCUUCAAAUAAG AUGGUCCCAUA | 2771 |
| AD-1964102 | gsuscug(Uhd)AfuCf CfAfaauaaugasasu | 3850 | asUfsucdAu(Tgn)auu uggAfuAfcagacsusa | 4210 | UAGUCUGUAUCC AAAUAAUGAAU | 2545 |
| AD-1964103 | uscsugu(Ahd)UfcCf AfAfauaaugaasusu | 3851 | asAfsuudCa(Tgn)uau uugGfaUfacagascsu | 4211 | AGUCUGUAUCCA AAUAAUGAAUC | 2553 |
| AD-1964104 | csusgua(Uhd)CfcAf AfAfuaaugaauscsu | 3852 | asGfsaudTc(Agn)uua uuuGfgAfuacagsasc | 4212 | GUCUGUAUCCAA AUAAUGAAUCU | 2627 |
| AD-1964106 | gsusauc(Chd)AfaAf UfAfaugaaucususu | 3853 | asAfsagdAu(Tgn)cau uauUfuGfgauacsasg | 4213 | CUGUAUCCAAAU AAUGAAUCUUC | 2561 |
| AD-1964107 | usasucc(Ahd)AfaUf AfAfugaaucuuscsu | 3854 | asGfsaadGa(Tgn)uca uuaUfuUfggauascsa | 4214 | UGUAUCCAAAUA AUGAAUCUUCG | 2576 |
| AD-1964108 | asuscca(Ahd)AfuAf AfUfgaaucuucsgsu | 3855 | asCfsgadAg(Agn)uuc auuAfuUfuggausasc | 4215 | GUAUCCAAAUAA UGAAUCUUCGG | 2614 |
| AD-1964116 | asasuga(Ahd)UfcUf UfCfggguguuuscsu | 3856 | asGfsaadAc(Agn)ccc gaaGfaUfucauusasu | 4216 | AUAAUGAAUCUU CGGGUGUUUCC | 2742 |
| AD-1964118 | usgsaau(Chd)UfuCf GfGfguguuuccscsu | 3857 | asGfsggdAa(Agn)cac ccgAfaGfauucasusu | 4217 | AAUGAAUCUUCG GGUGUUUCCCU | 2739 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964139 | ususagc(Uhd)AfaGf CfAfcagaucuascsu | 3858 | asGfsuadGa(Tgn)cug ugcUfuAfgcuaasasg | 4218 | CUUUAGCUAAGC ACAGAUCUACC | 2588 |
| AD-1964140 | usasgcu(Ahd)AfgCf AfCfagaucuacscsu | 3859 | asGfsgudAg(Agn)uc ugugCfuUfagcuasasa | 4219 | UUUAGCUAAGCA CAGAUCUACCU | 2635 |
| AD-1964142 | gscsuaa(Ghd)CfaCf AfGfaucuaccususu | 3860 | asAfsagdGu(Agn)ga ucugUfgCfuuagcsusa | 4220 | UAGCUAAGCACA GAUCUACCUUG | 2594 |
| AD-1964143 | csusaag(Chd)AfcAf GfAfucuaccuusgsu | 3861 | asCfsaadGg(Tgn)aga ucuGfuGfcuuagscsu | 4221 | AGCUAAGCACAG AUCUACCUUGG | 2600 |
| AD-1964150 | csasgau(Chd)UfaCf CfUfuggugauususu | 3862 | asAfsaadTc(Agn)cca aggUfaGfaucugsusg | 4222 | CACAGAUCUACC UUGGUGAUUUG | 2580 |
| AD-1964229 | asasuaa(Ahd)AfuGf UfGfaagacuagsasu | 3863 | asUfscudAg(Tgn)cuu cacAfuUfuuauusasg | 4223 | CUAAUAAAAUGU GAAGACUAGAC | 2768 |
| AD-1964267 | ascsaac(Uhd)GfcUf GfUfggcugguusgsu | 3864 | asCfsaadCc(Agn)gcc acaGfcAfguugusgsu | 4224 | ACACAACUGCUG UGGCUGGUUGG | 2845 |
| AD-1964274 | csusgug(Ghd)CfuGf GfUfuggugcuususu | 3865 | asAfsaadGc(Agn)cca accAfgCfcacagscsa | 4225 | UGCUGUGGCUGG UUGGUGCUUUG | 2813 |
| AD-1964284 | ususggu(Ghd)CfuUf UfGfuuuauggusasu | 3866 | asUfsacdCa(Tgn)aaa caaAfgCfaccaascsc | 4226 | GGUUGGUGCUUU GUUUAUGGUAG | 2651 |
| AD-1964289 | gscsuuu(Ghd)UfuUf AfUfgguaguagsusu | 3867 | asAfscudAc(Tgn)acc auaAfaCfaaagcsasc | 4227 | GUGCUUUGUUUA UGGUAGUAGUU | 2604 |
| AD-1964291 | ususugu(Uhd)UfaUf GfGfuaguaguususu | 3868 | asAfsaadCu(Agn)cua ccaUfaAfacaaasgsc | 4228 | GCUUUGUUUAUG GUAGUAGUUUU | 2723 |
| AD-1964292 | ususguu(Uhd)AfuGf GfUfaguaguuususu | 3869 | asAfsaadAc(Tgn)acu accAfuAfaacaasasg | 4229 | CUUUGUUUAUGG UAGUAGUUUUU | 2773 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964297 | asusggu(Ahd)GfuAf GfUfuuuucugusasu | 3870 | asUfsacdAg(Agn)aaa acuAfcUfaccausasa | 4230 | UUAUGGUAGUAG UUUUUCUGUAA | 2617 |
| AD-1964299 | gsgsuag(Uhd)AfgUf UfUfuucuguaascsu | 3871 | asGfsuudAc(Agn)gaa aaaCfuAfcuaccsasu | 4231 | AUGGUAGUAGUU UUUCUGUAACA | 2748 |
| AD-1964301 | usasgua(Ghd)UfuUf UfUfcuguaacascsu | 3872 | asGfsugdTu(Agn)cag aaaAfaCfuacuascsc | 4232 | GGUAGUAGUUUU UCUGUAACACA | 2740 |
| AD-1964302 | asgsuag(Uhd)UfuUf UfCfuguaacacsasu | 3873 | asUfsgudGu(Tgn)aca gaaAfaAfcuacusasc | 4233 | GUAGUAGUUUUU CUGUAACACAG | 2709 |
| AD-1964303 | gsusagu(Uhd)UfuUf CfUfguaacacasgsu | 3874 | asCfsugdTg(Tgn)uac agaAfaAfacuacsusa | 4234 | UAGUAGUUUUUC UGUAACACAGA | 2730 |
| AD-1964325 | asasuaa(Ghd)AfaUf AfAfaguaccuusgsu | 3875 | asCfsaadGg(Tgn)acu uuaUfuCfuuauusus c | 4235 | GAAAUAAGAAUA AAGUACCUUGA | 2708 |
| AD-1964328 | asasgaa(Uhd)AfaAf GfUfaccuugacsusu | 3876 | asAfsgudCa(Agn)gg uacuUfuAfuucuusasu | 4236 | AUAAGAAUAAAG UACCUUGACUU | 2571 |
| AD-1964329 | asgsaau(Ahd)AfaGf UfAfccuugacusus u | 3877 | asAfsagdTc(Agn)agg uacUfuUfauucusu sa | 4237 | UAAGAAUAAAGU ACCUUGACUUU | 2572 |
| AD-1964331 | asasuaa(Ahd)GfuAf CfCfuugacuuusgsu | 3878 | asCfsaadAg(Tgn)caa gguAfcUfuuauuscsu | 4238 | AGAAUAAAGUAC CUUGACUUUGU | 2706 |
| AD-1964335 | asasgua(Chd)CfuUf GfAfcuuuguucsasu | 3879 | asUfsgadAc(Agn)aag ucaAfgGfuacuususa | 4239 | UAAAGUACCUUG ACUUUGUUCAC | 2579 |
| AD-1964337 | gsusacc(Uhd)UfgAf CfUfuuguucacsasu | 3880 | asUfsgudGa(Agn)caa aguCfaAfgguacsusu | 4240 | AAGUACCUUGAC UUUGUUCACAG | 2661 |
| AD-1964338 | usasccu(Uhd)GfaCf UfUfuguucacasgsu | 3881 | asCfsugdTg(Agn)aca aagUfcAfagguascsu | 4241 | AGUACCUUGACU UUGUUCACAGC | 2667 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964340 | cscsuug(Ahd)CfuUf UfGfuucacagcsasu | 3882 | asUfsgcdTg(Tgn)gaa caaAfgUfcaaggsusa | 4242 | UACCUUGACUUU GUUCACAGCAU | 2618 |
| AD-1964342 | usugac(Uhd)UfuGf UfUfcacagcausgsu | 3883 | asCfsaudGc(Tgn)gug aacAfaAfgucaasgsg | 4243 | CCUUGACUUUGU UCACAGCAUGU | 2615 |
| AD-1964346 | csusuug(Uhd)UfcAf CfAfgcauguagsgsu | 3884 | asCfscudAc(Agn)ugc uguGfaAfcaaagsusc | 4244 | GACUUUGUUCAC AGCAUGUAGGG | 2707 |
| AD-1964353 | csascag(Chd)AfuGf UfAfgggugaugsasu | 3885 | asUfscadTc(Agn)ccc uacAfuGfcugugsasa | 4245 | UUCACAGCAUGU AGGGUGAUGAG | 2790 |
| AD-1964355 | csasgca(Uhd)GfuAf GfGfgugaugagscsu | 3886 | asGfscudCa(Tgn)cac ccuAfcAfugcugsusg | 4246 | CACAGCAUGUAG GGUGAUGAGCA | 2800 |
| AD-1964356 | asgscau(Ghd)UfaGf GfGfugaugagcsasu | 3887 | asUfsgcdTc(Agn)uca cccUfaCfaugcusgsu | 4247 | ACAGCAUGUAGG GUGAUGAGCAC | 2797 |
| AD-1964358 | csasugu(Ahd)GfgGf UfGfaugagcacsusu | 3888 | asAfsgudGc(Tgn)cau cacCfcUfacaugscsu | 4248 | AGCAUGUAGGGU GAUGAGCACUC | 2817 |
| AD-1964388 | gsascua(Ahd)AfaUf GfCfugcuuuuasasu | 3889 | asUfsuadAa(Agn)gca gcaUfuUfuagucsasa | 4249 | UUGACUAAAAUG CUGCUUUUAAA | 2609 |
| AD-1964392 | asasaau(Ghd)CfuGf CfUfuuuaaaacsasu | 3890 | asUfsgudTu(Tgn)aaa agcAfgCfauuuusasg | 4250 | CUAAAAUGCUGC UUUUAAAACAU | 2596 |
| AD-1964395 | asusgcu(Ghd)CfuUf UfUfaaaacauasgsu | 3891 | asCfsuadTg(Tgn)uuu aaaAfgCfagcaususu | 4251 | AAAUGCUGCUUU UAAAACAUAGG | 2549 |
| AD-1964397 | gscsugc(Uhd)UfuUf AfAfaacauaggsasu | 3892 | asUfsccdTa(Tgn)guu uuaAfaAfgcagcsasu | 4252 | AUGCUGCUUUUA AAACAUAGGAA | 2597 |
| AD-1964398 | csusgcu(Uhd)UfuAf AfAfacauaggasasu | 3893 | asUfsucdCu(Agn)ug uuuuAfaAfagcagscsa | 4253 | UGCUGCUUUUAA AACAUAGGAAA | 2591 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964399 | usgscuu(Uhd)UfaAf AfAfcauaggaasasu | 3894 | asUfsuudCc(Tgn)aug uuuUfaAfaagcasgsc | 4254 | GCUGCUUUUAAA ACAUAGGAAAG | 2582 |
| AD-1964402 | ususuua(Ahd)AfaCf AfUfaggaaagusasu | 3895 | asUfsacdTu(Tgn)ccu augUfuUfuaaaasgsc | 4255 | GCUUUUAAAACA UAGGAAAGUAG | 2719 |
| AD-1964407 | asasaca(Uhd)AfgGf AfAfaguagaausgsu | 3896 | asCfsaudTc(Tgn)acu uucCfuAfuguuusus a | 4256 | UAAAACAUAGGA AAGUAGAAUGG | 2733 |
| AD-1964428 | ususgag(Uhd)GfcAf AfAfuccauagcsasu | 3897 | asUfsgcdTa(Tgn)gga uuuGfcAfcucaascsc | 4257 | GGUUGAGUGCAA AUCCAUAGCAC | 2711 |
| AD-1964429 | usgsagu(Ghd)CfaAf AfUfccauagcascsu | 3898 | asGfsugdCu(Agn)ug gauuUfgCfacucasasc | 4258 | GUUGAGUGCAAA UCCAUAGCACA | 2669 |
| AD-1964449 | asasgau(Ahd)AfaUf UfGfagcuaguusasu | 3899 | asUfsaadCu(Agn)gcu caaUfuUfaucuusgsu | 4259 | ACAAGAUAAAUU GAGCUAGUUAA | 2587 |
| AD-1964450 | asgsaua(Ahd)AfuUf GfAfgcuaguuasasu | 3900 | asUfsuadAc(Tgn)agc ucaAfuUfuaucususg | 4260 | CAAGAUAAAUUG AGCUAGUUAAG | 2641 |
| AD-1964454 | asasauu(Ghd)AfgCf UfAfguuaaggcsasu | 3901 | asUfsgcdCu(Tgn)aac uagCfuCfaauuusasu | 4261 | AUAAAUUGAGCU AGUUAAGGCAA | 2722 |
| AD-1964455 | asasuug(Ahd)GfcUf AfGfuuaaggcasasu | 3902 | asUfsugdCc(Tgn)uaa cuaGfcUfcaauusus a | 4262 | UAAAUUGAGCUA GUUAAGGCAAA | 2676 |
| AD-1964459 | gsasgcu(Ahd)GfuUf AfAfggcaaaucsasu | 3903 | asUfsgadTu(Tgn)gcc uuaAfcUfagcucsasa | 4263 | UUGAGCUAGUUA AGGCAAAUCAG | 2620 |
| AD-1964462 | csusagu(Uhd)AfaGf GfCfaaaucaggsusu | 3904 | asAfsccdTg(Agn)uuu gccUfuAfacuagscsu | 4264 | AGCUAGUUAAGG CAAAUCAGGUA | 2758 |
| AD-1964464 | asgsuua(Ahd)GfgCf AfAfaucagguasasu | 3905 | asUfsuadCc(Tgn)gau uugCfcUfuaacusasg | 4265 | CUAGUUAAGGCA AAUCAGGUAAA | 2638 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964467 | usasagg(Chd)AfaAf UfCfagguaaaasusu | 3906 | asAfsuudTu(Agn)ccu gauUfuGfccuuasasc | 4266 | GUUAAGGCAAAU CAGGUAAAAUA | 2585 |
| AD-1964468 | asasggc(Ahd)AfaUf CfAfgguaaaausasu | 3907 | asUfsaudTu(Tgn)acc ugaUfuUfgccuusasa | 4267 | UUAAGGCAAAUC AGGUAAAAUAG | 2628 |
| AD-1964471 | gscsaaa(Uhd)CfaGf GfUfaaaauaguscsu | 3908 | asGfsacdTa(Tgn)uuu accUfgAfuuugcscsu | 4268 | AGGCAAAUCAGG UAAAAUAGUCA | 2695 |
| AD-1964472 | csasaau(Chd)AfgGf UfAfaaauagucsasu | 3909 | asUfsgadCu(Agn)uu uuacCfuGfauuugscsc | 4269 | GGCAAAUCAGGU AAAAUAGUCAU | 2670 |
| AD-1964473 | asasauc(Ahd)GfgUf AfAfaauagucasusu | 3910 | asAfsugdAc(Tgn)auu uuaCfcUfgauuusgsc | 4270 | GCAAAUCAGGUA AAAUAGUCAUG | 2697 |
| AD-1964475 | asuscag(Ghd)UfaAf AfAfuagucaugsasu | 3911 | asUfscadTg(Agn)cua uuuUfaCfcugaususu | 4271 | AAAUCAGGUAAA AUAGUCAUGAU | 2647 |
| AD-1964477 | csasggu(Ahd)AfaAf UfAfgucaugaususu | 3912 | asAfsaudCa(Tgn)gac uauUfuUfaccugsasu | 4272 | AUCAGGUAAAAU AGUCAUGAUUC | 2626 |
| AD-1964478 | asgsgua(Ahd)AfaUf AfGfucaugauuscsu | 3913 | asGfsaadTc(Agn)uga cuaUfuUfuaccusgsa | 4273 | UCAGGUAAAAUA GUCAUGAUUCU | 2660 |
| AD-1964480 | gsusaaa(Ahd)UfaGf UfCfaugauucusasu | 3914 | asUfsagdAa(Tgn)cau gacUfaUfuuuacscsu | 4274 | AGGUAAAAUAGU CAUGAUUCUAU | 2775 |
| AD-1964481 | usasaaa(Uhd)AfgUf CfAfugauucuasusu | 3915 | asAfsuadGa(Agn)uca ugaCfuAfuuuuascsc | 4275 | GGUAAAAUAGUC AUGAUUCUAUG | 2765 |
| AD-1964489 | uscsaug(Ahd)UfuCf UfAfuguaaugusasu | 3916 | asUfsacdAu(Tgn)aca uagAfaUfcaugascsu | 4276 | AGUCAUGAUUCU AUGUAAUGUAA | 2598 |
| AD-1964490 | csasuga(Uhd)UfcUf AfUfguaauguasasu | 3917 | asUfsuadCa(Tgn)uac auaGfaAfucaugsasc | 4277 | GUCAUGAUUCUA UGUAAUGUAAA | 2605 |

(continued)

| Modification | | | | | | |
|---|---|---|---|---|---|---|
| Duplex Name | Sense Sequence 5' to 3' | SEQ ID NO: | Antisense Sequence 5' to 3' | SEQ ID NO: | mRNA Target Sequence 5' to 3' | SEQ ID NO: |
| AD-1964493 | gsasuuc(Uhd)AfuGf UfAfauguaaacscsu | 3918 | asGfsgudTu(Agn)cau uacAfuAfgaaucsasu | 4278 | AUGAUUCUAUGU AAUGUAAACCA | 2689 |
| AD-1964551 | asusgac(Uhd)UfuUf GfAfauuacagasgsu | 3919 | asCfsucdTg(Tgn)aau ucaAfaAfgucaususa | 4279 | UAAUGACUUUUG AAUUACAGAGA | 2654 |
| AD-1964553 | gsascuu(Uhd)UfgAf AfUfuacagagasusu | 3920 | asAfsucdTc(Tgn)gua auuCfaAfaagucsasu | 4280 | AUGACUUUUGAA UUACAGAGAUA | 2716 |
| AD-1964578 | usasuaa(Uhd)UfaGf AfGfuugugauascsu | 3921 | asGfsuadTc(Agn)caa cucUfaAfuuauasasc | 4281 | GUUAUAAUUAGA GUUGUGAUACA | 2791 |
| AD-1964580 | usasauu(Ahd)GfaGf UfUfgugauacasgsu | 3922 | asCfsugdTa(Tgn)cac aacUfcUfaauuasusa | 4282 | UAUAAUUAGAGU UGUGAUACAGA | 2786 |
| AD-1964581 | asasuua(Ghd)AfgUf UfGfugauacagsasu | 3923 | asUfscudGu(Agn)uca caaCfuCfuaauusasu | 4283 | AUAAUUAGAGUU GUGAUACAGAG | 2685 |
| AD-1964582 | asusuag(Ahd)GfuUf GfUfgauacagasgsu | 3924 | asCfsucdTg(Tgn)auc acaAfcUfcuaaususa | 4284 | UAAUUAGAGUUG UGAUACAGAGU | 2714 |
| AD-1964584 | usasgag(Uhd)UfgUf GfAfuacagagusasu | 3925 | asUfsacdTc(Tgn)gua ucaCfaAfcucuasasu | 4285 | AUUAGAGUUGUG AUACAGAGUAU | 2668 |
| AD-1964586 | gsasguu(Ghd)UfgAf UfAfcagaguausasu | 3926 | asUfsaudAc(Tgn)cug uauCfaCfaacucsusa | 4286 | UAGAGUUGUGAU ACAGAGUAUAU | 2666 |
| AD-1964588 | gsusugu(Ghd)AfuAf CfAfgaguauaususu | 3927 | asAfsaudAu(Agn)cuc uguAfuCfacaacsusc | 4287 | GAGUUGUGAUAC AGAGUAUAUUU | 2639 |
| AD-1964610 | csasuuc(Ahd)GfaCf AfAfuauaucausasu | 3928 | asUfsaudGa(Tgn)aua uugUfcUfgaaugsgsa | 4288 | UCCAUUCAGACA AUAUAUCAUAA | 2550 |
| AD-1964611 | asusuca(Ghd)AfcAf AfUfauaucauasasu | 3929 | asUfsuadTg(Agn)uau auuGfuCfugaausgsg | 4289 | CCAUUCAGACAA UAUAUCAUAAC | 2555 |

**Example 2. *In vitro* screening of CA2 siRNA**

**Experimental Methods**

**Cell culture and transfections:**

Hep3b or RPE-J cells (ATCC, Manassas, VA) are grown to near confluence at 37°C in an atmosphere of 5% $CO_2$ in Eagle's Minimum Essential Medium (Gibco) supplemented with 10% FBS (ATCC) before being released from the plate by trypsinization. Transfection is carried out by adding 14.8 μL of Opti-MEM plus 0.2 μL of Lipofectamine RNAiMax per well (Invitrogen, Carlsbad CA. cat # 13778-150) to 5 μL of each siRNA duplex to an individual well in a 96-well plate. The mixture is then incubated at room temperature for 15 minutes. Eighty μL of complete growth media without antibiotic containing ~2 x$10^4$ Hep3B cells is then added to the siRNA mixture. Cells are incubated for 24 hours prior to RNA purification. Single dose experiments are performed at 10 nM, 1 nM and 0.1 nM final duplex concentration.

**Total RNA isolation using DYNABEADS mRNA Isolation Kit:**

Cells are lysed in 75 μL of Lysis/Binding Buffer containing 3 μL of beads per well and are mixed for 10 minutes on an electrostatic shaker. The washing steps are automated on a Biotek EL406, using a magnetic plate support. Beads are washed (in 90 μL) once in Buffer A, once in Buffer B, and twice in Buffer E, with aspiration steps in between. Following a final aspiration, complete 10μL RT mixture is added to each well, as described below.

**cDNA synthesis using ABI High capacity cDNA reverse transcription kit (Applied Biosystems, Foster City, CA, Cat #4368813):**

A master mix of 1μl 10X Buffer, 0.4μl 25X dNTPs, 1 μL Random primers, 0.5 μL Reverse Transcriptase, 0.5 μL RNase inhibitor and 6.6 μL of $H_2O$ per reaction is added per well. Plates are sealed, are agitated for 10 minutes on an electrostatic shaker, and then are incubated at 37 degrees C for 2 hours. Following this, the plates are agitated at 80 degrees C for 8 minutes.

**Real time PCR:**

Two microliter (μL) of cDNA is added to a master mix containing 0.5 μL of human GAPDH TaqMan Probe, 0.5 μL human CA2 probe, 2 μL nuclease-free water and 5 μL Lightcycler 480 probe master mix (Roche Cat # 04887301001) per well in a 384 well plate (Roche cat # 04887301001). Real time PCR is done in a LightCycler480 Real Time PCR system (Roche). Each duplex is tested at least two times and data are normalized to cells transfected with a non-targeting control siRNA. To calculate relative fold change, real time data are analyzed using the ΔΔCt method and are normalized to assays performed with cells transfected with a non-targeting control siRNA.

**Example 3. Single dose *in vitro* screening of CA siRNAs**

**Cell culture and transfections:**

A253 cells or Hela cells were grown to near confluence at 37°C in an atmosphere of 5% $CO_2$ in Eagle's Minimum Essential Medium (Gibco) supplemented with 10% FBS (ATCC) before being released from the plate by trypsinization. Transfection was carried out by adding 14.8 μL of Opti-MEM plus 0.2 μL of Lipofectamine RNAiMax per well (Invitrogen, Carlsbad CA. cat # 13778-150) to 5 μL of each siRNA duplex to an individual well in a 96-well plate. The mixture was then incubated at room temperature for 15 minutes. Eighty μL of complete growth media without antibiotic containing ~1.5 x$10^4$ A253 cells was then added to the siRNA mixture. Cells were incubated for 24 hours prior to RNA purification. A single dose experiment was performed at 10 nM final duplex concentration in A253 cells. For Hela cells, a multi-dose experiment was performed at 0.1 nM, 1 nM, and 10 nM final duplex concentration.

**Total RNA isolation using DYNABEADS mRNA Isolation Kit:**

Cells were lysed in 75 μL of Lysis/Binding Buffer containing 3 μL of beads per well and were mixed for 10 minutes on an electrostatic shaker. The washing steps were automated on a Biotek EL406, using a magnetic plate support. Beads were washed (in 90 μL) once in Buffer A, once in Buffer B, and twice in Buffer E, with aspiration steps in between. Following a final aspiration, complete 10μL RT mixture was added to each well, as described below.

**cDNA synthesis using ABI High capacity cDNA reverse transcription kit (Applied Biosystems, Foster City, CA, Cat #4368813):**

A master mix of 1µl 10X Buffer, 0.4µl 25X dNTPs, 1 µL Random primers, 0.5 µL Reverse Transcriptase, 0.5 µL RNase inhibitor and 6.6 µL of $H_2O$ per reaction was added per well. Plates were sealed, were agitated for 10 minutes on an electrostatic shaker, and then were incubated at 37 degrees C for 2 hours. Following this, the plates were agitated at 80 degrees C for 8 minutes.

**Real time PCR:**

Two microliter (µL) of cDNA was added to a master mix containing 0.5 µL of human GAPDH TaqMan Probe, 0.5 µL human CA2 probe, 2 µL nuclease-free water and 5 µL Lightcycler 480 probe master mix (Roche Cat # 04887301001) per well in a 384 well plate (Roche cat # 04887301001). Real time PCR is done in a LightCycler480 Real Time PCR system (Roche). Each duplex was tested at least two times and data were normalized to cells transfected with a non-targeting control siRNA. To calculate relative fold change, real time data were analyzed using the ΔΔCt method and were normalized to assays performed with cells transfected with a non-targeting control siRNA. The results for A253 cells are shown in Table 11 and the results for Hela cells are shown in Table 12. The results are presented as the pecent message remaining.

**Table 11. CA2 Single Dose Screen in A253 Cells**

| Duplex Name | % CA Message Remaining | | Duplex Name | % CA Message Remaining | |
|---|---|---|---|---|---|
| | Mean | SD | | Mean | SD |
| AD-1447598 | 7.20 | 0.47 | AD-1561130 | 70.78 | 12.81 |
| AD-1561703 | 6.89 | 0.25 | AD-1561122 | 7.10 | 1.78 |
| AD-1561694 | 9.47 | 0.96 | AD-1561116 | 7.00 | 1.56 |
| AD-1561686 | 11.90 | 0.80 | AD-1561112 | 8.15 | 5.14 |
| AD-1561679 | 9.05 | 0.73 | AD-1561106 | 26.09 | 6.97 |
| AD-1561651 | 8.50 | 0.24 | AD-1561100 | 3.10 | 0.99 |
| AD-1561613 | 9.92 | 0.47 | AD-1561092 | 3.09 | 0.86 |
| AD-1561601 | 10.70 | 1.11 | AD-1475424 | 18.12 | 4.10 |
| AD-1561591 | 8.82 | 0.65 | AD-1561072 | 28.20 | 2.22 |
| AD-1561581 | 8.17 | 0.73 | AD-1561066 | 6.47 | 1.72 |
| AD-1561570 | 9.46 | 1.69 | AD-1561056 | 6.94 | 1.51 |
| AD-1561562 | 9.92 | 1.09 | AD-1561050 | 5.54 | 1.17 |
| AD-1561551 | 6.50 | 1.45 | AD-1561043 | 21.07 | 1.67 |
| AD-1561542 | 9.21 | 0.97 | AD-1561037 | 9.53 | 1.21 |
| AD-1561534 | 9.59 | 0.36 | AD-1561031 | 33.42 | 2.51 |
| AD-1561527 | 9.25 | 1.54 | AD-1561015 | 7.38 | 0.60 |
| AD-1561521 | 8.37 | 0.27 | AD-1561009 | 15.37 | 1.80 |
| AD-1561513 | 13.07 | 1.38 | AD-1561002 | 50.31 | 2.37 |
| AD-1561504 | 9.70 | 0.78 | AD-1560996 | 7.63 | 0.53 |
| AD-1561498 | 8.73 | 1.80 | AD-1560989 | 74.31 | 4.13 |
| AD-1561489 | 10.24 | 1.19 | AD-1560976 | 5.17 | 0.44 |
| AD-1561478 | 10.18 | 1.11 | AD-1560970 | 8.59 | 3.64 |
| AD-1561471 | 11.22 | 1.21 | AD-1560963 | 6.77 | 3.46 |
| AD-1561465 | 15.01 | 1.09 | AD-1560954 | 27.23 | 5.41 |
| AD-1561456 | 40.43 | 3.66 | AD-1560948 | 26.42 | 0.76 |

(continued)

| Duplex Name | % CA Message Remaining | | Duplex Name | % CA Message Remaining | |
|---|---|---|---|---|---|
| | Mean | SD | | Mean | SD |
| AD-1561450 | 24.74 | 1.42 | AD-1560941 | 6.44 | 1.39 |
| AD-1561444 | 17.51 | 2.01 | AD-1560930 | 27.16 | 6.71 |
| AD-1561433 | 11.61 | 0.78 | AD-1560921 | 5.44 | 1.37 |
| AD-1561422 | 8.20 | 0.63 | AD-1560915 | 5.44 | 1.54 |
| AD-1561414 | 10.20 | 0.58 | AD-1560904 | 89.40 | 10.68 |
| AD-1561408 | 6.88 | 0.89 | AD-1560895 | 37.15 | 2.86 |
| AD-1561402 | 8.58 | 1.21 | AD-1560892 | 5.67 | 1.90 |
| AD-1561396 | 20.33 | 6.80 | AD-1560880 | 48.60 | 7.79 |
| AD-1561390 | 7.95 | 1.75 | AD-1560874 | 10.82 | 1.27 |
| AD-1561384 | 15.34 | 3.97 | AD-1560862 | 14.92 | 1.69 |
| AD-1561378 | 16.76 | 4.22 | AD-1560843 | 33.98 | 3.79 |
| AD-1561366 | 20.14 | 5.16 | AD-1560851 | 4.79 | 0.38 |
| AD-1561360 | 11.28 | 1.92 | AD-1560845 | 20.26 | 2.36 |
| AD-1561349 | 10.47 | 3.67 | AD-1560837 | 9.58 | 0.89 |
| AD-1561342 | 19.22 | 2.75 | AD-1560816 | 11.19 | 0.83 |
| AD-1561336 | 6.48 | 2.35 | AD-1560810 | 10.58 | 1.27 |
| AD-1561327 | 7.80 | 0.93 | AD-1560804 | 9.93 | 1.78 |
| AD-1561319 | 5.93 | 1.03 | AD-1560798 | 11.16 | 1.31 |
| AD-1561313 | 6.39 | 0.67 | AD-1560792 | 5.26 | 0.83 |
| AD-1561306 | 6.03 | 0.49 | AD-1560783 | 2.91 | 0.76 |
| AD-1561300 | 5.62 | 0.50 | AD-1560777 | 6.73 | 1.62 |
| AD-1561294 | 3.77 | 1.38 | AD-1560765 | 31.74 | 3.45 |
| AD-1561285 | 6.79 | 0.61 | AD-1560759 | 7.85 | 2.77 |
| AD-1561279 | 5.80 | 0.61 | AD-1560752 | 23.53 | 6.36 |
| AD-1561272 | 5.68 | 0.49 | AD-1560745 | 13.34 | 2.85 |
| AD-1561261 | 6.74 | 1.09 | AD-1560735 | 53.63 | 10.67 |
| AD-1561254 | 5.60 | 1.23 | AD-1560726 | 6.10 | 0.45 |
| AD-1561245 | 7.01 | 1.68 | AD-1560720 | 43.72 | 14.97 |
| AD-1561239 | 6.15 | 1.94 | AD-1560711 | 6.65 | 0.92 |
| AD-1561231 | 6.36 | 1.73 | AD-1560701 | 29.81 | 3.54 |
| AD-1561225 | 8.26 | 1.82 | AD-1560693 | 5.41 | 0.94 |
| AD-1561218 | 4.88 | 1.54 | AD-1560684 | 4.00 | 0.86 |
| AD-1561210 | 3.65 | 0.76 | AD-1560678 | 20.06 | 5.49 |
| AD-1561203 | 4.31 | 0.92 | AD-1560672 | 11.26 | 3.35 |
| AD-1561196 | 7.63 | 1.46 | AD-1560665 | 11.82 | 2.15 |
| AD-1561190 | 10.83 | 1.95 | AD-1560655 | 21.40 | 3.19 |
| AD-1561181 | 7.36 | 1.12 | AD-1560644 | 18.35 | 1.71 |
| AD-1561175 | 2.23 | 0.49 | AD-1560638 | 27.36 | 7.62 |

(continued)

| Duplex Name | % CA Message Remaining | | Duplex Name | % CA Message Remaining | |
|---|---|---|---|---|---|
| | Mean | SD | | Mean | SD |
| AD-1561168 | 6.44 | 1.74 | AD-1560628 | 22.60 | 5.09 |
| AD-1446763 | 4.73 | 1.43 | AD-1560622 | 40.19 | 10.35 |
| AD-1561158 | 4.31 | 1.24 | AD-1560617 | 21.41 | 5.73 |
| AD-1561152 | 11.35 | 3.57 | AD-1560600 | 19.85 | 2.56 |
| AD-1561146 | 9.71 | 2.38 | | | |

**Table 12. CA2 Multi-Dose Screen in Hela Cells**

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784188.1 | 7.4 | 3.9 | 10.7 | 1.5 | 12.3 | 2.5 |
| AD-1784196.1 | 13.2 | 2.7 | 15.8 | 6.5 | 32.2 | 9.4 |
| AD-1784204.1 | 15.1 | 4.8 | 21 | 4.8 | 37.4 | 7.7 |
| AD-1784211.1 | 10.4 | 3.2 | 10.1 | 3.7 | 16.5 | 3.5 |
| AD-1784218.1 | 6.4 | 1.6 | 13.3 | 3.2 | 21.4 | 2.9 |
| AD-1784226.1 | 23.8 | 2 | 21.9 | 4.3 | 46 | 9.3 |
| AD-1784233.1 | 15.7 | 3.2 | 19.1 | 5.9 | 34.6 | 6 |
| AD-1784241.1 | 30.1 | 5.7 | 39 | 6.6 | 48.9 | 16.8 |
| AD-1784249.1 | 12.6 | 3.1 | 13.9 | 4 | 28.2 | 8.1 |
| AD-1784256.1 | 15.6 | 3.3 | 19.4 | 4.4 | 23.8 | 1.9 |
| AD-1784263.1 | 22.8 | 5.4 | 25 | 6.5 | 31.2 | 11 |
| AD-1784271.1 | 14.7 | 4.4 | 10.8 | 1.8 | 15.6 | 2.3 |
| AD-1784189.1 | 17.4 | 4.3 | 12.2 | 3.8 | 25.9 | 1 |
| AD-1784197.1 | 24.4 | 4.7 | 29 | 6.7 | 33 | 5.5 |
| AD-1784205.1 | 18 | 2.7 | 22.2 | 9.6 | 40.1 | 9.3 |
| AD-1784212.1 | 23.8 | 3.8 | 13.9 | 2 | 30.6 | 6.8 |
| AD-1784219.1 | 13.1 | 5.1 | 12.4 | 2.4 | 26.9 | 4.1 |
| AD-1784227.1 | 20.3 | 4.4 | 15.1 | 2.9 | 28.9 | 9.1 |
| AD-1784234.1 | 22.1 | 3.9 | 24.6 | 10.6 | 35.3 | 12 |
| AD-1784242.1 | 38 | 3.1 | 41.7 | 8.6 | 50.6 | 7.7 |
| AD-1784250.1 | 18.2 | 3.5 | 20.4 | 8.5 | 31.7 | 9.6 |
| AD-1784257.1 | 15 | 4.8 | 14.4 | 5.9 | 24.5 | 9.9 |
| AD-1784264.1 | 23.5 | 6.6 | 22.8 | 9 | 30.6 | 6.1 |
| AD-1784272.1 | 15.3 | 5.1 | 13.8 | 7.1 | 26.8 | 6 |
| AD-1784190.1 | 8.8 | 3.4 | 10.5 | 1.5 | 18.2 | 4.8 |
| AD-1784198.1 | 24.4 | 4.1 | 25.4 | 5.1 | 23.4 | 8.2 |
| AD-1955 | 79.5 | 6.1 | | | | |
| AD-1784220.1 | 25.3 | 7.3 | 25.3 | 6.2 | 48.8 | 10.5 |
| AD-1784228.1 | 20.2 | 4.6 | 16.5 | 3.4 | 31.8 | 6.7 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784235.1 | 23.4 | 5.9 | 23.6 | 5.8 | 24.3 | 2.5 |
| AD-1784243.1 | 23 | 6.9 | 26.4 | 1.6 | 24.6 | 5.9 |
| AD-1784265.1 | 28.5 | 9.4 | 24.1 | 2.9 | 31.7 | 2.4 |
| AD-1784273.1 | 16.4 | 5.2 | 14.4 | 1.1 | 31.6 | 6.1 |
| AD-1784191.1 | 9.1 | 4.9 | 8.7 | 2.2 | 20.2 | 1.2 |
| AD-1784199.1 | 26.6 | 8.7 | 18 | 2.8 | 44.8 | 14.2 |
| AD-1784206.1 | 25.3 | 6.8 | 30.1 | 9.6 | 74.8 | 17.3 |
| AD-1784213.1 | 25.9 | 9.1 | 12.7 | 1.5 | 33.3 | 5.8 |
| AD-1784221.1 | 29.8 | 6 | 34.4 | 7.6 | 70.4 | 14.3 |
| AD-1784229.1 | 39.8 | 5.5 | 36.9 | 8.1 | 56.2 | 5.6 |
| AD-1784236.1 | 26.9 | 10 | 24.6 | 9 | 39.8 | 10.6 |
| AD-1784244.1 | 22.4 | 4.9 | 32.7 | 10.2 | 30 | 5.1 |
| AD-1784251.1 | 72.8 | 13.6 | 77.5 | 12.6 | 95.7 | 34 |
| AD-1784258.1 | 21.6 | 7.6 | 20.8 | 1.8 | 24 | 6.7 |
| AD-1784266.1 | 16.1 | 3.4 | 25.6 | 2.1 | 19.4 | 7.5 |
| AD-1784274.1 | 53.4 | 6.3 | 51.4 | 14.9 | 51.5 | 11.2 |
| AD-1784192.1 | 15.5 | 6 | 12.7 | 2.9 | 18.1 | 4.6 |
| AD-1784200.1 | 25.1 | 4.2 | 22 | 3.6 | 26.4 | 7.7 |
| AD-1784207.1 | 60.8 | 8.8 | 48.1 | 5.8 | 98.4 | 18.9 |
| AD-1784214.1 | 42.6 | 9.9 | 50.2 | 17.4 | 120.6 | 40.2 |
| AD-1784222.1 | 52.6 | 10.7 | 56.4 | 4.1 | 85.1 | 17.5 |
| AD-1784230.1 | 69.9 | 5.9 | 63.4 | 12.5 | 123.9 | 38.2 |
| AD-1784237.1 | 20.8 | 2.8 | 26.7 | 15.9 | 50.3 | 13.8 |
| AD-1784245.1 | 25 | 3.9 | 25.8 | 8.6 | 70.6 | 19.9 |
| AD-1784252.1 | 46.8 | 8.5 | 60.2 | 16.7 | 101.9 | 24.6 |
| AD-1784259.1 | 29.4 | 4.6 | 19.1 | 3.5 | 23.1 | 5 |
| AD-1784267.1 | 32.3 | 3.4 | 29.1 | 4.5 | 34.3 | 12.5 |
| AD-1784275.1 | 24.9 | 6.8 | 38.6 | 9.8 | 34.9 | 10.6 |
| AD-1784193.1 | 19.5 | 5.2 | 19 | 4.1 | 23.6 | 9 |
| AD-1784201.1 | 32.3 | 1.2 | 22.8 | 3.4 | 34.4 | 5.3 |
| AD-1784208.1 | 66.4 | 7.8 | 63.2 | 20.5 | 90.2 | 21.9 |
| AD-1784215.1 | 52.8 | 10.5 | 56.7 | 12.6 | 72.6 | 7.7 |
| AD-1784223.1 | 77.3 | 25.6 | 69.2 | 17.5 | 87 | 29.8 |
| AD-1784231.1 | 29.8 | 6.5 | 27.9 | 4.4 | 32.1 | 13.3 |
| AD-1784238.1 | 13.1 | 1.8 | 25.9 | 8.8 | 37.9 | 7.4 |
| AD-1784246.1 | 35.5 | 8.8 | 41.9 | 15.7 | 38.3 | 10.6 |
| AD-1784253.1 | 23.8 | 5.2 | 21.3 | 2.6 | 34.7 | 9.7 |
| AD-1784260.1 | 33.1 | 9.8 | 18.8 | 6.1 | 23.1 | 6.8 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784268.1 | 25.1 | 3 | 18.6 | 7.5 | 29.7 | 13.2 |
| AD-1784276.1 | 29.1 | 12.1 | 24.7 | 7.8 | 50 | 10.4 |
| AD-1784194.1 | 18.7 | 6.1 | 16.1 | 5.8 | 29.1 | 9.3 |
| AD-1784202.1 | 63 | 4.5 | 53.5 | 5.7 | 56.6 | 5.7 |
| AD-1784209.1 | 19.2 | 5.4 | 16.2 | 2 | 25.7 | 3 |
| AD-1784216.1 | 23.4 | 4.8 | 19.9 | 5.1 | 33.1 | 8.1 |
| AD-1784224.1 | 49.9 | 6.3 | 33.8 | 6 | 38.7 | 11 |
| AD-1784232.1 | 34.9 | 1.9 | 60.7 | 12.4 | 54.5 | 12.2 |
| AD-1784239.1 | 15.9 | 5.5 | 17.9 | 7.2 | 21.9 | 7.2 |
| AD-1784247.1 | 15.8 | 2.2 | 17 | 4.2 | 28.1 | 3.6 |
| AD-1784254.1 | 42.5 | 10.2 | 39.8 | 14.8 | 46.6 | 13.5 |
| AD-1784261.1 | 38.9 | 6.3 | 31.5 | 11.6 | 35.8 | 9.1 |
| AD-1784269.1 | 36.1 | 8.3 | 29.6 | 10.6 | 40.4 | 7.7 |
| AD-1784277.1 | 15.5 | 4.4 | 19.8 | 8.5 | 20 | 8.6 |
| AD-1784195.1 | 13.1 | 5.3 | 13 | 5.1 | 19.6 | 2.6 |
| AD-1784203.1 | 43.5 | 5.1 | 24.6 | 2.5 | 43.5 | 13.4 |
| AD-1784210.1 | 18.7 | 2.3 | 17.4 | 6.4 | 36.2 | 8.5 |
| AD-1784217.1 | 44 | 7.8 | 33.4 | 10.7 | 61.5 | 19.7 |
| AD-1784225.1 | 49.9 | 9.5 | 53.3 | 17.1 | 59.7 | 18.9 |
| AD-1784240.1 | 25 | 7.5 | 21.4 | 11.6 | 46.1 | 9 |
| AD-1784248.1 | 36.8 | 10.1 | 20.4 | 4.5 | 76.8 | 12 |
| AD-1784255.1 | 60.4 | 14.7 | 54.7 | 21.1 | 65.3 | 16.7 |
| AD-1784262.1 | 13.4 | 3 | 27.9 | 8 | 40 | 12 |
| AD-1784270.1 | 39.5 | 11.6 | 41.7 | 12.8 | 28.2 | 4.8 |
| AD-1784458.1 | 26.6 | 1 | 22.6 | 7 | 22.6 | 7 |
| AD-1784466.1 | 26 | 3.1 | 18 | 4.6 | 24.1 | 6 |
| AD-1784474.1 | 12.5 | 2.3 | 18.1 | 5.2 | 16.5 | 4 |
| AD-1784481.1 | 13.8 | 3.9 | 17.3 | 3 | 25.2 | 6 |
| AD-1784488.1 | 10.2 | 4.6 | 14.4 | 5.8 | 21.4 | 4.2 |
| AD-1784496.1 | 24.7 | 5.8 | 50.6 | 4 | 69.1 | 14.9 |
| AD-1784503.1 | 31.4 | 4.6 | 64.7 | 18.4 | 67.8 | 10.3 |
| AD-1784511.1 | 17.8 | 0.7 | 23.5 | 4.8 | 54 | 13.8 |
| AD-1784519.1 | 39.7 | 9.7 | 52.2 | 12.3 | 56.2 | 10.2 |
| AD-1784526.1 | 37 | 6.1 | 48.6 | 12.4 | 51.2 | 2.4 |
| AD-1784533.1 | 12 | 3.5 | 16.4 | 6 | 38.2 | 10 |
| AD-1784541.1 | 59.3 | 13.2 | 34.8 | 5.8 | 42.1 | 10.2 |
| AD-1784459.1 | 42.7 | 10.2 | 38.4 | 3.4 | 51 | 7.4 |
| AD-1784467.1 | 16.9 | 4.3 | 27.8 | 4.5 | 46.5 | 13 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784475.1 | 16.9 | 1.8 | 14.4 | 4.8 | 21.2 | 3.9 |
| AD-1784482.1 | 24.8 | 4.6 | 29.8 | 6.6 | 59.6 | 15.2 |
| AD-1784489.1 | 25.7 | 5.1 | 20.6 | 5 | 38.5 | 6.4 |
| AD-1784497.1 | 66.4 | 18.4 | 67.3 | 7.6 | 74 | 15.3 |
| AD-1784504.1 | 13.5 | 4.5 | 25.1 | 9.6 | 24.3 | 3.4 |
| AD-1784512.1 | 37.7 | 10.7 | 41.8 | 10.5 | 46.7 | 15.9 |
| AD-1784520.1 | 11.1 | 3.9 | 22.5 | 4.7 | 28.1 | 7.2 |
| AD-1784527.1 | 30.6 | 9.3 | 36.9 | 9.6 | 45.4 | 22.2 |
| AD-1784534.1 | 13.9 | 5.7 | 20.4 | 4.7 | 32.5 | 8.9 |
| AD-1784542.1 | 22.2 | 4.1 | 13.8 | 5.1 | 28.3 | 11.9 |
| AD-1784460.1 | 13.5 | 1.3 | 21.2 | 2.6 | 24.3 | 5.6 |
| AD-1784468.1 | 36.4 | 8.5 | 44.7 | 8.5 | 53.6 | 13.7 |
| AD-1784490.1 | 29.2 | 9.6 | 24.4 | 6.3 | 38.9 | 5.5 |
| AD-1784498.1 | 51.8 | 4.2 | 71.4 | 5.6 | 96.9 | 3.5 |
| AD-1784505.1 | 31 | 9.6 | 50.9 | 5.9 | 46.1 | 3.5 |
| AD-1784513.1 | 57.4 | 14.8 | 51.5 | 9.9 | 63 | 4.5 |
| AD-1784535.1 | 42.5 | 8.8 | 30.3 | 5.2 | 53.9 | 5.1 |
| AD-1784543.1 | 14 | 0.5 | 28.9 | 4.1 | 46.4 | 4.1 |
| AD-1784461.1 | 18 | 5.7 | 17.5 | 5.4 | 23 | 6.2 |
| AD-1784469.1 | 72.7 | 13.2 | 65.3 | 10 | 44.9 | 8.2 |
| AD-1784476.1 | 29.9 | 5.5 | 36.7 | 4.8 | 54.1 | 7.2 |
| AD-1784483.1 | 34.4 | 3.4 | 52.9 | 12.9 | 71.4 | 10.3 |
| AD-1784491.1 | 30.9 | 8.7 | 44.5 | 11.3 | 78.2 | 16.1 |
| AD-1784499.1 | 132.2 | 30.7 | 91.9 | 28.5 | 114.8 | 20.2 |
| AD-1784506.1 | 51 | 16.8 | 78.8 | 22 | 108.9 | 26.3 |
| AD-1784514.1 | 26.4 | 8.4 | 38.8 | 4.9 | 36.9 | 15.8 |
| AD-1784521.1 | 58.5 | 16 | 71.2 | 12.6 | 98.8 | 18 |
| AD-1784528.1 | 47.6 | 6.6 | 56.7 | 13 | 69.4 | 9 |
| AD-1784536.1 | 74.9 | 11.5 | 43.2 | 9.4 | 107.7 | 31.8 |
| AD-1784544.1 | 39.1 | 6.3 | 74.5 | 3.5 | 65.1 | 21.6 |
| AD-1784462.1 | 19.1 | 1.2 | 22.1 | 0.5 | 25.4 | 2.8 |
| AD-1784470.1 | 35.1 | 10 | 45.4 | 11.7 | 64.1 | 11.1 |
| AD-1784477.1 | 33.1 | 7.8 | 45.5 | 7.3 | 60.3 | 6.7 |
| AD-1784484.1 | 24.3 | 2.4 | 28.7 | 3.4 | 39.9 | 1.8 |
| AD-1784492.1 | 48.1 | 13.1 | 56.6 | 12 | 82.4 | 10.4 |
| AD-1784500.1 | 40.3 | 6.9 | 33.4 | 16.8 | 58.6 | 1 |
| AD-1784507.1 | 47.2 | 13.5 | 62.6 | 20.5 | 71.9 | 13.1 |
| AD-1784515.1 | 29.1 | 1.9 | 59.6 | 19.7 | 86.9 | 25.1 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784522.1 | 29.6 | 4.9 | 38.4 | 0.8 | 61.1 | 20.6 |
| AD-1784529.1 | 98.4 | 22.8 | 88.5 | 20.4 | 64.6 | 15.5 |
| AD-1784537.1 | 20.5 | 8.2 | 18.2 | 6.6 | 55.7 | 5.6 |
| AD-1784545.1 | 38 | 10.1 | 26 | 2.8 | 58.3 | 10.4 |
| AD-1784463.1 | 50 | 15.3 | 58 | 10.4 | 52.4 | 6.6 |
| AD-1784471.1 | 30.1 | 2.1 | 47.8 | 10.7 | 50.3 | 7.1 |
| AD-1784478.1 | 53.8 | 8.6 | 64.9 | 8.3 | 68.1 | 15.6 |
| AD-1784485.1 | 23.8 | 7.1 | 28.5 | 8.5 | 32.9 | 4.9 |
| AD-1784493.1 | 34.5 | 10.1 | 33.5 | 11.7 | 48.4 | 14.7 |
| AD-1784501.1 | 78.8 | 20.3 | 70.4 | 28.6 | 89.6 | 23.1 |
| AD-1784508.1 | 21.4 | 5.8 | 46 | 9.5 | 42.5 | 19.5 |
| AD-1784516.1 | 31.1 | 5.7 | 66.3 | 17.3 | 76 | 24.5 |
| AD-1784523.1 | 40.9 | 13 | 60.2 | 12.3 | 70.1 | 21.4 |
| AD-1784530.1 | 61.2 | 18.8 | 62.2 | 10.8 | 63.8 | 18.8 |
| AD-1784538.1 | 79.1 | 26.9 | 73.2 | 13.2 | 73.8 | 38.3 |
| AD-1784546.1 | 21.9 | 7.1 | 17.3 | 7.3 | 40 | 13.6 |
| AD-1784464.1 | 44.3 | 8.1 | 50.6 | 15.3 | 71.4 | 11.4 |
| AD-1784472.1 | 26.5 | 3.8 | 29.7 | 8.7 | 29.5 | 6.5 |
| AD-1784479.1 | 26.6 | 3.9 | 38.9 | 6.4 | 58.9 | 16.9 |
| AD-1784486.1 | 26.2 | 8.9 | 50.5 | 10.7 | 47.6 | 11.2 |
| AD-1784494.1 | 25.2 | 6.6 | 54.1 | 16.8 | 40.7 | 10.5 |
| AD-1784502.1 | 93.8 | 15.5 | 74.6 | 20.4 | 92.5 | 26.3 |
| AD-1784509.1 | 45 | 6.2 | 41.3 | 11.5 | 60.7 | 15.7 |
| AD-1784517.1 | 29 | 7.6 | 30.4 | 6.9 | 48.9 | 11.3 |
| AD-1784524.1 | 77 | 23.1 | 75 | 23.6 | 75.9 | 26.8 |
| AD-1784531.1 | 43.8 | 7.1 | 38 | 12.8 | 92.8 | 22.1 |
| AD-1784539.1 | 30.5 | 4 | 27.7 | 22 | 50 | 16.9 |
| AD-1784547.1 | 25.3 | 1.2 | 22.8 | 6.8 | 46.3 | 18.8 |
| AD-1784465.1 | 30.6 | 7.8 | 45.5 | 11.9 | 46.5 | 15.2 |
| AD-1784473.1 | 27.4 | 5.4 | 26.3 | 7.3 | 44.9 | 6.6 |
| AD-1784480.1 | 47.5 | 9.9 | 67.9 | 13.8 | 60.1 | 13.6 |
| AD-1784487.1 | 34 | 12.8 | 37.3 | 7.1 | 45.5 | 13 |
| AD-1784495.1 | 37 | 6.7 | 79.2 | 22.2 | 74.7 | 6 |
| AD-1784510.1 | 26.5 | 6.6 | 23 | 4.8 | 36.1 | 11.5 |
| AD-1784518.1 | 48.2 | 13.8 | 82.2 | 12.4 | 82.7 | 23.5 |
| AD-1784525.1 | 78.7 | 25.6 | 103 | 34.2 | 88.9 | 21.9 |
| AD-1784532.1 | 29.5 | 4.2 | 32.4 | 8.7 | 53.5 | 24.5 |
| AD-1784540.1 | 106.9 | 27.5 | 86.8 | 21 | 94.4 | 31.8 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784278.1 | 15 | 3.3 | 15 | 8.5 | 23.3 | 7.1 |
| AD-1784286.1 | 12.7 | 4.1 | 16.3 | 5.2 | 24.7 | 2.7 |
| AD-1784294.1 | 31.7 | 2.9 | 41.4 | 6.9 | 36.7 | 6.6 |
| AD-1784301.1 | 60.4 | 9.6 | 73.2 | 11.8 | 71.4 | 13 |
| AD-1784308.1 | 11 | 3.4 | 18 | 3.2 | 38.8 | 10 |
| AD-1784316.1 | 15.4 | 4.2 | 20.5 | 3.3 | 40.3 | 2.7 |
| AD-1784323.1 | 21.2 | 7.5 | 36.4 | 7.3 | 33.9 | 6.6 |
| AD-1784331.1 | 17.2 | 7.4 | 31.7 | 8.2 | 31 | 4.5 |
| AD-1784339.1 | 17.6 | 5.5 | 19.4 | 2.8 | 8.4 | 3.2 |
| AD-1784346.1 | 13.1 | 3.3 | 13.6 | 3 | 23.4 | 6.1 |
| AD-1784353.1 | 12.1 | 8.1 | 10.2 | 3.6 | 13.3 | 3.2 |
| AD-1784361.1 | 14.9 | 5.8 | 12.1 | 2.5 | 24.5 | 7.5 |
| AD-1784279.1 | 12.3 | 3.6 | 20.4 | 6.3 | 31.4 | 7 |
| AD-1784287.1 | 47.8 | 10.5 | 45.7 | 1.1 | 45.4 | 7.3 |
| AD-1784295.1 | 13.3 | 4 | 14.5 | 6.5 | 15.5 | 4.9 |
| AD-1784302.1 | 19.3 | 2.7 | 20.5 | 3.4 | 22.7 | 6.8 |
| AD-1784309.1 | 26.9 | 4.8 | 26.6 | 4.2 | 39.7 | 8.7 |
| AD-1784317.1 | 14.4 | 7.6 | 20 | 3 | 64.2 | 16.2 |
| AD-1784324.1 | 30.4 | 7.3 | 53.2 | 11.4 | 24.8 | 6.9 |
| AD-1784332.1 | 9.6 | 4.1 | 23 | 9.5 | 37.7 | 8 |
| AD-1784340.1 | 29.8 | 10.4 | 38.4 | 14 | 43.4 | 28.7 |
| AD-1784347.1 | 14.4 | 4 | 17.4 | 7.3 | 37.9 | 15 |
| AD-1784354.1 | 28.4 | 11.4 | 29.5 | 7.4 | 31.9 | 5.5 |
| AD-1784362.1 | 12.1 | 8.6 | 22.4 | 10.6 | 29.3 | 12.1 |
| AD-1784280.1 | 19.6 | 6.7 | 22.4 | 3.1 | 33.9 | 11.5 |
| AD-1784288.1 | 26.4 | 6.3 | 44.4 | 8.1 | 60.3 | 12.2 |
| AD-1784310.1 | 19.6 | 5.7 | 51.2 | 12.8 | 60.7 | 14.6 |
| AD-1784318.1 | 53 | 9.7 | 114.8 | 43.9 | 84.4 | 10.1 |
| AD-1784325.1 | 26.7 | 5.8 | 36.3 | 11.1 | 38.3 | 12.7 |
| AD-1784333.1 | 18.6 | 5.9 | 35.7 | 4.7 | 38.1 | 7.4 |
| AD-1784355.1 | 23.2 | 6.1 | 28.6 | 6.8 | 50.3 | 8.3 |
| AD-1784363.1 | 11.2 | 5.6 | 19.2 | 7.6 | 28.5 | 4.7 |
| AD-1784281.1 | 15.1 | 5.9 | 16.4 | 4.3 | 23.1 | 3.1 |
| AD-1784289.1 | 53.4 | 10.1 | 43.7 | 7.6 | 60.6 | 12.1 |
| AD-1784296.1 | 60.6 | 10.9 | 36.5 | 9.4 | 85.8 | 22 |
| AD-1784303.1 | 32.1 | 6.8 | 38.6 | 1 | 38.6 | 4.1 |
| AD-1784311.1 | 40.2 | 4 | 43.2 | 13.1 | 81.6 | 8.8 |
| AD-1784319.1 | 28.7 | 2.4 | 26.5 | 0.3 | 50 | 17.6 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784326.1 | 35.8 | 8.3 | 41.8 | 5.9 | 65.1 | 16.4 |
| AD-1784334.1 | 36 | 9 | 55.2 | 12.1 | 46.5 | 10.9 |
| AD-1784341.1 | 38.6 | 8.8 | 40.7 | 8.4 | 40.2 | 8.8 |
| AD-1784348.1 | 23 | 9.2 | 28.2 | 3 | 44.5 | 7.6 |
| AD-1784356.1 | 18.1 | 8.1 | 23 | 7.4 | 39.7 | 6.8 |
| AD-1784364.1 | 14.2 | 6.5 | 18.3 | 10.7 | 36.7 | 21.7 |
| AD-1784282.1 | 20.5 | 3.7 | 24.7 | 2 | 25 | 4.1 |
| AD-1784290.1 | 32.9 | 6.2 | 34 | 7.9 | 36.1 | 6 |
| AD-1784297.1 | 56.2 | 6.6 | 52.3 | 8.1 | 50.5 | 13.6 |
| AD-1784304.1 | 98.2 | 9.8 | 101.8 | 9.5 | 103.8 | 13.7 |
| AD-1784312.1 | 42.8 | 2.3 | 43.7 | 9.7 | 72.8 | 15 |
| AD-1784320.1 | 41.8 | 4.3 | 39.3 | 7.5 | 83.3 | 18.2 |
| AD-1784327.1 | 26.3 | 2.8 | 32.9 | 6.2 | 59.8 | 4.9 |
| AD-1784335.1 | 21.6 | 2 | 48.9 | 9.4 | 46.4 | 11.9 |
| AD-1784342.1 | 24.9 | 9.4 | 36.3 | 6.1 | 34.4 | 8.4 |
| AD-1784349.1 | 19.7 | 7.5 | 22.5 | 3.5 | 35.8 | 7.1 |
| AD-1784357.1 | 17 | 4.8 | 31 | 8.8 | 51.7 | 14.1 |
| AD-1784365.1 | 31.2 | 1.9 | 59.4 | 3.9 | 58.6 | 5.5 |
| AD-1784283.1 | 25.7 | 4.6 | 27.4 | 10.2 | 22.1 | 6.4 |
| AD-1784291.1 | 24.8 | 5.1 | 19.6 | 7.4 | 34.5 | 10.2 |
| AD-1784298.1 | 23.1 | 6.2 | 29.6 | 11.7 | 27.2 | 4.1 |
| AD-1784305.1 | 28.8 | 7.3 | 23.2 | 2.8 | 27.8 | 9.7 |
| AD-1784313.1 | 34.9 | 7.8 | 31.9 | 7.4 | 43.1 | 6.1 |
| AD-1784321.1 | 31.1 | 9.4 | 39.2 | 2.6 | 55.4 | 8.6 |
| AD-1784328.1 | 40.5 | 9.9 | 50.2 | 8.3 | 60.2 | 11.1 |
| AD-1784336.1 | 20 | 5.8 | 36 | 5.3 | 33.7 | 9.5 |
| AD-1784343.1 | 20.7 | 1.8 | 30.4 | 9.1 | 61.8 | 7.4 |
| AD-1784350.1 | 17.4 | 9.9 | 34.4 | 7.8 | 84.8 | 9.2 |
| AD-1784358.1 | 20.2 | 11 | 19.2 | 1.7 | 42.8 | 11.8 |
| AD-1784366.1 | 20.4 | 10.9 | 19.2 | 4.6 | 38 | 13.3 |
| AD-1784284.1 | 33.2 | 9.7 | 32 | 1.1 | 33.5 | 0.6 |
| AD-1784292.1 | 23.7 | 6.8 | 31.7 | 6.1 | 38.5 | 12.6 |
| AD-1784299.1 | 30.4 | 6.8 | 34.9 | 10.4 | 36.9 | 1.9 |
| AD-1784306.1 | 34.2 | 8.6 | 45.3 | 8.1 | 52.9 | 6.8 |
| AD-1784314.1 | 46.2 | 11.5 | 87.7 | 7.7 | 54.5 | 3.2 |
| AD-1784322.1 | 43 | 9.2 | 83.4 | 10 | 94.9 | 20.4 |
| AD-1784329.1 | 15.8 | 6.1 | 30.4 | 10.4 | 39.8 | 11.8 |
| AD-1784337.1 | 29.3 | 7.8 | 42.1 | 10.5 | 46.3 | 15.9 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784344.1 | 45.4 | 15.2 | 33.3 | 1.3 | 50.7 | 22.5 |
| AD-1784351.1 | 20.5 | 10.4 | 28.2 | 8.4 | 47.8 | 12.7 |
| AD-1784359.1 | 14.2 | 1.3 | 42.2 | 12.6 | 33.8 | 4.8 |
| AD-1784367.1 | 16.3 | 7.9 | 25.2 | 9 | 27.4 | 5.7 |
| AD-1784285.1 | 24 | 3.7 | 19.1 | 1.5 | 21.5 | 3.3 |
| AD-1784293.1 | 26.2 | 5.4 | 21.3 | 5 | 18 | 3.7 |
| AD-1784300.1 | 24.3 | 6.9 | 38.7 | 6 | 36.8 | 7.1 |
| AD-1784307.1 | 32.9 | 8.2 | 52.5 | 8.6 | 44.3 | 10.1 |
| AD-1784315.1 | 25 | 12.1 | 64.4 | 10.2 | 69.2 | 18.3 |
| AD-1784330.1 | 27.8 | 8.9 | 35.1 | 2.7 | 45.7 | 1.2 |
| AD-1784338.1 | 24.4 | 10.8 | 36 | 7.2 | 49.5 | 13.8 |
| AD-1784345.1 | 23.7 | 9.6 | 45.3 | 10.6 | 64.7 | 19 |
| AD-1784352.1 | 16.5 | 7.1 | 26.5 | 7.6 | 46.6 | 12.4 |
| AD-1784360.1 | 23.4 | 1.2 | 66.1 | 14.9 | 66.1 | 21 |
| AD-1784368.1 | 8.6 | 2.2 | 12.6 | 3.6 | 21.5 | 2.2 |
| AD-1784384.1 | 14.5 | 5.4 | 37.3 | 9.8 | 34 | 1 |
| AD-1784391.1 | 16.2 | 1.8 | 24.6 | 5.6 | 22.9 | 4.1 |
| AD-1784398.1 | 18.9 | 4.3 | 18.8 | 2.3 | 8.4 | 2.1 |
| AD-1784406.1 | 36.1 | 9.8 | 45 | 18.6 | 38.2 | 7.6 |
| AD-1784413.1 | 23 | 3.3 | 16 | 3 | 36.4 | 7.9 |
| AD-1784421.1 | 22.8 | 3.3 | 16.1 | 3.3 | 25 | 1.9 |
| AD-1784429.1 | 16.9 | 3.4 | 18.1 | 5 | 34 | 6.9 |
| AD-1784436.1 | 13.5 | 6.2 | 35.1 | 3.6 | 32.5 | 9.4 |
| AD-1784443.1 | 22 | 9 | 28.9 | 6.6 | 50.3 | 11.5 |
| AD-1784451.1 | 16.9 | 7.2 | 19.3 | 5.7 | 21.4 | 4.2 |
| AD-1784369.1 | 15 | 2.7 | 12.4 | 1.8 | 22.6 | 2.8 |
| AD-1784377.1 | 32.5 | 2.8 | 33.4 | 8 | 33.1 | 9.4 |
| AD-1784385.1 | 25.6 | 3.2 | 65.1 | 20.8 | 41.5 | 11.3 |
| AD-1784392.1 | 20.2 | 4.1 | 59.2 | 11.1 | 43.3 | 18.8 |
| AD-1784399.1 | 30.8 | 9.1 | 31.2 | 3.8 | 19.9 | 4.9 |
| AD-1784407.1 | 35.6 | 9.6 | 35.1 | 2.8 | 31.5 | 4.6 |
| AD-1784414.1 | 35.5 | 7.8 | 30.7 | 3.5 | 34 | 13 |
| AD-1784422.1 | 10.8 | 1.4 | 17.8 | 3.2 | 21.8 | 8.1 |
| AD-1784430.1 | 13.5 | 3.5 | 21.2 | 7.5 | 21.2 | 4.9 |
| AD-1784437.1 | 25.3 | 8.1 | 37.8 | 5.8 | 35.1 | 8.3 |
| AD-1784444.1 | 11.2 | 5.6 | 18.3 | 3 | 15.4 | 3.5 |
| AD-1784452.1 | 10.7 | 4.9 | 12.4 | 6.2 | 22.5 | 9.2 |
| AD-1784370.1 | 24.4 | 6.4 | 24.3 | 3.1 | 34.7 | 11 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784378.1 | 14.2 | 5.7 | 41.9 | 13.1 | 32.7 | 7.7 |
| AD-1784400.1 | 55.7 | 12.3 | 87.4 | 16.5 | 66.6 | 14 |
| AD-1784408.1 | 81.7 | 21.8 | 88 | 18.2 | 46.2 | 1.5 |
| AD-1784415.1 | 74.8 | 15.3 | 74.9 | 18.4 | 89 | 12.7 |
| AD-1784423.1 | 30.1 | 5 | 30.7 | 4.7 | 43.6 | 6.3 |
| AD-1784445.1 | 25.5 | 2.7 | 39.5 | 11.1 | 39.3 | 10.9 |
| AD-1784453.1 | 31.2 | 6.5 | 26.4 | 11.6 | 17.6 | 7 |
| AD-1784371.1 | 24.2 | 1.9 | 25.7 | 7.1 | 36.4 | 5 |
| AD-1784379.1 | 32.9 | 3.3 | 60 | 13.9 | 59.7 | 12.1 |
| AD-1784386.1 | 32.7 | 2.9 | 54.2 | 15.7 | 48.7 | 21.5 |
| AD-1784393.1 | 33.8 | 3.6 | 46.8 | 15.6 | 34.8 | 7.6 |
| AD-1784401.1 | 51.3 | 15.2 | 63.4 | 28.2 | 45.8 | 7 |
| AD-1784409.1 | 72.2 | 8.5 | 79.6 | 20.1 | 54.6 | 12.5 |
| AD-1784416.1 | 42.4 | 12.3 | 55.9 | 16.2 | 48.9 | 2.7 |
| AD-1784424.1 | 22.9 | 7.1 | 49.6 | 21.5 | 50.4 | 10.4 |
| AD-1784431.1 | 20.1 | 3.5 | 36.3 | 6.3 | 52.1 | 10.2 |
| AD-1784438.1 | 34.1 | 9.1 | 36.6 | 9.3 | 39.7 | 8.4 |
| AD-1784446.1 | 43.4 | 8.7 | 63.9 | 10.2 | 88 | 24.2 |
| AD-1784454.1 | 33.2 | 9.2 | 22.5 | 3.3 | 33.8 | 11.6 |
| AD-1784372.1 | 22.8 | 3.7 | 29.4 | 6.7 | 24.2 | 3.2 |
| AD-1784380.1 | 53.5 | 18.4 | 80.5 | 9.8 | 70.8 | 13.8 |
| AD-1784387.1 | 28.5 | 5.4 | 53.4 | 27.4 | 51 | 1.9 |
| AD-1784394.1 | 34 | 5.9 | 92.8 | 24.5 | 70.6 | 8.4 |
| AD-1784402.1 | 43.7 | 6.3 | 65.4 | 16.2 | 42.8 | 14.7 |
| AD-1784410.1 | 77.9 | 12.3 | 106.6 | 26.1 | 82.8 | 7.8 |
| AD-1784417.1 | 25.2 | 6.8 | 45.4 | 7.9 | 53.3 | 12.4 |
| AD-1784425.1 | 30.4 | 9 | 40.5 | 12 | 39.3 | 8.8 |
| AD-1784432.1 | 63 | 17.4 | 64 | 19.8 | 70.6 | 13.2 |
| AD-1784439.1 | 37.6 | 9.1 | 46.9 | 15.2 | 44.7 | 12.9 |
| AD-1784447.1 | 32.4 | 8.1 | 50.5 | 19.1 | 44.2 | 2.2 |
| AD-1784455.1 | 42.7 | 11.6 | 60.9 | 3.1 | 75.9 | 12.2 |
| AD-1784373.1 | 15.7 | 5.9 | 37.1 | 13.7 | 40.8 | 4.7 |
| AD-1784381.1 | 47.1 | 12.1 | 106.9 | 9.6 | 71.4 | 9.2 |
| AD-1784388.1 | 32.2 | 5.6 | 38.4 | 8.6 | 27.9 | 8.8 |
| AD-1784395.1 | 31 | 8.3 | 69.2 | 8.8 | 32.7 | 9.5 |
| AD-1784403.1 | 27.2 | 10.8 | 88 | 15.5 | 40.6 | 6.6 |
| AD-1784411.1 | 27.8 | 3.2 | 47.3 | 3.6 | 48.6 | 7.1 |
| AD-1784418.1 | 22.6 | 0.5 | 62.9 | 8.9 | 68.7 | 23.1 |

(continued)

| Duplex Name | % CA Message Remaining | | | | | |
|---|---|---|---|---|---|---|
| | 10 nM Mean | 10 nM SD | 1 nM Mean | 1 nM SD | 0.1 nM Mean | 0.1 nM SD |
| AD-1784426.1 | 26.2 | 8 | 37.5 | 9.8 | 33.7 | 9.7 |
| AD-1784433.1 | 70.9 | 22.4 | 76.7 | 28.8 | 55 | 13.4 |
| AD-1784440.1 | 29.5 | 8.3 | 36.7 | 11.7 | 43.7 | 12.9 |
| AD-1784448.1 | 47.4 | 15 | 43.2 | 16.8 | 62.6 | 21.7 |
| AD-1784456.1 | 15 | 0.8 | 35.8 | 6.5 | 25 | 7 |
| AD-1784382.1 | 26 | 7.1 | 57.6 | 27 | 45.1 | 11.4 |
| AD-1784389.1 | 29.6 | 8.3 | 64.5 | 11.1 | 55.9 | 2.4 |
| AD-1784396.1 | 33.6 | 8.3 | 73.3 | 13.1 | 46.8 | 7.8 |
| AD-1784404.1 | 28.3 | 5.6 | 45.7 | 10.2 | 58.1 | 10.2 |
| AD-1784412.1 | 46.9 | 16.2 | 97.1 | 12.2 | 79.9 | 16.7 |
| AD-1784419.1 | 20.5 | 5.4 | 58 | 6.9 | 36.9 | 7.6 |
| AD-1784427.1 | 55.7 | 13.1 | 56.5 | 5.6 | 67.5 | 11.8 |
| AD-1784434.1 | 37.8 | 0 | 41 | 13.1 | 45.1 | 8.4 |
| AD-1784441.1 | 64.6 | 22 | 106.3 | 18.5 | 79.5 | 23.7 |
| AD-1784449.1 | 18.9 | 3.4 | 55.5 | 5.4 | 35.6 | 4 |
| AD-1784457.1 | 24.8 | 10.7 | 39.5 | 16.5 | 54.1 | 10.8 |
| AD-1784375.1 | 23.4 | 10.5 | 32 | 1.7 | 45.8 | 11.2 |
| AD-1784383.1 | 23.3 | 5.7 | 42.7 | 18.5 | 54 | 12.9 |
| AD-1784390.1 | 30.9 | 6.4 | 72.2 | 22.6 | 50 | 16.2 |
| AD-1784397.1 | 25.4 | 4.2 | 33.6 | 9.5 | 30.1 | 6.1 |
| AD-1784405.1 | 65.2 | 19 | 143.6 | 50.9 | 110.5 | 38.4 |
| AD-1784420.1 | 22.4 | 3.6 | 29.8 | 6.2 | 52.2 | 11.2 |
| AD-1784428.1 | 22.1 | 2.2 | 55.9 | 15.1 | 54.5 | 2.3 |
| AD-1784435.1 | 26.4 | 11.1 | 81.9 | 10.3 | 43.4 | 0.5 |
| AD-1784442.1 | 23.9 | 7.1 | 46.6 | 13.4 | 40.5 | 13.5 |
| AD-1784450.1 | 20 | 0.8 | 46.5 | 12 | 47.3 | 14.6 |

**CA2 Sequences**

SEQ ID NO:1
>NM_000067.3 Homo sapiens carbonic anhydrase 2 (CA2), transcript variant 1, mRNA

ACACAGTGCAGGCGCCCAAGCCGCCGCCGCCAGATCGGTGCCGATTCCTGCCCTGCCCCGACCGCCAGCGCGACCAT
GTCCCATCACTGGGGGTACGGCAAACACAACGGACCTGAGCACTGGCATAAGGACTTCCCCATTGCCAAGGGAGAGC
GCCAGTCCCCTGTTGACATCGACACTCATACAGCCAAGTATGACCCTTCCCTGAAGCCCCTGTCTGTTTCCTATGAT
CAAGCAACTTCCCTGAGGATCCTCAACAATGGTCATGCTTTCAACGTGGAGTTTGATGACTCTCAGGACAAAGCAGT
GCTCAAGGGAGGACCCCTGGATGGCACTTACAGATTGATTCAGTTTCACTTTCACTGGGGTTCACTTGATGGACAAG
GTTCAGAGCATACTGTGGATAAAAAGAAATATGCTGCAGAACTTCACTTGGTTCACTGGAACACCAAATATGGGGAT
TTTGGGAAAGCTGTGCAGCAACCTGATGGACTGGCCGTTCTAGGTATTTTTTTGAAGGTTGGCAGCGCTAAACCGGG
CCTTCAGAAAGTTGTTGATGTGCTGGATTCCATTAAAACAAAGGGCAAGAGTGCTGACTTCACTAACTTCGATCCTC
GTGGCCTCCTTCCTGAATCCTTGGATTACTGGACCTACCCAGGCTCACTGACCACCCCTCCTCTTCTGGAATGTGTG
ACCTGGATTGTGCTCAAGGAACCCATCAGCGTCAGCAGCGAGCAGGTGTTGAAATTCCGTAAACTTAACTTCAATGG
GGAGGGTGAACCCGAAGAACTGATGGTGGACAACTGGCGCCCAGCTCAGCCACTGAAGAACAGGCAAATCAAAGCTT
CCTTCAAATAAGATGGTCCCATAGTCTGTATCCAAATAATGAATCTTCGGGTGTTTCCCTTTAGCTAAGCACAGATC
TACCTTGGTGATTTGGACCCTGGTTGCTTTGTGTCTAGTTTTCTAGACCCTTCATCTCTTACTTGATAGACTTACTA
ATAAAATGTGAAGACTAGACCAATTGTCATGCTTGACACAACTGCTGTGGCTGGTTGGTGCTTTGTTTATGGTAGTA
GTTTTTCTGTAACACAGAATATAGGATAAGAAATAAGAATAAAGTACCTTGACTTTGTTCACAGCATGTAGGGTGAT
GAGCACTCACAATTGTTGACTAAAATGCTGCTTTTAAAACATAGGAAAGTAGAATGGTTGAGTGCAAATCCATAGCA
CAAGATAAATTGAGCTAGTTAAGGCAAATCAGGTAAAATAGTCATGATTCTATGTAATGTAAACCAGAAAAAATAAA
TGTTCATGATTTCAAGATGTTATATTAAAGAAAAACTTTAAAAATTATTATATATTTATAGCAAAGTTATCTTAAAT
ATGAATTCTGTTGTAATTTAATGACTTTTGAATTACAGAGATATAAATGAAGTATTATCTGTAAAAATTGTTATAAT
TAGAGTTGTGATACAGAGTATATTTCCATTCAGACAATATATCATAACTTAATAAATATTGTATTTTAGATATATTC
TCTAATAAAATTCAGAATTCTA

SEQ ID NO:2
>Reverse complement of SEQ ID NO:1

TAGAATTCTGAATTTTATTAGAGAATATATCTAAAATACAATATTTATTAAGTTATGATATATTGTCTGAATGGAAA
TATACTCTGTATCACAACTCTAATTATAACAATTTTTACAGATAATACTTCATTTATATCTCTGTAATTCAAAAGTC
ATTAAATTACAACAGAATTCATATTTAAGATAACTTTGCTATAAATATATAATAATTTTTAAAGTTTTTCTTTAATA
TAACATCTTGAAATCATGAACATTTATTTTTTCTGGTTTACATTACATAGAATCATGACTATTTTACCTGATTTGCC
TTAACTAGCTCAATTTATCTTGTGCTATGGATTTGCACTCAACCATTCTACTTTCCTATGTTTTAAAAGCAGCATTT
TAGTCAACAATTGTGAGTGCTCATCACCCTACATGCTGTGAACAAAGTCAAGGTACTTTATTCTTATTTCTTATCCT
ATATTCTGTGTTACAGAAAAACTACTACCATAAACAAAGCACCAACCAGCCACAGCAGTTGTGTCAAGCATGACAAT
TGGTCTAGTCTTCACATTTTATTAGTAAGTCTATCAAGTAAGAGATGAAGGGTCTAGAAAACTAGACACAAAGCAAC
CAGGGTCCAAATCACCAAGGTAGATCTGTGCTTAGCTAAAGGGAAACACCCGAAGATTCATTATTTGGATACAGACT
ATGGGACCATCTTATTTGAAGGAAGCTTTGATTTGCCTGTTCTTCAGTGGCTGAGCTGGGCGCCAGTTGTCCACCAT
CAGTTCTTCGGGTTCACCCTCCCCATTGAAGTTAAGTTTACGGAATTTCAACACCTGCTCGCTGCTGACGCTGATGG
GTTCCTTGAGCACAATCCAGGTCACACATTCCAGAAGAGGAGGGGTGGTCAGTGAGCCTGGGTAGGTCCAGTAATCC
AAGGATTCAGGAAGGAGGCCACGAGGATCGAAGTTAGTGAAGTCAGCACTCTTGCCCTTTGTTTTAATGGAATCCAG
CACATCAACAACTTTCTGAAGGCCCGGTTTAGCGCTGCCAACCTTCAAAAAAATACCTAGAACGGCCAGTCCATCAG
GTTGCTGCACAGCTTTCCCAAAATCCCCATATTTGGTGTTCCAGTGAACCAAGTGAAGTTCTGCAGCATATTTCTTT
TTATCCACAGTATGCTCTGAACCTTGTCCATCAAGTGAACCCCAGTGAAAGTGAAACTGAATCAATCTGTAAGTGCC
ATCCAGGGGTCCTCCCTTGAGCACTGCTTTGTCCTGAGAGTCATCAAACTCCACGTTGAAAGCATGACCATTGTTGA
GGATCCTCAGGGAAGTTGCTTGATCATAGGAAACAGACAGGGGCTTCAGGGAAGGGTCATACTTGGCTGTATGAGTG
TCGATGTCAACAGGGGACTGGCGCTCTCCCTTGGCAATGGGGAAGTCCTTATGCCAGTGCTCAGGTCCGTTGTGTTT
GCCGTACCCCCAGTGATGGGACATGGTCGCGCTGGCGGTCGGGGCAGGGCAGGAATCGGCACCGATCTGGCGGCGGC
GGCTTGGGCGCCTGCACTGTGT

The following numbered paragraphs further describe aspects and embodiemnts of the invention:

1. A double stranded ribonucleic acid (dsRNA) agent for inhibiting expression of Carbonic anhydrase 2 (CA2), wherein the dsRNA agent comprises a sense strand and an antisense strand forming a double stranded region, wherein the antisense strand comprises a nucleotide sequence comprising at least 15 contiguous nucleotides, with 0, 1, 2, or 3 mismatches, from one of the antisense sequences listed in any one of Tables 3-10, and wherein the sense strand comprises a nucleotide sequence comprising at least 15 contiguous nucleotides, with 0, 1, 2, or 3 mismatches, from a sense sequence listed in any one of Tables 3-10 that corresponds to the antisense sequence and wherein the dsRNA agent comprises at least one modified nucleotide.

2. The dsRNA agent of paragraph 1, wherein at least one of the sense strand and the antisense strand is conjugated to one or more lipophilic moieties.

3. The dsRNA agent of paragraph 2, wherein the lipophilic moiety is conjugated via a linker or carrier.

4. The dsRNA agent of paragraph 2 or 3, wherein one or more lipophilic moieties are conjugated to one or more internal positions on at least one strand.

5. The dsRNA agent of paragraph 4, wherein the one or more lipophilic moieties are conjugated to one or more internal positions on at least one strand via a linker or carrier.

6. The dsRNA agent of any one of paragraphs 2-5, wherein the lipophilic moiety is an aliphatic, alicyclic, or polyalicyclic compound.

7. The dsRNA agent of paragraph 6, wherein the lipophilic moiety contains a saturated or unsaturated C16 hydrocarbon chain.

8. The dsRNA agent of any one of paragraphs 2-7, wherein the lipophilic moiety is conjugated via a carrier that replaces one or more nucleotide(s) in the internal position(s) or the double stranded region.

9. The dsRNA agent of any one of paragraphs 2-7, wherein the lipophilic moiety is conjugated to the double-stranded iRNA agent via a linker containing an ether, thioether, urea, carbonate, amine, amide, maleimide-thioether, disulfide, phosphodiester, sulfonamide linkage, a product of a click reaction, or carbamate.

10. The double-stranded iRNA agent of any one of paragraphs 2-8, wherein the lipophilic moiety is conjugated to a nucleobase, sugar moiety, or internucleosidic linkage.

11. The dsRNA agent of any of the preceding paragraphs, wherein no more than five of the sense strand nucleotides and not more than five of the nucleotides of the antisense strand are unmodified nucleotides.

12. The dsRNA agent of any of the preceding paragraphs, wherein all of the nucleotides of the sense strand and all of the nucleotides of the antisense strand comprise a modification.

13. The dsRNA agent of any of the preceding paragraphs, wherein at least one of the modified nucleotides is selected from the group consisting of a deoxy-nucleotide, a 3'-terminal deoxythimidine (dT) nucleotide, a 2'-O-methyl modified nucleotide, a 2'-fluoro modified nucleotide, a 2'-deoxy-modified nucleotide, a locked nucleotide, an unlocked nucleotide, a conformationally restricted nucleotide, a constrained ethyl nucleotide, an abasic nucleotide, a 2'-amino-modified nucleotide, a 2'-O-allyl-modified nucleotide, 2'-C-alkyl-modified nucleotide, a 2'-methoxyethyl modified nucleotide, a 2'-O-alkyl-modified nucleotide, a morpholino nucleotide, a phosphoramidate, a non-natural base comprising nucleotide, a tetrahydropyran modified nucleotide, a 1,5-anhydrohexitol modified nucleotide, a cyclohexenyl modified nucleotide, a nucleotide comprising a phosphorothioate group, a nucleotide comprising a methylphosphonate group, a nucleotide comprising a 5'-phosphate, a nucleotide comprising a 5'-phosphate mimic, a glycol modified nucleotide, and a 2-O-(N-methylacetamide) modified nucleotide; and combinations thereof.

14. The dsRNA agent of any of the preceding paragraphs, wherein at least one strand comprises a 3' overhang of at least 2 nucleotides.

15. The dsRNA agent of any of the preceding paragraphs, wherein the double stranded region is 15-30 nucleotide pairs in length.

16. The dsRNA agent of paragraph 15, wherein the double stranded region is 17-23 nucleotide pairs in length.

17. The dsRNA agent of any of the preceding paragraphs, wherein each strand has 19-30 nucleotides.

18. The dsRNA agent of any of the preceding paragraphs, wherein the agent comprises at least one phosphorothioate or methylphosphonate internucleotide linkage.

19. The dsRNA agent of any one of paragraphs 2-18, further comprising a targeting ligand, e.g., a ligand that targets an ocular tissue.

20. The dsRNA agent of paragraph 19, wherein the ocular tissue is ciliary epithelium, an optic nerve, a trabecular

meshwork, a juxtacanalicular tissue, a ganglion (e.g., including a retinal ganglion), episcleral veins or a Schlemm's canal (e.g., including an endothelial cell).

21. The dsRNA agent of any one of the preceding paragraphs, further comprising a phosphate or phosphate mimic at the 5'-end of the antisense strand.

22. The dsRNA agent of paragraph 21, wherein the phosphate mimic is a 5'-vinyl phosphonate (VP).

23. The dsRNA of any one of paragraphs 1-22 wherein the dsRNA agent targets a hotspot region of an mRNA encoding CA2.

24. A dsRNA agent that targets a hotspot region of a Carbonic anhydrase 2 (CA2) mRNA.

25. A cell containing the dsRNA agent of any one of paragraphs 1-24.

26. A pharmaceutical composition for inhibiting expression of a CA2, comprising the dsRNA agent of any one of paragraphs 1-24 and a pharmaceutically acceptable buffer.

27. A method of inhibiting expression of CA2 in a cell, the method comprising:

a. contacting the cell with the dsRNA agent of any one of paragraphs 1-24, or a pharmaceutical composition of paragraph 26; and
b. maintaining the cell produced in step (a) for a time sufficient to reduce levels of CA2 mRNA, CA2 protein, or both of CA2 mRNA and protein, thereby inhibiting expression of CA2 in the cell.

28. The method of paragraph 27, wherein the cell is within a subject.

29. The method of paragraph 28, wherein the subject is a human.

30. The method of paragraph 29, wherein the subject has been diagnosed with a CA2-associated disorder.

31. A method of treating a subject diagnosed with a CA2-associated disorder comprising administering to the subject a therapeutically effective amount of the dsRNA agent of any one of paragraphs 1-24 or a pharmaceutical composition of paragraph 26, thereby treating the disorder.

32. The method of paragraph 31, wherein the CA2-associated disorder is glaucoma.

33. The method of paragraph 31 or 32, wherein treating comprises amelioration of at least one sign or symptom of the disorder.

34. The method of any one of paragraphs 31-33, wherein the treating comprises one or more of (a) inhibiting or reducing intraocular pressure; (b) inhibiting or reducing the expression or activity of CA2; (c) decreasing the amount of aqueous humor; (d) inhibiting or reducing optic nerve damage; (e) inhibiting or reducing retinal ganglion cell death; (f) medication to reduce intraocular pressure; (g) laser treatment; (h) surgery; (i) or trabeculectomy.

35. The method of any one of paragraphs 24-34, wherein the dsRNA agent is administered to the subject intraocularly, intravenously, or topically.

36. The method of paragraph 35, wherein the intraocular administration comprises intravitreal administration (e.g., intravitreal injection), transscleral administration (e.g., transscleral injection), subconjunctival administration (e.g., subconjunctival injection), retrobulbar administration (e.g., retrobulbar injection), intracameral administration (e.g., intracameral injection), or subretinal administration (e.g., subretinal injection).

37. The method of any one of paragraphs 24-36, further comprising administering to the subject an additional agent or therapy comprising one or more of a prostaglandin analog, a beta blocker, an alpha-adrenergic agonist, a carbonic anhydrase inhibitor, or an anti-CA2 agent suitable for treatment or prevention of a CA2-associated disorder.

## Claims

1. A double stranded ribonucleic acid (dsRNA) agent for inhibiting expression of Carbonic anhydrase 2 (CA2),

   wherein the dsRNA agent comprises a sense strand and an antisense strand forming a double stranded region, wherein the antisense strand comprises a nucleotide sequence comprising at least 15 contiguous nucleotides, with 0, 1, 2, or 3 mismatches, from SEQ ID NO: 1717, and
   wherein the sense strand comprises a nucleotide sequence comprising at least 15 contiguous nucleotides, with 0, 1, 2, or 3 mismatches, from SEQ ID NO: 1388;
   and wherein the dsRNA agent comprises at least one modified nucleotide.

2. The dsRNA agent of claim 1,

   wherein the antisense strand comprises a nucleotide sequence comprising 0 mismatches from SEQ ID NO: 1717; and
   wherein the sense strand comprises a nucleotide sequence comprising 0 mismatches, from SEQ ID NO: 1388.

3. The dsRNA agent of claim 1 or 2,
   wherein all of the nucleotides on the antisense strand and all of the nucleotides on the sense strand comprise a modification.

4. The dsRNA agent of claim 3,

   wherein the antisense strand comprises a nucleotide sequence of SEQ ID NO: 2377, and
   wherein the antisense strand comprises a nucleotide sequence of SEQ ID NO: 2039.

5. The dsRNA agent of any one of claims 1-4, wherein at least one of the sense strand and the antisense strand is conjugated to a lipophilic moiety.

6. The dsRNA agent of any one of claims 1-4, wherein the sense strand is conjugated to a lipophilic moiety.

7. The dsRNA agent of claim 6, wherein the lipophilic moiety is conjugated to one or more internal positions selected from the group consisting of positions 4-8 and 13-18 on the sense strand, counting from the 5'end.

8. The dsRNA agent of claim 7, wherein the lipophilic moiety is conjugated to one or more internal positions selected from the group consisting of positions 5, 6, 7, 15, and 17 on the sense strand, counting from the 5'-end.

9. The dsRNA agent of claim 8, wherein the lipophilic moiety is conjugated to position 6, counting from the 5'-end of the sense strand.

10. The dsRNA agent of any one of claims 5-9, wherein the lipophilic moiety is an aliphatic, alicyclic, or polyalicyclic compound.

11. The dsRNA agent of claim 10, wherein the lipophilic moiety contains a saturated or unsaturated C16 hydrocarbon chain.

12. The dsRNA agent of claim 11, wherein the C16 hydrocarbon chain is conjugated to the dsRNA agent as shown in the following structure:

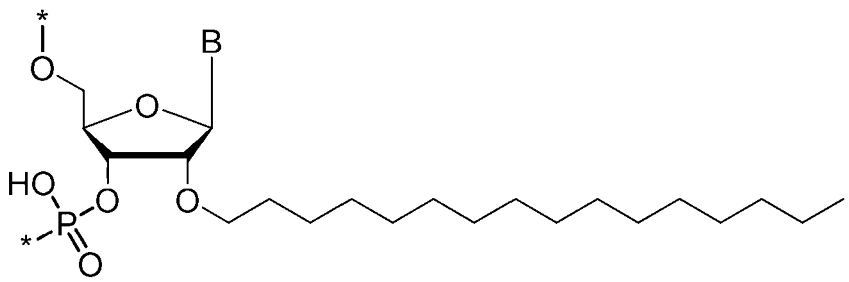

wherein * denotes a bond to an adjacent nucleotide, and B is a nucleobase or a nucleobase analog, optionally wherein B is adenine.

**13.** A pharmaceutical composition for inhibiting expression of a CA2, comprising the dsRNA agent of any one of claims 1-12 and a pharmaceutically acceptable buffer.

**14.** The dsRNA agent of any one of claims 1-12 or a pharmaceutical composition of claim 13 for use in treating a subject diagnosed with a CA2-associated disorder, wherein the use comprises administering to the subject a therapeutically effective amount of the dsRNA agent of any one of claims 1-11 or a pharmaceutical composition of claim 12, thereby treating the disorder.

**15.** The method of claim 14, wherein the CA2-associated disorder is glaucoma.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63194073 A
- US 63289319 A
- US 2019031170 W
- US 20070111963 A
- US 2005226848 A
- US 5032401 A
- US 5607677 A
- US 20050281781 A
- US US B
- US 8101348 B
- US 6858225 B
- US 6815432 B
- US 8158601 B
- US 8058069 B
- US 20060240093
- US 20070135372 A
- WO 2009082817 A
- US 3687808 A
- US 4469863 A
- US 4476301 A
- US 5023243 A
- US 5177195 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541316 A
- US 5550111 A
- US 5563253 A
- US 5571799 A
- US 5587361 A
- US 5625050 A
- US 6028188 A
- US 6124445 A
- US 6160109 A
- US 6169170 B
- US 6172209 B
- US 6239265 B
- US 6277603 B
- US 6326199 B
- US 6346614 B
- US 6444423 B
- US 6531590 B
- US 6534639 B
- US 6608035 B
- US 6683167 B
- US 6858715 B
- US 6867294 B
- US 6878805 B
- US 7015315 B
- US 7041816 B
- US 7273933 B
- US 7321029 B
- US RE39464 A
- US 5034506 A
- US 5166315 A
- US 5185444 A
- US 5214134 A
- US 5216141 A
- US 5235033 A
- US 564562 A
- US 5264564 A
- US 5405938 A
- US 5434257 A
- US 5470967 A
- US 5489677 A
- US 5541307 A
- US 5561225 A
- US 5596086 A
- US 5602240 A
- US 5608046 A
- US 5610289 A
- US 5618704 A
- US 5623070 A
- US 5663312 A
- US 5633360 A
- US 5677437 A
- US 5677439 A
- US 5539082 A
- US 5714331 A
- US 5719262 A
- US 8314227 B
- US 4981957 A
- US 5118800 A
- US 5319080 A
- US 5359044 A
- US 5393878 A
- US 5446137 A

- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5700920 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5681941 A
- US 6015886 A
- US 6147200 A
- US 6166197 A
- US 6222025 A
- US 6235887 A
- US 6380368 A
- US 6528640 A
- US 6639062 A
- US 6617438 A
- US 7045610 A
- US 7427672 A
- US 7495088 A
- US 5750692 A
- US 7399845 B
- US 8278283 B
- US 8278425 B
- US 20040171570 A
- US 7427672 B
- US 8278426 B
- US 6268490 B
- US 6670461 B
- US 6794499 B
- US 6998484 B
- US 7053207 B
- US 7084125 B
- WO 0066604 A, Wengel
- WO 9914226 A
- US 20130190383 A
- WO 2013036868 A
- US 20130096289 A
- US 20130011922 A
- US 20110313020 A
- WO 2011005861 A
- US 20120157511 A
- WO 2013075035 A
- WO 2007091269 A
- WO 2010141511 A
- WO 2007117686 A
- WO 2009014887 A
- WO 2011031520 A
- US 7858769 B
- US 3904682 A
- US 4009197 A
- US 3228831 A
- US 7626014 B
- WO 2014179620 A
- WO 2014179627 A
- US 8106022 B
- WO 2011133876 A
- WO 20100011895 A
- US 4828979 A
- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A

- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- US 6294664 A
- US 6320017 A
- US 6576752 A
- US 6783931 A
- US 6900297 A
- US 7037646 A
- US 8106022 A
- WO 9402595 A
- US 7427605 B
- WO 0022113 A, Skillern, A.
- WO 0022114 A, Conrad
- US 6054299 A, Conrad
- US 6143520 A
- US 5665557 A
- US 5981276 A
- WO 9412649 A
- US 5436146 A
- US 5252479 A
- US 5139941 A
- WO 9413788 A
- WO 9324641 A
- US 6747014 B
- US 4837028 A
- WO 8804924 A
- US 5543152 A, Webb
- WO 9713499 A, Lim
- US 4426330 A
- US 4534899 A
- EP 0445131 B1
- WO 9004384 A
- US 5013556 A, Woodle
- US 5356633 A
- US 5213804 A, Martin
- EP 0496813 B1
- WO 9105545 A
- US 5225212 A, Martin
- WO 9420073 A, Zalipsky
- WO 9610391 A, Choi
- US 5540935 A, Miyazaki
- US 5556948 A, Tagawa
- WO 9640062 A, Thierry
- US 5264221 A, Tagawa
- US 5665710 A, Rahman
- WO 9704787 A, Love
- WO 0003683 A
- US 5976567 A
- US 5981501 A
- US 6534484 B
- US 6586410 B
- WO 9640964 A
- US 61107998 A
- US 05623008 A
- WO 2008042973 A
- WO 2009127060 A
- US 61148366
- US 61156851
- US 61185712
- US 61228373
- US 61239686
- US 2010022614 W
- US 61244834
- US 61185800
- US 1028224 W
- US 0963933 W
- US 61175770
- US 1033777 W
- US 6887906 B
- US 20030027780 A
- US 6191105 B
- US 7063860 B
- US 7070802 B
- US 7157099 B
- US 5705188 A, Junichi
- WO 9730731 A, Lollo

**Non-patent literature cited in the description**


- **LI et al.** *Proc. Natl. Acad. Sci. U.S.A.*, 2006, vol. 103, 17337-42
- **LIMA et al.** *Cell*, 2012, vol. 150, 883-894
- **SHARP et al.** *Genes Dev.*, 2001, vol. 15, 485
- **BERNSTEIN et al.** *Nature*, 2001, vol. 409, 363
- **NYKANEN et al.** *Cell*, 2001, vol. 107, 309
- **ELBASHIR et al.** *Genes Dev.*, 2001, vol. 15, 188
- **TETKO et al.** *J. Chem. Inf. Comput. Sci.*, 2001, vol. 41, 1407-21
- **ELBASHIR et al.** *EMBO*, 2001, vol. 20, 6877-6888
- **JACKSON et al.** *Nat. Biotechnol.*, 2003, vol. 21, 635-637
- **LIN et al.** *Nucleic Acids Res.*, 2005, vol. 33 (14), 4527-4535

- Current protocols in nucleic acid chemistry. John Wiley & Sons, Inc.
- **NIELSEN et al.** *Science*, 1991, vol. 254, 1497-1500
- **MARTIN et al.** *Helv. Chim. Acta*, 1995, vol. 78, 486-504
- Modified Nucleosides in Biochemistry. **HERDEWIJN, P.** Biotechnology and Medicine. Wiley-VCH, 2008
- **KROSCHWITZ, J. L**. The Concise Encyclopedia of Polymer Science and Engineering. John Wiley & Sons, 1990, 858-859
- **ENGLISCH et al.** Angewandte Chemie. *International Edition*, 1991, vol. 30, 613
- **SANGHVI, Y S.** dsRNA Research and Applications. CRC Press, 1993, vol. 15, 289-302
- **SANGHVI, Y. S.** ; **CROOKE, S. T.** ; **LEBLEU, B.** dsRNA Research and Applications. CRC Press, 1993, 276-278
- **ELMEN, J. et al.** *Nucleic Acids Research*, 2005, vol. 33 (1), 439-447
- **MOOK, OR. et al.** *Mol Canc Ther*, 2007, vol. 6 (3), 833-843
- **GRUNWELLER, A. et al.** *Nucleic Acids Research*, 2003, vol. 31 (12), 3185-3193
- **CHATTOPADHYAYA et al.** *J. Org. Chem.*, 2009, vol. 74, 118-134
- *Nuc. Acids Symp. Series*, 2008, vol. 52, 133-134
- **FLUITER et al.** *Mol. Biosyst.*, 2009, vol. 10, 1039
- **WANG et al.** *Am. Chem. Soc.*, 2000, vol. 122 (36), 8595-8602
- **LIN** ; **MATTEUCCI**. *J. Am. Chem. Soc.*, 1998, vol. 120, 8531-8532
- **LETSINGER et al.** *Proc. Natl. Acid. Sci. USA*, 1989, vol. 86, 6553-6556
- **MANOHARAN et al.** *Biorg. Med. Chem. Let.*, 1994, vol. 4, 1053-1060
- **MANOHARAN et al.** *Ann. N.Y. Acad. Sci.*, 1992, vol. 660, 306-309
- **MANOHARAN et al.** *Biorg. Med. Chem. Let.*, 1993, vol. 3, 2765-2770
- **OBERHAUSER et al.** *Nucl. Acids Res.*, 1992, vol. 20, 533-538
- **SAISON-BEHMOARAS et al.** *EMBO J*, 1991, vol. 10, 1111-1118
- **KABANOV et al.** *FEBS Lett.*, 1990, vol. 259, 327-330
- **SVINARCHUK et al.** *Biochimie*, 1993, vol. 75, 49-54
- **MANOHARAN et al.** *Tetrahedron Lett.*, 1995, vol. 36, 3651-3654
- **SHEA et al.** *Nucl. Acids Res.*, 1990, vol. 18, 3777-3783
- **MANOHARAN et al.** *Nucleosides & Nucleotides*, 1995, vol. 14, 969-973
- **MISHRA et al.** *Biochim. Biophys. Acta*, 1995, vol. 1264, 229-237
- **CROOKE et al.** *J. Pharmacol. Exp. Ther.*, 1996, vol. 277, 923-937
- **LAM et al.** *Nature*, 1991, vol. 354, 82-84
- **ZITZMANN et al.** *Cancer Res.*, 2002, vol. 62, 5139-43
- **AOKI et al.** *Cancer Gene Therapy*, 2001, vol. 8, 783-787
- **HAUBNER et al.** *Jour. Nucl. Med.*, 2001, vol. 42, 326-336
- **SIMEONI et al.** *Nucl. Acids Res.*, 2003, vol. 31, 2717-2724
- **MIKHAILOV**. *Tetrahedron Letters*, 1985, vol. 26 (17), 2059
- **KUBO, T. et al.** *Biochem. Biophys. Res. Comm.*, 2007, vol. 365 (1), 54-61
- **LETSINGER et al.** *Proc. Natl. Acad. Sci. USA*, 1989, vol. 86, 6553
- **MANOHARAN et al.** *Bioorg. Med. Chem. Lett.*, 1994, vol. 4, 1053
- **MANOHARAN et al.** *Ann. N.Y. Acad. Sci.*, 1992, vol. 660, 306
- **MANOHARAN et al.** *Bioorg. Med. Chem. Let.*, 1993, vol. 3, 2765
- **OBERHAUSER et al.** *Nucl. Acids Res.*, 1992, vol. 20, 533
- **SAISON-BEHMOARAS et al.** *EMBO J.*, 1991, vol. 10, 111
- **KABANOV et al.** *FEBS Lett.*, 1990, vol. 259, 327
- **SVINARCHUK et al.** *Biochimie*, 1993, vol. 75, 49
- **MANOHARAN et al.** *Tetrahedron Lett.*, 1995, vol. 36, 3651
- **SHEA et al.** *Nucl. Acids Res.*, 1990, vol. 18, 3777
- **MANOHARAN et al.** *Nucleosides & Nucleotides*, 1995, vol. 14, 969
- **MISHRA et al.** *Biochim. Biophys. Acta*, 1995, vol. 1264, 229
- **CROOKE et al.** *J. Pharmacol. Exp. Ther.*, 1996, vol. 277, 923
- **AKHTAR S.** ; **JULIAN RL.** *Trends Cell. Biol.*, 1992, vol. 2 (5), 139-144
- **TOLENTINO, MJ. et al.** *Retina*, 2004, vol. 24, 132-138
- **REICH, SJ. et al.** *Mol. Vis.*, 2003, vol. 9, 210-216
- **PILLE, J. et al.** *Mol. Ther.*, 2005, vol. 11, 267-274
- **KIM, WJ. et al.** *Mol. Ther.*, 2006, vol. 14, 343-350
- **LI, S. et al.** *Mol. Ther.*, 2007, vol. 15, 515-523
- **DORN, G. et al.** *Nucleic Acids*, 2004, vol. 32, e49
- **TAN, PH. et al.** *Gene Ther.*, 2005, vol. 12, 59-66

- **MAKIMURA, H. et al.** *BMC Neurosci.*, 2002, vol. 3, 18
- **SHISHKINA, GT. et al.** *Neuroscience*, 2004, vol. 129, 521-528
- **THAKKER, ER. et al.** *Proc. Natl. Acad. Sci. U.S.A.*, 2004, vol. 101, 17270-17275
- **AKANEYA,Y. et al.** *J. Neurophysiol.*, 2005, vol. 93, 594-602
- **HOWARD, KA. et al.** *Mol. Ther.*, 2006, vol. 14, 476-484
- **ZHANG, X. et al.** *J. Biol. Chem.*, 2004, vol. 279, 10677-10684
- **BITKO, V. et al.** *Nat. Med.*, 2005, vol. 11, 50-55
- **SOUTSCHEK, J. et al.** *Nature*, 2004, vol. 432, 173-178
- **MCNAMARA, JO. et al.** *Nat. Biotechnol.*, 2006, vol. 24, 1005-1015
- **KIM SH. et al.** *Journal of Controlled Release*, 2008, vol. 129 (2), 107-116
- **SORENSEN, DR. et al.** *J. Mol. Biol*, 2003, vol. 327, 761-766
- **VERMA, UN. et al.** *Clin. Cancer Res.*, 2003, vol. 9, 1291-1300
- **ARNOLD, AS et al.** *J. Hypertens.*, 2007, vol. 25, 197-205
- **ZIMMERMANN, TS. et al.** *Nature*, 2006, vol. 441, 111-114
- **CHIEN, PY. et al.** *Cancer Gene Ther.*, 2005, vol. 12, 321-328
- **PAL, A. et al.** *Int J. Oncol.*, 2005, vol. 26, 1087-1091
- **BONNET ME. et al.** *Pharm. Res.*, 16 August 2008
- **AIGNER, A.** *J. Biomed. Biotechnol.*, 2006, 71659
- **LIU, S.** *Mol. Pharm.*, 2006, vol. 3, 472-487
- **TOMALIA, DA. et al.** *Biochem. Soc. Trans.*, 2007, vol. 35, 61-67
- **YOO, H. et al.** *Pharm. Res.*, 1999, vol. 16, 1799-1804
- **COUTURE, A et al.** *TIG.*, 1996, vol. 12, 5-10
- **GASSMANN et al.** *Proc. Natl. Acad. Sci. USA*, 1995, vol. 92, 1292
- **DOCHERTY et al.** *FASEB J.*, 1994, vol. 8, 20-24
- **MILLER et al.** *Meth. Enzymol.*, 1993, vol. 217, 581-599
- **BOESEN et al.** *Biotherapy*, 1994, vol. 6, 291-302
- **CLOWES et al.** *J. Clin. Invest.*, 1994, vol. 93, 644-651
- **KIEM et al.** *Blood*, 1994, vol. 83, 1467-1473
- **SALMONS** ; **GUNZBERG**. *Human Gene Therapy*, 1993, vol. 4, 129-141
- **GROSSMAN** ; **WILSON**. *Curr. Opin. in Genetics and Devel.*, 1993, vol. 3, 110-114
- **KOZARSKY** ; **WILSON**. *Current Opinion in Genetics and Development*, 1993, vol. 3, 499-503
- **BOUT et al.** *Human Gene Therapy*, 1994, vol. 5, 3-10
- **ROSENFELD et al.** *Science*, 1991, vol. 252, 431-434
- **ROSENFELD et al.** *Cell*, 1992, vol. 68, 143-155
- **MASTRANGELI et al.** *J. Clin. Invest.*, 1993, vol. 91, 225-234
- **WANG et al.** *Gene Therapy*, 1995, vol. 2, 775-783
- **XIA H et al.** *Nat. Biotech.*, 2002, vol. 20, 1006-1010
- **WALSH et al.** *Proc. Soc. Exp. Biol. Med.*, 1993, vol. 204, 289-300
- **SAMULSKI R et al.** *J. Virol.*, 1987, vol. 61, 3096-3101
- **FISHER K J et al.** *J. Virol.*, 1996, vol. 70, 520-532
- **SAMULSKI R et al.** *J. Virol.*, 1989, vol. 63, 3822-3826
- **RABINOWITZ J E et al.** *J Virol*, 2002, vol. 76, 791-801
- **ROSOFF**. Pharmaceutical Dosage Forms, Lieberman. Marcel Dekker, Inc., 1988, vol. 1, 245
- **WANG et al.** *Biochem. Biophys. Res. Commun.*, 1987, vol. 147, 980-985
- **ZHOU et al.** *Journal of Controlled Release*, 1992, vol. 19, 269-274
- **WEINER et al.** *Journal of Drug Targeting*, 1992, vol. 2, 405-410
- **PLESSIS et al.** *Antiviral Research*, 1992, vol. 18, 259-265
- **HU et al.** *S.T.P. Pharma. Sci.*, 1994, vol. 4 (6), 466
- **ALLEN et al.** *FEBS Letters*, 1987, vol. 223, 42
- **WU et al.** *Cancer Research*, 1993, vol. 53, 3765
- **GABIZON et al.** *Proc. Natl. Acad. Sci. U.S.A.*, 1988, vol. 85, 6949
- **SUNAMOTO et al.** *Bull. Chem. Soc. Jpn.*, 1980, vol. 53, 2778
- **ILLUM et al.** *FEBS Lett.*, 1984, vol. 167, 79
- **KLIBANOV et al.** *FEBS Lett.*, 1990, vol. 268, 235
- **BLUME et al.** *Biochimica et Biophysica Acta*, 1990, vol. 1029, 91
- **RIEGER**. Pharmaceutical Dosage Forms. Marcel Dekker, Inc., 1988, 285
- **GREEN, T.W. et al.** PROTECTIVE GROUPS IN ORGANIC SYNTHESIS. Wiley-Interscience, 1999
- **ALLEN, LV.** ; **POPOVICH NG.** ; **ANSEL HC.** Ansel's Pharmaceutical Dosage Forms and Drug Delivery Systems. Lippincott Williams & Wilkins, 2004

- **IDSON**. Pharmaceutical Dosage Forms. Marcel Dekker, Inc., 1988, vol. 1, 199
- **ROSOFF**. Pharmaceutical Dosage Forms. Marcel Dekker, Inc., 1988, vol. 1, 245
- **BLOCK**. Pharmaceutical Dosage Forms. Marcel Dekker, Inc., 1988, vol. 2, 335
- **HIGUCHI et al.** Remington's Pharmaceutical Sciences. Mack Publishing Co., 1985, 301
- **RIEGER**. Pharmaceutical Dosage Forms. Marcel Dekker, Inc., 1988, vol. 1, 285
- **BLOCK**. Pharmaceutical Dosage Forms. Marcel Dekker, Inc., 1988, vol. 1, 335
- **ALLEN, LV.** ; **POPOVICH NG.** ; **ANSEL HC.** Ansel's Pharmaceutical Dosage Forms and Drug Delivery Systems,. Lippincott Williams & Wilkins, 2004
- **LEUNG** ; **SHAH**. Controlled Release of Drugs: Polymers and Aggregate Systems. VCH Publishers, 1989, 185-215
- **SCHOTT**. Remington's Pharmaceutical Sciences. Mack Publishing Co., 1985, 271
- **CONSTANTINIDES et al.** *Pharmaceutical Research*, 1994, vol. 11, 1385-1390
- **RITSCHEL**. *Meth. Find. Exp. Clin. Pharmacol.*, 1993, vol. 13, 205
- **CONSTANTINIDES et al.** *Pharmaceutical Research*, 1994, vol. 11, 1385
- **HO et al.** *J. Pharm. Sci.*, 1996, vol. 85, 138-143
- **LEE et al.** *Critical Reviews in Therapeutic Drug Carrier Systems*, 1991, 92
- **MALMSTEN, M.** Surfactants and polymers in drug delivery. Informa Health Care, 2002
- **LEE et al.** *ritical Reviews in Therapeutic Drug Carrier Systems*, 1991, 92
- **TAKAHASHI et al.** *J. Pharm. Pharmacol.*, 1988, vol. 40, 252
- **TOUITOU, E. et al.** Enhancement in Drug Delivery. CRC Press, 2006
- **MURANISHI**. *Critical Reviews in Therapeutic Drug Carrier Systems*, 1990, vol. 7, 1-33
- **EL HARIRI et al.** *J. Pharm. Pharmacol.*, 1992, vol. 44, 651-654
- **BRUNTON et al.** Goodman & Gilman's The Pharmacological Basis of Therapeutics. McGraw-Hill, 1996, 934-935
- **LEE et al.** Critical Reviews in Therapeutic Drug Carrier Systems. 1991, 92
- **SWINYARD**. Remington's Pharmaceutical Sciences. Mack Publishing Co., 1990, 782-783
- **YAMAMOTO et al.** *J. Pharm. Exp. Ther.*, 1992, vol. 263, 25
- **YAMASHITA et al.** *J. Pharm. Sci.*, 1990, vol. 79, 579-583
- **JARRETT, J.** *Chromatogr.*, 1993, vol. 618, 315-339
- **KATDARE, A. et al.** Excipient development for pharmaceutical, biotechnology, and drug delivery. CRC Press, 2006
- **BUUR et al.** *J. Control Rel.*, 1990, vol. 14, 43-51
- **YAMASHITA et al.** *J. Pharm. Pharmacol.*, 1987, vol. 39, 621-626
- **MIYAO et al.** *DsRNA Res. Dev.*, 1995, vol. 5, 115-121
- **TAKAKURA et al.** *DsRNA & Nucl. Acid Drug Dev.*, 1996, vol. 6, 177-183